# EUROPEAN PATENT APPLICATION

(11) **EP 4 066 896 A1**
(43) Date of publication of application: **05.10.2022**
(21) Application number: 20893619.5
(22) Date of filing: 26.11.2020
(51) Int. Cl.: A61P 7/00, A61P 21/00, A61P 25/00, A61P 25/04, A61P 29/00, A61P 31/12, A61P 33/06, A61P 35/00, A61P 35/02, A61P 35/04, A61P 43/00, C07D 401/12, C07D 401/14, C07D 403/12, C07D 405/12, C07D 405/14, C07D 417/12, C07D 417/14, C07D 471/04, C07D 491/107, C07D 209/42

(54) **G9A INHIBITOR**

(30) Priority: 27.11.2019 JP 2019213990
(71) Applicant: RIKEN, Wako-shi, Saitama 351-0198 (JP); Tokyo University of Pharmacy & Life Sciences, Hachioji-shi, Tokyo 192-0392 (JP); Microbial Chemistry Research Foundation, Tokyo 141-0021 (JP)
(72) Inventor: SUMIYA Tatsunobu, Shimotsuga-gun, Tochigi 329-0114 (JP); NISHIGAYA Yosuke, Shimotsuga-gun, Tochigi 329-0114 (JP); NAMIE Ryosuke, Shimotsuga-gun, Tochigi 329-0114 (JP); HASHIMOTO Noriaki, Shimotsuga-gun, Tochigi 329-0114 (JP); ITO Akihiro, Hachioji-shi, Tokyo 192-0392 (JP); SHIRAI Fumiyuki, Wako-shi, Saitama 351-0198 (JP); KIKUZATO Ko, Wako-shi, Saitama 351-0198 (JP); YOSHIDA Minoru, Wako-shi, Saitama 351-0198 (JP)
(74) Representative: J A Kemp LLP
(86) International application number: PCT/JP2020/043966
(87) International publication number: WO 2021/106988

(57) **Abstract**

A compound represented by the general formula (I) given below or a pharmacologically acceptable salt thereof has been found to have a strong G9a inhibitory effect. The compound (I) or the pharmacologically acceptable salt thereof inhibits G9a and thereby has high usefulness for the treatment, prevention or suppression of various pathological conditions (proliferative disease such as cancer, β-globin abnormality, fibrosis, pain, neurodegenerative disease, Prader-Willi syndrome, malaria, viral infection, myopathy, autism, etc.).

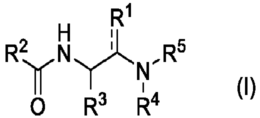

## Description

### Technical Field

The present invention relates to a derivative or a pharmacologically acceptable salt thereof that has a histone methyltransferase G9a inhibitory effect and is useful as a medicament, and a pharmaceutical composition comprising the same and pharmaceutical use thereof.

### Background Art

The lysine methylation of histone is biochemical reaction to add a methyl group to an ε amino group of a lysine residue of histone by using S-adenosylmethionine (SAM) as a methyl group donor. The lysine methylation of histone plays an important role in transcriptional regulation and is important for various biological processes including cell proliferation and cell differentiation. Histone methyltransferase (also called lysine methyltransferase) is known as an enzyme that catalyzes the lysine methylation reaction of histone (Non Patent Literature 1).

G9a and GLP (G9a like protein) are major enzymes that catalyze the mono- and dimethylation of a lysine residue at position 9 of histone H3 (H3K9me1 and H3K9me2). These enzymes are also known as EHMT2 and EHMT1 (euchromatin histone-lysine N-methyltransferases 2 and 1).

H3K9me2 is an epigenetic mark related to transcriptional repression. G9a and GLP are involved in epigenetic transcriptional repression through H3K9me2.

Accordingly, the inhibition of G9a is considered useful for the control of biological processes, such as cell proliferation and cell differentiation, mediated by transcriptional repression by H3K9me2. Examples of diseases for which a G9a inhibitor may be effective include β-globin abnormality such as sickle cell disease, stomach cancer, hepatocellular cancer, leukemia such as acute myeloid leukemia and chronic myeloid leukemia, uterine cervical cancer, neuroblastoma, glioma, pancreatic cancer, colorectal cancer, squamous cell carcinoma of the head and neck, breast cancer, lung cancer, ovary cancer, melanoma, fibrosis such as lung fibrosis and renal fibrosis, pain, neurodegenerative disease such as Alzheimer's disease, Prader-Willi syndrome, malaria, viral infection such as foot-and-mouth disease and vesicular stomatitis, cardiomyopathy, myopathy, and autism. Further, the possibility has been suggested that the inhibition of G9a is also effective for the suppression of cancer metastasis. In addition, the inhibition of G9a has been shown to be also effective for sex change. (Non Patent Literatures 1 to 24)

For example, BIX-01294 (Patent Literature 1), quinazolines (Patent Literature 2), 2-aminoindoles (Patent Literature 3), heteroaryls (Patent Literature 4), and tricyclic compounds (Patent Literature 5) are known as compounds having G9a inhibitory activity. However, these compounds structurally differ from the compound of the present invention.

### Citation List

### Patent Literature

Patent Literature 1: International Publication No. WO 2012/023285
Patent Literature 2: International Publication No. WO 2013/140148
Patent Literature 3: US2015274660
Patent Literature 4: JP Patent Publication (Kokai) No. 2019-513778 A (2019)
Patent Literature 5: JP Patent Publication (Kokai) No. 2019-511472 A (2019)

### Non Patent Literature

Non Patent Literature 1: Krivega I. et al., Blood. Vol. 126, No. 5, pp. 665-672, 2015
Non Patent Literature 2: Lin X. et al., Oncol. Rep. Vol. 35, No. 5, pp. 3041-3049, 2016
Non Patent Literature 3: Qin J. et al., Oncol Lett. Vol. 15, No. 6, pp. 9757-9765, 2017
Non Patent Literature 4: Lehnertz B. et al., Genes Dev. Vol. 28, No. 4, pp. 317-327, 2014
Non Patent Literature 5: Chen R.J. et al., Oncotarget. Vol. 8, No. 37, pp. 62081-62098, 2017
Non Patent Literature 6: Ke X.X. et al., Anticancer Drugs. Vol. 24, No. 5, pp. 484-493, 2013
Non Patent Literature 7: Guo A.S. et al., Mol. Med. Rep. Vol. 14, No. 5, pp. 4613-4621, 2016
Non Patent Literature 8: Pan M.R. et al., Oncotarget. Vol. 7, No. 38, pp. 61136-61151, 2016
Non Patent Literature 9: Zhang J. et al., Oncotarget. Vol. 6, No. 5, pp. 2917-2927, 2015
Non Patent Literature 10: Li K.C. et al., Mol. Cancer. Vol. 13, No. 172, 2014
Non Patent Literature 11: Dong C. et al., J. Clin. Invest. Vol. 122, No. 4, pp. 1469-1486, 2012
Non Patent Literature 12: Chen M.W. et al., Cancer Res. Vol. 70, No. 20, pp. 7830-7840, 2010
Non Patent Literature 13: Hua K.T. et al., Mol Cancer. Vol. 13, No. 189, 2014
Non Patent Literature 14: Loh S.W. et al., PLoS One. Vol. 9, No. 7, e103915, 2014
Non Patent Literature 15: Ligresti G. et al., JCI Insight. Vol. 5, pii: 127111, 2019
Non Patent Literature 16: Irifuku T. et al., Kidney Int. Vol. 89, No. 1, pp. 147-157, 2016
Non Patent Literature 17: Sharma M. et al., Aging Cell. Vol. 16, No. 5, pp. 1062-1072, 2017
Non Patent Literature 18: Laumet G. et al., Nat. Neurosci. Vol. 18, No. 12, pp. 1746-1755, 2015
Non Patent Literature 19: Kim Y. et al., Nat. Med. Vol. 23, No. 2, pp. 213-222, 2017
Non Patent Literature 20: Singh N. et al., J. Interferon Cytokine Res. Vol. 36, No. 1, pp. 37-47, 2016
Non Patent Literature 21: Ow J.R. et al., Sci. Rep. Vol. 6, 34163, 2016
Non Patent Literature 22: Wang Z.J. et al., Mol. Psychiatry. doi: 10.1038/s41380-019-0351-2, 2019
Non Patent Literature 23: Miura S. et al., Am. J. Dermatopathol. Vol. 36, No. 3, pp. 211-216, 2014
Non Patent Literature 24: Kuroki S. et al., PLoS Genet. Vol. 13, No. 9, e1007034, 2017

### Summary of Invention

### Technical Problem

The present invention has been made in light of the problems of the conventional techniques. An object of the present invention is to provide a compound and a pharmacologically acceptable salt thereof which have excellent G9a inhibitory activity, are useful in the treatment of, for example, β-globin abnormality such as sickle cell disease, and are also useful in the treatment of other diseases including proliferative disease such as cancer, fibrosis, pain, neurodegenerative disease, Prader-Willi syndrome, malaria, viral infection, myopathy, and autism, and a G9a inhibitor and a pharmaceutical composition comprising the same. Another object of the present invention is to provide a method for producing the compound and the pharmacologically acceptable salt thereof, and an intermediate compound useful in the production.

Any compound that has an excellent G9a inhibitory effect and is capable of serving as a sufficiently satisfactory medicament has not yet been found as prophylactic and therapeutic drugs for various pathological conditions mentioned above.

An object of the present invention is to provide a compound having a G9a inhibitory effect.

### Solution to Problem

The present inventors have conducted diligent studies and consequently completed the present invention by finding that a compound represented by the general formula (I) given below (hereinafter, also referred to as a compound (I)), or a pharmacologically acceptable salt thereof has a G9a inhibitory effect and can be sufficiently satisfied as a medicament.

Specifically, the present invention is as follows.

[1] A compound represented by the general formula (I): 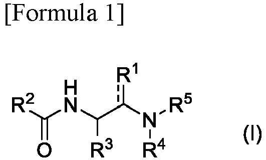 wherein
   R¹ is an oxygen atom, a nitrogen atom or a hydrogen atom;
   when R¹ is an oxygen atom or a nitrogen atom, the bond between R¹ and the carbon atom is a double bond;
   when R¹ is a hydrogen atom, the bond between R¹ and the carbon atom is a single bond;
   R² is the following A1), A2) or A3), and ^{∗} represents a binding position to -CO- in the formula (I): 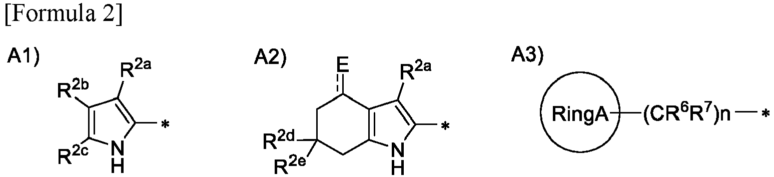 E is an oxygen atom or a hydrogen atom;
      when E is an oxygen atom, the bond between E and the carbon atom is a double bond;
      when E is a hydrogen atom, the bond between E and the carbon atom is a single bond;
      R^{2a}, R^{2b} and R^{2c} are each independently a hydrogen atom, a C₁ to C₆ alkyl group, a C₂ to C₆ alkenyl group, a halo-Ci to C₆ alkyl group, a C₁ to C₆ alkoxy group, a C₁ to C₆ alkylamino group, a C₁ to C₆ acyl group, a C₁ to C₆ alkoxycarbonyl group, a C₃ to C₁₀ cycloalkyl group or a hydroxy-Ci to C₆ alkyl group (the C₁ to C₆ alkyl group, the C₂ to C₆ alkenyl group, the halo-C₁ to C₆ alkyl group, the C₁ to C₆ alkoxy group, the C₁ to C₆ alkylamino group, the C₁ to C₆ acyl group, the C₁ to C₆ alkoxycarbonyl group, the C₃ to C₁₀ cycloalkyl group and the hydroxy-Ci to C₆ alkyl group are each optionally substituted with one or more substituents selected from the group consisting of a C₁ to C₆ alkyl group, a C₁ to C₆ alkoxy group, a C₁ to C₆ alkylamino group and a hydroxy-Ci to C₆ alkyl group, and these substituents are optionally bonded to each other to form a ring);
      R^{2b} and R^{2c} are optionally bonded to each other to form a ring;
      R^{2d} and R^{2e} are each independently a C₁ to C₆ alkyl group or a hydroxy-C₁ to C₆ alkyl group; R^{2d} and R^{2e} are optionally bonded to each other to form a ring;
      R⁶ and R⁷ are each independently a hydrogen atom, a C₁ to C₆ alkyl group, an aromatic hydrocarbon ring group, a C₃ to C₁₀ cycloalkyl group, a 5- to 10-membered heteroaryl group or a 3- to 10-membered heterocycloalkyl group;
      R⁶ and R⁷ are optionally bonded to each other to form a ring;
      n is 0 or 1;
      RingA is an aromatic hydrocarbon ring group, a C₃ to C₁₀ cycloalkyl group, a 5- to 10-membered heteroaryl group or a 3- to 10-membered heterocycloalkyl group and is optionally substituted with R⁸ and R⁹;
      R⁸ is a hydrogen atom, a halogen atom, a cyano group, an amino group, an aminosulfonyl group (-SO₂NH₂), a C₁ to C₆ alkyl group, a halo-C₁ to C₆ alkyl group or a C₁ to C₆ alkoxy group;
      R⁹ is -Y-Z;
      Y is a bond, -O-, -NR¹⁰- or -(CR¹¹R¹²)ₛ-;
      R¹⁰, R¹¹ and R¹² are each independently a hydrogen atom or a C₁ to C₆ alkyl group;
      s is an integer of 0 to 6;
      Z is a hydrogen atom, a C₁ to C₆ alkyl group, a halo-C₁ to C₆ alkyl group, a C₁ to C₆ alkoxy group, a C₁ to C₆ alkylamino group, an aromatic hydrocarbon ring group, a C₃ to C₁₀ cycloalkyl group, a 5- to 10-membered heteroaryl group or a 3- to 10-membered heterocycloalkyl group (the C₁ to C₆ alkyl group, the aromatic hydrocarbon ring group, the C₃ to C₁₀ cycloalkyl group, the 5- to 10-membered heteroaryl group and the 3- to 10-membered heterocycloalkyl group are each optionally substituted with one or more substituents selected from the group consisting of a C₁ to C₆ alkyl group, a halo-Ci to C₆ alkyl group, a C₁ to C₆ alkoxy group, a C₁ to C₆ alkylamino group, a C₁ to C₆ acyl group and a C₁ to C₆ alkoxycarbonyl group);
      R³ is ^{∗}-(CH₂)ₓ-T-(CR¹³R¹⁴)_{y}-U, and ^{∗} represents a binding position to -CH- in the formula (I); T is a bond, -NH-, -O- or -S(O)ₚ-;
      U is a hydrogen atom, a C₃ to C₁₀ cycloalkyl group or an aromatic hydrocarbon ring group (the aromatic hydrocarbon ring group is optionally substituted with one or more halogen atoms); R¹³ and R¹⁴ are each independently a hydrogen atom or a C₁ to C₆ alkyl group;
      x and y are each independently an integer of 0 to 4;
      p is an integer of 0 to 2;
      R⁴ is a hydrogen atom or a C₁ to C₆ alkyl group;
      R⁵ is the following B1) or a C₁ to C₆ alkyl group, and ^{∗} represents a binding position to -N- in the formula (I): 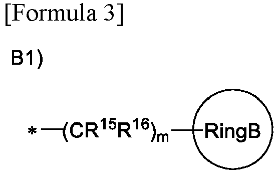 R¹⁵ and R¹⁶ are each independently a hydrogen atom, a C₁ to C₆ alkyl group or a hydroxy-Ci to C₆ alkyl group;
         R¹⁵ and R¹⁶ are optionally bonded to each other to form a ring;
         R¹⁵ and R¹⁶ are optionally bonded to RingB to form a ring;
         m is 0 or 1;
         RingB is an aromatic hydrocarbon ring group, a C₃ to C₁₀ cycloalkyl group, a 5- to 10-membered heteroaryl group or a 3- to 10-membered heterocycloalkyl group and is optionally substituted with R¹⁷, R¹⁸ and R¹⁹;
         R¹⁷ and R¹⁸ are each independently a hydrogen atom, a halogen atom, a hydroxy group, an amino group, a cyano group, a carbamoyl group (-CONH₂), a C₁ to C₆ alkyl group, a halo-Ci to C₆ alkyl group, a hydroxy-Ci to C₆ alkyl group, a C₁ to C₆ alkoxy group, a C₁ to C₆ alkylamino group, a C₁ to C₆ alkylsulfanyl group, a halo-Ci to C₆ alkylsulfanyl group, a C₁ to C₆ acyl group, a C₁ to C₆ alkoxycarbonyl group, a C₁ to C₆ alkylaminocarbonyl group or a C₁ to C₆ acylamino group;
         R¹⁹ is -(CR²⁰R²¹)ᵣ-V-(CR²²R²³)_{q}-Q;
         V is a bond, -O-, -NR²⁴- or -S(O)ₜ-;
         t is an integer of 0 to 2;
         R²⁰, R²¹, R²², R²³ and R²⁴ are each independently a hydrogen atom or a C₁ to C₆ alkyl group; q and r are each independently an integer of 0 to 6;
         Q is a hydrogen atom, an amino group, a hydroxy group, a C₁ to C₆ alkyl group, a C₁ to C₆ alkylamino group, a C₃ to C₁₀ cycloalkyl group, a 5- to 10-membered heteroaryl group or a 3-to 10-membered heterocycloalkyl group (the C₁ to C₆ alkylamino group, the C₃ to C₁₀ cycloalkyl group, the 5- to 10-membered heteroaryl group and the 3- to 10-membered heterocycloalkyl group are each optionally substituted with one or more substituents selected from the group consisting of a halogen atom, a C₁ to C₆ alkyl group, a C₁ to C₆ alkoxy group, a C₁ to C₆ alkylamino group, a C₁ to C₆ alkoxycarbonyl group, a C₁ to C₆ alkylaminocarbonyl group and a hydroxy-Ci to C₆ alkyl group);
         R¹⁷, R¹⁸ and R¹⁹ are optionally bonded to each other to form a ring;
         R⁴ and R⁵ are optionally bonded to each other to form a ring; and
         when R¹ is a nitrogen atom, m is 0, and RingB is a phenyl group optionally substituted with R¹⁷, R¹⁸ and R¹⁹, R¹ is optionally bonded to the phenyl group to form a benzimidazole ring, or a pharmacologically acceptable salt thereof.
[2] The compound according to [1] or a pharmacologically acceptable salt thereof, wherein in the formula (I), R¹ is an oxygen atom.
[3] The compound according to [2] or a pharmacologically acceptable salt thereof, wherein in the formula (I), R² is the following A1) or A2): 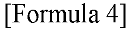 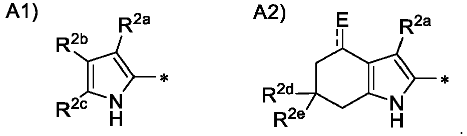
[4] The compound according to [3] or a pharmacologically acceptable salt thereof, wherein in the formula (I), R² is the following A2): 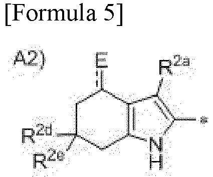 wherein R^{2a} is a C₁ to C₆ alkyl group, a C₃ to C₁₀ cycloalkyl group or a hydroxy-Ci to C₆ alkyl group.
[5] The compound according to [4] or a pharmacologically acceptable salt thereof, wherein in the formula (I), R² is the following A2b): 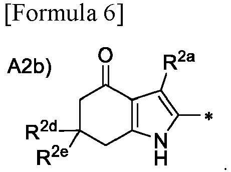
[6] The compound according to [5] or a pharmacologically acceptable salt thereof, wherein in the formula (I), R² is the following A2b): 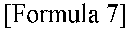 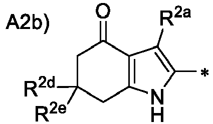
   R^{2a} is a C₁ to C₆ alkyl group or a C₃ to C₁₀ cycloalkyl group;
   R^{2d} and R^{2e} are each independently a C₁ to C₆ alkyl group; and
   R^{2d} and R^{2e} are optionally bonded to each other to form a ring.
[7] The compound according to [6] or a pharmacologically acceptable salt thereof, wherein in the formula (I),
   R³ is ^{∗}-(CH₂)ₓ-T-(CR¹³R¹⁴)_{y}-U, and ^{∗} represents a binding position to -CH- in the formula (I); T is a bond or -S(O)ₚ-;
   U is a hydrogen atom, a C₃ to C₁₀ cycloalkyl group or an aromatic hydrocarbon ring group (the aromatic hydrocarbon ring group is optionally substituted with one or more halogen atoms); R¹³ and R¹⁴ are each independently a hydrogen atom or a C₁ to C₆ alkyl group;
   x and y are each independently an integer of 0 to 2;
   p is 0;
   (except for the case where R³ is a hydrogen atom or a methyl group)
   R⁵ is the following B1) or a C₁ to C₆ alkyl group, and ^{∗} represents a binding position to -N- in the formula (I): 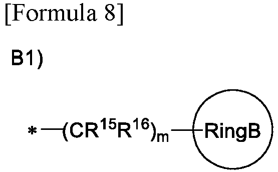 RingB is an aromatic hydrocarbon ring group, a C₃ to C₁₀ cycloalkyl group, a 5- to 10-membered heteroaryl group or a 3- to 10-membered heterocycloalkyl group and is optionally substituted with R¹⁷, R¹⁸ and R¹⁹; and
      R¹⁷ and R¹⁸ are each independently a hydrogen atom, a halogen atom, a hydroxy group, an amino group, a cyano group, a C₁ to C₆ alkyl group, a halo-Ci to C₆ alkyl group, a hydroxy-Ci to C₆ alkyl group, a C₁ to C₆ alkoxy group, a C₁ to C₆ alkylamino group, a C₁ to C₆ alkylsulfanyl group, a C₁ to C₆ alkoxycarbonyl group, a C₁ to C₆ alkylaminocarbonyl group or a C₁ to C₆ acylamino group.
[8] The compound according to [7] or a pharmacologically acceptable salt thereof, wherein in the formula (I),
   R³ is a group represented by ^{∗}-(CH₂)ₓ-T-(CR¹³R¹⁴)_{y}-U, and ^{∗} represents a binding position to - CH- in the formula (I);
   T is a bond;
   U is a hydrogen atom or a C₃ to C₁₀ cycloalkyl group;
   each of R¹³ and R¹⁴ is a hydrogen atom;
   x and y are each independently an integer of 1 or 2;
   R⁵ is the following B1) or a C₁ to C₆ alkyl group, and ^{∗} represents a binding position to -N- in the formula (I): 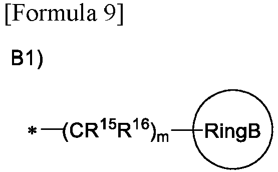 RingB is an aromatic hydrocarbon ring group, a C₃ to C₁₀ cycloalkyl group, a 5- to 10-membered heteroaryl group or a 3- to 10-membered heterocycloalkyl group and is optionally substituted with R¹⁷, R¹⁸ and R¹⁹; and
      R¹⁷ and R¹⁸ are each independently a hydrogen atom, a halogen atom, a hydroxy group, an amino group, a cyano group, a C₁ to C₆ alkyl group, a halo-Ci to C₆ alkyl group, a hydroxy-Ci to C₆ alkyl group or a C₁ to C₆ alkoxy group.
[9] The compound according to [8] or a pharmacologically acceptable salt thereof, wherein in the formula (I),
   R^{2a}, R^{2d} and R^{2e} are each independently a C₁ to C₃ alkyl group; and
   R³ is a n-butyl group or a 2-cyclopropylethan-1-yl group.
[10] The compound according to [3] or a pharmacologically acceptable salt thereof, wherein in the formula (I),
   R² is the following A1): 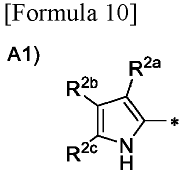 R^{2a}, R^{2b} and R^{2c} are each independently a C₁ to C₆ alkyl group, a C₁ to C₆ alkoxycarbonyl group or a C₃ to C₁₀ cycloalkyl group;
   R³ is ^{∗}-(CH₂)ₓ-T-(CR¹³R¹⁴)_{y}-U, and ^{∗} represents a binding position to -CH- in the formula (I): T is a bond;
   U is a hydrogen atom or a C₃ to C₁₀ cycloalkyl group;
   each of R¹³ and R¹⁴ is a hydrogen atom; and
   x and y are each independently an integer of 1 or 2.
[11] The compound according to [3] or a pharmacologically acceptable salt thereof, wherein in the formula (I),
   R² is the following A1a): 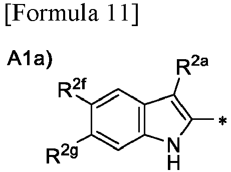 R^{2a} is a hydrogen atom or a C₁ to C₆ alkyl group;
   R^{2f} is a C₁ to C₆ alkoxy group;
   R^{2g} is a hydrogen atom or a C₁ to C₆ alkoxy group;
   R³ is ^{∗}-(CH₂)ₓ-T-(CR¹³R¹⁴)_{y}-U, and ^{∗} represents a binding position to -CH- in the formula (I): T is a bond;
   U is a hydrogen atom or a C₃ to C₁₀ cycloalkyl group;
   each of R¹³ and R¹⁴ is a hydrogen atom; and
   x and y are each independently an integer of 1 or 2.
[12] The compound according to [2] or a pharmacologically acceptable salt thereof, wherein in the formula (I),
   R⁵ is the following B1a), and ^{∗} represents a binding position to -N- in the formula (I): 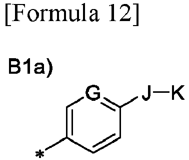 G is CH or N;
   J is a bond, -O- or -NR²⁵-;
   R²⁵ is a hydrogen atom or a C₁ to C₆ alkyl group; and
   K is a hydrogen atom, a cyano group, a C₁ to C₆ alkyl group, a halo-C₁ to C₆ alkyl group or a 3- to 10-membered heterocycloalkyl group (the 3- to 10-membered heterocycloalkyl group is optionally substituted with one or more C₁ to C₆ alkyl groups).
[13] The compound according to [12] or a pharmacologically acceptable salt thereof, wherein in the formula (I),
   R³ is ^{∗}-(CH₂)ₓ-T-(CR¹³R¹⁴)_{y}-U, and ^{∗} represents a binding position to -CH- in the formula (I); T is a bond;
   U is a hydrogen atom or a C₃ to C₁₀ cycloalkyl group;
   each of R¹³ and R¹⁴ is a hydrogen atom;
   x and y are each independently an integer of 1 or 2;
   R⁵ is the following B1b), and ^{∗} represents a binding position to -N- in the formula (I): 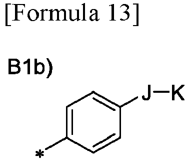 J is a bond, -O- or -NR²⁵-;
      R²⁵ is a hydrogen atom or a C₁ to C₆ alkyl group; and
      K is a hydrogen atom, a cyano group, a C₁ to C₆ alkyl group, a halo-C₁ to C₆ alkyl group or a 3- to 10-membered heterocycloalkyl group (the 3- to 10-membered heterocycloalkyl group is optionally substituted with one or more C₁ to C₆ alkyl groups).
[14] The compound according to [12] or a pharmacologically acceptable salt thereof, wherein in the formula (I),
   R⁵ is the following B1c), and ^{∗} represents a binding position to -N- in the formula (I): 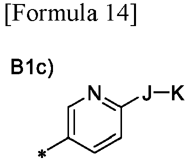 J is a bond, -O- or -NR²⁵-;
   R²⁵ is a hydrogen atom or a C₁ to C₆ alkyl group; and
   K is a hydrogen atom, a cyano group, a C₁ to C₆ alkyl group, a halo-C₁ to C₆ alkyl group or a 3- to 10-membered heterocycloalkyl group (the 3- to 10-membered heterocycloalkyl group is optionally substituted with one or more C₁ to C₆ alkyl groups).
[15] The compound according to [14] or a pharmacologically acceptable salt thereof, wherein in the formula (I), R² is the following A3): 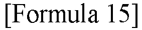 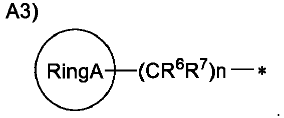
[16] The compound according to [15] or a pharmacologically acceptable salt thereof, wherein in the formula (I),
   R² is the following A3): 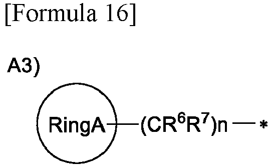 n is 0;
   R³ is ^{∗}-(CH₂)ₓ-T-(CR¹³R¹⁴)_{y}-U, and ^{∗} represents a binding position to -CH- in the formula (I); T is a bond;
   U is a hydrogen atom or a C₃ to C₁₀ cycloalkyl group;
   each of R¹³ and R¹⁴ is a hydrogen atom; and
   x and y are each independently an integer of 1 or 2.
[17] The compound according to [16] or a pharmacologically acceptable salt thereof, wherein in the formula (I),
   R² is the following A3): 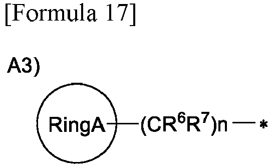 n is 0; and
   RingA is an aromatic hydrocarbon ring group optionally substituted with R⁸ and R⁹.
[18] The compound according to [17] or a pharmacologically acceptable salt thereof, wherein in the formula (I),
   R² is the following A3a): 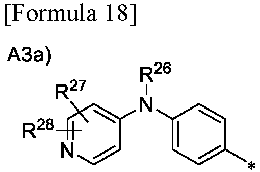 R²⁶ is a hydrogen atom or a C₁ to C₆ alkyl group; and
   R²⁷ and R²⁸ are each independently a hydrogen atom, a C₁ to C₆ alkyl group or a halo-C₁ to C₆ alkyl group.
[19] The compound according to [18] or a pharmacologically acceptable salt thereof, wherein in the formula (I),
   R⁵ is the following B1c), and ^{∗} represents a binding position to -N- in the formula (I): 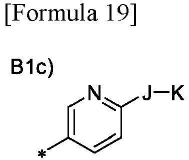 J is a bond or -O-; and
   K is a hydrogen atom or a C₁ to C₆ alkyl group.
[20] The compound according to [15] or a pharmacologically acceptable salt thereof, wherein in the formula (I),
   R² is the following A3): 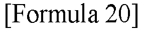 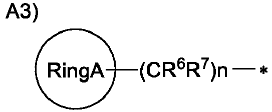 n is 1;
   R³ is ^{∗}-(CH₂)ₓ-T-(CR¹³R¹⁴)_{y}-U, and ^{∗} represents a binding position to CH- in the formula (I); T is a bond;
   U is a hydrogen atom or a C₃ to C₁₀ cycloalkyl group;
   each of R¹³ and R¹⁴ is a hydrogen atom; and
   x and y are each independently an integer of 1 or 2.
[21] The compound according to [20] or a pharmacologically acceptable salt thereof, wherein in the formula (I),
   R² is the following A3): 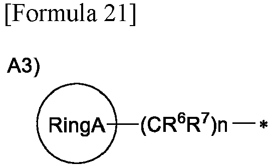 n is 1;
   RingA is an aromatic hydrocarbon ring group optionally substituted with R⁸ and R⁹;
   R⁹ is -Y-Z;
   Y is a bond, -O-, -NR¹⁰- or -(CR¹¹R¹²)ₛ-;
   R¹⁰, R¹¹ and R¹² are each independently a hydrogen atom or a C₁ to C₆ alkyl group;
   s is an integer of 0 to 6; and
   Z is a hydrogen atom, a C₁ to C₆ alkyl group, a halo-C₁ to C₆ alkyl group, a C₁ to C₆ alkoxy group or a C₁ to C₆ alkyl amino group.
[22] The compound according to [1] or a pharmacologically acceptable salt thereof, wherein the compound represented by the general formula (I) is represented by the following formula (II): 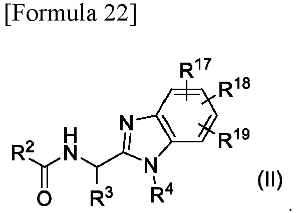
[23] The compound according to [22] or a pharmacologically acceptable salt thereof, wherein the compound represented by the general formula (I) is represented by the following formula (II): 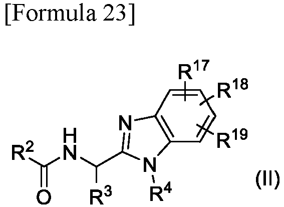 wherein
   R² is the following A2b): 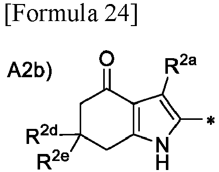 R^{2a} is a C₁ to C₆ alkyl group, a C₃ to C₁₀ cycloalkyl group or a hydroxy-C₁ to C₆ alkyl group;
   R^{2d} and R^{2e} are each independently a C₁ to C₆ alkyl group or a hydroxy-Ci to C₆ alkyl group;
   R^{2d} and R^{2e} are optionally bonded to each other to form a ring;
   R³ is ^{∗}-(CH₂)ₓ-T-(CR¹³R¹⁴)_{y}-U, and ^{∗} represents a binding position to -CH- in the formula (II); T is a bond;
   U is a hydrogen atom or a C₃ to C₁₀ cycloalkyl group;
   each of R¹³ and R¹⁴ is a hydrogen atom; and
   x and y are each independently an integer of 1 or 2.
[24] The compound according to [1] or a pharmacologically acceptable salt thereof, wherein the compound represented by the general formula (I) is represented by the following formula (III): 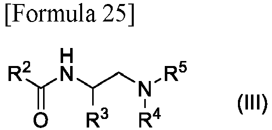 wherein
   R² is the following A2b): 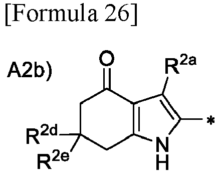 R^{2a}, R^{2d}, and R^{2e} are each independently a C₁ to C₃ alkyl group;
   R³ is ^{∗}-(CH₂)ₓ-T-(CR¹³R¹⁴)_{y}-U, and ^{∗} represents a binding position to -CH- in the formula (III) ;
   T is a bond;
   U is a hydrogen atom or a C₃ to C₁₀ cycloalkyl group;
   each of R¹³ and R¹⁴ is a hydrogen atom;
   x and y are each independently an integer of 1 or 2;
   R⁵ is the following B1), and ^{∗} represents a binding position to -N- in the formula (I): 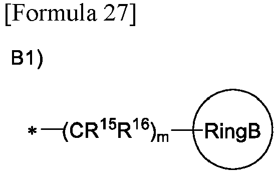 R¹⁵ and R¹⁶ are each independently a hydrogen atom or a C₁ to C₆ alkyl group;
      m is 0 or 1;
      RingB is an aromatic hydrocarbon ring group or a 5- to 10-membered heteroaryl group and is optionally substituted with R¹⁷, R¹⁸ and R¹⁹;
      R¹⁷ and R¹⁸ are each independently a hydrogen atom, a cyano group, a C₁ to C₆ alkyl group or a C₁ to C₆ alkoxy group;
      R¹⁹ is -(CR²⁰R²¹)ᵣ-V-(CR²²R²³)_{q}-Q;
      V is a bond, -O- or -NR²⁴-;
      each of R²⁰, R²¹, R²², R²³ and R²⁴ is a hydrogen atom;
      q and r are each independently an integer of 0 to 2;
      Q is a hydrogen atom, a C₁ to C₆ alkylamino group or a 3- to 10-membered heterocycloalkyl group (the 3- to 10-membered heterocycloalkyl group is optionally substituted with one or more C₁ to C₆ alkyl groups); and
      R⁴ and R⁵ are optionally bonded to each other to form a ring.
[25] A compound selected from the following: 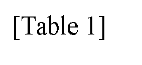 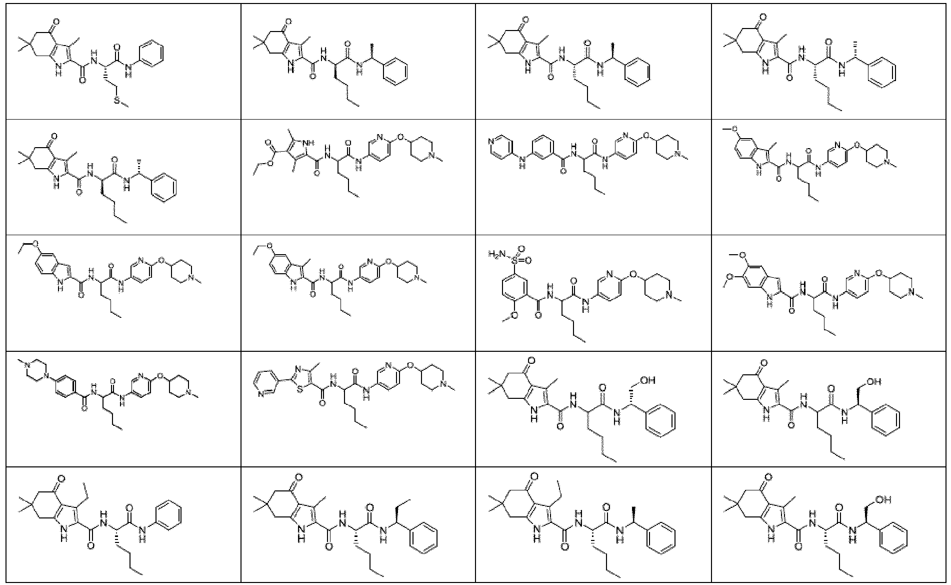 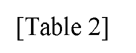 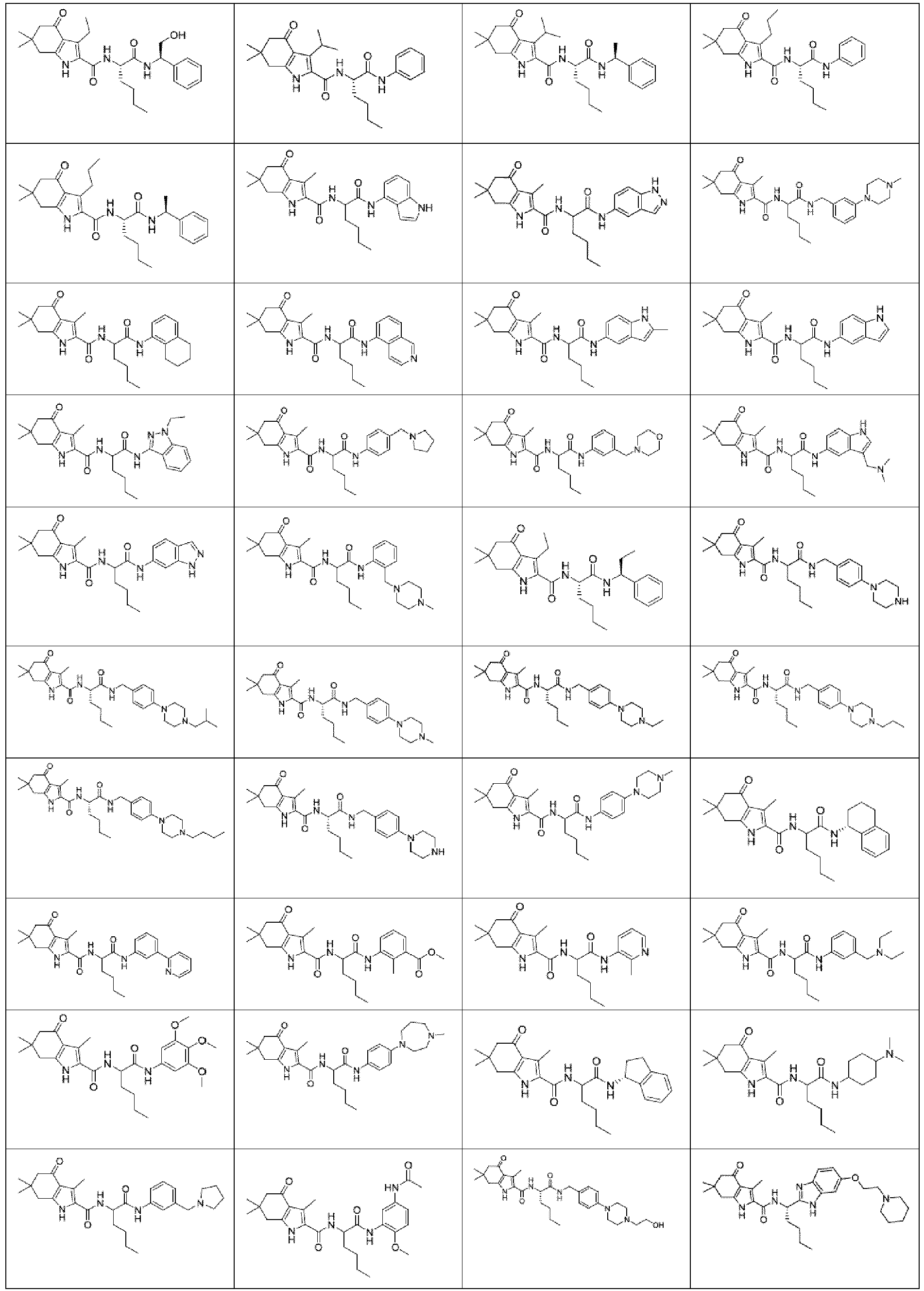 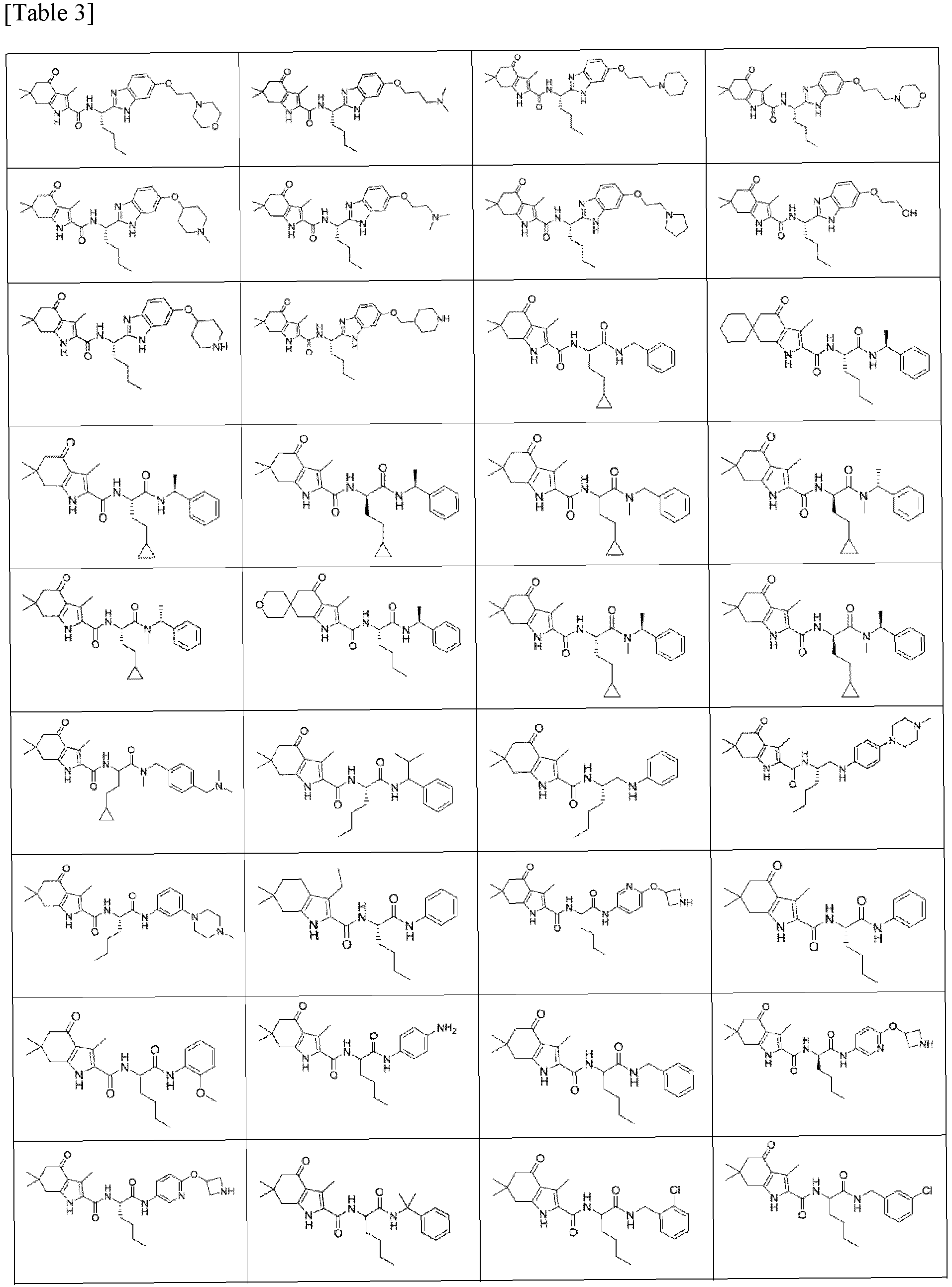 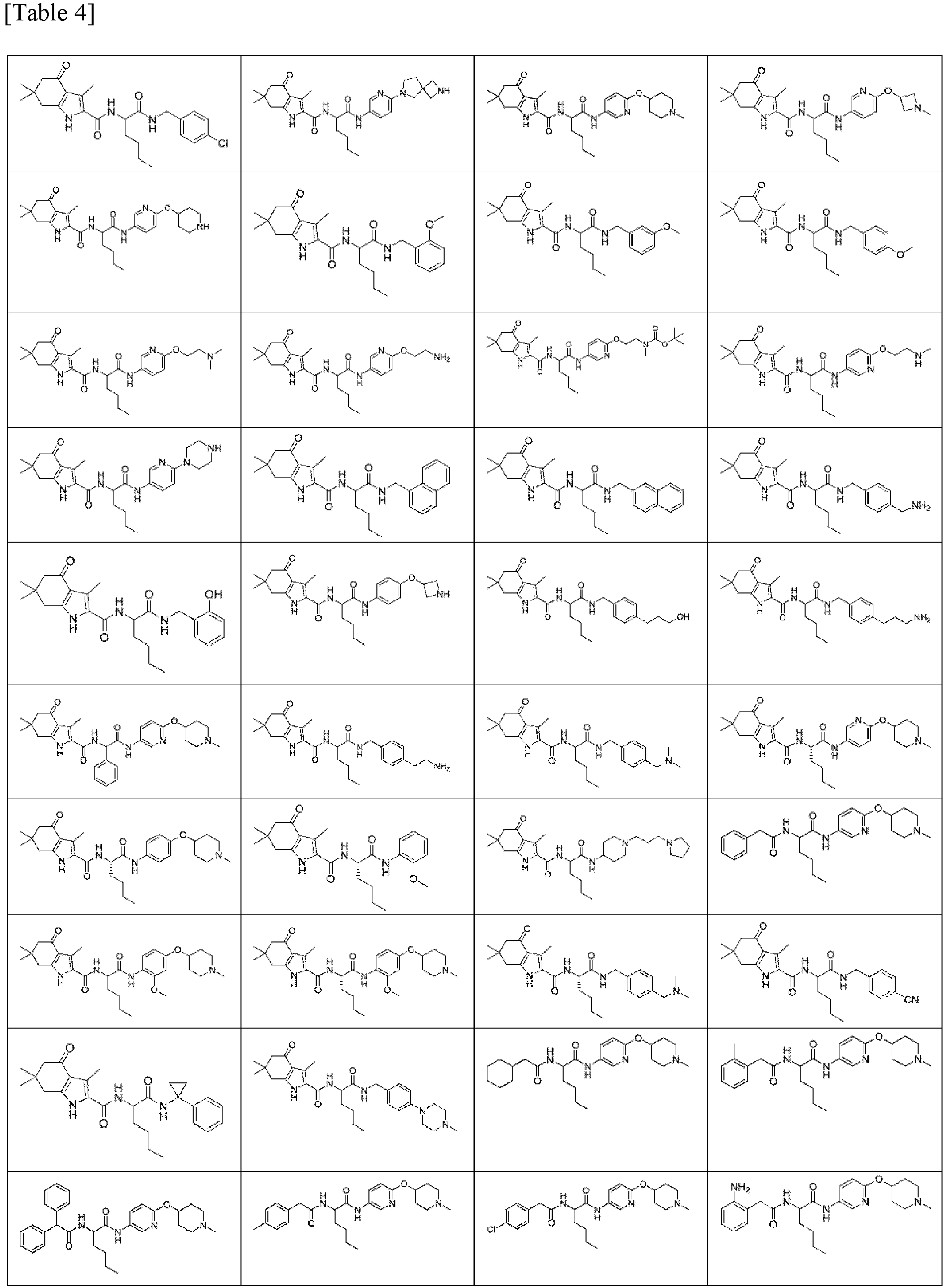 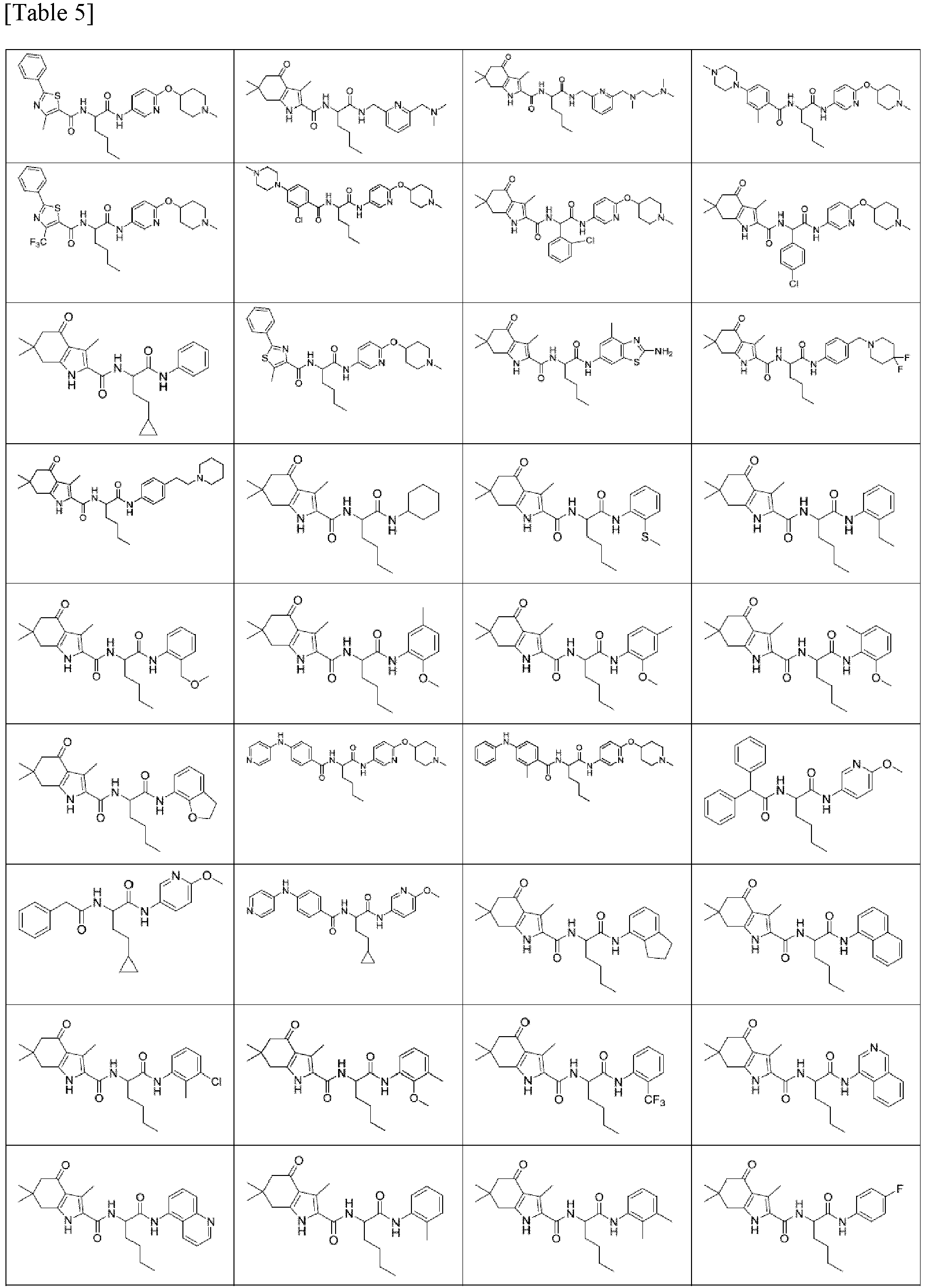 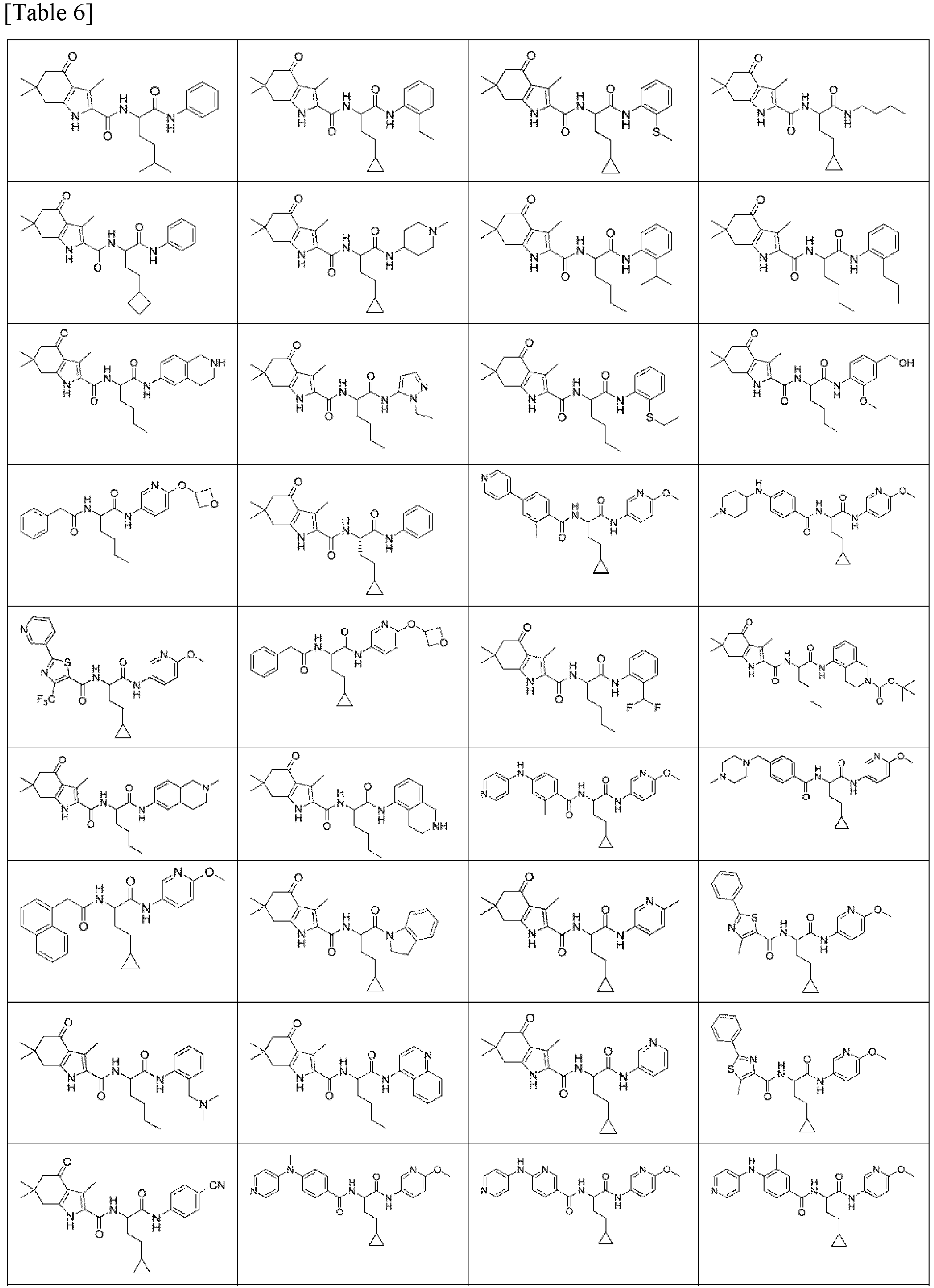 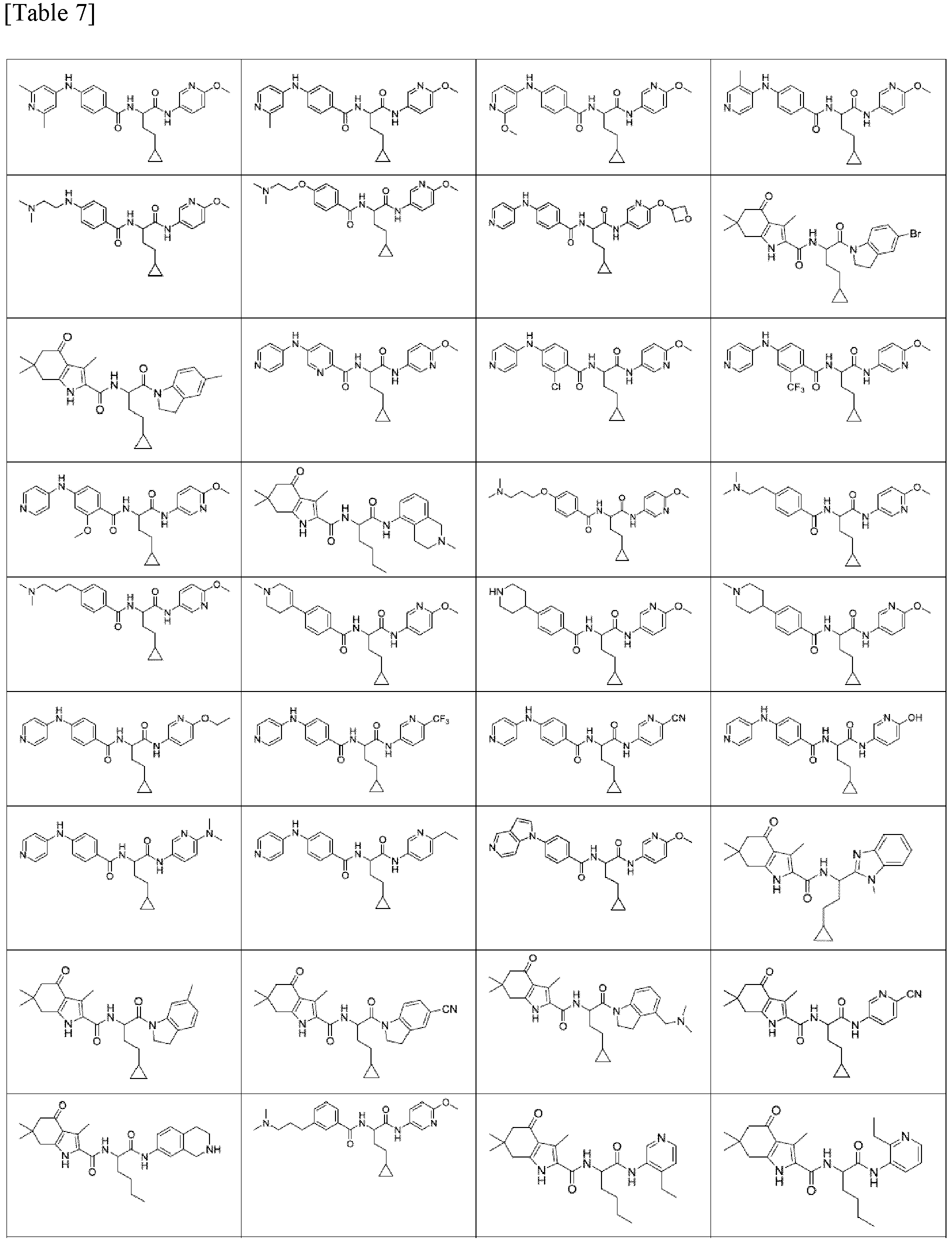 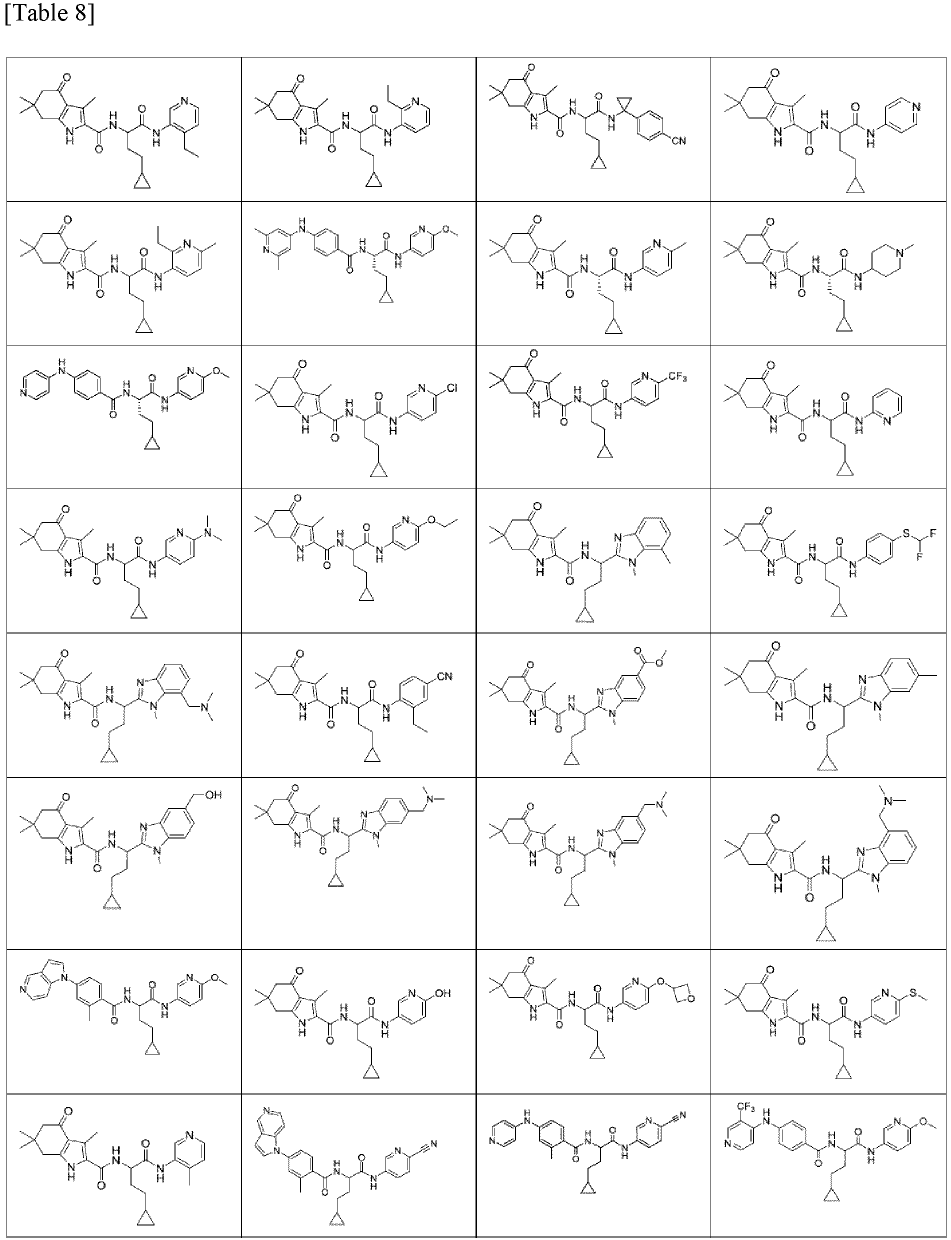 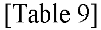 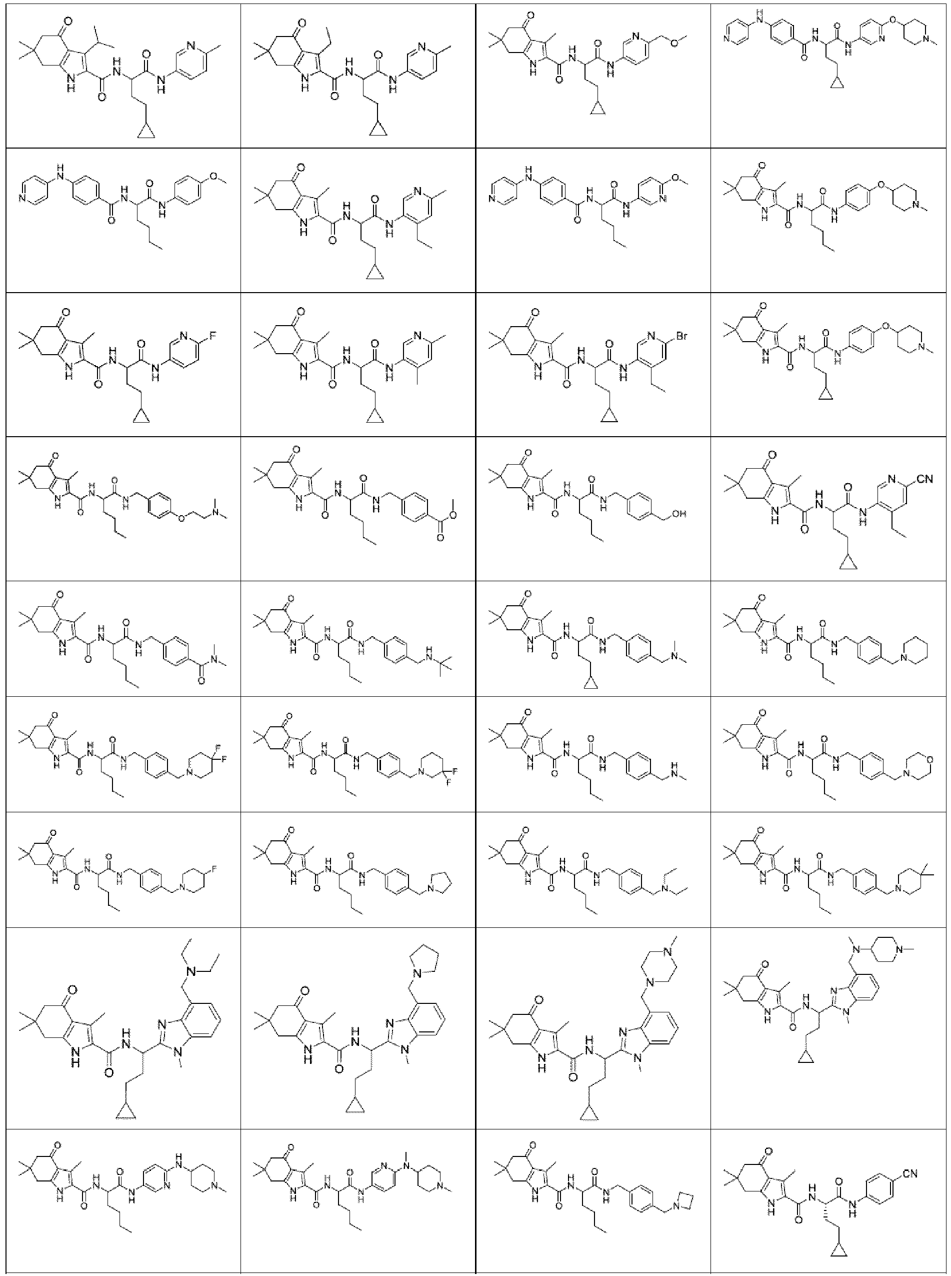 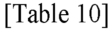 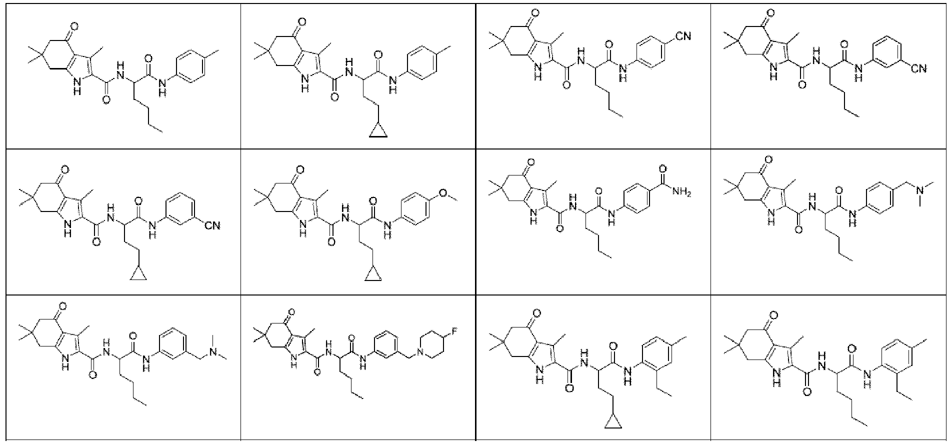 or a pharmacologically acceptable salt thereof.
[26] A G9a enzyme-inhibiting composition comprising a compound according to any one of [1] to [25] or a pharmacologically acceptable salt thereof as an active ingredient.
[27] A pharmaceutical composition comprising a compound according to any one of [1] to [25] or a pharmacologically acceptable salt thereof as an active ingredient.
[28] A method for preventing or treating at least one disease selected from the disease group consisting of proliferative disease such as cancer, β-globin abnormality, fibrosis, pain, neurodegenerative disease, Prader-Willi syndrome, malaria, viral infection, myopathy, and autism, comprising administering a compound according to any one of [1] to [25] or a pharmacologically acceptable salt thereof.
[29] Use of a compound according to any one of [1] to [25] or a pharmacologically acceptable salt thereof for producing a medicament for the prevention or treatment of at least one disease selected from the disease group consisting of proliferative disease such as cancer, β-globin abnormality, fibrosis, pain, neurodegenerative disease, Prader-Willi syndrome, malaria, viral infection, myopathy, and autism.
[30] A pharmaceutical composition comprising a compound according to any one of [1] to [25] or a pharmacologically acceptable salt thereof and a pharmaceutically acceptable carrier for use in the prevention or treatment of at least one disease selected from the disease group consisting of proliferative disease such as cancer, β-globin abnormality, fibrosis, pain, neurodegenerative disease, Prader-Willi syndrome, malaria, viral infection, myopathy, and autism.

### Advantageous Effects of Invention

A compound (I) or a pharmacologically acceptable salt thereof exhibited, for example, strong G9a enzyme inhibitory activity.

Accordingly, the compound (I) or the pharmacologically acceptable salt thereof according to the present invention is useful as a therapeutic drug for proliferative disease such as cancer, β-globin abnormality, fibrosis, pain, neurodegenerative disease, Prader-Willi syndrome, malaria, viral infection, myopathy, autism, and the like, or a prophylactic drug therefor.

Furthermore, the compound (I) or the pharmacologically acceptable salt thereof according to the present invention has high usefulness for the treatment, prevention or suppression of various pathological conditions (e.g., β-globin abnormality such as sickle cell disease, stomach cancer, hepatocellular cancer, leukemia such as acute myeloid leukemia and chronic myeloid leukemia, uterine cervical cancer, neuroblastoma, glioma, pancreatic cancer, colorectal cancer, squamous cell carcinoma of the head and neck, breast cancer, lung cancer, ovary cancer, melanoma, fibrosis such as lung fibrosis and renal fibrosis, pain, neurodegenerative disease such as Alzheimer's disease, Prader-Willi syndrome, malaria, viral infection such as foot-and-mouth disease and vesicular stomatitis, cardiomyopathy, myopathy, and autism). Moreover, the compound (I) or the pharmacologically acceptable salt thereof according to the present invention has high usefulness for the suppression of cancer metastasis and sex change.

### Description of Embodiments

Terms in the present specification will be described.

The "halogen atom" described in the present specification means a fluorine atom, a chlorine atom, a bromine atom, or an iodine atom.

The "C₁ to C₆ alkyl group" described in the present specification means a linear or branched saturated hydrocarbon group having 1 to 6 carbon atoms. Examples of the C₁ to C₆ alkyl group include a methyl group, an ethyl group, a 1-propyl group, an isopropyl group, a 1-butyl group, an isobutyl group, a sec-butyl group, a tert-butyl group, a 1-pentyl group, an isopentyl group, a neopentyl group, a 1-methylbutyl group, a 2-methylbutyl group, a 1,2-dimethylpropyl group, a 1-hexyl group, and an isohexyl group. The "C₁ to C₃ alkyl group" means a linear or branched saturated hydrocarbon group having 1 to 3 carbon atoms.

The "C₂ to C₆ alkenyl group" described in the present specification means a linear or branched unsaturated hydrocarbon group having 2 to 6 carbon atoms and having at least one double bond. Examples of the C₂ to C₆ alkenyl group include a vinyl group, a 2-propenyl group, a 1-propenyl group, a 1-buten-1-yl group, a 1-buten-2-yl group, a 1-buten-3-yl group, a 2-buten-1-yl group, a 2-buten-2-yl group, a 1-penten-1-yl group, a 1-penten-2-yl group, a 1-penten-3-yl group, a 2-penten-1-yl group, a 2-penten-2-yl group, a 2-penten-3-yl group, a 1-hexen-1-yl group, a 1-hexen-2-yl group, a 1-hexen-3-yl group, and a 2-methyl-1-propen-1-yl group.

The "C₁ to C₆ acyl group" described in the present specification means a linear or branched aliphatic carboxylic acid-derived acyl group having 1 to 6 carbon atoms. Examples thereof include a formyl group, an acetyl group, a propanoyl group, a 1-butanoyl group, a 1-pentanoyl group, and a 1-hexanoyl group.

The "halo-C₁ to C₆ alkyl group" described in the present specification means a C₁ to C₆ alkyl group in which at least one hydrogen atom is replaced with halogen atom(s) (of the same type or different types). Examples of the halo-Ci to C₆ alkyl group include a fluoromethyl group, a difluoromethyl group, a trifluoromethyl group, a 2-fluoroethyl group, a 2-chloroethyl group, a 2,2-difluoroethyl group, a 1,1-difluoroethyl group, a 1,2-difluoroethyl group, a 1-chloro-2-fluoroethyl group, a 2,2,2-trifluoroethyl group, a 1,1,2,2,2-pentafluoroethyl group, a 2,2,2-trichloroethyl group, a 3-fluoropropyl group, a 2-fluoropropyl group, a 1-fluoropropyl group, a 3,3-difluoropropyl group, a 2,2-difluoropropyl group, a 1,1-difluoropropyl group, a 4-fluorobutyl group, a 5-fluoropentyl group, and a 6-fluorohexyl group.

The "hydroxy-Ci to C₆ alkyl group" described in the present specification means the C₁ to C₆ alkyl group in which at least one hydrogen atom is replaced with a hydroxy group. Examples of the hydroxy-Ci to C₆ alkyl group include a hydroxymethyl group, a 1-hydroxyethyl group, a 1-hydroxy-1,1-dimethylmethyl group, a 2-hydroxyethyl group, a 2-hydroxy-2-methylpropyl group, and a 3-hydroxypropyl group. The "hydroxy-Ci to C₃ alkyl group" means a hydroxyalkyl group having 1 to 3 carbon atoms.

The "C₁ to C₆ alkoxy group" described in the present specification means a linear or branched alkoxy group having 1 to 6 carbon atoms. Examples of the C₁ to C₆ alkoxy group include a methoxy group, an ethoxy group, a 1-propoxy group, an isopropoxy group, an isobutoxy group, a 1-butoxy group, a sec-butoxy group, a tert-butoxy group, a 1-pentyloxy group, and a 1-hexyloxy group.

The "halo-Ci to C₆ alkoxy group" described in the present specification means a C₁ to C₆ alkoxy group in which at least one hydrogen atom is replaced with halogen atom(s) (of the same type or different types). Examples of the halo-Ci to C₆ alkoxy group include a monofluoromethoxy group, a difluoromethoxy group, a trifluoromethoxy group, a 2-chloroethoxy group, a 2-fluoroethoxy group, a 2,2-difluoroethoxy group, a 1,1-difluoroethoxy group, a 1,2-difluoroethoxy group, a 1-chloro-2-fluoroethoxy group, a 2,2,2-trifluoroethoxy group, a 1,1,2,2,2-pentafluoroethoxy group, a 2,2,2-trichloroethoxy group, a 3-fluoropropoxy group, a 2-fluoropropoxy group, a 1-fluoropropoxy group, a 3,3-difluoropropoxy group, a 2,2-difluoropropoxy group, a 1,1-difluoropropoxy group, a 4-fluorobutoxy group, a 5-fluoropentyloxy group, and a 6-fluorohexyloxy group.

The "C₁ to C₆ alkoxycarbonyl group" described in the present specification means a carbonyl group bonded to a linear or branched alkoxy group having 1 to 6 carbon atoms. Examples of the C₁ to C₆ alkoxycarbonyl group include a methoxycarbonyl group, an ethoxycarbonyl group, a 1-propoxycarbonyl group, an isopropoxycarbonyl group, an isobutoxycarbonyl group, a 1-butoxycarbonyl group, a sec-butoxycarbonyl group, a tert-butoxycarbonyl group, a 1-pentyloxycarbonyl group, and a 1-hexyloxycarbonyl group.

The "C₁ to C₆ alkylamino group" described in the present specification means an amino group in which one or two hydrogen atoms of the amino group are replaced with linear or branched alkyl group(s) having a total of 1 to 6 carbon atoms. Examples of the C₁ to C₆ alkylamino group include a methylamino group, an ethylamino group, a 1-propylamino group, an isopropylamino group, a 1-butylamino group, an isobutylamino group, a sec-butylamino group, a tert-butylamino group, a 1-pentylamino group, an isopentylamino group, a neopentylamino group, a 1-methylbutylamino group, a 2-methylbutylamino group, a 1,2-dimethylpropylamino group, a 1-hexylamino group, an isohexylamino group, a dimethylamino group, a diethylamino group, a N-ethyl-N-methylamino group, and a N-ethyl-N-propylamino group.

The "C₁ to C₆ alkylaminocarbonyl group" described in the present specification means a carbonyl group bonded to a linear or branched alkylamino group having a total of 1 to 6 carbon atoms. Examples of the C₁ to C₆ alkylaminocarbonyl group include a methylaminocarbonyl group, an ethylaminocarbonyl group, a 1-propylaminocarbonyl group, an isopropylaminocarbonyl group, a 1-butylaminocarbonyl group, an isobutylaminocarbonyl group, a sec-butylaminocarbonyl group, a tert-butylaminocarbonyl group, a 1-pentylaminocarbonyl group, an isopentylaminocarbonyl group, a neopentylaminocarbonyl group, a 1-methylbutylaminocarbonyl group, a 2-methylbutylaminocarbonyl group, a 1,2-dimethylpropylaminocarbonyl group, a 1-hexylaminocarbonyl group, an isohexylaminocarbonyl group, a dimethylaminocarbonyl group, a diethylaminocarbonyl group, a N-ethyl-N-methylaminocarbonyl group, and a N-ethyl-N-propylaminocarbonyl group.

The "C₁ to C₆ acylamino group" described in the present specification means an amino group in which one or two hydrogen atoms of the amino group are replaced with linear or branched acyl group(s) having 1 to 6 carbon atoms. Examples of the C₁ to C₆ acylamino group include a formylamino group, an acetylamino group, a 1-propanoylamino group, a 1-butanoylamino group, a 1-pentanoylamino group, and a hexanoylamino group.

The "C₁ to C₆ alkylsulfanyl group" described in the present specification means a group in which a linear or branched alkyl group having 1 to 6 carbon atoms is bonded to a sulfur atom. Examples of the C₁ to C₆ alkylsulfanyl group include a methylsulfanyl group, an ethylsulfanyl group, a 1-propylsulfanyl group, an isopropylsulfanyl group, a 1-butylsulfanyl group, an isobutylsulfanyl group, a sec-butylsulfanyl group, and a tert-butylsulfanyl group.

The "halo-Ci to C₆ alkylsulfanyl group" described in the present specification means a C₁ to C₆ alkylsulfanyl group in which at least one hydrogen atom is replaced with halogen atom(s) (of the same type or different types). Examples of the halo-Ci to C₆ alkylsulfanyl group include a fluoromethylsulfanyl group, a difluoromethylsulfanyl group, a trifluoromethylsulfanyl group, a 2-fluoroethylsulfanyl group, a 2-chloroethylsulfanyl group, a 2,2-difluoroethylsulfanyl group, a 1,1-difluoroethylsulfanyl group, a 1,2-difluoroethylsulfanyl group, a 1-chloro-2-fluoroethylsulfanyl group, a 2,2,2-trifluoroethylsulfanyl group, a 1,1,2,2,2-pentafluoroethylsulfanyl group, a 2,2,2-trichloroethylsulfanyl group, a 3-fluoropropylsulfanyl group, a 2-fluoropropylsulfanyl group, a 1-fluoropropylsulfanyl group, a 3,3-difluoropropylsulfanyl group, a 2,2-difluoropropylsulfanyl group, a 1,1-difluoropropylsulfanyl group, a 4-fluorobutylsulfanyl group, a 5-fluoropentylsulfanyl group, and a 6-fluorohexylsulfanyl group.

Examples of the "aromatic hydrocarbon ring group" described in the present specification include a phenyl group, an indenyl group, a 1-naphthyl group, a 2-naphthyl group, an azulenyl group, a heptalenyl group, a biphenyl group, an indacenyl group, an acenaphthyl group, a fluorenyl group, a phenalenyl group, a phenanthrenyl group, an anthracenyl group, and a benzocyclooctenyl group.

The "5- to 10-membered heteroaryl group" described in the present specification means a 5- to 10-membered monocyclic aromatic heterocyclic group or a condensed-ring aromatic heterocyclic group having 1 to 4 intra-ring heteroatoms each independently selected from the group consisting of a nitrogen atom, an oxygen atom and a sulfur atom. The nitrogen and sulfur atoms may be oxidized, if desired (i.e., N → O, SO or SO₂). Examples of the 5- to 10-membered heteroaryl group include, but are not limited to, a benzimidazolyl group, a benzofuranyl group, a benzothiofuranyl group, a benzothiophenyl group, a benzoxazolyl group, a benzoxazolinyl group, a benzothiazolyl group, a benzotriazolyl group, a benzisoxazolyl group, a benzisothiazolyl group, a benzimidazolinyl group, a furanyl group, an imidazolidinyl group, an imidazolyl group, a 1H-indazolyl group, an imidazolopyridinyl group, an indolenyl group, an indolizinyl group, a 3H-indolyl group, an isobenzofuranyl group, an isoindazolyl group, an isoindolyl group, an isoquinolinyl group, an isothiazolyl group, an isothiazolopyridinyl group, an isoxazolyl group, an isoxazolopyridinyl group, a naphthyridinyl group, a 1,2,3-oxadiazolyl group, a 1,2,4-oxadiazolyl group, a 1,2,5-oxadiazolyl group, a 1,3,4-oxadiazolyl group, an oxazolidinyl group, an oxazolyl group, an oxazolopyridinyl group, an oxazolidinylperimidinyl group, an oxindolyl group, a pyrimidinyl group, a pyrazinyl group, a pyrazolidinyl group, a pyrazolinyl group, a pyrazolopyridinyl group, a pyrazolyl group, a pyridazinyl group, a pyridoxazolyl group, a pyridoimidazolyl group, a pyridothiazolyl group, a pyridinyl group, a pyrrolopyridinyl group, a quinazolinyl group, a quinolinyl group, a 4H-quinolizinyl group, a quinoxalinyl group, a quinuclidinyl group, a tetrazolyl group, a 6H-1,2,5-thiadiazinyl group, a 1,2,3-thiadiazolyl group, a 1,2,4-thiadiazolyl group, a 1,2,5-thiadiazolyl group, a 1,3,4-thiadiazolyl group, a thianthrenyl group, a thiazolyl group, a thienyl group, a thiazolopyridinyl group, a thienothiazolyl group, a thienooxazolyl group, a thienoimidazolyl group, a thiophenyl group, a triazinyl group, a 1,2,3-triazolyl group, a 1,2,4-triazolyl group, a 1,2,5-triazolyl group and a 1,3,4-triazolyl group. A condensed ring and a spiro ring compound containing the heteroring described above is also encompassed thereby.

The "C₃ to C₁₀ cycloalkyl group" described in the present specification means a monocyclic or bicyclic saturated alicyclic hydrocarbon group having 3 to 10 carbon atoms and can be in a cross-linked form or a spiro form. Examples of the C₃ to C₁₀ cycloalkyl group include a cyclopropyl group, a cyclobutyl group, a cyclopentyl group, a cyclohexyl group, a cycloheptyl group, a cyclooctyl group, a spiroheptyl group, a spirooctyl group, and an octahydropentalenyl group. The C₃ to C₁₀ cycloalkyl group may be condensed with an additional aromatic hydrocarbon ring group or 5- to 10-membered heteroaryl group. Examples of the C₃ to C₁₀ cycloalkyl group condensed with an aromatic hydrocarbon ring group or a 5- to 10-membered heteroaryl group include a dihydroindenyl group and a tetrahydronaphthyl group.

The "C₃ to C₄ cycloalkyl group" means a cycloalkyl group having 3 to 4 carbon atoms.

The "3- to 10-membered heterocycloalkyl group" described in the present specification means a heterocycloalkyl group having a monocyclic, bicyclic, or tricyclic 3- to 10-membered ring and containing 1 to 4 intra-ring heteroatoms each independently selected from the group consisting of a nitrogen atom, an oxygen atom and a sulfur atom. The nitrogen and sulfur heteroatoms may be oxidized, if desired (i.e., N → O, SO or SO₂). The nitrogen atom may or may not be substituted. The 3- to 10-membered heterocycloalkyl group may have 1 to 3 carbonyl groups and may have one double bond in the ring. The 3- to 10-membered heterocycloalkyl group may be in a cross-linked form or a spiro form. The 3- to 10-membered heterocycloalkyl group may be condensed with an additional aromatic hydrocarbon ring group or 5- to 10-membered heteroaryl group. Examples of the 3- to 10-membered heterocycloalkyl group include an aziridinyl group, an azetidinyl group, a pyrrolidinyl group, a piperidinyl group, an azepanyl group, an azocanyl group, a dihydropyrrolyl group, a tetrahydropyridinyl group, a piperazinyl group, a morpholinyl group, a thiomorpholinyl group, a 1-oxidothiomorpholinyl group, a 1,1-dioxidothiomorpholinyl group, an oxazepinyl group, a thiazepanyl group, a 1-oxido-1,4-thiazepanyl group, a 1,1-dioxido-1,4-thiazepanyl group, a 1,4-diazepanyl group, a 1,4-oxazocanyl group, a 1,5-oxazocanyl group, an oxetanyl group, a tetrahydrofuranyl group, a tetrahydropyranyl group, an octahydrocyclopenta[c]pyrrolyl group, a 3-azabicyclo[3.2.0]heptanyl group, a 3-azabicyclo[3.1.0]hexanyl group, a 5-azabicyclo[2.1.1]hexanyl group, a 2-azabicyclo[2.1.1]hexanyl group, a 2-azabicyclo[4.1.0]heptanyl group, a 3-azabicyclo[4.1.0]heptanyl group, a 2-azabicyclo[4.2.0]octanyl group, a 3-azabicyclo[4.2.0]octanyl group, a 3-azabicyclo[3.1.1]heptanyl group, a 2-azabicyclo[2,2,1]heptanyl group, a 6-azabicyclo[3.1.1]heptanyl group, a 8-azabicyclo[3.2.1]octanyl group, a 3-azabicyclo[3.2.1]octanyl group, a 6-azabicyclo[3.2.1]octanyl group, a 4-azaspiro[2.4]heptanyl group, a 5-azaspiro[2.4]heptanyl group, a 1-oxo-5-azaspiro[2.4]heptanyl group, a 5-azaspiro[3.4]octanyl group, a 6-azaspiro[3.4]octanyl group, a 2-oxo-6-azaspiro[3.4]octanyl group, a 1-oxo-6-azaspiro[3.4]octanyl group, a 4-azaspiro[2.5]octanyl group, a 5-azaspiro[2.5]octanyl group, a 6-azaspiro[2.5]octanyl group, a 1-oxa-5-azaspiro[2.5]octanyl group, a 4-oxa-7-azaspiro[2.5]octanyl group, a 1-oxa-6-azaspiro[2.5]octanyl group, and a 2,6-diazaspiro[3.4]octanyl group.

The phrase "bonded to each other to form a ring" described in the present specification means that, from each of two substituents to form a ring, one arbitrary hydrogen atom is further removed and the resulting sites are bonded to each other. For example, when a methylene group has two substituents and the two substituents to form a ring are a methyl group and a 1-hydroxyethyl group, examples thereof include

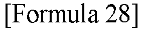

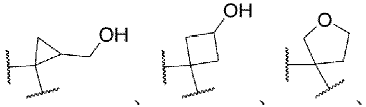

Both the terms "3,6,6-trimethyl-4-oxo-5,7-dihydro-1H-indole-2-carboxylic acid" and "3,6,6-trimethyl-4-oxo-4,5,6,7-tetrahydro-1H-indole-2-carboxylic acid" described in the present specification represent the following same compound A, and related compounds were similarly designated.

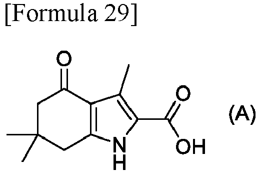

Hereinafter, the present embodiment will be described in more detail.

Hereinafter, description about the definition of a functional group in a general formula may be omitted by citing the already described definition. The cited definition refers to a definition described in the description of embodiments given below.

The same reference sign will be commonly used to designate the same or similar definitions as to a functional group in a general formula among general formulas including the reference sign, unless otherwise specified.

The present embodiment relates to a compound represented by the following general formula (I) or a pharmacologically acceptable salt thereof:
a compound represented by the general formula (I): 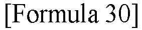 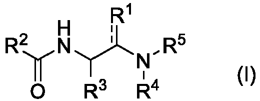 wherein
R¹ is an oxygen atom, a nitrogen atom or a hydrogen atom;
when R¹ is an oxygen atom or a nitrogen atom, the bond between R¹ and the carbon atom is a double bond;
when R¹ is a hydrogen atom, the bond between R¹ and the carbon atom is a single bond;
R² is the following A1), A2) or A3), and ^{∗} represents a binding position to -CO- in the formula (I): 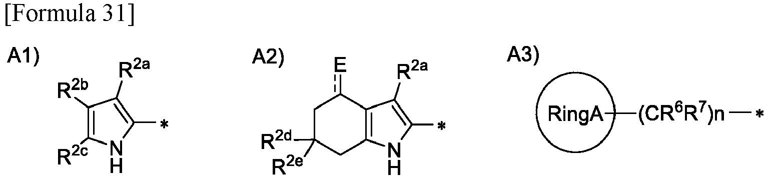 E is an oxygen atom or a hydrogen atom;
   when E is an oxygen atom, the bond between E and the carbon atom is a double bond;
   when E is a hydrogen atom, the bond between E and the carbon atom is a single bond;
   R^{2a}, R^{2b} and R^{2c} are each independently a hydrogen atom, a C₁ to C₆ alkyl group, a C₂ to C₆ alkenyl group, a halo-Ci to C₆ alkyl group, a C₁ to C₆ alkoxy group, a C₁ to C₆ alkylamino group, a C₁ to C₆ acyl group, a C₁ to C₆ alkoxycarbonyl group, a C₃ to C₁₀ cycloalkyl group or a hydroxy-Ci to C₆ alkyl group (the C₁ to C₆ alkyl group, the C₂ to C₆ alkenyl group, the halo-C₁ to C₆ alkyl group, the C₁ to C₆ alkoxy group, the C₁ to C₆ alkylamino group, the C₁ to C₆ acyl group, the C₁ to C₆ alkoxycarbonyl group, the C₃ to C₁₀ cycloalkyl group and the hydroxy-Ci to C₆ alkyl group are each optionally substituted with one or more substituents selected from the group consisting of a C₁ to C₆ alkyl group, a C₁ to C₆ alkoxy group, a C₁ to C₆ alkylamino group and a hydroxy-Ci to C₆ alkyl group, and these substituents are optionally bonded to each other to form a ring);
   R^{2b} and R^{2c} are optionally bonded to each other to form a ring;
   R^{2d} and R^{2e} are each independently a C₁ to C₆ alkyl group or a hydroxy-C₁ to C₆ alkyl group;
   R^{2d} and R^{2e} are optionally bonded to each other to form a ring;
   R⁶ and R⁷ are each independently a hydrogen atom, a C₁ to C₆ alkyl group, an aromatic hydrocarbon ring group, a C₃ to C₁₀ cycloalkyl group, a 5- to 10-membered heteroaryl group or a 3- to 10-membered heterocycloalkyl group;
   R⁶ and R⁷ are optionally bonded to each other to form a ring;
   n is 0 or 1;
   RingA is an aromatic hydrocarbon ring group, a C₃ to C₁₀ cycloalkyl group, a 5- to 10-membered heteroaryl group or a 3- to 10-membered heterocycloalkyl group and is optionally substituted with R⁸ and R⁹;
   R⁸ is a hydrogen atom, a halogen atom, a cyano group, an amino group, an aminosulfonyl group (-SO₂NH₂), a C₁ to C₆ alkyl group, a halo-C₁ to C₆ alkyl group or a C₁ to C₆ alkoxy group;
   R⁹ is -Y-Z;
   Y is a bond, -O-, -NR¹⁰- or -(CR¹¹R¹²)ₛ-;
   R¹⁰, R¹¹ and R¹² are each independently a hydrogen atom or a C₁ to C₆ alkyl group;
   s is an integer of 0 to 6;
   Z is a hydrogen atom, a C₁ to C₆ alkyl group, a halo-C₁ to C₆ alkyl group, a C₁ to C₆ alkoxy group, a C₁ to C₆ alkylamino group, an aromatic hydrocarbon ring group, a C₃ to C₁₀ cycloalkyl group, a 5- to 10-membered heteroaryl group or a 3- to 10-membered heterocycloalkyl group (the C₁ to C₆ alkyl group, the aromatic hydrocarbon ring group, the C₃ to C₁₀ cycloalkyl group, the 5- to 10-membered heteroaryl group and the 3- to 10-membered heterocycloalkyl group are each optionally substituted with one or more substituents selected from the group consisting of a C₁ to C₆ alkyl group, a halo-Ci to C₆ alkyl group, a C₁ to C₆ alkoxy group, a C₁ to C₆ alkylamino group, a C₁ to C₆ acyl group and a C₁ to C₆ alkoxycarbonyl group);
   R³ is ^{∗}-(CH₂)ₓ-T-(CR¹³R¹⁴)_{y}-U, and ^{∗} represents a binding position to -CH- in the formula (I); T is a bond, -NH-, -O- or -S(O)ₚ-;
   U is a hydrogen atom, a C₃ to C₁₀ cycloalkyl group or an aromatic hydrocarbon ring group (the aromatic hydrocarbon ring group is optionally substituted with one or more halogen atoms); R¹³ and R¹⁴ are each independently a hydrogen atom or a C₁ to C₆ alkyl group;
   x and y are each independently an integer of 0 to 4;
   p is an integer of 0 to 2;
   R⁴ is a hydrogen atom or a C₁ to C₆ alkyl group;
   R⁵ is the following B1) or a C₁ to C₆ alkyl group, and ^{∗} represents a binding position to -N- in the formula (I): 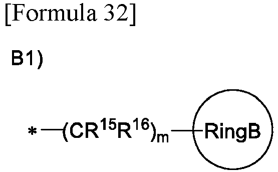 R¹⁵ and R¹⁶ are each independently a hydrogen atom, a C₁ to C₆ alkyl group or a hydroxy-Ci to C₆ alkyl group;
      R¹⁵ and R¹⁶ are optionally bonded to each other to form a ring;
      R¹⁵ and R¹⁶ are optionally bonded to RingB to form a ring;
      m is 0 or 1;
      RingB is an aromatic hydrocarbon ring group, a C₃ to C₁₀ cycloalkyl group, a 5- to 10-membered heteroaryl group or a 3- to 10-membered heterocycloalkyl group and is optionally substituted with R¹⁷, R¹⁸ and R¹⁹;
      R¹⁷ and R¹⁸ are each independently a hydrogen atom, a halogen atom, a hydroxy group, an amino group, a cyano group, a carbamoyl group (-CONH₂), a C₁ to C₆ alkyl group, a halo-C₁ to C₆ alkyl group, a hydroxy-Ci to C₆ alkyl group, a C₁ to C₆ alkoxy group, a C₁ to C₆ alkylamino group, a C₁ to C₆ alkylsulfanyl group, a halo-Ci to C₆ alkylsulfanyl group, a C₁ to C₆ acyl group, a C₁ to C₆ alkoxycarbonyl group, a C₁ to C₆ alkylaminocarbonyl group or a C₁ to C₆ acylamino group;
      R¹⁹ is -(CR²⁰R²¹)ᵣ-V-(CR²²R²³)_{q}-Q;
      V is a bond, -O-, -NR²⁴- or -S(O)ₜ-;
      t is an integer of 0 to 2;
      R²⁰, R²¹, R²², R²³ and R²⁴ are each independently a hydrogen atom or a C₁ to C₆ alkyl group;
      q and r are each independently an integer of 0 to 6;
      Q is a hydrogen atom, an amino group, a hydroxy group, a C₁ to C₆ alkyl group, a C₁ to C₆ alkylamino group, a C₃ to C₁₀ cycloalkyl group, a 5- to 10-membered heteroaryl group or a 3-to 10-membered heterocycloalkyl group (the C₁ to C₆ alkylamino group, the C₃ to C₁₀ cycloalkyl group, the 5- to 10-membered heteroaryl group and the 3- to 10-membered heterocycloalkyl group are each optionally substituted with one or more substituents selected from the group consisting of a halogen atom, a C₁ to C₆ alkyl group, a C₁ to C₆ alkoxy group, a C₁ to C₆ alkylamino group, a C₁ to C₆ alkoxycarbonyl group, a C₁ to C₆ alkylaminocarbonyl group and a hydroxy-C₁ to C₆ alkyl group);
      R¹⁷, R¹⁸ and R¹⁹ are optionally bonded to each other to form a ring;
      R⁴ and R⁵ are optionally bonded to each other to form a ring; and
      when R¹ is a nitrogen atom, m is 0, and RingB is a phenyl group optionally substituted with R¹⁷, R¹⁸ and R¹⁹, R¹ is optionally bonded to the phenyl group to form a benzimidazole ring,
      or a pharmacologically acceptable salt thereof.

Preferred examples of the compound of the present embodiment include the following compounds:

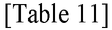

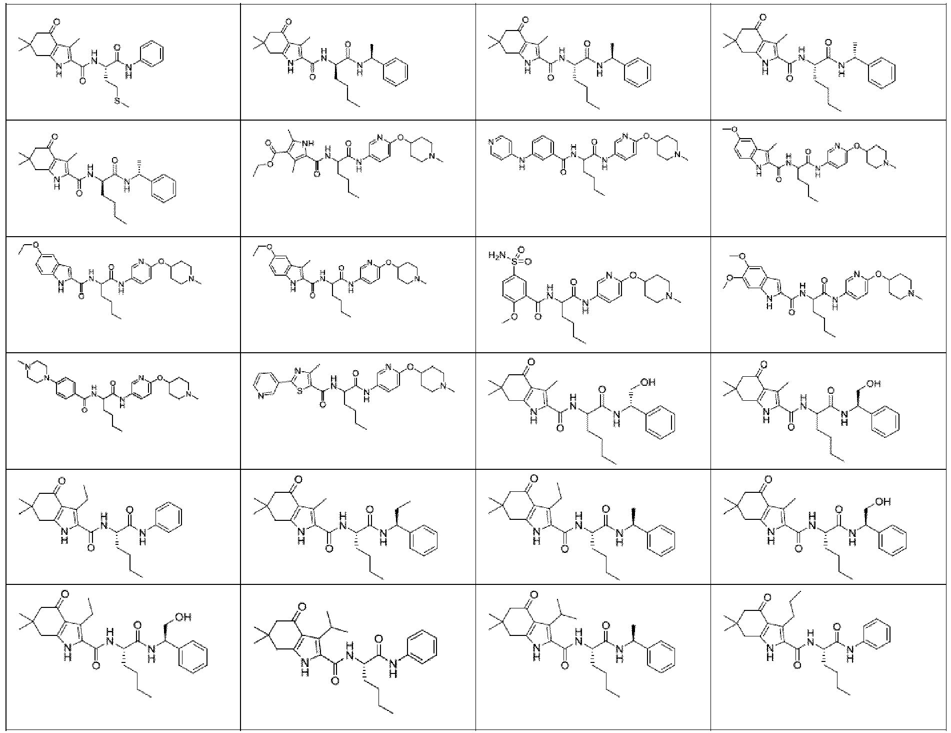

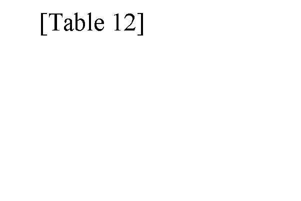

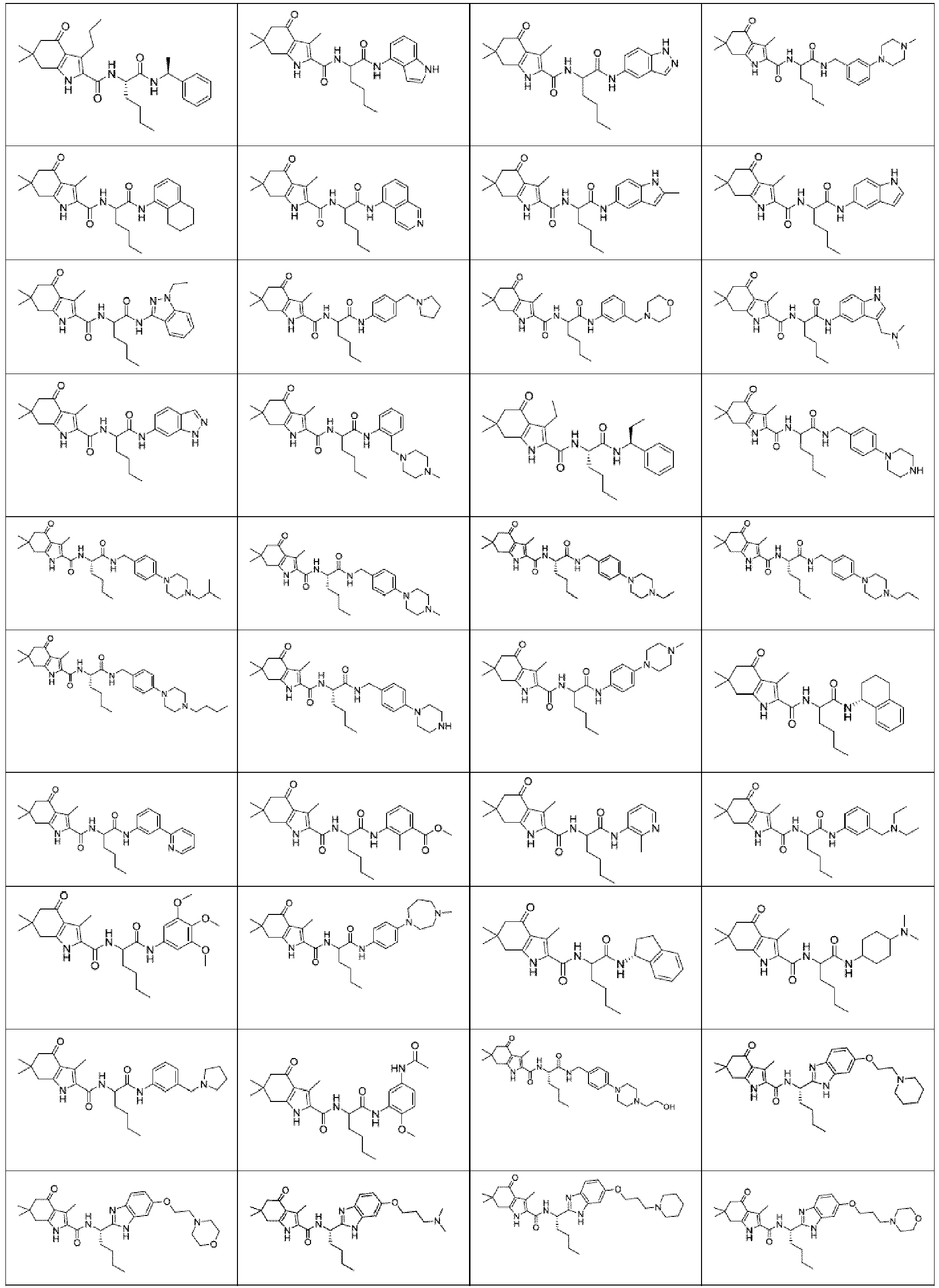

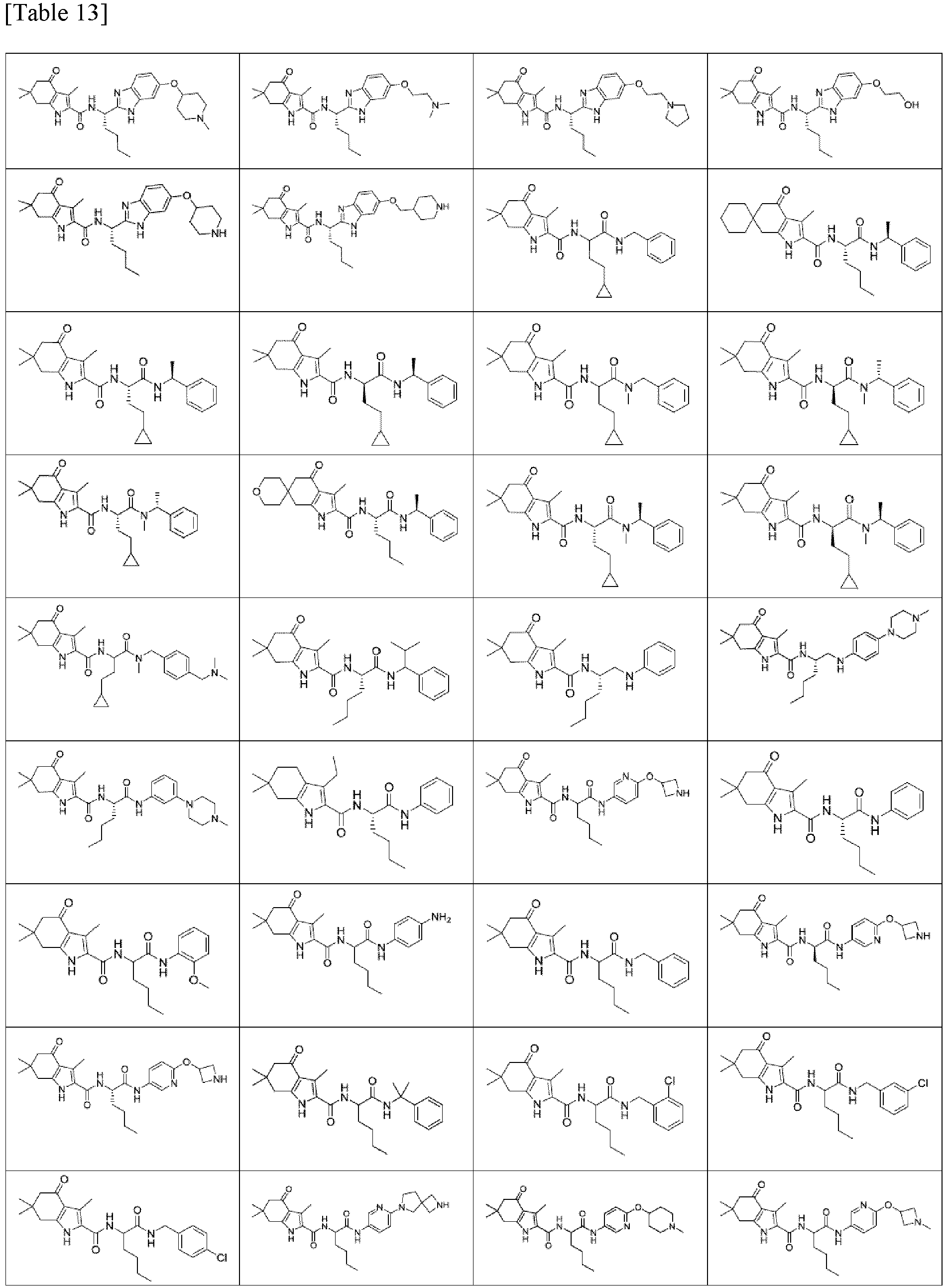

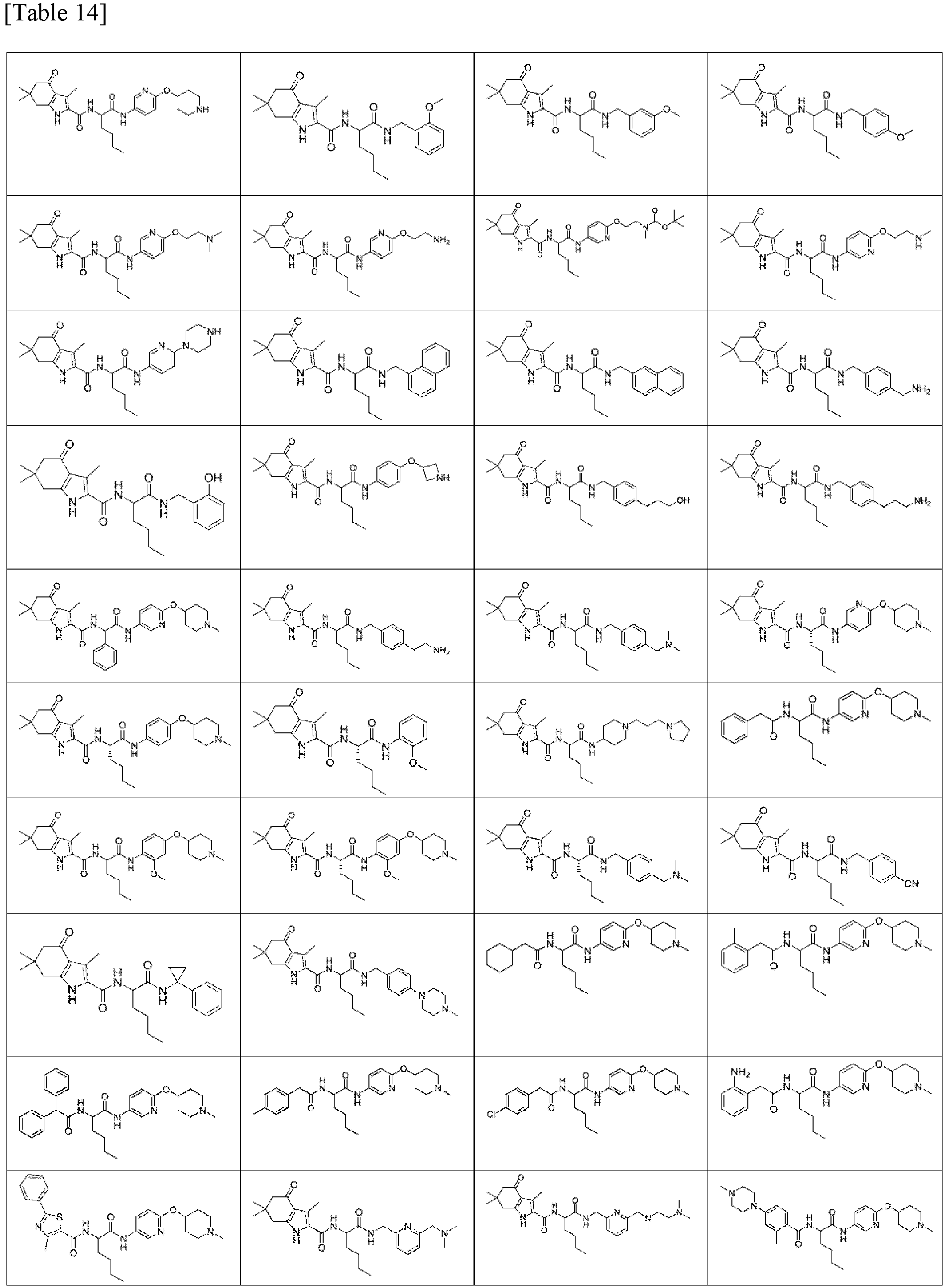

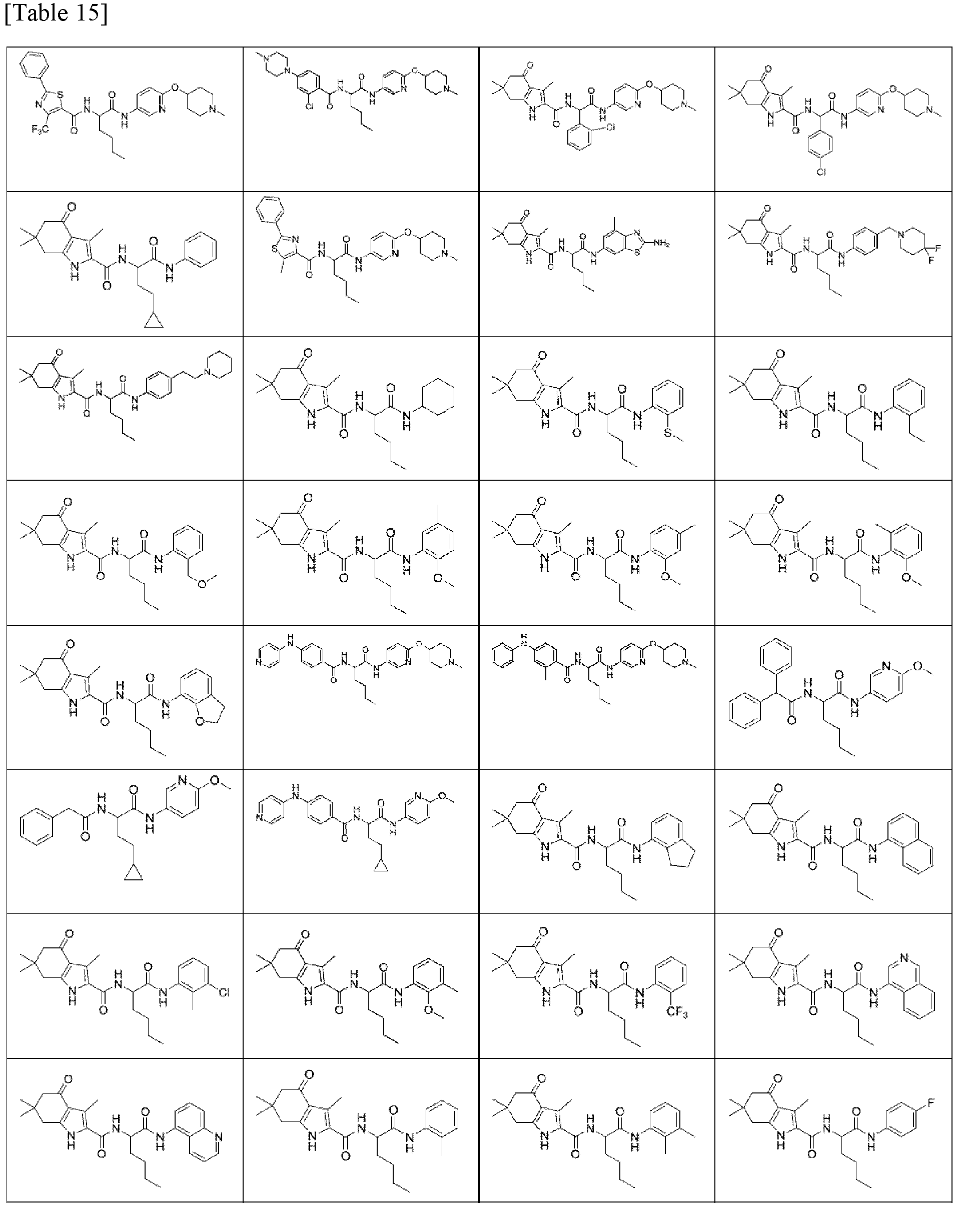

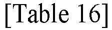

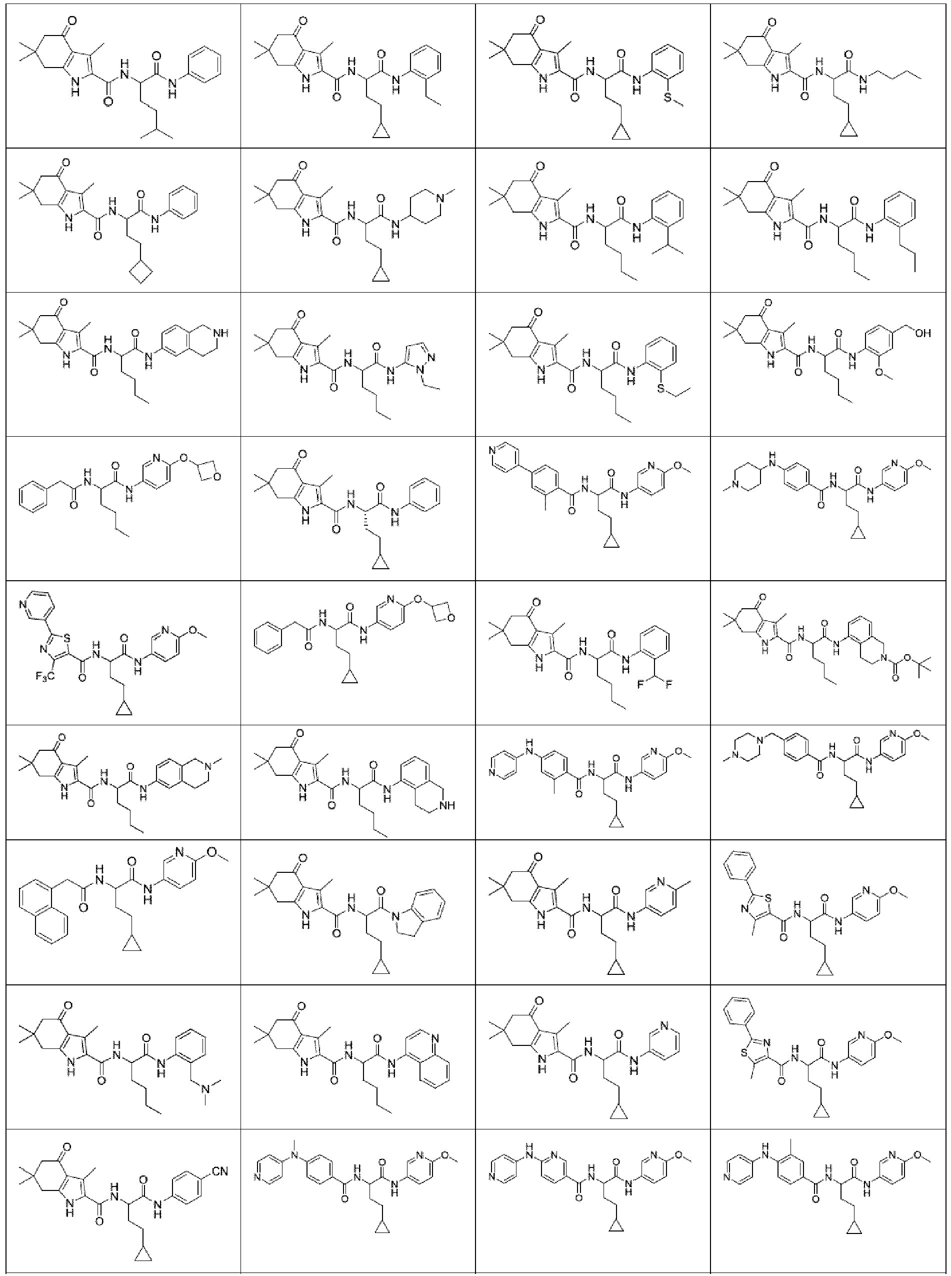

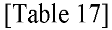

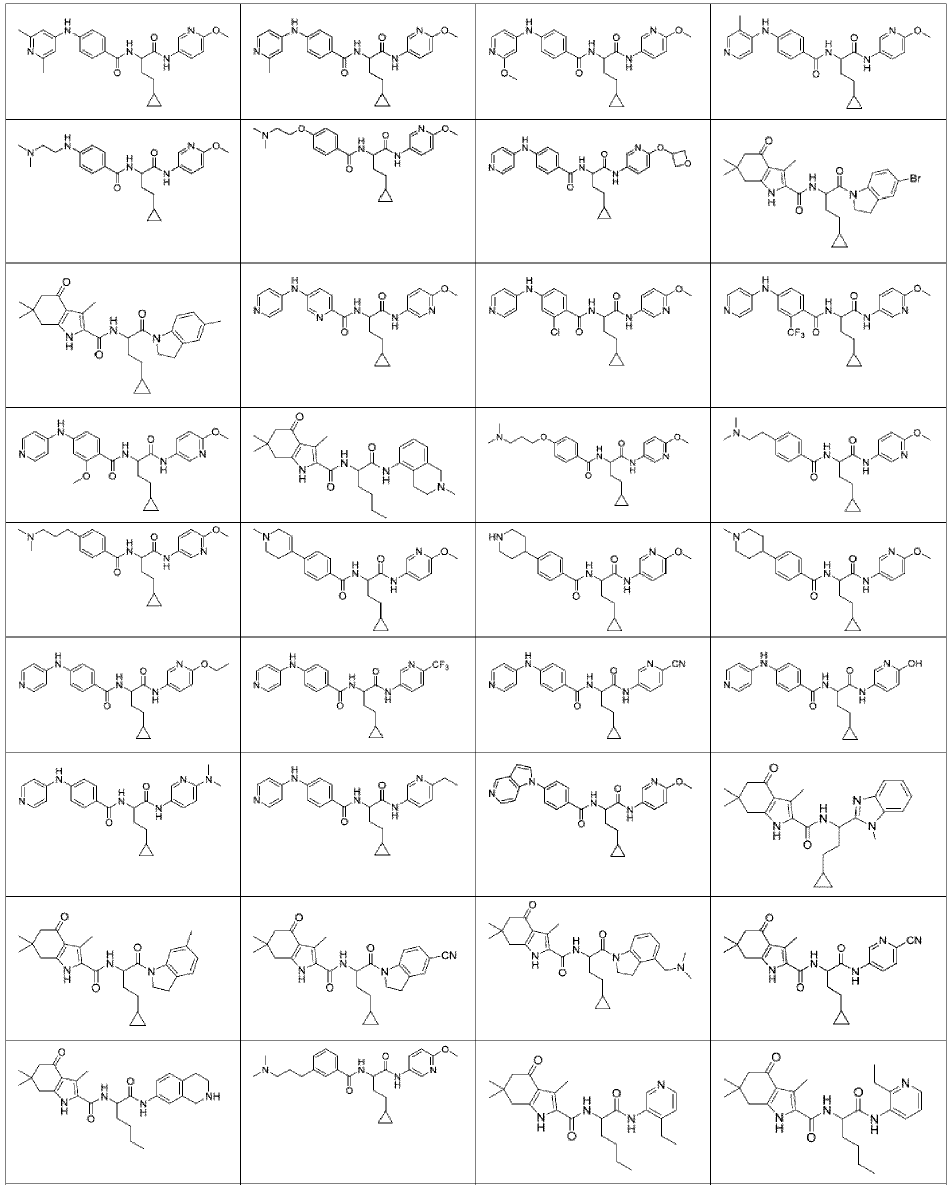

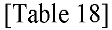

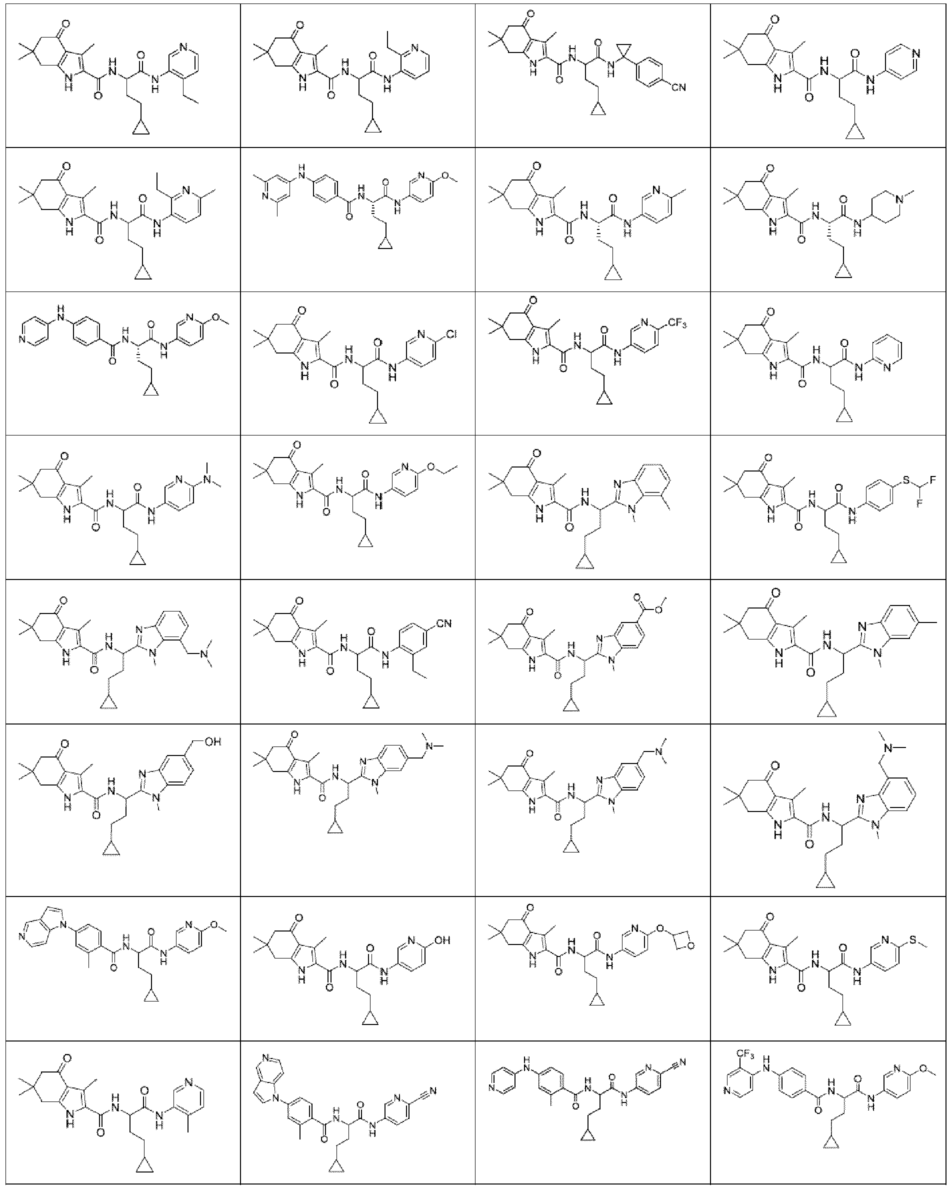

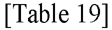

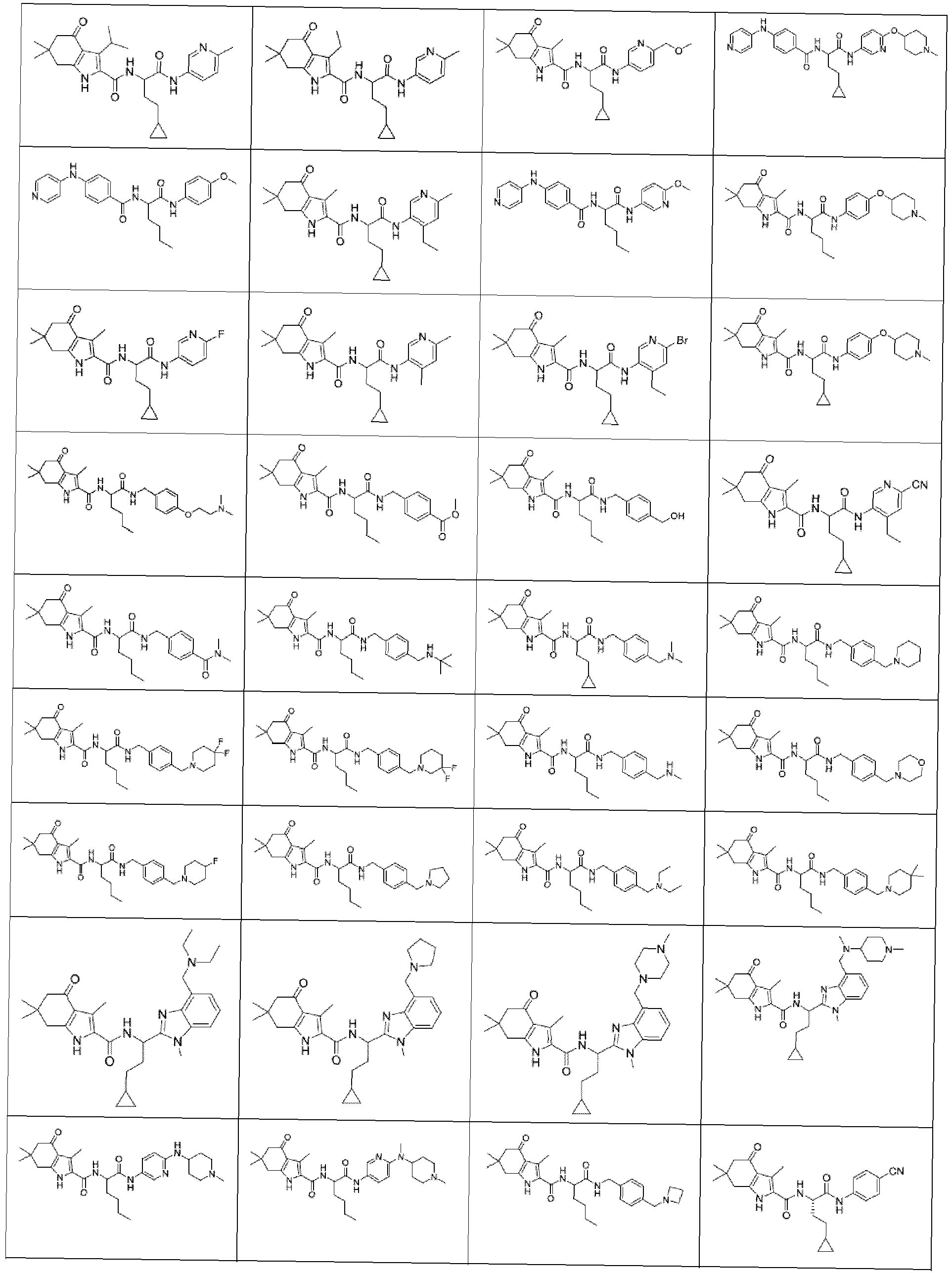

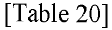

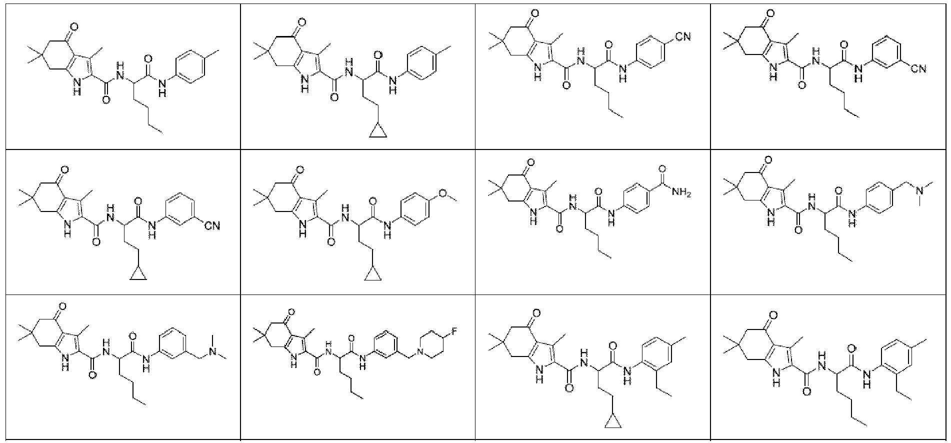

The compound (I) of the present embodiment or the pharmacologically acceptable salt thereof may exist as a hydrate or a solvate. Arbitrary hydrates and solvates formed by derivatives represented by the general formula (I) or salts thereof, including the preferred compounds specifically described above are all encompassed by the scope of the present invention. Examples of the solvent capable of forming such a solvate include methanol, ethanol, isopropyl alcohol, acetone, ethyl acetate, dichloromethane, and diisopropyl ether.

The compound (I) of the present embodiment can be a pharmacologically acceptable salt thereof, if necessary. The pharmacologically acceptable salt means a salt with a pharmaceutically acceptable nontoxic base or acid (e.g., an inorganic or organic base and an inorganic or organic acid). The pharmaceutically acceptable salt of the compound (I) of the present embodiment can be produced by methods described in Jikken Kagaku Koza (Encyclopedia of Experimental Chemistry in English), the 5th edition (ed. by The Chemical Society of Japan, published by Maruzen Co., Ltd.), J. Pharm. Sci. 1977, 66, 1-19, and "Handbook of Pharmaceutical Salts: Properties, Selection, and Use" by Stahl and Wermuth (Wiley-VCH, Weinheim, Germany, 2002) and methods equivalent thereto.

Examples of the salt derived from the pharmaceutically acceptable nontoxic base can include salts with inorganic bases, such as sodium salt, potassium salt, calcium salt, and magnesium salt, and salts with organic bases such as piperidine, morpholine, pyrrolidine, arginine, and lysine.

Examples of the salt derived from the pharmaceutically acceptable nontoxic acid include acid-addition salts with mineral acids such as hydrochloric acid, hydrobromic acid, sulfuric acid, and nitric acid, and acid-addition salts with organic acids such as formic acid, acetic acid, maleic acid, fumaric acid, succinic acid, lactic acid, malic acid, tartaric acid, citric acid, methanesulfonic acid, p-toluenesulfonic acid, salicylic acid, stearic acid, and palmitic acid.

The compound (I) of the present embodiment or the pharmacologically acceptable salt thereof also includes stereoisomers such as racemates and optically active forms.

When the compound (I) of the present embodiment is an optical isomer having one or more asymmetric carbon atoms or sulfur atoms, the conformation of the compound (I) of the present embodiment at each asymmetric carbon atom or sulfur atom may be any of R conformation and S conformation. The present invention also encompasses all of single enantiomers, single diastereomers, mixtures of enantiomers and mixtures of diastereomers. In a mixture of optically active forms, a racemate consisting of the respective optically active isomers in equal amounts is also included in the scope of the present invention. When the compound (I) of the present embodiment is a solid or crystals of a racemate, racemic compounds, racemic mixtures and racemic solid solutions are also included in the scope of the present invention.

In the compound (I) of the present embodiment, a diastereomer mixture can be resolved into respective diastereomers by a common method such as chromatography or crystallization. Such respective diastereomers may be prepared by using a stereochemically single starting material or by a synthesis method using stereoselective reaction.

When the compound (I) of the present embodiment has geometric isomers such as a cis isomer and a trans isomer, the present invention encompasses all the geometric isomers.

When the compound (I) of the present embodiment has tautomers, the present invention encompasses all the tautomers.

The compound (I) of the present embodiment or the pharmacologically acceptable salt thereof may be a compound labeled with a radioisotope (e.g., ³H, ¹⁴C, and ³⁵S) or the like. Such a compound is also included in the present invention.

The compound (I) of the present embodiment or the pharmacologically acceptable salt thereof may be a deuterium converter obtained by converting ¹H to ²H (D). Such a compound is also included in the present invention.

### Method for producing compound (I) of present embodiment

The compound (I) of the present embodiment can be produced in accordance with, for example, methods mentioned in detail in synthetic routes 1 to 46 given below or methods equivalent thereto, or methods described in other literatures or methods equivalent thereto.

Compounds (2) to (122) in the formulas may each form a salt. Examples of such a salt include the same as the salt of the compound (I). A compound obtained in each step may be used, in next reaction, either directly as a reaction solution or after being obtained as a crude product, and can be easily isolated or purified by a separation approach such as recrystallization, distillation, or chromatography from a reaction mixture in accordance with a routine method.

### [Synthetic route 1]

When the compound (I) is represented by a compound wherein R¹ is an oxygen atom, i.e., a compound (2), this compound can be produced in accordance with, for example, a method shown in synthetic route 1 or a method equivalent thereto, or methods described in other literatures or methods equivalent thereto. 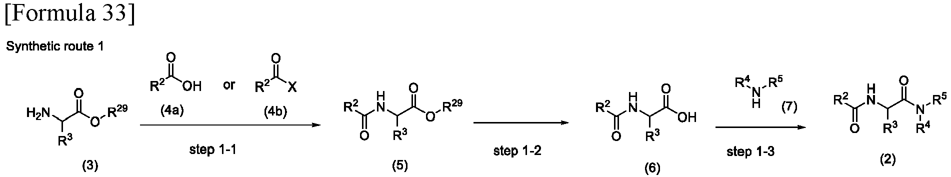 wherein R²⁹ represents a C₁ to C₆ alkyl group, X represents a halogen atom such as a chlorine atom or a bromine atom, and R², R³, R⁴ and R⁵ are as defined above.

### Step 1-1

A compound (5) can be produced by amidating a compound (3) using a compound (4a) or a compound (4b).

Reaction conditions can involve adding the compound (4a) or the compound (4b), etc. in N,N-dimethylformamide, N,N-dimethylacetamide, N-methyl-2-pyrrolidone, ethyl acetate, dichloromethane, acetonitrile, toluene, benzene, 1,4-dioxane, tetrahydrofuran, or the like, or a mixed solvent thereof, and performing the reaction at 0°C to room temperature, or optionally by heating to reflux. A base such as triethylamine or N,N-diisopropylethylamine may be added, if necessary. A condensing agent such as 1-[bis(dimethylamino)methylene]-1H-1,2,3-triazolo[4,5-b]pyridinium-3-oxide hexafluorophosphate (HATU), 1-ethyl-3-(3-dimethylaminopropyl)carbodiimide (EDCI), N,N'-dicyclohexylcarbodiimide (DCC), or 4-(4,6-dimethoxy-1,3,5-triazin-2-yl)-4-methylmorpholinium chloride (DMT-MM), or a reaction accelerator such as N,N-dimethyl-4-aminopyridine, pyridine, 1-hydroxybenzotriazole (HOBT), or 1-hydroxy-7-azabenzotriazole (HOAt) can be added, if necessary.

### Step 1-2

A compound (6) can be produced by hydrolyzing the compound (5).

Reaction conditions can involve adding an alkali metal salt such as lithium hydroxide, sodium hydroxide, potassium hydroxide, potassium carbonate, sodium carbonate, or cesium carbonate in water, methanol, ethanol, 1-propanol, isopropyl alcohol, tetrahydrofuran, 1,4-dioxane, or the like, or an aqueous mixed solvent thereof, and thereby performing the reaction under basic conditions at 0°C to a reflux temperature under heating. Alternatively, the reaction can be performed under acidic conditions at 0°C to a reflux temperature under heating by adding hydrogen chloride or the like in water, tetrahydrofuran, or 1,4-dioxane, or an aqueous mixed solvent thereof.

### Step 1-3

A compound (2) can be produced by amidating the compound (6) using a compound (7).

Reaction conditions can involve adding the compound (7) or a salt thereof, for example, aniline or benzylamine, and performing the reaction in the same manner as in step 1-1.

### [Synthetic route 2]

The compound (2) mentioned above can also be produced in accordance with, for example, a method shown in synthetic route 2 or a method equivalent thereto, or methods described in other literatures or methods equivalent thereto. 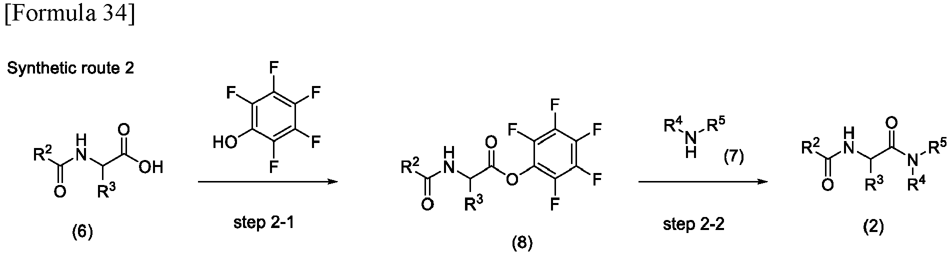 wherein R², R³, R⁴ and R⁵ are as defined above.

### Step 2-1

A compound (8) can be produced by esterifying a compound (6) using pentafluorophenol.

Reaction conditions can involve adding pentafluorophenol, and performing the reaction in the same manner as in step 1-3.

### Step 2-2

The compound (2) can be produced by amidating the compound (8) using a compound (7).

Reaction conditions can involve adding the compound (7) or a salt thereof, for example, aniline or benzylamine, in N,N-dimethylformamide, N,N-dimethylacetamide, N-methyl-2-pyrrolidone, ethyl acetate, dichloromethane, acetonitrile, toluene, benzene, 1,4-dioxane, tetrahydrofuran, or the like, or a mixed solvent thereof, and performing the reaction at -78°C to a reflux temperature under heating. Triethylamine, N,N-diisopropylethylamine, pyridine, or the like can be added, if necessary.

### [Synthetic route 3]

The compound (2) mentioned above can also be produced in accordance with, for example, a method mentioned in detail in synthetic route 3 or a method equivalent thereto, or methods described in other literatures or methods equivalent thereto. 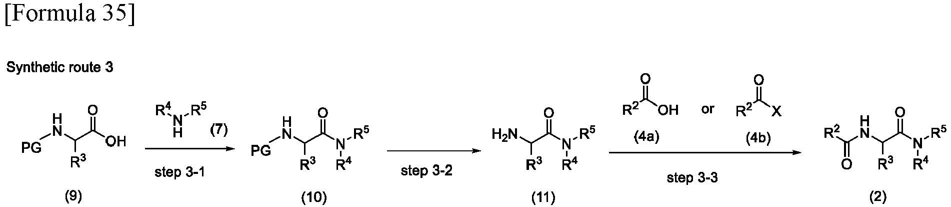 wherein PG represents a protective group such as a tert-butoxycarbonyl group, a benzyloxycarbonyl group, a 9-fluorenylmethyloxycarbonyl group, a benzyl group, an acetyl group, a benzoyl group or a tert-butyldimethylsilyl group, and X, R², R³, R⁴ and R⁵ are as defined above.

### Step 3-1

A compound (10) can be produced by amidating a compound (9) using a compound (7). Reaction conditions can involve performing the reaction in the same manner as in step 1-3.

### Step 3-2

A compound (11) can be produced by removing the protective group from the compound (10).

When PG is a tert-butoxycarbonyl group, reaction conditions can involve adding an acid such as trifluoroacetic acid, p-toluenesulfonic acid, hydrogen chloride, hydrobromic acid, sulfuric acid, a boron trifluoride-diethyl ether complex, boron tribromide, or aluminum chloride in dichloromethane, chloroform, 1,4-dioxane, tetrahydrofuran, toluene, benzene, methanol, ethanol, ethyl acetate, or water, or the like, or a mixed solvent thereof, and performing the reaction at -78°C to room temperature, or optionally by heating to reflux.

When PG is a benzyloxycarbonyl group, the reaction can be performed at 0°C to a reflux temperature under heating by adding a catalyst such as palladium carbon, rhodium carbon, platinum carbon, or platinum oxide in a hydrogen atmosphere in methanol, ethanol, isopropyl alcohol, 1,4-dioxane, 1,2-dimethoxyethane, ethyl acetate, water, tetrahydrofuran, tert-butyl methyl ether, N,N-dimethylformamide, toluene, or the like, or a mixed solvent thereof. An acid such as acetic acid, trifluoroacetic acid, or 2,2,2-trifluoroethanol can be added as a reaction accelerator, if necessary. Alternatively, the reaction can be performed at 0°C to a reflux temperature under heating by adding an acid such as trifluoroacetic acid in methanol, ethanol, isopropyl alcohol, 1,4-dioxane, 1,2-dimethoxyethane, ethyl acetate, water, tetrahydrofuran, tert-butyl methyl ether, N,N-dimethylformamide, toluene, or the like, or a mixed solvent thereof.

### Step 3-3

The compound (2) can be produced by amidating the compound (11) using a compound (4a) or a compound (4b).

Reaction conditions can involve adding the compound (4a), or the compound (4b), etc., and performing the reaction in the same manner as in step 1-1.

### [Synthetic route 4]

When the compound (2) mentioned above is represented by a compound (2a) or a compound (2b), this compound can be produced in accordance with, for example, a method mentioned in detail in the following synthetic route 4 or a method equivalent thereto, or methods described in other literatures or methods equivalent thereto. 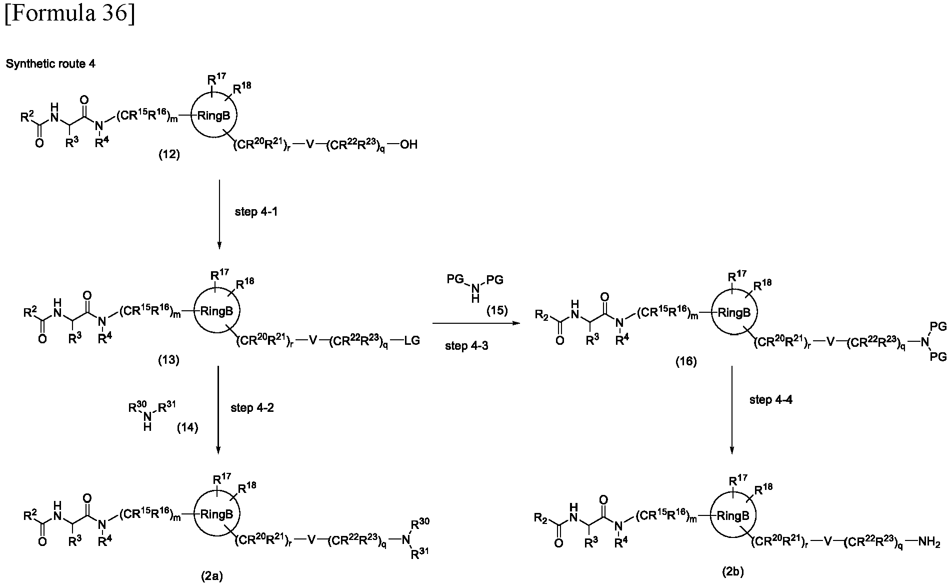 wherein LG represents a halogen atom such as a chlorine atom or a bromine atom, or a leaving group such as a methanesulfonyloxy group, a trifluoromethanesulfonyloxy group or a p-toluenesulfonyloxy group, R³⁰ and R³¹ each independently represent a hydrogen atom, a C₁ to C₆ alkyl group or a halo-Ci to C₆ alkyl group, R³⁰ and R³¹ are optionally bonded to each other to form a ring, and PG, R², R³, R⁴, R¹⁵, R¹⁶, R¹⁷, R¹⁸, R²⁰, R²¹, R²², R²³, RingB, V, m, r and q are as defined above.

### Step 4-1

A compound (13) can be produced by converting the hydroxy group of a compound (12) to an appropriate leaving group (LG) such as a halogen atom, a methanesulfonyloxy group, or a p-toluenesulfonyloxy group.

For example, when LG is a chlorine atom, the reaction can be performed at -78°C to a reflux temperature under heating by adding a chlorinating agent such as thionyl chloride or phosphorus oxychloride in dichloromethane, chloroform, benzene, toluene, N,N-dimethylformamide, tetrahydrofuran, pyridine, diethyl ether, or the like, or a mixed solvent thereof.

For example, when LG is a bromine atom, the reaction can be performed at -78°C to a reflux temperature under heating by adding a brominating agent such as carbon tetrabromide or N-bromosuccinimide, and a phosphorus reagent such as triphenylphosphine in dichloromethane, 1,2-dichloroethane, acetonitrile, tetrahydrofuran, toluene, or the like, or a mixed solvent thereof.

For example, when LG is a methanesulfonyloxy group, the reaction can be performed at -78°C to room temperature, or optionally by heating to reflux, by adding a methanesulfonylating agent such as methanesulfonyl chloride in dichloromethane, chloroform, 1,4-dioxane, tetrahydrofuran, toluene, benzene, water, or the like, or a mixed solvent thereof. A base such as triethylamine, N,N-diisopropylethylamine, or pyridine may be added, if necessary.

For example, when LG is a p-toluenesulfonyloxy group, the reaction can be performed at -78°C to room temperature, or optionally by heating to reflux, by adding a p-toluenesulfonylating agent such as p-toluenesulfonyl chloride in dichloromethane, chloroform, 1,4-dioxane, tetrahydrofuran, toluene, benzene, water, or the like, or a mixed solvent thereof. A base such as triethylamine, N,N-diisopropylethylamine, or pyridine may be added, if necessary.

### Step 4-2

The compound (2a) can be produced by reacting the compound (13) with a compound (14).

Reaction conditions can involve adding the compound (14) or a salt thereof, for example, methylamine or dimethylamine, or a tetrahydrofuran solution or the like containing the compound or the salt in dichloromethane, 1,2-dichloroethane, benzene, toluene, tetrahydrofuran, N,N-dimethylformamide, 1,4-dioxane, acetonitrile, or the like, or a mixed solvent thereof, and performing the reaction at -78°C to a reflux temperature under heating. A base such as sodium hydride, potassium carbonate, sodium carbonate, cesium carbonate, triethylamine, N,N-diisopropylethylamine, pyridine, 1,8-diazabicyclo[5.4.0]undec-7-ene (DBU), or 1,5-diazabicyclo[4.3.0]non-5-ene (DBN) can be added, if necessary.

### Step 4-3

A compound (16) can be produced by reacting the compound (13) with a compound (15).

Reaction conditions can involve adding the compound (15) or a salt thereof, for example, di-tert-butyl iminodicarboxylate, in dichloromethane, 1,2-dichloroethane, benzene, toluene, tetrahydrofuran, N,N-dimethylformamide, 1,4-dioxane, acetonitrile, or the like, or a mixed solvent thereof, and performing the reaction at -78°C to a reflux temperature under heating. A base such as sodium hydride, potassium carbonate, sodium carbonate, cesium carbonate, triethylamine, N,N-diisopropylethylamine, pyridine, 1,8-diazabicyclo[5.4.0]undec-7-ene (DBU), or 1,5-diazabicyclo[4.3.0]non-5-ene (DBN) can be added, if necessary.

### Step 4-4

The compound (2b) can be produced by removing the protective group from the compound (16).

When PG is a tert-butoxycarbonyl group, reaction conditions can involve performing the reaction in the same manner as in step 3-2.

### [Synthetic route 5]

When the compound (2) mentioned above is represented by a compound (2c), this compound can be produced in accordance with, for example, a method mentioned in detail in the following synthetic route 5 or a method equivalent thereto, or methods described in other literatures or methods equivalent thereto. 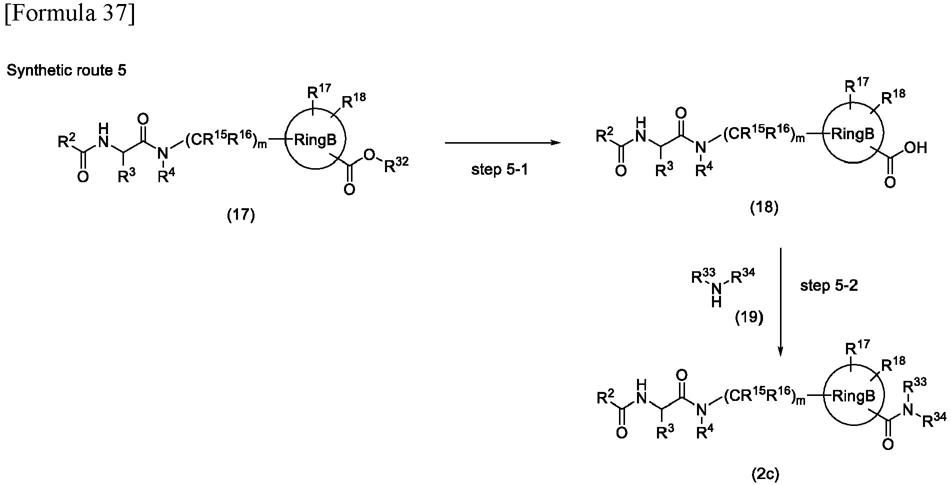 wherein R³² represents a C₁ to C₆ alkyl group, R³³ and R³⁴ each independently represent a hydrogen atom or a C₁ to C₆ alkyl group, R³³ and R³⁴ are optionally bonded to each other to form a ring, and R², R³, R⁴, R¹⁵, R¹⁶, R¹⁷, R¹⁸, RingB and m are as defined above.

### Step 5-1

A compound (18) can be produced by hydrolyzing a compound (17).

Reaction conditions can involve performing the reaction in the same manner as in step 1-2.

### Step 5-2

The compound (2c) can be produced by amidating the compound (18) using a compound (19).

Reaction conditions can involve adding the compound (19), for example, a solution such as methanol, ethanol, 1,4-dioxane, or water containing ammonia, ammonium chloride, ammonium acetate, ammonium formate, primary amine, secondary amine, or a salt thereof, for example, methylamine or dimethylamine, or a tetrahydrofuran solution or the like containing the compound or the salt, and performing the reaction in the same manner as in step 1-3.

### [Synthetic route 6]

When the compound (2) mentioned above is represented by a compound (2d), this compound can be produced in accordance with, for example, a method mentioned in detail in the following synthetic route 6 or a method equivalent thereto, or methods described in other literatures or methods equivalent thereto. 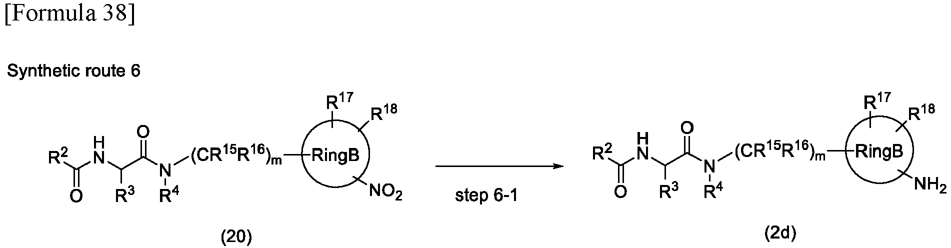 wherein R², R³, R⁴, R¹⁵, R¹⁶, R¹⁷, R¹⁸, RingB and m are as defined above.

### Step 6-1

The compound (2d) can be produced by reducing the nitro group of a compound (20).

General nitro group reduction conditions can be used as reaction conditions. For example, the reaction can be performed at 0°C to room temperature, or optionally by heating to reflux, using an iron powder, a zinc powder, tin(II) chloride, metal tin, metal indium, metal samarium, Raney nickel, formic acid, sodium borohydride, nickel borohydride, cobalt borohydride, lithium aluminum hydride, sodium dithionite, sodium sulfide, sodium bisulfide or hydrazine, or the like in methanol, ethanol, isopropyl alcohol, 1,4-dioxane, 1,2-dimethoxyethane, ethyl acetate, water, tetrahydrofuran, diethyl ether, tert-butyl methyl ether, N,N-dimethylformamide, toluene, n-hexane, or the like, or a mixed solvent thereof. An acid such as ammonium chloride, hydrogen chloride, acetic acid, trifluoroacetic acid, or sulfuric acid, or a base such as potassium carbonate, sodium carbonate, cesium carbonate, sodium hydroxide, potassium hydroxide, tripotassium phosphate, sodium bicarbonate, potassium bicarbonate, pyridine, triethylamine, or N,N-diisopropylethylamine can be added, if necessary. Alternatively, the reduction may be performed by adding a catalyst such as palladium carbon, rhodium carbon, platinum carbon, or platinum oxide in a hydrogen atmosphere in methanol, ethanol, isopropyl alcohol, 1,4-dioxane, 1,2-dimethoxyethane, acetic acid, ethyl acetate, water, tetrahydrofuran, tert-butyl methyl ether, N,N-dimethylformamide, toluene, or the like, or a mixed solvent thereof.

### [Synthetic route 7]

When the compound (2) mentioned above is represented by a compound (2e) or a compound (2f), this compound can be produced in accordance with, for example, a method mentioned in detail in the following synthetic route 7 or a method equivalent thereto, or methods described in other literatures or methods equivalent thereto. 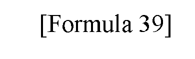 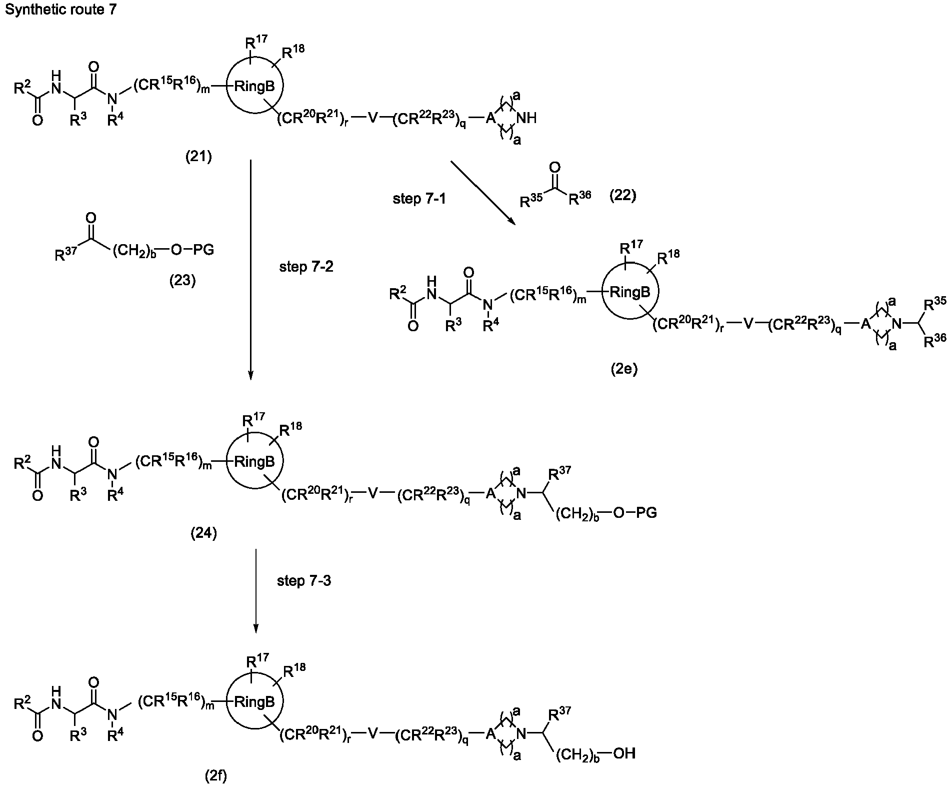 wherein A represents a carbon atom or a nitrogen atom, R³⁵, R³⁶ and R³⁷ each independently represent a hydrogen atom or a C₁ to C₆ alkyl group, a and b each independently represent an integer of 0 to 3, and PG, R², R³, R⁴, R¹⁵, R¹⁶, R¹⁷, R¹⁸, R²⁰, R²¹, R²², R²³, RingB, V, m, r and q are as defined above.

### Step 7-1

The compound (2e) can be produced by performing reductive alkylation using a compound (21) and a compound (22).

Reaction conditions can involve adding, the compound (22), for example, paraformaldehyde, an aqueous formalin solution, or a glycolaldehyde dimer, in dichloromethane, 1,2-dichloroethane, chloroform, 1,4-dioxane, tetrahydrofuran, toluene, benzene, methanol, ethanol, or the like, or a mixed solvent thereof, adding a reducing agent such as sodium triacetoxyborohydride, sodium cyanoborohydride, sodium borohydride, lithium borohydride, a borane-dimethyl sulfide complex, lithium aluminum hydride, or 2-picoline borane, and performing the reaction at -78°C to room temperature, or optionally by heating to reflux. Alternatively, the reduction may be performed by adding a catalyst such as palladium carbon, rhodium carbon, platinum carbon, or platinum oxide in a hydrogen atmosphere in methanol, ethanol, isopropyl alcohol, 1,4-dioxane, 1,2-dimethoxyethane, ethyl acetate, water, tetrahydrofuran, tert-butyl methyl ether, N,N-dimethylformamide, toluene, or the like, or a mixed solvent thereof. A reaction accelerator such as acetic acid, trifluoroacetic acid, p-toluenesulfonic acid, a boron trifluoride-diethyl ether complex, boron tribromide, aluminum chloride, chlorotrimethylsilane, 2,2,2-trifluoroethanol, or tetraisopropyl o-titanate can be added, if necessary.

### Step 7-2

A compound (24) can be produced by performing reductive alkylation using a compound (21) and a compound (23).

Reaction conditions can involve performing the reaction in the same manner as in step 7-1.

### Step 7-3

The compound (2f) can be produced by removing the protective group from the compound (24).

When PG is a benzyl group, reaction conditions can involve performing the reaction at 0°C to room temperature, or optionally by heating to reflux, using Raney nickel, hydrogen, ammonium formate, or the like in methanol, ethanol, isopropyl alcohol, 1,4-dioxane, 1,2-dimethoxyethane, acetic acid, ethyl acetate, water, tetrahydrofuran, diethyl ether, tert-butyl methyl ether, N,N-dimethylformamide, toluene, n-hexane, or the like, or a mixed solvent thereof. A catalyst such as palladium carbon, rhodium carbon, platinum carbon, palladium hydroxide, or platinum oxide can be added, if necessary. An acid such as trifluoroacetic acid may be added as a reaction accelerator, if necessary.

Alternatively, the reaction can be performed at -78°C to a reflux temperature under heating by adding an acid such as trifluoroacetic acid, p-toluenesulfonic acid, sulfuric acid, hydrogen chloride, hydrobromic acid, a boron trifluoride-diethyl ether complex, boron tribromide, or aluminum chloride in dichloromethane, chloroform, 1,4-dioxane, tetrahydrofuran, toluene, benzene, water, or the like, or a mixed solvent thereof. Anisole, pentamethylbenzene, dimethyl sulfide, or the like can be added as a reaction accelerator, if necessary.

### [Synthetic route 8]

When the compound (2) mentioned above is represented by a compound (2g), this compound can be produced in accordance with, for example, a method mentioned in detail in the following synthetic route 8 or a method equivalent thereto, or methods described in other literatures or methods equivalent thereto. 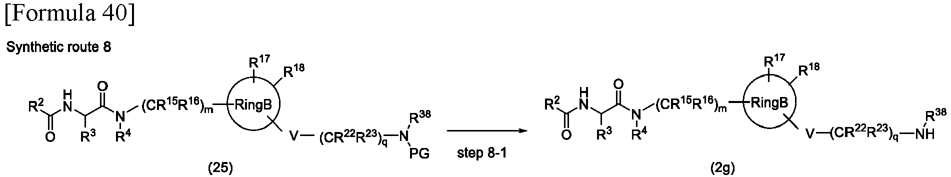 wherein R³⁸ represents a hydrogen atom or a C₁ to C₆ alkyl group, and PG, R², R³, R⁴, R¹⁵, R¹⁶, R¹⁷, R¹⁸, R²², R²³, RingB, V, m and q are as defined above.

### Step 8-1

The compound (2g) can be produced by removing the protective group from a compound (25).

When PG is a tert-butoxycarbonyl group, reaction conditions can involve performing the reaction in the same manner as in step 3-2.

### [Synthetic route 9]

When the compound (2) mentioned above is represented by a compound (2h), this compound can be produced in accordance with, for example, a method mentioned in detail in the following synthetic route 9 or a method equivalent thereto, or methods described in other literatures or methods equivalent thereto. 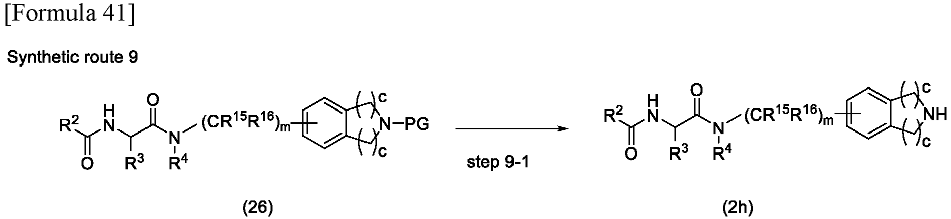 wherein each c independently represents an integer of 0 to 3, and PG, R², R³, R⁴, R¹⁵, R¹⁶ and m are as defined above.

### Step 9-1

The compound (2h) can be produced by removing the protective group from a compound (26).

When PG is a tert-butoxycarbonyl group, reaction conditions can involve performing the reaction in the same manner as in step 3-2.

### [Synthetic route 10]

When the compound (2) mentioned above is represented by a compound (2i), this compound can be produced in accordance with, for example, a method mentioned in detail in the following synthetic route 10 or a method equivalent thereto, or methods described in other literatures or methods equivalent thereto. 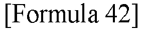 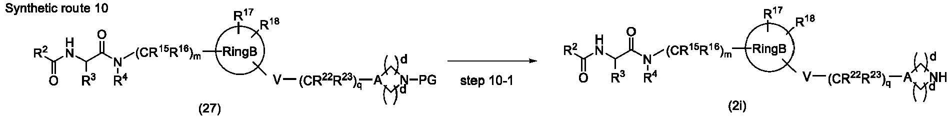 wherein each d is independently an integer of 0 to 3, and A, PG, R², R³, R⁴, R¹⁵, R¹⁶, R¹⁷, R¹⁸, R²², R²³, RingB, V, m and q are as defined above.

### Step 10-1

The compound (2i) can be produced by removing the protective group from a compound (27).

When PG is a tert-butoxycarbonyl group, reaction conditions can involve performing the reaction in the same manner as in step 3-2.

### [Synthetic route 11]

When the compound (2) mentioned above is represented by a compound (2j), this compound can be produced in accordance with, for example, a method mentioned in detail in the following synthetic route 11 or a method equivalent thereto, or methods described in other literatures or methods equivalent thereto. 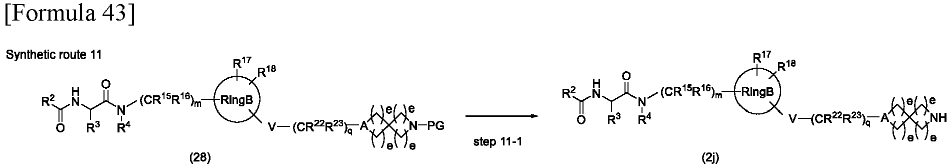 wherein each e is independently an integer of 0 to 3, and A, PG, R², R³, R⁴, R¹⁵, R¹⁶, R¹⁷, R¹⁸, R²², R²³, RingB, V, m, and q are as defined above.

### Step 11-1

The compound (2j) can be produced by removing the protective group from a compound (28).

When PG is a tert-butoxycarbonyl group, reaction conditions can involve performing the reaction in the same manner as in step 3-2.

### [Synthetic route 12]

When the compound (2) mentioned above is represented by a compound (2k), this compound can be produced in accordance with, for example, a method mentioned in detail in the following synthetic route 12 or a method equivalent thereto, or methods described in other literatures or methods equivalent thereto. 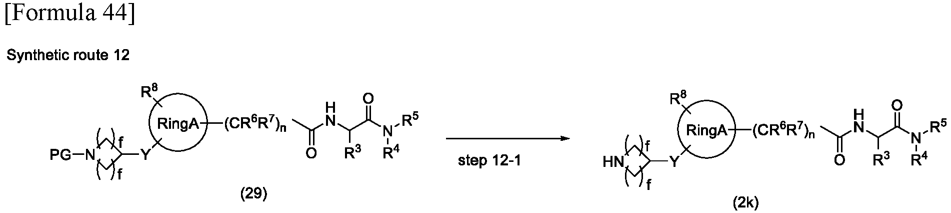 wherein each f is independently an integer of 0 to 3, and PG, R³, R⁴, R⁵, R⁶, R⁷, R⁸, RingA, Y and n are as defined above.

### Step 12-1

The compound (2k) can be produced by removing the protective group from a compound (29).

When PG is a tert-butoxycarbonyl group, reaction conditions can involve performing the reaction in the same manner as in step 3-2.

### [Synthetic route 13]

When the compound (2) mentioned above is represented by a compound (21), this compound can be produced in accordance with, for example, a method mentioned in detail in the following synthetic route 13 or a method equivalent thereto, or methods described in other literatures or methods equivalent thereto. 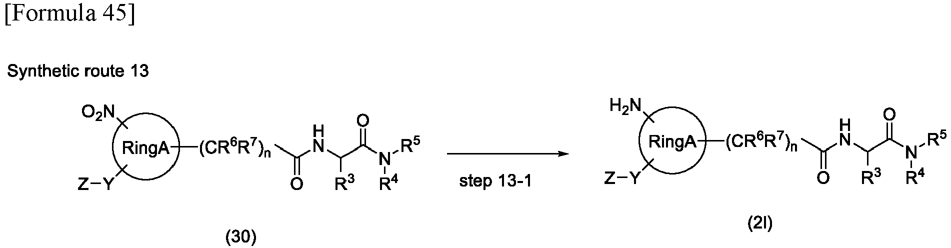 wherein R³, R⁴, R⁵, R⁶, R⁷, RingA, Y, Z and n are as defined above.

### Step 13-1

The compound (21) can be produced by reducing the nitro group of a compound (30).

Reaction conditions can involve performing the reaction in the same manner as in step 6-1.

### [Synthetic route 14]

When the compound (7) mentioned above is represented by a compound (7a), this compound can be produced in accordance with, for example, a method mentioned in detail in the following synthetic route 14 or a method equivalent thereto, or methods described in other literatures or methods equivalent thereto. 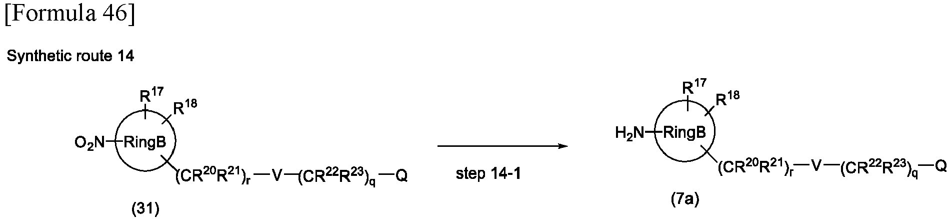 wherein R¹⁷, R¹⁸, R²⁰, R²¹, R²², R²³, RingB, V, Q, r and q are as defined above.

### Step 14-1

The compound (7a) can be produced by reducing the nitro group of a compound (31).

Reaction conditions can involve performing the reaction in the same manner as in step 6-1.

### [Synthetic route 15]

When the compound (31) mentioned above is represented by a compound (31a) or a compound (31b), this compound can be produced in accordance with, for example, a method mentioned in detail in the following synthetic route 15 or a method equivalent thereto, or methods described in other literatures or methods equivalent thereto. 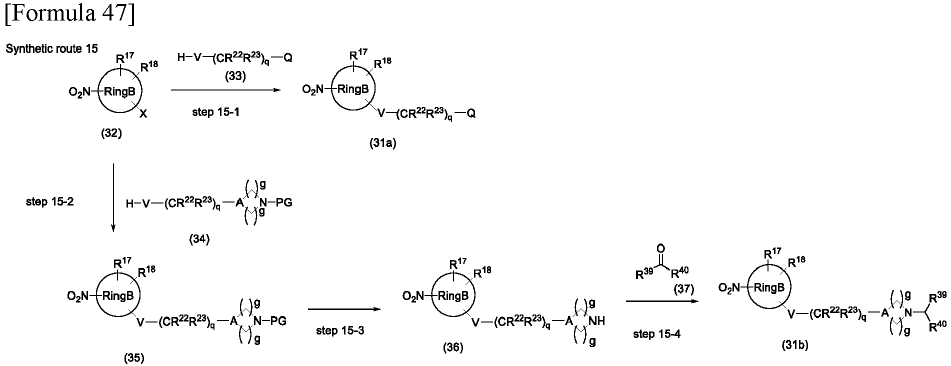 wherein each g is independently an integer of 0 to 3, R³⁹ and R⁴⁰ each independently represent a hydrogen atom or a C₁ to C₆ alkyl group, and A, PG, R¹⁷, R¹⁸, R²², R²³, RingB, V, Q and q are as defined above.

### Step 15-1

The compound (31a) can be produced by reacting a compound (32) with a compound (33).

Reaction conditions can involve adding the compound (33) in acetonitrile, N,N-dimethylformamide, N,N-dimethylacetamide, N-methyl-2-pyrrolidone, 1,4-dioxane, tetrahydrofuran, dimethyl sulfoxide, or the like, or a mixed solvent thereof, and performing the reaction at 0°C to a reflux temperature under heating. A base such as sodium carbonate, potassium carbonate, cesium carbonate, tripotassium phosphate, triethylamine, N,N-diisopropylethylamine, pyridine, 1,8-diazabicyclo[5.4.0]-7-undecene, sodium hydride, or n-butyllithium can be added, if necessary.

### Step 15-2

A compound (35) can be produced by reacting a compound (32) with a compound (34).

Reaction conditions can involve performing the reaction in the same manner as in step 15-1.

### Step 15-3

A compound (36) can be produced by removing the protective group from the compound (35).

When PG is a tert-butoxycarbonyl group, reaction conditions can involve performing the reaction in the same manner as in step 3-2.

### Step 15-4

The compound (31b) can be produced by reacting the compound (36) with a compound (37).

Reaction conditions can involve performing the reaction in the same manner as in step 7-1.

### [Synthetic route 16]

When the compound (31) mentioned above is represented by a compound (31c), this compound can be produced in accordance with, for example, a method mentioned in detail in the following synthetic route 16 or a method equivalent thereto, or methods described in other literatures or methods equivalent thereto. 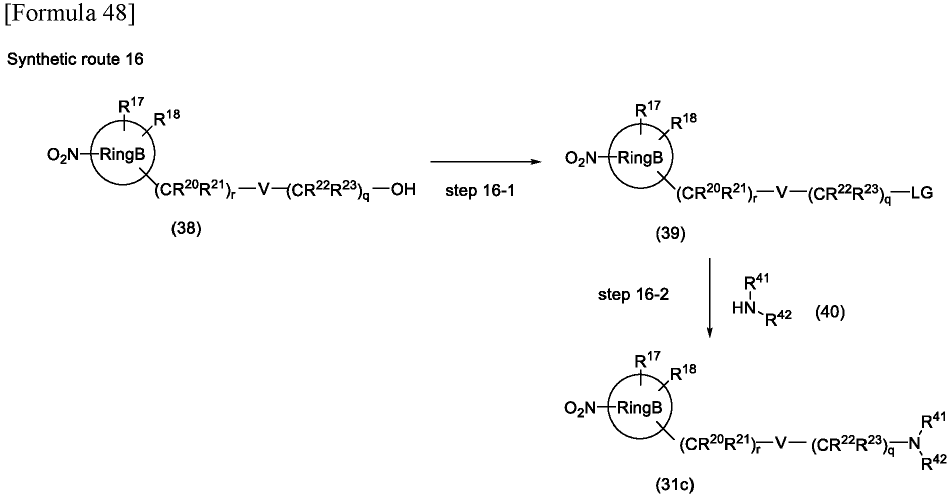 wherein R⁴¹ and R⁴² each independently represent a hydrogen atom, a C₁ to C₆ alkyl group or a halo-C₁ to C₆ alkyl group, R⁴¹ and R⁴² are optionally bonded to each other to form a ring, and LG, R¹⁷, R¹⁸, R²⁰, R²¹, R²², R²³, RingB, V, r and q are as defined above.

### Step 16-1

A compound (39) can be produced by converting the hydroxy group of a compound (38) to an appropriate leaving group (LG).

Reaction conditions can involve performing the reaction in the same manner as in step 4-1.

### Step 16-2

The compound (31c) can be produced by reacting the compound (39) with a compound (40).

Reaction conditions can involve performing the reaction in the same manner as in step 4-2.

### [Synthetic route 17]

When the compound (7) mentioned above is represented by a compound (7b), this compound can be produced in accordance with, for example, a method mentioned in detail in the following synthetic route 17 or a method equivalent thereto, or methods described in other literatures or methods equivalent thereto. 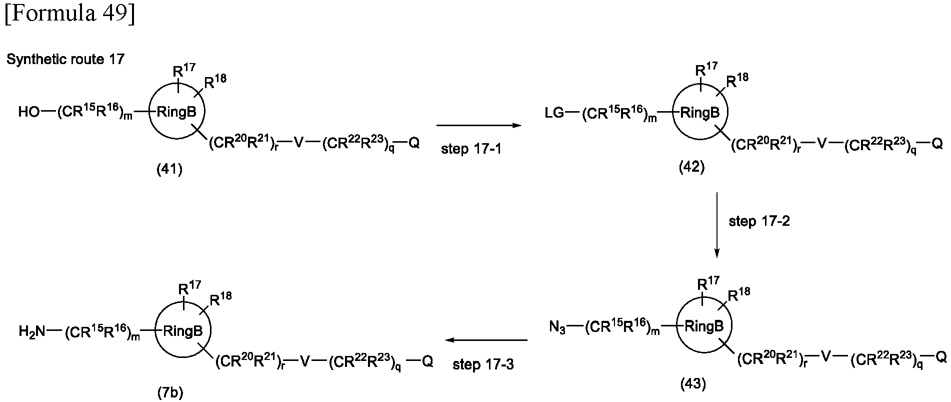 wherein LG, R¹⁵, R¹⁶, R¹⁷, R¹⁸, R²⁰, R²¹, R²², R²³, RingB, V, Q, m, r and q are as defined above.

### Step 17-1

A compound (42) can be produced by converting the hydroxy group of a compound (41) to an appropriate leaving group (LG).

Reaction conditions can involve performing the reaction in the same manner as in step 4-1.

### Step 17-2

A compound (43) can be produced by azidating the compound (42).

General azidation reaction conditions can be applied to reaction conditions. For example, the reaction can be performed at -78°C to a reflux temperature under heating by adding an azidating agent, for example, sodium azide or trimethylsilylazide in water, dimethyl sulfoxide, N,N-dimethylformamide, N,N-dimethylacetamide, N-methyl-2-pyrrolidone, tetrahydrofuran, 1,4-dioxane, acetonitrile, acetone, ethanol, methanol, or the like, or a mixed solvent thereof. Tetra-n-butylammonium fluoride (TBAF), a boron trifluoride-diethyl ether complex, aluminum chloride, or the like can be added as a reaction accelerator, if necessary.

### Step 17-3

The compound (7b) can be produced by reducing the azide group of the compound (43).

General azide group reduction reaction conditions can be applied to reaction conditions. For example, the reaction can be performed at -78°C to a reflux temperature under heating by adding a reducing agent such as lithium aluminum hydride, triphenylphosphine, or hydrogen in water, tetrahydrofuran, 1,4-dioxane, diethyl ether, ethanol, methanol, dimethyl sulfoxide, N,N-dimethylformamide, N,N-dimethylacetamide, N-methyl-2-pyrrolidone, acetonitrile, acetone, ethyl acetate, or the like, or a mixed solvent thereof. A catalyst such as palladium carbon, rhodium carbon, platinum carbon, palladium hydroxide, or platinum oxide can be added, if necessary.

### [Synthetic route 18]

When the compound (7) mentioned above is represented by a compound (7c), this compound can be produced in accordance with, for example, a method mentioned in detail in the following synthetic route 18 or a method equivalent thereto, or methods described in other literatures or methods equivalent thereto. 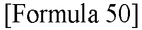 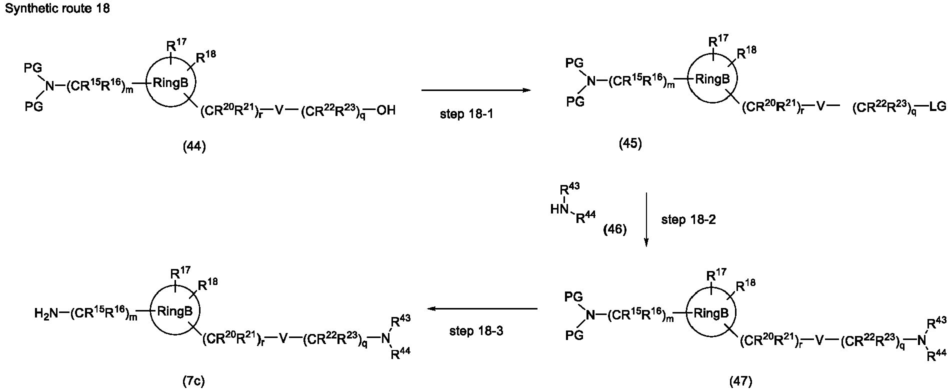 wherein R⁴³ and R⁴⁴ each independently represent a hydrogen atom or a C₁ to C₆ alkyl group (the C₁ to C₆ alkyl group is optionally substituted with one or more C₁ to C₆ alkylamino groups), R⁴³ and R⁴⁴ are optionally bonded to each other to form a ring, and LG, PG, R¹⁵, R¹⁶, R¹⁷, R¹⁸, R²⁰, R²¹, R²², R²³, RingB, V, m, r and q are as defined above.

### Step 18-1

A compound (45) can be produced by converting the hydroxy group of a compound (44) to an appropriate leaving group (LG).

Reaction conditions can involve performing the reaction in the same manner as in step 4-1.

### Step 18-2

A compound (47) can be produced by reacting the compound (45) with a compound (46).

Reaction conditions can involve performing the reaction in the same manner as in step 4-2.

### Step 18-3

The compound (7c) can be produced by removing the protective group from the compound (47).

When PG is a tert-butoxycarbonyl group, reaction conditions can involve performing the reaction in the same manner as in step 3-2.

### [Synthetic route 19]

When the compound (7) mentioned above is represented by a compound (7d), this compound can be produced in accordance with, for example, a method mentioned in detail in the following synthetic route 19 or a method equivalent thereto, or methods described in other literatures or methods equivalent thereto. 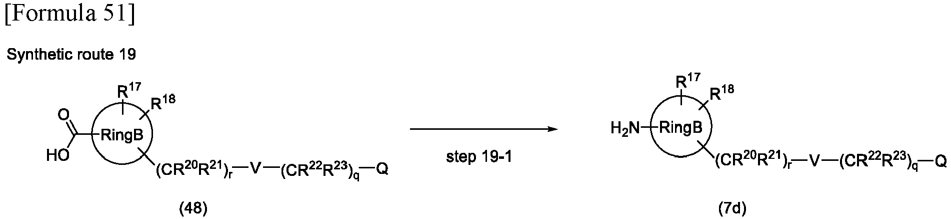 wherein R¹⁷, R¹⁸, R²⁰, R²¹, R²², R²³, RingB, V, Q, r and q are as defined above.

### Step 19-1

The compound (7d) can be produced by subjecting compound (48) to Curtius rearrangement reaction.

General Curtius rearrangement reaction conditions can be applied to reaction conditions. For example, the reaction can be performed at 0°C to a reflux temperature under heating by adding an azidating agent, for example, diphenylphosphorylazide or sodium azide, and a base such as triethylamine or pyridine in toluene, benzene, diphenyl ether, tetrahydrofuran, 1,4-dioxane, acetonitrile, N,N-dimethylformamide, N,N-dimethylacetamide, or the like, or a mixed solvent thereof, and then performed at 0°C to a reflux temperature under heating by adding water or the like. An acid such as hydrogen chloride can be added as a reaction accelerator, if necessary.

### [Synthetic route 20]

When the compound (7) mentioned above is represented by a compound (7e), this compound can be produced in accordance with, for example, a method mentioned in detail in the following synthetic route 20 or a method equivalent thereto, or methods described in other literatures or methods equivalent thereto. 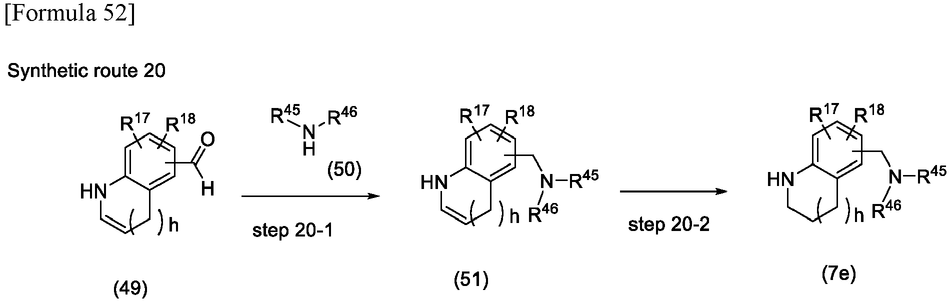 wherein h is an integer of 0 to 3, R⁴⁵ and R⁴⁶ each independently represent a hydrogen atom or a C₁ to C₆ alkyl group, R⁴⁵ and R⁴⁶ are optionally bonded to each other to form a ring, and R¹⁷ and R¹⁸ are as defined above.

### Step 20-1

A compound (51) can be produced by reacting a compound (49) with a compound (50).

Reaction conditions can involve performing the reaction in the same manner as in step 7-1.

### Step 20-2

The compound (7e) can be produced by reducing the compound (51).

Reaction conditions can involve adding a reducing agent such as triethylsilane, sodium triacetoxyborohydride, sodium cyanoborohydride, sodium borohydride, lithium borohydride, a borane-dimethyl sulfide complex, lithium aluminum hydride, or hydrogen without a solvent or in water, tetrahydrofuran, 1,4-dioxane, diethyl ether, ethanol, methanol, dimethyl sulfoxide, N,N-dimethylformamide, N,N-dimethylacetamide, N-methyl-2-pyrrolidone, acetonitrile, acetone, ethyl acetate, dichloromethane, or the like, or a mixed solvent thereof, and performing the reaction at -78°C to a reflux temperature under heating. An acid such as trifluoroacetic acid, acetic acid, or a boron trifluoride-diethyl ether complex, or a catalyst such as palladium carbon, rhodium carbon, platinum carbon, palladium hydroxide, or platinum oxide can be added, if necessary.

### [Synthetic route 21]

When the compound (7) mentioned above is represented by a compound (7f), this compound can be produced in accordance with, for example, a method mentioned in detail in the following synthetic route 21 or a method equivalent thereto, or methods described in other literatures or methods equivalent thereto. 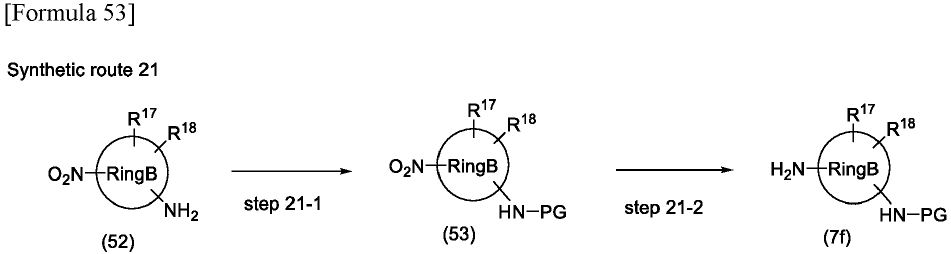 wherein PG, RingB, R¹⁷ and R¹⁸ are as defined above.

### Step 21-1

A compound (53) can be produced by protecting the amino group of a compound (52).

When PG is a tert-butoxycarbonyl group, reaction conditions can involve adding di-tert-butyl dicarbonate in dichloromethane, 1,4-dioxane, tetrahydrofuran, toluene, ethyl acetate, water, or the like, or a mixed solvent thereof, and performing the reaction at -78°C to room temperature, or optionally by heating to reflux. A base such as triethylamine, N,N-diisopropylethylamine, potassium carbonate, or sodium carbonate can be added, if necessary. A reaction accelerator such as pyridine or N,N-dimethyl-4-aminopyridine can be added, if necessary.

### Step 21-2

The compound (7f) can be produced by reducing the nitro group of the compound (53).

Reaction conditions can involve performing the reaction in the same manner as in step 6-1.

### [Synthetic route 22]

When the compound (7) mentioned above is represented by a compound (7g), this compound can be produced in accordance with, for example, a method mentioned in detail in the following synthetic route 22 or a method equivalent thereto, or methods described in other literatures or methods equivalent thereto. 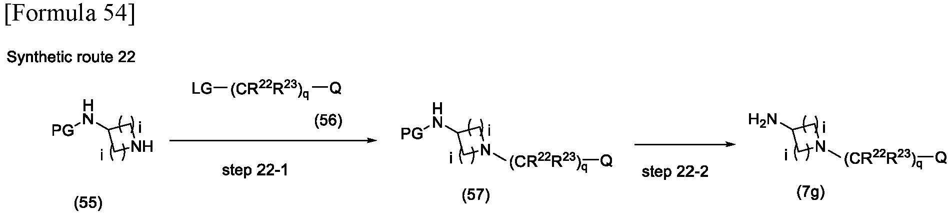 wherein each i independently represents an integer of 0 to 3, and PG, LG, R²², R²³, q and Q are as defined above.

### Step 22-1

A compound (57) can be produced by reacting a compound (55) with a compound (56).

Reaction conditions can involve performing the reaction in the same manner as in step 4-2.

### Step 22-2

The compound (7g) can be produced by removing the protective group from the compound (57).

When PG is a tert-butoxycarbonyl group, reaction conditions can involve performing the reaction in the same manner as in step 3-2.

### [Synthetic route 23]

When the compound (7) mentioned above is represented by a compound (7h), this compound can be produced in accordance with, for example, a method mentioned in detail in the following synthetic route 23 or a method equivalent thereto, or methods described in other literatures or methods equivalent thereto. 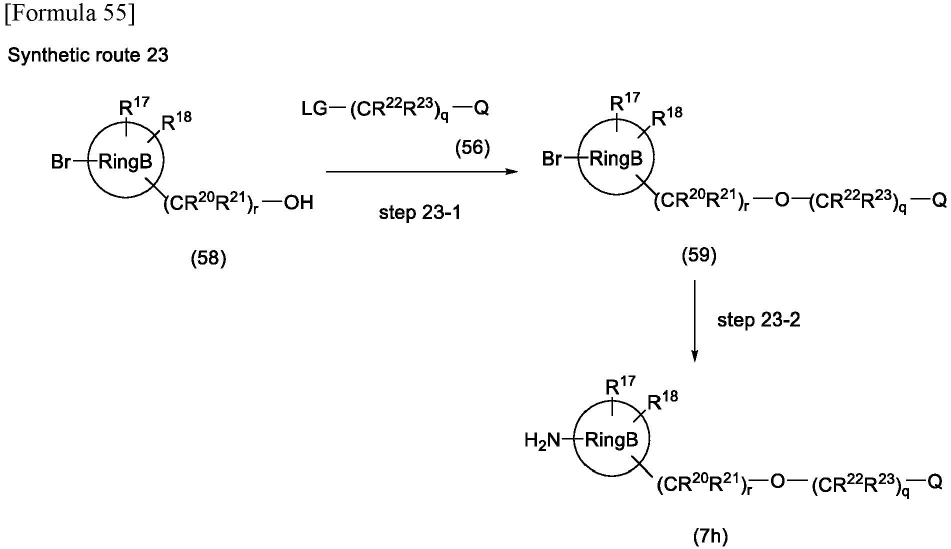 wherein LG, R¹⁷, R¹⁸, R²⁰, R²¹, R²², R²³, RingB, r, q and Q are as defined above.

### Step 23-1

A compound (59) can be produced by reacting a compound (58) with a compound (56).

Reaction conditions can involve adding the compound (56) or a salt thereof, etc. in dichloromethane, 1,2-dichloroethane, benzene, toluene, tetrahydrofuran, N,N-dimethylformamide, 1,4-dioxane, acetonitrile, or the like, or a mixed solvent thereof, and performing the reaction at -78°C to a reflux temperature under heating. A base such as sodium hydride, potassium carbonate, sodium carbonate, cesium carbonate, triethylamine, N,N-diisopropylethylamine, pyridine, 1,8-diazabicyclo[5.4.0]undec-7-ene (DBU), or 1,5-diazabicyclo[4.3.0]non-5-ene (DBN) can be added, if necessary.

### Step 23-2

The compound (7h) can be produced by aminating the compound (59).

Reaction conditions can involve adding an aminating agent such as benzophenone imine in 1,2-dimethoxyethane, 1,4-dioxane, toluene, N,N-dimethylformamide, N,N-dimethylacetamide, dimethyl sulfoxide, acetonitrile, or the like, or a mixed solvent thereof, adding a base such as potassium carbonate, tripotassium phosphate, sodium carbonate, cesium carbonate, potassium acetate, or triethylamine, adding a palladium catalyst such as tris(dibenzylideneacetone)dipalladium(0) (Pd₂(dba)₃), palladium acetate (Pd(OAc)₂), bis(triphenylphosphine)palladium(II) dichloride (PdCl₂(Ph₃P)₂), [1,1'-bis(diphenylphosphino)ferrocene]palladium(II) dichloride (PdCl₂(dppf)), tetrakis(triphenylphosphine)palladium (Pd(Ph₃P)₄), or (2-dicyclohexylphosphino-2',4',6'-triisopropylbiphenyl)aminobiphenylpalladium chloride (XPhos Pd G3), and, if necessary, a ligand such as 2-di-tert-butylphosphino-2',4',6'-triisopropylbiphenyl (tert-BuXPhos), 2-dicyclohexylphosphino-2',6'-dimethoxybiphenyl (SPhos), 2,2'-bis(diphenylphosphino)-1,1'-binaphthyl (BINAP), or 4,5-bis(diphenylphosphino)-9,9-dimethylxanthene (XantPhos), performing the reaction at room temperature to a reflux temperature under heating, then adding an acidic aqueous solution such as hydrochloric acid, and performing the reaction at - 78°C to a reflux temperature under heating.

### [Synthetic route 24]

When the compound (7) mentioned above is represented by a compound (7i), this compound can be produced in accordance with, for example, a method mentioned in detail in the following synthetic route 24 or a method equivalent thereto, or methods described in other literatures or methods equivalent thereto. 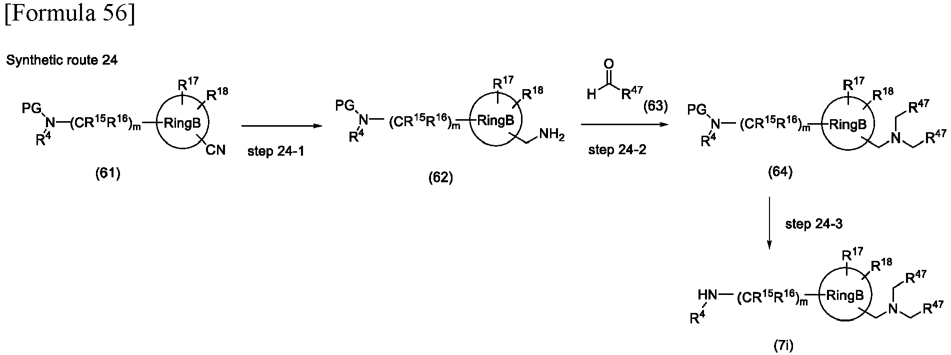 wherein R⁴⁷ represents a hydrogen atom or a C₁ to C₆ alkyl group, and PG, R⁴, R¹⁵, R¹⁶, R¹⁷, R¹⁸, RingB and m are as defined above.

### Step 24-1

A compound (62) can be produced by reducing the cyano group of a compound (61).

General cyano group reduction conditions can be used as reaction conditions. For example, the reaction can be performed at 0°C to room temperature, or optionally by heating to reflux, by adding a reducing agent such as Raney nickel, formic acid, sodium borohydride, nickel borohydride, cobalt borohydride, or lithium aluminum hydride in methanol, ethanol, isopropyl alcohol, 1,4-dioxane, 1,2-dimethoxyethane, acetic acid, water, tetrahydrofuran, diethyl ether, tert-butyl methyl ether, N,N-dimethylformamide, toluene, n-hexane, or the like, or a mixed solvent thereof. Alternatively, the reduction may be performed by adding a catalyst such as palladium carbon, rhodium carbon, platinum carbon, or platinum oxide in a hydrogen atmosphere in methanol, ethanol, isopropyl alcohol, 1,4-dioxane, 1,2-dimethoxyethane, acetic acid, ethyl acetate, water, tetrahydrofuran, tert-butyl methyl ether, N,N-dimethylformamide, toluene, or the like, or a mixed solvent thereof.

### Step 24-2

A compound (64) can be produced by subjecting a compound (62) to reductive alkylation using a compound (63).

Reaction conditions can involve adding aldehyde represented by the compound (63) or a compound equivalent thereto, for example, paraformaldehyde, an aqueous formalin solution, or a glycolaldehyde dimer, in dichloromethane, 1,2-dichloroethane, chloroform, 1,4-dioxane, tetrahydrofuran, toluene, benzene, methanol, ethanol, or the like, or a mixed solvent thereof, adding a reducing agent such as sodium triacetoxyborohydride, sodium cyanoborohydride, sodium borohydride, lithium borohydride, a borane-dimethyl sulfide complex, lithium aluminum hydride, or 2-picoline borane, and performing the reaction at -78°C to room temperature, or optionally by heating to reflux. Alternatively, the reduction may be performed by adding a catalyst such as palladium carbon, rhodium carbon, platinum carbon, or platinum oxide in a hydrogen atmosphere in methanol, ethanol, isopropyl alcohol, 1,4-dioxane, 1,2-dimethoxyethane, ethyl acetate, water, tetrahydrofuran, tert-butyl methyl ether, N,N-dimethylformamide, toluene, or the like, or a mixed solvent thereof. A reaction accelerator such as acetic acid, trifluoroacetic acid, p-toluenesulfonic acid, a boron trifluoride-diethyl ether complex, boron tribromide, aluminum chloride, chlorotrimethylsilane, 2,2,2-trifluoroethanol, or tetraisopropyl o-titanate can be added, if necessary.

### Step 24-3

The compound (7i) can be produced by removing the protective group from the compound (64).

When PG is a tert-butoxycarbonyl group, reaction conditions can involve performing the reaction in the same manner as in step 3-2.

### [Synthetic route 25]

The compound (4a) mentioned above can be produced in accordance with, for example, a method mentioned in detail in the following synthetic route 25 or a method equivalent thereto, or methods described in other literatures or methods equivalent thereto. 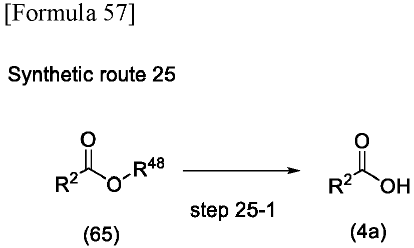 wherein R⁴⁸ represents a C₁ to C₆ alkyl group, and R² is as defined above.

### Step 25-1

The compound (4a) can be produced by hydrolyzing a compound (65).

Reaction conditions can involve performing the reaction in the same manner as in step 1-2.

### [Synthetic route 26]

When the compound (65) mentioned above is represented by a compound (65a) or a compound (65b), this compound can be produced in accordance with, for example, a method mentioned in detail in the following synthetic route 26 or a method equivalent thereto, or methods described in other literatures or methods equivalent thereto. 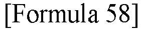 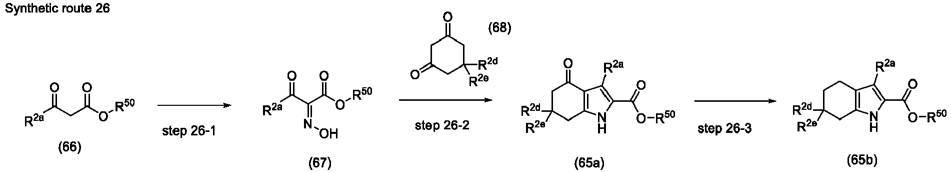 wherein R⁵⁰ represents a C₁ to C₆ alkyl group, and R^{2a}, R^{2d} and R^{2e} are as defined above.

### Step 26-1

A compound (67) can be produced by oximating a compound (66).

Reaction conditions can involve adding sodium nitrite, isoamyl nitrite, or the like in water, tetrahydrofuran, diethyl ether, dichloromethane, ethyl acetate, or the like, or a mixed solvent thereof, and performing the reaction at -78°C to room temperature, or optionally by heating to reflux. An acid such as acetic acid or hydrogen chloride can be added, if necessary.

### Step 26-2

The compound (65a) can be produced by reacting the compound (67) with a compound (68).

Reaction conditions can involve adding a zinc powder or the like in a solvent such as acetic acid, and performing the reaction at 0°C to a reflux temperature under heating. Sodium acetate or the like can be added, if necessary.

### Step 26-3

The compound (65b) can be produced by reducing the carbonyl group of the compound (65a).

Reaction conditions can involve adding a reducing agent such as sodium borohydride in 1,4-dioxane, tetrahydrofuran, diethyl ether, or the like, or a mixed solvent thereof, and performing the reaction at -78°C to room temperature, or optionally by heating to reflux. A reaction accelerator such as a boron trifluoride-diethyl ether complex can be added, if necessary.

### [Synthetic route 27]

When the compound (65) mentioned above is represented by a compound (65c), this compound can be produced in accordance with, for example, a method mentioned in detail in the following synthetic route 27 or a method equivalent thereto, or methods described in other literatures or methods equivalent thereto. 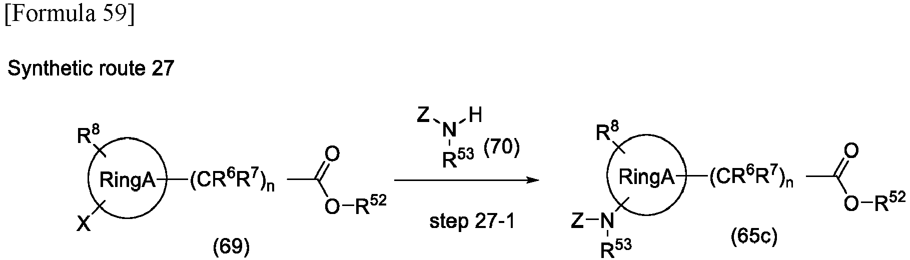 wherein R⁵² represents a C₁ to C₆ alkyl group, R⁵³ represents a hydrogen atom, a C₁ to C₆ alkyl group, or a C₂ to C₆ alkenyl group, R⁵³ and Z are optionally bonded to each other to form a ring, and X, R⁶, R⁷, R⁸, RingA, n and Z are as defined above.

### Step 27-1

The compound (65c) can be produced by reacting a compound (69) with a compound (70).

Reaction conditions can involve adding the compound (70) in tetrahydrofuran, 1,2-dimethoxyethane, 1,4-dioxane, toluene, N,N-dimethylformamide, N,N-dimethylacetamide, dimethyl sulfoxide, acetonitrile, ethanol, or the like, or a mixed solvent thereof, and performing the reaction at room temperature to a reflux temperature under heating. A base such as potassium carbonate, tripotassium phosphate, sodium carbonate, cesium carbonate, potassium acetate, or triethylamine, a palladium catalyst such as tris(dibenzylideneacetone)dipalladium(O) (Pd₂(dba)₃), palladium acetate (Pd(OAc)₂), bis(triphenylphosphine)palladium(II) dichloride (PdCl₂(Ph₃P)₂), [1,1'-bis(diphenylphosphino)ferrocene]palladium(II) dichloride (PdCl₂(dppf)), tetrakis(triphenylphosphine)palladium (Pd(Ph₃P)₄), or (2-dicyclohexylphosphino-2',4',6'-triisopropylbiphenyl)aminobiphenylpalladium chloride (XPhos Pd G3), or a ligand such as 2-di-tert-butylphosphino-2',4',6'-triisopropylbiphenyl (tert-BuXPhos), 2-dicyclohexylphosphino-2',6'-dimethoxybiphenyl (SPhos), 2,2'-bis(diphenylphosphino)-1,1'-binaphthyl (BINAP), or 4,5-bis(diphenylphosphino)-9,9-dimethylxanthene (XantPhos) can be added, if necessary.

### [Synthetic route 28]

When the compound (65) mentioned above is represented by a compound (65d), this compound can be produced in accordance with, for example, a method mentioned in detail in the following synthetic route 28 or a method equivalent thereto, or methods described in other literatures or methods equivalent thereto. 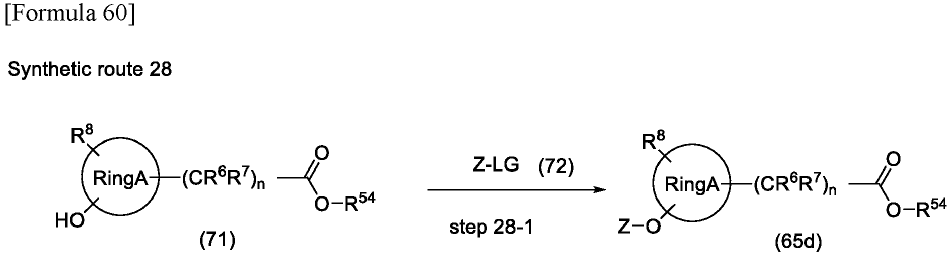 wherein R⁵⁴ represents a C₁ to C₆ alkyl group, and LG, R⁶, R⁷, R⁸, RingA, n, and Z are as defined above.

### Step 28-1

The compound (65d) can be produced by reacting a compound (71) with a compound (72).

Reaction conditions can involve performing the reaction in the same manner as in step 23-1.

### [Synthetic route 29]

When the compound (65) mentioned above is represented by a compound (65e), this compound can be produced in accordance with, for example, a method mentioned in detail in the following synthetic route 29 or a method equivalent thereto, or methods described in other literatures or methods equivalent thereto. 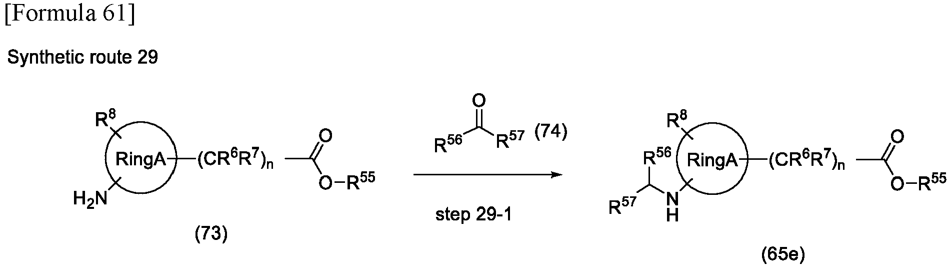 wherein R⁵⁵ represents a C₁ to C₆ alkyl group, R⁵⁶ and R⁵⁷ each independently represent a hydrogen atom or a C₁ to C₆ alkyl group (the C₁ to C₆ alkyl group is optionally substituted with one or more C₁ to C₆ alkylamino groups), R⁵⁶ and R⁵⁷ are optionally bonded to each other to form a ring, and R⁶, R⁷, R⁸, RingA and n are as defined above.

### Step 29-1

The compound (65e) can be produced by subjecting a compound (73) to reductive alkylation using a compound (74).

Reaction conditions can involve performing the reaction in the same manner as in step 7-1.

### [Synthetic route 30]

When the compound (65) mentioned above is represented by a compound (65f), this compound can be produced in accordance with, for example, a method mentioned in detail in the following synthetic route 30 or a method equivalent thereto, or methods described in other literatures or methods equivalent thereto. 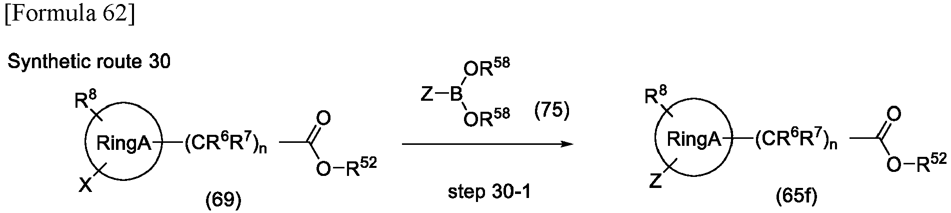 wherein R⁵⁸ represents a hydrogen atom or a C₁ to C₆ alkyl group, R⁵⁸ is optionally bonded to another R⁵⁸ to form a ring, and X, R⁶, R⁷, R⁸, R⁵², RingA, n and Z are as defined above.

### Step 30-1

The compound (65f) can be produced by subjecting a compound (69) and a compound (75) to coupling reaction.

General Suzuki-Miyaura coupling reaction conditions can be applied to reaction conditions. For example, the reaction can be performed at 0°C to a reflux temperature under heating by adding the compound (75) in dimethyl sulfoxide, N,N-dimethylformamide, 1,4-dioxane, toluene, tetrahydrofuran, 1,2-dimethoxyethane, methanol, ethanol, water, or the like, or a mixed solvent thereof, adding a base such as potassium carbonate, sodium carbonate, cesium carbonate, sodium hydroxide, potassium hydroxide, tripotassium phosphate, cesium fluoride, triethylamine, or N,N-diisopropylethylamine, and using a palladium catalyst such as bis(triphenylphosphine)palladium(II) dichloride (PdCl₂(PPh₃)₂), a [1,1'-bis(diphenylphosphino)ferrocene]palladium (II) dichloride-dichloromethane complex (PdCl₂(dppf) CH₂Cl₂), [1,1'-bis(diphenylphosphino)ferrocene]palladium(II) dichloride (PdCl₂(dppf)), tetrakis(triphenylphosphine)palladium (Pd(Ph₃P)₄), palladium acetate (Pd(OAc)₂), tris(dibenzylideneacetone)dipalladium (Pd₂(dba)₃), or 2-dicyclohexylphosphino-2',4',6'-triisopropylbiphenyl)aminobiphenylpalladium chloride (XPhos Pd G3). A ligand such as 2-di-tert-butylphosphino-2',4',6'-triisopropylbiphenyl (tert-BuXPhos) or 2-dicyclohexylphosphino-2',6'-dimethoxybiphenyl (SPhos) can be added, if necessary.

### [Synthetic route 31]

When the compound (65) mentioned above is represented by a compound (65g) or a compound (65h), this compound can be produced in accordance with, for example, a method mentioned in detail in the following synthetic route 31 or a method equivalent thereto, or methods described in other literatures or methods equivalent thereto. 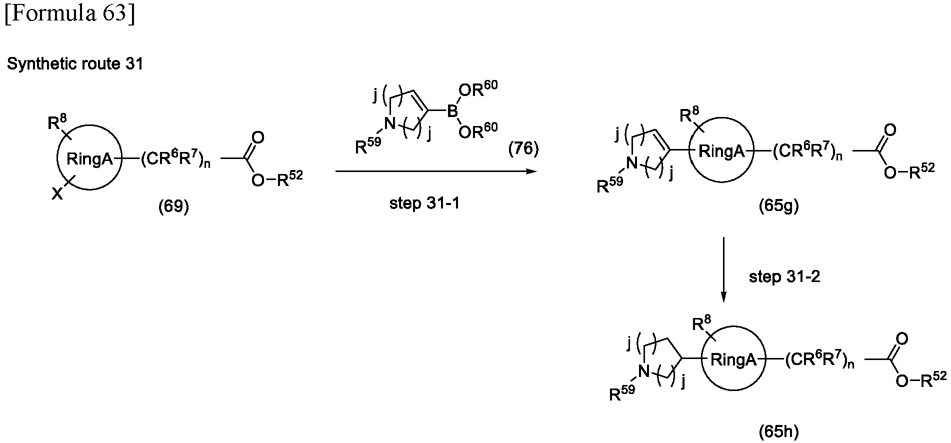 wherein each j independently represents an integer of 0 to 3, R⁵⁹ represents a protective group such as a tert-butoxycarbonyl group, a benzyloxycarbonyl group, a benzyl group, an acetyl group, a benzoyl group or a 9-fluorenylmethyloxycarbonyl group, or a C₁ to C₆ alkyl group, R⁶⁰ represents a hydrogen atom or a C₁ to C₆ alkyl group, R⁶⁰ is optionally bonded to another R⁶⁰ to form a ring, and X, R⁶, R⁷, R⁸, R⁵², RingA and n are as defined above.

### Step 31-1

The compound (65g) can be produced by subjecting a compound (69) and a compound (76) to coupling reaction.

Reaction conditions can involve performing the reaction in the same manner as in step 30-1.

### Step 31-2

The compound (65h) can be produced by reducing the unsaturated bond of the compound (65g).

Reaction conditions can involve adding a catalyst such as palladium carbon, rhodium carbon, platinum carbon, or platinum oxide in a hydrogen atmosphere in methanol, ethanol, isopropyl alcohol, 1,4-dioxane, 1,2-dimethoxyethane, ethyl acetate, water, tetrahydrofuran, tert-butyl methyl ether, N,N-dimethylformamide, toluene, or the like, or a mixed solvent thereof, and performing the reaction at 0°C to a reflux temperature under heating. Acetic acid, trifluoroacetic acid, 2,2,2-trifluoroethanol, or the like can be added as a reaction accelerator, if necessary.

### [Synthetic route 32]

When the compound (65) mentioned above is represented by a compound (65i), this compound can be produced in accordance with, for example, a method mentioned in detail in the following synthetic route 32 or a method equivalent thereto, or methods described in other literatures or methods equivalent thereto. 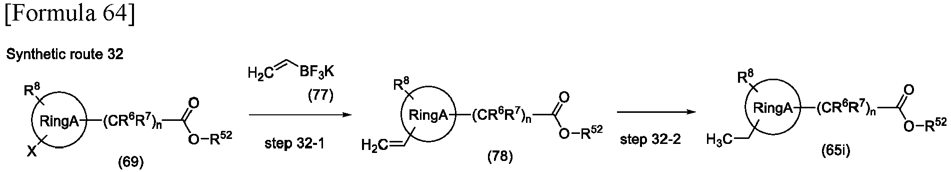 wherein X, R⁶, R⁷, R⁸, R⁵², RingA and n are as defined above.

### Step 32-1

A compound (78) can be produced by subjecting a compound (69) and a compound (77) to coupling reaction.

Reaction conditions can involve performing the reaction in the same manner as in step 30-1.

### Step 32-2

The compound (65i) can be produced by reducing the unsaturated bond of the compound (78).

Reaction conditions can involve performing the reaction in the same manner as in step 31-2.

### [Synthetic route 33]

When the compound (65) mentioned above is represented by a compound (65j), this compound can be produced in accordance with, for example, a method mentioned in detail in the following synthetic route 33 or a method equivalent thereto, or methods described in other literatures or methods equivalent thereto. 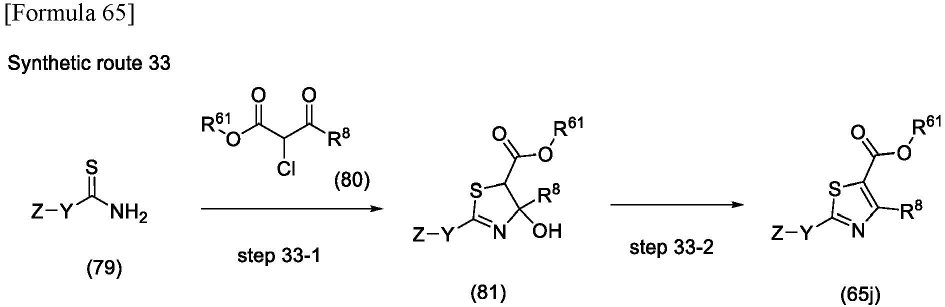 wherein R⁶¹ represents a C₁ to C₆ alkyl group, and R⁸, Y, and Z are as defined above.

### Step 33-1

A compound (81) can be produced by reacting a compound (79) with a compound (80).

Reaction conditions can involve adding the compound (80) in methanol, ethanol, 1-propanol, isopropyl alcohol, tetrahydrofuran, 1,4-dioxane, toluene, N,N-dimethylformamide, or the like, or a mixed solvent thereof, and performing the reaction at room temperature to a reflux temperature under heating.

### Step 33-2

The compound (65j) can be produced by dehydrating the compound (81).

Reaction conditions can involve adding an acid such as p-toluenesulfonic acid or camphor sulfonic acid in benzene, toluene, xylene, or the like, or a mixed solvent thereof, and performing the reaction at room temperature to a reflux temperature under heating.

### [Synthetic route 34]

When the compound (3) mentioned above is represented by a compound (3a) or when the compound (9) mentioned above is represented by a compound (9a), this compound can be produced in accordance with, for example, a method mentioned in detail in the following synthetic route 34 or a method equivalent thereto, or methods described in other literatures or methods equivalent thereto. 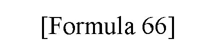 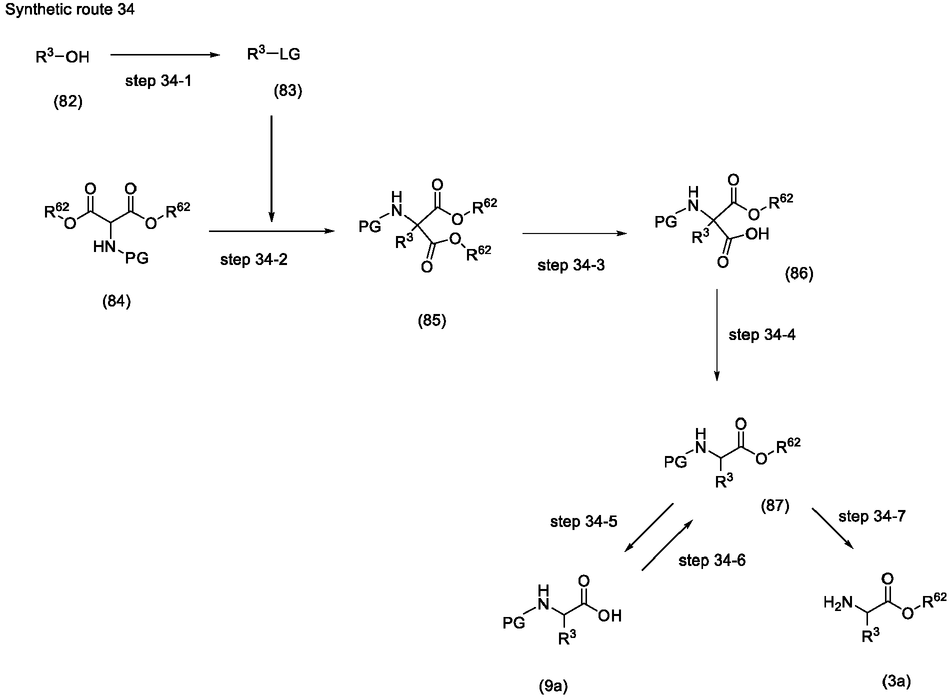 wherein R⁶² represents a C₁ to C₆ alkyl group, and LG, PG and R³ are as defined above.

### Step 34-1

A compound (83) can be produced by converting the hydroxy group of a compound (82) to an appropriate leaving group (LG).

Reaction conditions can involve performing the reaction in the same manner as in step 4-1.

### Step 34-2

A compound (85) can be produced by reacting a compound (84) with a compound (83).

Reaction conditions can involve adding the compound (83) in N,N-dimethylformamide, N,N-dimethylacetamide, tetrahydrofuran, 1,4-dioxane, or the like, or a mixed solvent thereof, and performing the reaction at 0°C to a reflux temperature under heating. A base such as sodium hydride, potassium tert-butoxide, 1,8-diazabicyclo[5.4.0]undec-7-ene (DBU), or 1,5-diazabicyclo[4.3.0]non-5-ene (DBN) can be added, if necessary.

### Step 34-3

A compound (86) can be produced by hydrolyzing the compound (85).

Reaction conditions can involve performing the reaction in the same manner as in step 1-2.

### Step 34-4

A compound (87) can be produced by decarbonating the compound (86).

Reaction conditions can involve performing the reaction at room temperature to a reflux temperature under heating in benzene, toluene, xylene, 1,2-dichlorobenzene, dimethyl sulfoxide, or the like, or a mixed solvent thereof. Hydrogen chloride, p-toluenesulfonic acid, or the like can be added as a reaction accelerator, if necessary.

### Step 34-5

The compound (9a) can be produced by hydrolyzing the compound (87).

Reaction conditions can involve performing the reaction in the same manner as in step 1-2.

### Step 34-6

A compound (87) can be produced by esterifying the compound (9a).

Reaction conditions can involve adding trimethylsilyldiazomethane or the like in methanol, diethyl ether, tetrahydrofuran, n-hexane, benzene, toluene, or the like, or a mixed solvent thereof, and performing the reaction at 0°C to room temperature.

### Step 34-7

The compound (3a) can be produced by removing the protective group from the compound (87).

When PG is a tert-butoxycarbonyl group, reaction conditions can involve performing the reaction in the same manner as in step 3-2.

### [Synthetic route 35]

When the compound (3) mentioned above is represented by a compound (3b) or when the compound (9) mentioned above is represented by a compound (9b), this compound can also be produced in accordance with, for example, a method mentioned in detail in the following synthetic route 35 or a method equivalent thereto, or methods described in other literatures or methods equivalent thereto. 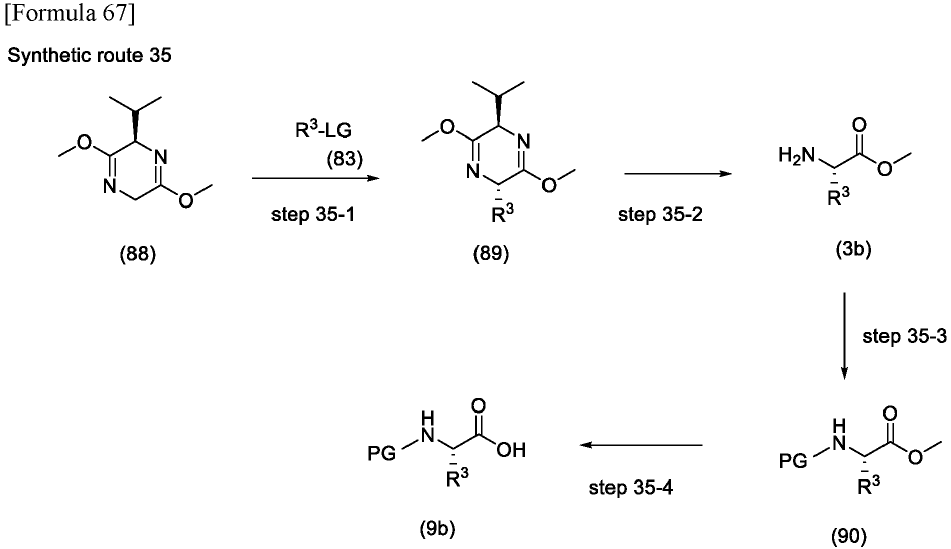 wherein LG, PG and R³ are as defined above.

### Step 35-1

A compound (89) can be produced by reacting a compound (88) with a compound (83).

Reaction conditions can involve adding the compound (83) in tetrahydrofuran, n-hexane, or the like, or a mixed solvent thereof, adding a base such as n-butyllithium, and performing the reaction at -78°C to room temperature.

### Step 35-2

The compound (3b) can be produced by hydrolyzing the compound (89).

Reaction conditions can involve adding hydrogen chloride or the like in water, tetrahydrofuran, 1,4-dioxane, or the like, or an aqueous mixed solvent thereof, and performing the reaction at 0°C to a reflux temperature under heating.

### Step 35-3

A compound (90) can be produced by protecting the amino group of a compound (3b).

When PG is a tert-butoxycarbonyl group, reaction conditions can involve performing the reaction in the same manner as in step 21-1.

### Step 35-4

The compound (9b) can be produced by hydrolyzing the compound (90).

Reaction conditions can involve performing the reaction in the same manner as in step 1-2.

### [Synthetic route 36]

When the compound (10) mentioned above is represented by a compound (10a), this compound can be produced in accordance with, for example, a method mentioned in detail in the following synthetic route 36 or a method equivalent thereto, or methods described in other literatures or methods equivalent thereto. 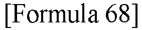 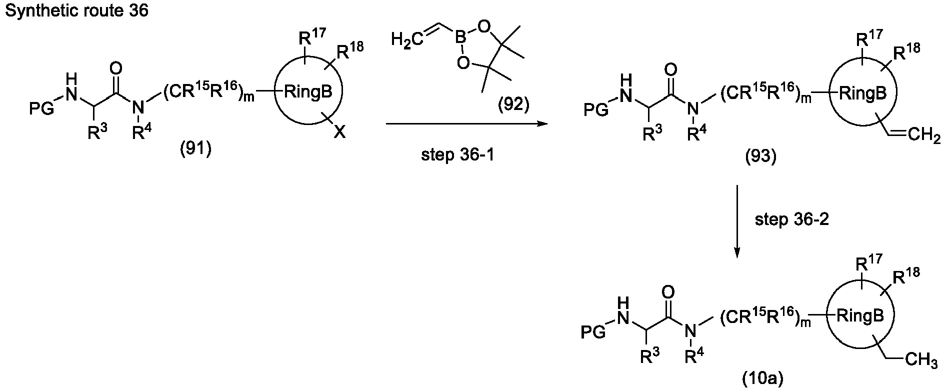 wherein X, PG, R³, R⁴, R¹⁵, R¹⁶, R¹⁷, R¹⁸, RingB and m are as defined above.

### Step 36-1

A compound (93) can be produced by subjecting a compound (91) and a compound (92) to coupling reaction.

Reaction conditions can involve performing the reaction in the same manner as in step 30-1.

### Step 36-2

The compound (10a) can be produced by reducing the unsaturated bond of a compound (93).

Reaction conditions can involve performing the reaction in the same manner as in step 31-2.

### [Synthetic route 37]

When the compound (10) mentioned above is represented by a compound (10b), this compound can be produced in accordance with, for example, a method mentioned in detail in the following synthetic route 37 or a method equivalent thereto, or methods described in other literatures or methods equivalent thereto. 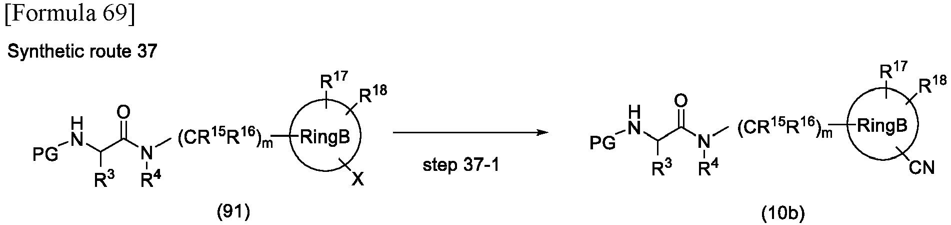 wherein X, PG, R³, R⁴, R¹⁵, R¹⁶, R¹⁷, R¹⁸, RingB and m are as defined above.

### Step 37-1

The compound (10b) can be produced by subjecting a compound (91) to cyanation reaction.

Reaction conditions can involve adding a cyanating agent such as zinc cyanide in dimethyl sulfoxide, N,N-dimethylformamide, 1,4-dioxane, toluene, tetrahydrofuran, 1,2-dimethoxyethane, water, or the like, or a mixed solvent thereof, and performing the reaction at 0°C to a reflux temperature under heating using a palladium catalyst such as bis(triphenylphosphine)palladium(II) dichloride (PdCh(PPh₃)₂), a [1,1'-bis(diphenylphosphino)ferrocene]palladium(II) dichloride-dichloromethane complex (PdCl₂(dppf) CH₂Cl₂), [1,1'-bis(diphenylphosphino)ferrocene]palladium(II) dichloride (PdCl₂(dppf)), tetrakis(triphenylphosphine)palladium (Pd(Ph₃P)₄), palladium acetate (Pd(OAc)₂), tris(dibenzylideneacetone)dipalladium (Pd₂(dba)₃), or 2-dicyclohexylphosphino-2',4',6'-triisopropylbiphenyl)aminobiphenylpalladium chloride (XPhos Pd G3). A ligand such as 2-di-tert-butylphosphino-2',4',6'-triisopropylbiphenyl (tert-BuXPhos) or 2-dicyclohexylphosphino-2',6'-dimethoxybiphenyl (SPhos) can be used, if necessary.

### [Synthetic route 38]

When the compound (10) mentioned above is represented by a compound (10c), this compound can be produced in accordance with, for example, a method mentioned in detail in the following synthetic route 38 or a method equivalent thereto, or methods described in other literatures or methods equivalent thereto. 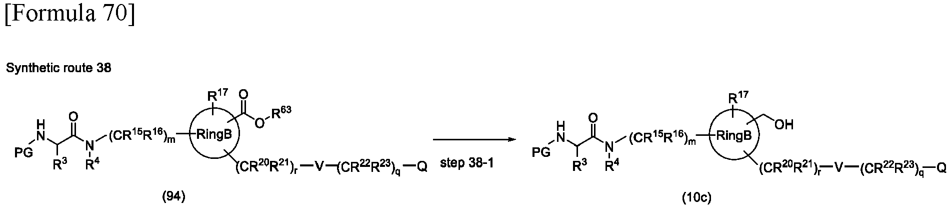 wherein R⁶³ represents a C₁ to C₆ alkyl group, and PG, R³, R⁴, R¹⁵, R¹⁶, R¹⁷, R²⁰, R²¹, R²², R²³, RingB, m, r, q, V and Q are as defined above.

### Step 38-1

The compound (10c) can be produced by reducing a compound (94).

Reaction conditions can involve performing the reaction at -78°C to a reflux temperature under heating using a hydride reducing agent such as diisobutyl aluminum hydride, lithium aluminum hydride, lithium borohydride, sodium borohydride, sodium bis(2-methoxyethoxy)aluminum hydride (Red-Al), or lithium tri(sec-butyl)borohydride in methanol, ethanol, tetrahydrofuran, diethyl ether, dichloromethane, toluene, benzene, n-hexane, or the like, or a mixed solvent thereof.

### [Synthetic route 39]

When the compound (I) mentioned above is represented by a compound wherein R¹ is a nitrogen atom, R⁵ is a phenyl group optionally substituted with R¹⁷, R¹⁸ and R¹⁹, and R¹ and R⁵ are bonded to each other to form a benzimidazole ring, i.e., a compound (98), this compound can be produced in accordance with, for example, a method mentioned in detail in the following synthetic route 39 or a method equivalent thereto, or methods described in other literatures or methods equivalent thereto. 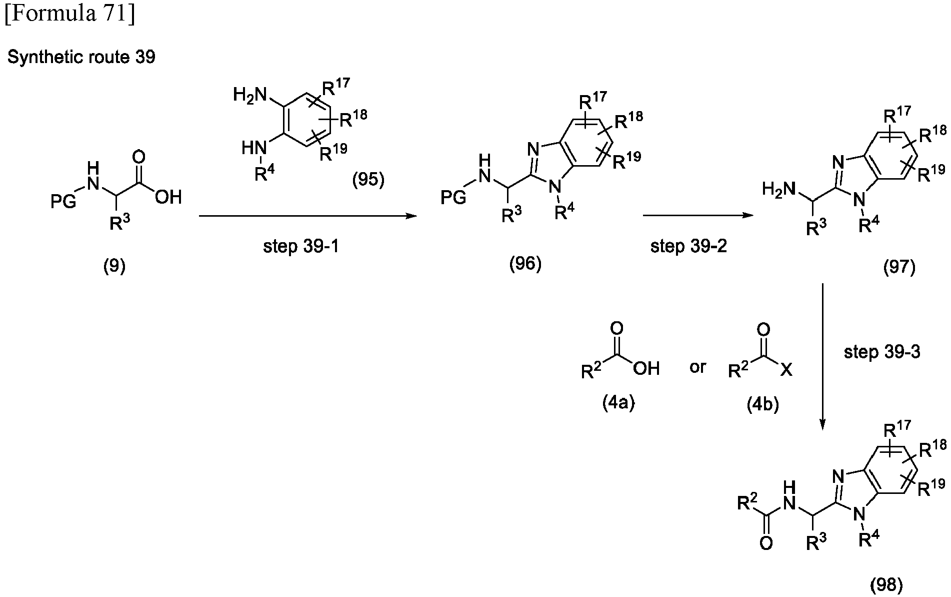 wherein PG, X, R², R³, R⁴, R¹⁷, R¹⁸ and R¹⁹ are as defined above.

### Step 39-1

A compound (96) can be produced by reacting a compound (9) with a compound (95).

Reaction conditions can involve adding the compound (95), performing dehydration condensation reaction in the same manner as in step 1-3, then adding acetic acid, hydrogen chloride, p-toluenesulfonic acid, or the like without a solvent or in water, 1,4-dioxane, toluene, xylene, ethanol, acetonitrile, or the like, or a mixed solvent thereof, and performing the reaction at 0°C to a reflux temperature under heating.

### Step 39-2

A compound (97) can be produced by removing the protective group from the compound (96).

When PG is a tert-butoxycarbonyl group, reaction conditions can involve performing the reaction in the same manner as in step 3-2.

### Step 39-3

A compound (98) can be produced by amidating the compound (97) using a compound (4a) or a compound (4b).

Reaction conditions can involve adding the compound (4a) or the compound (4b), etc., and performing the reaction in the same manner as in step 1-1.

### [Synthetic route 40]

When the compound (96) mentioned above is represented by a compound (96a), this compound can be produced in accordance with, for example, a method mentioned in detail in the following synthetic route 40 or a method equivalent thereto, or methods described in other literatures or methods equivalent thereto. 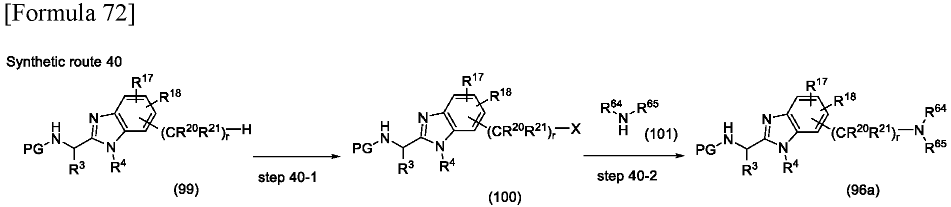 wherein R⁶⁴ and R⁶⁵ each independently represent a hydrogen atom, a C₁ to C₆ alkyl group or a 3- to 10-membered heterocycloalkyl group (the C₁ to C₆ alkyl group and the 3- to 10-membered heterocycloalkyl group are each optionally substituted with one or more C₁ to C₆ alkyl groups or C₁ to C₆ alkylamino groups), R⁶⁴ and R⁶⁵ are optionally bonded to each other to form a ring, and X, PG, R², R³, R⁴, R¹⁷, R¹⁸, R²⁰, R²¹ and r are as defined above.

### Step 40-1

A compound (100) can be produced by halogenating a compound (99).

Reaction conditions can involve adding a halogenating agent such as N-bromosuccinimide or bromine, and a radical initiator such as 2,2'-azobis(isobutyronitrile) or benzoyl peroxide without a solvent or in carbon tetrachloride, acetonitrile, or the like, or a mixed solvent thereof, and performing the reaction at room temperature to a reflux temperature under heating. Light irradiation may be performed, if necessary.

### Step 40-2

The compound (96a) can be produced by reacting a compound (100) with a compound (101).

Reaction conditions can involve performing the reaction in the same manner as in step 4-2.

### [Synthetic route 41]

When the compound (96) mentioned above is represented by a compound (96b), this compound can be produced in accordance with, for example, a method mentioned in detail in the following synthetic route 41 or a method equivalent thereto, or methods described in other literatures or methods equivalent thereto. 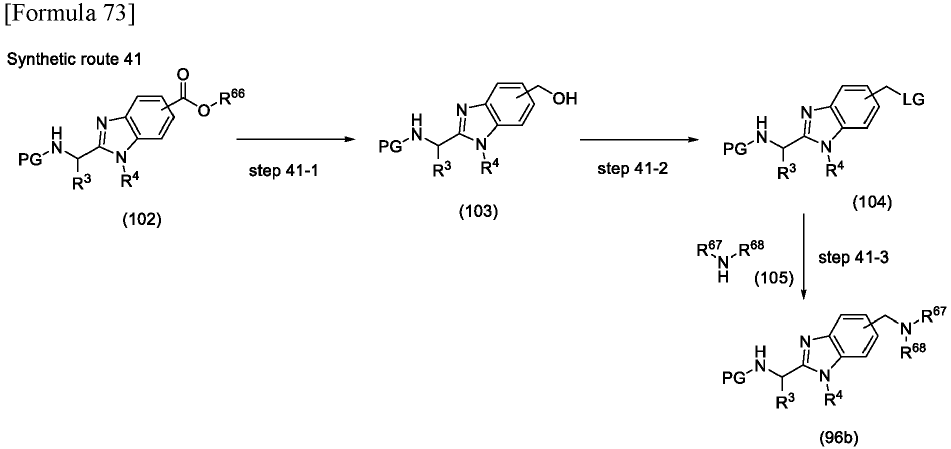 wherein R⁶⁶ represents a C₁ to C₆ alkyl group, R⁶⁷ and R⁶⁸ each independently represent a hydrogen atom or a C₁ to C₆ alkyl group, R⁶⁷ and R⁶⁸ are optionally bonded to each other to form a ring, and LG, PG, R³ and R⁴ are as defined above.

### Step 41-1

A compound (103) can be produced by reducing a compound (102).

Reaction conditions can involve performing the reaction in the same manner as in step 38-1.

### Step 41-2

A compound (104) can be produced by converting the hydroxy group of a compound (103) to an appropriate leaving group (LG).

Reaction conditions can involve performing the reaction in the same manner as in step 4-1.

### Step 41-3

The compound (96b) can be produced by reacting the compound (104) with a compound (105).

Reaction conditions can involve performing the reaction in the same manner as in step 4-2.

### [Synthetic route 42]

When the compound (98) mentioned above is represented by a compound (98a), this compound can be produced in accordance with, for example, a method mentioned in detail in the following synthetic route 42 or a method equivalent thereto, or methods described in other literatures or methods equivalent thereto. 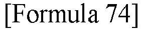 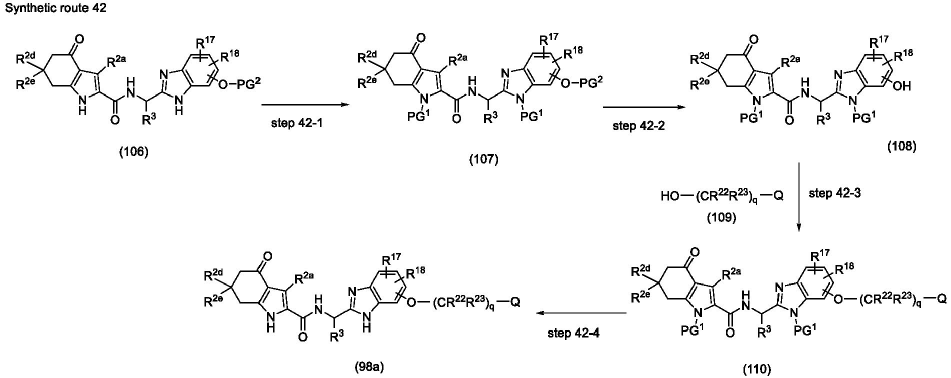 wherein PG¹ represents a protective group such as a tert-butoxycarbonyl group or a benzyloxycarbonyl group, PG² represents a protective group such as a benzyl group or a tert-butyldimethylsilyl group, and R^{2a}, R^{2d}, R^{2e}, R³, R¹⁷, R¹⁸, R²², R²³, Q and q are as defined above.

### Step 42-1

A compound (107) can be produced by adding a protective group (PG¹) onto the nitrogen atoms of the pyrrole ring and the benzimidazole ring of a compound (106).

When PG¹ is a tert-butoxycarbonyl group, reaction conditions can involve performing the reaction in the same manner as in step 21-1.

### Step 42-2

A compound (108) can be produced by removing the protective group (PG²) from the hydroxy group of the compound (107).

When PG² is a benzyl group, reaction conditions can involve performing the reaction in the same manner as in step 7-3.

### Step 42-3

A compound (110) can be produced through the Mitsunobu reaction of the compound (108) with a compound (109).

General Mitsunobu reaction conditions can be used as reaction conditions. For example, the reaction can be performed at room temperature to a reflux temperature under heating by adding the compound (109) without a solvent or in tetrahydrofuran, 1,4-dioxane, toluene, benzene, or the like, or a mixed solvent thereof, and adding a phosphorus reagent such as triphenylphosphine, tributylphosphine, or trimethylphosphine, and an azo compound such as diisopropyl azodicarboxylate (DIAD), diethyl azodicarboxylate (DEAD), or 1,1'-azobis(N,N-dimethylformamide) (TMAD).

### Step 42-4

The compound (98a) can be produced by removing the protective groups (PG¹) from the nitrogen atoms of the pyrrole ring and the benzimidazole ring of the compound (110).

When PG¹ is a tert-butoxycarbonyl group, reaction conditions can involve performing the reaction in the same manner as in step 3-2.

### [Synthetic route 43]

When the compound (98) mentioned above is represented by a compound (98b), this compound can be produced in accordance with, for example, a method mentioned in detail in the following synthetic route 43 or a method equivalent thereto, or methods described in other literatures or methods equivalent thereto. 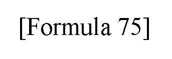 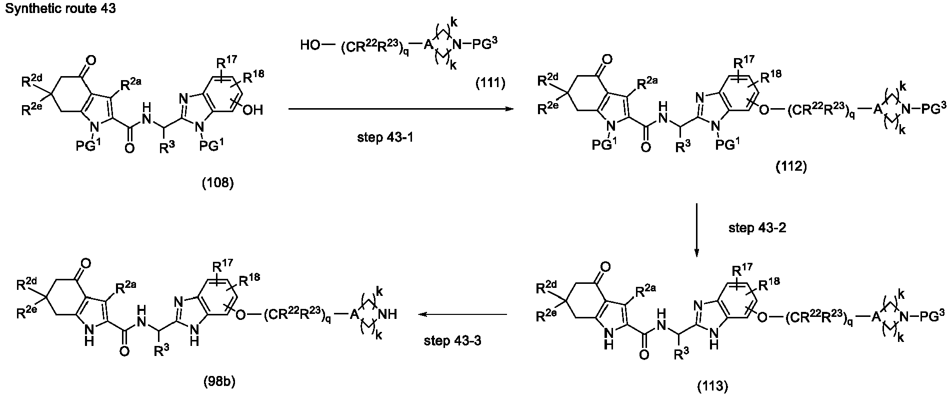 wherein PG³ represents a protective group such as a tert-butoxycarbonyl group or a benzyloxycarbonyl group, each k independently represents an integer of 0 to 3, and A, PG¹, R^{2a}, R^{2d}, R^{2e}, R³, R¹⁷, R¹⁸, R²², R²³, and q are as defined above.

### Step 43-1

A compound (112) can be produced through the Mitsunobu reaction of a compound (108) with a compound (111).

Reaction conditions can involve performing the reaction in the same manner as in step 42-3.

### Step 43-2

A compound (113) can be produced by removing the protective groups (PG¹) from the nitrogen atoms of the pyrrole ring and the benzimidazole ring of the compound (112).

When PG¹ is a tert-butoxycarbonyl group, reaction conditions can involve performing the reaction in the same manner as in step 3-2.

### Step 43-3

The compound (98b) can be produced by removing the protective group (PG³) from the amino group of the compound (113).

When PG³ is a benzyloxycarbonyl group, reaction conditions can involve performing the reaction in the same manner as in step 3-2.

### [Synthetic route 44]

When the compound (98) mentioned above is represented by a compound (98c), this compound can be produced in accordance with, for example, a method mentioned in detail in the following synthetic route 44 or a method equivalent thereto, or methods described in other literatures or methods equivalent thereto. 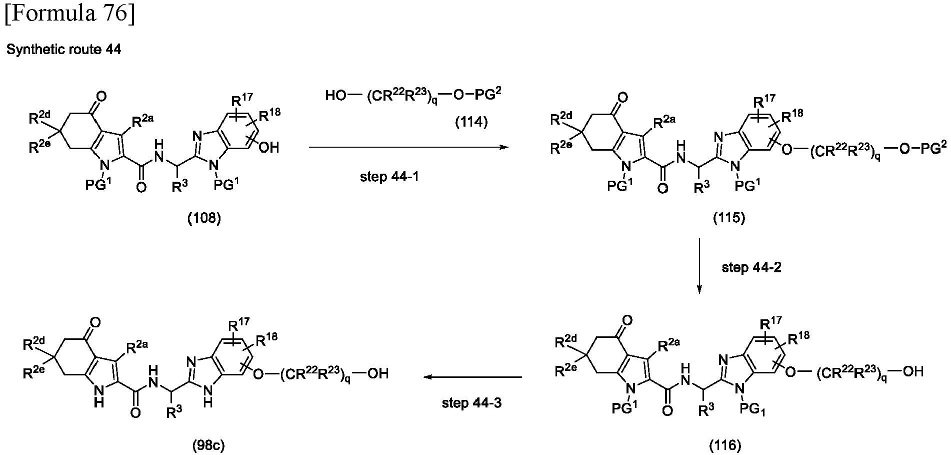 wherein PG¹, PG², R^{2a}, R^{2d}, R^{2e}, R³, R¹⁷, R¹⁸, R²², R²³ and q are as defined above.

### Step 44-1

A compound (115) can be produced through the Mitsunobu reaction of a compound (108) with a compound (114).

Reaction conditions can involve performing the reaction in the same manner as in step 42-3.

### Step 44-2

A compound (116) can be produced by removing the protective group (PG²) from the hydroxy group of the compound (115).

When PG² is a tert-butyldimethylsilyl group, reaction conditions can involve adding an acid such as tetra-n-butylammonium fluoride (TBAF), cesium fluoride, tris(dimethylamino)sulfonium difluorotrimethylsilicate (TASF), trifluoroacetic acid, p-toluenesulfonic acid, sulfuric acid, hydrogen chloride, hydrobromic acid, a boron trifluoride-diethyl ether complex, boron tribromide, or aluminum chloride in water, acetone, dichloromethane, chloroform, 1,4-dioxane, tetrahydrofuran, toluene, benzene, methanol, ethanol, or the like, or a mixed solvent thereof, and performing the reaction at -78°C to a reflux temperature under heating.

### Step 44-3

The compound (98c) can be produced by removing the protective groups (PG¹) from the nitrogen atoms of the pyrrole ring and the benzimidazole ring of the compound (116).

When PG¹ is a tert-butoxycarbonyl group, reaction conditions can involve performing the reaction in the same manner as in step 3-2.

### [Synthetic route 45]

When the compound (98) mentioned above is represented by a compound (98d), this compound can be produced in accordance with, for example, a method mentioned in detail in the following synthetic route 45 or a method equivalent thereto, or methods described in other literatures or methods equivalent thereto. 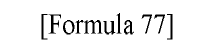 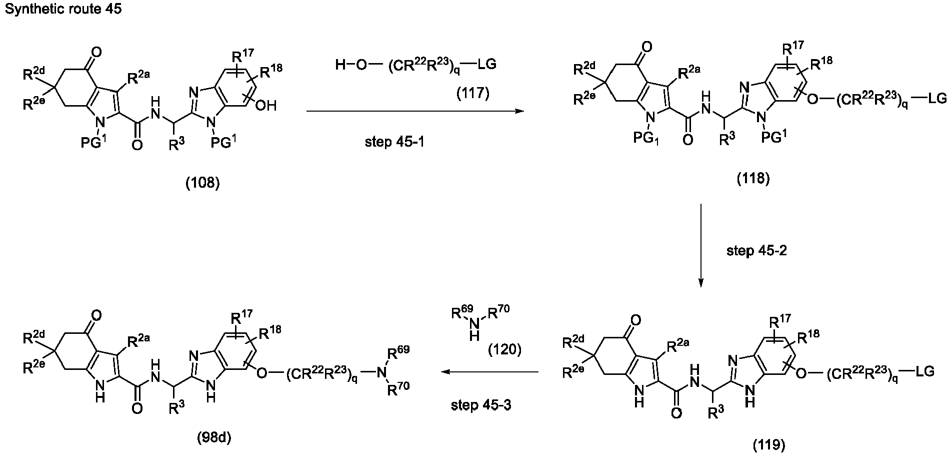 wherein R⁶⁹ and R⁷⁰ each independently represent a hydrogen atom, a C₁ to C₆ alkyl group or a hydroxy-Ci to C₆ alkyl group, R⁶⁹ and R⁷⁰ are optionally bonded to each other to form a ring, and PG¹, LG, R^{2a}, R^{2d}, R^{2e}, R³, R¹⁷, R¹⁸, R²², R²³ and q are as defined above.

### Step 45-1

A compound (118) can be produced through the Mitsunobu reaction of a compound (108) with a compound (117).

Reaction conditions can involve performing the reaction in the same manner as in step 42-3.

### Step 45-2

A compound (119) can be produced by removing the protective groups (PG¹) from the nitrogen atoms of the pyrrole ring and the benzimidazole ring of the compound (118).

When PG¹ is a tert-butoxycarbonyl group, reaction conditions can involve performing the reaction in the same manner as in step 3-2.

### Step 45-3

The compound (98d) can be produced by reacting the compound (119) with a compound (120).

Reaction conditions can involve performing the reaction in the same manner as in step 4-2 using the compound (120).

### [Synthetic route 46]

When the compound (I) mentioned above is represented by a compound wherein R¹ is a hydrogen atom, i.e., a compound (122), this compound can be produced in accordance with, for example, a method mentioned in detail in the following synthetic route 46 or a method equivalent thereto, or methods described in other literatures or methods equivalent thereto. 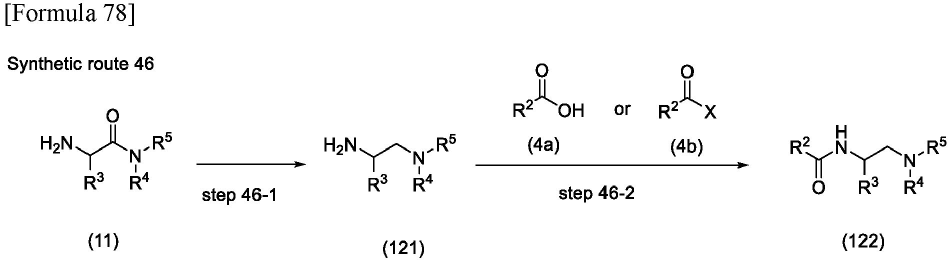 wherein R², R³, R⁴, R⁵ and X are as defined above.

### Step 46-1

A compound (121) can be produced by reducing the carbonyl group of a compound (11).

Reaction conditions can involve performing the reaction at 0°C to a reflux temperature under heating using a hydride reducing agent such as a borane-tetrahydrofuran complex, a borane-dimethyl sulfide complex, or lithium aluminum hydride in tetrahydrofuran, diethyl ether, methanol, ethanol, dichloromethane, toluene, benzene, n-hexane, or the like, or a mixed solvent thereof.

### Step 46-2

A compound (122) can be produced by amidating the compound (121) using a compound (4a) or a compound (4b).

Reaction conditions can involve adding the compound (4a) or the compound (4b), etc., and performing the reaction in the same manner as in step 1-1.

The synthetic routes shown above illustrate methods for producing the compound (I) of the present embodiment. The compound (I) of the present embodiment can be produced in accordance with the methods shown above or methods equivalent thereto, or methods described in other literatures or methods equivalent thereto. These production methods may be variously modified into schemes as easily understandable by those skilled in the art.

Depending on the type of a functional group, the operations of introduction and elimination can be appropriately carried out in combination in accordance with routine methods if a protective group is necessary. Examples of the type, introduction, and elimination of the protective group can include methods described in Theodora W. Greene & Peter G. M. Wuts, "Greene's Protective Groups in Organic Synthesis", fourth edition, Wiley-Interscience, 2006.

Intermediates that are used for producing the compound (I) of the present embodiment can be isolated or purified, if necessary, by an isolation or purification approach well known to those skilled in the art, such as solvent extraction, crystallization, recrystallization, chromatography, or preparative high-performance liquid chromatography.

Alternatively, the intermediates may be used directly as crude products in next reaction, if necessary, without being isolated or purified.

The "G9a inhibitory effect" described in the present embodiment is an effect of inhibiting G9a which is a major enzyme involved in the mono- and dimethylation of a lysine residue at position 9 of histone H3 (H3K9me1 and H3K9me2).

The compound (I) of the present embodiment or the pharmacologically acceptable salt thereof exhibits, for example, strong inhibitory activity in a G9a inhibitory activity test.

As a result, the compound (I) of the present embodiment or the pharmacologically acceptable salt thereof can be understood as being useful as a therapeutic drug for proliferative disease such as cancer, β-globin abnormality, fibrosis, pain, neurodegenerative disease, Prader-Willi syndrome, malaria, viral infection, myopathy, autism, and the like, or a prophylactic drug therefor.

A medicament containing the compound (I) of the present embodiment as an active ingredient can be in various dosage forms according to a usage. Examples of such a dosage form can include powders, granules, fine granules, dry syrups, tablets, capsules, injections, solutions, ointments, suppositories, patches, and sublingual formulations.

These medicaments can be configured as pharmaceutical compositions comprising the compound (I) of the present embodiment as an active ingredient and a pharmaceutically acceptable additive by an approach known in the art according to their dosage forms. Examples of the additive contained in the pharmaceutical compositions can include excipients, disintegrants, binders, lubricants, diluents, buffers, tonicity agents, antiseptics, wetting agents, emulsifiers, dispersants, stabilizers, and solubilizers. The pharmaceutical compositions can be formulated by appropriately mixing the compound (I) of the present embodiment with an additive or by diluting or dissolving the compound (I) in an additive.

The medicament according to the present embodiment can be administered systemically or locally through an oral or parenteral (transnasal, transpulmonary, intravenous, intrarectal, subcutaneous, intramuscular, percutaneous, etc.) route.

### (Examples)

Hereinafter, the present invention will be described in more detail with reference to Test Examples, Examples and Reference Examples. Since starting compounds for use in the production of the compound (I) also include novel compounds, Production Examples of the starting compounds will also be described as Reference Examples. The present invention is not limited by compounds described in Examples given below, and various changes or modifications may be made therein without departing from the scope of the present invention.

Of the symbols used in each reference example, each example, and each table, ¹H-NMR means a spectrum measured by proton nuclear magnetic resonance spectroscopy. CDCl₃ means chloroform-d, DMSO-D₆ means dimethyl sulfoxide-d₆, and CD₃OD means methanol-d4. MS (ESI⁺) and MS (ESI⁻) are electrospray ionization methods, MS (FI⁺) is electrospray ionization method, MS (FD⁺) is electrospray ionization method, MS (EI⁺) is electron ionization method, MS (CI⁺) is electron ionization method. It means mass spectrometric data measured by a chemical ionization method. Room temperature means 1 to 30 ° C.

### <Reference example 1-1>

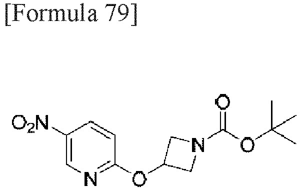

Under an argon atmosphere, 55% sodium hydride (550 mg) was gradually added to a solution of 2-chloro-5-nitropyridine (1.00 g) and tert-butyl 3-hydroxyazetidine-1-carboxylate (2.18 g) in tetrahydrofuran (15.0 mL) at 0 °C, and the mixture was stirred at room temperature for 30 minutes. The reaction mixture was added to water and extracted once with ethyl acetate. The organic layer was washed with water and saturated brine, dried over anhydrous sodium sulfate, and concentrated under reduced pressure after filtration of the insoluble material. The residue was purified by silica gel column chromatography (hexane : ethyl acetate = 10 : 1 to 2 : 1) to obtain tert-butyl 3- (5-nitropyridin-2-yl)oxyazetidine-1-carboxylate (1.82 g).

¹H-NMR (CDCl₃, 400MHz) δ: 1.45 (9H, s), 3.97-4.04 (2H, m), 4.32-4.40 (2H, m), 5.38-5.47 (1H, m), 6.90 (1H, d, J = 9.2 Hz), 8.40 (1H, dd, J = 9.2, 2.4 Hz), 9.03 (1H, d, J = 2.4 Hz).

MS (FI⁺): 296.1 [M+H]⁺

Reference Examples 1-2 to 1-6 below were obtained, using the corresponding starting materials and reactants, by the same method as in Reference Example 1-1, the method described in Step 15-1 or Step 15-2, or a method similar thereto.

**[Table 21]**

| Reference Example | Structure | Equipment Data |
|---|---|---|
| 1 - 2 | 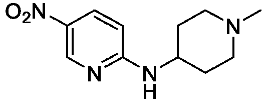 | ¹H-NMR (CDCl₃, 400 MHz) δ: 1.65-1.75 (2H, m), 2.07-2.13 (2H, m), 2.23-2.31 (2H, m), 2.38 (3H, s), 2.89-2.96 (2H, m), 3.87 (1H, brs), 5.26 (1H, brs), 6.36 (1H, d, J = 9.1 Hz), 8.17 (1H, dd, J = 9.1, 2.4 Hz), 9.00 (1H, d, J = 2.4 Hz). |
| | | HRMS (ESI⁺):237.13509 [M+H]⁺ |
| 1 - 3 | 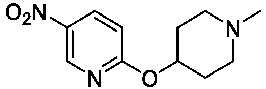 | ¹H-NMR (CDCl₃, 400MHz) δ: 1.80-1.93 (2H, m), 2.01-2.12 (2H, m), 2.26-2.36 (5H, m), 2.65-2.79 (2H, m), 5.14-5.25 (1H, m), 6.79 (1H, d, J = 9.1 Hz), 8.34 (1H, dd, J = 9.1, 3.0 Hz), 9.05 (1H, d, J = 3.0 Hz). |
| | | MS (ESI⁺): 238.1 [M+H]⁺ |
| 1 - 4 | 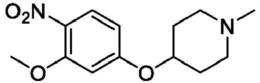 | ¹H-NMR (CDCl₃, 400MHz) δ: 1.81-1.93 (2H, m), 1.98-2.08 (2H, m), 2.26-2.37 (5H, m), 2.61-2.75 (2H, m), 3.94 (3H, s), 4.37-4.47 (1H, m), 6.49 (1H, dd, J = 9.1, 2.4 Hz), 6.54 (1H, d, J = 2.4 Hz), 7.99 (1H, d, J = 9.1 Hz). |
| | | MS (ESI⁺): 267.1 [M+H]⁺ |
| 1 - 5 | 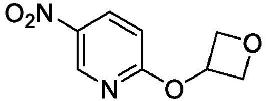 | ¹H-NMR (CDCl₃, 400MHz) δ: 4.68-4.79 (2H, m), 4.99-5.07 (2H, m), 5.64-5.78 (1H, m), 6.92 (1H, d, J = 9.2 Hz), 8.41 (1H, dd, J = 9.2, 3.1 Hz), 9.01 (1H, d, J = 3.1 Hz). |
| | | MS (FI⁺): 196.0 [M]⁺ |

**[Table 22]**

| Reference Example | Structure | Equipment Data |
|---|---|---|
| 1 - 6 | 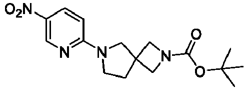 | ¹H-NMR (CDCl₃, 400MHz) δ: 1.45 (9H, s), 2.22-2.34 (2H, m), 3.35-3.86 (4H, m), 3.87-3.98 (4H, m), 6.32 (1H, d, J = 9.7 Hz), 8.22 (1H, dd, J = 9.7, 2.4 Hz), 9.06 (1H, d, J = 2.4 Hz). |
| | | MS (ESI⁺): 335.2 [M+H]⁺ |

### <Reference example 2-1>

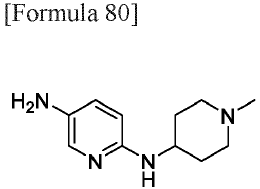

Under an argon atmosphere, 5% palladium carbon (66.6 mg) was added to a solution of N-(1-methylpiperidin-4-yl)-5-nitropyridin-2-amine (333 mg) in tetrahydrofuran-ethanol (7.04 mL, 1:1) and the mixture was stirred at room temperature for 8 hours under a hydrogen atmosphere. After replacing with a argon atmosphere, the reaction mixture was filtered through Celite, and the filtrate was concentrated. The residue was purified by amino-silica gel column chromatography (ethyl acetate: methanol = 9 : 1) to obtain 2-N-(1-methylpiperidin-4-yl)pyridine-2,5-diamine (232 mg).

¹H-NMR (CDCl₃, 400MHz) δ: 1.47-1.58 (2H, m), 2.01-2.08 (2H, m), 2.13-2.21 (2H, m), 2.31 (3H, s), 2.78-2.86 (2H, M), 3.19 (2H, brs), 3.49-3.57 (1H, m), 3.96 (1H, d, J = 7.3 Hz), 6.30 (1H, d, J = 8.5 Hz), 6.94 (1H, dd, J = 8.5, 3.0 Hz), 7.68 (1H, d, J = 3.0 Hz).

HRMS (ESI⁺): 207.16063 [M+H]⁺

Using the corresponding starting materials and reactants, the following Reference Examples 2-2 to 2-7 were obtained by the same method as in Reference Example 2-1 and the method described in Step 14-1 or a method similar thereto.

**[Table 23]**

| Reference Example | Structure | Equipment Data |
|---|---|---|
| 2 - 2 | 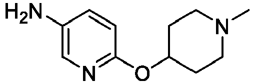 | ¹H-NMR (CDCl₃, 400MHz) δ: 1.74-1.86 (2H, m), 1.96-2.07 (2H, m), 2.21-2.33 (5H, m), 2.60-2.80 (2H, m), 3.34 (2H, br s), 4.85-4.95 (1H, m), 6.57 (1H, d, J = 9.1 Hz), 7.02 (1H, dd, J = 9.1, 3.0 Hz), 7.64 (1H, d, J = 3.0 Hz). |
| | | MS (ESI⁺): 208.1 [M+H]⁺ |

**[Table 24]**

| Reference Example | Structure | Equipment Data |
|---|---|---|
| 2 - 3 | 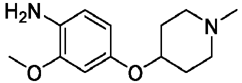 | ¹H-NMR (CDCl₃, 400MHz) δ: 1.75-1.86 (2H, m), 1.92-2.03 (2H, m), 2.18-2.27 (2H, m), 2.29 (3H, s), 2.63-2.76 (2H, m), 3.53 (2H, br s), 3.82 (3H, s), 4.07-4.18 (1H, m), 6.37 (1H, dd, J = 8.5, 2.4 Hz), 6.47 (1H, d, J = 2.4 Hz), 6.62 (1H, d, J = 8.5 Hz). |
| | | MS (ESI⁺): 237.2 [M+H]⁺ |
| 2 - 4 | 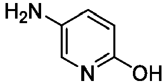 | ¹H-NMR (DMSO-D₆, 400MHz) δ: 4.24 (2H, br s), 6.20 (1H, d, J = 9.2 Hz), 6.73 (1H, d, J = 3.1 Hz), 7.02 (1H, dd, J = 9.2, 3.1 Hz), 10.57 (1H, br s). |
| 2 - 5 | 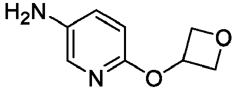 | ¹H-NMR (CDCl₃, 400MHz) δ: 3.38 (2H, brs), 4.66-4.75 (2H, m), 4.92-5.01 (2H, m), 5.46-5.56 (1H, m), 6.63 (1H, d, J = 8.5 Hz), 7.04 (1H, dd, J = 8.5, 3.0 Hz), 7.56 (1H, d, J = 3.0 Hz). |
| | | MS (ESI⁺): 167.1 [M+H]⁺ |
| 2 - 6 | 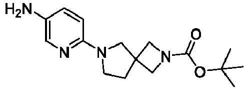 | ¹H-NMR (DMSO-D₆, 400MHz) δ: 1.37 (9H, s), 2.08 (2H, t, J = 7.0 Hz), 3.27 (2H, t, J = 7.0 Hz), 3.41 (2H, s), 3.71-3.84 (4H, m), 4.33 (2H, s), 6.24 (1H, d, J = 8.5 Hz), 6.89 (1H, dd, J = 8.5, 3.0 Hz), 7.54 (1H, d, J = 3.0 Hz). |
| | | MS (ESI⁺): 305.2 [M+H]⁺ |
| 2 - 7 | 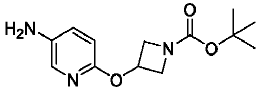 | ¹H-NMR (CDCl₃, 400MHz) δ: 1.44 (9H, s), 3.39 (2H, brs), 3.89-3.99 (2H, m), 4.28 (2H, ddd, J = 9.7, 6.7, 1.2 Hz), 5.18-5.26 (1H, m), 6.61 (1H, d, J = 8.5 Hz), 7.03 (1H, dd, J = 8.5 , 3.0 Hz), 7.58 (1H, d, J = 3.0 Hz). |
| | | MS (ESI⁺): 266.2 [M+H]⁺ |

### <Reference example 3>

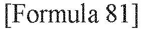

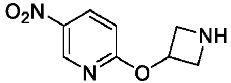

Trifluoroacetic acid (3.00 mL) was added to a solution of tert-butyl 3-(5-nitropyridin-2-yl)oxyazetidine-1-carboxylate (500 mg) in dichloromethane (3.00 mL) at room temperature and the mixture was stirred for 1 hour. The reaction mixture was concentrated under reduced pressure, and the residue was used in Reference Example 4 as a trifluoroacetate of 2-(azetidin-3-yloxy)-5-nitropyridine without further purification.

### <Reference example 4>

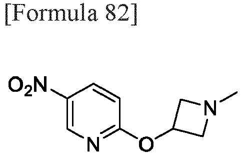

37% aqueous formaldehyde solution (0.672 mL) and sodium triacetoxyborohydride (897 mg) were added to a solution of 2-(azetidin-3-yloxy)-5-nitropyridine trifluoroacetate, which is a crude product of Reference Example 3, in dichloromethane (10.0 mL), and the mixture was stirred for 2 hours. The reaction mixture was concentrated under reduced pressure, and the residue was purified by amino-silica gel column chromatography (hexane : ethyl acetate = 3 : 1 to 0 : 1) to obtain 2-(1-methylazetidin-3-yl)oxy-5-nitropyridine (142 mg).

¹H-NMR (CDCl₃, 400MHz) δ: 2.63 (3H, s), 3.47-3.55 (2H, m), 4.23-4.31 (2H, m), 5.39-5.48 (1H, m), 6.88 (1H, d, J = 9.1 Hz), 8.40 (1H, dd, J = 9.1, 3.0 Hz), 9.02 (1H, d, J = 3.0 Hz).

MS (ESI⁺): 210.1 [M+H]⁺

### <Reference example 5>

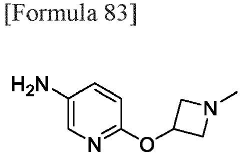

Under an argon atmosphere, 10% palladium carbon (14.0 mg) was added to a solution of 2-(1-methylazetidin-3-yl)oxy-5-nitropyridine (140 mg) in ethanol (6.50 mL) at room temperature, and the mixture was stirred under a hydrogen atmosphere for 1.5 hours. After replacing with a argon atmosphere, the reaction mixture was filtered through Celite, and the filtrate was concentrated under reduced pressure. The residue was purified by amino-silica gel column chromatography (ethyl acetate: methanol = 1 0 to 9 1) to obtain 6-(1-methylazetidin-3-yl)oxypyridin-3-amine (46.0 mg).

¹H-NMR (CDCl₃, 400MHz) δ: 2.39 (3H, s), 3.04-3.11 (2H, m), 3.35 (2H, brs), 3.75-3.82 (2H, m), 5.06-5.15 (1H, m), 6.58 (1H, d, J = 8.5 Hz), 7.02 (1H, dd, J = 8.5, 3.0 Hz), 7.60 (1H, d, J = 3.0 Hz).

MS (EI⁺): 179.1 [M]⁺

### <Reference example 6>

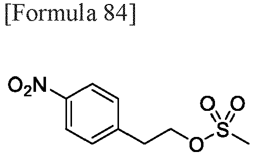

It was synthesized in the same manner as Reference Example 80 using 2-(4-nitrophenyl)ethanol.

### <Reference example 7>

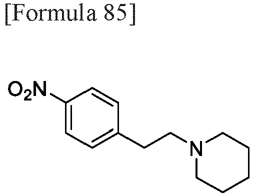

Under an argon atmosphere, piperidine (0.404 mL) was added to a solution of 2-(4-nitrophenyl)ethyl methanesulfonate (200 mg) in acetonitrile (1.00 mL) at room temperature, and the mixture was stirred for 69 hours. The reaction mixture was added to water and extracted twice with ethyl acetate. The combined organic layer was washed with water and saturated brine, dried over anhydrous sodium sulfate, and concentrated under reduced pressure after filtration of the insoluble material. The residue was purified by amino-silica gel column chromatography (hexane ethyl acetate = 9 1 to 0 1) to obtain 1-[2-(4-nitrophenyl)ethyl]piperidine (191 mg).

¹H-NMR (CDCl₃, 400MHz) δ: 1.41-1.51 (2H, m), 1.56-1.65 (4H, m), 2.38-2.52 (4H, m), 2.55-2.61 (2H, m), 2.86-2.96 (2H, m), 7.36 (2H, d, J = 8.5 Hz), 8.14 (2H, d, J = 8.5 Hz).

MS (FI⁺): 234.1 [M]⁺

### <Reference example 8>

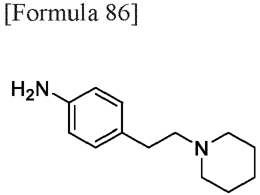

The title compound was synthesized in the same manner as Reference Example 5 using 1-[2-(4-nitrophenyl)ethyl]piperidine.

¹H-NMR (DMSO-D₆, 400MHz) δ: 1.31-1.39 (2H, m), 1.42-1.51 (4H, m), 2.28-2.41 (6H, m), 2.47-2.54 (2H, m), 4.78 (2H, s), 6.45 (2H, d, J = 8.5 Hz), 6.82 (2H, d, J = 7.9 Hz).

MS (ESI⁺): 205.2 [M+H]⁺

### <Reference example 9>

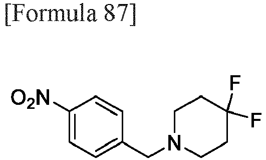

Potassium carbonate (384 mg) and 1-(bromomethyl)-4-nitrobenzene (200 mg) were added to a solution of 4,4-difluoropiperidine hydrochloride (219 mg) in acetonitrile (2.00 mL) under an argon atmosphere at room temperature and the mixture was stirred for 1 hour. The reaction mixture was added to water, and extracted twice with ethyl acetate. The combined organic layer was washed with water and saturated brine, dried over anhydrous sodium sulfate, and concentrated under reduced pressure after filtration of the insoluble material. The residue was purified by silica gel column chromatography (hexane : ethyl acetate = 9 : 1 to 1 : 1) to obtain 4,4-difluoro-1-[(4-nitrophenyl)methyl]piperidine (220 mg).

¹H-NMR (CDCl₃, 400MHz) δ: 1.94-2.08 (4H, m), 2.51-2.61 (4H, m), 3.64 (2H, s), 7.51 (2H, d, J = 8.5 Hz), 8.19 (2H, d, J = 8.5 Hz).

MS (EI⁺): 256.1 [M]⁺

### <Reference example 10>

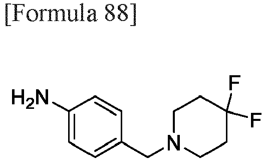

The title compound was synthesized in the same manner as Reference Example 5 using 4,4-difluoro-1-[(4-nitrophenyl)methyl]piperidine.

¹H-NMR (CDCl₃, 400MHz) δ: 1.90-2.03 (4H, m), 2.45-2.58 (4H, m), 3.43 (2H, s), 3.62 (2H, br s), 6.64 (2H, d, J = 8.5 Hz), 7.08 (2H, d, J = 8.5 Hz).

MS (EI⁺): 226.1 [M]⁺

### <Reference example 11>

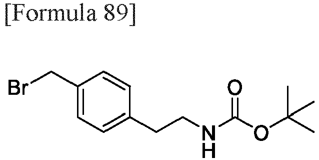

Under an argon atmosphere, triphenylphosphine (2.35 g) and carbon tetrabromide (2.97 g) were added to a solution of tert-butyl N-[2-[4-(hydroxymethyl)phenyl]ethyl]carbamate (2.05 g) in tetrahydrofuran (40.7 mL) under ice cooling condition. The reaction mixture was stirred at room temperature for 30 minutes. Diethyl ether (50.0 mL) and hexane (50.0 mL) were added to the reaction mixture, and the resulting solid was filtered through Celite and washed with diethyl ether. The filtrate was concentrated under reduced pressure to obtain tert-butyl N-[2-[4-(bromomethyl)phenyl]ethyl]carbamate. The crude product was used in the next step without further purification.

### <Reference example 12>

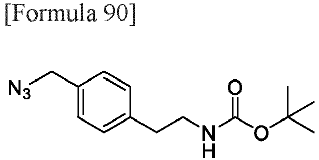

Sodium azide (636 mg) was added to a solution of tert-butyl N-[2-[4-(bromomethyl)phenyl]ethyl]carbamate, which is a crude product in N,N-dimethylformamide (40.8 mL), under an argon atmosphere. The reaction mixture was stirred at room temperature for 17 hours. Water was added, and the mixture was extracted with ethyl acetate. The organic layer was washed with saturated brine, dried over anhydrous sodium sulfate, and concentrated under reduced pressure after filtration of the insoluble material. The residue was purified by silica gel column chromatography (hexane : ethyl acetate = 2 : 1) to obtain tert-butyl N-[2-[4-(azidomethyl)phenyl]ethyl]carbamate (1.85 g).

¹H-NMR (CDCl₃, 400MHz) δ: 1.43 (9H, s), 2.80 (2H, t, J = 7.3 Hz), 3.33-3.41 (2H, m), 4.31 (2H, s), 4.56 (1H, brs), 7.21 (2H, d, J = 7.9 Hz), 7.26 (2H, d, J = 7.9 Hz).

HRMS (FI⁺): 276.15809 [M]⁺

### <Reference example 13>

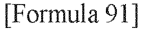

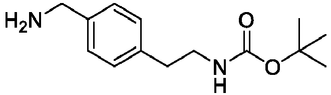

Under an argon atmosphere, a solution of tert-butyl N-[2-[4-(azidomethyl)phenyl]ethyl]carbamate (1.14 g) in tetrahydrofuran (10.3 mL) was added to a solution of lithium aluminum hydride (626 mg) in tetrahydrofuran (10.3 mL) under ice cooling condition. The reaction mixture was stirred under ice cooling temperature for 4 hours. Water (0.626 mL), 15% aqueous sodium hydroxide solution (0.626 mL), and water (1.88 mL) were added to the mixture, and the mixture was stirred for 2 hours. The resulting suspension was filtered through Celite, and the filtrate was concentrated to obtain tert-butyl N-[2-[4-(aminomethyl)phenyl]ethyl]carbamate (915 mg).

¹H-NMR (CDCl₃, 400MHz) δ: 1.43 (9H, s), 2.78 (2H, t, J = 6.7 Hz), 3.36 (2H, q, J = 6.7 Hz), 3.84 (2H, s), 4.50-4.70 (1H, m), 7.16 (2H, d, J = 7.9 Hz), 7.25 (2H, d, J = 7.9 Hz).

HRMS (ESI⁺): 251.17652 [M+H]⁺

### <Reference example 14>

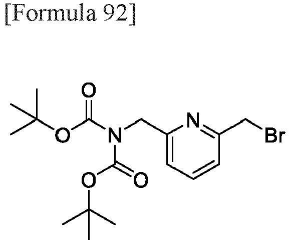

Under an argon atmosphere, triphenylphosphine (186 mg) and carbon tetrabromide (274 mg) were added to a solution of tert-butyl N-[[6-(hydroxymethyl)pyridin-2-yl]methyl]-N-[(2-methylpropan-2-yl)oxycarbonyl]carbamate (200 mg), in dichloromethane (2.96 mL) under ice cooling condition. The reaction mixture was stirred under ice cooling condition for 1 hour, and then concentrated under reduced pressure. The residue was purified by silica gel column chromatography (ethyl acetate hexane = 1 4) to obtain tert-butyl N-[[6-(bromomethyl)pyridin-2-yl]methyl]-N-[(2-methylpropan-2-yl)oxycarbonyl]carbamate (222 mg).

¹H-NMR (CDCl₃, 400MHz) δ: 1.45 (18H, s), 4.51 (2H, s), 4.91 (2H, s), 7.09 (1H, d, J = 7.9 Hz), 7.31 (1H, d, J = 7.9 Hz), 7.65 (1H, t, J = 7.9 Hz).

HRMS (ESI⁺): 401.10834 [M+H]⁺

### <Reference example 15-1>

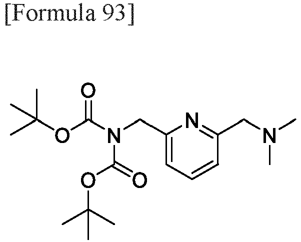

Under an argon atmosphere, dimethylamine (0.820 mL, 2 mol/L tetrahydrofuran solution) and potassium carbonate (227 mg) were added to a solution of tert-butyl N- [[6-(bromomethyl)pyridin-2-yl]methyl]-N-[(2-methylpropan-2-yl)oxycarbonyl]carbamate (220 mg) in N,N-dimethylformamide (2.74 mL), and the reaction mixture was stirred at room temperature for 1 hour. Water was added, and the mixture was extracted with ethyl acetate. The organic layer was washed with saturated brine, dried over anhydrous sodium sulfate, and concentrated after filtration of the insoluble material. The residue was purified by amino-silica gel column chromatography (ethyl acetate / hexane = 33%) to obtain tert-butyl N-[[6-[(dimethylamino)methyl]pyridin-2-yl]methyl]-N-[(2-methylpropan-2-yl)oxycarbonyl]carbamate (177 mg).

¹H-NMR (CDCl₃, 400MHz) δ: 1.43 (18H, s), 2.28 (6H, s), 3.56 (2H, s), 4.92 (2H, s), 7.03 (1H, d, J = 7.9 Hz), 7.25 (1H, d, J = 7.9 Hz), 7.61 (1H, t, J = 7.9 Hz).

HRMS (ESI⁺): 366.23889 [M+H]⁺

Using the corresponding starting materials and reactants, the following Reference Example 15-2 was obtained by the same method in Reference Example 15-1, the method described in Step 18-2, or a method similar thereto.

**[Table 25]**

| Reference Example | Structure | Equipment Data |
|---|---|---|
| 15 - 2 | 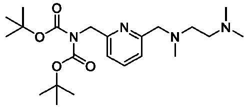 | ¹H-NMR (CDCl₃, 400 MHz) δ:1.43 (18H, s), 2.23 (6H, s), 2.28 (3H, s), 2.44 (1H, dd, J = 7.9, 1.2 Hz), 2.46 (1H, d, J = 7.9 Hz), 2.54 (1H, d, J = 7.9 Hz), 2.56 (1H, dd, J = 7.9, 1.2 Hz), 3.66 (2H, s), 4.90 (2H, s), 7.02 (1H, d, J = 7.9 Hz), 7.31 (1H, d, J = 7.9 Hz), 7.61 (1H, t, J = 7.9 Hz). |
| | | HRMS (ESI⁺):423.29703 [M+H]⁺ |

### <Reference example 16>

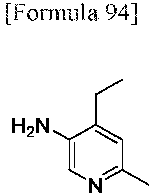

Under an argon atmosphere, triethylamine (0.379 mL) and diphenylphosphoryl azide (0.585 mL) were added to a solution of 4-ethyl-6-methylpyridine-3-carboxylic acid (224 mg) in 1,4-dioxane (13.0 mL) at room temperature. The reaction mixture was stirred at 60 °C for 1 hour and stirred at 80 °C for 30 minutes. After 1 mol/L hydrochloric acid (5.00 mL) was added, the mixture was stirred at the same temperature for 15 minutes. Diisopropylamine (1 mL) was added, and the mixture was purified by amino-silica gel column chromatography (hexane : ethyl acetate = 5 : 1 to 0 : 1) to obtain 4-ethyl-6-methylpyridin-3-amine (100 mg).

¹H-NMR (DMSO-D₆, 400MHz) δ: 1.25 (3H, t, J = 7.3 Hz), 2.43 (3H, s), 2.48 (2H, q, J = 7.3 Hz), 3.48 (2H, brs), 6.85 (1H, s), 7.16 (1H, s).

MS (EI⁺): 136.1 [M]⁺

### <Reference example 17>

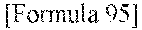

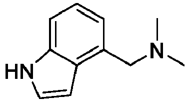

Under an argon atmosphere, dimethylamine (1.55 mL, 2 mol/L tetrahydrofuran solution) and acetic acid (0.589 mL) were added to a solution of 1H-indole-4-carbaldehyde (300 mg) in tetrahydrofuran (10.3 mL). After stirring at room temperature for 1 hour, sodium triacetoxyborohydride (875 mg) was added, and the reaction mixture was stirred at room temperature for 4 hours. Saturated sodium hydrogen carbonate was added, and the mixture was extracted with ethyl acetate. The organic layer was washed with saturated brine, dried over anhydrous sodium sulfate, and concentrated after filtration of the insoluble material. The residue was purified by amino-silica gel column chromatography (hexane : ethyl acetate = 2 : 1) to obtain 1-(1H-indol-4-yl)-N,N-dimethylmethanamine (334 mg).

¹H-NMR (CDCl₃, 400MHz) δ: 2.30 (6H, s), 3.71 (2H, s), 6.69 (1H, t, J = 2.4 Hz), 7.07 (1H, d, J = 7.3 Hz), 7.16 (1H, t, J = 7.3 Hz), 7.21 (1H, t, J = 2.4 Hz), 7.31 (1H, d, J = 7.3 Hz), 8.22 (1H, brs).

HRMS (ESI⁺): 175.12376 [M+H]⁺

### <Reference example 18>

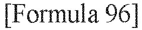

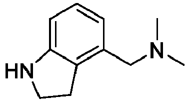

Under an argon atmosphere, triethylsilane (0.240 mL) was added to a solution (0.752 mL) of 1-(1H-indol-4-yl)-N,N-dimethylmethanamine (131 mg) in trifluoroacetic acid (0.752 mL) under ice cooling codition. The reaction mixture was stirred under ice cooling condition for 2 hours. Saturated sodium hydrogen carbonate was added, and the mixture was extracted with ethyl acetate. The organic layer was washed with saturated brine, dried over anhydrous sodium sulfate, and concentrated after filtration of the insoluble material. The residue was purified by amino-silica gel column chromatography (hexane : ethyl acetate = 2 : 1) to obtain 1-(2,3-dihydro-1H-indol-4-yl)-N,N-dimethylmethanamine (103 mg).

¹H-NMR (CDCl₃, 400MHz) δ: 2.29 (6H, s), 3.04 (2H, t, J = 8.3 Hz), 3.40 (2H, s), 3.56 (2H, t, J = 8.3 Hz), 6.57 (1H, d, J = 7.9 Hz), 6.68 (1H, d, J = 7.9 Hz), 7.00 (1H, t, J = 7.9 Hz).

HRMS (EI⁺): 176.13135 [M]⁺

### <Reference example 19>

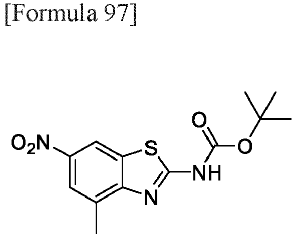

Under an argon atmosphere, di-tert-butyl dicarbonate (1.15 g) and N,N-dimethyl-4-aminopyridine were added to a solution of 4-methyl-6-nitro-1,3-benzothiazole-2-amine (1.00 g) in dichloromethane (25.0 mL) and tetrahydrofuran (25.0 mL) at room temperature. The reaction mixture was stirred for 20 hours. After removing the insoluble material by filtration of the insoluble material, the filtrate was concentrated under reduced pressure, and the residue was purified by silica gel column chromatography (hexane: ethyl acetate = 9 : 1 to 1 : 1) to obtain tert-butyl N-(4-methyl-6-nitro-1,3-benzothiazol-2-yl)carbamate (1.30 g).

¹H-NMR (DMSO-D₆, 400MHz) δ: 1.51 (9H, s), 2.60 (3H, s), 8.10 (1H, dd, J = 2.4, 1.2 Hz), 8.81 (1H, d, J = 2.4 Hz), 12.27 (1H, s).

MS (ESI⁻): 308.1 [M-H]⁻

### <Reference example 20>

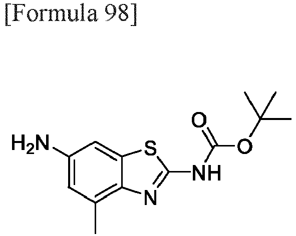

The title compound was synthesized in the same manner as Reference Example 5 using tert-butyl N-(4-methyl-6-nitro-1,3-benzothiazol-2-yl)carbamate.

¹H-NMR (DMSO-D₆, 400MHz) δ: 1.47 (9H, s), 2.38 (3H, s), 4.97 (2H, s), 6.48 (1H, d, J = 2.4 Hz), 6.78 (1H, d, J = 2.4 Hz), 11.37 (1H, s).

MS (ESI⁺): 280.1 [M+H]⁺

### <Reference example 21>

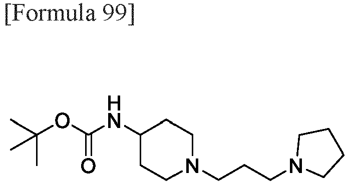

Under an argon atmosphere, N,N-diisopropylethylamine (0.340 mL) and 1-(3-bromopropyl)pyrrolidine hydrobromide (300 mg) were added to a solution of tert-butyl N-piperidin-4-ylcarbamate (200 mg) in tetrahydrofuran (5.00 mL) at room temperature. After stirring at 40 °C for 24 hours, the reaction mixture was added to water and extracted 3 times with ethyl acetate. The combined organic layer was dried over anhydrous sodium sulfate and concentrated under reduced pressure after filtration of the insoluble material. The residue was roughly purified by amino-silica gel column chromatography (ethyl acetate : methanol = 1 : 0 to 9 : 1) to obtain tert-butyl N-[1-(3-pyrrolidin-1-ylpropyl)piperidin-4-yl]carbamate (169 mg) including impurities. The crude product was used in Reference Example 22 without further purification.

### <Reference example 22>

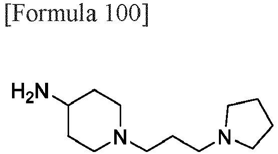

Trifluoroacetic acid (1.00 mL) was added to a solution of tert-butyl N-[1-(3-pyrrolidin-1-ylpropyl) piperidin-4-yl]carbamate (163 mg) including impurities obtained in Reference Example 21 in dichloromethane (1.00 mL) at room temperature, and the mixture was stirred for 10 minutes. After the reaction mixture was concentrated under reduced pressure, the residue was used in Example 1-56 as a trifluoroacetic acid salt of 1-(3-pyrrolidin-1-ylpropyl)piperidin-4-amine.

### <Reference example 23>

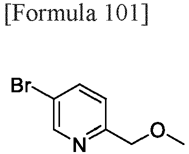

Under an argon atmosphere, a solution of (5-bromopyridin-2-yl) methanol (200 mg) in N,N-dimethylformamide (1.00 mL) was added dropwise to a solution of 55% sodium hydride (55.4 mg) in N,N-dimethylformamide (2.00 mL) at 0 °C. The reaction mixture was stirred for 15 minutes, and then a solution of methyl iodide (72.8 µL) in N,N-dimethylformamide (1.00 mL) was added dropwise. After stirring at room temperature for 1 hour, the reaction mixture was added to saturated brine, and extracted twice with ethyl acetate. The combined organic layer was washed with water and saturated brine, dried over anhydrous sodium sulfate, and concentrated under reduced pressure after filtration of the insoluble material to obtain a mixture of 5-bromo-2-(methoxymethyl)pyridine : N,N-dimethylformamide = 1 : 0.45 (227 mg).

¹H-NMR (CDCl₃, 400MHz) δ: 3.48 (3H, s), 4.54 (2H, s), 7.34 (1H, d, J = 8.5 Hz), 7.82 (1H, dd, J = 8.5, 2.4 Hz), 8.62 (1H, d, J = 2.4 Hz).

### <Reference example 24>

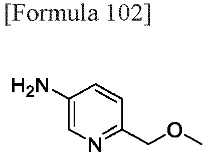

Under an argon atmosphere, a solution of 5-bromo-2-(methoxymethyl)pyridine (210 mg), benzophenone imine (0.210 mL), tripotassium phosphate (552 mg), tris (dibenzylideneacetone) dipalladium (0) chloroform adduct (26.9 mg) and 2-di-tert-butylphosphino-2', 4', 6'-triisopropylbiphenyl (26.5 mg) in 1,2-dimethoxyethane (2.00 mL) was stirred at 40 °C for 4 hours. After filtering the insoluble material through Celite, 1 mol/L hydrochloric acid was added to the reaction mixture to adjust the pH to 1, and the mixture was stirred at room temperature for 10 minutes. After washing the mixture was washed with ethyl acetate, a saturated aqueous sodium hydrogen carbonate was added to adjust the pH to 8. The mixture was purified by amino-silica gel column chromatography (hexane : ethyl acetate = 3 : 1 to 0 : 1) to obtain 6- (methoxymethyl)pyridin-3-amine (66.0 mg).

¹H-NMR (CDCl₃, 400MHz) δ: 3.43 (3H, s), 3.69 (2H, brs), 4.46 (2H, s), 6.99 (1H, dd, J = 8.5, 2.4 Hz), 7.18 (1H, d, J = 8.5 Hz), 8.06 (1H, d, J = 2.4 Hz).

MS (EI⁺): 138.1 [M]⁺

### <Reference example 25>

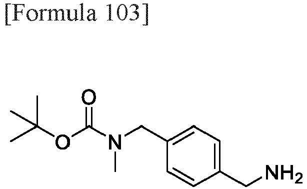

tert-Butyl (4-cyanobenzyl)(methyl)carbamate (626 mg), 10% palladium carbon (130 mg) and methanol (20 mL) were stirred under a hydrogen atmosphere for 5 days. The reaction mixture was filtered, and the filtrate was concentrated under reduced pressure to obtain tert-butyl (4-(aminomethyl)benzyl)(methyl)carbamate (597 mg). The crude product was used in the next reaction without further purification.

¹H-NMR (270MHz, CDCl₃) δ: 1.48 (9H, s), 1.68 (2H, s), 2.81 (3H, s), 4.41 (2H, s), 7.09-7.34 (4H, m).

MS (ESI⁺): 251.28 [M+H]⁺

### <Reference example 26>

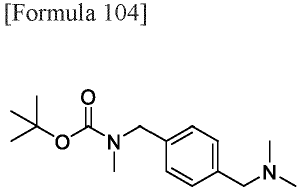

A mixture of tert-butyl (4-(aminomethyl)benzyl)(methyl)carbamate (597 mg), paraformaldehyde (573 mg) and trifluoroethanol (20 mL) was stirred at room temperature for 15 minutes, and then sodium borohydride (361 mg) was added, and the reaction mixture was stirred overnight at room temperature, and then concentrated under reduced pressure. After water was added, the mixture was extracted twice with ethyl acetate. The organic layer was concentrated under reduced pressure and purified by amino-silica gel chromatography to obtain tert-butyl (4-((dimethylamino)methyl)benzyl)(methyl)carbamate (424 mg).

¹H-NMR (270MHz, CDCl₃) δ: 1.48 (9H, s), 2.23 (6H, s), 2.81 (3H, br s), 3.40 (2H, s), 4.40 (2H, s), 7.17 (2H, d, J = 8.1 Hz), 7.26 (2H, d, J = 8.1 Hz).

MS (ESI⁺): 278.20 [M+H]⁺

### <Reference example 27>

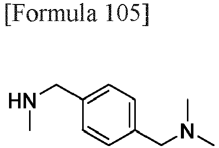

Trifluoroacetic acid (1 mL) was added to a solution of tert-butyl (4-((dimethylamino)methyl)benzyl)(methyl)carbamate (424 mg) in dichloromethane (5 mL) and the mixture was stirred overnight. After 2N aqueous sodium hydroxide solution was added to adjust the pH between 13 and 14, the reaction mixture was extracted twice with dichloromethane. The organic layer was dried over anhydrous sodium sulfate, concentrated under reduced pressure to obtain N,N-dimethyl-1-(4-((methylamino)methyl)phenyl)methanamine (244 mg).

¹H-NMR (270MHz, CDCl₃) δ: 2.23 (s, 6H), 2.45 (s, 3H), 3.40 (s, 2H), 3.73 (s, 2H), 7.26 (s, 4H).

MS (ESI⁺): 179.18 [M+H]⁺

### <Reference example 28>

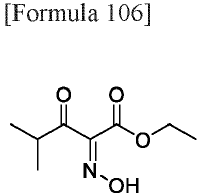

A solution of ethyl 4-methyl-3-oxopentanoate (1.5 g) in acetic acid (1.2 mL) was cooled to 10 °C and then aqueous sodium nitrite (654 mg) was added dropwise so that the temperature of the mixture was below 20 °C. After completion of the dropping, the reaction mixture was warmed to room temperature and stirred for 1 hour. Then, the reaction mixture was extracted 3 times with diethyl ether. The combined organic layer was washed with water, dried over magnesium sulfate, filtered, and concentrated under reduced pressure to obtain ethyl 2-(hydroxyimino)-4-methyl-3-oxopentanoate (1.85 g). The crude product was used in the next reaction without further purification.

MS (ESI⁺): 188.10 [M+H]⁺

### <Reference example 29>

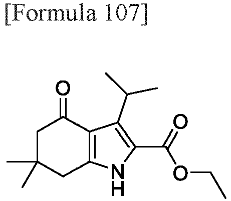

A solution of sodium acetate trihydrate (811 mg) and 5,5-dimethyl-1,3-cyclohexanedione (1.39 g) in acetic acid (40 mL) was heated and stirred at 70 °C. While controlling the reaction temperature between 70 °C and 80 °C, a solution of ethyl 2-(hydroxyimino)-4-methyl-3-oxopentanoate (1.85 g) in acetic acid (20 mL) was added dropwise, and at the same time, zinc powder (1.03 g) was also added over 30 minutes. The reaction mixture was heated to 100 °C and stirred for 1 hour, then cooled to 70 °C, and water was added. The reaction mixture was heated to 100 °C again and stirred for 6 hours. After cooling to room temperature, the reaction mixture was added to an ice-water mixed solution and extracted with dichloromethane. The combined organic layer was washed with water, dried over magnesium sulfate, filtered, and concentrated under reduced pressure. The residue was purified by silica gel column chromatography (chloroform/methanol) to obtain ethyl 3-isopropyl-6,6-dimethyl-4-oxo-4,5,6,7-tetrahydro-1H-indole-2-carboxylate (986 mg).

¹H-NMR (270MHz, CDCl₃) δ: 1.10 (6H, s), 1.32 (6H, d, J = 6.9 Hz), 1.38 (3H, t, J = 7.3 Hz), 2.37 (2H, s), 2.66 (2H, s), 4.07 (1H, m), 4.35 (2H, q, J = 7.3 Hz), 8.95 (1H, brs).

MS (ESI⁺): 278.23 [M+H]⁺

### <Reference example 30-1>

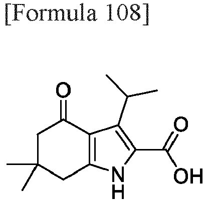

A solution of ethyl 3-isopropyl-6,6-dimethyl-4-oxo-4,5,6,7-tetrahydro-1H-indole-2-carboxylate (980 mg) and potassium hydroxide (496 mg) in ethanol (10 mL) and water (3 mL), was heated and stirred for 6 hours under reflux condition. The reaction mixture was cooled to 60 °C, neutralized with acetic acid, and water was added. The resulting precipitate was filtered, washed with water, and dried to obtain 3-isopropyl-6,6-dimethyl-4-oxo-4,5,6,7-tetrahydro-1H-indole-2-carboxylic acid (570 mg).

¹H-NMR (500MHz, CDCl₃ + CD₃OD) δ: 1.10 (6H, s), 1.31 (6H, d, J = 7.0 Hz), 2.36 (2H, s), 2.66 (2H, s), 3.42 (1H, m), 4.06 (1H, m, J = 7.0 Hz).

MS (ESI⁺): 250.19 [M+H]⁺

Using the corresponding starting material and reactant, the following Reference Examples 30-2 to 30-6 were obtained by the same method in Reference Example 30-1, the method described in Steps 26-1 to 26-2 and 25-1, or a method similar thereto, or the method described in the literature, or a method similar thereto.

**[Table 26]**

| Reference Example | Structure | Equipment Data |
|---|---|---|
| 3 0 - 2 | 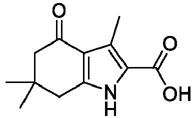 | ¹H-NMR (CDCl₃+CD₃OD, 500 MHz) δ: 1.10 (6H, s), 2.34 (2H, s), 2.60 (3H, s), 2.66 (2H, s). |
| | | MS (ESI⁺): 222.25 [M+H]⁺ |
| 3 0 - 3 | 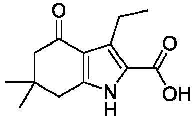 | ¹H-NMR (CDCl₃+CD₃OD, 500 MHz) δ: 1.10 (6H, s), 1.16 (3H, t, J = 7.5 Hz), 2.34 (2H, s), 2.66 (2H, s), 3.11 (2H, q, J = 7.5 Hz). |
| | | MS (ESI⁺): 236.08 [M+H]⁺ |
| 3 0 - 4 | 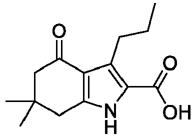 | ¹H-NMR (CDCl₃+CD₃OD, 500 MHz) δ: 0.93 (3H, t, J = 7.0 Hz), 1.10 (6H, s), 1.58 (2H, m), 2.33 (2H, s), 2.66 (2H, s), 3.07 (2H, t, J = 7.0 Hz). |
| | | MS (ESI⁺): 250.21 [M+H]⁺ |
| 3 0 - 5 | 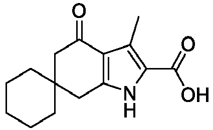 | ¹H-NMR (CDCl₃+CD₃OD, 500 MHz) δ: 1.35-1.60 (10H, m), 2.40 (2H, s), 2.58 (3H, s), 2.72 (2H, s). |
| | | MS (ESI⁺): 262.17 [M+H]⁺ |
| 3 0 - 6 | 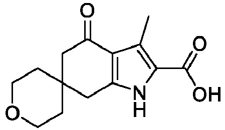 | ¹H-NMR (CDCl₃+CD₃OD, 500 MHz) δ: 1.55-1.70 (4H, m), 2.52 (2H, s), 2.58 (3H, s), 2.82 (2H, s), 3.69 - 3.72 (4H, m). |
| | | MS (ESI⁺): 264.15 [M+H]⁺ |

### <Reference example 31>

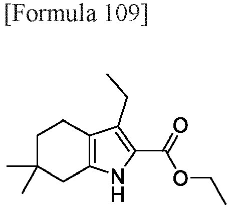

Ethyl 3-ethyl-6,6-dimethyl-4-oxo-4,5,6,7-tetrahydro-1H-indole-2-carboxylate (200 mg) was added to a solution of sodium borohydride (115 mg) in tetrahydrofuran (10 mL) and the mixture was cooled to -5 °C. Boron trifluoride diethyl ether complex (0.54 mL) was slowly added dropwise to the mixture under a nitrogen stream. After dropping, the cooling bath was removed, and the reaction mixture was allowed to warm to room temperature, stirred for 2 hours, and then added to ice water. The resulting precipitate was collected by filtration, washed with water, and dried under reduced pressure to obtain ethyl 3-ethyl-6,6-dimethyl-4,5,6,7-tetrahydro-1H-indole-2-carboxylate (193 mg).

¹H-NMR (270MHz, CDCl₃) δ: 0.95 (6H, s), 1.10 (3H, t, J = 7.5 Hz), 1.31 (3H, t, J = 7.0 Hz), 1.52 (2H, t, J = 6.5 Hz), 2.34 (2H, s), 2.43 (2H, t, J = 6.5 Hz), 2.72 (2H, q, J = 7.5 Hz), 4.29 (2H, q, J = 7.0 Hz), 8.45 (1H, brs).

MS (ESI⁺): 250.33 [M+H]⁺

### <Reference example 32>

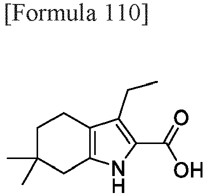

IN aqueous potassium hydroxide solution (5 mL) was added to a solution of ethyl 3-ethyl-6,6-dimethyl-4,5,6,7-tetrahydro-1H-indole-2-carboxylate (182 mg) and the mixture was stirred at 70 °C for 12 hours. The reaction mixture was neutralized with 1 N hydrochloric acid and extracted twice with chloroform. After drying over magnesium sulfate, the extract was filtered and concentrated under reduced pressure to obtain 3-ethyl-6,6-dimethyl-4,5,6,7-tetrahydro-1H-indole-2-carboxylic acid (115 mg).

MS (ESI⁺): 222.26 [M+H]⁺

### <Reference example 33-1>

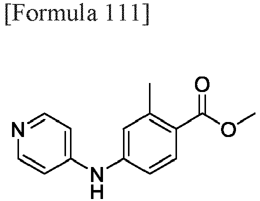

Methyl 4-bromo-2-methylbenzoate (500 mg), rac-2,2'-bis(diphenylphosphino)-1,1'-binaphthyl (22.7 mg), palladium (II) acetate (8.2 mg) and cesium carbonate (2.96 g) were added to a solution (18.2 mL) of pyridine-4-amine (171 mg) in toluene (18.2 mL) under an argon atmosphere. The reaction mixture was stirred at 120 °C for 8 hours. Water was added, and the mixture was extracted with ethyl acetate. The organic layer was washed with saturated brine, dried over anhydrous sodium sulfate, and concentrated after filtration of the insoluble material. The residue was purified by silica gel column chromatography (hexane : ethyl acetate = 1 : 1 to ethyl acetate : methanol = 5 : 1) to obtain methyl 2-methyl-4- (pyridin-4-ylamino)benzoate (79.0 mg).

¹H-NMR (CDCl₃, 400MHz) δ: 2.61 (3H, s), 3.88 (3H, d, J = 1.2 Hz), 6.26 (1H, brs), 6.94 (2H, dd, J = 6.1, 1.2 Hz), 7.01 (1H, s), 7.02-7.05 (1H, m), 7.95 (1H, d, J = 8.5 Hz), 8.38 (2H, d, J = 6.1 Hz).

HRMS (FI⁺): 242.10567 [M]⁺

Using the corresponding starting materials and reactants, the following Reference Examples 33-2 to 33-14 were obtained by the same method in Reference Example 33-1, the method described in Step 27-1, or a method similar thereto.

**[Table 27]**

| Reference Example | Structure | Equipment Data |
|---|---|---|
| 3 3 - 2 | 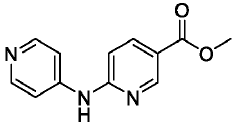 | ¹H-NMR (CDCl₃, 400 MHz) δ: 3.93 (3H, s), 6.89 (1H, d, J = 8.6 Hz), 6.98 (1H, brs), 7.46 (2H, d, J = 6.1 Hz), 8.18 (1H, dd, J = 8.6, 2.4 Hz), 8.49 (2H, d, J = 6.1 Hz), 8.95 (1H, d, J = 2.4 Hz). |
| | | HRMS (FD⁺):229.08494 [M]⁺ |
| 3 3 - 3 | 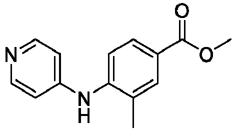 | ¹H-NMR (DMSO-D₆, 400MHz) δ: 2.28 (3H, s), 3.83 (3H, s), 6.89 (2H, d, J = 6.1 Hz), 7.39 (1H, d, J = 8.6 Hz), 7.77 (1H, dd, J = 8.6, 2.4 Hz), 7.86 (1H, d, J = 2.4 Hz), 8.23 (2H, d, J = 6.1 Hz), 8.40 (1H, s). |
| | | HRMS (FI⁺):242.10568 [M]⁺ |
| 3 3 - 4 | 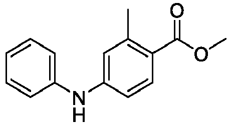 | ¹H-NMR (CDCl₃, 400 MHz) δ: 2.57 (3H, s), 3.85 (3H, s), 5.93 (1H, brs), 6.80-6.85 (2H, m), 7.05 (1H, tt, J = 7.3, 1.2 Hz), 7.16 (2H, dd, J = 7.9, 1.2 Hz), 7.33 (2H, dd, J = 7.9, 7.3 Hz), 7.88 (1H, d, J = 8.5 Hz). |
| | | HRMS (FI⁺):241.11082 [M]⁺ |
| 3 3 - 5 | 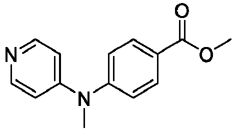 | ¹H-NMR (CDCl₃, 400 MHz) δ: 3.39 (3H, s), 3.93 (3H, s), 6.74 (2H, d, J = 6.7 Hz), 7.27 (2H, d, J = 8.6 Hz), 8.06 (2H, d, J = 8.6 Hz), 8.30 (2H, d, J = 6.7 Hz). |
| | | HRMS (ESI⁺):243.11409 [M+H]⁺ |
| 3 3 - 6 | 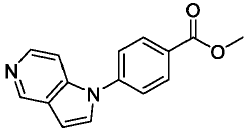 | ¹H-NMR (CDCl₃, 400 MHz) δ: 3.98 (3H, s), 6.83 (1H, d, J = 3.7 Hz), 7.42 (1H, d, J= 3.7 Hz), 7.50 (1H, dd, J = 6.1, 1.2 Hz), 7.58 (2H, d, J = 8.6 Hz), 8.24 (2H, d, J = 8.6 Hz), 8.40 (1H, d, J = 6.1 Hz), 9.01 (1H, d, J = 1.2 Hz). |
| | | HRMS (ESI⁺):253.09770 [M+H]⁺ |
| 3 3 - 7 | 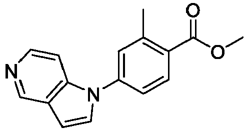 | ¹H-NMR (CDCl₃, 400MHz) δ: 2.71 (3H, s), 3.95 (3H, s), 6.81 (1H, d, J = 4.2 Hz), 7.36-7.43 (3H, m), 7.47-7.51 (1H, m), 8.09-8.14 (1H, m), 8.39 (1H, d, J = 5.4 Hz), 9.00 (1H, d, J = 1.2 Hz). |
| | | MS (ESI⁺): 267.1 [M+H]⁺ |
| 3 3 - 8 | 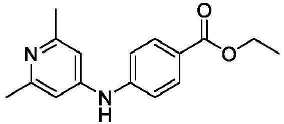 | ¹H-NMR (DMSO-D₆, 400MHz) δ: 1.29 (3H, t, J = 7.3 Hz), 2.31 (6H, s), 4.26 (2H, q, J = 7.3 Hz), 6.73 (2H, s), 7.22 (2H, d, J = 9.1 Hz), 7.88 (2H, d, J = 9.1 Hz), 9.05 (1H, s). |

**[Table 28]**

| Reference Example | Structure | Equipment Data |
|---|---|---|
| 3 3 - 9 | 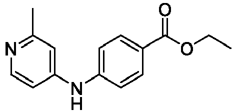 | ¹H-NMR (DMSO-D₆, 400MHz) δ: 1.30 (3H, t, J = 7.3 Hz), 2.36 (3H, s), 4.26 (2H, q, J = 7.3 Hz), 6.86 (1H, dd, J = 5.5, 2.4 Hz), 6.91 (1H, d, J = 2.4 Hz), 7.24 (2H, d, J = 8.6 Hz), 7.88 (2H, d, J = 8.6 Hz), 8.16 (1H, d, J = 5.5 Hz), 9.15 (1H, s). |
| | | MS (ESI⁺): 257.1 [M+H]⁺ |
| 3 3 - 1 0 | 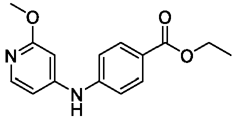 | ¹H-NMR (DMSO-D₆, 400MHz) δ: 1.29 (3H, t, J = 7.3 Hz), 3.79 (3H, s), 4.26 (2H, q, J = 7.3 Hz), 6.41 (1H, d, J = 1.8 Hz), 6.69 (1H, dd, J = 5.5, 1.8 Hz), 7.24 (2H, d, J = 9.2 Hz), 7.89 (2H, d, J = 9.2 Hz), 7.92 (1H, d, J = 5.5 Hz), 9.21 (1H, s). |
| | | MS (ESI⁺): 273.1 [M+H]⁺ |
| 3 3 - 1 1 | 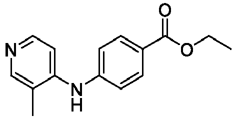 | ¹H-NMR (DMSO-D₆) δ: 1.29 (3H, t, J = 7.3 Hz), 2.21 (3H, s), 4.26 (2H, q, J = 7.3 Hz), 7.15 (1H, d, J = 5.5 Hz), 7.23 (2H, d, J = 9.2 Hz), 7.87 (2H, d, J = 9.2 Hz), 8. 17 (1H, d, J = 5.5 Hz), 8.24 (1H, s), 8.30 (1H, s). |
| | | MS (FI⁺): 256.1 [M]⁺ |
| 3 3 - 1 2 | 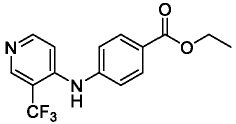 | ¹H-NMR (DMSO-D₆, 400MHz) δ: 1.31 (3H, t, J = 7.3 Hz), 4.29 (2H, q, J = 7.3 Hz), 7.18 (1H, d, J = 5.4 Hz), 7.38 (2H, d, J = 8.5 Hz), 7.94 (2H, d, J = 8.5 Hz), 8.42 (1H, d, J = 5.4 Hz), 8.58 (1H, s), 8.64 (1H, s). |
| | | MS (FI⁺): 310.1 [M]⁺ |
| 3 3 - 1 3 | 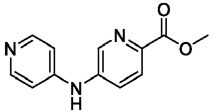 | ¹H-NMR (DMSO-D₆, 400MHz) δ: 3.85 (3H, s), 7.11 (2H, dd, J = 4.9, 1.8 Hz), 7.76 (1H, dd, J = 8.6, 3.1 Hz), 8.00 (1H, d, J = 8.6 Hz), 8.34 (2H, dd, J = 4.9, 1.8 Hz), 8.52 (1H, d, J = 3.1 Hz), 9.47 (1H, s). |
| | | HRMS (ESI⁺) : 230.09315 [M+H]⁺ |
| 3 3 - 1 4 | 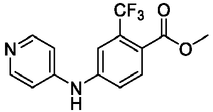 | ¹H-NMR (DMSO-D₆, 400MHz) δ: 3.82 (3H, s), 7.08 (2H, d, J = 6.1 Hz), 7.51 (1H, d, J = 2.4 Hz), 7.56 (1H, dd, J = 8.6, 2.4 Hz), 7.87 (1H, d, J = 8.6 Hz), 8.34 (2H, d, J = 6.1 Hz), 9.48 (1H, s). |
| | | HRMS (ESI⁺) : 297.08453 [M+H]⁺ |

### <Reference example 34-1>

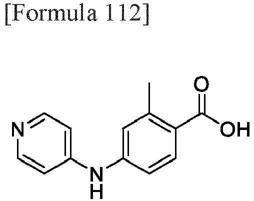

Lithium hydroxide monohydrate (20.5 mg) was added to a solution of methyl 2-methyl-4-(pyridin-4-ylamino)benzoate (79.0 mg) in a methanol-tetrahydrofuran-water (1 : 1 : 1) mixed solution (1.63 mL) under an argon atmosphere and the mixture was stirred at room temperature for 18 hours. After the reaction mixture was concentrated under reduced pressure, 1 mol/L hydrochloric acid was added to adjust the pH to 5. The resulting solid was collected by filtration to obtain 2-methyl-4-(pyridin-4-ylamino)benzoic acid (60.6 mg).

¹H-NMR (DMSO-D₆, 400MHz) δ: 2.52 (3H, s), 7.03 (2H, d, J = 6.1 Hz), 7.05 (1H, d, J = 1.8 Hz), 7.08 (1H, dd, J = 8.5, 1.8 Hz), 7.84 (1H, d, J = 8.5 Hz), 8.27 (2H, d, J = 6.1 Hz), 9.10 (1H, s), 12.44 (1H, brs).

HRMS (ESI⁺): 229.09846 [M+H]⁺

Using the corresponding starting materials and reactants, the following Reference Examples 34-2 to 34-14 were obtained by the same method in Reference Example 34-1 and the method described in Step 25-1 or a method similar thereto.

**[Table 29]**

| Reference Example | Structure | Equipment Data |
|---|---|---|
| 3 4 - 2 | 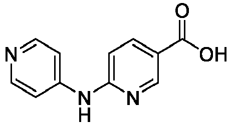 | ¹H-NMR (DMSO-D₆, 400MHz) δ: 7.08 (1H, d, J = 8.6 Hz), 7.89 (2H, d, J = 6.1 Hz), 8.16 (1H, dd, J = 8.6, 1.8 Hz), 8.43 (2H, d, J = 6.1 Hz), 8.83 (1H, d, J = 1.8 Hz), 10.49 (1H, s). |
| | | HRMS (ESI⁺):216.07752 [M+H]⁺ |
| 3 4 - 3 | 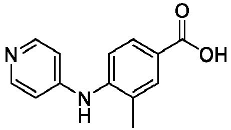 | ¹H-NMR (DMSO-D₆, 400MHz) δ: 2.27 (3H, s), 6.92 (2H, d, J = 6.1 Hz), 7.40 (1H, d, J = 8.6 Hz), 7.81 (1H, dd, J = 8.6, 1.8 Hz), 7.90 (1H, d, J = 1.8 Hz), 8.25 (2H, d, J = 6.1 Hz), 9.33 (1H, s). |
| | | HRMS (ESI⁺):229.09824 [M+H]⁺ |
| 3 4 - 4 | 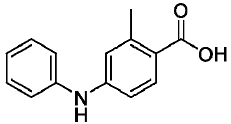 | ¹H-NMR (DMSO-D₆, 400MHz) δ: 2.49 (3H, s), 6.87 (1H, d, J = 2.4 Hz), 6.90 (1H, dd, J = 8.5, 2.4 Hz), 6.96 (1H, tt, J = 7.3, 1.2 Hz), 7.17 (2H, dd, J = 8.5, 1.2 Hz), 7.32 (2H, dd, J = 8.5, 7.3 Hz), 7.78 (1H, d, J = 8.5 Hz), 8.57 (1H, s), 12.15 (1H, brs). |
| | | HRMS (FI⁺):227.09469 [M]⁺ |
| 3 4 - 5 | 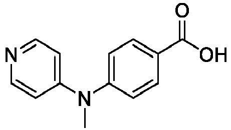 | ¹H-NMR (DMSO-D₆, 400MHz) δ: 3.38 (3H, s), 6.85 (2H, d, J = 6.1 Hz), 7.38 (2H, d, J = 8.6 Hz), 7.99 (2H, d, J = 8.6 Hz), 8.24 (2H, d, J = 6.1 Hz). |
| | | HRMS (ESI⁺):229.09828 [M+H]⁺ |

**[Table 30]**

| Reference Example | Structure | Equipment Data |
|---|---|---|
| 3 4 - 6 | 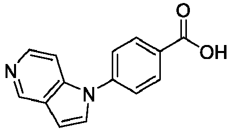 | ¹H-NMR (DMSO-D₆, 400MHz) δ: 6.93 (1H, d, J = 3.1 Hz), 7.65 (1H, d, J = 6.1 Hz), 7.77 (2H, d, J = 8.6 Hz), 7.89 (1H, d, J = 3.1 Hz), 8.14 (2H, d, J = 8.6 Hz), 8.31 (1H, d, J = 6.1 Hz), 8.96 (1H, s). |
| | | HRMS (ESI⁺):239.08266 [M+H]⁺ |
| 3 4 - 7 | 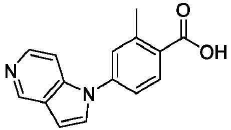 | ¹H-NMR (DMSO-D₆, 400MHz) δ: 2.63 (3H, s), 6.90 (1H, d, J = 2.4 Hz), 7.52-7.66 (3H, m), 7.85 (1H, d, J = 3.0 Hz), 8.03 (1H, d, J = 8.5 Hz), 8.30 (1H, d, J = 5.4 Hz), 8.94 (1H, s). |
| | | MS (ESI⁺): 253.1 [M+H]⁺ |
| 3 4 - 8 | 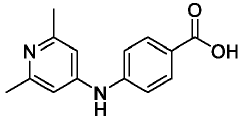 | ¹H-NMR (DMSO-D₆, 400MHz) δ: 2.31 (6H, s), 6.72 (2H, s), 7.20 (2H, d, J = 8.6 Hz), 7.85 (2H, d, J = 8.6 Hz), 9.00 (1H, s). |
| | | MS (EI⁺): 242.1 [M]⁺ |
| 3 4 - 9 | 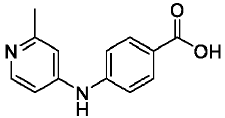 | ¹H-NMR (DMSO-D₆, 400MHz) δ: 2.35 (3H, s), 6.86 (1H, dd, J = 5.5, 2.4 Hz), 6.90 (1H, d, J = 2.4 Hz), 7.22 (2H, d, J = 9.2 Hz), 7.87 (2H, d, J = 9.2 Hz), 8.15 (1H, d, J = 5.5 Hz), 9.11 (1H, s). |
| | | MS (ESI⁺): 229.1 [M+H]⁺ |
| 3 4 - 1 0 | 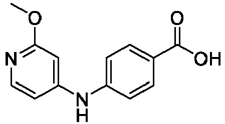 | ¹H-NMR (DMSO-D₆) δ: 3.79 (3H, s), 6.40 (1H, d, J = 1.8 Hz), 6.68 (1H, dd, J = 5.5, 1.8 Hz), 7.21 (2H, d, J = 9.2 Hz), 7.87 (2H, d, J = 9.2 Hz), 7.90 (1H, d, J = 5.5 Hz), 9.16 (1H, s). |
| | | MS (ESI⁺): 245.1 [M+H]⁺ |
| 3 4 - 1 1 | 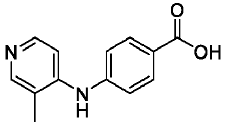 | ¹H-NMR (DMSO-D₆) δ: 2.21 (3H, s), 7.14 (1H, d, J = 5.5 Hz), 7.22 (2H, d, J = 8.6 Hz), 7.86 (2H, d, J = 8.6 Hz), 8.16 (1H, d, J = 5.5 Hz), 8.23 (1H, s), 8.25 (1H, s). |
| | | MS (ESI⁺): 229.1 [M+H]⁺ |
| 3 4 - 1 2 | 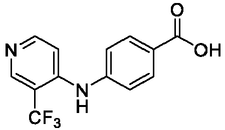 | ¹H-NMR (DMSO-D₆, 400MHz) δ: 7.16 (1H, d, J = 6.1 Hz), 7.36 (2H, d, J = 8.5 Hz), 7.92 (2H, d, J = 8.5 Hz), 8.41 (1H, d, J = 6.1 Hz), 8.53 (1H, s), 8.63 (1H, s), 12.85 (1H, brs). |
| | | MS (EI⁺): 282.1 [M]⁺ |
| 3 4 - 1 3 | 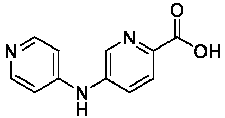 | ¹H-NMR (DMSO-D₆, 400MHz) δ: 7.10 (2H, d, J = 6.1 Hz), 7.75 (1H, dd, J = 8.6, 2.4 Hz), 7.99 (1H, d, J = 8.6 Hz), 8.33 (2H, d, J = 6.1 Hz), 8.51 (1H, d, J = 2.4 Hz), 9.43 (1H, s). |
| | | HRMS (ESI⁺) : 216.07714 [M+H]⁺ |

**[Table 31]**

| Reference Example | Structure | Equipment Data |
|---|---|---|
| 3 4 - 1 4 | 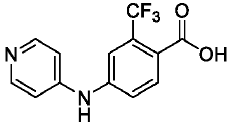 | ¹H-NMR (DMSO-D₆, 400MHz) δ: 7.06 (2H, d, J = 6.1 Hz), 7.48 (1H, d, J = 2.4 Hz), 7.53 (1H, dd, J = 8.6, 2.4 Hz), 7.86 (1H, d, J = 8.6 Hz), 8.32 (2H, d, J = 6.1 Hz), 9.44 (1H, s). HRMS (ESI⁺) : 283.07017 [M+H]⁺ |

### <Reference example 35>

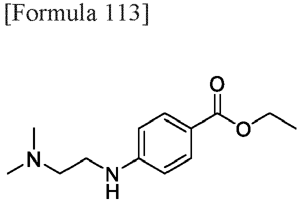

Under an argon atmosphere, potassium carbonate (1.66 g) was added to a solution of N,N-dimethylethane-1,2-diamine (1.31 mL) and ethyl 4-fluorobenzoate (0.877 mL) in dimethyl sulfoxide (10.0 mL) at room temperature and stirred at 80 °C for 25.5 hours. The reaction mixture was added to water and extracted 3 times with ethyl acetate. The combined organic layer was washed with water and saturated brine, dried over anhydrous sodium sulfate, and concentrated under reduced pressure after filtration of the insoluble material. The residue was purified by amino-silica gel column chromatography (hexane : ethyl acetate = 9 : 1 to 0 : 1) to obtain ethyl 4-[2-(dimethylamino)ethylamino]benzoate (510 mg).

¹H-NMR (CDCl₃, 400MHz) δ: 1.36 (3H, t, J = 7.3 Hz), 2.25 (6H, s), 2.53-2.59 (2H, m), 3.15-3.21 (2H, m), 4.31 (2H, q, J = 7.3 Hz), 4.71-4.83 (1H, br s), 6.56 (2H, d, J = 8.6 Hz), 7.87 (2H, d, J = 8.6 Hz).

### <Reference example 36>

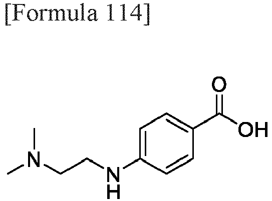

Lithium hydroxide (4.30 mg) was added to a solution of Ethyl 4-[2-(dimethylamino) ethylamino]benzoate (35.4 mg) in ethanol (0.250 mL) and water (0.250 mL) at room temperature. The reaction mixture was stirred at the same temperature for 15 hours, at 50 °C for 2.5 hours, and then at 70 °C for 5 hours. 1 mol/L hydrochloric acid was added to the mixture to adjust the pH to 1. After concentrating under reduced pressure, the residue was used in Example 6-66 as a hydrochloride salt of 4-[2-(dimethylamino)ethylamino]benzoic acid without further purification.

### <Reference example 37>

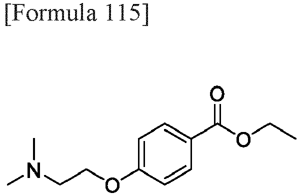

2-Chloro-N,N-dimethylethanamine hydrochloride was added to a solution of ethyl 4-hydroxybenzoate (332 mg) and potassium carbonate (967 mg) in N,N-dimethylformamide (4.00 mL) at room temperature under an argon atmosphere. The reaction mixture was stirred at 110 °C for 29 hours. The mixture was added to water, extracted twice with ethyl acetate. The combined organic layer was washed with water and saturated brine, dried over anhydrous sodium sulfate, and concentrated under reduced pressure after filtration of the insoluble material. The residue was purified by amino-silica gel column chromatography (hexane : ethyl acetate = 9 : 1 to 1 : 2) to obtain ethyl 4-[2-(dimethylamino)ethoxy]benzoate (398 mg).

¹H-NMR (CDCl₃, 400MHz) δ: 1.38 (3H, t, J = 7.3 Hz), 2.34 (6H, s), 2.75 (2H, t, J = 5.5 Hz), 4.11 (2H, t, J = 5.5 Hz), 4.34 (2H, q, J = 7.3 Hz), 6.93 (2H, d, J = 8.6 Hz), 7.99 (2H, d, J = 8.6 Hz).

### <Reference example 38>

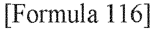

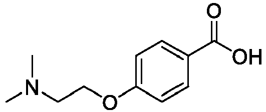

Using ethyl 4-[2-(dimethylamino)ethoxy]benzoate, the title compound was synthesized in the same manner in Reference Example 36, and the crude product was used in the next step without purification.

### <Reference example 39>

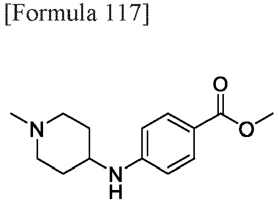

Under an argon atmosphere, acetic acid (0.755 mL) was added to a solution of 1-methylpiperidin-4-one (1.00 g) and methyl 4-aminobenzoate (0.917 mL) in dichloromethane (30.0 mL) at room temperature, and the mixture was stirred for 15 minutes, then sodium triacetoxyborohydride (4.22 g) was added. After stirring for 25 hours, the reaction mixture was added to saturated aqueous sodium hydrogen carbonate solution and extracted twice with ethyl acetate. The combined organic layer was washed with water, saturated brine, dried over anhydrous sodium sulfate, and concentrated under reduced pressure after filtration of the insoluble material. The residue was purified by amino-silica gel column chromatography (ethyl acetate) to obtain methyl 4-[(1-methylpiperidin-4-yl)amino]benzoate (705 mg).

¹H-NMR (DMSO-D₆, 400MHz) δ: 1.32-1.46 (2H, m), 1.79-1.89 (2H, m), 1.93-2.04 (2H, m), 2.15 (3H, s), 2.65-2.76 (2H, m), 3.15-3.29 (1H, m), 3.72 (3H, s), 6.38 (1H, d, J = 7.3 Hz), 6.57 (2H, d, J = 9.1 Hz), 7.65 (2H, d, J = 9.1 Hz).

MS (FI⁺): 248.2 [M]⁺

### <Reference example 40>

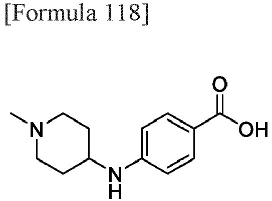

Lithium hydroxide (68.0 mg) was added to a solution of methyl 4-[(1-methylpiperidin-4-yl)amino]benzoate (705 mg) in methanol (7.50 mL) and water (7.50 mL) at room temperature, and the mixture was stirred at the same temperature for 18 hours, at 40 °C for 7 hours, and at 60 °C for 2.5 hours. The reaction mixture was washed with ethyl acetate, 1 mol/L hydrochloric acid was added to the aqueous layer to adjust the pH to 1. The mixture was concentrated under reduced pressure, the residue was used in Example 6-59 as a hydrochloride salt of 4-[(1-methylpiperidin-4-yl)amino]benzoic acid.

### <Reference example 41>

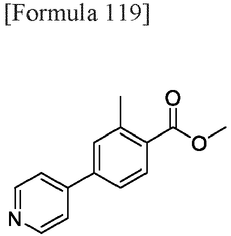

Under an argon atmosphere, pyridine-4-ylboronic acid (241 mg), bis(triphenylphosphine)palladium (II) dichloride (91.9 mg) and 2 mol/L aqueous sodium carbonate solution (1.97 mL) were added to a solution of methyl 4-bromo-2-methylbenzoate (300 mg) in 1,2-dimethoxyethane (6.55 mL), and the mixture was stirred at 60 °C for 8 hours. Water was added and the reaction mixture was extracted with ethyl acetate. The organic layer was washed with saturated brine, dried over anhydrous sodium sulfate, and concentrated after filtration of the insoluble material. The residue was purified by silica gel column chromatography (hexane : ethyl acetate = 3 : 2) to obtain methyl 2-methyl-4-pyridin-4-ylbenzoate (215 mg).

¹H-NMR (CDCl₃, 400MHz) δ: 2.69 (3H, s), 3.93 (3H, s), 7.49-7.53 (4H, m), 8.03 (1H, d, J = 8.5 Hz), 8.69 (2H, d, J = 6.1 Hz).

HRMS (ESI⁺): 228.10273 [M+H]⁺

### <Reference example 42>

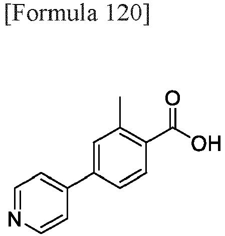

Lithium hydroxide monohydrate (59.6 mg) was added to a solution of methyl 2-methyl-4-pyridin-4-ylbenzoate (215 mg) in methanol-tetrahydrofuran-water (1 : 1: 1) mixed solution (4.74 mL) under an argon atmosphere. The reaction mixture was stirred at room temperature for 7 hours. After the mixture was concentrated under reduced pressure, 1 mol/L hydrochloric acid was added to adjust the pH to 5, the resulting solid was collected by filtration to obtain 2-methyl-4-pyridin-4-ylbenzoic acid (164 mg).

¹H-NMR (DMSO-D₆, 400MHz) δ: 2.62 (3H, s), 7.72 (1H, dd, J = 7.9, 1.8 Hz), 7.75-7.78 (3H, m), 7.95 (1H, d, J = 7.9 Hz), 8.67 (2H, d, J = 6.1 Hz), 12.98 (1H, brs).

HRMS (ESI⁺): 214.08724 [M+H]⁺

### <Reference example 43-1>

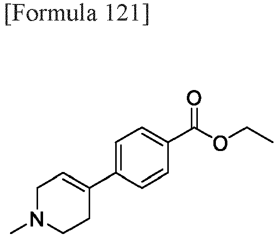

Under an argon atmosphere, potassium carbonate (929 mg), tetrakis(triphenylphosphine)palladium (0) (518 mg), 1-methyl-4-(4,4,5,5-tetramethyl-1,3,2-dioxaborolan-2-yl)-3,6-dihydro-2H-pyridine (1. 00 g) were added to a solution of ethyl 4-bromobenzoate (0.718 mL) in 1,4-dioxane (15.0 mL), and the mixture was stirred at 110 °C for 22 hours. The reaction mixture was added to water and extracted twice with ethyl acetate. The combined organic layer was washed with water and saturated brine, dried over anhydrous sodium sulfate, and concentrated under reduced pressure after filtration of the insoluble material. The residue was purified by amino-silica gel column chromatography (hexane : ethyl acetate = 9 : 1 to 1 : 2) to obtain ethyl 4-(1-methyl-3,6-dihydro-2H-pyridin-4-yl)benzoate (1.16 g).

¹H-NMR (CDCl₃, 400MHz) δ: 1.39 (3H, t, J = 6.7 Hz), 2.42 (3H, s), 2.56-2.64 (2H, m), 2.65-2.71 (2H, m), 3.12-3.16 (2H, m), 4.37 (2H, q, J = 6.7 Hz), 6.17-6.22 (1H, m), 7.44 (2H, d, J = 8.6 Hz), 7.99 (2H, d, J = 8.6 Hz)

Using the corresponding starting materials and reactants, the following Reference Example 43-2 was obtained by the same method in Reference Example 43-1, the method described in Step 31-1 or a method similar thereto.

**[Table 32]**

| Reference Example | Structure | Equipment Data |
|---|---|---|
| 4 3 - 2 | 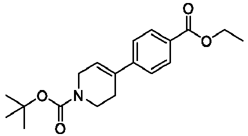 | ¹H-NMR (CDCl₃, 400MHz) δ: 1.40 (3H, t, J = 7.3 Hz), 1.50 (9H, s), 2.48-2.60 (2H, m), 3.65 (2H, t, J = 6.1 Hz), 4.06-4.14 (2H, m), 4.38 (2H, q, J = 7.3 Hz), 6.16 (1H, br s), 7.43 (2H, d, J = 8.6 Hz), 8.01 (2H, d, J = 8.6 Hz). |

### <Reference example 44-1>

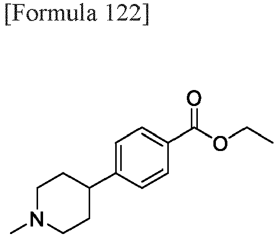

Under an argon atmosphere, 10% palladium carbon (60.0 mg) was added to a solution of ethyl 4-(1-methyl-3,6-dihydro-2H-pyridin-4-yl)benzoate (300 mg) in ethanol (6.00 mL). The reaction mixture was stirred at room temperature under a hydrogen atmosphere for 4 hours, and then the atmosphere was replaced with argon. After the mixture was filtered through Celite, the filtrate was concentrated under reduced pressure and the residue was purified by amino-silica gel column chromatography (hexane : ethyl acetate = 5 : 1 to 0 : 1) to obtain ethyl 4-(1-methylpiperidin-4-yl)benzoate (295 mg).

¹H-NMR (CDCl₃, 400MHz) δ: 1.38 (3H, t, J = 7.3 Hz), 1.75-1.88 (4H, m), 2.00-2.10 (2H, m), 2.33 (3H, s), 2.47-2.59 (1H, m), 2.94-3.02 (2H, m), 4.36 (2H, q, J = 7.3 Hz), 7.29 (2H, d, J = 8.6 Hz), 7.98 (2H, d, J = 8.6 Hz).

Using the corresponding starting materials and reactants, the following Reference Example 44-2 was obtained by the same method as in Reference Example 44-1, the method described in Step 31-2 or a method similar thereto.

**[Table 33]**

| Reference Example | Structure | Equipment Data |
|---|---|---|
| 4 4 - 2 | 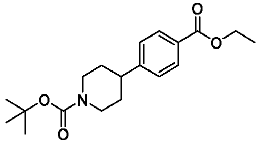 | ¹H-NMR (CDCl₃, 400MHz) δ: 1.38 (3H, t, J = 6.7 Hz), 1.48 (9H, s), 1.56-1.70 (2H, m), 1.77-1.88 (2H, m), 2.63-2.92 (3H, m), 4.26 (2H, br s), 4.37 (2H, q, J = 6.7 Hz), 7.27 (2H, d, J = 8.6 Hz), 7.98 (2H, d, J = 8.6 Hz). |

### <Reference example 45>

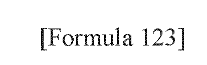

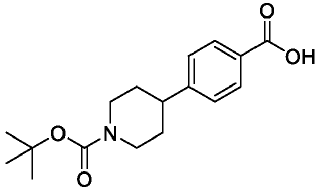

Lithium hydroxide (15.1 mg) was added to a solution of tert-butyl 4-(4-ethoxycarbonylphenyl)piperidin-1-carboxylate (140 mg) in ethanol (1.00 mL) and water (1.00 mL) and the mixture was stirred for 22 hours. 1 mol/L hydrochloric acid was added to the reaction mixture to adjust the pH to 3, the mixture was extracted twice with ethyl acetate. The combined organic layer was washed with water and saturated brine, dried over anhydrous sodium sulfate, and concentrated under reduced pressure after filtration of the insoluble material. The residue was washed with diisopropyl ether to obtain 4-[1-[(2-methylpropan-2-yl)oxycarbonyl]piperidin-4-yl]benzoic acid (97.0 mg).

¹H-NMR (DMSO-D₆, 400MHz) δ: 1.40 (9H, s), 1.42-1.57 (2H, m), 1.69-1.81 (2H, m), 2.69-2.93 (3H, m), 3.96-4.17 (2H, m), 7.36 (2H, d, J = 8.6 Hz), 7.86 (2H, d, J = 8.6 Hz), 12.80 (1H, br s).

### <Reference example 46>

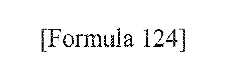

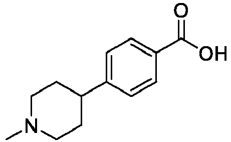

Lithium hydroxide (4.31 mg) was added to a solution of ethyl 4-(1-methylpiperidin-4-yl)benzoate (37.1 mg) in ethanol (0.250 mL) and water (0.250 mL) at room temperature, and the mixture was stirred for 18 hours. 1 mol/L hydrochloric acid was added to the reaction mixture to adjust the pH to 1. After concentrating under reduced pressure, the residue was used in Example 6-69 as a hydrochloride of 4-(1-methylpiperidin-4-yl)benzoic acid without further purification.

### <Reference example 47>

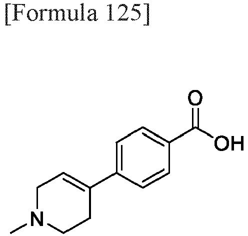

Lithium hydroxide (4.31 mg) was added to a solution of ethyl 4-(1-methyl-3,6-dihydro-2H-pyridin-4-yl)benzoate (36.8 mg) in ethanol (0.250 mL) and water (0.250 mL). The reaction mixture was stirred at room temperature for 44 hours, and at 70 °C for 20 hours. 1 mol/L hydrochloric acid was added to the mixture to adjust pH 1. After the mixture was concentrated under reduced pressure, the residue was used in Example 6-68 as a hydrochloride salt of 4-(1-methyl-3,6-dihydro-2H-pyridin-4-yl)benzoic acid without further purification.

### <Reference example 48>

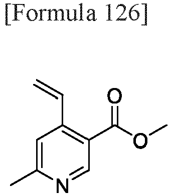

Under an argon atmosphere, [1,1'-Bis(diphenylphosphino)ferrocene]palladium (II) dichloride dichloromethane complex (154 mg) was added to a solution of methyl 4-chloro-6-methylpyridine-3-carboxylate (700 mg), potassium vinyl trifluoroborate (556 mg) and cesium carbonate (3.68 g) in tetrahydrofuran (15.0 mL)-water (3.00 mL) at room temperature. The reaction mixture was heated and stirred for 14.5 hours under reflux condition. The mixture was filtered through Celite to remove the insoluble material, then water was added, and extracted twice with ethyl acetate. The combined organic layer was washed with water and saturated brine, dried over anhydrous sodium sulfate, and concentrated under reduced pressure after filtration of the insoluble material. The residue was purified by silica gel column chromatography (hexane : ethyl acetate = 1 : 0 to 3 : 1) to obtain methyl 4-ethenyl-6-methylpyridine-3-carboxylate (345 mg).

¹H-NMR (CDCl₃, 400MHz) δ: 2.61 (3H, s), 3.92 (3H, s), 5.53 (1H, dd, J = 10.9, 1.2 Hz), 5.83 (1H, dd, J = 17.6, 1.2 Hz), 7.32 (1H, s), 7.51 (1H, dd, J = 17.6, 10.9 Hz), 8.99 (1H, s).

MS (ESI⁺): 178.1 [M+H]⁺

### <Reference example 49>

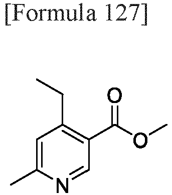

Under an argon atmosphere, 10% palladium carbon (34.5 mg) was added to a solution of methyl 4-ethenyl-6-methylpyridine-3-carboxylate (345 mg) in methanol (10.0 mL) at room temperature. The reaction mixture was stirred under a hydrogen atmosphere for 1.5 hours, and then the atmosphere was replaced with argon. After the mixture was filtered through Celite, the filtrate was concentrated under reduced pressure to obtain methyl 4-ethyl-6-methylpyridine-3-carboxylate (349 mg).

¹H-NMR (CDCl₃, 400MHz) δ: 1.24 (3H, t, J = 7.3 Hz), 2.58 (3H, s), 2.99 (2H, q, J = 7.3 Hz), 3.92 (3H, s), 7.07 (1H, s), 8.95 (1H, s).

MS (EI⁺): 179.1 [M]⁺

### <Reference example 50>

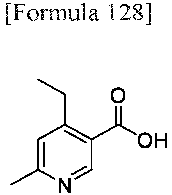

Lithium hydroxide monohydrate (123 mg) was added to a solution of methyl 4-ethyl-6-methylpyridine-3-carboxylate (349 mg) in methanol (5.00 mL) and water (5.00 mL). 123 mg) and the mixture was stirred for 18 hours. 1 mol/L hydrochloric acid was added to the reaction mixture to adjust the pH between 5 and 6, the mixture was concentrated under reduced pressure. The residue was purified by silica gel column chromatography (ethyl acetate : methanol = 4 : 1) to obtain 4-ethyl-6-methylpyridine-3-carboxylic acid (322 mg).

¹H-NMR (DMSO-D₆, 400MHz) δ: 1.17 (3H, t, J = 7.9 Hz), 2.49 (3H, s), 2.94 (2H, q, J = 7.9 Hz), 7.22 (1H, s), 8.79 (1H, s).

MS (ESI⁺): 166.1 [M+H]⁺

### <Reference example 51>

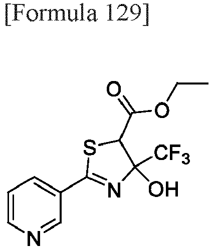

A solution of ethyl 2-chloro-4,4,4-trifluoro-3-oxobutanoate (1.14 mL) and pyridine-3-carbothioamide (1.00 g) in ethanol (10.0 mL) was heated and stirred for 26 hours under reflux condition. The reaction mixture was added to water, and extracted twice with ethyl acetate. The combined organic layer was washed with water and saturated brine, dried over anhydrous sodium sulfate, and concentrated under reduced pressure after filtration of the insoluble material. The residue was purified by silica gel column chromatography (hexane : ethyl acetate = 9 : 1 to 0 : 1) and then washed with diisopropyl ether to obtain ethyl 4-hydroxy-2-pyridin-3-yl-4-(trifluoromethyl)-5H-1,3-thiazole-5-carboxylate (677 mg).

¹H-NMR (CDCl₃, 400MHz) δ: 1.33 (3H, t, J = 7.3 Hz), 4.31 (2H, q, J = 7.1 Hz), 4.90 (1H, s), 7.40-7.43 (1H, m), 8.00 (1H, dt, J = 8.1, 2.0 Hz), 8.67 (1H, dd, J = 4.8, 1.8 Hz), 8.86 (1H, s), 9.54 (1H, d, J = 3.0 Hz).

MS (FI⁺): 320.1 [M]⁺

### <Reference example 52>

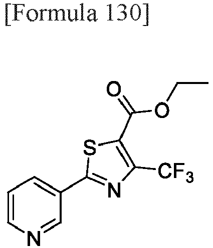

p-Toluenesulfonic acid monohydrate (1.12 g) was added to a solution of ethyl 4-hydroxy-2-pyridin-3-yl-4-(trifluoromethyl)-5H-1,3-thiazole-5-carboxylate (632 mg) in toluene (10.0 mL) at room temperature, and the mixture was heated and stirred for 1 hour under reflux condition. The mixture was added to saturated aqueous sodium hydrogen carbonate solution and extracted twice with ethyl acetate. The combined organic layer was washed with water and saturated brine, dried over anhydrous sodium sulfate, and concentrated under reduced pressure after filtration of the insoluble material. The residue was purified by amino-silica gel column chromatography (ethyl acetate) to obtain ethyl 2-pyridin-3-yl-4-(trifluoromethyl)-1,3-thiazole-5-carboxylate (155 mg).

¹H-NMR (CDCl₃, 400MHz) δ: 1.42 (3H, t, J = 7.3 Hz), 4.44 (2H, q, J = 7.3 Hz), 7.45 (1H, dd, J = 7.3, 4.8 Hz), 8.32 (1H, td, J = 4.8, 2.8 Hz), 8.76 (1H, dd, J = 4.8, 1.8 Hz), 9.19 (1H, d, J = 1.8 Hz).

MS (ESI⁺): 303.0 [M+H]⁺

### <Reference example 53>

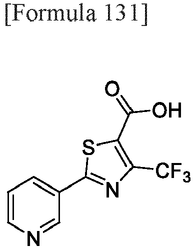

Lithium hydroxide (17.8 mg) was added to a solution of ethyl 2-pyridin-3-yl-4-(trifluoromethyl)-1,3-thiazole-5-carboxylate (150 mg) in ethanol (2.50 mL) and water (2.50 mL) at room temperature, and the mixture was stirred for 30 minutes. The reaction mixture was washed with ethyl acetate, 1 mol/L hydrochloric acid was added to the aqueous layer to adjust the pH to 3 and extracted twice with ethyl acetate. The combined organic layer was washed with water and saturated brine, dried over anhydrous sodium sulfate, and concentrated under reduced pressure after filtration of the insoluble material. The residue was washed with diisopropyl ether to obtain 2-pyridin-3-yl-4-(trifluoromethyl)-1,3-thiazole-5-carboxylic acid (95.0 mg).

¹H-NMR (DMSO-D₆, 400MHz) δ: 7.60 (1H, dt, J = 7.7, 3.2 Hz), 8.40 (1H, dt, J = 8.1, 2.0 Hz), 8.77 (1H, td, J = 5.3, 1.4 Hz), 9.19 (1H, d, J = 2.4 Hz).

MS (ESI⁺): 275.0 [M+H]⁺

### <Reference example 54>

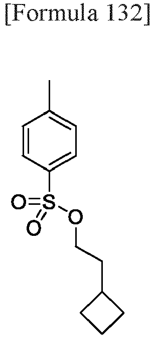

Under an argon atmosphere, pyridine (1.12 mL) and p-toluenesulfonyl chloride (1.76 g) were added to a solution of 2-cyclobutylethanol (926 mg) in dichloromethane (9.25 mL) and the mixture was stirred at room temperature for 26 hours. After water was added, the mixture was extracted with ethyl acetate. The organic layer was washed with 1 mol/L hydrochloric acid and saturated aqueous sodium hydrogen carbonate solution, dried over anhydrous sodium sulfate, and concentrated after filtration of the insoluble material. The residue was purified by amino-silica gel column chromatography (hexane : ethyl acetate = 9 : 1) to obtain 2-cyclobutylethyl 4-methylbenzenesulfonate (1.94 g).

¹H-NMR (CDCl₃, 400MHz) δ: 1.54-1.62 (2H, m), 1.73 (2H, q, J = 6.7 Hz), 1.76-1.87 (2H, m), 1.94-2.02 (2H, m), 2.25-2.38 (1H, m), 2.45 (3H, s), 3.95 (2H, t, J = 6.7 Hz), 7.34 (2H, d, J = 8.5 Hz), 7.78 (2H, d, J = 8.5 Hz).

HRMS (FI⁺): 254.09769 [M]⁺

### <Reference example 55-1>

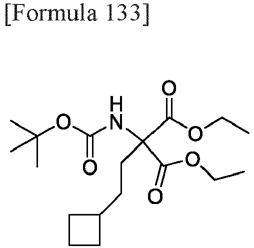

Under an argon atmosphere, potassium tert-butoxide (1.20 g) was added to a solution of diethyl 2-[(2-methylpropan-2-yl)oxycarbonylamino]propanedioate (3.14 g) in N,N-dimethylformamide (19.1 mL), and the mixture was stirred at 70 °C for 30 minutes. After allowing to cool at room temperature, a solution of 2-cyclobutylethyl 4-methylbenzenesulfonate (1.94 g) in N, N-dimethylformamide (19.0 mL) was added. The reaction mixture was stirred at 70 °C for 6 hours, then water was added, and extracted with ethyl acetate. The organic layer was washed with saturated brine, dried over anhydrous sodium sulfate, and concentrated after filtration of the insoluble material. The residue was purified by silica gel column chromatography (hexane : ethyl acetate = 9 : 1) to obtain diethyl 2-(2-cyclobutylethyl)-2-[(2-methylpropan-2-yl)oxcarbonylamino]propanedioate (1.76 g).

¹H-NMR (CDCl₃, 400MHz) δ: 1.19-1.24 (2H, m), 1.25 (6H, t, J = 7.3 Hz), 1.43 (9H, s), 1.50-1.58 (2H, m), 1.74-1.87 (2H, m), 2.00-2.08 (2H, m), 2.12-2.20 (2H, m), 2.20-2.28 (1H, m), 4.18-4.28 (4H, m), 5.91 (1H, brs).

HRMS (ESI⁺): 358.22297 [M+H]⁺

Using the corresponding starting materials and reactants, the following Reference Examples 55-2 to 55-3 were obtained by the same method in Reference Example 55-1, the method described in Step 34-2 or a method similar thereto.

**[Table 34]**

| Reference Example | Structure | Equipment Data |
|---|---|---|
| 5 5 - 2 | 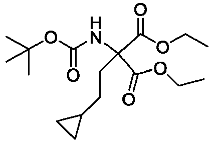 | ¹H-NMR (CDCl₃, 400 MHz) δ: -0.05-0.00 (2H, m), 0.38-0.44 (2H, m), 0.60-0.71 (1H, m), 1.03-1.10 (2H, m), 1.20-1.30 (6H, m), 1.43 (9H, s), 2.36-2.44 (2H, m), 4.15-4.35 (4H, m), 5.89 (1H, brs). HRMS (FI⁺):343.19898 [M]⁺ |
| 5 5 - 3 | 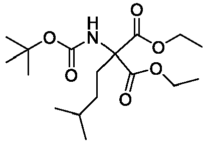 | ¹H-NMR (CDCl₃, 400 MHz) δ: 0.87 (6H, d, J = 6.7 Hz), 0.98-1.06 (2H, m), 1.25 (6H, t, J = 7.3 Hz), 1.43 (9H, s), 1.49-1.57 (1H, m), 2.24-2.30 (2H, m), 4.19-4.27 (4H, m), 5.91 (1H, brs). HRMS (ESI⁺):346.22300 [M+H]⁺ |

### <Reference example 56-1>

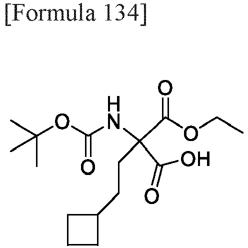

Under an argon atmosphere, 2 mol/L aqueous potassium hydroxide solution (2.71 mL) was added to a solution of diethyl 2-(2-cyclobutylethyl)-2-[(2-methylpropan-2-yl)oxycarbonylamino]propanedioate (1.76 g) in ethanol (24.6 mL), and the mixture was stirred at room temperature for 4 hours. After concentrating, 1 mol/L hydrochloric acid was added to the reaction mixture to adjust the pH to 2, and the mixture was extracted with ethyl acetate. The organic layer was washed with saturated brine, dried over anhydrous sodium sulfate, and concentrated after filtration to obtain 4-cyclobutyl-2-ethoxycarbonyl-2-[(2-methylpropan-2-yl)oxycarbonylamino]butanoic acid (1.64 g).

¹H-NMR (DMSO-D₆, 400MHz) δ: 1.14 (3H, t, J = 7.3 Hz), 1.19-1.24 (2H, m), 1.37 (9H, s), 1.43-1.52 (2H, m), 1.71-1.84 (2H, m), 1.90-2.01 (4H, m), 2.12-2.22 (1H, m), 4.06-4.18 (2H, m), 6.38 (1H, brs).

HRMS (ESI⁺): 330.19229 [M+H]⁺

Using the corresponding starting materials and reactants, the following Reference Examples 56-2 to 56-3 were obtained by the same method in Reference Example 56-1, the method described in Step 34-3, or a method similar thereto.

**[Table 35]**

| Reference Example | Structure | Equipment Data |
|---|---|---|
| 5 6 - 2 | 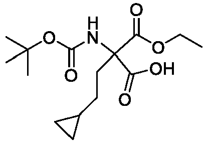 | ¹H-NMR (CDCl₃, 400 MHz) δ: -0.01-0.03 (2H, m), 0.40-0.45 (2H, m), 0.61-0.70 (1H, m), 1.08-1.20 (2H, m), 1.30 (3H, t, J = 7.3 Hz), 1.43 (9H, s), 2.30-2.36 (2H, m), 4.20-4.35 (2H, m), 5.75 (1H, brs). HRMS (ESI⁻):314.15966 [M-H]⁻ |
| 5 6 - 3 | 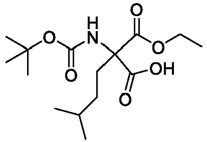 | ¹H-NMR (DMSO-D₆, 400MHz) δ: 0.82 (6H, dd, J = 6.7, 1.8 Hz), 0.89-1.07 (2H, m), 1.14 (3H, t, J = 7.3 Hz), 1.35 (9H, s), 1.41-1.52 (1H, m), 2.01-2.13 (2H, m), 4.04-4.15 (2H, m), 6.35 (1H, brs). HRMS (ESI⁺):318.19171 [M+H]⁺ |

### <Reference example 57-1>

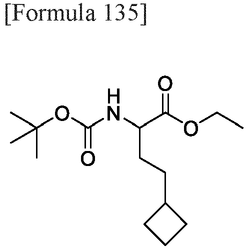

Under an argon atmosphere, a solution of 4-cyclobutyl-2-ethoxycarbonyl-2-[(2-methylpropan-2-yl)oxycarbonylamino]butanoic acid (1.62 g) in 1,2-dichlorobenzene (9.84 mL) was stirred at 110 °C for 1 hour. After allowing to cool at room temperature, the reaction mixture was purified by silica gel column chromatography (hexane : ethyl acetate = 1 : 0 - 4 : 1) to obtain ethyl 4-cyclobutyl-2-[(2-methylpropan-2-yl)oxycarbonylamino]butanoate (1.27 g).

¹H-NMR (CDCl₃, 400MHz) δ: 1.28 (3H, t, J = 7.3 Hz), 1.36-1.43 (2H, m), 1.45 (9H, s), 1.47-1.62 (4H, m), 1.65-1.74 (1H, m), 1.76-1.88 (2H, m), 1.99-2.07 (2H, m), 2.19-2.27 (1H, m), 4.14-4.26 (2H, m), 4.98 (1H, d, J = 7.9 Hz).

HRMS (FI⁺): 286.20177 [M]⁺

Using the corresponding starting materials and reactants, the following Reference Examples 57-2 to 57-3 were obtained by the same method in Reference Example 57-1, the method described in Step 34-4, or a method similar thereto.

**[Table 36]**

| Reference Example | Structure | Equipment Data |
|---|---|---|
| 5 7 - 2 | 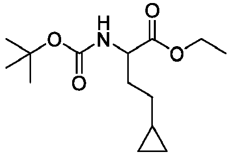 | ¹H-NMR (CDCl₃, 400 MHz) δ: -0.02-0.04 (2H, m), 0.40-0.45 (2H, m), 0.61-0.72 (1H, m), 1.20-1.30 (5H, m), 1.44 (9H, s), 1.67-1.77 (1H, m), 1.86-1.96 (1H, m), 4.16-4.23 (2H, m), 4.26-4.33 (1H, m), 4.98 (1H, d, J = 7.3 Hz). HRMS (FI⁺):272.18690 [M+H]⁺ |
| 5 7 - 3 | 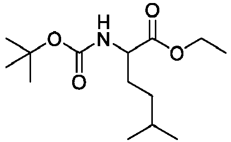 | ¹H-NMR (CDCl₃, 400 MHz) δ:0.87 (3H, d, J = 6.7 Hz), 0.88 (3H, d, J = 6.7 Hz), 1.17-1.25 (2H, m), 1.28 (3H, t, J = 7.3 Hz), 1.45 (9H, s), 1.49-1.55 (1H, m), 1.58-1.67 (1H, m), 1.74-1.85 (1H, m), 4.15-4.22 (2H, m), 4.22-4.27 (1H, m), 4.99 (1H, d, J = 7.3 Hz). HRMS (ESI⁺):274.20250 [M+H]⁺ |

### <Reference example 58-1>

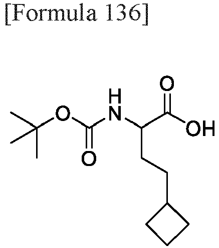

Under argon atmosphere, 2 mol/L aqueous potassium hydroxide solution (3.34 mL) was added to ethyl 4-cyclobutyl-2-[(2-methylpropan-2-yl)oxycarbonylamino]butanoate (1.27 g) in ethanol (14.8 mL), and the mixture was stirred at room temperature for 16 hours. After concentration, 1 mol/L hydrochloric acid was added to the reaction mixture to adjust the pH to 2, and the resulting solid was collected by filtration to obtain 4-cyclobutyl-2-[(2-methylpropan-2-yl)oxycarbonylamino]butyric acid (1.02 g).

¹H-NMR (DMSO-D₆, 400MHz) δ: 1.32-1.48 (2H, m), 1.38 (9H, s), 1.48-1.62 (4H, m), 1.72-1.84 (2H, m), 1.92-2.02 (2H, m), 2.14-2.23 (1H, m), 3.79 (1H, td, J = 7.9, 4.8 Hz), 7.00 (1H, d, J = 7.9 Hz).

HRMS (FI⁺): 258.16971 [M+H]⁺

Using the corresponding starting materials and reactants, the following Reference Examples 58-2 to 58-3 were obtained by the same method in Reference Example 58-1, the method described in Step 34-5, or a method similar thereto.

**[Table 37]**

| Reference Example | Structure | Equipment Data |
|---|---|---|
| 5 8 - 2 | 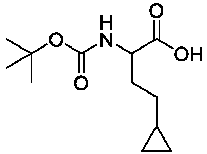 | ¹H-NMR (DMSO-D₆, 400MHz) δ: -0.02-0.02 (2H, m), 0.31-0.40 (2H, m), 0.57-0.67 (1H, m), 1.15-1.25 (2H, m), 1.37 (9H, s), 1.56-1.67 (1H, m), 1.67-1.78 (1H, m), 3.82-3.90 (1H, m), 6.96 (1H, d, J = 7.9 Hz). HRMS (ESI⁻):242.13830 [M-H]⁻ |
| 5 8 - 3 | 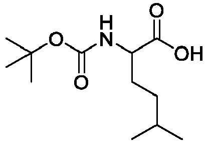 | ¹H-NMR (DMSO-D₆, 400MHz) δ: 0.82 (3H, d, J = 6.7 Hz), 0.83 (3H, d, J = 6.7 Hz), 1.12-1.22 (2H, m), 1.36 (9H, s), 1.44-1.64 (3H, m), 3.80 (1H, td, J = 7.9, 4.8 Hz), 6.98 (1H, d, J = 7.9 Hz). HRMS (ESI⁺):246.17049 [M+H]⁺ |

### <Reference example 59>

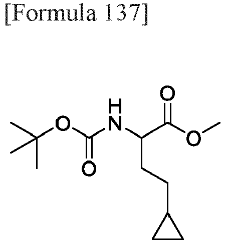

2.0 mol/L Trimethylsilyldiazomethane-diethyl ether solution (0.615 mL) was added dropwise to a solution of 4-cyclopropyl-2-[(2-methylpropan-2-yl)oxycarbonylamino]butanoic acid (200 mg) in a mixture of methanol (4.00 mL) and toluene (4.00 mL) at room temperature, and the mixture was stirred for 20 minutes. Acetic acid was added to the reaction mixture until the stopped foaming and disappearance of the yellow color. The mixture was concentrated under reduced pressure, and the residue was used in reference example 60 and reference example 66 as methyl 4-cyclopropyl-2-[(2-methylpropan-2-yl)oxycarbonylamino]butanoate without further purification.

### <Reference example 60>

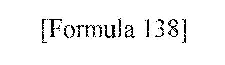

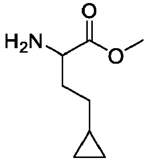

Under argon atmosphere, trifluoroacetic acid (4.00 mL) was added to a solution of methyl 4-cyclopropyl-2-[(2-methylpropan-2-yl)oxycarbonylamino]butanoate obtained as a residue of Reference Example 59 in dichloromethane (4.00 mL) at room temperature, and the mixture was stirred for 20 minutes. The reaction mixture was concentrated under reduced pressure, and the residue was used in reference example 65-6 as trifluoroacetate of methyl 2-amino-4-cyclopropylbutanoate without further purification.

### <Reference example 61>

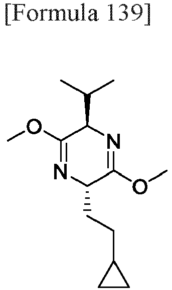

Under argon atmosphere, n-butyllithium (2.55 mL, 1.63 mol/L, n-hexane solution) was added slowly to a solution of (2R)-3,6-dimethoxy-2-propan-2-yl-2,5-dihydropyrazine (766 mg) in tetrahydrofuran (13.9 mL) at -78 °C, and the mixture was stirred at -78 °C for 15 minutes. Then, a solution of 2-cyclopropylethyl 4-methylbenzenesulfonate (1.00 g) in tetrahydrofuran (6.94 mL) was slowly added to a mixture. After stirring at -78 °C for 1 hour, the reaction mixture was gradually raised to room temperature and stirred for 16 hours. After a saturated aqueous ammonium chloride solution was added, the mixture was extracted with ethyl acetate. The organic layer was washed with saturated brine, dried over anhydrous sodium sulfate, and concentrated under reduced pressure after filtration of the insoluble material. The residue was purified by silica gel column chromatography (hexane : ethyl acetate = 30 : 1) to obtain (2S,5R)-2- (2-cyclopropylethyl)-3,6-dimethoxy-5-propan-2-yl-2,5-dihydropyrazine (555 mg).

¹H-NMR (CDCl₃, 400MHz) δ: 0.00-0.04 (2H, m), 0.38 (1H, d, J = 1.8 Hz), 0.40 (1H, d, J = 1.8 Hz), 0.59-0.67 (1H, 1H, m), 0.69 (3H, d, J = 6.7 Hz), 1.05 (3H, d, J = 6.7 Hz), 1.08-1.26 (2H, m), 1.75-1.84 (1H, m), 1.88-1.97 (1H, m), 2.22-2.30 (1H, m), 3.67 (3H, s), 3.69 (3H, s), 3.92 (1H, dd, J = 3.6, 3.0 Hz), 4.04 (1H, td, J = 6.7, 3.0 Hz).

HRMS (ESI⁺): 253.19083 [M+H]⁺

### <Reference example 62>

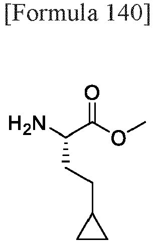

Under argon atmosphere, 0.5 mol/L hydrochloric acid (11.0 mL) was added to a solution of (2S,5R)-2-(2-cyclopropylethyl)-3,6-dimethoxy-5-propan-2-yl-2,5-dihydropyrazine (555 mg) in tetrahydrofuran (11.0 mL), and the mixture was stirred at room temperature for 5 hours. A 25% aqueous ammonium solution was added to the reaction mixture to adjust the pH to 7, and the mixture was extracted with ethyl acetate. The organic layer was washed with saturated brine, dried over anhydrous sodium sulfate, and concentrated under reduced pressure after filtration of the insoluble material, to give methyl (2S)-2-amino-4-cyclopropylbutanoate. The obtained crude product was used in Reference Example 63 without further purification.

### <Reference example 63>

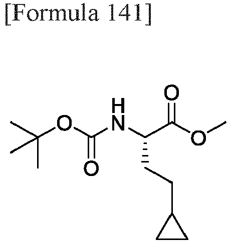

Under argon atmosphere, di-tert-butyl-dicarbonate (1.06 g) and sodium carbonate (700 mg) were added to a solution of methyl (2S)-2-amino-4-cyclopropylbutanoate and impurities in a mixture of ethyl acetate-water (11.0 mL, 1 : 1) under ice cooling condition, and the mixture was stirred at room temperature for 1 hour. After water was added, the mixture was extracted with ethyl acetate. The organic layer was washed with water and saturated brine, dried over anhydrous sodium sulfate, and concentrated under reduced pressure after filtration of the insoluble material. The residue was purified by silica gel column chromatography

(hexane : ethyl acetate = 9 : 1) to obtain methyl (2S)-4-cyclopropyl-2-[(2-methylpropan-2-yl)oxycarbonylamino]butanoate (490 mg).

¹H-NMR (CDCl₃, 400MHz) δ: 0.00-0.04 (2H, m), 0.40-0.45 (2H, m), 0.61-0.71 (1H, m), 1.21-1.29 (2H, m), 1.44 (9H, s), 1.67-1.77 (1H, m), 1.86-1.96 (1H, m), 3.74 (3H, s), 4.25-4.38 (1H, m), 4.90-5.10 (1H, m).

HRMS (ESI⁺): 258.17006 [M+H]⁺

### <Reference example 64>

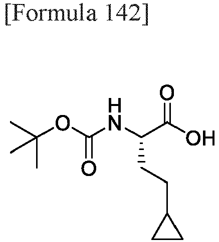

Under argon atmosphere, lithium hydroxide monohydrate (120 mg) was added to a solution of methyl (2S)-4-cyclopropyl-2-[(2-methylpropan-2-yl)oxycarbonylamino]butanoate (490 mg) in a methanol-tetrahydrofuran-water (3 : 3 : 1) mixed solution (9.52 mL) under ice cooling condition, and the mixture was stirred at room temperature for 1 hour. The reaction mixture was concentrated under reduced pressure, 1 mol/L hydrochloric acid was added to adjust the pH to 2, and the mixture was extracted with ethyl acetate. The organic layer was washed with water and saturated brine, dried over anhydrous sodium sulfate, and concentrated under reduced pressure after filtration of the insoluble material to obtain (2S)-4-cyclopropyl-2-[(2-methylpropan-2-yl)oxycarbonylamino]butanoic acid (418 mg).

¹H-NMR (DMSO-d₆, 400MHz) δ: 0.00-0.02 (2H, m), 0.32-0.41 (2H, m), 0.59-0.67 (1H, m), 1.18-1.26 (2H, m), 1.37 (9H, s), 1.58-1.68 (1H, m), 1.69-1.78 (1H, m), 3.85-3.92 (1H, m), 7.04 (1H, d, J = 7.9 Hz), 12.38 (1H, brs).

HRMS (ESI⁺): 244.15499 [M+H]⁺

### <Reference example 65-1>

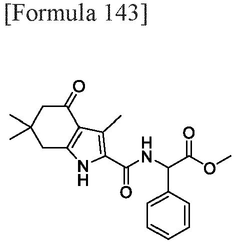

Under argon atmosphere, 1-hydroxybenzotriazole monohydrate (165 mg), 1-ethyl-3-(3-dimethylaminopropyl)carbodiimide hydrochloride (191 mg) and N,N-diisopropylethylamine (0.616 mL) were added to a solution of 3,6,6-trimethyl-4-oxo-5,7-dihydro-1H-indole-2-carboxylic acid (200 mg) and methyl 2-amino-2-phenylacetate hydrochloride (182 mg) in N,N-dimethylformamide (4.50 mL) at room temperature, and the mixture was stirred for 14 hours. The reaction mixture was added to water, the resulting insoluble material was collected by filtration, and then washed with diisopropyl ether to obtain methyl 2-phenyl-2-[(3,6,6-trimethyl-4-oxo-5,7-dihydro-1H-indole-2-carbonyl)amino]acetate (296 mg).

¹H-NMR (DMSO-d₆, 400MHz) δ: 1.03 (3H, s), 1.04 (3H, s), 2.24 (2H, s), 2.49 (3H, s), 2.65 (2H, s), 3.68 (3H, s), 5.57 (1H, d, J = 6.7 Hz), 7.37-7.51 (5H, m), 8.20 (1H, d, J = 6.7 Hz), 11.71 (1H, s).

MS (ESI⁺): 369.2 [M+H]⁺

Using the corresponding starting materials and reactants, the following Reference Examples 65-2 to 65-6 were obtained by the same method in Reference Example 65-1, the method described in Step 1-1, or a method similar thereto.

**[Table 38]**

| Reference Example | Structure | Equipment Data |
|---|---|---|
| 6 5 - 2 | 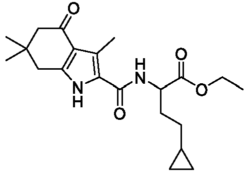 | ¹H-NMR (CDCl₃, 400 MHz) δ: 0.00-0.04 (2H, m), 0.41-0.46 (2H, m), 0.63-0.73 (1H, m), 1.10 (6H, s), 1.20-1.28 (1H, m), 1.31 (3H, t, J = 7.3 Hz), 1.32-1.38 (1H, m), 1.84-1.95 (1H, m), 2.02-2.13 (1H, m), 2.34 (2H, s), 2.64 (2H, s), 2.67 (3H, s), 4.20-4.28 (2H, m), 4.79 (1H, td, J = 7.3, 5.4 Hz), 6.38 (1H, d, J = 7.3 Hz), 9.40 (1H, brs). HRMS (FD⁺):374.22088 [M]⁺ |

**[Table 39]**

| Reference Example | Structure | Equipment Data |
|---|---|---|
| 6 5 - 3 | 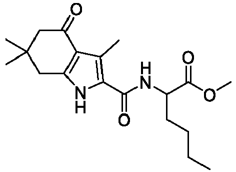 | ¹H-NMR (DMSO-D₆, 400MHz) δ: 0.92 (3H, t, J = 7.0 Hz), 1.06 (3H, s), 1.07 (3H, s), 1.25-1.48 (4H, m), 1.69-1.89 (2H, m), 2.26 (2H, s), 2.49 (3H, s), 2.68 (2H, s), 3.69 (3H, s), 4.35-4.46 (1H, m), 7.79 (1H, d, J = 7.3 Hz), 11.65 (1H, s). HRMS (ESI⁺): 349.21341 [M+H]⁺ |
| 6 5 - 4 | 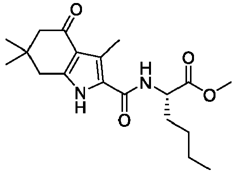 | ¹H-NMR (CDCl₃, 400 MHz) δ: 0.91 (3H, t, J = 7.3 Hz), 1.10 (6H, s), 1.31-1.39 (4H, m), 1.77-1.85 (1H, m), 1.92-2.02 (1H, m), 2.34 (2H, s), 2.67 (2H, s), 2.68 (3H, s), 3.79 (3H, s), 4.75-4.81 (1H, m), 6.42 (1H, d, J = 7.9 Hz), 10.22 (1H, brs). MS (FI⁺): 348.20508 [M]⁺ |
| 6 5 - 5 | 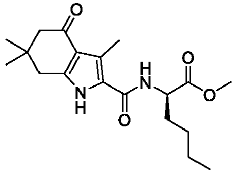 | ¹H-NMR (CDCl₃, 400 MHz) δ: 0.90 (3H, t, J = 7.3 Hz), 1.10 (6H, s), 1.25-1.39 (4H, m), 1.74-1.84 (1H, m), 1.91-2.01 (1H, m), 2.35 (2H, s), 2.66 (2H, s), 2.68 (3H, s), 3.79 (3H, s), 4.75-4.81 (1H, m), 6.37 (1H, d, J = 7.3 Hz), 9.55 (1H, brs). MS (ESI⁺): 349.21288 [M+H]⁺ |
| 6 5 - 6 | 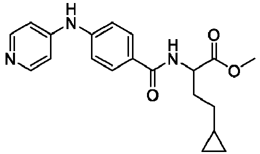 | ¹H-NMR (DMSO-D₆, 400MHz) δ: -0.03-0.07 (2H, m), 0.31-0.44 (2H, m), 0.60-0.73 (1H, m), 1.23-1.32 (2H, m), 1.82-1.93 (2H, m), 3.63 (3H, s), 4.46 (1H, q, J = 7.3 Hz), 7.00 (2H, dd, J = 4.9, 1.8 Hz), 7.24 (2H, d, J = 8.6 Hz), 7.86 (2H, d, J = 8.6 Hz), 8.25 (2H, dd, J = 4.9, 1.8 Hz), 8.54 (1H, d, J = 7.3 Hz), 9.10 (1H, s). MS (ESI⁺): 354.2 [M+H]⁺ |

### <Reference example 66>

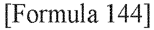

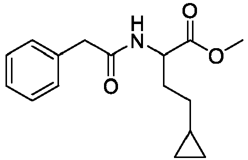

Under argon atmosphere, trifluoroacetic acid (4.00 mL) was added to a solution of methyl 4-cyclopropyl-2-[(2-methylpropan-2-yl)oxycarbonylamino]butanoate obtained as a residue of Reference Example 59 in dichloromethane (4.00 mL) at room temperature, and the mixture was stirred for 20 minutes. The reaction mixture was concentrated under reduced pressure, the residue in dichloromethane (8.00 mL) was added to N,N-diisopropylethylamine (0.699 mL) and 2-phenylacetyl chloride (0.109 mL) at 0 °C, and the mixture was stirred at room temperature for 30 minutes. The reaction mixture was purified by silica gel column chromatography (hexane : ethyl acetate = 9 : 1 to 0 : 1) to obtain methyl 4-cyclopropyl-2-[(2-phenylacetyl) amino]butanoate (189 mg).
¹H-NMR (CDCl₃, 400MHz) δ: -0.13-0.03 (2H, m), 0.32-0.40 (2H, m), 0.51-0.63 (1H, m), 1.01-1.17 (2H, m), 1.63-1.73 (1H, m), 1.83-1.95 (1H, m), 3.60 (2H, s), 3.70 (3H, s), 4.58-4.67 (1H, m), 5.85 (1H, d, J = 7.3 Hz), 7.26-7.41 (5H, m).

MS (ESI⁺): 276.2 [M+H]⁺

### <Reference example 67-1>

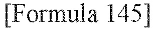

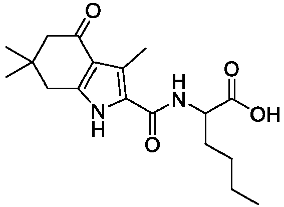

Lithium hydroxide monohydrate (220 mg) was added to a solution of methyl 2-[(3,6,6-trimethyl-4-oxo-5,7-dihydro-1H-indole-2-carbonyl) amino]hexanoate (1.22 g) in a methanol (7.50 mL), tetrahydrofuran (7.50 mL) and water (15.0 mL) mixed solution at room temperature, and the mixture was stirred for 30 minutes. After 1 mol/L hydrochloric acid was added to the reaction mixture, the resulting insoluble solid was collected by filtration to obtain 2-[(3,6,6-trimethyl-4-oxo-5,7-dihydro-1H-indole-2-carbonyl) amino]caproic acid (1.09 g).

HRMS (ESI⁺): 335.19704 [M+H]⁺

Using the corresponding starting materials and reactants, the following Reference Examples 67-2 to 67-7 were obtained by the same method in Reference Example 67-1, the method described in Step 1-2, or a method similar thereto.

**[Table 40]**

| Reference Example | Structure | Equipment Data |
|---|---|---|
| 6 7 - 2 | 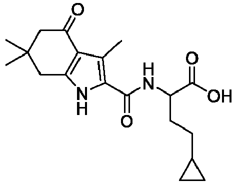 | ¹H-NMR (DMSO-D₆, 400 MHz) δ: -0.05-0.00 (2H, m), 0.32-0.37 (2H, m), 0.59-0.69 (1H, m), 1.01 (6H, s), 1.13-1.31 (2H, m), 1.69-1.80 (1H, m), 1.81-1.91 (1H, m), 2.20 (2H, s), 2.46 (3H, s), 2.63 (2H, s), 4.24 (1H, q, J = 7.3 Hz), 7.71 (1H, brs). |
| | | HRMS (ESI⁺):347.19652 [M+H]⁺ |

**[Table 41]**

| Reference Example | Structure | Equipment Data |
|---|---|---|
| 6 7 - 3 | 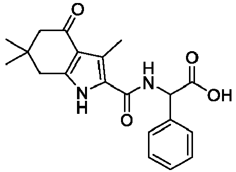 | ¹H-NMR (DMSO-D₆, 400MHz) δ: 1.02 (3H, s), 1.03 (3H, s), 2.23 (2H, s), 2.49 (3H, s), 2.64 (2H, s), 5.48 (1H, d, J = 6.7 Hz), 7.32-7.50 (5H, m), 8.03 (1H, d, J = 6.7 Hz), 11.74 (1H, s), 13.01 (1H, brs). |
| | | HRMS (ESI⁺): 355.16531 [M+H]⁺ |
| 6 7 - 4 | 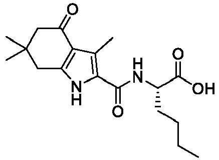 | ¹H-NMR (DMSO-D₆, 400MHz) δ: 0.86 (3H, t, J = 7.3 Hz), 1.01 (6H, s), 1.25-1.34 (4H, m), 1.66-1.79 (2H, m), 2.21 (2H, s), 2.45 (3H, s), 2.63 (2H, s), 4.27-4.33 (1H, m), 7.56 (1H, d, J = 7.3 Hz), 11.65 (1H, s). |
| | | HRMS (ESI⁺):335.19698 [M+H]⁺ |
| 6 7 - 5 | 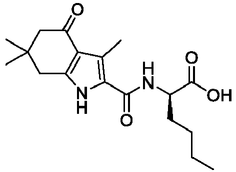 | ¹H-NMR (DMSO-D₆, 400MHz) δ: 0.86 (3H, t, J = 7.3 Hz), 1.01 (6H, s), 1.25-1.35 (4H, m), 1.64-1.72 (1H, m), 1.73-1.82 (1H, m), 2.21 (2H, s), 2.45 (3H, s), 2.63 (2H, s), 4.28-4.35 (1H, m), 7.56 (1H, d, J = 7.3 Hz), 11.63 (1H, s). |
| | | HRMS (ESI⁺):335.19805 [M+H]⁺ |
| 6 7 - 6 | 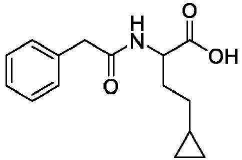 | ¹H-NMR (DMSO-D₆, 400MHz) δ: -0.47--0.34 (2H, m), -0.06-0.04 (2H, m), 0.20-0.33 (1H, m), 0.74-0.90 (2H, m), 1.24-1.36 (1H, m), 1.38-1.49 (1H, m), 3.07 (1H, d, J = 13.9 Hz), 3.13 (1H, d, J = 13.9 Hz), 3.79-3.89 (1H, m), 6.80-6.96 (5H, m), 7.94 (1H, d, J = 7.9 Hz), 12.15 (1H, s). |
| | | MS (ESI⁺): 262.1 [M+H]⁺ |
| 6 7 - 7 | 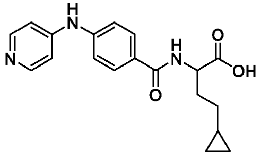 | ¹H-NMR (DMSO-D₆, 400MHz) δ: -0.06-0.04 (2H, m), 0.32-0.40 (2H, m), 0.61-0.72 (1H, m), 1.19-1.31 (2H, m), 1.77-1.93 (2H, m), 4.27-4.36 (1H, m), 7.00 (2H, dd, J = 4.9, 1.8 Hz), 7.24 (2H, d, J = 8.6 Hz), 7.84 (2H, d, J = 8.6 Hz), 8.24 (2H, dd, J = 4.9, 1.8 Hz), 8.28 (1H, d, J = 7.3 Hz), 9.16 (1H, s). |
| | | MS (ESI⁺): 340.2 [M+H]⁺ |

### <Example 1-1>

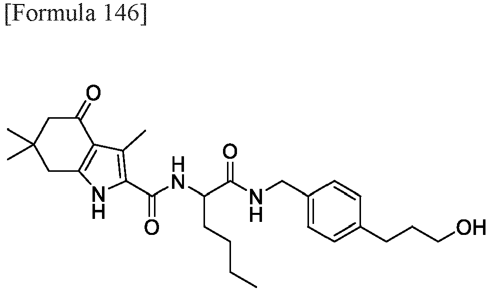

Under argon atmosphere, 4-(4,6-dimethoxy-1,3,5-triazin-2-yl)-4-methylmorpholinium chloride (248 mg) was added to a solution of 2-[(3,6,6-trimethyl-4-oxo-5,7-dihydro-1H-indole-2-carbonyl)amino]hexanoic acid (250 mg) and 3-[4-(aminomethyl)phenyl]propan-1-ol (148 mg) in N, N-dimethylformamide (4.00 mL) at room temperature, and the mixture was stirred for 1 hour. After water was added, the mixture was extracted twice with ethyl acetate. The combined organic layer was washed in the order of water and saturated brine, dried over anhydrous sodium sulfate, and concentrated under reduced pressure after filtration of the insoluble material. The residue was purified by silica gel column chromatography (hexane : ethyl acetate = 3 : 1 to 0 : 1) to obtain N-[1-[[4-(3-hydroxypropyl)phenyl]methylamino]-1-oxohexan-2-yl]-3,6,6-trimethyl-4-oxo-5,7-dihydro-1H-indole-2-carboxamide (241 mg). ¹H-NMR (DMSO-d₆, 400MHz) δ: 0.87 (3H, t, J = 7.0 Hz), 1.04 (6H, s), 1.23-1.37 (4H, m), 1.59-1.82 (4H, m), 2.24 (2H, s), 2.49 (3H, s), 2.55-2.62 (2H, m), 2.65 (2H, s), 3.38-3.44 (2H, m), 4.20-4.33 (2H, m), 4.41-4.50 (2H, m), 7.11-7.20 (4H, m), 7.47 (1H, d, J = 7.9 Hz), 8.53 (1H, t, J = 6.1 Hz), 11.72 (1H, s). HRMS (ESI⁺): 482.30238 [M+H]⁺

Using the corresponding starting materials and reactants, the following Examples 1-2 to 1-149 were obtained by the same method in Example 1-1, the method described in Step 1-3, or a method similar thereto.

**[Table 42]**

| Example | Structure | Equipment Data |
|---|---|---|
| 1 - 2 | 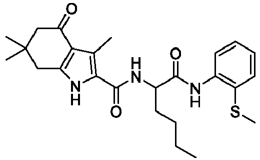 | ¹H-NMR (DMSO-D₆, 400MHz) δ: 0.89 (3H, t, J = 7.0 Hz), 1.01 (6H, s), 1.28-1.43 (4H, m), 1.69-1.95 (2H, m), 2.21 (2H, s), 2.36 (3H, s), 2.50 (3H, s), 2.64 (2H, s), 4.64 (1H, brs), 7.19 (2H, dt, J = 8.9, 2.6 Hz), 7.33-7.35 (1H, m), 7.45 (1H, dd, J = 7.0, 2.1 Hz), 7.60 (1H, d, J = 7.9 Hz), 9.51 (1H, s), 11.70 (1H, s). |
| | | HRMS (ESI⁺): 456.2310 [M+H]⁺ |
| 1 - 3 | 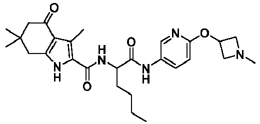 | ¹H-NMR (DMSO-D₆, 400MHz) δ: 0.89 (3H, t, J = 6.7 Hz), 1.04 (6H, s), 1.29-1.46 (4H, m), 1.65-1.89 (2H, m), 2.24 (2H, s), 2.29 (3H, s), 2.49 (3H, s), 2.66 (2H, s), 2.92-3.00 (2H, m), 3.66-3.73 (2H, m), 4.51-4.62 (1H, m), 5.02-5.10 (1H, m), 6.82 (1H, d, J = 9.1 Hz), 7.66 (1H, d, J = 7.9 Hz), 7.94 (1H, dd, J = 9.1, 3.0 Hz), 8.35 (1H, d, J = 3.0 Hz), 10.21 (1H, s), 11.72 (1H, s). |
| | | HRMS (FD⁺): 496.29235 [M+H]⁺ |

**[Table 43]**

| Example | Structure | Equipment Data |
|---|---|---|
| 1 - 4 | 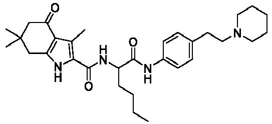 | ¹H-NMR (DMSO-D₆, 400MHz) δ: 0.86 (3H, t, J = 7.0 Hz), 1.01 (6H, s), 1.25-1.41 (6H, m), 1.43-1.52 (4H, m), 1.63-1.84 (2H, m), 2.21 (2H, s), 2.29-2.45 (6H, m), 2.47 (3H, s), 2.60-2.69 (4H, m), 4.51-4.60 (1H, m), 7.13 (2H, d, J = 8.5 Hz), 7.49 (2H, d, J = 8.5 Hz), 7.55 (1H, d, J = 7.9 Hz), 10.04 (1H, s), 11.67 (1H, s). |
| | | HRMS (ESI⁺): 521.34859 [M+H]⁺ |
| 1 - 5 | 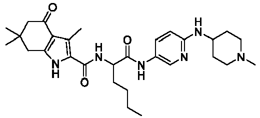 | ¹H-NMR (DMSO-D₆, 400 MHz) δ: 0.83-0.91 (3H, m), 1.01 (6H, s), 1.27-1.38 (6H, m), 1.71-1.87 (4H, m), 1.95-2.03 (2H, m), 2.16 (3H, s), 2.21 (2H, s), 2.46 (3H, s), 2.63 (2H, s), 2.69-2.75 (2H, m), 3.74 (1H, brs), 4.49-4.56 (1H, m), 6.22 (1H, d, J = 7.9 Hz), 6.41 (1H, d, J = 8.5 Hz), 7.48-7.72 (2H, m), 8.08-8.15 (1H, m), 9.80 (1H, s), 11.67 (1H, brs). |
| | | MS (ESI⁺):523.3 [M+H]⁺ |
| 1 - 6 | 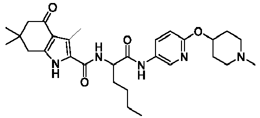 | ¹H-NMR (DMSO-D₆, 400MHz) δ: 0.89 (3H, t, J = 7.0 Hz), 1.04 (6H, s), 1.28-1.46 (4H, m), 1.57-1.87 (4H, m), 1.90-2.00 (2H, m), 2.09-2.22 (5H, m), 2.24 (2H, s), 2.49 (3H, s), 2.58-2.71 (4H, m), 4.52-4.61 (1H, m), 4.87-4.97 (1H, m), 6.77 (1H, d, J = 9.1 Hz), 7.67 (1H, d, J = 7.9 Hz), 7.91 (1H, dd, J = 9.1, 2.4 Hz), 8.36 (1H, d, J = 2.4 Hz), 10.18 (1H, s), 11.73 (1H, s). |
| | | HRMS (ESI⁺): 524.32424 [M+H]⁺ |
| 1 - 7 | 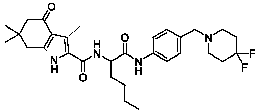 | ¹H-NMR (DMSO-D₆, 400MHz) δ: 0.89 (3H, t, J = 7.0 Hz), 1.04 (6H, s), 1.29-1.47 (4H, m), 1.66-1.86 (2H, m), 1.88-2.02 (4H, m), 2.24 (2H, s), 2.42-2.50 (7H, m), 2.66 (2H, s), 3.49 (2H, s), 4.53-4.63 (1H, m), 7.25 (2H, d, J = 8.5 Hz), 7.55-7.62 (3H, m), 10.13 (1H, s), 11.69 (1H, s). |
| | | HRMS (ESI⁺): 543.31374 [M+H]⁺ |
| 1 - 8 | 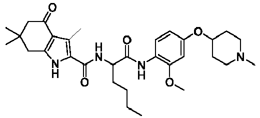 | ¹H-NMR (DMSO-D₆, 400MHz) δ: 0.88 (3H, t, J = 7.0 Hz), 1.02 (6H, s), 1.28-1.44 (4H, m), 1.55-1.97 (6H, m), 2.12-2.20 (5H, m), 2.23 (2H, s), 2.49 (3H, s), 2.56-2.71 (4H, m), 3.77 (3H, s), 4.27-4.38 (1H, m), 4.59-4.70 (1H, m), 6.51 (1H, dd, J = 9.1, 2.4 Hz), 6.60 (1H, d, J = 2.4 Hz), 7.61 (1H, d, J = 7.9 Hz), 7.67 (1H, d, J = 9.1 Hz), 9.14 (1H, s), 11.72 (1H, s). |
| | | HRMS (ESI⁺): 553.33923 [M+H]⁺ |

**[Table 44]**

| Example | Structure | Equipment Data |
|---|---|---|
| 1 - 9 | 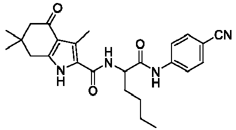 | ¹H-NMR (DMSO-D₆, 400MHz) δ: 0.88 (3H, t, J = 6.7 Hz), 1.02 (6H, s), 1.29-1.43 (4H, m), 1.70-1.83 (2H, m), 2.22 (2H, s), 2.47 (3H, s), 2.65 (2H, s), 4.54 (1H, td, J = 8.6, 6.1 Hz), 7.73 (1H, d, J = 8.6 Hz), 7.78 (2H, d, J = 8.6 Hz), 7.81 (2H, d, J = 8.6 Hz), 10.60 (1H, s), 11.69 (1H, s). |
| | | HRMS (ESI⁺):435.23922 [M+H]⁺ |
| 1 - 1 0 | 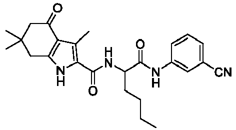 | ¹H-NMR (DMSO-D₆, 400MHz) δ: 0.88 (3H, t, J = 6.7 Hz), 1.03 (6H, s), 1.32-1.42 (4H, m), 1.72-1.79 (2H, m), 2.23 (2H, s), 2.47 (3H, s), 2.65 (2H, s), 4.54 (1H, td, J = 7.9, 4.9 Hz), 7.53-7.55 (2H, m), 7.70 (1H, d, J = 7.9 Hz), 7.82-7.86 (1H, m), 8.12 (1H, d, J = 1.2 Hz), 10.51 (1H, s), 11.69 (1H, s). |
| | | HRMS (ESI⁺):435.23878 [M+H]⁺ |
| 1 - 1 1 | 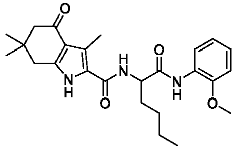 | ¹H-NMR (DMSO-D₆, 400MHz) δ: 0.88 (3H, t, J = 7.0 Hz), 1.02 (6H, s), 1.26-1.46 (4H, m), 1.64-1.92 (2H, m), 2.22 (2H, s), 2.49 (3H, s), 2.64 (2H, s), 3.79 (3H, s), 4.61-4.73 (1H, m), 6.85-6.93 (1H, m), 7.01-7.11 (2H, m), 7.70 (1H, d, J = 7.3 Hz), 7.95 (1H, dd, J = 7.9, 1.2 Hz), 9.26 (1H, s), 11.74 (1H, s). |
| | | HRMS (ESI⁺): 440.25465 [M+H]⁺ |
| 1 - 1 2 | 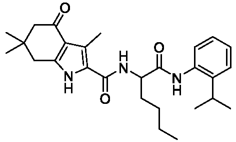 | ¹H-NMR (DMSO-D₆, 400MHz) δ: 0.90 (3H, t, J = 7.0 Hz), 1.02 (6H, s), 1.11-1.18 (6H, m), 1.25-1.48 (4H, m), 1.69-1.90 (2H, m), 2.22 (2H, s), 2.49 (3H, s), 2.65 (2H, s), 3.13-3.20 (1H, m), 4.63-4.69 (1H, m), 7.17-7.22 (3H, m), 7.31 (1H, dd, J = 7.3, 1.8 Hz), 7.56 (1H, d, J = 7.3 Hz), 9.54 (1H, s), 11.71 (1H, s). |
| | | HRMS (ESI⁺): 452.29216 [M+H]⁺ |
| 1 - 1 3 | 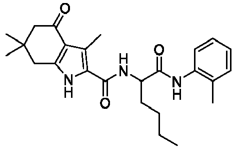 | ¹H-NMR (DMSO-D₆, 400MHz) δ: 0.90 (3H, t, J = 7.0 Hz), 1.02 (6H, s), 1.30-1.50 (4H, m), 1.70-1.90 (2H, m), 2.20 (3H, s), 2.23 (2H, s), 2.49 (3H, s), 2.65 (2H, s), 4.59-4.69 (1H, m), 7.09 (1H, t, J = 7.0 Hz), 7.17 (1H, t, J = 7.6 Hz), 7.21 (1H, d, J = 7.3 Hz), 7.35 (1H, d, J = 6.7 Hz), 7.57 (1H, d, J = 7.3 Hz), 9.51 (1H, s), 11.69 (1H, s). |
| | | HRMS (ESI⁺): 424.2588 [M+H]⁺ |

**[Table 45]**

| Example | Structure | Equipment Data |
|---|---|---|
| 1 - 1 4 | 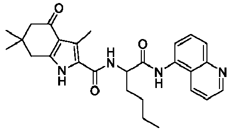 | ¹H-NMR (DMSO-D₆, 400MHz) δ: 0.98 (3H, t, J = 7.0 Hz), 1.09 (6H, s), 1.37-1.65 (4H, m), 1.83-2.05 (2H, m), 2.29 (2H, s), 2.57 (3H, s), 2.71 (2H, s), 4.75-4.84 (1H, m), 7.60-7.68 (1H, m), 7.72-7.85 (3H, m), 7.95 (1H, d, J = 7.9 Hz), 8.53 (1H, d, J = 7.9 Hz), 8.98 (1H, dd, J = 4.8, 1.8 Hz), 10.33 (1H, s), 11.77 (1H, s). |
| | | MS (ESI⁺): 461.2539 [M+H]⁺ |
| 1 - 1 5 | 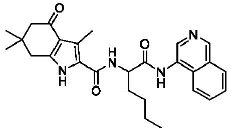 | ¹H-NMR (DMSO-D₆, 400MHz) δ: 0.90 (3H, t, J = 7.0 Hz), 1.00 (6H, s), 1.30-1.50 (4H, m), 1.78-1.95 (2H, m), 2.21 (2H, s), 2.49 (3H, s), 2.63 (2H, s), 4.70-4.80 (1H, m), 7.65-7.75 (2H, m), 7.81 (1H, t, J = 7.3 Hz), 8.06 (1H, d, J = 8.5 Hz), 8.14 (1H, d, J = 8.5 Hz), 8.61 (1H, s), 9.15 (1H, s), 10.22 (1H, s), 11.68 (1H, s). |
| | | HRMS (ESI⁺): 461.2541 [M+H]⁺ |
| 1 - 1 6 | 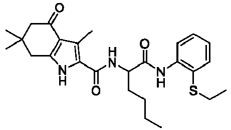 | ¹H-NMR (DMSO-D₆, 400MHz) δ: 0.96 (3H, t, J = 7.0 Hz), 1.08 (6H, s), 1.15 (3H, t, J = 7.3 Hz), 1.33-1.54 (4H, m), 1.78-2.04 (2H, m), 2.29 (2H, s), 2.55 (3H, s), 2.71 (2H, s), 2.85 (2H, q, J = 7.5 Hz), 4.63-4.70 (1H, m), 7.22 (1H, t, J = 7.6 Hz), 7.30-7.34 (1H, m), 7.50 (1H, dd, J = 7.9, 1.2 Hz), 7.71-7.82 (2H, m), 9.56 (1H, s), 11.76 (1H, s). |
| | | HRMS (ESI⁺): 470.24687 [M+H]⁺ |
| 1 - 1 7 | 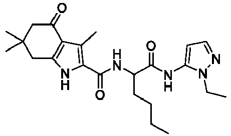 | ¹H-NMR (DMSO-D₆, 400MHz) δ: 0.96 (3H, t, J = 7.0 Hz), 1.09 (6H, s), 1.32 (3H, t, J = 7.3 Hz), 1.35-1.55 (4H, m), 1.74-1.94 (2H, m), 2.29 (2H, s), 2.54 (3H, s), 2.71 (2H, s), 4.06 (2H, q, J = 7.3 Hz), 4.61-4.71 (1H, m), 6.21 (1H, d, J = 2.1 Hz), 7.42 (1H, d, J = 2.1 Hz), 7.70 (1H, d, J = 7.3 Hz), 10.11 (1H, s), 11.72 (1H, s). |
| | | HRMS (ESI⁺): 428.26569 [M+H]⁺ |
| 1 - 1 8 | 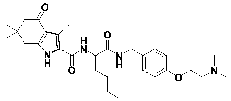 | ¹H-NMR (DMSO-D₆, 400MHz) δ: 0.75-0.95 (3H, m), 1.01 (6H, s), 1.23-1.30 (4H, m), 1.57-1.76 (2H, m), 2.18 (6H, s), 2.21 (2H, s), 2.46 (3H, s), 2.57 (2H, t, J = 5.8 Hz), 2.62 (2H, s), 3.99 (2H, t, J = 5.8 Hz), 4.17-4.23 (2H, m), 4.38-4.45 (1H, m), 6.86 (2H, d, J = 8.5 Hz), 7.15 (2H, d, J = 8.5 Hz), 7.46 (1H, d, J = 7.9 Hz), 8.48 (1H, t, J = 6.1 Hz), 11.71 (1H, s). |
| | | HRMS (ESI⁺):511.32795 [M+H]⁺ |

**[Table 46]**

| Example | Structure | Equipment Data |
|---|---|---|
| 1 - 1 9 | 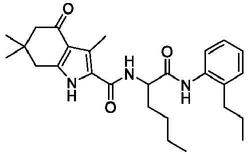 | ¹H-NMR (DMSO-D₆, 400MHz) δ: 0.83 (3H, t, J = 7.3 Hz), 0.89 (3H, t, J = 7.0 Hz), 1.01 (6H, s), 1.30-1.42 (4H, m), 1.44-1.48 (2H, m), 1.67-1.90 (2H, m), 2.21 (2H, s), 2.48 (3H, s), 2.53-2.55 (2H, m), 2.63 (2H, s), 4.57-4.67 (1H, m), 7.10-7.20 (3H, m), 7.32 (1H, d, J = 7.3 Hz), 7.62 (1H, brs), 9.46 (1H, s), 11.74 (1H, brs). |
| | | HRMS (ESI⁺): 452.29107 [M+H]⁺ |
| 1 - 2 0 | 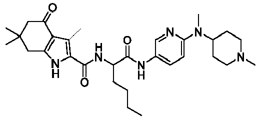 | ¹H-NMR (DMSO-D₆, 400MHz) δ: 0.80-0.90 (3H, m), 1.01 (6H, s), 1.28-1.35 (4H, m), 1.47-1.52 (2H, m), 1.71-1.78 (4H, m), 2.00-2.13 (2H, m), 2.15-2.25 (5H, m), 2.47 (3H, s), 2.63 (2H, s), 2.78 (3H, s), 2.84-2.89 (2H, m), 4.25-4.40 (1H, m), 4.54 (1H, td, J = 8.5, 5.4 Hz), 6.59 (1H, d, J = 9.1 Hz), 7.55 (1H, d, J = 7.9 Hz), 7.71 (1H, dd, J = 9.1, 2.4 Hz), 8.24 (1H, d, J = 2.4 Hz), 9.90 (1H, s), 11.68 (1H, s). |
| | | HRMS (ESI⁺):537.35570 [M+H]⁺ |
| 1 - 2 1 | 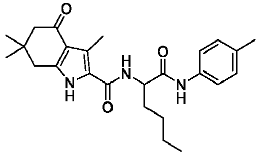 | ¹H-NMR (DMSO-D₆, 400MHz) δ: 0.87 (3H, t, J = 6.7 Hz), 1.02 (6H, s), 1.29-1.40 (4H, m), 1.69-1.77 (2H, m), 2.22 (2H, s), 2.25 (3H, s), 2.48 (3H, s), 2.64 (2H, s), 4.56 (1H, td, J = 8.6, 6.1 Hz), 7.11 (2H, d, J = 8.6 Hz), 7.49 (2H, d, J = 8.6 Hz), 7.60 (1H, d, J = 8.6 Hz), 10.05 (1H, s), 11.71 (1H, s). |
| | | HRMS (ESI⁺):424.25962 [M+H]⁺ |
| 1 - 2 2 | 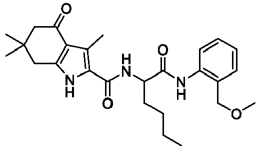 | ¹H-NMR (DMSO-D₆, 400MHz) δ: 0.90 (3H, t, J = 7.0 Hz), 1.01 (6H, s), 1.30-1.50 (4H, m), 1.70-1.93 (2H, m), 2.22 (2H, s), 2.50 (3H, s), 2.64 (2H, s), 3.17 (3H, s), 4.33 (1H, d, J = 12.1 Hz), 4.42 (1H, d, J = 12.1 Hz), 4.50-4.57 (1H, m), 7.15 (1H, t, J = 7.3 Hz), 7.27-7.34 (2H, m), 7.60 (1H, d, J = 7.9 Hz), 7.67 (1H, d, J = 7.3 Hz), 9.46 (1H, s), 11.68 (1H, s). |
| | | HRMS (ESI⁺): 454.2697 [M+H]⁺ |

**[Table 47]**

| Example | Structure | Equipment Data |
|---|---|---|
| 1 - 2 3 | 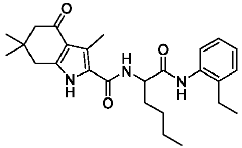 | ¹H-NMR (DMSO-D₆, 400MHz) δ: 0.89 (3H, t, J = 7.0 Hz), 1.01 (6H, s), 1.08 (3H, t, J = 7.6 Hz), 1.32-1.40 (4H, m), 1.68-1.89 (2H, m), 2.21 (2H, s), 2.48 (3H, s), 2.54-2.61 (2H, m), 2.62 (2H, s), 4.64 (1H, dt, J = 7.9, 5.4 Hz), 7.10-7.20 (2H, m), 7.22 (1H, dd, J = 6.7, 2.4 Hz), 7.31 (1H, dd, J = 7.3, 1.8 Hz), 7.56 (1H, d, J = 7.9 Hz), 9.48 (1H, s), 11.69 (1H, brs). |
| | | HRMS (ESI⁺): 438.2747 [M+H]⁺ |
| 1 - 2 4 | 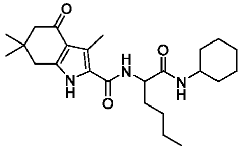 | ¹H-NMR (DMSO-D₆, 400MHz) δ: 0.84 (3H, t, J = 6.7 Hz), 1.01 (6H, s), 1.08-1.34 (9H, m), 1.48-1.75 (7H, m), 2.21 (2H, s), 2.45 (3H, s), 2.62 (2H, s), 3.46-3.58 (1H, m), 4.35-4.44 (1H, m), 7.32 (1H, d, J = 7.9 Hz), 7.90 (1H, d, J = 7.9 Hz), 11.68 (1H, s). |
| | | HRMS (ESI⁺): 416.29060 [M+H]⁺ |
| 1 - 2 5 | 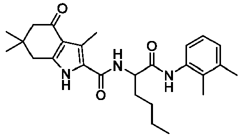 | ¹H-NMR (DMSO-D₆, 400MHz) δ: 0.89 (3H, t, J = 7.0 Hz), 1.01 (6H, s), 1.28-1.47 (4H, m), 1.69-1.90 (2H, m), 2.06 (3H, s), 2.21 (2H, s), 2.23 (3H, s), 2.47 (3H, s), 2.63 (2H, s), 4.57-4.66 (1H, m), 6.97-7.12 (3H, m), 7.53 (1H, d, J = 7.9 Hz), 9.55 (1H, s), 11.68 (1H, s). |
| | | HRMS (ESI⁺): 438.27524 [M+H]⁺ |
| 1 - 2 6 | 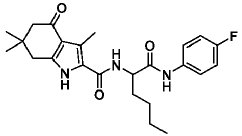 | ¹H-NMR (DMSO-D₆, 400MHz) δ: 0.86 (3H, t, J = 7.0 Hz), 1.01 (6H, s), 1.26-1.44 (4H, m), 1.64-1.84 (2H, m), 2.21 (2H, s), 2.46 (3H, s), 2.63 (2H, s), 4.49-4.58 (1H, m), 7.10-7.18 (2H, m), 7.54-7.67 (3H, m), 10.18 (1H, s), 11.67 (1H, s). |
| | | HRMS (ESI⁺): 428.23586 [M+H]⁺ |
| 1 - 2 7 | 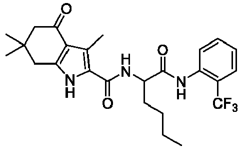 | ¹H-NMR (DMSO-D₆, 400MHz) δ: 0.88 (3H, t, J = 6.7 Hz), 1.01 (6H, s), 1.26-1.43 (4H, m), 1.67-1.91 (2H, m), 2.21 (2H, s), 2.50 (3H, s), 2.64 (2H, s), 4.60-4.72 (1H, m), 7.44-7.49 (2H, m), 7.66-7.70 (3H, m), 9.76 (1H, s), 11.74 (1H, brs). |
| | | HRMS (ESI⁺): 478.2305 [M+H]⁺ |
| 1 - 2 8 | 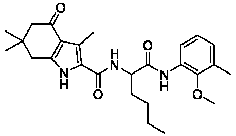 | ¹H-NMR (DMSO-D₆, 400MHz) δ: 0.89 (3H, t, J = 7.3 Hz), 1.03 (6H, s), 1.30-1.50 (4H, m), 1.70-1.90 (2H, m), 2.23 (5H, s), 2.50 (3H, s), 2.66 (2H, s), 3.62 (3H, s), 4.63-4.71 (1H, m), 6.90-7.00 (2H, m), 7.65-7.75 (1H, m), 7.80 (1H, d, J = 7.9 Hz), 9.37 (1H, s), 11.70 (1H, s). |
| | | HRMS (ESI⁺): 454.2693 [M+H]⁺ |

**[Table 48]**

| Example | Structure | Equipment Data |
|---|---|---|
| 1 - 2 9 | 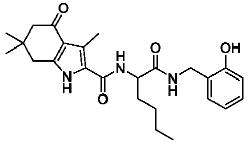 | ¹H-NMR (DMSO-D₆, 400MHz) δ: 0.80-0.90 (3H, m), 1.01 (6H, s), 1.20-1.33 (4H, m), 1.59-1.77 (2H, m), 2.21 (2H, s), 2.46 (3H, s), 2.62 (2H, s), 4.18 (1H, dd, J = 15.1, 5.4 Hz), 4.24 (1H, dd, J = 15.1, 5.4 Hz), 4.46 (1H, td, J = 7.9, 5.4 Hz), 6.72 (1H, t, J = 7.3 Hz), 6.77 (1H, d, J = 7.9 Hz), 7.04 (1H, td, J = 7.9, 1.8 Hz), 7.08 (1H, dd, J = 7.3, 1.8 Hz), 7.49 (1H, d, J = 7.9 Hz), 8.43 (1H, t, J = 5.4 Hz), 9.53 (1H, brs), 11.72 (1H, brs). |
| | | HRMS (ESI⁺):440.25427 [M+H]⁺ |
| 1 - 3 0 | 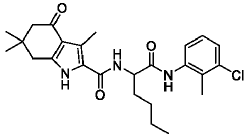 | ¹H-NMR (DMSO-D₆, 400MHz) δ: 0.90 (3H, t, J = 7.0 Hz), 1.03 (6H, s), 1.30-1.50 (4H, m), 1.74-1.90 (2H, m), 2.23 (3H, s), 2.23 (2H, s), 2.49 (3H, s), 2.65 (2H, s), 4.59-4.67 (1H, m), 7.21 (1H, t, J = 8.2 Hz), 7.30 (2H, t, J = 6.7 Hz), 7.60 (1H, d, J = 7.9 Hz), 9.79 (1H, s), 11.69 (1H, s). |
| | | HRMS (ESI⁺): 458.2197 [M+H]⁺ |
| 1 - 3 1 | 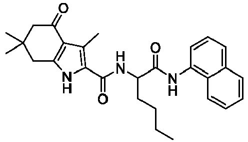 | ¹H-NMR (DMSO-D₆, 400MHz) δ: 0.91 (3H, t, J = 6.7 Hz), 1.02 (6H, s), 1.30-1.55 (4H, m), 1.75-1.99 (2H, m), 2.22 (2H, s), 2.51 (3H, s), 2.64 (2H, s), 4.71-4.82 (1H, m), 7.44-7.58 (3H, m), 7.58-7.71 (2H, m), 7.78 (1H, d, J = 7.9 Hz), 7.90-7.98 (1H, m), 8.02-8.10 (1H, m), 10.12 (1H, s), 11.72 (1H, s). |
| | | HRMS (ESI⁺): 460.2589 [M+H]⁺ |
| 1 - 3 2 | 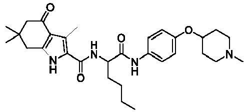 | ¹H-NMR (DMSO-D₆, 400MHz) δ: 0.89 (3H, t, J = 6.1 Hz), 1.04 (6H, s), 1.28-1.46 (4H, m), 1.55-1.66 (2H, m), 1.66-1.85 (2H, m), 1.86-1.97 (2H, m), 2.07-2.20 (5H, m), 2.24 (2H, s), 2.49 (3H, s), 2.56-2.70 (4H, m), 4.23-4.33 (1H, m), 4.51-4.61 (1H, m), 6.91 (2H, d, J = 9.2 Hz), 7.51 (2H, d, J = 9.2 Hz), 7.58 (1H, d, J = 7.3 Hz), 10.01 (1H, s), 11.71 (1H, s). |
| 1 - 3 3 | 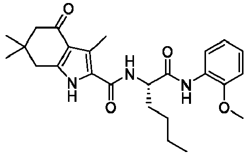 | ¹H-NMR (DMSO-D₆, 400MHz) δ: 0.88 (3H, t, J = 7.0 Hz), 1.02 (6H, s), 1.26-1.47 (4H, m), 1.62-1.92 (2H, m), 2.23 (2H, s), 2.49 (3H, s), 2.65 (2H, s), 3.79 (3H, s), 4.62-4.72 (1H, m), 6.87-6.94 (1H, m), 7.01-7.10 (2H, m), 7.67 (1H, d, J = 7.9 Hz), 7.95 (1H, dd, J = 7.9, 1.2 Hz), 9.27 (1H, s), 11.70 (1H, s). |
| | | HRMS (ESI⁺): 440.25440 [M+H]⁺ |

**[Table 49]**

| Example | Structure | Equipment Data |
|---|---|---|
| 1 - 3 4 | 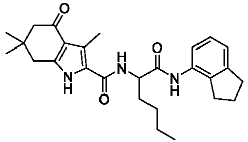 | ¹H-NMR (DMSO-D₆, 400MHz) δ: 0.88 (3H, t, J = 7.0 Hz), 1.01 (6H, s), 1.20-1.42 (4H, m), 1.67-1.85 (2H, m), 1.93-2.03 (2H, m), 2.21 (2H, s), 2.48 (3H, s), 2.63 (2H, s), 2.78 (2H, t, J = 7.6 Hz), 2.86 (2H, t, J = 7.6 Hz), 4.60-4.70 (1H, m), 7.01 (1H, d, J = 7.3 Hz), 7.08 (1H, t, J = 7.6 Hz), 7.34 (1H, d, J = 7.9 Hz), 7.50-7.65 (1H, m), 9.52 (1H, s), 11.70 (1H, brs). |
| | | HRMS (ESI⁺): 450.2744 [M+H]⁺ |
| 1 - 3 5 | 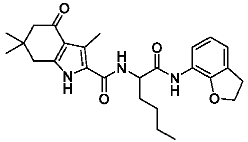 | ¹H-NMR (DMSO-D₆, 400MHz) δ: 0.87 (3H, t, J = 7.0 Hz), 1.01 (6H, s), 1.24-1.43 (4H, m), 1.63-1.84 (2H, m), 2.21 (2H, s), 2.47 (3H, s), 2.63 (2H, s), 3.20 (2H, t, J = 8.8 Hz), 4.55 (2H, t, J = 9.1 Hz), 4.66 (1H, td, J = 8.2, 5.0 Hz), 6.76 (1H, t, J = 7.6 Hz), 6.98 (1H, d, J = 7.3 Hz), 7.52-7.60 (2H, m), 9.47 (1H, s), 11.68 (1H, s). |
| | | HRMS (ESI⁺): 452.2539 [M+H]⁺ |
| 1 - 3 6 | 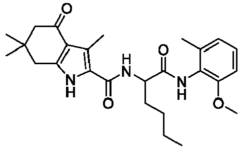 | ¹H-NMR (DMSO-D₆, 400MHz) δ: 0.89 (3H, t, J = 7.3 Hz), 1.01 (6H, s), 1.29-1.45 (4H, m), 1.68-1.90 (2H, m), 2.09 (3H, s), 2.21 (2H, s), 2.47 (3H, s), 2.63 (2H, s), 3.70 (3H, s), 4.61-4.71 (1H, m), 6.80 (1H, d, J = 7.3 Hz), 6.85 (1H, d, J = 7.9 Hz), 7.12 (1H, t, J = 7.9 Hz), 7.46 (1H, d, J = 8.5 Hz), 9.27 (1H, s), 11.73 (1H, s). |
| | | HRMS (ESI⁺): 454.27057 [M+H]⁺ |
| 1 - 3 7 | 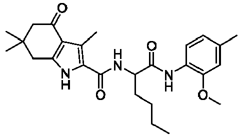 | ¹H-NMR (DMSO-D₆, 400MHz) δ: 0.87 (3H, t, J = 7.0 Hz), 1.01 (6H, s), 1.25-1.45 (4H, m), 1.64-1.87 (2H, m), 2.21 (2H, s), 2.26 (3H, s), 2.48 (3H , s), 2.63 (2H, s), 3.76 (3H, s), 4.59-4.68 (1H, m), 6.70 (1H, d, J = 7.3 Hz), 6.85 (1H, s), 7.64 (1H, d, J = 7.9 Hz), 7.76 (1H, d, J = 7.9 Hz), 9.16 (1H, s), 11.70 (1H, s). |
| | | HRMS (ESI⁺): 454.27119 [M+H]⁺ |
| 1 - 3 8 | 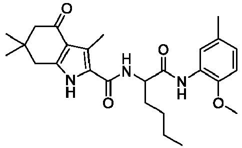 | ¹H-NMR (DMSO-D₆, 400MHz) δ: 0.87 (3H, t, J = 7.0 Hz), 1.01 (6H, s), 1.20-1.40 (4H, m), 1.67-1.87 (2H, m), 2.21 (3H, s), 2.22 (2H, s), 2.50 (3H, s), 2.64 (2H, s), 3.74 (3H, s), 4.60-4.67 (1H, m), 6.84-6.92 (2H, m), 7.65 (1H, d, J = 7.9 Hz), 7.78 (1H, s), 9.18 (1H, s), 11.70 (1H, s). |
| | | HRMS (ESI⁺): 454.27065 [M+H]⁺ |

**[Table 50]**

| Example | Structure | Equipment Data |
|---|---|---|
| 1 - 3 9 | 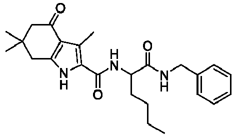 | ¹H-NMR (DMSO-D₆, 400MHz) δ: 0.84 (3H, t, J = 6.7 Hz), 1.01 (6H, s), 1.24-1.31 (4H, m), 1.60-1.77 (2H, m), 2.21 (2H, s), 2.46 (3H, s), 2.62 (2H, s), 4.22-4.34 (2H, m), 4.44 (1H, td, J = 7.9, 5.4 Hz), 7.19-7.26 (3H, m), 7.27-7.32 (2H, m), 7.49 (1H, d, J = 7.9 Hz), 8.55 (1H, t, J = 6.1 Hz), 11.73 (1H, s). |
| | | HRMS (ESI⁺):424.26083 [M+H]⁺ |
| 1 - 4 0 | 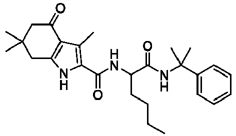 | ¹H-NMR (DMSO-D₆, 400MHz) δ: 0.86 (3H, t, J = 6.7 Hz), 1.00 (6H, s), 1.23-1.33 (4H, m), 1.54 (3H, s), 1.55 (3H, s), 1.58-1.75 (2H, m), 2.20 (2H, s), 2.45 (3H, s), 2.61 (2H, s), 4.48-4.55 (1H, m), 7.14 (1H, tt, J = 7.3, 1.8 Hz), 7.24 (2H, t, J = 7.6 Hz), 7.27-7.32 (2H, m), 7.33 (1H, s), 8.26 (1H, s), 11.74 (1H, s). |
| | | HRMS (ESI⁺):452.29181 [M+H]⁺ |
| 1 - 4 1 | 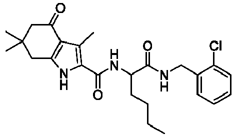 | ¹H-NMR (DMSO-D₆, 400MHz) δ: 0.85 (3H, t, J = 6.7 Hz), 1.01 (6H, s), 1.26-1.32 (4H, m), 1.59-1.80 (2H, m), 2.21 (2H, s), 2.47 (3H, s), 2.63 (2H, s), 4.31 (1H, dd, J = 15.7, 6.1 Hz), 4.37 (1H, dd, J = 15.7, 6.1 Hz), 4.42-4.49 (1H, m), 7.25-7.32 (2H, m), 7.33-7.37 (1H, m), 7.40-7.43 (1H, m), 7.52 (1H, d, J = 7.9 Hz), 8.58 (1H, t, J = 6.1 Hz), 11.71 (1H, s). |
| | | HRMS (ESI⁺):458.22071 [M+H]⁺ |
| 1 - 4 2 | 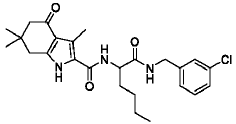 | ¹H-NMR (DMSO-D₆, 400MHz) δ: 0.80-0.90 (3H, m), 1.01 (6H, s), 1.24-1.32 (4H, m), 1.60-1.78 (2H, m), 2.21 (2H, s), 2.47 (3H, s), 2.63 (2H, s), 4.23-4.35 (2H, m), 4.38-4.46 (1H, m), 7.21 (1H, d, J = 7.3 Hz), 7.25-7.35 (3H, m), 7.52 (1H, d, J = 7.9 Hz), 8.61 (1H, t, J = 5.4 Hz), 11.71 (1H, s). |
| | | HRMS (ESI⁺):458.22044 [M+H]⁺ |
| 1 - 4 3 | 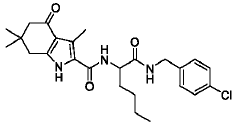 | ¹H-NMR (DMSO-D₆, 400MHz) δ: 0.84 (3H, t, J = 6.7 Hz), 1.01 (6H, s), 1.24-1.30 (4H, m), 1.59-1.77 (2H, m), 2.21 (2H, s), 2.46 (3H, s), 2.63 (2H, s), 4.24 (1H, dd, J = 15.7, 6.1 Hz), 4.29 (1H, dd, J = 15.7, 6.1 Hz), 4.38-4.45 (1H, m), 7.27 (2H, d, J = 8.5 Hz), 7.36 (2H, d, J = 8.5 Hz), 7.51 (1H, d, J = 7.9 Hz), 8.59 (1H, t, J = 6.1 Hz), 11.73 (1H, s). |
| | | HRMS (ESI⁺):458.22130 [M+H]⁺ |

**[Table 51]**

| Example | Structure | Equipment Data |
|---|---|---|
| 1 - 4 4 | 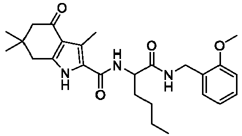 | ¹H-NMR (DMSO-D₆, 400MHz) δ: 0.85 (3H, t, J = 6.7 Hz), 1.01 (6H, s), 1.24-1.32 (4H, m), 1.60-1.78 (2H, m), 2.21 (2H, s), 2.46 (3H, s), 2.62 (2H, s), 3.77 (3H, s), 4.20 (1H, dd, J = 15.6, 6.1 Hz), 4.27 (1H, dd, J = 15.6, 6.1 Hz), 4.46 (1H, td, J = 7.9, 5.5 Hz), 6.87 (1H, td, J = 7.3, 1.2 Hz), 6.95 (1H, dd, J = 7.9, 1.2 Hz), 7.16 (1H, dd, J = 7.3, 1.8 Hz), 7.22 (1H, td, J = 7.9, 1.8 Hz), 7.52 (1H, d, J = 7.9 Hz), 8.37 (1H, t, J = 6.1 Hz), 11.76 (1H, s). |
| | | HRMS (ESI⁺):454.27128 [M+H]⁺ |
| 1 - 4 5 | 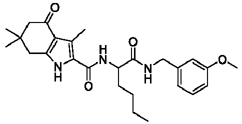 | ¹H-NMR (DMSO-D₆, 400MHz) δ: 0.80-0.90 (3H, m), 1.01 (6H, s), 1.23-1.32 (4H, m), 1.59-1.78 (2H, m), 2.21 (2H, s), 2.46 (3H, s), 2.63 (2H, s), 3.71 (3H, s), 4.23 (1H, dd, J = 15.3, 6.1 Hz), 4.29 (1H, dd, J = 15.3, 6.1 Hz), 4.43 (1H, td, J = 7.9, 5.5 Hz), 6.75-6.83 (3H, m), 7.20 (1H, d, J = 7.9 Hz), 7.51 (1H, d, J = 7.9 Hz), 8.55 (1H, t, J = 6.1 Hz), 11.72 (1H, s). |
| | | HRMS (ESI⁺):454.27001 [M+H]⁺ |
| 1 - 4 6 | 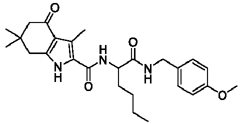 | ¹H-NMR (DMSO-D₆, 400MHz) δ: 0.84 (3H, t, J = 6.7 Hz), 1.01 (6H, s), 1.22-1.30 (4H, m), 1.57-1.75 (2H, m), 2.21 (2H, s), 2.46 (3H, s), 2.62 (2H, s), 3.70 (3H, s), 4.18 (1H, dd, J = 15.3, 6.1 Hz), 4.23 (1H, dd, J = 15.3, 6.1 Hz), 4.42 (1H, td, J = 7.9, 5.5 Hz), 6.85 (2H, d, J = 8.6 Hz), 7.16 (2H, d, J = 8.6 Hz), 7.48 (1H, d, J = 7.9 Hz), 8.49 (1H, t, J = 6.1 Hz), 11.74 (1H, s). |
| | | HRMS (ESI⁺):454.27009 [M+H]⁺ |
| 1 - 4 7 | 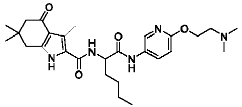 | ¹H-NMR (DMSO-D₆, 400MHz) δ: 0.87 (3H, t, J = 6.7 Hz), 1.01 (6H, s), 1.27-1.40 (4H, m), 1.64-1.83 (2H, m), 2.17 (6H, s), 2.21 (2H, s), 2.46 (3H, s), 2.57 (2H, t, J = 5.8 Hz), 2.63 (2H, s), 4.27 (2H, t, J = 5.8 Hz), 4.49-4.56 (1H, m), 6.77 (1H, d, J = 8.6 Hz), 7.72 (1H, d, J = 7.3 Hz), 7.89 (1H, dd, J = 8.6, 2.4 Hz), 8.35 (1H, d, J = 2.4 Hz), 10.19 (1H, s), 11.78 (1H, s). |
| | | HRMS (ESI⁺):498.30770 [M+H]⁺ |

**[Table 52]**

| Example | Structure | Equipment Data |
|---|---|---|
| 1 - 4 8 | 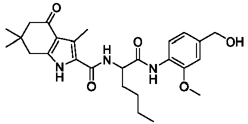 | ¹H-NMR (DMSO-D₆, 400MHz) δ: 0.88 (3H, t, J = 7.0 Hz), 1.02 (6H, s), 1.26-1.47 (4H, m), 1.63-1.90 (2H, m), 2.23 (2H, s), 2.49 (3H, s), 2.64 (2H, s), 3.79 (3H, s), 4.45 (2H, d, J = 5.5 Hz), 4.66 (1H, td, J = 8.2, 5.2 Hz), 5.15 (1H, t, J = 5.8 Hz), 6.85 (1H, d, J = 8.5 Hz), 6.99 (1H, d, J = 1.8 Hz), 7.66 (1H, d, J = 7.9 Hz), 7.86 (1H, d, J = 8.5 Hz), 9.22 (1H, s), 11.71 (1H, s). |
| | | HRMS (ESI⁺): 470.26480 [M+H]⁺ |
| 1 - 4 9 | 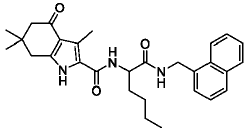 | ¹H-NMR (DMSO-D₆, 400MHz) δ: 0.80 (3H, t, J = 6.7 Hz), 1.01 (6H, s), 1.19-1.30 (4H, m), 1.59-1.74 (2H, m), 2.21 (2H, s), 2.46 (3H, s), 2.62 (2H, s), 4.46 (1H, td, J = 7.9, 5.4 Hz), 4.71 (1H, dd, J = 15.1, 5.4 Hz), 4.79 (1H, dd, J = 15.1, 5.4 Hz), 7.42-7.47 (2H, m), 7.50-7.58 (3H, m), 7.84 (1H, dd, J = 6.1, 3.0 Hz), 7.93 (1H, dd, J = 6.1, 3.0 Hz), 8.05 (1H, dd, J = 6.1, 3.0 Hz), 8.60 (1H, t, J = 5.4 Hz), 11.79 (1H, s). |
| | | HRMS (ESP):474.27602 [M+H]⁺ |
| 1 - 5 0 | 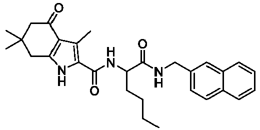 | ¹H-NMR (DMSO-D₆, 400MHz) δ: 0.84 (3H, t, J = 6.7 Hz), 1.01 (6H, s), 1.25-1.34 (4H, m), 1.61-1.80 (2H, m), 2.21 (2H, s), 2.48 (3H, s), 2.63 (2H, s), 4.39-4.51 (3H, m), 7.41 (1H, dd, J = 8.5, 1.8 Hz), 7.43-7.51 (2H , m), 7.54 (1H, d, J = 7.9 Hz), 7.74 (1H, d, J = 1.8 Hz), 7.81 (1H, dd, J = 7.3, 2.4 Hz), 7.84-7.89 (2H, m), 8.67 (1H, t, J = 5.4 Hz), 11.73 (1H, s). |
| | | HRMS (ESI⁺):474.27612 [M+H]⁺ |
| 1 - 5 1 | 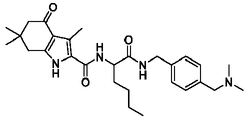 | ¹H-NMR (DMSO-D₆, 400MHz) δ: 0.84 (3H, t, J = 6.7 Hz), 1.01 (6H, s), 1.22-1.32 (4H, m), 1.59-1.76 (2H, m), 2.10 (6H, s), 2.21 (2H, s), 2.46 (3H, s), 2.63 (2H, s), 3.32 (2H, s), 4.24 (1H, dd, J = 15.1, 6.1 Hz), 4.30 (1H, dd, J = 15.1, 6.1 Hz), 4.43 (1H, td, J = 7.9, 5.4 Hz), 7.12-7.23 (4H, m), 7.43 (1H, d, J = 7.9 Hz), 8.53 (1H, t, J = 5.4 Hz), 11.66 (1H, s). |
| | | HRMS (ESI⁺):481.31701 [M+H]⁺ |

**[Table 53]**

| Example | Structure | Equipment Data |
|---|---|---|
| 1 - 5 2 | 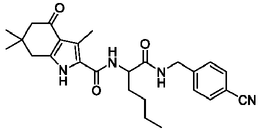 | ¹H-NMR (DMSO-D₆, 400MHz) δ: 0.80-0.90 (3H, m), 1.01 (6H, s), 1.21-1.33 (4H, m), 1.61-1.78 (2H, m), 2.21 (2H, s), 2.46 (3H, s), 2.63 (2H, s), 4.36 (2H, d, J = 6.1 Hz), 4.42 (1H, td, J = 7.9, 5.4 Hz), 7.43 (2H, d, J = 8.5 Hz), 7.48 (1H, d, J = 7.9 Hz), 7.77 (2H, d, J = 8.5 Hz), 8.66 (1H, t, J = 6.1 Hz), 11.65 (1H, s). |
| | | HRMS (ESI⁻):447.23983 [M-H]⁻ |
| 1 - 5 3 | 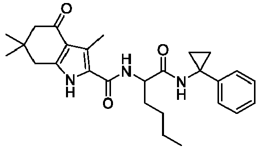 | ¹H-NMR (DMSO-D₆, 400MHz) δ: 0.85 (3H, t, J = 6.7 Hz), 1.01 (6H, s), 1.08-1.13 (2H, m), 1.14-1.19 (2H, m), 1.23-1.32 (4H, m), 1.58-1.76 (2H, m), 2.21 (2H, s), 2.47 (3H, s), 2.63 (2H, s), 4.40 (1H, td, J = 7.9, 5.4 Hz), 7.10-7.15 (3H, m), 7.21-7.25 (2H, m), 7.38 (1H, d, J = 7.9 Hz), 8.77 (1H, s), 11.67 (1H, s). |
| | | HRMS (ESI⁺):450.27506 [M+H]⁺ |
| 1 - 5 4 | 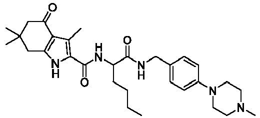 | ¹H-NMR (DMSO-D₆, 400MHz) δ: 0.80-0.90 (3H, m), 1.01 (6H, s), 1.21-1.30 (4H, m), 1.57-1.75 (2H, m), 2.19 (3H, s), 2.21 (2H, s), 2.41 (4H, t, J = 4.8 Hz), 2.46 (3H, s), 2.62 (2H, s), 3.07 (4H, t, J = 4.8 Hz), 4.14 (1H, dd, J = 15.1, 6.1 Hz), 4.20 (1H, dd, J = 15.1, 6.1 Hz), 4.42 (1H, td, J = 7.9, 5.4 Hz), 6.85 (2H, d, J = 8.5 Hz), 7.08 (2H, d, J = 8.5 Hz), 7.40 (1H, d, J = 7.9 Hz), 8.41 (1H, t, J = 6.1 Hz), 11.66 (1H, s). |
| | | HRMS (ESI⁺):522.34462 [M+H]⁺ |
| 1 - 5 5 | 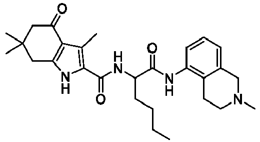 | ¹H-NMR (DMSO-D₆, 400MHz) δ: 0.88 (3H, t, J = 7.3 Hz), 1.01 (6H, s), 1.29-1.44 (4H, m), 1.69-1.88 (2H, m), 2.21 (2H, s), 2.31 (3H, s), 2.47 (3H, s), 2.53-2.70 (6H, m), 3.45 (2H, s), 4.57-4.66 (1H, m), 6.88 (1H, d, J = 7.9 Hz), 7.09 (1H, t, J = 7.9 Hz), 7.21 (1H, d, J = 7.3 Hz), 7.52-7.75 (1H, m), 9.40 (1H, s), 11.70 (1H, br s). |
| | | HRMS (ESI⁺): 479.30239 [M+H]⁺ |

**[Table 54]**

| Example | Structure | Equipment Data |
|---|---|---|
| 1 - 5 6 | 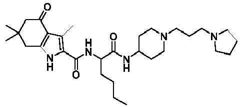 | ¹H-NMR (DMSO-D₆, 400MHz) δ: 0.88 (3H, t, J = 6.7 Hz), 1.04 (6H, s), 1.22-1.49 (6H, m), 1.53-1.76 (10H, m), 1.88-1.99 (2H, m), 2.21-2.32 (4H, m), 2.34-2.43 (6H, m), 2.49 (3H, s), 2.66 (2H, s), 2.73-2.84 (2H, m), 3.47-3.61 (1H, m), 4.38-4.49 (1H, m), 7.38 (1H, d, J = 7.9 Hz), 7.99 (1H, d, J = 7.9 Hz), 11.72 (1H, s). |
| | | HRMS (ESI⁺): 528.39144 [M+H]⁺ |
| 1 - 5 7 | 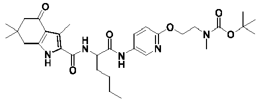 | ¹H-NMR (DMSO-D₆, 400MHz) δ: 0.86 (3H, t, J = 6.7 Hz), 1.01 (6H, s), 1.29 (9H, s), 1.30-1.39 (4H, m), 1.67-1.82 (2H, m), 2.21 (2H, s), 2.46 (3H, s), 2.63 (2H, s), 2.80 (3H, s), 3.46-3.53 (2H, m), 4.28-4.35 (2H, m), 4.53 (1H, td, J = 8.6, 5.5 Hz), 6.77 (1H, d, J = 8.6 Hz), 7.70 (1H, d, J = 8.6 Hz), 7.91 (1H, dd, J = 8.6, 2.4 Hz), 8.36 (1H, d, J = 2.4 Hz), 10.19 (1H, s), 11.76 (1H, s). |
| | | HRMS (ESI⁺):584.34497 [M+H]⁺ |
| 1 - 5 8 | 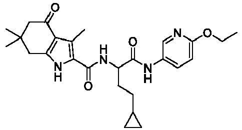 | ¹H-NMR (DMSO-D₆, 400MHz) δ: -0.02-0.04 (2H, m), 0.35-0.42 (2H, m), 0.65-0.76 (1H, m), 1.01 (6H, s), 1.21-1.35 (5H, m), 1.74-1.94 (2H, m), 2.21 (2H, s), 2.46 (3H, s), 2.63 (2H, s), 4.24 (2H, q, J = 7.1 Hz), 4.52-4.61 (1H, m), 6.76 (1H, d, J = 8.8 Hz), 7.62 (1H, d, J = 7.9 Hz), 7.89 (1H, dd, J = 8.8, 3.0 Hz), 8.34 (1H, d, J = 3.0 Hz), 10.14 (1H, s), 11.66 (1H, s). |
| | | HRMS (ESI⁺): 467.26546 [M+H]⁺ |
| 1 - 5 9 | 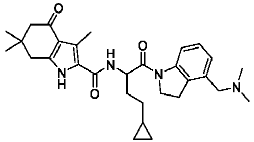 | ¹H-NMR (DMSO-D₆, 400MHz) δ: 0.02-0.07 (2H, m), 0.38-0.43 (2H, m), 0.70-0.77 (1H, m), 1.02 (6H, s), 1.29-1.36 (2H, m), 1.75-1.84 (1H, m), 1.87-1.95 (1H, m), 2.15 (6H, s), 2.22 (2H, s), 2.45 (3H, s), 2.64 (2H, s), 3.17-3.23 (2H, m), 3.34 (2H, s), 4.18-4.26 (1H, m), 4.30-4.37 (1H, m), 4.76-4.83 (1H, m), 6.95 (1H, d, J = 7.9 Hz), 7.13 (1H, t, J = 7.9 Hz), 7.79 (1H, d, J = 7.9 Hz), 8.01 (1H, d, J = 7.9 Hz), 11.65 (1H, s). |
| | | HRMS (ESI⁺):505.31725 [M+H]⁺ |

**[Table 55]**

| Example | Structure | Equipment Data |
|---|---|---|
| 1 - 6 0 | 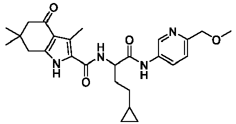 | ¹H-NMR (DMSO-D₆, 400MHz) δ: -0.02-0.05 (2H, m), 0.35-0.43 (2H, m), 0.64-0.78 (1H, m), 1.01 (6H, s), 1.19-1.39 (2H, m), 1.74-1.97 (2H, m), 2.21 (2H, s), 2.46 (3H, s), 2.63 (2H, s), 3.32 (3H, s), 4.42 (2H, s), 4.53-4.63 (1H, m), 7.36 (1H, d, J = 8.5 Hz), 7.65 (1H, d, J = 7.3 Hz), 8.05 (1H, dd, J = 8.5, 2.4 Hz), 8.70 (1H, d, J = 2.4 Hz), 10.35 (1H, s), 11.65 (1H, s). |
| | | HRMS (ESI⁺): 467.26568 [M+H]⁺ |
| 1 - 6 1 | 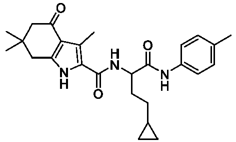 | ¹H-NMR (DMSO-D₆, 400MHz) δ: 0.00-0.03 (2H, m), 0.37-0.42 (2H, m), 0.70-0.73 (1H, m), 1.02 (6H, s), 1.22-1.33 (2H, m), 1.76-1.92 (2H, m), 2.22 (2H, s), 2.25 (3H, s), 2.47 (3H, s), 2.64 (2H, s), 4.58 (1H, td, J = 8.6, 4.9 Hz), 7.11 (2H, d, J = 8.6 Hz), 7.49 (2H, d, J = 8.6 Hz), 7.61 (1H, d, J = 8.6 Hz), 10.04 (1H, s), 11.70 (1H, s). |
| | | HRMS (ESI⁺):436.25952 [M+H]⁺ |
| 1 - 6 2 | 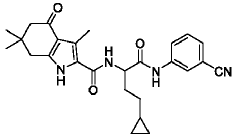 | ¹H-NMR (DMSO-D₆, 400MHz) δ: 0.01-0.05 (2H, m), 0.38-0.42 (2H, m), 0.70-0.74 (1H, m), 1.02 (6H, s), 1.24-1.35 (2H, m), 1.79-1.94 (2H, m), 2.23 (2H, s), 2.47 (3H, s), 2.65 (2H, s), 4.56 (1H, td, J = 7.9, 4.9 Hz), 7.52-7.57 (2H, m), 7.71 (1H, d, J = 7.9 Hz), 7.82-7.86 (1H, m), 8.12 (1H, d, J = 1.2 Hz), 10.50 (1H, s), 11.67 (1H, s). |
| | | HRMS (ESI⁺):447.23865 [M+H]⁺ |
| 1 - 6 3 | 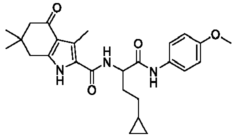 | ¹H-NMR (DMSO-D₆, 400MHz) δ: 0.00-0.04 (2H, m), 0.37-0.41 (2H, m), 0.68-0.75 (1H, m), 1.02 (6H, s), 1.24-1.32 (2H, m), 1.77-1.92 (2H, m), 2.22 (2H, s), 2.47 (3H, s), 2.64 (2H, s), 3.72 (3H, s), 4.57 (1H, td, J = 8.6, 6.1 Hz), 6.88 (2H, d, J = 8.6 Hz), 7.51 (2H, d, J = 8.6 Hz), 7.58 (1H, d, J = 8.6 Hz), 9.99 (1H, s), 11.68 (1H, s). |
| | | HRMS (ESI⁺):452.25496 [M+H]⁺ |
| 1 - 6 4 | 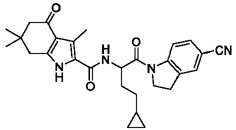 | ¹H-NMR (DMSO-D₆, 400MHz) δ: 0.02-0.07 (2H, m), 0.38-0.44 (2H, m), 0.69-0.77 (1H, m), 1.02 (6H, s), 1.30-1.37 (2H, m), 1.75-1.85 (1H, m), 1.88-1.97 (1H, m), 2.22 (2H, s), 2.45 (3H, s), 2.64 (2H, s), 3.22-3.29 (2H, m), 4.25-4.41 (2H, m), 4.77 (1H, brs), 7.65 (1H, d, J = 8.6 Hz), 7.71 (1H, s), 7.87 (1H, d, J = 7.9 Hz), 8.18 (1H, d, J = 8.6 Hz), 11.62 (1H, s). |
| | | HRMS (ESI⁺):473.25507 [M+H]⁺ |

**[Table 56]**

| Example | Structure | Equipment Data |
|---|---|---|
| 1 - 6 5 | 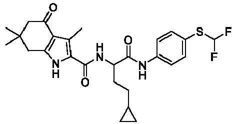 | ¹H-NMR (DMSO-D₆, 400MHz) δ: -0.08-0.08 (2H, m), 0.30-0.44 (2H, m), 0.62-0.76 (1H, m), 1.00 (6H, s), 1.17-1.34 (2H, m), 1.73-1.94 (2H, m), 2.20 (2H, s), 2.44 (3H, s), 2.62 (2H, s), 4.49-4.62 (1H, m), 7.19-7.39 (1H, m), 7.43-7.77 (5H, m), 10.35 (1H, s), 11.64 (1H, s). |
| | | HRMS (ESI⁺):504.21333 [M+H]⁺ |
| 1 - 6 6 | 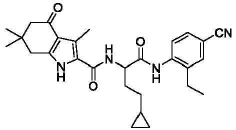 | ¹H-NMR (DMSO-D₆, 400MHz) δ: -0.03-0.03 (2H, m), 0.33-0.41 (2H, m), 0.65-0.74 (1H, m), 0.99 (6H, s), 1.05-1.15 (3H, m), 1.25-1.33 (2H, m), 1.78-1.96 (2H, m), 2.19 (2H, s), 2.47 (3H, s), 2.60-2.66 (4H, m), 4.66 (1H, brs), 7.59-7.74 (4H, m), 9.66 (1H, s), 11.63 (1H, s). |
| | | HRMS (ESI⁺):475.27143 [M+H]⁺ |
| 1 - 6 7 | 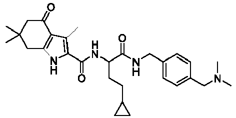 | ¹H-NMR (DMSO-D₆, 400MHz) δ: -0.03-0.02 (2H, m), 0.38-0.42 (2H, m), 0.65-0.75 (1H, m), 1.05 (6H, s), 1.20-1.27 (2H, m), 1.74-1.90 (2H, m), 2.14 (6H, s), 2.25 (2H, s), 2.49 (3H, s), 2.66 (2H, s), 3.36 (2H, s), 4.27 (1H, dd, J = 15.1, 6.1 Hz), 4.33 (1H, dd, J = 15.1, 6.1 Hz), 4.50 (1H, td, J = 8.5, 5.4 Hz), 7.21 (2H, d, J = 8.5 Hz), 7.24 (2H, d, J = 8.5 Hz), 7.35-7.58 (1H, m), 8.56 (1H, t, J = 6.1 Hz), 11.69 (1H, s). |
| | | HRMS (ESI⁺):493.31802 [M+H]⁺ |
| 1 - 6 8 | 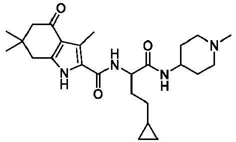 | ¹H-NMR (DMSO-D₆, 400MHz) δ: -0.04-0.03 (2H, m), 0.35-0.44 (2H, m), 0.63-0.75 (1H, m), 1.03 (6H, s), 1.15-1.27 (2H, m), 1.35-1.50 (2H, m), 1.62-1.85 (4H, m), 1.87-1.98 (2H, m), 2.14 (3H, s), 2.23 (2H, s), 2.47 (3H, s), 2.62-2.74 (4H, m), 3.45-3.57 (1H, m), 4.40-4.48 (1H, m), 7.36 (1H, d, J = 7.9 Hz), 7.96 (1H, d, J = 7.9 Hz), 11.69 (1H, s). |
| | | HRMS (ESI⁺): 443.30260 [M+H]⁺ |
| 1 - 6 9 | 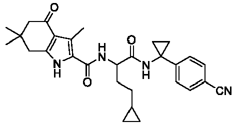 | ¹H-NMR (DMSO-D₆, 400MHz) δ: -0.04-0.02 (2H, m), 0.35-0.42 (2H, m), 0.63-0.75 (1H, m), 1.01 (6H, s), 1.17-1.36 (6H, m), 1.66-1.90 (2H, m), 2.21 (2H, s), 2.47 (3H, s), 2.63 (2H, s), 4.36-4.45 (1H, m), 7.30 (2H, d, J = 8.6 Hz), 7.43 (1H, d, J = 7.3 Hz), 7.70 (2H, d, J = 8.6 Hz), 8.87 (1H, s), 11.63 (1H, s). |
| | | HRMS (ESI⁺): 487.27040 [M+H]⁺ |

**[Table 57]**

| Example | Structure | Equipment Data |
|---|---|---|
| 1 - 7 0 | 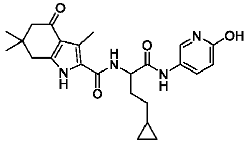 | ¹H-NMR (DMSO-D₆, 400MHz) δ: -0.04-0.04 (2H, m), 0.33-0.43 (2H, m), 0.63-0.75 (1H, m), 1.01 (6H, s), 1.18-1.34 (2H, m), 1.69 - 1.93 (2H, m), 2.21 (2H, s), 2.45 (3H, s), 2.63 (2H, s), 4.45-4.54 (1H, m), 6.34 (1H, d, J = 9.7 Hz), 7.46 (1H, dd, J = 9.7, 3.0 Hz), 7.59 (1H, d, J = 7.9 Hz), 7.83 (1H, d, J = 3.0 Hz), 9.88 (1H, s), 11.36 (1H, brs), 11.64 (1H, s). |
| | | HRMS (ESI⁺): 439.23488 [M+H]⁺ |
| 1 - 7 1 | 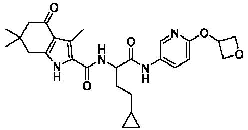 | ¹H-NMR (DMSO-D₆, 400MHz) δ: -0.03-0.04 (2H, m), 0.34-0.42 (2H, m), 0.63-0.77 (1H, m), 1.01 (6H, s), 1.19-1.36 (2H, m), 1.73-1.94 (2H, m), 2.21 (2H, s), 2.45 (3H, s), 2.63 (2H, s), 4.48-4.61 (3H, m), 4.82-4.90 (2H, m), 5.46-5.54 (1H, m), 6.88 (1H, d, J = 8.5 Hz), 7.62 (1H, d, J = 7.9 Hz), 7.94 (1H, dd, J = 8.5, 3.0 Hz), 8.31 (1H, d, J = 3.0 Hz), 10.19 (1H, s), 11.65 (1H, s). |
| | | HRMS (ESI⁺): 495.26094 [M+H]⁺ |
| 1 - 7 2 | 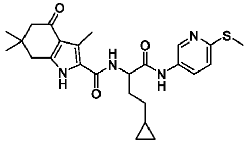 | ¹H-NMR (DMSO-D₆, 400MHz) δ: -0.02-0.05 (2H, m), 0.35-0.42 (2H, m), 0.65-0.77 (1H, m), 1.01 (6H, s), 1.20-1.36 (2H, m), 1.73-1.96 (2H, m), 2.21 (2H, s), 2.45 (3H, s), 2.47 (3H, s), 2.63 (2H, s), 4.52-4.61 (1H, m), 7.26 (1H, d, J = 8.5 Hz), 7.65 (1H, d, J = 7.9 Hz), 7.91 (1H, dd, J = 8.5, 2.4 Hz), 8.65 (1H, d, J = 2.4 Hz), 10.29 (1H, s), 11.65 (1H, s). |
| | | HRMS (ESI⁺): 469.22655 [M+H]⁺ |
| 1 - 7 3 | 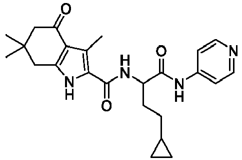 | ¹H-NMR (DMSO-D₆, 400MHz) δ: -0.05-0.04 (2H, m), 0.32-0.42 (2H, m), 0.62-0.75 (1H, m), 1.00 (6H, s), 1.15-1.41 (2H, m), 1.70-1.95 (2H, m), 2.21 (2H, s), 2.44 (3H, s), 2.62 (2H, s), 4.46-4.59 (1H, m), 7.57 (2H, d, J = 6.1 Hz), 7.68 (1H, d, J = 7.3 Hz), 8.41 (2H, d, J = 6.1 Hz), 10.50 (1H, s), 11.63 (1H, s). |
| | | HRMS (ESI⁺): 423.23947 [M+H]⁺ |
| 1 - 7 4 | 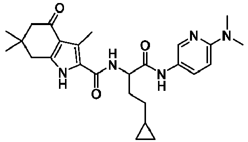 | ¹H-NMR (DMSO-D₆, 400MHz) δ: -0.05-0.05 (2H, m), 0.32-0.40 (2H, m), 0.65-0.75 (1H, m), 1.00 (6H, s), 1.20-1.33 (2H, m), 1.69-1.92 (2H, m), 2.20 (2H, s), 2.45 (3H, s), 2.62 (2H, s), 2.96 (6H, s), 4.50-4.60 (1H, m), 6.60 (1H, d, J = 9.2 Hz), 7.54 (1H, d, J = 7.9 Hz), 7.70 (1H, dd, J = 9.2, 2.4 Hz), 8.23 (1H, d, J = 2.4 Hz), 9.88 (1H, s), 11.65 (1H, s). |
| | | HRMS (ESI⁺): 466.28206 [M+H]⁺ |

**[Table 58]**

| Example | Structure | Equipment Data |
|---|---|---|
| 1 - 7 5 | 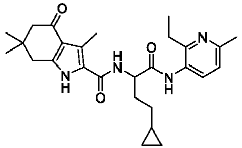 | ¹H-NMR (DMSO-D₆, 400MHz) δ: -0.05-0.05 (2H, m), 0.34-0.43 (2H, m), 0.65-0.77 (1H, m), 0.99 (6H, s), 1.10 (3H, t, J = 7.2 Hz), 1.25-1.33 (2H, m), 1.74-1.97 (2H, m), 2.20 (2H, s), 2.39 (3H, s), 2.45 (3H, s), 2.62 (2H, s), 2.63-2.69 (2H, m), 4.59-4.67 (1H, m), 7.03 (1H, d, J = 8.6 Hz), 7.53 (1H, d, J = 8.6 Hz), 7.56 (1H, d, J = 7.2 Hz), 9.58 (1H, s), 11.64 (1H, s). HRMS (ESI⁺): 465.28610 [M+H]⁺ |
| 1 - 7 6 | 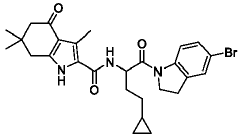 | ¹H-NMR (DMSO-D₆, 400MHz) δ: 0.02-0.07 (2H, m), 0.37-0.43 (2H, m), 0.69-0.76 (1H, m), 1.02 (6H, s), 1.28-1.35 (2H, m), 1.76-1.83 (1H, m), 1.87-1.94 (1H, m), 2.22 (2H, s), 2.45 (3H, s), 2.64 (2H, s), 3.18-3.25 (2H, m), 4.21-4.27 (1H, m), 4.30-4.37 (1H, m), 4.70-4.85 (1H, m), 7.34 (1H, dd, J = 8.6, 1.8 Hz), 7.45 (1H, d, J = 1.8 Hz), 7.81 (1H, d, J = 7.3 Hz), 8.01 (1H, d, J = 8.6 Hz), 11.62 (1H, s). HRMS (ESI⁺):526.17123 [M+H]⁺ |
| 1 - 7 7 | 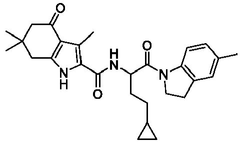 | ¹H-NMR (DMSO-D₆, 400MHz) δ: 0.02-0.07 (2H, m), 0.37-0.43 (2H, m), 0.69-0.77 (1H, m), 1.02 (6H, s), 1.28-1.35 (2H, m), 1.73-1.84 (1H, m), 1.85-1.95 (1H, m), 2.22 (2H, s), 2.25 (3H, s), 2.45 (3H, s), 2.64 (2H, s), 3.16 (2H, t, J = 7.9 Hz), 4.15-4.24 (1H, m), 4.27-4.35 (1H, m), 4.76-4.83 (1H, m), 6.96 (1H, d, J = 7.9 Hz), 7.07 (1H, s), 7.76 (1H, d, J = 7.9 Hz), 7.96 (1H, d, J = 7.9 Hz), 11.63 (1H, s). HRMS (ESI⁺):462.27484 [M+H]⁺ |
| 1 - 7 8 | 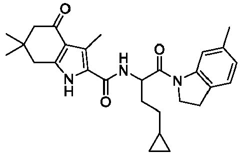 | ¹H-NMR (DMSO-D₆, 400MHz) δ: 0.00-0.04 (2H, m), 0.35-0.41 (2H, m), 0.67-0.75 (1H, m), 1.00 (6H, s), 1.30 (2H, q, J = 7.9 Hz), 1.72-1.82 (1H, m), 1.84-1.92 (1H, m), 2.20 (2H, s), 2.24 (3H, s), 2.43 (3H, s), 2.62 (2H, s), 3.11 (2H, t, J = 8.6 Hz), 4.14-4.21 (1H, m), 4.26-4.33 (1H, m), 4.76 (1H, td, J = 7.9, 5.5 Hz), 6.81 (1H, dd, J = 7.9, 1.8 Hz), 7.10 (1H, d, J = 7.9 Hz), 7.73 (1H, d, J = 7.9 Hz), 7.92 (1H, d, J = 1.8 Hz), 11.61 (1H, s). HRMS (ESI⁺):462.27534 [M+H]⁺ |

**[Table 59]**

| Example | Structure | Equipment Data |
|---|---|---|
| 1 - 7 9 | 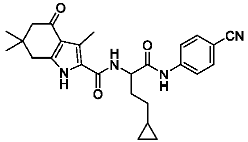 | ¹H-NMR (DMSO-D₆, 400MHz) δ: 0.01-0.05 (2H, m), 0.37-0.42 (2H, m), 0.68-0.75 (1H, m), 1.02 (6H, s), 1.22-1.36 (2H, m), 1.79-1.93 (2H, m), 2.22 (2H, s), 2.46 (3H, s), 2.64 (2H, s), 4.57 (1H, td, J = 7.9, 5.5 Hz), 7.71 (1H, d, J = 7.9 Hz), 7.78 (2H, d, J = 9.2 Hz), 7.81 (2H, d, J = 9.2 Hz), 10.58 (1H, s), 11.65 (1H, s). HRMS (ESI⁺):447.24011 [M+H]⁺ |
| 1 - 8 0 | 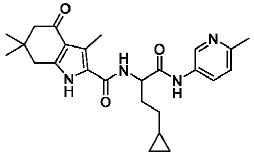 | ¹H-NMR (DMSO-D₆, 400MHz) δ: 0.01-0.05 (2H, m), 0.38-0.42 (2H, m), 0.68-0.76 (1H, m), 1.02 (6H, s), 1.22-1.34 (2H, m), 1.78-1.94 (2H, m), 2.22 (2H, s), 2.41 (3H, s), 2.47 (3H, s), 2.65 (2H, s), 4.59 (1H, td, J = 7.9, 5.5 Hz), 7.20 (1H, d, J = 8.6 Hz), 7.63 (1H, d, J = 7.9 Hz), 7.92 (1H, dd, J = 8.6, 2.4 Hz), 8.63 (1H, d, J = 2.4 Hz), 10.25 (1H, s), 11.66 (1H, s). HRMS (ESI⁺):437.25459 [M+H]⁺ |
| 1 - 8 1 | 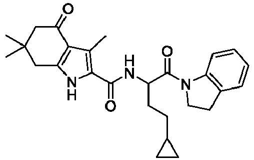 | ¹H-NMR (DMSO-D₆, 400MHz) δ: 0.02-0.08 (2H, m), 0.41 (2H, d, J = 7.9 Hz), 0.70-0.77 (1H, m), 1.02 (6H, s), 1.29-1.36 (2H, m), 1.75-1.83 (1H, m), 1.87-1.95 (1H, m), 2.22 (2H, s), 2.46 (3H, s), 2.64 (2H, s), 3.17-3.24 (2H, m), 4.18-4.25 (1H, m), 4.29-4.35 (1H, m), 4.75-4.90 (1H, m), 7.02 (1H, t, J = 7.3 Hz), 7.16 (1H, t, J = 7.3 Hz), 7.26 (1H, d, J = 7.3 Hz), 7.78 (1H, d, J = 7.3 Hz), 8.08 (1H, d, J = 7.3 Hz), 11.64 (1H, s). HRMS (ESI⁺):448.25997 [M+H]⁺ |
| 1 - 8 2 | 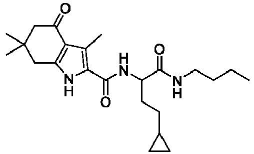 | ¹H-NMR (DMSO-D₆, 400MHz) δ: -0.04-0.01 (2H, m), 0.35-0.40 (2H, m), 0.63-0.71 (1H, m), 0.86 (3H, t, J = 7.3 Hz), 1.02 (6H, s), 1.16-1.32 (4H, m), 1.34-1.42 (2H, m), 1.65-1.74 (1H, m), 1.75-1.83 (1H, m), 2.22 (2H, s), 2.46 (3H, s), 2.63 (2H, s), 2.98-3.13 (2H, m), 4.41 (1H, td, J = 7.9, 5.4 Hz), 7.36 (1H, d, J = 7.9 Hz), 7.99 (1H, t, J = 5.4 Hz), 11.65 (1H, s). HRMS (ESI⁺):402.27615 [M+H]⁺ |

**[Table 60]**

| Example | Structure | Equipment Data |
|---|---|---|
| 1 - 8 3 | 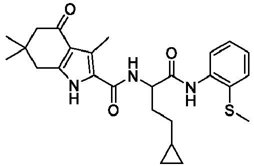 | ¹H-NMR (DMSO-D₆, 400MHz) δ: 0.01-0.07 (2H, m), 0.31-0.36 (1H, m), 0.38-0.43 (2H, m), 1.02 (6H, s), 1.30-1.37 (2H, m), 1.83-1.90 (1H, m), 1.95-2.04 (1H, m), 2.23 (2H, s), 2.37 (3H, s), 2.45 (2H, s), 3.95 (3H, s), 4.67 (1H, td, J = 7.9, 5.4 Hz), 7.17-7.23 (2H, m), 7.33-7.38 (1H, m), 7.43-7.48 (1H, m), 7.61 (1H, d, J = 7.9 Hz), 9.51 (1H, s), 11.68 (1H, s). HRMS (ESI⁺):468.23163 [M+H]⁺ |
| 1 - 8 4 | 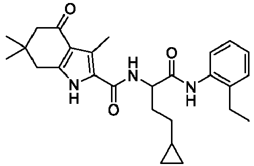 | ¹H-NMR (DMSO-D₆, 400MHz) δ: 0.01-0.06 (2H, m), 0.38-0.45 (2H, m), 0.70-0.78 (1H, m), 1.02 (6H, s), 1.09 (3H, t, J = 7.3 Hz), 1.28-1.37 (2H, m), 1.81-1.90 (1H, m), 1.91-2.00 (1H, m), 2.22 (2H, s), 2.48 (3H, s), 2.58 (2H, q, J = 7.3 Hz), 2.64 (2H, s), 4.67 (1H, td, J = 7.9, 5.4 Hz), 7.12-7.19 (2H, m), 7.23 (1H, dd, J = 7.3, 2.4 Hz), 7.32 (1H, dd, J = 7.3, 2.4 Hz), 7.57 (1H, d, J = 7.9 Hz), 9.47 (1H, s), 11.68 (1H, s). HRMS (ESI⁺):450.27621 [M+H]⁺ |
| 1 - 8 5 | 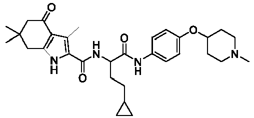 | ¹H-NMR (DMSO-D₆, 400MHz) δ: -0.01-0.02 (2H, m), 0.36-0.41 (2H, m), 0.67-0.74 (1H, m), 1.01 (6H, s), 1.23-1.30 (2H, m), 1.54-1.63 (2H, m), 1.77-1.90 (4H, m), 2.08-2.13 (2H, m), 2.15 (3H, s), 2.21 (2H, s), 2.46 (3H, s), 2.53-2.61 (2H, m), 2.63 (2H, s), 4.24-4.28 (1H, m), 4.55 (1H, td, J = 8.5, 5.4 Hz), 6.88 (2H, d, J = 9.1 Hz), 7.48 (2H, d, J = 9.1 Hz), 7.64 (1H, d, J = 8.5 Hz), 9.98 (1H, s), 11.75 (1H, s). HRMS (ESI⁺):535.32806 [M+H]⁺ |
| 1 - 8 6 | 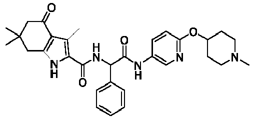 | ¹H-NMR (DMSO-D₆, 400MHz) δ: 1.00 (3H, s), 1.01 (3H, s), 1.53-1.67 (2H, m), 1.85-1.95 (2H, m), 2.05-2.16 (5H, m), 2.21 (2H, s), 2.48 (3H, s), 2.55-2.69 (4H, m), 4.83-4.92 (1H, m), 5.76 (1H, d, J = 7.3 Hz), 6.74 (1H, d, J = 9.1 Hz), 7.29-7.42 (3H, m), 7.50-7.57 (2H, m), 7.87 (1H, dd, J = 9.1, 3.0 Hz), 8.08-8.15 (1H, m), 8.33 (1H, d, J = 3.0 Hz), 10.47 (1H, s), 11.85 (1H, br s). HRMS (ESI⁺): 544.29288 [M+H]⁺ |

**[Table 61]**

| Example | Structure | Equipment Data |
|---|---|---|
| 1 - 8 7 | 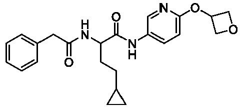 | ¹H-NMR (DMSO-D₆, 400MHz) δ: -0.12-0.01 (2H, m), 0.30-0.39 (2H, m), 0.59-0.72 (1H, m), 1.11-1.25 (2H, m), 1.62-1.86 (2H, m), 3.45 (1H, d, J = 13.9 Hz), 3.52 (1H, d, J = 13.9 Hz), 4.34-4.43 (1H, m), 4.53 (2H, dd, J = 7.9, 4.8 Hz), 4.83-4.90 (2H, m), 5.46-5.54 (1H, m), 6.88 (1H, d, J = 9.1 Hz), 7.17-7.31 (5H, m), 7.92 (1H, dd, J = 9.1, 2.4 Hz), 8.30 (1H, d, J = 2.4 Hz), 8.37 (1H, d, J = 7.9 Hz), 10.12 (1H, s). HRMS (ESI⁺): 410.20701 [M+H]⁺ |
| 1 - 8 8 | 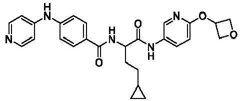 | ¹H-NMR (DMSO-D₆, 400MHz) δ: -0.03-0.08 (2H, m), 0.34-0.43 (2H, m), 0.65-0.76 (1H, m), 1.23-1.38 (2H, m), 1.84-1.95 (2H, m), 4.48-4.59 (3H, m), 4.86 (2H, t, J = 7.0 Hz), 5.46-5.54 (1H, m), 6.87 (1H, d, J = 9.2 Hz), 7.00 (2H, dd, J = 4.9, 1.8 Hz), 7.23 (2H, d, J = 8.6 Hz), 7.89 (2H, d, J = 8.6 Hz), 7.95 (1H, dd, J = 8.9, 2.4 Hz), 8.25 (2H, dd, J = 4.9, 1.8 Hz), 8.32 (1H, d, J = 2.4 Hz), 8.43 (1H, d, J = 7.3 Hz), 9.09 (1H, s), 10.14 (1H, s). HRMS (ESI⁺): 488.22928 [M+H]⁺ |
| 1 - 8 9 | 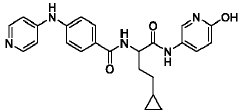 | ¹H-NMR (DMSO-D₆, 400MHz) δ: -0.04-0.07 (2H, m), 0.34-0.43 (2H, m), 0.64-0.75 (1H, m), 1.18-1.36 (2H, m), 1.87 (2H, q, J = 7.5 Hz), 4.49 (1H, q, J = 7.9 Hz), 6.34 (1H, d, J = 9.8 Hz), 7.00 (2H, dd, J = 4.9, 1.8 Hz), 7.23 (2H, d, J = 8.6 Hz), 7.47 (1H, dd, J = 9.8, 3.1 Hz), 7.84 (1H, d, J = 3.1 Hz), 7.88 (2H, d, J = 8.6 Hz), 8.25 (2H, dd, J = 4.9, 1.8 Hz), 8.40 (1H, d, J = 7.9 Hz), 9.09 (1H, s), 9.83 (1H, s), 11.29 (1H, s). HRMS (ESI⁺): 432.20395 [M+H]⁺ |
| 1 - 9 0 | 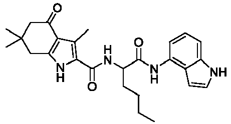 | MS (ESI⁺): 449.47[M+H]⁺ |
| 1 - 9 1 | 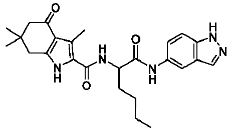 | MS (ESI⁺): 450.42[M+H]⁺ |

**[Table 62]**

| Example | Structure | Equipment Data |
|---|---|---|
| 1 - 9 2 | 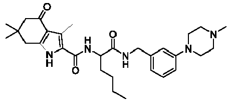 | MS (ESI⁺): 522.5[M+H]⁺ |
| 1 - 9 3 | 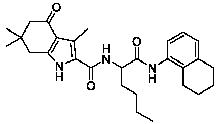 | MS (ESI⁺): 464.52[M+H]⁺ |
| 1 - 9 4 | 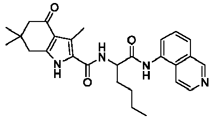 | MS (ESI⁺): 461.47[M+H]⁺ |
| 1 - 9 5 | 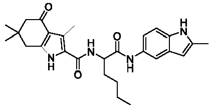 | MS (ESI⁺): 463.5[M+H]⁺ |
| 1 - 9 6 | 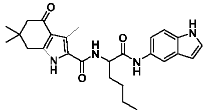 | MS (ESI⁺): 449.4[M+H]⁺ |
| 1 - 9 7 | 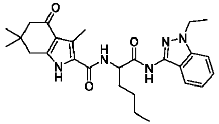 | MS (ESI⁺): 478.49[M+H]⁺ |
| 1 - 9 8 | 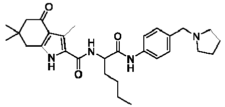 | MS (ESI⁺): 493.54[M+H]⁺ |
| 1 - 9 9 | 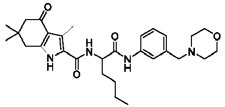 | MS (ESI⁺): 509.54[M+H]⁺ |
| 1 - 1 0 0 | 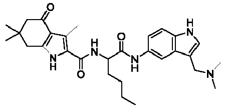 | MS (ESI⁺): 506.56[M+H]⁺ |

**[Table 63]**

| Example | Structure | Equipment Data |
|---|---|---|
| 1 - 1 0 1 | 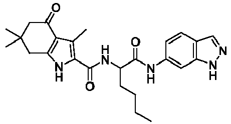 | MS (ESI⁺): 450.48[M+H]⁺ |
| 1 - 1 0 2 | 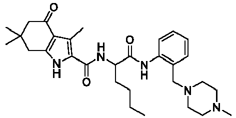 | MS (ESI⁺): 522.63[M+H]⁺ |
| 1 - 1 0 3 | 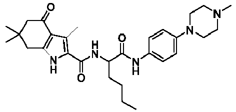 | MS (ESI⁺): 508.46[M+H]⁺ |
| 1 - 1 0 4 | 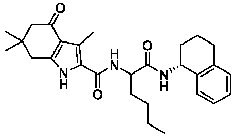 | MS (ESI⁺): 464.39[M+H]⁺ |
| 1 - 1 0 5 | 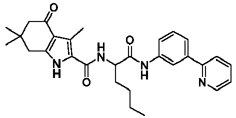 | MS (ESI⁺): 487.44[M+H]⁺ |
| 1 - 1 0 6 | 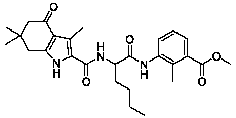 | MS (ESI⁺): 482.49[M+H]⁺ |
| 1 - 1 0 7 | 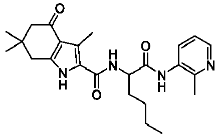 | MS (ESI⁺): 425.4[M+H]⁺ |
| 1 - 1 0 8 | 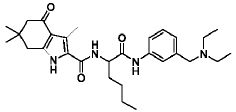 | MS (ESI⁺): 495.44[M+H]⁺ |

**[Table 64]**

| Example | Structure | Equipment Data |
|---|---|---|
| 1 - 1 0 9 | 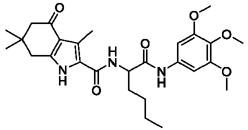 | MS (ESI⁺): 500.46[M+H]⁺ |
| 1 - 1 1 0 | 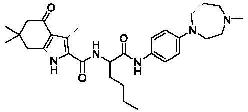 | MS (ESI⁺): 522.5[M+H]⁺ |
| 1 - 1 1 1 | 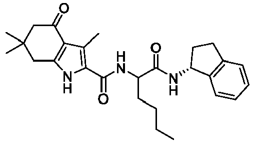 | MS (ESI⁺): 450.42[M+H]⁺ |
| 1 - 1 1 2 | 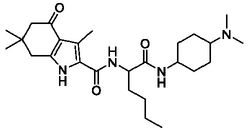 | MS (ESI⁺): 459.44[M+H]⁺ |
| 1 - 1 1 3 | 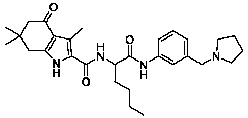 | MS (ESI⁺): 493.41[M+H]⁺ |
| 1 - 1 1 4 | 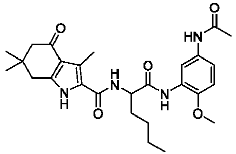 | MS (ESI⁺): 497.41[M+H]⁺ |
| 1 - 1 1 5 | 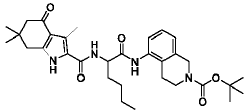 | ¹H-NMR (DMSO-D₆, 400MHz) δ: 0.89 (3H, t, J = 6.4 Hz), 1.01 (6H, s), 1.29-1.47 (14H, m), 1.65-1.90 (2H, m), 2.21 (2H, s), 2.63 (4H, s), 3.35-3.39 (2H, m), 3.44-3.57 (2H, m), 4.49 (2H, s), 4.55-4.64 (1H, m), 7.01 (1H, d, J = 6.7 Hz), 7.14-7.22 (2H, m), 7.62 (1H, brs), 9.55 (1H, s), 11.75 (1H, brs). HRMS (ESI⁺): 565.33957 [M+H]⁺ |

**[Table 65]**

| **Example** | | **¹HNMR Data** | **MS Data** [M+1]⁺ |
|---|---|---|---|
| 1-116 | 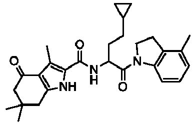 | ¹H-NMR (CDCl₃, 500 MHz) δ: -0.04-0.09 (2H, s), 0.37-0.47 (2H, m), 0.67-0.76 (1H, m), 1.07 (3H, s),1.08 (3H, s), 1.24-1.35 (1H, m), 1.38-1.48 (1H, m), 1.82-1.92 (1H, m), 1.99-2.07 (1H, m), 2.25 (3H, s), 2.33 (2H, s), 2.61 (2H, s), 2.69 (3H, s), 3.09-3.19 (2H, m), 4.15-4.22 (1H, m), 4.33-4.43 (1H, m), 5.02-5.11 (1H, m), 6.88 (1H, d, J= 7.7 Hz), 6.90 (1H, d, J= 7.7 Hz), 7.12 (1H, dd, J= 7.7, 7.7 Hz), 8.05 (1H, d, J=8.2 Hz), 9.33 (1H, br. s). | 462.36 |
| 1-117 | 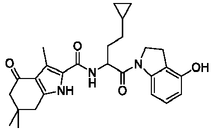 | ¹H-NMR (DMSO-D₆, 400 MHz) δ: -0.04-0.90 (2H, m), 0.36-0.45 (2H, m), 0.68-0.78 (1H, m), 1.02 (6H, s), 1.27-1.35 (2H, m), 1.72-1.84 (1H, m) 1.85-1.96 (1H, m), 2.22 (2H, s), 2.46 (3H, s), 2.64 (2H, s), 3.04 (2H, t, J = 8.7 Hz), 4.20 (1H, dt, J = 8.7, 8.7 Hz), 4.32 (1H, dt, J = 8.7, 8.7 Hz), 4.73-4.83 (1H, m), 6.50 (1H, d, J = 8.0 Hz), 6.97 (1H, dd, J = 8.0, 8.0 Hz), 7.57 (1H, d, J = 8.0 Hz), 7.77 (1H, d, J = 7.3 Hz), 9.48 (1H, s), 11.65 (1H, s). | 464.3 |
| 1-118 | 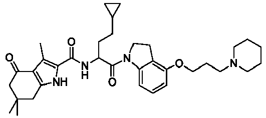 | ¹H-NMR (DMSO-D₆, 400 MHz) δ: -0.02-0.09 (2H, m), 0.32-0.47 (2H, m), 0.65-0.78 (1H, m), 1.01 (6H, s), 1.26-1.41 (4H, m), 1.41-1.53 (4H, m), 1.71-1.96 (4H, m), 2.21 (2H, s), 2.24-2.39 (6H, m), 2.47 (3H, s), 2.63 (2H, s), 3.06 (2H, t, J = 8.5 Hz), 4.01 (2H, t, J = 6.2 Hz), 4.22 (1H, dt, J = 8.5, 8.5 Hz), 4.34 (1H, dt, J = 8.5, 8.5 Hz), 4.73-4.86 (1H, m), 6.67 (1H, d, J = 8.2 Hz), 7.11 (1H, dd, J = 8.2, 8.2 Hz), 7.70 (1H, d, J = 8.2 Hz), 7.78 (1H, d, J = 7.3 Hz), 11.66 (1H, s). | 589.67 |
| 1-119 | 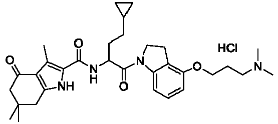 | ¹H-NMR (DMSO-D₆, 400 MHz) δ: 0.03-0.07 (2H, m), 0.35-0.45 (2H, m), 0.67-0.79 (1H, m), 1.02 (6H, s), 1.33 (2H, dt, J = 7.3, 7.3 Hz), 1.76-1.94 (2H, m), 2.09-2.19 (2H, m), 2.22 (2H, s), 2.45 (3H, s), 2.65 (2H, s), 2.75-2.79 (6H, m), 3.04-3.14 (2H, m), 3.16-3.23 (2H, m), 4.07-4.13 (2H, m), 4.23 (1H, dt, J = 9.2, 9.2 Hz), 4.36 (1H, dt, J = 9.2, 9.2 Hz), 4.70-4.80 (1H, m), 6.71 (1H, d, J = 8.2 Hz), 7.14 (1H, dd, J = 8.2, 8.2 Hz), 7.72 (1H, d, J = 7.8 Hz), 7.96 (1H, d, J = 7.3 Hz), 10.03-10.60 (1H, m), 11.93 (1H, s). | 549.66 |
| 1-120 | 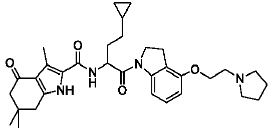 | ¹H-NMR (DMSO-D₆, 400 MHz) δ: -0.01-0.09 (2H, m), 0.35-0.46 (2H, m), 0.67-0.79 (1H, m), 1.02 (6H, s), 1.32 (2H, dt, J = 7.3, 7.3 Hz), 1.62-1.74 (4H, m), 1.74-1.98 (2H, m), 2.22 (2H, s), 2.46 (3H, s), 2.49-2.59 (4H, m), 2.64 (2H, s), 2.78 (2H, t, J = 5.7 Hz), 3.07 (2H, t, J = 8.7 Hz), 4.10 (2H, t, J = 5.8 Hz), 4.23 (1H, dt, J = 8.7 Hz), 4.32 (1H, dt, J = 8.7 Hz), 4.73-4.84 (1H, m), 6.70 (1H, d, J = 8.0 Hz), 7.12 (1H, dd, J = 8.0, 8.0 Hz), 7.70 (1H, d, J = 8.0 Hz), 7.77 (1H, d, J = 7.8 Hz), 1 1.63 (1H, s). | 561.65 |
| 1-121 | 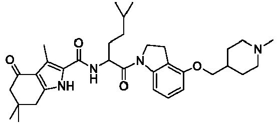 | ¹H-NMR (DMSO-D₆, 400 MHz) δ: -0.03-0.10 (2H, m), 0.34-0.47 (2H, m), 0.66-0.79 (1H, m), 1.02 (6H, s), 1.23-1.37 (4H, m), 1.61-1.97 (7H, m), 2.14 (3H, s), 2.22 (2H, s), 2.46 (3H, s), 2.64 (2H, s), 2.73-2.80 (2H, m), 3.07 (2H, t, J = 9.1 Hz), 3.80-3.90 (2H, m), 4.22 (1H, dt, J = 9.1, 9.1 Hz), 4.34 (1H, dt, J = 9.1, 9.1 Hz), 4.73-4.83 (1H, m), 6.68 (1H, d, J = 8.2 Hz), 7.11 (1H, dd, J = 8.2, 8.2 Hz), 7.69 (1H, d, J = 8.2 Hz), 7.77 (1H, d, J = 7.8 Hz), 11.63 (1H, s). | 575.66 |
| 1-122 | 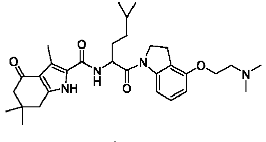 | ¹H-NMR (DMSO-D₆, 400 MHz) δ: -0.03-0.11 (2H, m), 0.33-0.48 (2H, m), 0.66-0.80 (1H, m), 1.02 (6H, s), 1.32 (2H, dt, J = 7.3, 7.3 Hz), 1.72-1.84 (1H, m), 1.84-1.96 (1H, m), 2.21-2.24 (8H, m), 2.46 (3H, s), 2.59-2.69 (4H, m), 3.06 (2H, t, J = 8.7 Hz), 4.09 (2H, t, J = 5.7 Hz), 4.22 (1H, dt, J = 8.7 Hz), 4.34 (1H, dt, J = 8.7 Hz), 4.73 -4.83 (1H, m), 6.71 (1H, d, J = 8.2 Hz), 7.13 (1H, dd, J = 8.2, 8.2 Hz), 7.70 (1H, d, J = 8.2 Hz), 7.77 (1H, d, J = 7.8 Hz), 11.64 (1H, s). | 535.54 |
| 1-123 | 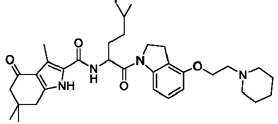 | ¹H-NMR (DMSO-D₆, 400 MHz) δ: -0.02-0.10 (2H, m), 0.33-0.48 (2H, m), 0.67-0.79 (1H, m), 1.02 (6H, s), 1.23-1.41 (4H, m), 1.45-1.53 (4H, m), 1.72-1.84 (1H, m), 1.84-1.96 (1H, m), 2.22 (2H, s), 2.40-2.47 (7H, m), 2.61-2.69 (4H, m), 3.06 (2H, t, J = 8.8 Hz), 4.10 (2H, t, J = 5.9 Hz), 4.22 (1H, dt, J = 8.8, 8.8 Hz), 4.33 (1H, dt, J = 8.8, 8.8 Hz), 4.73-4.83 (1H, m), 6.71 (1H, d, J = 8.0 Hz), 7.12 (1H, dd, J = 8.0, 8.0 Hz), 7.70 (1H, d, J = 8.0 Hz), 7.77 (1H, d, J = 7.6 Hz), 11.64 (1H, s). | 575.50 |

**[Table 66]**

| **Example** | | **¹HNMR Data** | **MS Data** [M+1]⁺ |
|---|---|---|---|
| 1-124 | 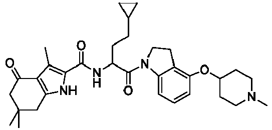 | ¹H-NMR (DMSO-D₆, 400 MHz) *δ* : -0.01-0.09 (2H, m), 0.35-0.46 (2H, m), 0.67-0.79 (1H, m), 1.02 (6H, s), 1.32 (2H, q, J = 7.3 Hz), 1.57-1.71 (2H, m), 1.72-1.98 (4H, m), 2.14-2.25 (7H, m), 2.46 (3H, s), 2.49-2.60 (2H, m), 2.64 (2H, s), 3.07 (2H, t, J = 8.5 Hz), 4.15-4.46 (3H, m), 4.72-4.83 (1H, m), 6.73 (1H, d, J = 8.2 Hz), 7.10 (1H, dd, J = 8.2, 8.2 Hz), 7.69 (1H, d, J = 8.2 Hz), 7.76 (1H, d, J = 7.8 Hz), 11.63 (1H, s). | 561.49 |
| 1-125 | 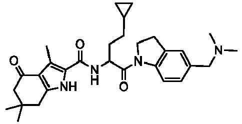 | ¹H-NMR (CDCl₃, 500 MHz) δ: -0.02-0.10 (2H, m), 0.39-0.48 (2H, m), 0.70-0.77 (1H, m), 1.09 (3H, s), 1.10 (3H, s), 1.26-1.35 (1H, m), 1.39-1.48 (1H, m), 1.83-1.92 (1H, m), 2.00-2.08 (1H, m), 2.24 (6H, s), 2.34 (2H, s), 2.63 (2H, s), 2.70 (3H, s), 3.21-3.27 (2H, m), 3.40 (2H, s), 4.18 (1H, q, J= 9.4 Hz), 4.37 (1H, q, J=9.5 Hz), 5.05-5.11 (1H, m), 6.79 (1H, d, J=8.2 Hz), 7.12 (1H, d, J=8.2 Hz), 7.21 (1H, s), 8.13 (1H, d, J=8.3 Hz), 9.15 (1H, br. s). | 505.44 |
| 1-126 | 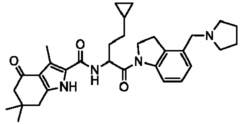 | ¹H-NMR (CDCl₃, 500 MHz) δ: -0.02-0.11 (2H, m), 0.38-0.47 (2H, m), 0.69-0.78 (1H, m), 1.09 (6H, s), 1.31 (1H, s), 1.26-1.35 (1H, m), 1.39-1.48 (1H, m), 1.77 (4H, m), 1.82-1.92 (1H, m), 2.00-2.09 (1H, m), 2.34 (2H, s), 2.49 (4H, m), 2.63 (2H, s), 2.69 (3H, s), 3.28 (1H, t, J=8.4, 8.4 Hz), 3.47-3.60 (2H, m), 4.18 (1H, q, J= 9.0, 9.0, 8.4 Hz), 4.37 (1H, q, J= 8.9, 8.9, 8.4 Hz), 5.06-5.11 (1H, m), 6.82 (1H, d, J=8.1 Hz), 7.05 (1H, d, J=7.3 Hz), 7.18 (1H, t, J=8.1, 7.3 Hz), 8.12 (1H, d, J=8.0 Hz), 9.18 (1H, br. s). | 531.42 |
| 1-127 | 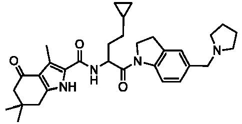 | ¹H-NMR (CDCl₃, 500 MHz) δ: -0.02-0.10 (2H, m), 0.39-0.48 (2H, m), 0.69-0.78 (1H, m), 1.09 (3H, s), 1.09 (3H, s), 1.24-1.35 (1H, m), 1.39-1.48 (1H, m), 1.75-1.82 (4H, m), 1.83-1.91 (1H, m), 2.00-2.08 (1H, m), 2.34 (2H, s), 2.47-2.54 (4H, m), 2.63 (2H, s), 2.69 (3H, s), 3.20-3.26 (2H, m), 3.57 (2H, s), 4.14-4.21 (1H, m), 4.32-4.39 (1H, m), 5.08 (1H, dt, J= 4.6, 8.1 Hz), 6.81 (1H, d, J= 8.1 Hz), 7.14 (1H, d, J= 8.1 Hz), 7.23 (1H, s), 8.12 (1H, d, J= 7.4 Hz), 9.18 (1H, br. s). | 531.49 |
| 1-128 | 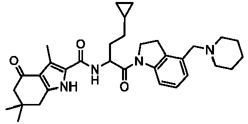 | 1H-NMR (CDCl₃, 500 MHz) δ: -0.02-0.11 (2H, m), 0.39-0.48 (2H, m), 0.69-0.78 (1H, m), 1.09 (3H, s), 1.09 (3H, s), 1.27-1.36 (1H, m), 1.39-1.48 (3H, m), 1.51-1.57 (4H, m), 1.83-1.92 (1H, m), 2.00-2.10 (1H, m), 2.30-2.40 (4H, m), 2.34 (2H, s), 2.63 (2H, s), 2.70 (3H, s), 3.28 (2H, t, J=8.5 Hz), 3.38 (2H, s), 4.17 (1H, ddd, J=9.5, 9.3, 8.5 Hz), 4.37 (1H, ddd, J=9.5, 9.3, 8.5 Hz), 5.09 (1H, td, J=8.2, 4.6 Hz), 6.83 (1H, d, J=8.0 Hz), 7.02 (1H, d, J=7.6 Hz), 7.17 (1H, dd, J=8.0, 7.6 Hz), 8.12 (1H, d, J=7.9 Hz), 9.17 (1H, br. s). | 545.45 |
| 1-129 | 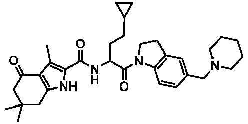 | ¹H-NMR (CDCl₃, 500 MHz) δ: -0.03-0.09 (2H, m), 0.37-0.46 (2H, m), 0.67-0.76 (1H, m), 1.06 (3H, s), 1.06 (3H, s), 1.25-1.34 (1H, m), 1.38-1.47 (3H, m), 1.52-1.58 (4H, m), 1.82-1.91 (1H, m), 1.99-2.07 (1H, m), 2.28 - 2.40 (m, 4H), 2.31 (2H, s), 2.59 (2H, s), 2.67 (3H, s), 3.16-3.28 (2H, m), 3.41 (2H, s), 4.13-4.19 (1H, m), 4.32-4.39 (1H, m), 5.06 (1H, ddd, J=8.2, 8.2, 4.6 Hz), 6.87 (1H, d, J=7.9 Hz), 7.11 (1H, d, J=7.9 Hz), 7.19 (1H, s), 8.10 (1H, d, J=8.2 Hz), 9.32 (1H, br. s). | 545.51 |
| 1-130 | 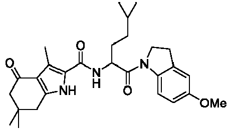 | ¹H-NMR (DMSO-D₆, 400 MHz) δ: -0.01-0.10 (2H, m), 0.34-0.46 (2H, m), 0.67-0.80 (1H, m), 1.02 (6H, s), 1.31 (2H, dt, J = 7.3, 7.3 Hz), 1.71-1.85 (1H, m), 1.85-1.98 (1H, m), 2.22 (2H, s), 2.46 (3H, s), 2.64 (2H, s), 3.18 (2H, t, J = 8.6 Hz), 3.72 (3H, s), 4.20 (1H, dt, J = 8.6, 8.6 Hz), 4.32 (1H, dt, J = 8.2, 8.2 Hz), 4.75-4.86 (1H, m), 6.72 (1H, dd, J = 8.7, 2.5 Hz), 6.87 (1H, d, J = 2.5 Hz), 7.76 (1H, d, J = 7.8 Hz), 8.00 (1H, d, J = 8.7 Hz), 11.64 (1H, s). | 478 |
| 1-131 | 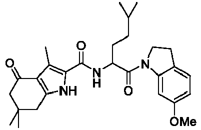 | ¹H-NMR (DMSO-D₆, 400 MHz) δ: -0.01-0.11 (2H, m), 0.35-0.47 (2H, m), 0.68-0.79 (1H, m), 1.02 (6H, s), 1.32 (2H, dt, J = 7.3 Hz), 1.73-1.85 (1H, m), 1.85-1.98 (1H, m), 2.22 (2H, s), 2.46 (3H, s), 2.65 (2H, s), 3.12 (2H, t, J = 8.5 Hz), 3.71 (3H, s), 4.23 (1H, dt, J =8.5, 8.5 Hz), 4.34 (1H, dt, J = 8.5, 8.5 Hz), 4.75-4.83 (1H, m), 6.60 (1H, dd, J = 8.2, 2.3 Hz), 7.14 (1H, d, J = 8.2 Hz), 7.75 (1H, d, J = 2.3 Hz), 7.78 (1H, d, J = 7.8 Hz), 11.64 (1H, s). | 478 |

**[Table 67]**

| Example | | **¹HNMR Data** | **MS Data [M+1]⁺** |
|---|---|---|---|
| 1-132 | 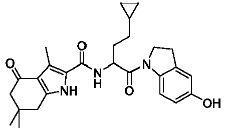 | ¹H-NMR (DMSO-D₆, 400 MHz) *δ*: -0.04-0.11 (2H, m), 0.32-0.47 (2H, m), 0.66-0.80 (1H, m), 1.02 (6H, s), 1.30 (2H, dt, J = 7.2, 7.2 Hz), 1.71-1.82 (1H, m), 1.82-1.95 (1H, m), 2.22 (2H, s), 2.46 (3H, s), 2.64 (2H, s), 3.12 (2H, t, J = 8.6 Hz), 4.16 (1H, dt, J = 8.6 , 8.6 Hz), 4.28 (1H, dt, J = 8.6, 8.6 Hz), 4.74-4.84 (1H, m), 6.53 (1H, dd, J = 8.6, 2.3 Hz), 6.66 (1H, d, J = 2.3 Hz), 7.73 (1H, d, J = 7.8 Hz), 7.89 (1H, d, J = 8.6 Hz), 9.20 (1H, s), 11.64 (1H, s). | 464 |
| 1-133 | 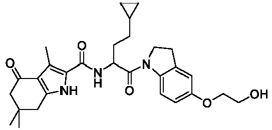 | ¹H-NMR (DMSO-D₆, 400 MHz) *δ* : -0.03-0.11 (2H, m), 0.33-0.48 (2H, m), 0.67-0.79 (1H, m), 1.03 (6H, d, J = 8.2 Hz), 1.31 (2H, dt, J = 7.3, 7.3 Hz), 1.72-1.85 (1H, m), 1.85-1.97 (1H, m), 2.22 (2H, s), 2.46 (3H, s), 2.64 (2H, s), 3.17 (2H, t, J = 8.6 Hz), 3.69 (2H, dt, J = 5.5, 5.0 Hz), 3.94 (2H, t, J = 5.0 Hz), 4.20 (1H, dt, J = 8.6, 8.6 Hz), 4.31 (1H, dt, J = 8.6, 8.6 Hz), 4.74-4.88 (2H, m), 6.72 (1H, dd, J = 8.7, 2.7 Hz), 6.87 (1H, d, J = 2.7 Hz), 7.76 (1H, d, J = 7.3 Hz), 7.99 (1H, d, J = 8.7 Hz), 11.64 (1H, s). | 508 |
| 1-134 | 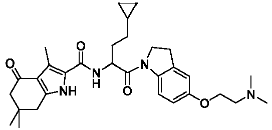 | ¹H-NMR (DMSO-D₆, 400 MHz) *δ* : -0.02-0.10 (2H, m), 0.34-0.46 (2H, m), 0.67-0.79 (1H, m), 1.02 (6H, s), 1.31(2H, dt, J = 7.3, 7.3 Hz), 1.71-1.85 (1H, m), 1.85-1.97 (1H, m), 2.21 (6H, s), 2.22 (2H, s), 2.46 (3H, s), 2.60 (2H, t, J = 5.9 Hz), 2.64 (2H, s), 3.17 (2H, t, J = 8.5 Hz), 4.00 (2H, t, J = 5.9 Hz), 4.20 (1H, dt, J =8.5, 8.5 Hz), 4.32 (1H, dt, J = 8.5, 8.5 Hz), 4.74-4.84 (1H, m), 6.72 (1H, dd, J = 8.7, 2.4 Hz), 6.87 (1H, d, J = 2.4 Hz), 7.77 (1H, d, J = 7.8 Hz), 7.99 (1H, d, J = 8.7 Hz), 11.65 (1H, s). | 535 |
| 1-135 | 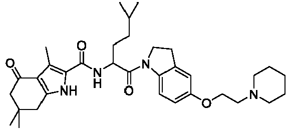 | ¹H-NMR (DMSO-D₆, 400 MHz) *δ* : -0.01-0.09 (2H, m), 0.34-0.46 (2H, m), 0.68-0.79 (1H, m), 1.02 (6H, s), 1.25-1.42 (4H, m), 1.44-1.53 (4H, m), 1.71-1.84 (1H, m), 1.84-1.97 (1H, m), 2.22 (2H, s), 2.36-2.44 (4H, m), 2.46 (3H, s), 2.58-2.67 (4H, m), 3.16 (2H, t, J = 8.6 Hz), 4.01 (2H, t, J = 6.0 Hz), 4.19 (1H, dt, J = 8.6, 8.6 Hz), 4.31 (1H, dt, J = 8.6, 8.6 Hz), 4.74-4.86 (1H, m), 6.72 (1H, dd, J = 8.7, 2.5 Hz), 6.87 (1H, d, J = 2.5 Hz), 7.76 (1H, d, J = 7.8 Hz), 7.98 (1H, d, J = 8.7 Hz), 11.64 (1H, s). | 575 |
| 1-136 | 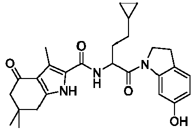 | ¹H-NMR (DMSO-D₆, 400 MHz) *δ* : -0.02-0.10 (2H, m), 0.35-0.46 (2H, m), 0.67-0.79 (1H, m), 1.02 (6H, s), 1.31 (2H, dt, J = 7.3, 7.3 Hz), 1.72-1.85 (1H, m), 1.85-1.98 (1H, m), 2.22 (2H, s), 2.46 (3H, s), 2.64 (2H, s), 3.06 (2H, t, J = 8.2 Hz), 4.19 (1H, dt, J = 10.0, 8.2 Hz), 4.31 (1H, dt, J = 10.2, 8.2 Hz), 4.73-4.84 (1H, m), 6.42 (1H, dd, J = 7.8, 2.3 Hz), 7.00 (1H, d, J = 7.8 Hz), 7.63 (1H, d, J = 2.3 Hz), 7.78 (1H, d, J = 7.3 Hz), 9.25 (1H, s), 11.65 (1H, s). | 464 |
| 1-137 | 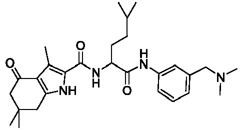 | ¹H-NMR (CDCl₃, 500 MHz) *δ* : -0.24- -0.17 (1H, m), -0.15- - 0.09 (1H, m), 0.18-0.33 (2H, m), 0.36-0.47 (1H, m), 1.06 (5H, s), 0.92-1.35 (1H, m), 1.84 (1H, s), 2.06 2.18 (2H, m), 2.33 (2H, s), 2.60 (2H, s), 2.70 (3H, s), 2.55-2.87 (6H, m), 3.27-3.37 (2H, m), 4.26-4.39 (1H, m), 5.52-5.62 (1H, m), 6.78-6.85 (1H, m), 7.08-7.28 (3H, m), 7.35-7.43 (1H, m), 9.92 (2H, s). | 479.43 |
| 1-138 | 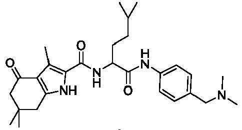 | ¹H-NMR (CDCl₃, 500 MHz) *δ* : -0.01-0.54 (2H, m), 0.41-0.46 (2H, m), 0.66-0.74 (1H, m), 1.09 (3H, s), 1.09 (3H, s), 1.33-1.40 (2H, m), 1.87-1.96 (1H, m), 2.13-2.20 (1H, m), 2.22 (6H, s), 2.34 (2H, s), 2.63 (2H, s), 2.65 (3H, s), 3.38 (2H, s), 4.73 (1H, q, J=7.8, 7.5, 7.5 Hz), 6.51 (1H, d, J=7.8 Hz), 7.25 (2H, d, J= 8.7 Hz), 7.48 (2H, d, J= 8.7 Hz), 8.34 (1H, s), 9.37 (1H, br. s). | 479.04 |
| 1-139 | 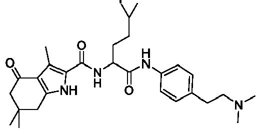 | ¹H-NMR (CDCl₃, 500 MHz) *δ* : -0.01-0.07 (2H, m), 0.39-0.47 (2H, m), 0.65-0.74 (1H, m), 1.09 (3H, s), 1.09 (3H, s), 1.31-1.41 (2H, m), 1.86-1.95 (1H, m), 2.12-2.22 (1H, m), 2.28 (6H, s), 2.34 (2H, s), 2.50 (2H, t, J=8.3 Hz), 2.63 (2H, s), 2.65 (3H, s), 2.74 (2H, t, J= 8.3 Hz), 4.72 (1H, q, J=7.4, 7.3 Hz), 6.49 (1H, d, J=7.4 Hz), 7.15 (2H, d, J= 8.4 Hz), 7.44 (2H, d, J= 8.4 Hz), 8.27 (1H, s), 9.32 (1H, br. s). | 493.49 |

**[Table 68]**

| Example | | **¹HNMR Data** | **MS Data [M+1]⁺** |
|---|---|---|---|
| 1-140 | 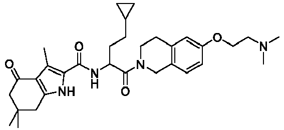 | ¹H-NMR (DMSO-D₆, 500 MHz, 333K) *δ* : -0.12-0.08 (2H, m), 0.31-0.44 (2H, m), 0.63-0.76 (1H, m), 1.02 (6H, s), 1.11-1.36 (2H, m), 1.62-1.89 (2H, m), 2.21 (2H, s), 2.29 (6H, s), 2.46 (3H, s), 2.62 (2H, s), 2.68-2.97 (4H, m), 3.60-3.89 (2H, m), 4.05 (2H, t, J = 5.7 Hz), 4.40-4.78 (2H, m), 5.01-5.08 (1H, m), 6.74-6.79 (2H, m), 7.06-7.15 (1H, m), 7.37-7.51 (1H, m), 11.53 (1H, s). | 549 |
| 1-141 | 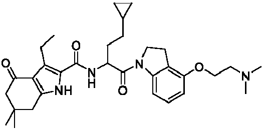 | ¹H-NMR (DMSO-D₆, 500 MHz) *δ* : -0.02-0.09 (2H, m), 0.33-0.48 (2H, m), 0.65-0.80 (1H, m), 0.97-1.08 (9H, m), 1.32 (2H, dt, J = 7.3, 7.3 Hz), 1.70-1.84 (1H, m), 1.84-1.98 (1H, m), 2.20-2.25 (8H, m), 2.60-2.68 (4H, m), 2.99 (2H, q, J = 7.3 Hz), 3.07 (2H, t, J = 8.7 Hz), 4.09 (2H, t, J = 5.7 Hz), 4.22 (1H, dt, J = 8.7, 8.7 Hz), 4.34 (1H, dt, J = 8.7, 8.7 Hz), 4.74-4.87 (1H, m), 6.71 (1H, d, J = 8.2 Hz,), 7.13 (1H, dd, J = 8.2, 8.2 Hz), 7.70 (1H, d, J = 8.2 Hz), 7.77 (1H, d, J = 7.3 Hz), 11.60 (1H, s). | 549 |
| 1-142 | 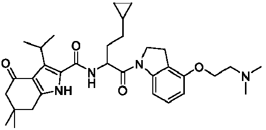 | ¹H-NMR (DMSO-D₆, 400 MHz) *δ* : -0.02-0.10 (2H, m), 0.35-0.47 (2H, m), 0.66-0.79 (1H, m), 1.02 (6H, s), 1.18 (6H, t, J = 7.0 Hz), 1.32 (2H, dt, J = 7.3, 7.3 Hz), 1.70-1.84 (1H, m), 1.84-1.97 (1H, m), 2.23 (6H, s), 2.25 (2H, s), 2.63 (2H, t, J = 5.8 Hz), 2.66 (2H, s), 3.07 (2H, t, J = 8.6 Hz), 3.94-4.05 (1H, m), 4.09 (2H, t, J = 5.8 Hz), 4.22 (1H, dt, J = 8.6, 8.6 Hz), 4.34 (1H, dt, J = 8.6, 8.6 Hz), 4.71-4.82 (1H, m), 6.71 (1H, d, J = 8.0 Hz), 7.13 (1H, dd, J = 8.0, 8.0 Hz), 7.70 (1H, d, J = 8.0 Hz), 7.89 (1H, d, J = 7.3 Hz), 11.54 (1H, s). | 563 |
| 1-143 | 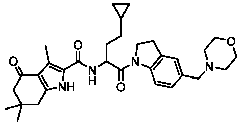 | ¹H-NMR (CDCl₃, 500 MHz) *δ* : -0.02-0.10 (2H, m), 0.39-0.48 (2H, m), 0.68-0.77 (1H, m), 1.09 (3H, s), 1.09 (3H, s), 1.24-1.35 (1H, m), 1.39-1.47 (1H, m), 1.83-1.92 (1H, m), 2.00-2.08 (1H, m), 2.33 (2H, s), 2.39-2.46 (4H, m), 2.61 (2H, s), 2.69 (3H, s), 3.18-3.30 (2H, m), 3.45 (2H, s), 3.66-3.75 (4H, m), 4.14-4.22 (1H, m), 4.34-4.41 (1H, m), 5.08 (1H, td, J=8.2, 4.6 Hz), 6.85 (1H d, J=8.1 Hz), 7.16 (1H, d, J=8.1), 7.21 (1H, s), 8.13 (1H, d, J=8.2 Hz), 9.36 (1H, br. s). | 547.45 |
| 1-144 | 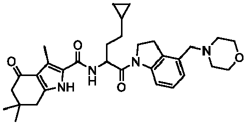 | ¹H-NMR (CDCl₃, 500 MHz) *δ* : -0.02-0.11 (2H, m), 0.40-0.47 (2H, m), 0.71-0.77 (1H, m), 1.09 (6H, s), 1.24-1.35 (1H, m), 1.40-1.48 (1H, m), 1.84-1.92 (1H, m), 2.00-2.10 (1H, m), 2.34 (2H, s), 2.42 (4H, s), 2.63 (2H, s), 2.70 (3H, s), 3.24-3.34 (2H, m), 3.44 (2H, s), 3.68 (4H, s), 4.19 (1H, dt, J=9.2, 9.4 Hz), 4.39 (1H, dt, J=9.0, 8.8 Hz), 5.09 (1H, d, J=4.9 Hz), 6.80 (1H, d, J=8.1 Hz), 7.01 (1H, d, J=7.5 Hz), 7.18 (1H, dd, J=8.1, 7.5 Hz), 8.14 (1H, d, J=8.1 Hz), 9.18 (1H, s). | 547.47 |
| 1-145 | 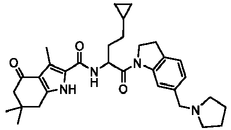 | ¹H-NMR (CDCl₃, 500 MHz) *δ* : -0.03-0.13 (2H, m), 0.38-0.49 (2H, m), 0.74 (1H, s), 1.10 (6H, s), 1.28-1.35 (1H, m), 1.39-1.49 (1H, m), 1.75-1.79 (4H, m), 1.81-1.91 (1H, m), 2.00-2.08 (1H, m), 2.34 (2H, s), 2.46-2.56 (4H, s), 2.64 (2H, s), 2.69 (3H, s), 3.23 (2H, s), 3.61 (2H, s), 4.14-4.23 (1H, m), 4.34-4.39 (1H, m), 5.05-5.12 (1H, m), 6.78 (1H, d, J=8.1 Hz), 7.10 (1H, d, J=7.5 Hz), 7.16 (1H, d, J=8.2 Hz), 8.16 (1H, s), 9.12 (1H, br. s). | 531.46 |
| 1-146 | 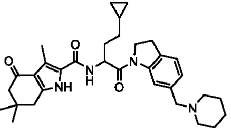 | ¹H-NMR (CDCl₃, 500 MHz) *δ* : -0.02-0.10 (2H, m), 0.40-0.48 (2H, m), 0.70-0.79 (1H, m), 1.09 (6H, s), 1.24-1.35 (1H, m), 1.39-1.46 (3H, m), 1.53-1.67 (4H, m), 1.85-1.90 (1H, m), 1.99-2.08 (1H, m), 2.34 (2H, s), 2.35-2.45 (4H, m), 2.63 (2H, s), 2.70 (3H, s), 3.19-3.26 (2H, m), 3.47 (2H, s), 4.16-4.23 (1H, m), 4.33-4.42 (1H, m), 5.05-5.13 (1H, m), 6.79 (1H, s), 7.08 (1H, d, J=7.7 Hz), 7.16 (1H, d, J=7.7 Hz), 8.13 (1H, s), 9.12-9.23 (1H, m). | 545.49 |
| 1-147 | 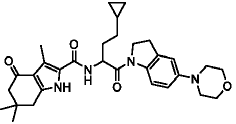 | 1H-NMR (DMSO-D6, 400 MHz) *δ* : -0.03-0.12 (2H, m), 0.33-0.48 (2H, m), 0.66-0.82 (1H, m), 1.02 (6H, s), 1.31 (2H, dt, J = 7.2, 7.2 Hz), 1.70-1.84 (1H, m), 1.84-1.97 (1H, m), 2.22 (2H, s), 2.46 (3H, s), 2.64 (2H, s), 3.03 (4H, t, J = 4.6 Hz), 3.15 (2H, t, J = 8.5 Hz), 3.72 (4H, t, J = 4.6 Hz), 4.17 (1H, dt, J = 8.5, 8.5 Hz), 4.29 (1H, dt, J = 8.5, 8.5 Hz), 4.75-4.85 (1H, m), 6.73 (1H, dd, J = 9.0, 2.1 Hz), 6.88 (1H, d, J = 2.1 Hz), 7.73 (1H, d, J = 7.8 Hz), 7.95 (1H, d, J = 9.0 Hz), 11.63 (1H, s). | 533 |

**[Table 69]**

| Example | | **¹HNMR Data** | **MS Data [M+1]⁺** |
|---|---|---|---|
| 1-148 | 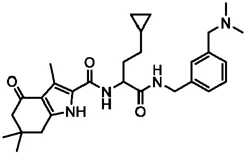 | ¹H-NMR (CDCl₃, 500 MHz) *δ* : -0.05-0.04 (2H, m), 0.38-0.45 (2H, m), 0.64-0.70 (1H, m), 1.09 (6H, s), 1.21-1.36 (2H, m), 1.80-1.88 (1H, m), 2.04-2.12 (1H, m), 2.21 (6H, s), 2.34 (2H, s), 2.63 (2H, s), 2.65 (3H, s), 3.38 (2H, s), 4.46 (2H, d, J=5.8 Hz), 4.62 (1H, td, J=7.6, 7.6, 5.8 Hz), 6.52 (1H, t, J=5.9, 5.9 Hz), 6.55 (1H, d, J=7.7 Hz), 7.16 (1H, d, J=7.7 Hz), 7.19 (1H, d, J=7.7 Hz), 7.23 (1H, d, J=1.9 Hz), 7.27 (1H, t, J=7.5, 7.5 Hz), 9.44 (1H, s) | 493.44 |
| 1-149 | 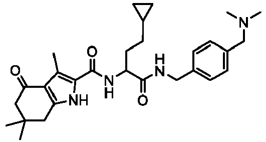 | ¹H-NMR (CDCl₃, 500 MHz) *δ* : -0.07-0.02 (2H, m), 0.36-0.43 (2H, m), 0.61-0.69 (1H, m), 1.08 (6H, s), 1.20-1.35 (2H, m), 1.79-1.87 (1H. m), 2.03-2.12 (1H. m), 2.21 (6H, s), 2.33 (2H, s), 2.62 (2H, s), 2.64 (3H, s), 3.38 (2H, s), 4.39 (1H, dd, J=14.8, 5.6 Hz), 4.49 (1H, dd, J=14.8, 6.0 Hz), 4.59 (1H, td, J=7.7, 5.8 Hz), 6.33 (1H, t, J=5.8, 5.8 Hz), 6.47 (1H, d, J=7.7 Hz), 7.20 (2H, d, J=8.1 Hz), 7.25 (2H, d, J=8.0 Hz), 9.22 (1H, s). | 493.44 |

### <Example 2-1>

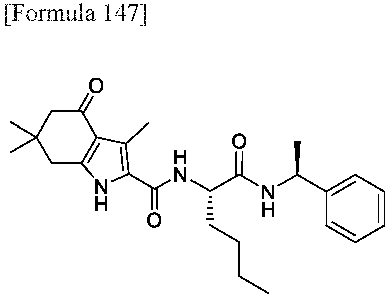

4-(4,6-dimethoxy-1,3,5-triazin-2-yl)-4-methylmorpholinium chloride (50 mg) was added to a solution of 2-(3,6,6-trimethyl-4-oxo-4,5,6,7-tetrahydro-1H-indole-2-carboxamide)hexanoic acid (50 mg) and (S)-1-phenethylamine (20 mg) in N,N-dimethylformamide (2 mL) at room temperature. The mixture was stirred for 2 hours. Ethyl acetate and water were added to the reaction mixture to separate the organic layer, The aqueous layer was re-extracted twice with ethyl acetate. The combined organic layer was washed with water and saturated brine, dried over magnesium sulfate, and concentrated under reduced pressure after filtration. The residue was purified by silica gel column chromatography to separate the two diastereomers to obtainand 3,6,6-trimethyl-4-oxo-N-((S)-1-oxo-1-(((S)-1-phenylethyl)amino)hexan-2-yl)-4,5,6,7-tetrahydro-1H-indole-2-carboxamide and its isomers.

¹H-NMR (270MHz, CDCl₃) δ: 0.90 (3H, t, J = 7.0 Hz), 1.03-1.12 (6H, m), 1.30-1.41 (4H, m), 1.50 (3H, d, J = 7.1 Hz), 1.68-1.77 (1H, m), 1.88-2.02 (1H, m), 2.32 (2H, s), 2.58 (2H, s), 2.66 (3H, s), 4.56 (1H, dt, J = 7.2, 7.1 Hz), 5.11 (1H, dq, J = 7.6, 7.1 Hz), 6.48 (1H, d, J = 7.2 Hz), 6.65 (1H, d, J = 7.6 Hz), 7.18-7.35 (5H, m), 9.55 (1H, brs).

MS (ESI+): 438.42 [M+H]⁺

### <Example 2-2>

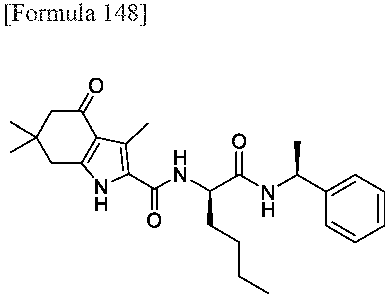

3,6,6-trimethyl-4-oxo-N-((R)-1-oxo-1-(((S)-1-phenylethyl)amino)hexan-2-yl)-4,5,6,7-tetrahydro-1H-indol-2-carboxamide.

¹H-NMR (270MHz, CDCl₃) δ: 0.81 (3H, t, J = 6.8 Hz), 1.07 (6H, brs), 1.18-1.31 (4H, m), 1.46 (3H, d, J = 6.9 Hz), 1.59-1.75 (1H, m), 1.77-1.93 (1H, m), 2.32 (2H, s), 2.58 (2H, s), 2.66 (3H, s), 4.59 (1H, dt, J = 7.9, 8.0 Hz), 5.11 (1H, dq, J = 7.7, 6.9 Hz), 6.69 (1H, d, J = 7.9 Hz), 6.83 (1H, d, J = 7.7 Hz), 7.21-7.38 (5H, m), 9.55 (1H, brs).

MS(ESI+): 438.41 [M+H]⁺

Using the corresponding starting materials and reactants, the following Examples 2-3 to 2-4 were obtained by the same method in Example 2-1 and the method described in Step 1-3 or a method similar thereto.

**[Table 70]**

| Example | Structure | Equipment Data |
|---|---|---|
| 2 - 3 | 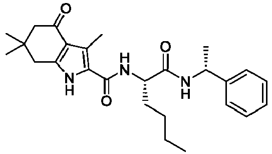 | ¹H-NMR (270 MHz, CDCl₃) δ: 0.90 (3H, t, J = 6.9 Hz), 1.07 (3H, s), 1.08 (3H, s),1.30-1.41 (4H, m), 1.51 (3H, d, J = 6.7 Hz), 1.65-1.80 (1H, m), 1.86-2.02 (1H, m), 2.32 (2H, s), 2.58 (2H, s), 2.62 (3H, s), 4.59 (1H, dt, J = 7.1, 7.1 Hz), 5.11 (1H, dq, J = 6.7, 6.9 Hz), 6.48 (1H, dd, J = 7.1, 5.9 Hz), 6.83 (1H, d, J = 6.7, 6.7 Hz), 7.21-7.38 (5H, m), 9.55 (1H, brs). |
| | | MS (ESI⁺): 438.41 [M+H]⁺ |
| 2 - 4 | 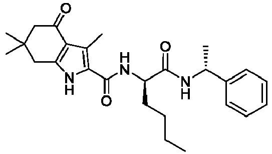 | ¹H-NMR (270 MHz, CDCl₃) δ: 0.81 (3H, t, J = 6.7 Hz), 1.07 (6H, brs), 1.17-1.35 (4H, m), 1.47 (3H, d, J = 7.3 Hz), 1.58-1.75 (1H, m), 1.75-1.95 (1H, m), 2.32 (2H, s), 2.58 (2H, s), 2.66 (3H, s), 4.59 (1H, dt, J = 7.9, 6.9 Hz), 5.11 (1H, dq, J = 7.3, 7.3 Hz), 6.69 (1H, d, J = 7.9 Hz), 6.83 (1H, d, J = 7.3 Hz), 7.21-7.38 (5H, m), 9.55 (1H, brs). |
| | | MS (ESI+): 438.4 [M+H]+ |

### <Example 3-1>

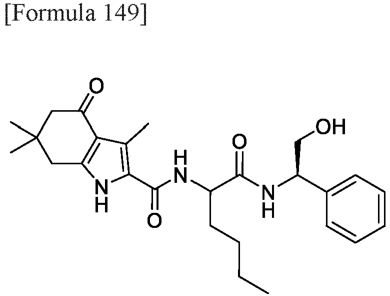

Using 2-(3,6,6-trimethyl-4-oxo-4,5,6,7-tetrahydro-1H-indole-2-carboxamide)hexanoic acid and (R)-2-amino-2-phenylethane-1-ol, N-(1-(((R)-2-hydroxy-1-phenylethyl)amino)-1-oxohexan-2-yl)-3,6,6-trimethyl-4-oxo-4,5,6,7-tetrahydro-1H-indole-2-carboxamide was obtained in the same method in Example 2-1.

¹H-NMR (270MHz, CDCl₃) δ: 0.80-0.90 (3H, m), 1.00-1.07 (6H, m), 1.21-1.42 (4H, m), 1.62-2.04 (2H, m), 2.29 (2H, s), 2.50 and 2.53 (2H, s), 2.62 and 2.64 (3H, s), 3.85-4.05 (2H, m), 4.88-5.22 (2H, m), 6.70 and 6.86 (1H, d, J = 7.3 Hz), 7.19-7.38 (5H, m,), 8.13 and 8.21 (1H, d, J = 7.2 Hz), 9.95 and 10.26 (1H, s).

MS (ESI⁺): 454.36 [M+H]⁺

Using the corresponding starting materials and reactants, the following Example 3-2 was obtained by the same method in Example 3-1 and the method described in Step 1-3 or a method similar thereto.

**[Table 71]**

| Example | Structure | Equipment Data |
|---|---|---|
| 3 - 2 | 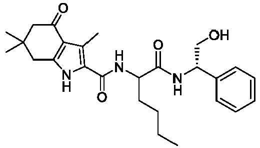 | ¹H-NMR (270 MHz, CDCl₃) δ: 0.80-0.90 (3H, m), 1.00-1.07 (6H, m), 1.21-1.45 (4H, m), 1.72-2.00 (2H, m), 2.27 (2H, s), 2.39-2.56 (2H, m), 2.61 and 2.64 (3H, s), 3.82- 4.02 (2H, m), 4.93-5.22 (2H, m), 6.79 and 6.97 (1H, d, J = 7.4 Hz), 7.13-7.41 (5H, m), 8.25 and 8.35 (1H, d, J = 7.3 Hz), 10.29 and 10.47 (1H, s). |
| | | MS (ESI+): 454.36 [M+H]+ |

### <Reference example 68-1>

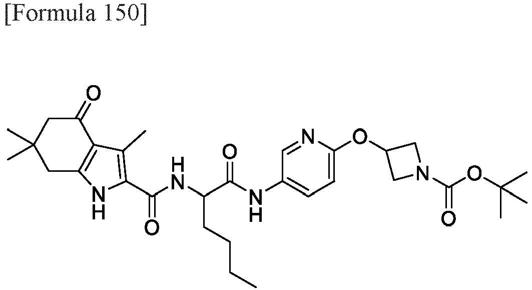

Using 2-[(3,6,6-trimethyl-4-oxo-5,7-dihydro-1H-indole-2-carbonyl)amino]hexanoic acid and tert-butyl 3-(5-aminopyridin-2-yl)oxyazetidine-1-carboxylate, the title compound was synthesized in the same manner in Reference Example 1-1.

¹H-NMR (CDCl₃, 400MHz) δ: 0.89-0.93 (3H, m), 1.095 (3H, s), 1.101 (3H, s), 1.25-1.33 (4H, m), 1.38-1.43 (2H, m), 1.44 (9H, s), 2.35 (2H, s), 2.646 (2H, s), 2.654 (3H, s), 3.90-3.98 (2H, m), 4.29 (2H, dd, J = 9.7, 6.7 Hz), 4.61-4.70 (1H, m), 5.22-5.31 (1H, m), 6.40 (1H, d, J = 7.3 Hz), 6.74 (1H, d, J = 9.1 Hz), 7.92 (1H, dd, J = 9.1, 2.4 Hz), 8.15 (1H, d, J = 2.4 Hz), 8.45 (1H, s), 9.25 (1H, s).

Using the corresponding starting materials and reactants, the following Reference Examples 68-2 to 68-17 were obtained by the same method in Reference Example 68-1, the method described in Step 1-3, or a method similar thereto.

**[Table 72]**

| Reference Example | Structure | Equipment Data |
|---|---|---|
| 6 8 - 2 | 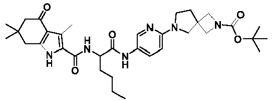 | ¹H-NMR (DMSO-D₆, 400MHz) δ: 0.89 (3H, t, J = 7.0 Hz), 1.04 (6H, s), 1.27-1.37 (4H, m), 1.40 (9H, s), 1.62-1.87 (2H, m), 2.15 (2H, t, J = 6.7 Hz), 2.24 (2H, s), 2.49 (3H, s), 2.66 (2H, s), 3.35-3.43 (2H, m), 3.53 (2H, s), 3.75-3.90 (4H, m), 4.52-4.60 (1H, m), 6.42 (1H, d, J = 9.1 Hz), 7.58 (1H, d, J = 7.9 Hz), 7.73 (1H, dd, J = 9.1, 2.4 Hz), 8.25 (1H, d, J = 2.4 Hz), 9.91 (1H, s), 11.71 (1H, s). |
| | | MS (ESI⁺): 621.4 [M+H]⁺ |
| 6 8 - 3 | 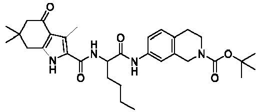 | ¹H-NMR (DMSO-D₆, 400MHz) δ: 0.86 (3H, t, J = 7.0 Hz), 1.01 (6H, s), 1.25-1.38 (4H, m), 1.41 (9H, s), 1.64-1.82 (2H, m), 2.21 (2H, s), 2.46 (3H, s), 2.63 (2H, s), 2.70 (2H, t, J = 6.1 Hz), 3.52 (2H, t, J = 6.1 Hz), 4.44 (2H, s), 4.49-4.58 (1H, m), 7.08 (1H, d, J = 7.9 Hz), 7.30-7.38 (1H, m), 7.42-7.46 (1H, m), 7.53-7.63 (1H, m), 10.07 (1H, s), 11.69 (1H, brs). |
| | | HRMS (ESI⁺): 565.33957 [M+H]⁺ |
| 6 8 - 4 | 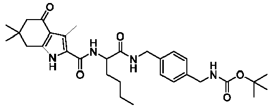 | ¹H-NMR (DMSO-D₆, 400MHz) δ: 0.84 (3H, t, J = 6.7 Hz), 1.01 (6H, s), 1.24-1.32 (4H, m), 1.37 (9H, s), 1.59-1.77 (2H, m), 2.21 (2H, s), 2.46 (3H, s), 2.62 (2H, s), 4.07 (2H, d, J = 6.1 Hz), 4.22 (1H, dd, J = 15.1, 6.1 Hz), 4.29 (1H, dd, J = 15.1, 6.1 Hz), 4.42 (1H, td, J = 8.5, 5.4 Hz), 7.14 (2H, d, J = 8.5 Hz), 7.18 (2H, d, J = 8.5 Hz), 7.34 (1H, t, J = 6.1 Hz), 7.48 (1H, d, J = 7.9 Hz), 8.53 (1H, t, J = 5.4 Hz), 11.73 (1H, s). |
| | | HRMS (ESI⁺):553.33803 [M+H]⁺ |
| 6 8 - 5 | 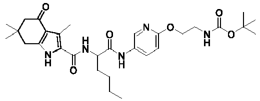 | ¹H-NMR (DMSO-D₆, 400MHz) δ: 0.87 (3H, t, J = 6.7 Hz), 1.01 (6H, s), 1.28-1.34 (4H, m), 1.36 (9H, s), 1.66-1.82 (2H, m), 2.21 (2H, s), 2.46 (3H, s), 2.63 (2H, s), 3.22-3.28 (2H, m), 4.17 (2H, t, J = 5.5 Hz), 4.53 (1H, td, J = 7.9, 5.5 Hz), 6.77 (1H, d, J = 8.6 Hz), 6.95 (1H, t, J = 5.5 Hz), 7.73 (1H, d, J = 7.3 Hz), 7.90 (1H, dd, J = 8.6, 2.4 Hz), 8.34 (1H, d, J = 2.4 Hz), 10.20 (1H, s), 11.79 (1H, s). |
| | | HRMS (ESI⁺):570.32998 [M+H]⁺ |

**[Table 73]**

| Reference Example | Structure | Equipment Data |
|---|---|---|
| 6 8 - 6 | 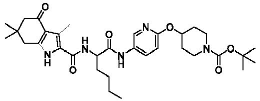 | ¹H-NMR (DMSO-D₆, 400MHz) δ: 0.89 (3H, t, J = 7.3 Hz), 1.04 (6H, s), 1.29-1.40 (4H, m), 1.42 (9H, s), 1.48-1.59 (2H, m), 1.66-1.87 (2H, m), 1.88-1.98 (2H, m), 2.24 (2H, s), 2.49 (3H, s), 2.66 (2H, s), 3.07-3.25 (2H, m), 3.64-3.75 (2H, m), 4.49-4.62 (1H, m), 5.05-5.16 (1H, m), 6.79 (1H, d, J = 9.2 Hz), 7.66 (1H, d, J = 7.9 Hz), 7.93 (1H, dd, J = 9.2, 3.1 Hz), 8.37 (1H, d, J = 3.1 Hz), 10.21 (1H, s), 11.72 (1H, s). |
| | | MS (FD⁺): 609.4 [M]⁺ |
| 6 8 - 7 | 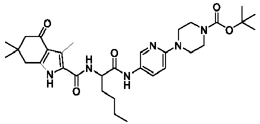 | ¹H-NMR (DMSO-D₆, 400MHz) δ: 0.89 (3H, t, J = 6.7 Hz), 1.04 (6H, s), 1.28-1.47 (13H, m), 1.66-1.87 (2H, m), 2.24 (2H, s), 2.49 (3H, s), 2.66 (2H, s), 3.42 (8H, s), 4.49-4.63 (1H, m), 6.85 (1H, d, J = 9.7 Hz), 7.61 (1H, d, J = 7.9 Hz), 7.81 (1H, dd, J = 9.7, 2.4 Hz), 8.34 (1H, d, J = 2.4 Hz), 10.04 (1H, s), 11.72 (1H, s). |
| | | MS (ESI⁺): 595.3 [M+H]⁺ |
| 6 8 - 8 | 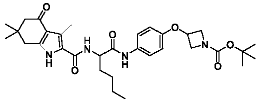 | ¹H-NMR (DMSO-D₆, 400 MHz) δ: 0.86 (3H, t, J = 7.0 Hz), 1.01 (6H, s), 1.25-1.39 (13H, m), 1.62-1.82 (2H, m), 2.21 (2H, s), 2.46 (3H, s), 2.63 (2H, s), 3.66-3.83 (2H, m), 4.16-4.36 (2H, m), 4.48-4.57 (1H, m), 4.87-4.95 (1H, m), 6.77 (2H, d, J = 9.1 Hz), 7.52 (2H, d, J = 9.1 Hz), 7.67 (1H, d, J = 8.5 Hz), 10.05 (1H, s), 11.79 (1H, s). |
| | | MS (ESI⁺): 581.3 [M+H]⁺ |
| 6 8 - 9 | 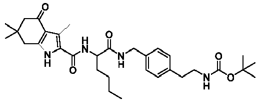 | ¹H-NMR (DMSO-D₆, 400MHz) δ: 0.84 (3H, t, J = 6.7 Hz), 1.01 (6H, s), 1.23-1.31 (4H, m), 1.35 (9H, s), 1.59-1.77 (2H, m), 2.21 (2H, s), 2.46 (3H, s), 2.63 (2H, s), 2.63-2.66 (2H, m), 3.04-3.11 (2H, m), 4.22 (1H, dd, J= 15.1, 6.1 Hz), 4.27 (1H, dd, J = 15.1, 6.1 Hz), 4.43 (1H, td, J = 7.9, 6.1 Hz), 6.84 (1H, t, J = 6.1 Hz), 7.11 (2H, d, J = 8.5 Hz), 7.15 (2H, d, J = 8.5 Hz), 7.43 (1H, d, J = 7.9 Hz), 8.51 (1H, t, J = 6.1 Hz), 11.67 (1H, s). |
| | | HRMS (ESI⁻):565.33895 [M-H]⁻ |

**[Table 74]**

| Reference Example | Structure | Equipment Data |
|---|---|---|
| 6 8 - 1 0 | 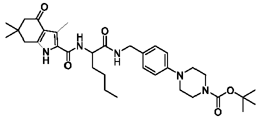 | ¹H-NMR (270 MHz, CDCl₃) δ: 0.88 (3H, t, J = 6.8 Hz), 1.10 (6H, s), 1.26-1.40 (4H, m), 1.48 (9H, s), 1.60-2.05 (2H, m), 2.34 (2H, s), 2.63 (2H, s), 2.66 (3H, s), 3.09 - 3.13 (4H, m), 3.55-3.59 (4H, m), 4.34 (1H, dd, J = 5.4, 13.5 Hz), 4.43 (1H, dd, J = 5.4, 13.5 Hz), 4.54 (1H, dt, J = 5.4, 5.4 Hz), 6.15 (1H, m), 6.50 (1H, d, J = 8.1 Hz), 6.87 (2H, d, J = 8.1 Hz), 7.18 (2H, d, J = 8.1 Hz), 9.11 (1H, m). |
| | | MS (ESI⁺): 608.8 [M+H]⁺ |
| 6 8 - 1 1 | 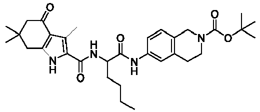 | ¹H-NMR (DMSO-D₆) δ: 0.88 (3H, t, J = 7.0 Hz), 1.04 (6H, s), 1.25-1.39 (4H, m), 1.43 (9H, s), 1.66-1.86 (2H, m), 2.24 (2H, s), 2.49 (3H, s), 2.66 (2H, s), 2.75 (2H, t, J = 6.1 Hz), 3.54 (2H, t, J = 5.8 Hz), 4.44 (2H, s), 4.52-4.61 (1H, m), 7.11 (1H, d, J = 8.5 Hz), 7.40 (1H, d, J = 8.5 Hz), 7.47 (1H, s), 7.58 (1H, d, J = 7.9 Hz), 10.07 (1H, s), 11.69 (1H, s). |
| | | HRMS (ESI⁺): 565.33920 [M+H]⁺ |
| 6 8 - 1 2 | 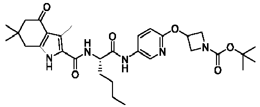 | ¹H-NMR (DMSO-D₆, 400MHz) δ: 0.90 (3H, t, J = 6.7 Hz), 1.04 (6H, s), 1.31-1.46 (13H, m), 1.68-1.87 (2H, m), 2.24 (2H, s), 2.49 (3H, s), 2.66 (2H, s), 3.70-3.88 (2H, m), 4.19-4.33 (2H, m), 4.51-4.62 (1H, m), 5.20-5.31 (1H, m), 6.89 (1H, d, J = 9.1 Hz), 7.69 (1H, d, J = 7.3 Hz), 7.97 (1H, dd, J = 8.8, 2.7 Hz), 8.37 (1H, d, J = 2.4 Hz), 10.25 (1H, s), 11.74 (1H, s). |
| 6 8 - 1 3 | 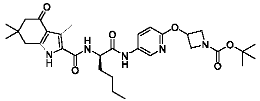 | ¹H-NMR (DMSO-D₆, 400MHz) δ: 0.90 (3H, t, J = 7.0 Hz), 1.04 (6H, s), 1.28-1.39 (4H, m), 1.40 (9H, s), 1.68-1.87 (2H, m), 2.24 (2H, s), 2.49 (3H, s), 2.66 (2H, s), 3.74-3.85 (2H, m), 4.20-4.31 (2H, m), 4.51-4.61 (1H, m), 5.22-5.30 (1H, m), 6.89 (1H, d, J = 9.1 Hz), 7.69 (1H, d, J = 7.9 Hz), 7.97 (1H, dd, J = 8.8, 2.7 Hz), 8.37 (1H, d, J = 2.4 Hz), 10.25 (1H, s), 11.73 (1H, s). |
| 6 8 - 1 4 | 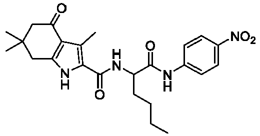 | ¹H-NMR (DMSO-D₆, 400MHz) δ: 0.90 (3H, t, J = 7.0 Hz), 1.04 (3H, s), 1.05 (3H, s), 1.28-1.52 (4H, m), 1.69-1.90 (2H, m), 2.25 (2H, s), 2.49 (3H, s), 2.67 (2H, s), 4.52-4.63 (1H, m), 7.77 (1H, d, J = 7.9 Hz), 7.90 (2H, d, J = 9.7 Hz), 8.25 (2H, d, J = 9.7 Hz), 10.79 (1H, s), 11.71 (1H, s). |
| | | HRMS (ESI⁺): 455.22873 [M+H]⁺ |

**[Table 75]**

| Reference Example | Structure | Equipment Data |
|---|---|---|
| 6 8 - 1 5 | 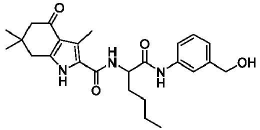 | ¹H-NMR (DMSO-D₆, 400MHz) δ: 0.88 (3H, t, J = 6.7 Hz), 1.03 (6H, s), 1.29-1.39 (4H, m), 1.68-1.82 (2H, m), 2.23 (2H, s), 2.48 (3H, s), 2.65 (2H, s), 4.46 (2H, d, J = 6.1 Hz), 4.56 (1H, td, J = 7.9, 4.9 Hz), 5.19 (1H, t, J = 6.1 Hz), 6.99 (1H, d, J = 7.9 Hz), 7.25 (1H, t, J = 7.9 Hz), 7.50 (1H, d, J = 7.9 Hz), 7.58 (1H, d, J = 7.9 Hz), 7.59 (1H, s), 10.11 (1H, s), 11.69 (1H, s). |
| | | HRMS (ESI⁺):440.25473 [M+H]⁺ |
| 6 8 - 1 6 | 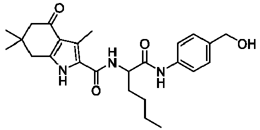 | ¹H-NMR (DMSO-D₆, 400MHz) δ: 0.87 (3H, t, J = 6.7 Hz), 1.02 (6H, s), 1.32-1.40 (4H, m), 1.72-1.78 (2H, m), 2.22 (2H, s), 2.48 (3H, s), 2.65 (2H, s), 4.43 (2H, d, J = 5.5 Hz), 4.56 (1H, dd, J = 13.8, 8.3 Hz), 5.10 (1H, t, J = 5.5 Hz), 7.24 (2H, d, J = 8.6 Hz), 7.56 (2H, d, J = 8.6 Hz), 7.63 (1H, d, J = 8.6 Hz), 10.11 (1H, s), 11.74 (1H, s). |
| 6 8 - 1 7 | 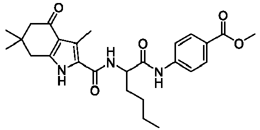 | ¹H-NMR (DMSO-D₆, 400MHz) δ: 0.88 (3H, t, J = 6.7 Hz), 1.02 (6H, s), 1.32-1.37 (4H, m), 1.70-1.84 (2H, m), 2.22 (2H, s), 2.47 (3H, s), 2.65 (2H, s), 3.82 (3H, s), 4.55 (1H, td, J = 8.6, 4.9 Hz), 7.73 (1H, d, J = 8.6 Hz), 7.76 (2H, d, J = 8.6 Hz), 7.93 (2H, d, J = 8.6 Hz), 10.51 (1H, s), 11.73 (1H, s). |
| | | HRMS (ESI⁺):468.24902 [M+H]⁺ |

### <Example 4>

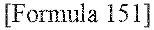

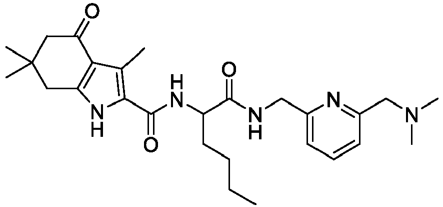

Under argon atmosphere, trifluoroacetic acid (1.16 mL) was added to a solution of tert-butyl N-[[6-[(dimethylamino)methyl]pyridin-2-yl]methyl]-N-[(2-methylpropan-2-yl)oxycarbonyl]carbamate (170 mg) in dichloromethane (1.16 mL) under ice cooling condition. After stirring at room temperature for 30 minutes, the reaction mixture was concentrated. 2-[(3,6,6-trimethyl-4-oxo-5,7-dihydro-1H-indole-2-carbonyl)amino]hexanoic acid (156 mg), 1-hydroxybenzotriazole monohydrate (75.4 mg), and then N,N-diisopropylethylamine (0.316 mL), 1-ethyl-3-(3-dimethylaminopropyl)carbodiimide hydrochloride (98.2 mg) were added to the obtained crude product in N,N-dimethylformamide (2.33 mL) under ice cooling condition, and the mixture was stirred at room temperature for 4 hours. After water was added, the mixture was extracted with ethyl acetate. The organic layer was washed with saturated brine, dried over anhydrous sodium sulfate, and concentrated under reduced pressure after filtration of the insoluble material. The residue was purified by amino-silica gel column chromatography (methanol / ethyl acetate = 10%), and the resulting solid was washed with diisopropyl ether to obtain N-[1-[[6-[(dimethylamino)methyl]pyridine-2yl]methylamino]-1-oxohexan-2-yl]-3,6,6-trimethyl-4-oxo-5,7-dihydro-1H-indole-2-carboxamide (155 mg).

¹H-NMR (DMSO-d₆, 400MHz) δ: 0.86 (3H, t, J = 6.7 Hz), 1.01 (6H, s), 1.24-1.37 (4H, m), 1.60-1.71 (1H, m), 1.72-1.82 (1H, m), 2.15 (6H, s), 2.21 (2H, s), 2.47 (3H, s), 2.63 (2H, s), 3.45 (2H, s), 4.30 (1H, dd, J = 16.3, 6.1 Hz), 4.38 (1H, dd, J = 16.3, 6.1 Hz), 4.45 (1H, td, J = 8.5, 5.4 Hz), 7.14 (1H, d, J = 7.9 Hz), 7.26 (1H, d, J = 7.9 Hz), 7.47 (1H, d, J = 7.9 Hz), 7.70 (1H, t, J = 7.9 Hz), 8.61 (1H, t, J = 5.4 Hz), 11.64 (1H, s).

HRMS (ESI⁺): 482.31381 [M+H]⁺

### <Reference example 69>

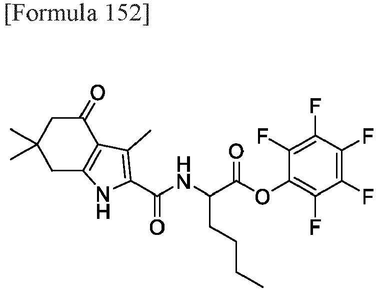

Under argon atmosphere, pentafluorophenol (289 mg) was added to a solution of 2-[(3,6,6-trimethyl-4-oxo-5,7-dihydro-1H-indole-2-carbonyl)amino]hexanoic acid (500 mg) and N,N'-dicyclohexylcarbodiimide (339 mg) in ethyl acetate (7.50 mL) at 50 °C, and the mixture was stirred for 2 hours. After filtering the reaction mixture with a cotton swab, the filtrate was concentrated under reduced pressure. Ethyl acetate was added to the residue, and the mixture was washed twice with saturated aqueous sodium hydrogen carbonate and saturated brine, dried over anhydrous sodium sulfate, and concentrated under reduced pressure after filtration of the insoluble material. The residue was suspended and the precipitate was washed with diisopropyl ether to obtain (2,3,4,5,6-pentafluorophenyl) 2-[(3,6,6-trimethyl-4-oxo-5,7-dihydro-1H-indole-2-carbonyl)amino]hexanoate (414 mg).

¹H-NMR (DMSO-d₆, 400MHz) δ: 0.86 (3H, t, J = 7.0Hz), 1.00 (6H, s), 1.24-1.43 (4H, m), 1.65-1.76 (1H, m), 1.80-1.89 (1H, m), 2.24 (2H, s), 2.46 (3H, s), 2.65 (2H, s), 4.51-4.57 (1H, m), 11.44 (1H, brs), 12.07 (1H, s).

### <Example 5-1>

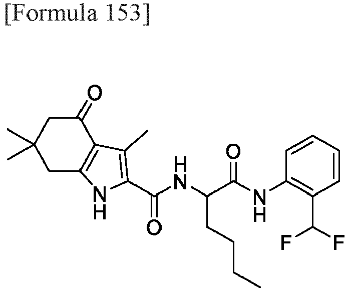

Under argon atmosphere, The mixture of (2,3,4,5,6-pentafluorophenyl) 2-[(3,6,6-trimethyl-4-oxo-5,7-dihydro-1H-indole-2-carbonyl)amino]hexanoate (70.0 mg), 2-(difluoromethyl) aniline (27.6 mg) and N, N-diisopropylethylamine (48.7 µL) in N,N-dimethylacetamide (0.70 mL) was stirred at 50 °C for 2 hours. The reaction mixture was concentrated under reduced pressure, and the residue was purified by silica gel column chromatography (hexane ethyl acetate = 6 1 to 0 1) to obtain N-[1-[2-(difluoromethyl)anilino]-1-oxohexan-2-yl]-3,6,6-trimethyl-4-oxo-5,7-dihydro-1H-indole-2-carboxamide (14.0 mg).

¹H-NMR (DMSO-d₆, 400MHz) δ: 0.89 (3H, t, J = 7.0 Hz), 1.01 (6H, s), 1.25-1.45 (4H, m), 1.67-1.89 (2H, m), 2.21 (2H, s), 2.47 (3H, s), 2.64 (2H, s), 4.47-4.60 (1H, m), 7.02 (1H, t, J = 55.1Hz), 7.31-7.43 (2H, m), 7.54 (1H, t, J = 7.6 Hz), 7.61 (2H, t, J = 7.3 Hz), 9.96 (1H, s), 11.66 (1H, s).

HRMS (ESI⁺): 460.24139 [M+H]⁺

Using the corresponding starting materials and reactants, the following Examples 5-2 to 5-3 were obtained by the same method in Example 5-1 and the method described in Step 2-2 or a method similar thereto.

**[Table 76]**

| Example | Structure | Equipment Data |
|---|---|---|
| 5-2 | 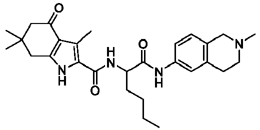 | ¹H-NMR (DMSO-D₆, 400MHz) δ: 0.93 (3H, t, J = 7.0 Hz), 1.08 (6H, s), 1.30-1.50 (4H, m), 1.68-1.92 (2H, m), 2.29 (2H, s), 2.37 (3H, s), 2.54 (3H, s), 2.61 (2H, t, J = 5.8 Hz), 2.70 (2H, s), 2.83 (2H, t, J = 5.8 Hz), 3.47 (2H, s), 4.56-4.68 (1H, m), 7.03 (1H, d, J = 8.5 Hz), 7.38 (1H, d, J = 8.5 Hz), 7.45 (1H, s), 7.61 (1H, d, J = 7.9 Hz), 10.06 (1H, s), 11.75 (1H, s). |
| | | HRMS (ESI⁺): 479.3009 [M+H]⁺ |

**[Table 77]**

| Example | Structure | Equipment Data |
|---|---|---|
| 5-3 | 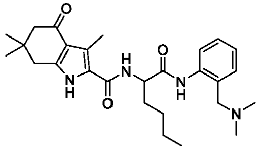 | ¹H-NMR (DMSO-D₆, 400MHz) δ: 0.89 (3H, t, J = 7.3 Hz), 1.00 (3H, s), 1.02 (3H, s), 1.30-1.41 (4H, m), 1.72-1.83 (2H, m), 1.89 (6H, s), 2.22-2.23 (2H, m), 2.50 (3H, s), 2.60-2.68 (2H, m), 3.20-3.30 (1H, m), 3.42-3.52 (1H, m), 4.37-4.39 (1H, m), 6.98-7.02 (1H, m), 7.15 (1H, d, J = 6.7 Hz), 7.23-7.27 (1H, m), 7.71 (1H, d, J = 7.3 Hz), 8.12 (1H, d, J = 7.9 Hz), 10.74 (1H, s), 11.69 (1H, brs). |
| | | HRMS (ESI⁺): 467.30212 [M+H]⁺ |

### <Reference example 70-1>

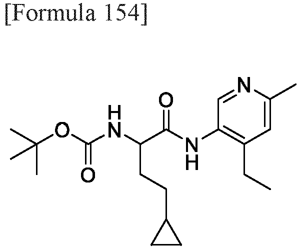

Under argon atmosphere, diisopropylamine (0.299 mL) and 1-[bis(dimethylamino)methylene]-1H-1,2,3-triazolo[4,5-b]pyridinium 3-oxide hexafluorophosphate (368 mg) were added to a solution of 4-cyclopropyl-2-[(2-methylpropan-2-yl)oxycarbonylamino]butanoic acid (214 mg) and 4-ethyl-6-methylpyridin-3-amine (120 mg) in N,N-dimethylformamide (4.50 mL) at room temperature, and the mixture was stirred for 120 hours. The reaction mixture was added to water, and extracted twice with ethyl acetate. The combined organic layer was washed with water and saturated brine, dried over anhydrous sodium sulfate, and concentrated under reduced pressure after filtration of the insoluble material. The residue was purified by amino-silica gel column chromatography (hexane : ethyl acetate = 3 : 1 to 0 : 1) to obtain tert-butyl N-[4-cyclopropyl-1-[(4-ethyl-6-methylpyridin-3-yl)amino]-1-oxobutan-2-yl]carbamate (288 mg).

¹H-NMR (DMSO-d₆, 400MHz) δ: -0.06-0.07 (2H, m), 0.32-0.43 (2H, m), 0.56-0.74 (1H, m), 1.09 (3H, t, J = 7.6 Hz), 1.20-1.29 (2H, m), 1.38 (9H, s), 1.60-1.86 (2H, m), 2.40 (3H, s), 2.47-2.54 (2H, m), 4.04-4.13 (1H, m), 7.03 (1H, d, J = 7.9 Hz), 7.12 (1H, s), 8.20 (1H, s), 9.38 (1H, s).

MS (ESI⁺): 362.2 [M+H]⁺

Using the corresponding starting materials and reactants, the following Reference Examples 70-2 to 70-58 were obtained by the same method in Reference Example 70-1, the method described in Step 3-1 or a method similar thereto.

**[Table 78]**

| Reference Example | Structure | Equipment Data |
|---|---|---|
| 7 0 - 2 | 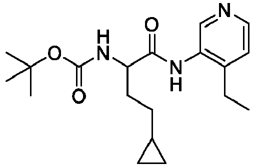 | ¹H-NMR (DMSO-D₆, 400MHz) δ: -0.02-0.07 (2H, m), 0.30-0.45 (2H, m), 0.64-0.74 (1H, m), 1.17 (3H, t, J = 7.0 Hz), 1.22-1.32 (2H, m), 1.39 (9H, s), 1.64-1.85 (2H, m), 2.57 (2H, q, J = 7.0 Hz), 4.07-4.15 (1H, m), 7.08 (1H, d, J = 7.3 Hz), 7.28 (1H, d, J = 4.9 Hz), 8.31 (1H, d, J = 4.9 Hz), 8.39 (1H, s), 9.49 (1H, s). |
| | | HRMS (ESI⁺): 348.22810 [M+H]⁺ |
| 70- 3 | 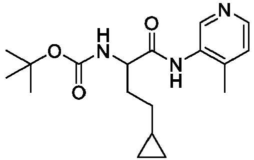 | ¹H-NMR (DMSO-D₆, 400MHz) δ: -0.06-0.06 (2H, m), 0.32-0.41 (2H, m), 0.60-0.73 (1H, m), 1.17-1.29 (2H, m), 1.36 (9H, s), 1.60-1.84 (2H, m), 2.16 (3H, s), 4.03-4.13 (1H, m), 7.05 (1H, d, J = 7.9 Hz), 7.23 (1H, d, J = 4.8 Hz), 8.22 (1H, d, J = 4.8 Hz), 8.38 (1H, s), 9.51 (1H, s). |
| | | MS (ESI⁺): 334.2 [M+H]⁺ |
| 7 0 - 4 | 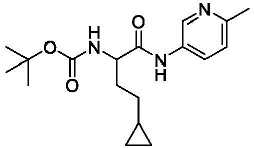 | ¹H-NMR (DMSO-D₆, 400MHz) δ: -0.04-0.04 (2H, m), 0.30-0.41 (2H, m), 0.59-0.71 (1H, m), 1.16-1.26 (2H, m), 1.36 (9H, s), 1.58-1.80 (2H, m), 2.39 (3H, s), 4.00-4.10 (1H, m), 7.05 (1H, d, J = 7.9 Hz), 7.18 (1H, d, J = 8.5 Hz), 7.90 (1H, dd, J = 8.5, 3.0 Hz), 8.59 (1H, d, J = 3.0 Hz), 10.05 (1H, s). |
| 7 0 - 5 | 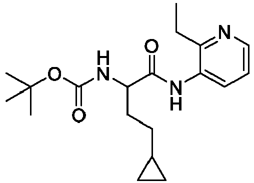 | ¹H-NMR (DMSO-D₆, 400MHz) δ: -0.05-0.07 (2H, m), 0.33 (2H, t, J = 6.4 Hz), 0.61-0.74 (1H, m), 1.15 (3H, t, J = 7.3 Hz), 1.19-1.31 (2H, m), 1.39 (9H, s), 1.62-1.84 (2H, m), 2.72 (2H, q, J = 7.5 Hz), 4.07-4.15 (1H, m), 7.08 (1H, d, J = 7.9 Hz), 7.21 (1H, dd, J = 7.9, 4.9 Hz), 7.69 (1H, dd, J = 7.9, 1.2 Hz), 8.31 (1H, dd, J = 4.9, 1.2 Hz), 9.42 (1H, s). |
| | | HRMS (ESI⁺): 348.22907 [M+H]⁺ |
| 7 0 - 6 | 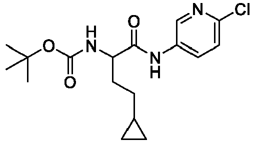 | ¹H-NMR (DMSO-D₆, 400MHz) δ: -0.05-0.04 (2H, m), 0.29-0.40 (2H, m), 0.59-0.68 (1H, m), 1.13-1.41 (11H, m), 1.61-1.78 (2H, m), 3.99-4.08 (1H, m), 7.12 (1H, d, J = 7.3 Hz), 7.45 (1H, d, J = 9.2 Hz), 8.08 (1H, dd, J = 9.2, 2.8 Hz), 8.59 (1H, d, J = 2.8 Hz), 10.32 (1H, s). |
| 7 0 - 7 | 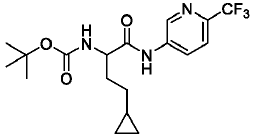 | ¹H-NMR (CDCl₃, 400MHz) δ: 0.01-0.07 (2H, m), 0.42-0.50 (2H, m), 0.65-0.72 (1H, m), 1.29-1.40 (2H, m), 1.47 (9H, s), 1.73-1.81 (1H, m), 2.05-2.13 (1H, m), 4.22-4.28 (1H, m), 4.89-4.95 (1H, m), 7.63 (1H, d, J = 8.6 Hz), 8.36 (1H, dd, J = 8.6, 2.4 Hz), 8.61 (1H, d, J = 2.4 Hz), 9.00 (1H, brs). |

**[Table 79]**

| Reference Example | Structure | Equipment Data |
|---|---|---|
| 7 0 - 8 | 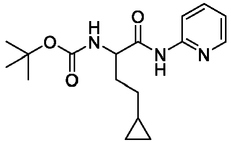 | ¹H-NMR (DMSO-D₆, 400MHz) δ: -0.05-0.04 (2H, m), 0.32-0.39 (2H, m), 0.62-0.68 (1H, m), 1.14-1.40 (11H, m), 1.59-1.77 (2H, m), 4.10-4.20 (1H, m), 7.05-7.11 (2H, m), 7.76 (1H, td, J = 8.0, 1.8 Hz), 8.04 (1H, d, J = 8.0 Hz), 8.30 (1H, dd, J = 4.9, 1.8 Hz), 10.32 (1H, s). |
| | | HRMS (ESI⁺): 320.1964 [M+H]⁺ |
| 7 0 - 9 | 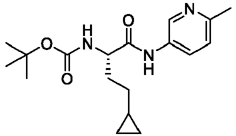 | ¹H-NMR (CDCl₃, 400 MHz) δ:0.02-0.06 (2H, m), 0.42-0.48 (2H, m), 0.63-0.73 (1H, m), 1.29-1.37 (2H, m), 1.46 (9H, s), 1.72-1.82 (1H, m), 2.02-2.11 (1H, m), 2.51 (3H, s), 4.20-4.28 (1H, m), 4.90-5.05 (1H, m), 7.10 (1H, d, J = 8.6 Hz), 7.98 (1H, dd, J = 8.6, 2.4 Hz), 8.35-8.47 (2H, m). |
| | | HRMS (ESI⁺):334.21299 [M+H]⁺ |
| 7 0 - 1 0 | 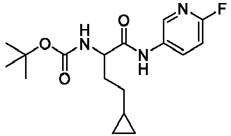 | ¹H-NMR (CDCl₃, 400 MHz) δ:-0.01-0.03 (2H, m), 0.40-0.46 (2H, m), 0.61-0.69 (1H, m), 1.27-1.33 (2H, m), 1.43 (9H, s), 1.68-1.79 (1H, m), 1.99-2.09 (1H, m), 4.15-4.26 (1H, m), 4.85-4.95 (1H, m), 6.88 (1H, dd, J = 8.5, 2.4 Hz), 8.13-8.18 (1H, m), 8.19 (1H, s), 8.60 (1H, brs). |
| | | HRMS (ESI⁺):338.18810 [M+H]⁺ |
| 7 0 - 1 1 | 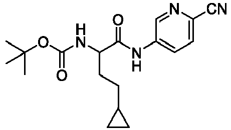 | ¹H-NMR (CDCl₃, 400 MHz) δ:0.03-0.07 (2H, m), 0.45-0.49 (2H, m), 0.65-0.72 (1H, m), 1.32-1.37 (2H, m), 1.57 (9H, s), 1.74-1.82 (1H, m), 2.06-2.12 (1H, m), 4.21-4.28 (1H, m), 4.88-4.93 (1H, m), 7.66 (1H, d, J = 8.6 Hz), 8.34 (1H, dd, J = 8.6, 2.4 Hz), 8.61 (1H, d, J = 2.4 Hz), 9.15 (1H, brs). |
| | | HRMS (ESI⁺):345.19234 [M+H]⁺ |
| 7 0 - 1 2 | 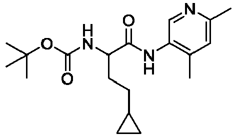 | ¹H-NMR (CDCl₃, 400 MHz) δ:-0.03-0.06 (2H, m), 0.39-0.46 (2H, m), 0.61-0.69 (1H, m), 1.22-1.32 (2H, m), 1.43 (9H, s), 1.74-1.83 (1H, m), 2.04-2.14 (1H, m), 2.45 (3H, s), 2.83 (3H, s), 4.20-4.26 (1H, m), 5.06-5.10 (1H, m), 7.32 (1H, s), 9.31 (1H, brs), 9.36 (1H, brs). |
| | | HRMS (ESI⁺):348.22808 [M+H]⁺ |

**[Table 80]**

| Reference Example | Structure | Equipment Data |
|---|---|---|
| 7 0 - 1 3 | 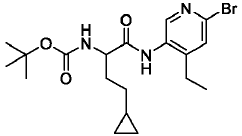 | ¹H-NMR (CDCl₃, 400 MHz) δ: -0.04-0.04 (2H, m), 0.39-0.45 (2H, m), 0.61-0.68 (1H, m), 1.20 (3H, t, J = 7.3 Hz), 1.26-1.33 (2H, m), 1.42 (9H, s), 1.68-1.78 (1H , m), 2.02-2.10 (1H, m), 2.49-2.57 (2H, m), 4.16-4.22 (1H, m), 4.88 (1H, d, J = 6.7 Hz), 7.28 (1H, s), 8.30 (1H, s), 8.79 (1H, s). |
| | | HRMS (ESI⁺):426.13984 [M+H]⁺ |
| 7 0 - 1 4 | 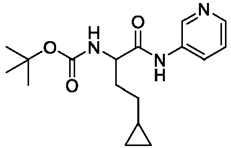 | ¹H-NMR (CDCls, 400 MHz) δ:0.03-0.07 (2H, m), 0.44-0.49 (2H, m), 0.66-0.71 (1H, m), 1.31-1.37 (2H, m), 1.56 (9H, s), 1.74-1.82 (1H, m), 2.04-2.12 (1H, m), 4.21-4.27 (1H, m), 4.92 (1H, brs), 7.21-7.25 (1H, m), 7.27-7.30 (1H, m), 8.11-8.15 (1H, m), 8.35 (1H, dd, J = 4.9, 1.2 Hz), 8.58 (1H, d, J = 2.4 Hz). |
| | | HRMS (ESI⁺):320.19777 [M+H]⁺ |
| 7 0 - 1 5 | 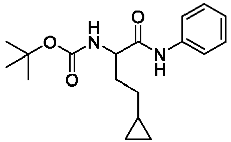 | ¹H-NMR (CDCl₃, 400 MHz) δ:0.01-0.06 (2H, m), 0.41-0.47 (2H, m), 0.63-0.72 (1H, m), 1.29-1.36 (2H, m), 1.46 (9H, s), 1.71-1.82 (1H, m), 2.01-2.11 (1H, m), 4.18-4.27 (1H, m), 5.01 (1H, brs), 7.10 (1H, tt, J = 7.3, 1.2 Hz), 7.31 (2H, dd, J = 8.5, 7.3 Hz), 7.51 (2H, dd, J = 8.5, 1.2 Hz), 8.23 (1H, brs). |
| | | HRMS (ESI⁺):319.20263 [M+H]⁺ |
| 70-16 | 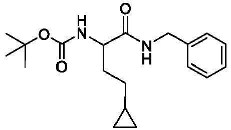 | ¹H-NMR (270 MHz, CDCl₃) δ: -0.06-0.11 (2H, m), 0.34-0.51 (2H, m), 0.57-0.75 (1H, m), 1.14-1.35 (2H, m), 1.42 (9H, s), 1.61-1.81 (1H, m), 1.89-2.11 (1H, m), 4.11 (1H, dd, J = 14.2, 7.9 Hz), 4.45 (2H, d, J = 5.6 Hz), 4.84-5.04 (1H, m), 6.28-6.51 (1H, m), 7.20-7.41 (5H, m). |
| | | MS (ESI⁺): 333.32 [M+H]⁺ |
| 70-17 | 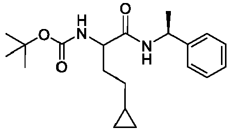 | ¹H-NMR (270 MHz, CDCl₃) δ: -0.09-0.09 (2H, m), 0.33-0.50 (2H, m), 0.54-0.74 (1H, m), 1.15-1.32 (2H, m), 1.42 (9H, s), 1.48 (3H, d, J = 6.9 Hz), 1.58-1.77 (1H, m), 1.83-2.05 (1H, m), 3.96-4.18 (1H, m), 4.79-5.21 (2H, m), 6.26-6.59 (1H, m), 7.20-7.39 (5H, m). |
| | | MS (ESI⁺): 346.23 [M+H]⁺ |
| 70-18 | 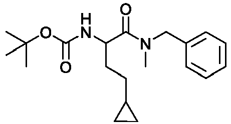 | ¹H-NMR (270 MHz, CDCl₃) δ: -0.13-0.12 (2H, m), 0.24-0.48 (2H, m), 0.55-0.79 (1H, m), 1.10-1.90 (4H, m), 1.44 (9H, s), 3.00 (3H, s), 4.42-4.78(3H, m), 5.26-5.50 (1H, m), 7.15-7.41 (5H, m). |
| | | MS (ESI⁺): 347.32 [M+H]⁺ |

**[Table 81]**

| Reference Example | Structure | Equipment Data |
|---|---|---|
| 70-19 | 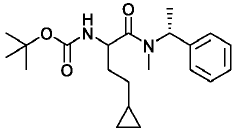 | ¹H-NMR (270 MHz, CDCl₃) δ: -0.14-0.19 (2H, m), 0.22-0.52 (2H, m), 0.55-0.86 (1H, m), 1.04-1.95 (16H, m), 2.73 (3H, d, J = 9.6 Hz), 4.52-4.72 (1H, m), 5.36-5.57 (1H, m), 5.91-6.12 (1H, m), 7.19-7.43 (5H, m). |
| | | MS (ESI⁺): 361.34 [M+H]⁺ |
| 70-20 | 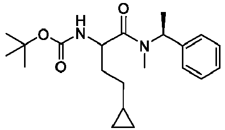 | ¹H-NMR (270 MHz, CDCl₃) δ: -0.13-0.15 (2H, m), 0.28-0.53 (2H, m), 0.53-0.80 (1H, m), 1.08-1.95 (16H, m), 2.73 (3H, d, J = 9.9 Hz), 4.57-4.71 (1H, m), 5.45 (1H, d, J = 8.6 Hz), 5.90-6.11 (1H, m), 7.19-7.43 (5H, m). |
| | | MS (ESI⁺): 361.37 [M+H]⁺ |
| 7 0 - 2 1 | 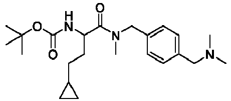 | ¹H-NMR (270 MHz, CDCl₃) δ: -0.13-0.12 (2H, m), 0.25-0.50 (2H, m), 0.50-0.78 (1H, m), 1.06-1.48 (11H, m), 1.54-1.92 (2H, m), 2.24 (6H, s), 2.90-3.04 (3H, m), 3.35-3.46 (2H, s), 4.35-4.80 (3H, m), 5.24-5.47 (1H, m), 7.12-7.20 (2H, m), 7.24-7.30 (2H, m). |
| | | MS (ESI⁺): 404.55 [M+H]⁺ |
| 7 0 - 2 2 | 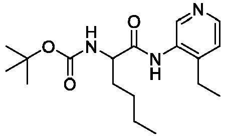 | ¹H-NMR (CDCl₃, 400MHz) δ: 0.93 (3H, t, J = 7.0 Hz), 1.24 (3H, t, J = 7.6 Hz), 1.33-1.45 (4H, m), 1.47 (9H, s), 1.61-1.76 (2H, m), 2.61 (2H, q, J = 7.7 Hz), 4.15-4.24 (1H, m), 4.94-5.04 (1H, m), 7.15 (1H, d, J = 5.5 Hz), 8.15 (1H, brs), 8.35 (1H, d, J = 4.9 Hz), 8.94 (1H, s). |
| 7 0 - 2 3 | 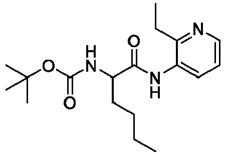 | ¹H-NMR (CDCl₃, 400MHz) δ: 0.93 (3H, t, J = 7.6 Hz), 1.31 (3H, t, J = 7.6 Hz), 1.36-1.46 (4H, m), 1.48 (9H, s), 1.58-1.74 (2H, m), 2.81 (2H, q, J = 7.6 Hz), 4.89-4.96 (1H, m), 7.15 (1H, dd, J = 7.9, 4.9 Hz), 8.27-8.33 (3H, m). |
| 7 0 - 2 4 | 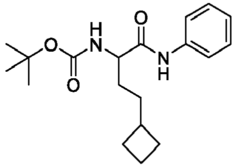 | ¹H-NMR (CDCl₃, 400 MHz) δ: 1.49 (9H, s), 1.52-1.64 (5H, m), 1.75-1.88 (3H, m), 2.00-2.08 (2H, m), 2.23-2.30 (1H, m), 4.10-4.17 (1H, m), 4.99 (1H, brs), 7.10 (1H, tt, J = 7.3, 1.2 Hz), 7.31 (2H, dd, J = 8.5, 7.3 Hz), 7.51 (2H, dd, J = 8.5, 1.2 Hz), 8.18 (1H, brs). |
| | | HRMS (ESI⁺):333.21797 [M+H]⁺ |

**[Table 82]**

| Reference Example | Structure | Equipment Data |
|---|---|---|
| 7 0 - 2 5 | 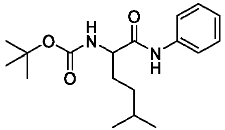 | ¹H-NMR (CDCls, 400 MHz) δ: 0.90 (3H, d, J = 6.7 Hz), 0.91 (3H, d, J = 6.7 Hz), 1.25-1.32 (2H, m), 1.46 (9H, s), 1.56-1.70 (2H, m), 1.90-2.00 (1H, m), 4.08-4.16 (1H, m), 4.96 (1H, brs), 7.11 (1H, tt, J = 7.3, 1.2 Hz), 7.32 (2H, dd, J = 8.5, 7.3 Hz), 7.52 (2H, dd, J = 8.5, 1.2 Hz), 8.12 (1H, brs). |
| | | HRMS (FI⁺):320.21010 [M]⁺ |
| 7 0 - 2 6 | 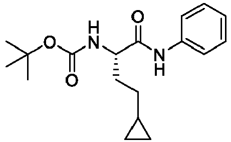 | ¹H-NMR (CDCls, 400 MHz) δ: 0.02-0.06 (2H, m), 0.42-0.47 (2H, m), 0.63-0.74 (1H, m), 1.29-1.36 (2H, m), 1.46 (9H, s), 1.72-1.82 (1H, m), 2.01-2.11 (1H, m), 4.24 (1H, d, J = 6.1 Hz), 5.01 (1H, brs), 7.10 (1H, tt, J= 7.3, 1.2 Hz), 7.30 (2H, dd, J = 8.5, 7.3 Hz), 7.51 (2H, dd, J = 8.5, 1.2 Hz), 8.25 (1H, brs). |
| | | HRMS (ESI⁺):319.20237 [M+H]⁺ |
| 7 0 - 2 7 | 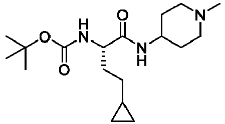 | ¹H-NMR (CDCl₃, 400 MHz) δ: 0.00-0.04 (2H, m), 0.38-0.47 (2H, m), 0.60-0.71 (1H, m), 1.21-1.30 (2H, m), 1.44 (9H, s), 1.47-1.53 (1H, m), 1.59-1.72 (1H, m), 1.82-1.99 (4H, m), 2.04-2.14 (2H, m), 2.27 (3H, s), 2.69-2.79 (2H, m), 3.70-3.81 (1H, m), 3.97-4.07 (1H, m), 4.96 (1H, brs), 6.01 (1H, brs). |
| | | HRMS (ESI⁺):340.26018 [M+H]⁺ |
| 7 0 - 2 8 | 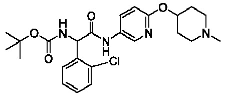 | ¹H-NMR (DMSO-D₆, 400MHz) δ: 1.38 (9H, s), 1.55-1.67 (2H, m), 1.86-1.97 (2H, m), 2.07-2.17 (5H, m), 2.54-2.65 (2H, m), 4.84-4.94 (1H, m), 5.59 (1H, d, J = 7.9 Hz), 6.74 (1H, d, J = 9.1 Hz), 7.29-7.36 (2H, m), 7.37-7.49 (2H, m), 7.73 (1H, d, J = 8.5 Hz), 7.88 (1H, dd, J = 9.1, 3.0 Hz), 8.32 (1H, d, J = 3.0 Hz), 10.24 (1H, s). |
| | | MS (ESI⁺): 475.2 [M+H]⁺ |
| 7 0 - 2 9 | 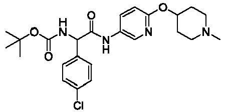 | ¹H-NMR (DMSO-D₆, 400MHz) δ: 1.37 (9H, s), 1.55-1.67 (2H, m), 1.86-1.96 (2H, m), 2.06-2.17 (5H, m), 2.55-2.64 (2H, m), 4.83-4.93 (1H, m), 5.31 (1H, d, J = 7.9 Hz), 6.73 (1H, d, J = 9.1 Hz), 7.41 (2H, d, J = 8.5 Hz), 7.48 (2H, d, J = 8.5 Hz), 7.60 (1H, d, J = 8.5 Hz), 7.83 (1H, dd, J = 9.1, 3.0 Hz), 8.27 (1H, d, J = 3.0 Hz), 10.28 (1H, s). |
| | | MS (ESI⁺): 475.2 [M+H]⁺ |

**[Table 83]**

| Reference Example | Structure | Equipment Data |
|---|---|---|
| 7 0 - 3 0 | 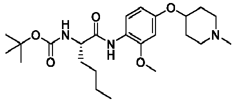 | ¹H-NMR (DMSO-D₆, 400MHz) δ: 0.85 (3H, t, J = 7.0 Hz), 1.23-1.43 (13H, m), 1.48-1.76 (4H, m), 1.79-1.95 (2H, m), 2.07-2.20 (5H, m), 2.54-2.63 (2H, m), 3.78 (3H, s), 3.95-4.06 (1H, m), 4.27-4.36 (1H, m), 6.49 (1H, dd, J = 8.8, 2.4 Hz), 6.60 (1H, d, J = 2.4 Hz), 7.16 (1H, d, J = 7.9 Hz), 7.79 (1H, d, J = 8.5 Hz), 8.80 (1H, s). |
| | | MS (ESI⁺): 450.3 [M+H]⁺ |
| 7 0 - 3 1 | 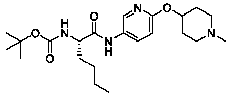 | ¹H-NMR (DMSO-D₆) δ: 0.84 (3H, t, J = 6.7 Hz), 1.20-1.42 (13H, m), 1.50-1.69 (4H, m), 1.87-1.96 (2H, m), 2.06-2.18 (5H, m), 2.56-2.66 (2H, m), 3.96-4.06 (1H, m), 4.84-4.94 (1H, m), 6.73 (1H, d, J = 8.6 Hz), 7.00 (1H, d, J = 7.9 Hz), 7.87 (1H, dd, J = 8.6, 2.4 Hz), 8.29 (1H, d, J = 2.4 Hz), 9.95 (1H, s). |
| | | MS (ESI⁺): 421.3 [M+H]⁺ |
| 7 0 - 3 2 | 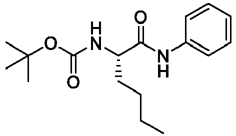 | ¹H-NMR (CDCls, 400 MHz) δ: 0.91 (3H, t, J = 7.3 Hz), 1.33-1.42 (4H, m), 1.46 (9H, s), 1.61-1.71 (1H, m), 1.89-1.99 (1H, m), 4.11-4.19 (1H, m), 4.98 (1H, brs), 7.10 (1H, t, J = 7.9 Hz), 7.31 (2H, t, J = 7.9 Hz), 7.51 (2H, d, J = 7.9 Hz), 8.15 (1H, brs). |
| | | HRMS (FI⁺):306.19426 [M]⁺ |
| 7 0 - 3 3 | 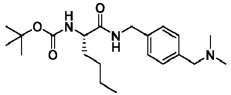 | ¹H-NMR (CDCls, 400 MHz) δ: 0.89 (3H, t, J = 6.7 Hz), 1.28-1.37 (4H, m), 1.42 (9H, s), 1.54-1.65 (1H, m), 1.81-1.90 (1H, m), 2.22 (6H, s), 3.40 (2H, s), 4.01-4.10 (1H, m), 4.43 (2H, d, J = 5.4 Hz), 5.03 (1H, brs), 6.48 (1H, t, J = 5.4 Hz), 7.21 (2H, d, J = 8.5 Hz), 7.25 (2H, d, J = 8.5 Hz). |
| | | HRMS (ESI⁺):378.27539 [M+H]⁺ |
| 7 0 - 3 4 | 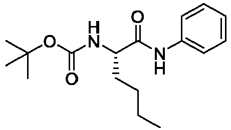 | ¹H-NMR (500 MHz, CDCl₃) δ: 0.90 (3H, t, J = 7.0 Hz), 1.30-1.43 (4H, m), 1.45 (9H, s), 1.63-1.74 (1H, m), 1.87-1.97 (1H, m), 4.25 (1H, brs), 5.22 (1H, d, J = 7.5 Hz), 7.07 (1H, dd, J = 7.0, 7.0 Hz), 7.26 (2H, dd, J = 8.0, 7.0 Hz), 7.50 (2H, d, J = 8.0 Hz), 8.53 (1H, brs). |
| | | MS (ESI⁺): 307.19 [M+H]⁺ |
| 7 0 - 3 5 | 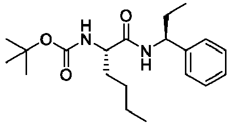 | ¹H-NMR (270 MHz, CDCl₃) δ: 0.85 (3H, t, J = 6.1 Hz), 0.92 (3H, t, J = 7.2 Hz), 1.15-1.35 (4H, m), 1.43 (9H, s), 1.50-1.70 (2H, m), 1.81 (2H, dt, J = 7.0, 7.2 Hz), 3.99 (1H, m), 4.87 (1H, dt, J = 8.1, 7.0 Hz), 4.97 (1H, m), 6.38 (1H, d, J = 8.1 Hz), 7.21 - 7.35 (5H, m). |
| | | MS (ESI⁺): 349.36 [M+H]⁺ |

**[Table 84]**

| Reference Example | Structure | Equipment Data |
|---|---|---|
| 7 0 - 3 6 | 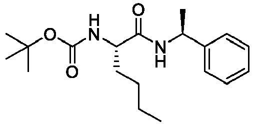 | ¹H-NMR (270 MHz, CDCl₃) δ: 0.86 (3H, t, J = 6.8 Hz), 1.20-1.40 (4H, m), 1.43 (9H, s), 1.48 (3H, d, J = 7.0 Hz),1.50-1.70 (1H, m), 1.70-1.90 (1H, m), 3.90-4.20 (1H, m), 4.98 (1H, brs), 5.11 (1H, dt, J = 7.3, 7.0 Hz), 6.38 (1H, brs), 7.21-7.38 (5H, m). |
| | | MS (ESI⁺): 335.32 [M+H]⁺ |
| 7 0 - 3 7 | 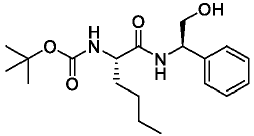 | ¹H-NMR (270 MHz, CDCl₃) δ: 0.87 (3H, t, J = 6.9 Hz), 1.20-1.40 (4H, m), 1.44 (9H, s), 1.50-1.90 (3H, m), 3.79-3.93 (2H, m), 4.05 (1H, dt, J= 6.2, 7.6 Hz), 5.05-5.13 (2H, m), 6.87 (1H, d, J = 7.6 Hz), 7.26-7.39 (5H, m). |
| | | MS (ESI⁺): 351.36 [M+H]⁺ |
| 7 0 - 3 8 | 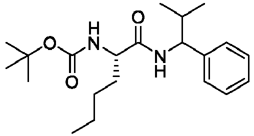 | ¹H-NMR (270 MHz, CDCl₃) δ: 0.79-0.97 (9H, m), 1.17-1.37 (4H, m), 1.43 (9H, s), 1.49-1.63 (1H, m), 1.74-1.93 (1H, m), 1.93-2.13 (1H, m), 3.89-4.12 (1H, m), 4.67-5.09 (2H, m), 6.39-6.73 (1H, m), 7.17-7.35 (5H, m). |
| | | MS (ESI+): 363.55 [M+H]⁺ |
| 7 0 - 3 9 | 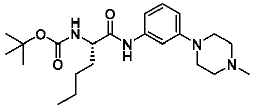 | ¹H-NMR (270 MHz, CDCl₃) δ: 0.88 (3H, t, J = 6.9 Hz), 1.44-1.44 (13H, m), 1.56-1.78 (1H, m), 1.78-2.00 (1H, m), 2.32 (3H, s), 2.46-2.60 (4H, m), 3.11-3.25 (4H, m), 4.14-4.38 (1H, m), 5.39 (1H, d, J = 8.2 Hz), 6.61 (1H, dd, J = 8.0, 1.5 Hz), 6.85 (1H, dd, J = 8.0, 1.5 Hz), 7.10 (1H, t, J = 8.0 Hz), 7.29 (1H, d, J = 1.5 Hz), 8.67 (1H, br s). |
| | | MS (ESI+): 405.46 [M+H]⁺ |
| 7 0 - 4 0 | 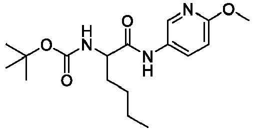 | ¹H-NMR (CDCl₃, 400 MHz) δ: 0.91 (3H, t, J = 6.7 Hz), 1.33-1.40 (4H, m), 1.46 (9H, s), 1.62-1.68 (1H, m), 1.88-1.98 (1H, m), 3.91 (3H, s), 4.13-4.20 (1H, m), 5.00 (1H, brs), 6.71 (1H, d, J = 9.1 Hz), 7.88 (1H, dd, J = 9.1, 2.4 Hz), 8.17 (1H, d, J = 2.4 Hz), 8.23 (1H, brs). |
| | | HRMS (ESI⁺):338.20806 [M+H]⁺ |

**[Table 85]**

| Reference Example | Structure | Equipment Data |
|---|---|---|
| 7 0 - 4 1 | 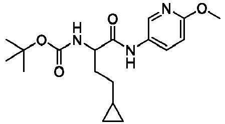 | ¹H-NMR (CDCl₃, 400 MHz) δ: 0.02-0.07 (2H, m), 0.45 (2H, d, J = 7.9 Hz), 0.64-0.73 (1H, m), 1.33 (2H, q, J = 7.9 Hz), 1.46 (9H, s), 1.71-1.82 (1H, m), 2.01-2.12 (1H, m), 3.91 (3H, s), 4.17-4.25 (1H, m), 4.94 (1H, brs), 6.72 (1H, d, J = 9.1 Hz), 7.89 (1H, dd, J = 9.1, 3.0 Hz), 8.17 (1H, d, J = 3.0 Hz), 8.18 (1H, brs). |
| | | HRMS (ESP):350.20720 [M+H]⁺ |
| 7 0 - 4 2 | 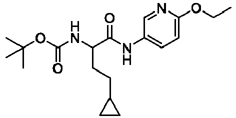 | ¹H-NMR (DMSO-D₆, 400MHz) δ: -0.05-0.04 (2H, m), 0.32-0.41 (2H, m), 0.59-0.71 (1H, m), 1.17-1.41 (14H, m), 1.57-1.79 (2H, m), 4.00-4.07 (1H, m), 4.23 (2H, q, J = 6.9 Hz), 6.75 (1H, d, J = 9.2 Hz), 7.01 (1H, d, J = 7.9 Hz), 7.87 (1H, dd, J = 9.2, 2.4 Hz), 8.31 (1H, d, J = 2.4 Hz), 9.94 (1H, s). |
| | | MS (ESI⁺): 364.2 [M+H]⁺ |
| 7 0 - 4 3 | 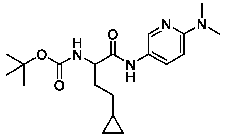 | ¹H-NMR (DMSO-D₆, 400MHz) δ: -0.05-0.05 (2H, m), 0.32-0.42 (2H, m), 0.59-0.73 (1H, m), 1.16-1.42 (11H, m), 1.57-1.79 (2H, m), 2.97 (6H, s), 3.97-4.08 (1H, m), 6.61 (1H, d, J = 9.2 Hz), 6.94 (1H, d, J = 7.9 Hz), 7.71 (1H, dd, J = 9.2, 2.4 Hz), 8.22 (1H, d, J = 2.4 Hz), 9.68 (1H, s). |
| | | MS (ESP): 363.2 [M+H]⁺ |
| 7 0 - 4 4 | 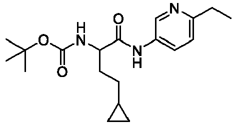 | ¹H-NMR (DMSO-D₆, 400MHz) δ: -0.04-0.05 (2H, m), 0.31-0.41 (2H, m), 0.60-0.71 (1H, m), 1.14-1.40 (14H, m), 1.58-1.79 (2H, m), 2.68 (2H, q, J = 7.5 Hz), 4.00-4.10 (1H, m), 7.04 (1H, d, J = 7.9 Hz), 7.19 (1H, d, J = 8.6 Hz), 7.92 (1H, dd, J = 8.6, 2.4 Hz), 8.61 (1H, d, J = 2.4 Hz), 10.05 (1H, s). |
| | | MS (ESP): 348.2 [M+H]⁺ |
| 7 0 - 4 5 | 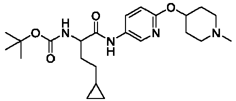 | ¹H-NMR (DMSO-D₆, 400MHz) δ: -0.08-0.05 (2H, m), 0.30-0.42 (2H, m), 0.57-0.72 (1H, m), 1.13-1.41 (11H, m), 1.55-1.78 (4H, m), 1.86-1.98 (2H, m), 2.07-2.20 (5H, m), 2.55-2.65 (2H, m), 3.97-4.08 (1H, m), 4.83-4.94 (1H, m), 6.73 (1H, d, J = 9.1 Hz), 7.02 (1H, d, J = 7.3 Hz), 7.87 (1H, dd, J = 9.1, 2.4 Hz), 8.29 (1H, d, J = 2.4 Hz), 9.94 (1H, s). |
| | | MS (ESP): 433.3 [M+H]⁺ |

**[Table 86]**

| Reference Example | Structure | Equipment Data |
|---|---|---|
| 7 0 - 4 6 | 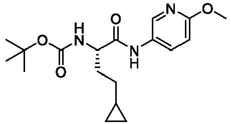 | ¹H-NMR (CDCl₃, 400 MHz) δ: 0.02-0.07 (2H, m), 0.42-0.48 (2H, m), 0.64-0.73 (1H, m), 1.29-1.37 (2H, m), 1.46 (9H, s), 1.72-1.82 (1H, m), 2.02-2.11 (1H, m), 3.91 (3H, s), 4.19-4.27 (1H, m), 4.96 (1H, brs), 6.71 (1H, d, J = 8.6 Hz), 7.87 (1H, dd, J = 8.6, 2.4 Hz), 8.16 (1H, d, J = 2.4 Hz), 8.23 (1H, brs). |
| | | HRMS (ESI⁺):350.20830 [M+H]⁺ |
| 7 0 - 4 7 | 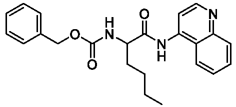 | ¹H-NMR (DMSO-D₆, 400MHz) δ: 0.87 (3H, t, J = 7.3 Hz), 1.26-1.40 (4H, m), 1.60-1.80 (2H, m), 4.42-4.52 (1H, m), 5.05 (2H, s), 7.27-7.40 (5H, m), 7.64 (1H, t, J = 7.9 Hz), 7.71 (1H, d, J = 7.9 Hz), 7. 77 (1H, t, J = 7.3 Hz), 7.96-8.04 (2H, m), 8.30 (1H, d, J = 8.5 Hz), 8.79 (1H, d, J = 4.8 Hz), 10.29 (1H, s). |
| | | HRMS (ESI⁺): 392.19681 [M+H]⁺ |
| 7 0 - 4 8 | 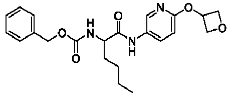 | ¹H-NMR (DMSO-D₆, 400MHz) δ: 0.84 (3H, t, J = 7.0 Hz), 1.20-1.40 (4H, m), 1.48-1.75 (2H, m), 4.05-4.14 (1H, m), 4.52 (2H, dd, J = 7.3, 5.4 Hz), 4.86 (2H, t, J = 7.3 Hz), 5.00 (1H, d, J = 12.1 Hz), 5.04 (1H, d, J = 12.1 Hz), 5.45-5.54 (1H, m), 6.87 (1H, d, J = 9.1 Hz), 7.15-7.41 (5H, m), 7.56 (1H, d, J = 7.9 Hz), 7.93 (1H, dd, J = 9.1, 3.0 Hz), 8.30 (1H, d, J = 3.0 Hz), 10.14 (1H, s). |
| | | MS (ESI⁺): 414.2 [M+H]⁺ |
| 7 0 - 4 9 | 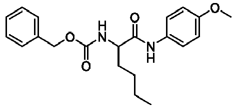 | ¹H-NMR (DMSO-D₆, 400MHz) δ: 0.84 (3H, t, J = 7.0 Hz), 1.19-1.40 (4H, m), 1.51-1.73 (2H, m), 3.71 (3H, s), 4.03-4.13 (1H, m), 5.00 (1H, d, J = 12.7 Hz), 5.04 (1H, d, J = 12.7 Hz), 6.87 (2H, d, J = 9.1 Hz), 7.26-7.39 (5H, m), 7.44-7.53 (3H, m), 9.84 (1H, s). |
| | | MS (ESI⁺): 371.2 [M+H]⁺ |
| 7 0 - 5 0 | 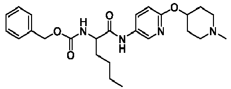 | ¹H-NMR (DMSO-D₆, 400MHz) δ: 0.84 (3H, t, J = 7.0 Hz), 1.20-1.40 (4H, m), 1.52-1.74 (4H, m), 1.87-1.97 (2H, m), 2.06-2.20 (5H, m), 2.55-2.65 (2H, m), 4.05-4.14 (1H, m), 4.84-4.94 (1H, m), 5.00 (1H, d, J = 12.7 Hz), 5.04 (1H, d, J = 12.7 Hz), 6.73 (1H, d, J = 8.5 Hz), 7.26-7.40 (5H, m), 7.56 (1H, d, J = 7.9 Hz), 7.87 (1H, dd, J = 8.5, 3.0 Hz), 8.31 (1H, d, J = 3.0 Hz), 10.03 (1H, s). |
| | | MS (ESI⁺): 455.3 [M+H]⁺ |

**[Table 87]**

| Reference Example | Structure | Equipment Data |
|---|---|---|
| 7 0 - 5 1 | 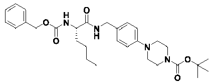 | ¹H-NMR (399 MHz, DMSO) δ: 0.87 (3H, t, J = 6.4 Hz), 1.25-1.40 (4H, m), 1.48 (9H, s), 1.55-1.70 (1H, m), 1.80-1.95 (1H, m), 3.10 (4H, t, J = 5.0 Hz), 3.57 (4H, t, J = 5.0 Hz), 4.11 (1H, m), 4.30-4.45 (2H, m), 5.07 (2H, s), 5.08 (1H, m), 6.18 (1H, brs), 6.86 (2H, d, J = 8.0 Hz), 7.15 (2H, d, J = 8.0 Hz), 7.30-7.40 (5H, m). |
| | | MS (ESI⁺): 540.24 [M+H]⁺ |
| 7 0 - 5 2 | 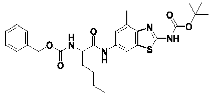 | MS (ESI⁺): 527.2 [M+H]⁺ |
| 7 0 - 5 3 | 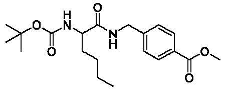 | ¹H-NMR (CDCl₃, 400 MHz) δ: 0.88 (3H, t, J = 6.7 Hz), 1.29-1.34 (4H, m), 1.40 (9H, s), 1.55-1.65 (1H, m), 1.81-1.90 (1H, m), 3.89 (3H, s), 4.04 (1H, brs), 4.49 (2H, s), 4.91 (1H, brs), 6.56 (1H, brs), 7.31 (2H, d, J = 7.9 Hz), 7.96 (2H, d, J = 7.9 Hz). |
| | | HRMS (ESI⁺):379.22295 [M+H]⁺ |
| 7 0 - 5 4 | 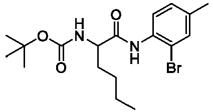 | ¹H-NMR (CDCl₃, 400 MHz) δ: 0.92 (3H, t, J = 6.7 Hz), 1.35-1.41 (4H, m), 1.47 (9H, s), 1.64-1.72 (1H, m), 1.92-2.01 (1H, m), 2.30 (3H, s), 4.23 (1H, brs), 4.97 (1H, brs), 7.11 (1H, dd, J = 8.6, 1.8 Hz), 7.36 (1H, d, J = 1.8 Hz), 8.20 (1H, d, J = 8.6 Hz), 8.32 (1H, brs). |
| | | HRMS (ESI⁺):399.12915 [M+H]⁺ |
| 7 0 - 5 5 | 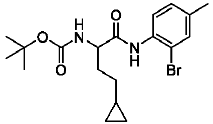 | ¹H-NMR (CDCl₃, 400 MHz) δ: 0.04-0.07 (2H, m), 0.43-0.48 (2H, m), 0.67-0.73 (1H, m), 1.30-1.37 (2H, m), 1.46 (9H, s), 1.75-1.84 (1H, m), 2.05-2.15 (1H, m), 2.30 (3H, s), 4.20-4.35 (1H, m), 4.90-5.02 (1H, m), 7.11 (1H, dd, J = 8.6, 1.8 Hz), 7.36 (1H, d, J = 1.8 Hz), 8.20 (1H, d, J = 8.6 Hz), 8.34 (1H, brs). |
| | | HRMS (ESI⁺):411.12871 [M+H]⁺ |
| 7 0 - 5 6 | 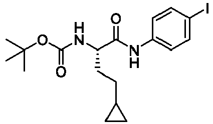 | ¹H-NMR (CDCl₃, 400 MHz) δ: -0.01-0.03 (2H, m), 0.40-0.44 (2H, m), 0.62-0.69 (1H, m), 1.25-1.32 (2H, m), 1.43 (9H, s), 1.69-1.76 (1H, m), 1.98-2.08 (1H, m), 4.10-4.25 (1H, m), 4.80-5.00 (1H, m), 7.27 (2H, d, J = 8.5 Hz), 7.58 (2H, d, J = 8.5 Hz), 8.31 (1H, s). |
| | | HRMS (ESI⁺):445.09866 [M+H]⁺ |

**[Table 88]**

| Reference Example | Structure | Equipment Data |
|---|---|---|
| 7 0 - 5 7 | 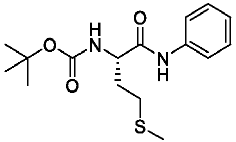 | ¹H-NMR (500 MHz, CDCl₃) δ: 1.45 (9H, s), 1.97-2.10 (1H, m), 2.12 (3H, s), 2.10-2.27 (1H, m), 2.56-2.70 (2H, m), 4.47(1H, brs), 5.44 (1H, brs), 7.09 (1H, dd, J = 7.5, 7.5 Hz), 7.28 (2H, dd, J = 7.5, 7.5 Hz), 7.50 (2H, d, J = 7.5 Hz), 8.57 (1H, brs). |
| | | MS (ESI⁺): 269.27 [M+H-t-Bu] ⁺ |
| 7 0 - 5 8 | 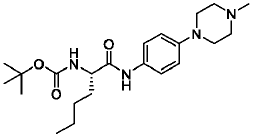 | ¹H-NMR (270 MHz, CDCl₃) δ: 0.90 (3H, t, J = 7.1 Hz), 1.30-1.43 (4H, m), 1.43-1.49 (9H, s), 1.56-1.81 (1H, m), 1.81-2.01 (1H, m), 2.35 (3H, s), 2.53-2.62 (4H, m), 3.12-3.22 (4H, m), 4.05-4.25 (1H, m), 4.89-5.13 (1H, m), 6.83-6.93 (2H, m), 7.34-7.43 (2H, m), 7.97 (1H, s). |
| | | MS (ESI⁺): 405.60 [M+H]⁺ |

### <Reference example 71>

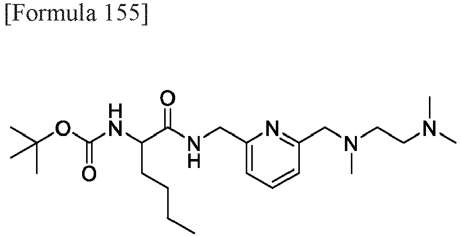

Under argon atmosphere, trifluoroacetic acid (0.663 mL) was added to a solution of tert-butyl N-[[6-[[2-(dimethylamino)ethyl-methylamino]methyl]pyridin-2-yl]methyl]-N-[(2-methylpropan-2-yl)oxycarbonyl]carbamate (112 mg) in dichloromethane (0.663 mL) under ice cooling condition, and the mixture was stirred at room temperature for 30 minutes, and then concentrated under reduced pressure. 2-[(2-Methylpropan-2-yl)oxycarbonylamino]hexanoic acid (61.3 mg) and 1-hydroxybenzotriazole monohydrate (43.0 mg) , and then N,N-diisopropylethylamine (0.180 mL) and 1-ethyl-3-(3-dimethylaminopropyl)-carbodiimide hydrochloride (56.0 mg) were added stepwise to a solution of the crude product in N,N-dimethylformamide solution (1.33 mL), and the mixture was stirred at room temperature for 7 hours. After water was added, the mixture was extracted with ethyl acetate. The organic layer was washed with saturated brine, dried over anhydrous sodium sulfate, and concentrated under reduced pressure after filtration of the insoluble material, the residue was purified by aminosilica gel column chromatography (ethyl acetate) to obtain tert-butyl N-[1-[[6-[[2-(dimethylamino)ethyl-methylamino]methyl]pyridin-2-yl]methylamino]-1-oxohexan-2-yl]carbamate (37.7 mg).

¹H-NMR (CDCl₃, 400MHz) δ: 0.88 (3H, t, J = 6.7 Hz), 1.29-1.36 (4H, m), 1.44 (9H, s), 1.55-1.65 (1H, m), 1.82-1.90 (1H, m), 2.23 (6H, s), 2.27 (3H, s), 2.41-2.51 (2H, m), 2.55-2.62 (2H, m), 3.68 (2H, s), 4.14-4.22 (1H, m), 4.51 (1H, dd, J = 16.3, 4.8 Hz), 4.59 (1H, dd, J = 16.3, 4.8 Hz), 5.34-5.56 (1H, m), 7.13 (1H, d, J = 7.9 Hz), 7.28 (1H, d, J = 7.9 Hz), 7.43 (1H, brs), 7.61 (1H, t, J = 7.9 Hz).

HRMS (ESI+): 436.32827 [M+H]⁺

### <Example 6-1>

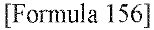

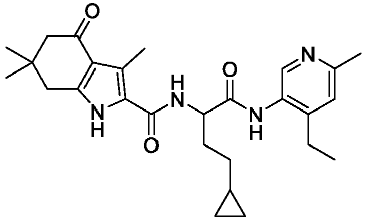

Under argon atmosphere, trifluoroacetic acid (4.00 mL) was added to a solution of tertbutyl N-[4-cyclopropyl-1-[(4-ethyl-6-methylpyridin-3-yl)amino]-1-oxobutan-2-yl]carbamate (288 mg) in dichloromethane (4.00 mL) at room temperature, and the mixture was stirred for 20 minutes. The reaction mixture was concentrated under reduced pressure. 3,6,6-trimethyl-4-oxo-5,7-dihydro-1H-indole-2-carboxylic acid (176 mg), 1-hydroxybenzotriazole monohydrate (146 mg), 1-(3-dimethylaminopropyl)-3-ethylcarbodiimide hydrochloride (168 mg), and N,N-diisopropylethylamine (0.679 mL) were added to a solution of the residue in N,N-dimethylformamide (4.00 mL) at room temperature, and the mixture was stirred for 51 hours. After the reaction mixture was added to water, and extracted twice with ethyl acetate. The combined organic layer was washed with water and saturated brine, dried over anhydrous sodium sulfate, and concentrated under reduced pressure after filtration of the insoluble material. The residue was purified by silica gel column chromatography (hexane : ethyl acetate = 3 1 to 0 : 1) to obtain N-[4-cyclopropyl-1-[(4-ethyl-6-methylpyridin-3-yl)amino]-1-oxobutan-2-yl]-3,6,6-trimethyl-4-oxo-5,7-dihydro-1H-indole-2-carboxamide (231 mg).

¹H-NMR (DMSO-D₆, 400MHz) δ: -0.02-0.06 (2H, m), 0.36-0.45 (2H, m), 0.65-0.80 (1H, m), 1.01 (6H, s), 1.09 (3H, t, J = 7.3 Hz), 1.27-1.36 (2H, m), 1.77-2.01 (2H, m), 2.21 (2H, s), 2.41 (3H, s), 2.47 (3H, s), 2.52 ( 2H, q, J = 7.3 Hz), 2.63 (2H, s), 4.59-4.70 (1H, m), 7.13 (1H, s), 7.58 (1H, d, J = 7.9 Hz), 8.24 (1H, s), 9.62 (1H, s), 11.66 (1H, s).

HRMS (ESI+): 465.28697 [M+H]⁺

Using the corresponding starting materials and reactants, the following Example 6-2 to 6-107 were obtained by the same method in Example 6-1, and the method described in Step 3-2 or Step 3-3 or a method similar thereto.

**[Table 89]**

| Example | Structure | Equipment Data |
|---|---|---|
| 6 - 2 | 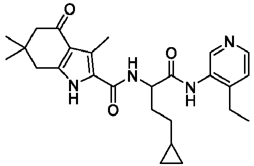 | ¹H-NMR (DMSO-D₆, 400MHz) δ: -0.04-0.04 (2H, m), 0.31-0.43 (2H, m), 0.65-0.74 (1H, m), 0.98 (6H, s), 1.08 (3H, t, J = 7.6 Hz), 1.25-1.34 (2H, m), 1.76-2.00 (2H, m), 2.19 (2H, s), 2.45 (3H, s), 2.55 (2H, q, J = 7.5 Hz), 2.61 (2H, s), 4.59-4.69 (1H, m), 7.25 (1H, d, J = 4.9 Hz), 7.59 (1H, d, J = 7.9 Hz), 8.28 (1H, d, J = 4.9 Hz), 8.39 (1H, s), 9.69 (1H, s), 11.64 (1H, s). |
| | | HRMS (ESI⁺): 451.2697 [M+H]⁺ |
| 6 - 3 | 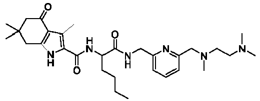 | ¹H-NMR (DMSO-D₆, 400MHz) δ: 0.80-0.90 (3H, m), 1.01 (6H, s), 1.23-1.36 (4H, m), 1.61-1.71 (1H, m), 1.72-1.82 (1H, m), 2.12 (6H, s), 2.21 (3H, s), 2.30-2.40 (2H, m), 2.47 (3H, s), 2.63 (2H, s), 2.94-3.17 (4H, m), 3.55 (2H, s), 4.32 (1H, dd, J = 16.3, 6.1 Hz), 4.38 (1H, dd, J = 16.3, 6.1 Hz), 4.42-4.49 (1H, m), 7.14 (1H, d, J = 7.9 Hz), 7.28 (1H, d, J = 7.9 Hz), 7.48 (1H, d, J = 7.9 Hz), 7.70 (1H, t, J = 7.9 Hz), 8.50-8.70 (1H, m), 11.67 (1H, s). |
| | | MS (ESI⁺):539.4 [M+H]⁺ |
| 6 - 4 | 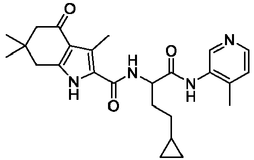 | ¹H-NMR (DMSO-D₆, 400MHz) δ: -0.01-0.07 (2H, m), 0.37-0.45 (2H, m), 0.68-0.79 (1H, m), 1.01 (6H, s), 1.27-1.38 (2H, m), 1.79-2.03 (2H, m), 2.20 (3H, s), 2.21 (2H, s), 2.47 (3H, s), 2.64 (2H, s), 4.61-4.70 (1H, m), 7.26 (1H, d, J = 4.8 Hz), 7.61 (1H, d, J = 7.9 Hz), 8.25 (1H, d, J = 4.8 Hz), 8.44 (1H, s), 9.76 (1H, s), 11.66 (1H, s). |
| | | HRMS (ESI⁺): 437.25465 [M+H]⁺ |
| 6 - 5 | 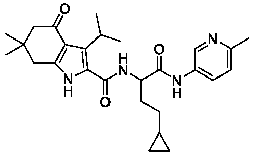 | ¹H-NMR (DMSO-D₆, 400MHz) δ: -0.03-0.06 (2H, m), 0.35-0.43 (2H, m), 0.64-0.76 (1H, m), 1.02 (6H, s), 1.14-1.38 (8H, m), 1.72-1.95 (2H, m), 2.24 (2H, s), 2.40 (3H, s), 2.65 (2H, s), 3.91-4.03 (1H, m), 4.49-4.59 (1H, m), 7.19 (1H, d, J = 8.5 Hz), 7.76 (1H, d, J = 7.3 Hz), 7.91 (1H, dd, J = 8.5, 2.4 Hz), 8.62 (1H, d, J = 2.4 Hz), 10.21 (1H, s), 11.56 (1H, s). |
| | | HRMS (ESI⁺): 465.28688 [M+H]⁺ |

**[Table 90]**

| Example | Structure | Equipment Data |
|---|---|---|
| 6 - 6 | 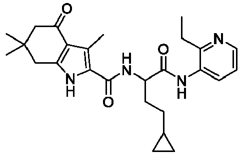 | ¹H-NMR (DMSO-D₆, 400MHz) δ: -0.06-0.04 (2H, m), 0.31-0.43 (2H, m), 0.65-0.76 (1H, m), 0.99 (6H, s), 1.13 (3H, t, J = 7.6 Hz), 1.25-1.35 (2H, m), 1.73-1.99 (2H, m), 2.19 (2H, s), 2.45 (3H, s), 2.61 (2H, s), 2.71 (2H, q, J = 7.3 Hz), 4.59-4.69 (1H, m), 7.19 (1H, dd, J = 7.9, 4.9 Hz), 7.59 (1H, d, J = 7.3 Hz), 7.69 (1H, d, J = 7.9 Hz), 8.30 (1H, d, J = 4.9 Hz), 9.66 (1H, s), 11.64 (1H, s). |
| | | MS (ESI⁺): 451.2696 [M+H]⁺ |
| 6 - 7 | 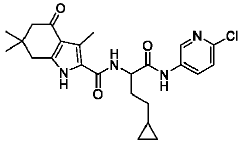 | ¹H-NMR (DMSO-D₆, 400MHz) δ: -0.05-0.07 (2H, m), 0.31-0.43 (2H, m), 0.63-0.74 (1H, m), 1.00 (6H, s), 1.18-1.37 (2H, m), 1.71-1.99 (2H, m), 2.20 (2H, s), 2.44 (3H, s), 2.62 (2H, s), 4.51-4.62 (1H, m), 7.72 (1H, d, J = 7.9 Hz), 7.85 (1H, d, J = 8.6 Hz), 8.32 (1H, dd, J = 8.6, 2.4 Hz), 8.88 (1H, d, J = 2.4 Hz), 10.74 (1H, s), 11.63 (1H, s). |
| | | HRMS (ESI⁺): 457.20108 [M+H]⁺ |
| 6 - 8 | 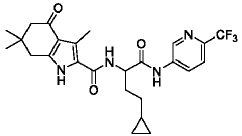 | ¹H-NMR (DMSO-D₆, 400MHz) δ: -0.04-0.02 (2H, m), 0.32-0.43 (2H, m), 0.63-0.76 (1H, m), 1.00 (6H, s), 1.21-1.34 (2H, m), 1.75-1.94 (2H, m), 2.20 (2H, s), 2.44 (3H, s), 2.62 (2H, s), 4.47-4.60 (1H, m), 7.46 (1H, d, J = 8.6 Hz), 7.69 (1H, d, J = 7.3 Hz), 8.08 (1H, dd, J = 8.6, 2.4 Hz), 8.61 (1H, d, J = 2.4 Hz), 10.50 (1H, s), 11.64 (1H, brs). |
| | | HRMS (ESI⁺): 491.22786 [M+H]⁺ |
| 6 - 9 | 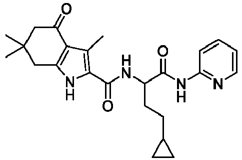 | ¹H-NMR (DMSO-D₆, 400MHz) δ: -0.05-0.05 (2H, m), 0.31-0.42 (2H, m), 0.64-0.75 (1H, m), 1.01 (3H, s), 1.01 (3H, s), 1.19-1.37 (2H, m), 1.76-1.93 (2H, m), 2.21 (2H, s), 2.45 (3H, s), 2.63 (2H, s), 4.59-4.66 (1H, m), 7.08-7.11 (1H, m), 7.61 (1H, d, J = 7.3 Hz), 7.74-7.79 (1H, m), 8.05 (1H, d, J = 8.6 Hz), 8.29-8.32 (1H, m), 10.53 (1H, s), 11.65 (1H, s). |
| | | HRMS (ESI⁺): 423.23982 [M+H]⁺ |
| 6 - 1 0 | 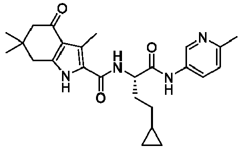 | ¹H-NMR (DMSO-D₆, 400MHz) δ: 0.00-0.04 (2H, m), 0.36-0.43 (2H, m), 0.67-0.76 (1H, m), 1.02 (6H, s), 1.24-1.35 (2H, m), 1.78-1.94 (2H, m), 2.22 (2H, s), 2.41 (3H, s), 2.47 (3H, s), 2.64 (2H, s), 4.59 (1H, td, J = 8.6, 5.5 Hz), 7.20 (1H, d, J = 8.6 Hz), 7.64 (1H, d, J = 7.9 Hz), 7.92 (1H, dd, J = 8.6, 2.4 Hz), 8.63 (1H, d, J = 2.4 Hz), 10.25 (1H, s), 11.66 (1H, s). |
| | | HRMS (ESI⁺):437.25474 [M+H]⁺ |

**[Table 91]**

| Example | Structure | Equipment Data |
|---|---|---|
| 6 - 1 1 | 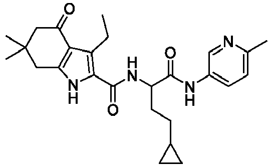 | ¹H-NMR (DMSO-D₆, 400MHz) δ: -0.03-0.04 (2H, m), 0.34-0.42 (2H, m), 0.64-0.77 (1H, m), 0.99-1.03 (9H, m), 1.19-1.33 (2H, m), 1.73-1.95 (2H, m), 2.22 (2H, s), 2.40 (3H, s), 2.64 (2H, s), 2.98 (2H, q, J = 7.5 Hz), 4.53-4.63 (1H, m), 7.19 (1H, d, J = 8.5 Hz), 7.64 (1H, d, J = 7.3 Hz), 7.91 (1H, dd, J = 8.5, 2.4 Hz), 8.62 (1H, d, J = 2.4 Hz), 10.25 (1H, s), 11.62 (1H, s). |
| | | HRMS (ESI⁺): 451.26996 [M+H]⁺ |
| 6 - 1 2 | 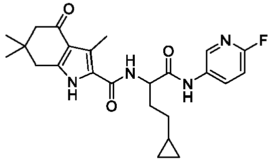 | ¹H-NMR (DMSO-D₆, 400MHz) δ: -0.02-0.02 (2H, m), 0.34-0.39 (2H, m), 0.65-0.72 (1H, m), 0.99 (6H, s), 1.21-1.32 (2H, m), 1.76-1.92 (2H, m), 2.19 (2H, s), 2.43 (3H, s), 2.61 (2H, s), 4.54 (1H, td, J = 8.5, 6.7 Hz), 7.14 (1H, dd, J = 8.5, 2.4 Hz), 7.69 (1H, d, J = 7.9 Hz), 8.12-8.18 (1H, m), 8.41 (1H, s), 10.44 (1H, s), 11.67 (1H, s). |
| | | HRMS (ESI⁺):441.23021 [M+H]⁺ |
| 6 - 1 3 | 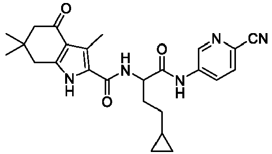 | ¹H-NMR (DMSO-D₆, 400MHz) δ: 0.02-0.06 (2H, m), 0.37-0.43 (2H, m), 0.69-0.74 (1H, m), 1.02 (6H, s), 1.22-1.39 (2H, m), 1.81-1.95 (2H, m), 2.22 (2H, s), 2.46 (3H, s), 2.64 (2H, s), 4.55-4.61 (1H, m), 7.78 (1H, d, J = 7.3 Hz), 7.99 (1H, d, J = 8.6 Hz), 8.30 (1H, dd, J = 8.6, 2.4 Hz), 8.90 (1H, d, J = 2.4 Hz), 10.85 (1H, s), 11.67 (1H, s). |
| | | HRMS (ESI⁺):448.23496 [M+H]⁺ |
| 6 - 1 4 | 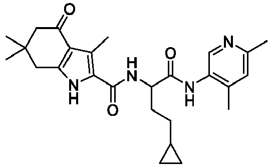 | ¹H-NMR (DMSO-D₆, 400MHz) δ: -0.03-0.02 (2H, m), 0.34-0.40 (2H, m), 0.66-0.73 (1H, m), 0.98 (6H, s), 1.25-1.32 (2H, m), 1.79-1.94 (2H, m), 2.11 (3H, s), 2.18 (2H, s), 2.36 (3H, s), 2.44 (3H, s), 2.60 (2H, s), 4.60 (1H, td, J = 7.9, 5.4 Hz), 7.08 (1H, s), 7.59 (1H, d, J = 7.9 Hz), 8.23 (1H, s), 9.64 (1H, s), 11.67 (1H, s). |
| | | HRMS (ESI⁺):451.27028 [M+H]⁺ |

**[Table 92]**

| Example | Structure | Equipment Data |
|---|---|---|
| 6 - 1 5 | 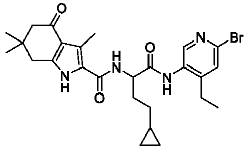 | ¹H-NMR (DMSO-D₆, 400MHz) δ: -0.02-0.03 (2H, m), 0.35-0.41 (2H, m), 0.65-0.74 (1H, m), 0.99 (6H, s), 1.08 (3H, t, J = 7.3 Hz), 1.20-1.35 (2H, m), 1.81-1.93 (2H, m), 2.19 (2H, s), 2.44 (3H, s), 2.56 (2H, q, J = 7.3 Hz), 2.61 (2H, s), 4.62 (1H, td, J = 8.5, 5.4 Hz), 7.52 (1H, s), 7.68 (1H, d, J = 8.5 Hz), 8.27 (1H, s), 9.76 (1H, s), 11.70 (1H, s). |
| | | HRMS (ESI⁺):529.18129 [M+H]⁺ |
| 6 - 1 6 | 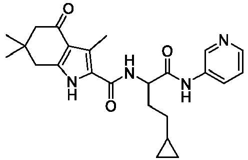 | ¹H-NMR (DMSO-D₆, 400MHz) δ: 0.01-0.05 (2H, m), 0.37-0.43 (2H, m), 0.68-0.76 (1H, m), 1.02 (6H, s), 1.24-1.37 (2H, m), 1.79-1.95 (2H, m), 2.22 (2H, s), 2.47 (3H, s), 2.65 (2H, s), 4.60 (1H, td, J = 7.9, 5.5 Hz), 7.35 (1H, dd, J = 8.6, 4.9 Hz), 7.67 (1H, d, J = 7.9 Hz), 8.03-8.07 (1H, m), 8.27 (1H, dd, J = 4.9, 1.2 Hz), 8.77 (1H, d, J = 2.4 Hz), 10.36 (1H, s), 11.66 (1H, s). |
| | | HRMS (ESI⁺):423.24008 [M+H]⁺ |
| 6 - 1 7 | 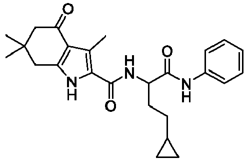 | ¹H-NMR (DMSO-D₆, 400MHz) δ: -0.02-0.03 (2H, m), 0.35-0.40 (2H, m), 0.66-0.74 (1H, m), 1.01 (6H, s), 1.20-1.34 (2H, m), 1.79-1.88 (2H, m), 2.21 (2H, s), 2.46 (3H, s), 2.63 (2H, s), 4.58 (1H, td, J = 8.5, 6.1 Hz), 7.04 (1H, tt, J = 7.3, 1.2 Hz), 7.29 (2H, dd, J = 8.5, 7.3 Hz), 7.60 (2H, dd, J = 8.5, 1.2 Hz), 7.64 (1H, brs), 10.11 (1H, s), 11.71 (1H, s). |
| | | HRMS (ESI⁺):422.24514 [M+H]⁺ |
| 6 - 1 8 | 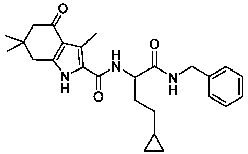 | ¹H-NMR (CDCl₃, 270MHz) δ: -0.06-0.05 (2H, m), 0.41 (2H, d, J = 7.6 Hz), 0.65-0.65 (1H, m), 1.09 (6H, s), 1.23-1.34 (2H, m), 1.74-2.17 (2H, m), 2.33 (2H, s), 2.62 (2H, s), 2.65 (3H, s), 4.33-4.70 (3H, m), 6.46 (1H, t, J = 5.9 Hz), 6.57 (1H, d, J = 7.6 Hz), 7.20-7.38 (5H, m), 9.29 (1H, s). |
| | | MS (ESI⁺): 436.35 [M+H]⁺ |
| 6 - 1 9 | 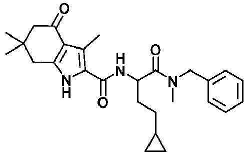 | ¹H-NMR (270 MHz, CDCl₃) δ: -0.12-0.12 (2H, m), 0.29-0.52 (2H, m), 0.55-0.80 (1H, m), 1.05-1.53 (8H, m), 1.71-2.06 (2H, m), 2.33 (2H, s), 2.63 (2H, s), 2.70 (3H, s), 3.05 (3H, s), 4.36-4.88 (2H, m), 5.11-5.31 (1H, m), 6.87-7.09 (1H, m), 7.21-7.39 (5H, m), 9.41 (1H, s). |
| | | MS (ESI⁺): 450.40 [M+H]⁺ |

**[Table 93]**

| Example | Structure | Equipment Data |
|---|---|---|
| 6 - 2 0 | 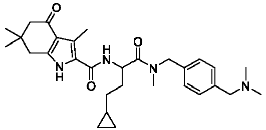 | ¹H-NMR (270 MHz, CDCl₃) δ: -0.11-0.13 (2H, m), 0.26-0.53 (2H, m), 0.53-0.81 (1H, m), 0.97-1.10 (6H, s), 1.10-1.54 (2H, m), 1.69-2.06 (2H, m), 2.10-2.27 (7H, s), 2.32 (2H, s), 2.61 (2H, s), 2.69 (3H, s), 3.05 (2H, s), 3.37-3.46 (2H, m), 4.37-4.86 (2H, m), 5.11-5.30 (1H, m), 6.93-7.09 (1H, m), 7.13-7.21 (2H, m), 7.24-7.34 (2H, m), 9.86 (1H, s). |
| | | MS (ESI⁺): 507.70 [M+H]⁺ |
| 6 - 2 1 | 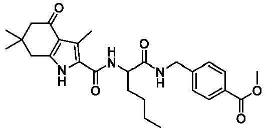 | ¹H-NMR (DMSO-D₆, 400MHz) δ: 0.80-0.90 (3H, m), 1.01 (6H, s), 1.25-1.32 (4H, m), 1.61-1.78 (2H, m), 2.21 (2H, s), 2.47 (3H, s), 2.63 (2H, s), 3.83 (3H, s), 4.36 (2H, d, J = 6.1 Hz), 4.42 (1H, td, J = 8.5, 5.4 Hz), 7.39 (2H, d, J = 8.5 Hz), 7.54 (1H, d, J = 7.9 Hz), 7.89 (2H, d, J = 8.5 Hz), 8.66 (1H, t, J = 6. 1 Hz), 11.73 (1H, s). |
| | | HRMS (ESI⁺):482.26515 [M+H]⁺ |
| 6 - 2 2 | 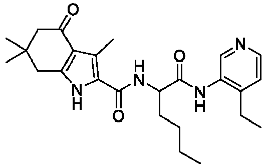 | ¹H-NMR (DMSO-D₆, 400MHz) δ: 0.89 (3H, t, J = 7.0 Hz), 1.01 (6H, s), 1.11 (3H, t, J = 7.2 Hz), 1.29-1.45 (4H, m), 1.70-1.92 (2H, m), 2.21 (2H, s), 2.48 (3H, s), 2.58 (2H, q, J = 7.2 Hz), 2.64 (2H, s), 4.60-4.68 (1H, m), 7.28 (1H, d, J = 5.2 Hz), 7.61 (1H, d, J = 7.9 Hz), 8.30 (1H, d, J = 5.2 Hz), 8.43 (1H, s), 9.73 (1H, s), 11.68 (1H, s). |
| | | HRMS (ESI⁺): 439.27123 [M+H]⁺ |
| 6 - 2 3 | 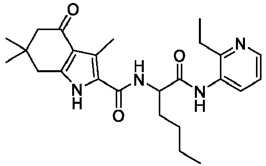 | ¹H-NMR (DMSO-D₆, 400MHz) δ: 0.96 (3H, t, J = 7.0 Hz), 1.08 (6H, s), 1.22 (3H, t, J = 7.6 Hz), 1.36-1.52 (4H, m), 1.74-1.96 (2H, m), 2.29 (2H, s), 2.55 (3H, s), 2.71 (2H, s), 2.81 (2H, q, J = 7.3 Hz), 4.70-4.74 (1H, m), 7.29 (1H, dd, J = 8.3, 4.6 Hz), 7.68 (1H, d, J = 7.3 Hz), 7.79 (1H, dd, J = 8.3, 1.5 Hz), 8.39 (1H, dd, J = 4.6, 1.5 Hz), 9.76 (1H, s), 11.75 (1H, s). |
| | | HRMS (ESI⁺): 439.27070 [M+H]⁺ |

**[Table 94]**

| Example | Structure | Equipment Data |
|---|---|---|
| 6 - 2 4 | 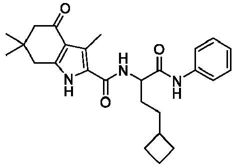 | ¹H-NMR (DMSO-D₆, 400MHz) δ: 1.03 (6H, s), 1.41-1.84 (8H, m), 1.98-2.04 (2H, m), 2.23 (2H, s), 2.24-2.29 (1H, m), 2.48 (3H, s), 2.65 (2H, s), 4.55 (1H, td, J = 7.9, 5.4 Hz), 7.05 (1H, tt, J = 7.3, 1.2 Hz), 7.31 (2H, dd, J = 8.5, 7.3 Hz), 7.56-7.63 (3H, m), 10.12 (1H, s), 11.68 (1H, s). |
| | | HRMS (ESI⁺):436.25967 [M+H]⁺ |
| 6 - 2 5 | 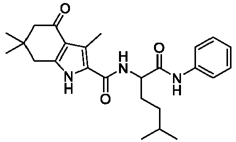 | ¹H-NMR (DMSO-D₆, 400MHz) δ: 0.87 (3H, d, J = 6.7 Hz), 0.88 (3H, d, J = 6.7 Hz), 1.03 (6H, s), 1.20-1.34 (2H, m), 1.53-1.60 (1H, m), 1.68-1.84 (2H, m), 2.23 (2H, s), 2.48 (3H, s), 2.65 (2H, s), 4.56 (III, td, J = 7.9, 5.4 Hz), 7.05 (1H, tt, J = 7.3, 1.2 Hz), 7.31 (2H, dd, J = 8.5, 7.3 Hz), 7.56-7.65 (3H, m), 10.13 (1H, s), 11.68 (1H, s). |
| | | HRMS (ESI⁺):424.26011 [M+H]⁺ |
| 6 - 2 6 | 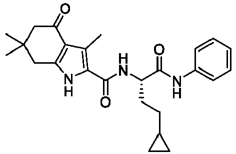 | ¹H-NMR (DMSO-D₆, 400MHz) δ: 0.01-0.04 (2H, m), 0.37-0.42 (2H, m), 0.67-0.76 (1H, m), 1.03 (6H, s), 1.23-1.35 (2H, m), 1.76-1.94 (2H, m), 2.23 (2H, s), 2.47 (3H, s), 2.65 (2H, s), 4.60 (1H, td, J = 7.9, 5.4 Hz), 7.05 (1H, tt, J = 7.3, 1.2 Hz), 7.31 (2H, dd, J = 8.5, 7.3 Hz), 7.60 (1H, d, J = 7.9 Hz), 7.61 (2H, dd, J = 8.5, 1.2 Hz), 10.12 (1H, s), 11.67 (1H, s). |
| | | HRMS (ESI⁺):422.24470 [M+H]⁺ |
| 6 - 2 7 | 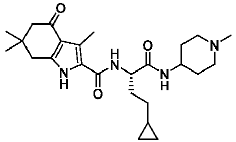 | ¹H-NMR (DMSO-D₆, 400MHz) δ: -0.02-0.01 (2H, m), 0.36-0.42 (2H, m), 0.66-0.71 (1H, m), 1.03 (6H, s), 1.17-1.24 (2H, m), 1.35-1.49 (2H, m), 1.65-1.83 (4H, m), 1.88-1.96 (2H, m), 2.15 (3H, s), 2.23 (2H, s), 2.47 (3H, s), 2.65 (2H, s), 2.66-2.73 (2H, m), 3.46-3.56 (1H, m), 4.44 (1H, td, J = 7.9, 5.5 Hz), 7.37 (1H, d, J = 7.9 Hz), 7.98 (1H, d, J = 7.9 Hz), 11.70 (1H, s). |
| | | HRMS (ESI⁺):443.30217 [M+H]⁺ |
| 6 - 2 8 | 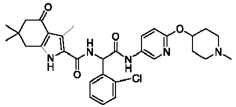 | ¹H-NMR (DMSO-D₆, 400MHz) δ: 1.01 (6H, s), 1.55-1.69 (2H, m), 1.86-1.96 (2H, m), 2.07-2.18 (5H, m), 2.21 (2H, s), 2.46 (3H, s), 2.56-2.65 (4H, m), 4.85-4.94 (1H, m), 5.99 (1H, d, J = 7.9 Hz), 6.75 (1H, d, J = 8.5 Hz), 7.35-7.42 (2H, m), 7.46-7.54 (2H, m), 7.90 (1H, dd, J = 8.5, 3.0 Hz), 8.21 (1H, d, J = 7.9 Hz), 8.36 (1H, d, J = 3.0 Hz), 10.41 (1H, s), 11.64 (1H, s). |
| | | HRMS (ESI⁺): 578.25260 [M+H]⁺ |

**[Table 95]**

| Example | Structure | Equipment Data |
|---|---|---|
| 6-29 | 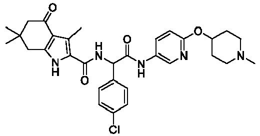 | ¹H-NMR (DMSO-D₆, 400MHz) δ: 1.00 (3H, s), 1.01 (3H, s), 1.54-1.67 (2H, m), 1.85-1.96 (2H, m), 2.06-2.17 (5H, m), 2.21 (2H, s), 2.48 (3H, s), 2.55-2.65 (4H, m), 4.84-4.93 (1H, m), 5.77 (1H, d, J = 7.3 Hz), 6.74 (1H, d, J = 9.1 Hz), 7.46 (2H, d, J = 8.5 Hz), 7.55 (2H, d, J = 8.5 Hz), 7.86 (1H, dd, J = 8.5, 2.4 Hz), 8.13 (1H, d, J = 7.3 Hz), 8.31 (1H, d, J = 2.4 Hz), 10.48 (1H, s), 11.79 (1H, s). |
| | | HRMS (ESI⁺): 578.25319 [M+H]⁺ |
| 6-30 | 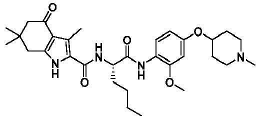 | ¹H-NMR (DMSO-D₆, 400MHz) δ: 0.88 (3H, t, J = 7.0 Hz), 1.02 (6H, s), 1.27-1.43 (4H, m), 1.55-1.67 (2H, m), 1.67-1.86 (2H, m), 1.87-1.96 (2H, m), 2.11-2.19 (5H, m), 2.23 (2H, s), 2.49 (3H, s), 2.56-2.69 (4H, m), 3.77 (3H, s), 4.28-4.38 (1H, m), 4.60-4.70 (1H, m), 6.51 (1H, dd, J = 9.1, 2.4 Hz), 6.60 (1H, d, J = 2.4 Hz), 7.60 (1H, d, J = 7.9 Hz), 7.67 (1H, d, J = 9.1 Hz), 9.14 (1H, s), 11.71 (1H, s). |
| | | HRMS (ESI⁺): 553.33831 [M+H]⁺ |
| 6-31 | 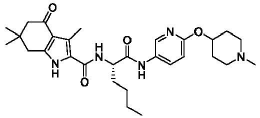 | ¹H-NMR (DMSO-D₆, 400MHz) δ: 0.89 (3H, t, J = 7.3 Hz), 1.04 (6H, s), 1.27-1.47 (4H, m), 1.57-1.86 (4H, m), 1.90-1.99 (2H, m), 2.10-2.20 (5H, m), 2.24 (2H, s), 2.49 (3H, s), 2.58-2.71 (4H, m), 4.52-4.62 (1H, m), 4.85-4.97 (1H, m), 6.77 (1H, d, J = 9.2 Hz), 7.63 (1H, d, J = 7.9 Hz), 7.91 (1H, dd, J = 9.2, 3.1 Hz), 8.35 (1H, d, J = 3.1 Hz), 10.18 (1H, s), 11.69 (1H, s). |
| | | HRMS (ESI⁺): 524.32421 [M+H]⁺ |
| 6-32 | 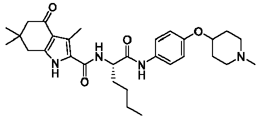 | ¹H-NMR (DMSO-D₆, 400MHz) δ: 0.89 (3H, t, J = 7.0 Hz), 1.04 (6H, s), 1.27-1.45 (4H, m), 1.54-1.66 (2H, m), 1.67-1.85 (2H, m), 1.86-1.97 (2H, m), 2.10-2.19 (5H, m), 2.24 (2H, s), 2.49 (3H, s), 2.57-2.67 (4H, m), 4.22-4.35 (1H, m), 4.49-4.61 (1H, m), 6.90 (2H, d, J = 9.1 Hz), 7.51 (2H, d, J = 9.1 Hz), 7.57 (1H, d, J = 7.9 Hz), 10.00 (1H, s), 11.70 (1H, s). |
| | | HRMS (ESI⁺): 523.32884 [M+H]⁺ |

**[Table 96]**

| Example | Structure | Equipment Data |
|---|---|---|
| 6-33 | 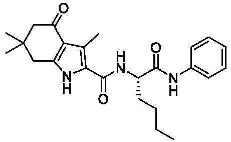 | ¹H-NMR (DMSO-D₆, 400MHz) δ: 0.87 (3H, t, J = 7.3 Hz), 1.01 (6H, s), 1.27-1.42 (4H, m), 1.67-1.82 (2H, m), 2.21 (2H, s), 2.47 (3H, s), 2.64 (2H, s), 4.53-4.59 (1H, m), 7.04 (1H, tt, J = 7.3, 1.2 Hz), 7.29 (2H, dd, J = 8.5, 7.3 Hz), 7.60 (2H, dd, J = 8.5, 1.2 Hz), 7.63 (1H, brs), 10.12 (1H, s), 11.72 (1H, s). |
| | | HRMS (ESI⁺):410.24438 [M+H]⁺ |
| 6-34 | 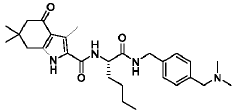 | ¹H-NMR (DMSO-D₆, 400MHz) δ: 0.84 (3H, t, J = 6.7 Hz), 1.01 (6H, s), 1.23-1.30 (4H, m), 1.59-1.78 (2H, m), 2.10 (6H, s), 2.21 (2H, s), 2.46 (3H, s), 2.62 (2H, s), 3.32 (2H, s), 4.24 (1H, dd, J = 15.1, 6.1 Hz), 4.29 (1H, dd, J = 15.1, 6.1 Hz), 4.43 (1H, td, J = 7.9, 5.4 Hz), 7.15-7.23 (4H, m), 7.47 (1H, d, J = 7.9 Hz), 8.53 (1H, t, J = 5.4 Hz), 11.71 (1H, br s). |
| | | HRMS (ESI⁺):481.31711 [M+H]⁺ |
| 6-35 | 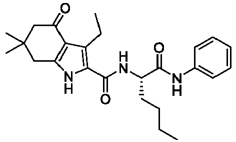 | ¹H-NMR (270 MHz, CDCl₃) δ: 0.89 (3H, t, J = 6.6 Hz), 1.04 (3H, s), 1.06 (3H, s), 1.26 (3H, t, J = 8.3 Hz), 1.29-1.51 (4H, m), 1.75-1.90 (1H, m), 1.96-2.12 (1H, m), 2.31 (2H, s), 2.57 (2H, s), 3.01-3.19 (2H, m), 4.76 (1H, q, J = 6.6 Hz), 6.86 (1H, brs), 7.08 (1H, t, J = 7.5 Hz), 7.26 (2H, dd, J = 7.5, 8.3 Hz), 7.49 (2H, d, J =8.3 Hz), 8.63 (1H, br s), 9.59 (1H, brs). |
| | | MS (ESI⁺): 424.34 [M+H]⁺ |
| 6-36 | 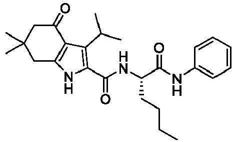 | ¹H-NMR (270 MHz, CDCl₃) δ: 0.91 (3H t, J = 7.1 Hz), 1.03 (3H, s), 1.04 (3H, s), 1.32-1.52 (4H, m), 1.37 (3H, d, J = 6.8 Hz), 1.39 (3H, d, J = 6.8 Hz), 1.75-1.92 (1H, m), 1.97-2.13 (1H, m), 2.32 (2H, s), 2.54 (2H, s), 3.56-3.72 (1H, m), 4.67-4.80 (1H, m), 6.68-6.82 (1H, m), 7.09 (1H, t, J = 7.4 Hz), 7.26 (2H, dd, J = 7.4, 7.7 Hz), 7.48 (2H, d, J = 7.7 Hz), 8.52 - 8.67 (1H, m), 9.43-9.55 (1H, m). |
| | | MS (ESI⁺): 438.49 [M+H]⁺ |
| 6-37 | 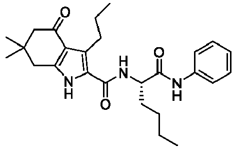 | ¹H-NMR (270 MHz, CDCl₃) δ: 0.91 (3H, t, J =6.9 Hz), 1.02 (3H, t, J = 7.3 Hz), 1.08 (6H, s), 1.32-1.50 (4H, m), 1.61-1.71 (2H, m), 1.76-1.88 (1H, m), 1.97-2.12 (1H, m), 2.33 (2H, s), 2.62 (2H, s), 2.94-3.15 (2H, m), 4.70 (1H, dt, J =7.6, 7.1 Hz), 6.59 (1H, d, J = 7.6 Hz), 7.11 (1H, t, J = 6.9 Hz), 7.31 (2H, dd, J = 6.9, 8.6 Hz), 7.52 (2H, d, J = 8.6 Hz), 8.34 (1H, brs), 9.35 (1H, brs) |
| | | MS (ESI⁺): 438.53 [M+H]⁺ |

**[Table 97]**

| Example | Structure | Equipment Data |
|---|---|---|
| 6-38 | 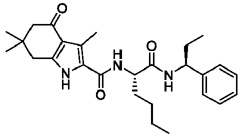 | ¹H-NMR (270 MHz, CDCl₃) δ: 0.71-0.94 (6H, m), 1.03 (3H, s), 1.04 (3H, s), 1.12-1.42 (4H, m), 1.47-2.08 (4H, m), 2.31 (2H, s), 2.55 (2H, s), 2.66 (3H, s), 4.66 (1H, dt, J = 7.1, 7.1 Hz), 4.77-4.91 (1H, m), 7.00 (1H, d, J = 7.1 Hz), 7.09 (1H, d, J = 7.3 Hz), 7.21-7.37 (5H, m), 10.24 (1H, br s) |
| | | MS (ESI⁺): 452.45 [M+H]⁺ |
| 6-39 | 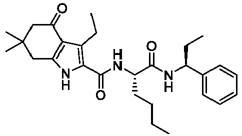 | ¹H-NMR (270 MHz, CDCl₃) δ: 0.80 (3H, t, J =6.9 Hz), 0.87 (3H, t, J = 7.3 Hz), 1.09 (6H, s), 1.15-1.34 (4H, m), 1.26 (3H, t, J = 7.6 Hz), 1.62-2.05 (4H, m), 2.34 (2H, s), 2.62 (2H, s), 3.08 (2H, q, J = 7.6 Hz), 4.58 (1H, dt, J = 7.6, 7.6 Hz), 5.05 (1H, dt, J = 7.6, 7.3 Hz), 6.38 (1H, d, J = 7.6 Hz), 6.67 (1H, d, J = 7.6 Hz), 7.17-7.38 (5H, m), 9.42 (1H, brs). |
| | | MS (ESI⁺): 466.64 [M+H]⁺ |
| 6-40 | 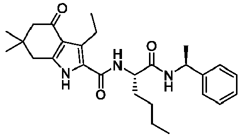 | ¹H-NMR (270 MHz, CDCl₃) δ: 0.77-0.88 (3H, m), 1.06 (6H, s), 1.15-1.13 (7H, m), 1.38 (3H, d, J = 7.1 Hz), 1.57-1.73 (1H, m), 1.73-1.89 (1H, m), 2.30 (2H, s), 2.52 (2H, s), 3.13 (2H, q, J = 7.3 Hz), 4.66 (1H, dt, J = 7.5, 7.7 Hz), 5.05 (1H, dq, J = 7.5, 7.1 Hz), 6.74 (1H, d, J = 7.5 Hz), 6.90 (1H, d, J = 7.5 Hz), 7.22-7.39 (5H, m), 9.85 (1H, brs) |
| | | MS (ESI⁺): 452.5 [M+H]⁺ |
| 6-41 | 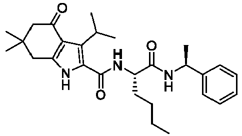 | ¹H-NMR (270 MHz, CDCl₃) δ: 0.79 - 0.88 (3H, m), 1.08 (6H, s), 1.12-1.34 (4H, m), 1.39 (3H, d, J = 6.7 Hz), 1.40 (3H, d, J = 6.7 Hz), 1.48 (3H, d, J = 7.1 Hz), 1.59-1.74 (1H, m), 1.81-1.97 (1H, m), 2.35 (2H, s), 2.60 (2H, s), 3.54-3.66 (1H, m), 4.55 (1H, dt, J =7.5, 7.1 Hz), 5.10 (1H, dq, J = 7.5, 7.1 Hz), 6.40 (1H, d, J = 7.5 Hz), 6.63 (1H, d, J = 7.5 Hz), 7.21-7.39 (5H, m), 9.38 (1H, brs). |
| | | MS (ESI⁺): 466.57 [M+H]⁺ |
| 6-42 | 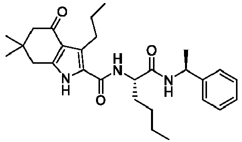 | ¹H-NMR (270 MHz, CDCl₃) δ: 0.84 (3H, t, J = 6.7 Hz), 1.02 (3H, t, J = 7.4 Hz), 1.08 (6H, s), 1.22-1.34 (4H, m), 1.48 (3H, d, J = 6.9 Hz), 1.58-1.75 (3H, m), 1.80-1.94 (1H, m), 2.33 (2H, s), 2.61 (2H, s), 2.96-3.10 (2H, m), 4.55 (1H, dt, J = 8.2, 5.9 Hz), 5.12 (1H, dq, J = 7.9, 6.9 Hz), 6.38 (1H, d, J = 8.2 Hz), 6.64 (1H, d, J = 7.9 Hz), 7.28-7.37 (5H, m), 9.45 (1H, brs) |
| | | MS (ESI⁺): 466.59 [M+H]⁺ |

**[Table 98]**

| Example | Structure | Equipment Data |
|---|---|---|
| 6-43 | 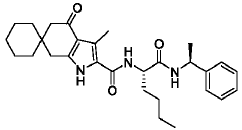 | ¹H-NMR (270 MHz, CDCl₃) δ: 0.81 (3H ,t, J = 6.8 Hz), 1.19-1.33 (4H, m), 1.33 - 1.52 (10H, m), 1.47 (3H, d, J = 6.9 Hz), 1.63 - 1.74 (1H, m), 1.80-1.93 (1H, m), 2.38 (2H, s), 2.62 (3H, s), 2.66 (2H, s), 4.53 (1H, dt, J = 7.6, 6.6 Hz), 5.09 (1H, dq, J = 7.9, 6.9 Hz), 6.31 (1H, d, J = 7.6 Hz), 6.56 (1H, d, J = 7.9 Hz), 7.21-7.37 (5H, m), 9.33 (1H, brs). |
| | | MS (ESI⁺): 478.39 [M+H]⁺ |
| 6-44 | 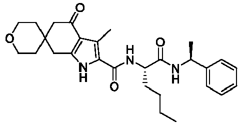 | ¹H-NMR (270 MHz, CDCl₃) δ: 0.81 (3H, t, J = 6.7 Hz), 1.18-1.35 (4H, m), 1.47 (3H, d, J = 6.9 Hz), 1.54-1.76 (5H, m), 1.76-1.95 (1H, m), 2.49 (2H, s), 2.62 (3H, s), 2.76 (2H, s), 3.57-3.75 (4H, m), 4.54 (1H, dt, J = 7.6, 6.8 Hz), 5.09 (1H, dq, J = 7.3, 7.4 Hz), 6.32 (1H, d, J = 7.6 Hz), 6.64 (1H, d, J = 7.3 Hz), 7.15-7.40 (5H, m), 9.59 (1H, brs). |
| | | MS (ESI⁺): 480.40 [M+H]⁺ |
| 6-45 | 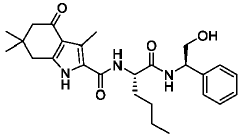 | ¹H-NMR (270 MHz, CDCl₃) δ: 0.86 (3H, t, J = 7.0 Hz), 1.03 (3H, s), 1.06 (3H, s), 1.22-1.40 (4H, m), 1.82-2.01 (2H, m), 2.29 (2H, s), 2.52 (2H, d, J = 7.25 Hz), 2.64 (3H, s), 3.19-3.28 (1H, m), 3.84-4.05 (2H, m), 5.02 (1H, dt, J = 7.6, 6.9 Hz), 5.15-5.25 (1H, m), 6.79 (1H, d, J = 6.9 Hz), 7.28-7.40 (5H, m), 8.15 (1H, d, J = 6.9 Hz), 10.17 (1H, brs) |
| | | MS (ESI⁺): 454.48 [M+H]⁺ |
| 6-46 | 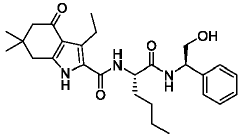 | ¹H-NMR (270 MHz, CDCl₃) δ: 0.77-0.91 (3H, m), 1.05 (3H, s), 1.07 (3H, s), 1.17-1.37 (4H, m), 1.25 (3H, t, J = 7.9 Hz), 1.70 - 2.05 (2H, m), 2.31 (2H, s),2.51-2.59 (2H, m), 3.09 (2H, q, J = 7.5 Hz), 3.62-3.67 (1H, m), 3.81-3.96 (2H, m), 4.93 (1H, dt, J = 6.8, 6.8 Hz), 5.12-5.22 (1H, m), 6.90 (1H, d, J = 6.8 Hz), 7.21-7.40 (5H, m), 7.87 (1H, d, J = 7.5 Hz), 10.21 (1H, brs) |
| | | MS (ESI⁺): 468.51 [M+H]⁺ |
| 6-47 | 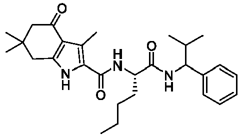 | ¹H-NMR (270 MHz, CDCl₃) δ: 0.75-1.03 (m, 9H), 1.09-1.10 (m, 6H), 1.10-214 (m, 7H), 2.33 (d, J = 2.6 Hz, 2H), 2.55-2.70 (m, 5H), 4.51-4.79 (m, 2H), 6.40-6.60 (m, 2H), 7.13-7.39 (m, 5H), 9.17-9.36 (m, 1H). |
| | | MS (ESI⁺): 466.69 [M+H]⁺ |

**[Table 99]**

| Example | Structure | Equipment Data |
|---|---|---|
| 6-48 | 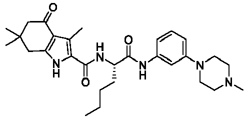 | ¹H-NMR (270 MHz, CDCl₃) δ: 0.91 (3H, t, J = 6.9 Hz), 1.08 (6H, s), 1.28-1.50 (4H, m), 1.69-1.90 (1H, m), 1.93-2.14 (1H, m), 2.31-2.36 (5H, m), 2.50-2.58 (4H, m), 2.62 (2H, s), 2.66 (3H, s), 3.16-3.25 (4H, m), 4.61-4.73 (1H, m), 6.47 (1H, d, J = 8.2 Hz), 6.67 (1H, dd, J = 8.2, 1.7 Hz), 6.85 (1H, dd, J = 8.2, 1.7 Hz), 7.17 (1H, t, J = 8.2 Hz), 7.32 (1H, s), 8.19 (1H, s), 9.29 (1H, s). |
| | | MS (ESI⁺): 508.56 [M+H]⁺ |
| 6-49 | 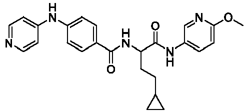 | ¹H-NMR (DMSO-D₆, 400MHz) δ: -0.04-0.04 (2H, m), 0.36 (2H, d, J = 7.9 Hz), 0.64-0.73 (1H, m), 1.18-1.34 (2H, m), 1.82-1.92 (2H, m), 3.78 (3H, s), 4.54 (1H, td, J = 7.9, 5.4 Hz), 6.76 (1H, d, J = 9.1 Hz), 6.98 (2H, d, J = 6.1 Hz), 7.21 (2H, d, J = 8.5 Hz), 7.85-7.91 (3H, m), 8.22 (2H, d, J = 6.1 Hz), 8.35 (1H, d, J = 2.4 Hz), 8.41 (1H, d, J = 7.9 Hz), 9.06 (1H, s), 10.08 (1H, s). |
| | | HRMS (ESI⁺):446.21925 [M+H]⁺ |
| 6-50 | 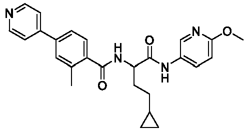 | ¹H-NMR (DMSO-D₆, 400MHz) δ: 0.03-0.07 (2H, m), 0.38-0.44 (2H, m), 0.68-0.77 (1H, m), 1.27-1.38 (2H, m), 1.78-1.93 (2H, m), 2.42 (3H, s), 3.82 (3H, s), 4.56 (1H, td, J = 7.9, 5.4 Hz), 6.82 (1H, d, J = 9.1 Hz), 7.50 (1H, d, J = 7.9 Hz), 7.67-7.71 (2H, m), 7.73 (2H, d, J= 6.1 Hz), 7.95 (1H, dd, J = 9.1, 2.4 Hz), 8.38 (1H, d, J = 2.4 Hz), 8.57 (1H, d, J = 7.9 Hz), 8.65 (2H, d, J = 6.1 Hz), 10.14 (1H, s). |
| | | HRMS (ESI⁺):445.22424 [M+H]⁺ |
| 6-51 | 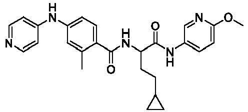 | ¹H-NMR (DMSO-D₆, 400MHz) δ: -0.03-0.03 (2H, m), 0.32-0.40 (2H, m), 0.63-0.72 (1H, m), 1.21-1.34 (2H, m), 1.75-1.87 (2H, m), 2.30 (3H, s), 3.77 (3H, s), 4.48 (1H, brs), 6.76 (1H, d, J = 7.9 Hz), 6.85-6.95 (2H, m), 6.97-7.04 (2H, m), 7.35 (1H, d, J = 7.3 Hz), 7.89 (1H, d, J = 7.3 Hz), 8.15-8.23 (2H, m), 8.29 (1H, d, J = 7.3 Hz), 8.33 (1H, s), 8.86 (1H, s), 10.05 (1H, s). |
| | | HRMS (ESI⁺):460.23387 [M+H]⁺ |

**[Table 100]**

| Example | Structure | Equipment Data |
|---|---|---|
| 6-52 | 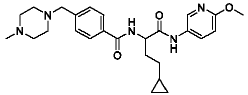 | ¹H-NMR (DMSO-D₆, 400MHz) δ: 0.01-0.06 (2H, m), 0.36-0.42 (2H, m), 0.68-0.75 (1H, m), 1.23-1.35 (2H, m), 1.87-1.94 (2H, m), 2.14 (3H, s), 2.23-2.40 (8H, m), 3.50 (2H, s), 3.81 (3H, s), 4.58 (1H, td, J = 8.5, 7.3 Hz), 6.80 (1H, d, J = 9.1 Hz), 7.38 (2H, d, J = 7.9 Hz), 7.87 (2H, d, J = 7.9 Hz), 7.91 (1H, dd, J = 9.1, 2.4 Hz), 8.37 (1H, d, J = 2.4 Hz), 8.53 (1H, d, J = 7.9 Hz), 10.11 (1H, s). |
| | | HRMS (ESI⁺):466.28113 [M+H]⁺ |
| 6-53 | 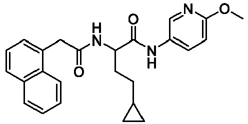 | ¹H-NMR (DMSO-D₆, 400MHz) δ: -0.08--0.02 (2H, m), 0.35 (2H, d, J = 7.9 Hz), 0.62-0.70 (1H, m), 1.17-1.24 (2H, m), 1.69-1.86 (2H, m), 3.81 (3H, s), 3.94 (1H, d, J = 15.1 Hz), 4.04 (1H, d, J = 15.1 Hz), 4.42 (1H, td, J = 8.5, 5.4 Hz), 6.79 (1H, d, J = 8.5 Hz), 7.44 (2H, d, J = 4.8 Hz), 7.48-7.52 (2H, m), 7.81 (1H, t, J = 4.8 Hz), 7.86-7.93 (2H, m), 8.09-8.14 (1H, m), 8.35 (1H, d, J = 2.4 Hz), 8.49 (1H, d, J = 8.5 Hz), 10.07 (1H, s). |
| | | HRMS (ESI⁺):418.21232 [M+H]⁺ |
| 6-54 | 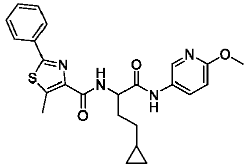 | ¹H-NMR (DMSO-D₆, 400MHz) δ: 0.02-0.07 (2H, m), 0.38-0.43 (2H, m), 0.68-0.77 (1H, m), 1.23-1.39 (2H, m), 1.85-1.96 (2H, m), 2.62 (3H, s), 3.82 (3H, s), 4.54 (1H, td, J = 7.9, 5.5 Hz), 6.81 (1H, d, J = 8.6 Hz), 7.51-7.54 (3H, m), 7.91-7.97 (3H, m), 8.38 (1H, d, J = 2.4 Hz), 8.50 (1H, d, J = 7.9 Hz), 10.17 (1H, s). |
| | | HRMS (ESI⁺):451.17980 [M+H]⁺ |
| 6-55 | 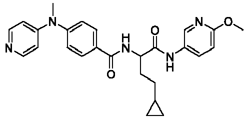 | ¹H-NMR (DMSO-D₆, 400MHz) δ: 0.01-0.07 (2H, m), 0.38-0.43 (2H, m), 0.69-0.77 (1H, m), 1.23-1.38 (2H, m), 1.86-1.96 (2H, m), 3.34 (3H, s), 3.82 (3H, s), 4.60 (1H, td, J = 7.9, 5.5 Hz), 6.75 (2H, d, J = 6.1 Hz), 6.81 (1H, d, J = 8.6 Hz), 7.37 (2H, d, J = 8.6 Hz), 7.92 (1H, dd, J = 8.6, 2.4 Hz), 7.99 (2H, d, J = 8.6 Hz), 8.19 (2H, d, J = 6.1 Hz), 8.38 (1H, d, J = 2.4 Hz), 8.58 (1H, d, J = 7.9 Hz), 10.12 (1H, s). |
| | | HRMS (ESI⁺):460.23388 [M+H]⁺ |

**[Table 101]**

| Example | Structure | Equipment Data |
|---|---|---|
| 6-56 | 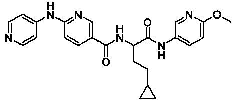 | ¹H-NMR (DMSO-D₆, 400MHz) δ: 0.02-0.08 (2H, m), 0.37-0.43 (2H, m), 0.69-0.75 (1H, m), 1.23-1.36 (2H, m), 1.88-1.95 (2H, m), 3.81 (3H, s), 4.54-4.60 (1H, m), 6.80 (1H, d, J = 8.6 Hz), 6.97 (1H, d, J = 8.6 Hz), 7.71 (2H, d, J = 6.1 Hz), 7.92 (1H, dd, J = 8.6, 2.4 Hz), 8.14 (1H, dd, J = 8.6, 2.4 Hz), 8.35 (2H, d, J = 6.1 Hz), 8.38 (1H, d, J = 2.4 Hz), 8.56 (1H, d, J = 7.3 Hz), 8.82 (1H, d, J = 2.4 Hz), 9.88 (1H, s), 10.12 (1H, s). |
| | | HRMS (ESI⁺):447.21408 [M+H]⁺ |
| 6-57 | 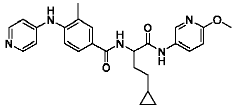 | ¹H-NMR (DMSO-D₆, 400MHz) δ: 0.02-0.07 (2H, m), 0.37-0.43 (2H, m), 0.69-0.76 (1H, m), 1.23-1.38 (2H, m), 1.87-1.96 (2H, m), 2.26 (3H, s), 3.82 (3H, s), 4.55-4.63 (1H, m), 6.77-6.82 (3H, m), 7.33 (1H, d, J = 8.6 Hz), 7.76 (1H, d, J = 8.6 Hz), 7.86 (1H, s), 7.92 (1H, d, J = 8.6 Hz), 8.18 (2H, d, J = 4.9 Hz), 8.30-8.40 (2H, m), 8.46 (1H, d, J = 7.3 Hz), 10.12 (1H, s). |
| | | HRMS (ESI⁺):460.23491 [M+H]⁺ |
| 6-58 | 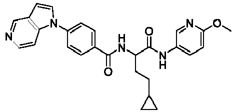 | ¹H-NMR (DMSO-D₆, 400MHz) δ: 0.01-0.07 (2H, m), 0.37-0.43 (2H, m), 0.69-0.76 (1H, m), 1.25-1.39 (2H, m), 1.89-1.98 (2H, m), 3.80 (3H, s), 4.61 (1H, td, J = 7.9, 5.5 Hz), 6.80 (1H, d, J = 8.6 Hz), 6.91 (1H, d, J = 3.1 Hz), 7.61 (1H, d, J = 6.1 Hz), 7.75 (2H, d, J = 8.6 Hz), 7.86 (1H, d, J = 3.1 Hz), 7.92 (1H, dd, J = 8.6, 2.4 Hz), 8.14 (2H, d, J = 8.6 Hz), 8.30 (1H, d, J = 6.1 Hz), 8.38 (1H, d, J = 2.4 Hz), 8.74 (1H, d, J = 7.9 Hz), 8.95 (1H, s), 10.14 (1H, s). |
| | | HRMS (ESI⁺):470.21867 [M+H]⁺ |
| 6-59 | 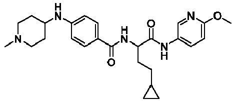 | ¹H-NMR (DMSO-D₆, 400MHz) δ: -0.05-0.06 (2H, m), 0.32-0.41 (2H, m), 0.62-0.75 (1H, m), 1.18-1.45 (4H, m), 1.80-1.90 (4H, m), 1.94-2.04 (2H, m), 2.14 (3H, s), 2.66-2.75 (2H, m), 3.16-3.27 (1H, m), 3.79 (3H, s), 4.51 (1H, q, J = 7.9 Hz), 6.02 (1H, d, J = 7.9 Hz), 6.55 (2H, d, J = 8.5 Hz), 6.77 (1H, d, J = 9.1 Hz), 7.66 (2H, d, J = 8.5 Hz), 7.89 (1H, dd, J = 9.1, 2.4 Hz), 8.02 (1H, d, J = 7.9 Hz), 8.35 (1H, d, J = 2.4 Hz), 10.03 (1H, s). |
| | | HRMS (ESI⁺): 466.28108 [M+H]⁺ |

**[Table 102]**

| Example | Structure | Equipment Data |
|---|---|---|
| 6-60 | 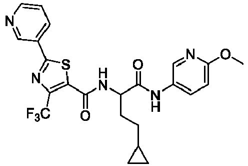 | ¹H-NMR (DMSO-D₆, 400MHz) δ: -0.04-0.04 (2H, m), 0.33-0.43 (2H, m), 0.63-0.74 (1H, m), 1.17-1.34 (2H, m), 1.70-1.93 (2H, m), 3.79 (3H, s), 4.50-4.59 (1H, m), 6.78 (1H, d, J = 8.5 Hz), 7.54-7.61 (1H, m), 7.89 (1H, dd, J = 8.5, 2.7 Hz), 8.31-8.39 (2H, m), 8.73 (1H, dd, J = 4.8, 1.8 Hz), 9.15 (1H, dd, J = 2.4, 1.2 Hz), 9.32 (1H, d, J = 7.9 Hz), 10.21 (1H, s). |
| | | HRMS (ESI⁺): 506.14689 [M+H]⁺ |
| 6-61 | 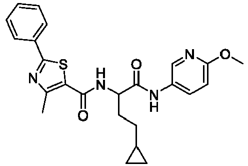 | ¹H-NMR (DMSO-D₆, 400MHz) δ: -0.02-0.08 (2H, m), 0.36-0.44 (2H, m), 0.65-0.77 (1H, m), 1.20-1.40 (2H, m), 1.80-1.96 (2H, m), 2.61 (3H, s), 3.81 (3H, s), 4.48-4.58 (1H, m), 6.80 (1H, d, J = 9.2 Hz), 7.49-7.55 (3H, m), 7.89-7.98 (3H, m), 8.36 (1H, d, J = 2.4 Hz), 8.49 (1H, d, J = 7.3 Hz), 10.16 (1H, s). |
| | | HRMS (ESI⁺): 451.17956 [M+H]⁺ |
| 6-62 | 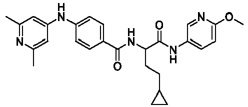 | ¹H-NMR (DMSO-D₆, 400MHz) δ: -0.04-0.08 (2H, m), 0.34-0.43 (2H, m), 0.64-0.77 (1H, m), 1.20-1.39 (2H, m), 1.84-1.95 (2H, m), 2.30 (6H, s), 3.80 (3H, s), 4.56 (1H, q, J = 7.3 Hz), 6.68 (2H, s), 6.79 (1H, d, J = 9.2 Hz), 7.20 (2H, d, J = 8.6 Hz), 7.87 (2H, d, J = 8.6 Hz), 7.91 (1H, dd, J = 9.2, 2.8 Hz), 8.35-8.42 (2H, m), 8.88 (1H, s), 10.09 (1H, s). |
| | | HRMS (ESI⁺): 474.25027 [M+H]⁺ |
| 6-63 | 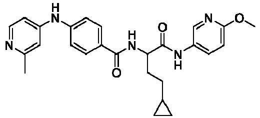 | ¹H-NMR (DMSO-D₆, 400MHz) δ: -0.03-0.07 (2H, m), 0.34-0.43 (2H, m), 0.65-0.76 (1H, m), 1.20-1.40 (2H, m), 1.85-1.95 (2H, m), 2.34 (3H, s), 3.80 (3H, s), 4.56 (1H, q, J = 7.3 Hz), 6.79 (1H, d, J = 9.2 Hz), 6.82 (1H, dd, J = 5.5, 2.4 Hz), 6.86 (1H, d, J = 2.4 Hz), 7.22 (2H, d, J = 9.2 Hz), 7.85-7.94 (3H, m), 8.12 (1H, d, J = 5.5 Hz), 8.37 (1H, d, J = 2.4 Hz), 8.40 (1H, d, J = 7.3 Hz), 8.98 (1H, s), 10.09 (1H, s). |
| | | HRMS (ESI⁺): 460.23528 [M+H]⁺ |

**[Table 103]**

| Example | Structure | Equipment Data |
|---|---|---|
| 6-64 | 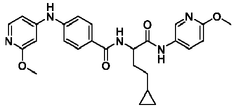 | ¹H-NMR (DMSO-D₆, 400MHz) δ: -0.04-0.08 (2H, m), 0.33-0.43 (2H, m), 0.65-0.77 (1H, m), 1.19-1.39 (2H, m), 1.84-1.95 (2H, m), 3.78 (3H, s), 3.80 (3H, s), 4.56 (1H, q, J = 7.9 Hz), 6.36 (1H, d, J = 1.8 Hz), 6.65 (1H, dd, J = 5.8, 1.8 Hz), 6.79 (1H, d, J = 9.2 Hz), 7.21 (2H, d, J = 8.6 Hz), 7.86-7.94 (4H, m), 8.37 (1H, d, J = 2.4 Hz), 8.41 (1H, d, J = 7.9 Hz), 9.05 (1H, s), 10.09 (1H, s). |
| | | HRMS (ESI⁺): 476.22955 [M+H]⁺ |
| 6-65 | 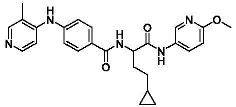 | ¹H-NMR (DMSO-D₆, 400MHz) δ: -0.03-0.08 (2H, m), 0.34-0.43 (2H, m), 0.65-0.77 (1H, m), 1.19-1.40 (2H, m), 1.84-1.95 (2H, m), 2.21 (3H, s), 3.80 (3H, s), 4.57 (1H, q, J = 7.3 Hz), 6.79 (1H, d, J = 9.2 Hz), 7.08 (1H, d, J = 5.5 Hz), 7.23 (2H, d, J = 8.6 Hz), 7.84-7.94 (3H, m), 8.09-8.14 (2H, m), 8.20 (1H, s), 8.37 (1H, d, J = 2.4 Hz), 8.41 (1H, d, J = 7.3 Hz), 10.10 (1H, s). |
| | | HRMS (ESI⁺): 460.23420 [M+H]⁺ |
| 6-66 | 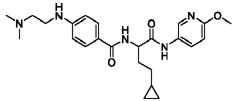 | ¹H-NMR (DMSO-D₆, 400MHz) δ: -0.04-0.07 (2H, m), 0.33-0.42 (2H, m), 0.64-0.75 (1H, m), 1.19-1.34 (2H, m), 1.81-1.92 (2H, m), 2.17 (6H, s), 2.42 (2H, t, J = 6.7 Hz), 3.09-3.18 (2H, m), 3.80 (3H, s), 4.52 (1H, q, J = 7.9 Hz), 5.98 (1H, t, J = 5.5 Hz), 6.57 (2H, d, J = 9.2 Hz), 6.78 (1H, d, J = 9.2 Hz), 7.68 (2H, d, J = 8.6 Hz), 7.90 (1H, dd, J = 9.2, 3.1 Hz), 8.06 (1H, d, J = 7.9 Hz), 8.36 (1H, d, J = 3.1 Hz), 10.05 (1H, s). |
| | | HRMS (ESI⁺): 440.26679 [M+H]⁺ |
| 6-67 | 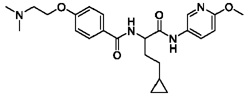 | ¹H-NMR (DMSO-D₆, 400MHz) δ: -0.04-0.08 (2H, m), 0.33-0.42 (2H, m), 0.64-0.76 (1H, m), 1.21-1.38 (2H, m), 1.83-1.94 (2H, m), 2.20 (6H, s), 2.62 (2H, t, J = 5.5 Hz), 3.80 (3H, s), 4.09 (2H, t, J = 5.5 Hz), 4.55 (1H, q, J = 7.3 Hz), 6.78 (1H, d, J = 8.6 Hz), 6.99 (2H, d, J = 9.2 Hz), 7.84-7.93 (3H, m), 8.36 (1H, d, J = 2.4 Hz), 8.40 (1H, d, J = 7.3 Hz), 10.09 (1H, s). |
| | | HRMS (ESI⁺): 441.25034 [M+H]⁺ |

**[Table 104]**

| Example | Structure | Equipment Data |
|---|---|---|
| 6 - 6 8 | 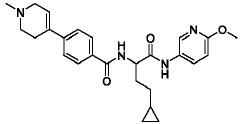 | ¹H-NMR (DMSO-D₆, 400MHz) δ: -0.05-0.08 (2H, m), 0.34-0.42 (2H, m), 0.65⁻0.77 (1H, m), 1.19-1.39 (2H, m), 1.84-1.96 (2H, m), 2.27 (3H, s), 2.47-2.51 (2H, m), 2.54-2.60 (2H, m), 2.99-3.05 (2H, m), 3.80 (3H, s), 4.57 (1H, q, J = 7.9 Hz), 6.25-6.31 (1H, m), 6.79 (1H, d, J = 8.6 Hz), 7.51 (2H, d, J = 8.6 Hz), 7.85-7.93 (3H, m), 8.36 (1H, d, J = 2.4 Hz), 8.54 (1H, d, J = 7.9 Hz), 10.11 (1H, s). |
| | | HRMS (ESI⁺): 449.25439 [M+H]⁺ |
| 6 - 6 9 | 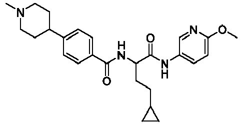 | ¹H-NMR (DMSO-D₆, 400MHz) δ: -0.04-0.06 (2H, m), 0.33-0.43 (2H, m), 0.64-0.77 (1H, m), 1.17-1.38 (2H, m), 1.59-1.76 (4H, m), 1.83-2.00 (4H, m), 2.18 (3H, s), 2.44-2.55 (1H, m), 2.79-2.91 (2H, m), 3.80 (3H, s), 4.50-4.62 (1H, m), 6.78 (1H, d, J = 9.2 Hz), 7.33 (2H, d, J = 7.9 Hz), 7.83 (2H, d, J = 7.9 Hz), 7.90 (1H, dd, J = 9.2, 2.4 Hz), 8.36 (1H, d, J = 2.4 Hz), 8.47 (1H, d, J = 7.9 Hz), 10.08 (1H, s). |
| | | HRMS (ESI⁺): 451.27058 [M+H]⁺ |
| 6 - 7 0 | 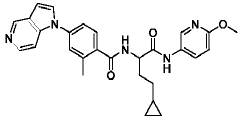 | ¹H-NMR (DMSO-D₆, 400MHz) δ: 0.01-0.09 (2H, m), 0.35-0.46 (2H, m), 0.66-0.78 (1H, m), 1.21-1.42 (2H, m), 1.77-1.96 (2H, m), 2.44 (3H, s), 3.81 (3H, s), 4.50-4.60 (1H, m), 6.81 (1H, d, J = 8.5 Hz), 6.88 (1H, d, J = 3.6 Hz), 7.48-7.60 (4H, m), 7.80 (1H, d, J = 3.6 Hz), 7.94 (1H, dd, J = 8.5, 2.4 Hz), 8.29 (1H, d, J = 5.4 Hz), 8.38 ( 1H, d, J = 2.4 Hz), 8.61 (1H, d, J = 7.3 Hz), 8.94 (1H, d, J = 1.2 Hz), 10.15 (1H, s). |
| | | HRMS (ESI⁺): 484.23549 [M+H]⁺ |
| 6 - 7 1 | 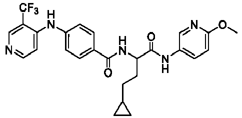 | ¹H-NMR (DMSO-D₆, 400MHz) δ: -0.04-0.10 (2H, m), 0.34-0.43 (2H, m), 0.65-0.78 (1H, m), 1.20-1.40 (2H, m), 1.84-1.97 (2H, m), 3.80 (3H, s), 4.53-4.63 (1H, m), 6.79 (1H, d, J = 9.1 Hz), 7.04 (1H, d, J = 6.1 Hz), 7.36 (2H, d, J = 8.5 Hz), 7.91 (1H, dd, J = 9.1, 3.0 Hz), 7.95 (2H, d, J = 8.5 Hz), 8.34-8.38 (2H, m), 8.48 (1H, s), 8.55 (1H, d, J = 7.3 Hz), 8.59 (1H, s), 10.13 (1H, s). |
| | | HRMS (ESI⁺): 514.20617 [M+H]⁺ |

**[Table 105]**

| Example | Structure | Equipment Data |
|---|---|---|
| 6 - 7 2 | 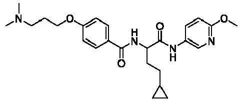 | ¹H-NMR (DMSO-D₆, 400MHz) δ: -0.04-0.07 (2H, m), 0.33-0.43 (2H, m), 0.64-0.76 (1H, m), 1.18-1.39 (2H, m), 1.79-1.94 (4H, m), 2.13 (6H, s), 2.34 (2H, t, J = 7.0 Hz), 3.80 (3H, s), 4.04 (2H, t, J = 6.4 Hz), 4.55 (1H, q, J = 7.9 Hz), 6.78 (1H, d, J = 9.2 Hz), 6.97 (2H, d, J = 9.2 Hz), 7.84-7.93 (3H, m), 8.36 (1H, d, J = 3.1 Hz), 8.40 (1H, d, J = 7.9 Hz), 10.09 (1H, s). |
| | | HRMS (ESI⁺): 455.26485 [M+H]⁺ |
| 6 - 7 3 | 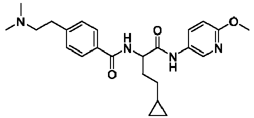 | ¹H-NMR (DMSO-D₆, 400MHz) δ: -0.04-0.08 (2H, m), 0.34-0.42 (2H, m), 0.65-0.76 (1H, m), 1.17-1.38 (2H, m), 1.82-1.96 (2H, m), 2.16 (6H, s), 2.41-2.47 (2H, m), 2.74 (2H, t, J = 7.3 Hz), 3.80 (3H, s), 4.56 (1H, q, J = 7.3 Hz), 6.78 (1H, d, J = 9.2 Hz), 7.30 (2H, d, J = 8.6 Hz), 7.81 (2H, d, J = 8.6 Hz), 7.90 (1H, dd, J = 9.2, 2.4 Hz), 8.36 (1H, d, J = 2.4 Hz), 8.47 (1H, d, J = 7.3 Hz), 10.09 (1H, s). |
| | | HRMS (ESI⁺): 425.25573 [M+H]⁺ |
| 6 - 7 4 | 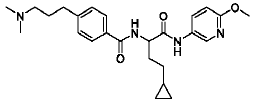 | ¹H-NMR (DMSO-D₆, 400MHz) δ: -0.05-0.07 (2H, m), 0.33-0.42 (2H, m), 0.64-0.77 (1H, m), 1.18-1.39 (2H, m), 1.64-1.75 (2H, m), 1.83-1.96 (2H, m), 2.12 (6H, s), 2.19 (2H, t, J = 7.0 Hz), 2.63 (2H, t, J = 7.6 Hz), 3.80 (3H, s), 4.56 (1H, q, J = 7.9 Hz), 6.78 (1H, d, J = 9.2 Hz), 7.28 (2H, d, J = 7.9 Hz), 7.82 (2H, d, J = 7.9 Hz), 7.90 (1H, dd, J = 9.2, 2.4 Hz), 8.36 (1H, d, J = 2.4 Hz), 8.48 (1H, d, J = 7.9 Hz), 10.09 (1H, s). |
| | | HRMS (ESI⁺): 439.27059 [M+H]⁺ |
| 6 - 7 5 | 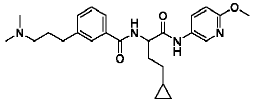 | ¹H-NMR (DMSO-D₆, 400MHz) δ: -0.03-0.07 (2H, m), 0.33-0.43 (2H, m), 0.65⁻0.77 (1H, m), 1.17-1.40 (2H, m), 1.65-1.76 (2H, m), 1.84-1.96 (2H, m), 2.11 (6H, s), 2.19 (2H, t, J = 7.0 Hz), 2.59-2.66 (2H, m), 3.80 (3H, s), 4.53-4.62 (1H, m), 6.79 (1H, d, J = 8.6 Hz), 7.33-7.39 (2H, m), 7.68-7.76 (2H, m), 7.91 (1H, dd, J = 8.6, 2.4 Hz), 8.37 (1H, d, J = 2.4 Hz), 8.53 (1H, d, J = 7.9 Hz), 10.12 (1H, s). |
| | | HRMS (ESI⁺): 439.27155 [M+H]⁺ |

**[Table 106]**

| Example | Structure | Equipment Data |
|---|---|---|
| 6 - 7 6 | 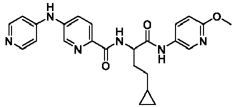 | ¹H-NMR (DMSO-D₆, 400MHz) δ: -0.01-0.03 (2H, m), 0.35-0.41 (2H, m), 0.68-0.76 (1H, m), 1.21-1.29 (2H, m), 1.90-1.99 (2H, m), 3.81 (3H, s), 4.69 (1H, td, J = 7.9, 4.9 Hz), 6.81 (1H, d, J = 8.6 Hz), 7.07 (2H, d, J = 4.9 Hz), 7.80 (1H, dd, J = 8.6, 1.8 Hz), 7.90 (1H, dd, J = 8.6, 1.8 Hz), 7.98 (1H, d, J = 8.6 Hz), 8.31 (2H, d, J = 4.9 Hz), 8.37 (1H, d, J = 1.8 Hz), 8.49-8.53 (2H, m), 9.38 (1H, s), 10.23 (1H, s). |
| | | HRMS (ESP⁺) : 447.21375 [M+H]⁺ |
| 6 - 7 7 | 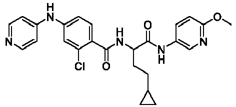 | ¹H-NMR (DMSO-D₆, 400MHz) δ: -0.02-0.01 (2H, m), 0.31-0.39 (2H, m), 0.63-0.71 (1H, m), 1.21-1.32 (2H, m), 1.72-1.87 (2H, m), 3.77 (3H, s), 4.50 (1H, td, J = 8.6, 5.5 Hz), 6.76 (1H, d, J = 8.6 Hz), 6.94 (2H, d, J = 6.1 Hz), 7.15-7.18 (2H, m), 7.40 (1H, d, J = 8.6 Hz), 7.88 (1H, dd, J = 8.6, 2.4 Hz), 8.23 (2H, d, J = 6.1 Hz), 8.33 (1H, d, J = 2.4 Hz), 8.51 (1H, d, J = 7.9 Hz), 9.08 (1H, s), 10.07 (1H, s). |
| | | HRMS (ESI⁺) : 480.17986 [M+H]⁺ |
| 6 - 7 8 | 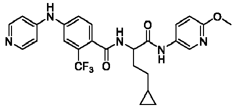 | ¹H-NMR (DMSO-D₆, 400MHz) δ: -0.03-0.02 (2H, m), 0.33-0.39 (2H, m), 0.64-0.71 (1H, m), 1.19-1.33 (2H, m), 1.73-1.86 (2H, m), 3.78 (3H, s), 4.50 (1H, td, J = 8.6, 5.5 Hz), 6.78 (1H, d, J = 8.6 Hz), 6.96 (2H, d, J = 6.1 Hz), 7.41 (1H, d, J = 1.8 Hz), 7.47-7.53 (2H, m), 7.89 (1H, dd, J = 8.6, 2.4 Hz), 8.26 (2H, d, J = 6.1 Hz), 8.34 (1H, d, J = 2.4 Hz), 8.64 (1H, d, J = 7.9 Hz), 9.22 (1H, s), 10.08 (1H, s). |
| | | HRMS (ESI⁺) : 514.20620 [M+H]⁺ |
| 6 - 7 9 | 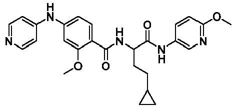 | ¹H-NMR (DMSO-D₆, 400MHz) δ: -0.02-0.02 (2H, m), 0.35-0.40 (2H, m), 0.66-0.75 (1H, m), 1.21-1.29 (2H, m), 1.78-1.96 (2H, m), 3.80 (3H, s), 3.94 (3H, s), 4.67 (1H, td, J = 7.9, 5.5 Hz), 6.80 (1H, d, J = 8.6 Hz), 6.86-6.90 (2H, m), 7.05 (2H, d, J = 6.1 Hz), 7.85 (1H, d, J = 8.6 Hz), 7.90 (1H, dd, J = 8.6, 2.4 Hz), 8.26 (2H, d, J = 6.1 Hz), 8.32 (1H, d, J = 7.9 Hz), 8.35 (1H, d, J = 2.4 Hz), 9.16 (1H, s), 10.18 (1H, s). |
| | | HRMS (ESI⁺) : 476.22968 [M+H]⁺ |

**[Table 107]**

| Example | Structure | Equipment Data |
|---|---|---|
| 6 - 8 0 | 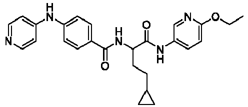 | ¹H-NMR (DMSO-D₆, 400MHz) δ: -0.02-0.08 (2H, m), 0.34-0.43 (2H, m), 0.64-0.77 (1H, m), 1.21-1.35 (5H, m), 1.84-1.95 (2H, m), 4.24 (2H, q, J = 7.1 Hz), 4.56 (1H, q, J = 7.9 Hz), 6.76 (1H, d, J = 9.2 Hz), 7.00 (2H, dd, J = 4.9, 1.8 Hz), 7.23 (2H, d, J = 8.6 Hz), 7.86-7.93 (3H, m), 8.25 (2H, dd, J = 4.9, 1.8 Hz), 8.35 (1H, d, J = 2.4 Hz), 8.42 (1H, d, J = 7.9 Hz), 9.09 (1H, s), 10.09 (1H, s). |
| | | HRMS (ESI⁺): 460.23503 [M+H]⁺ |
| 6 - 8 1 | 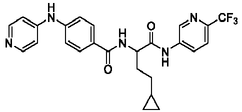 | ¹H-NMR (DMSO-D₆, 400MHz) δ: -0.01-0.09 (2H, m), 0.35-0.44 (2H, m), 0.67-0.77 (1H, m), 1.21-1.43 (2H, m), 1.87-1.97 (2H, m), 4.59 (1H, q, J = 7.3 Hz), 7.00 (2H, dd, J = 4.9, 1.2 Hz), 7.24 (2H, d, J = 8.6 Hz), 7.86 (1H, d, J = 8.6 Hz), 7.90 (2H, d, J = 8.6 Hz), 8.25 (2H, dd, J = 4.9, 1.2 Hz), 8.34 (1H, dd, J = 8.6, 2.4 Hz), 8.53 (1H, d, J = 7.3 Hz), 8.91 (1H, d, J = 2.4 Hz), 9.10 (1H, s), 10.73 (1H, s). |
| | | HRMS (ESI⁺): 484.19585 [M+H]⁺ |
| 6 - 8 2 | 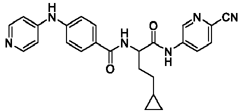 | ¹H-NMR (DMSO-D₆, 400MHz) δ: -0.03-0.10 (2H, m), 0.34-0.44 (2H, m), 0.66-0.77 (1H, m), 1.21-1.41 (2H, m), 1.92 (2H, q, J = 7.7 Hz), 4.57 (1H, q, J = 7.3 Hz), 7.00 (2H, dd, J = 4.9, 1.8 Hz), 7.24 (2H, d, J = 8.6 Hz), 7.89 (2H, d, J = 8.6 Hz), 7.98 (1H, d, J = 8.6 Hz), 8.25 (2H, dd, J = 4.9, 1.8 Hz), 8.29 (1H, dd, J = 8.6, 2.4 Hz), 8.54 (1H, d, J = 7.3 Hz), 8.90 (1H, d, J = 1.8 Hz), 9.10 (1H, s), 10.80 (1H, s). |
| | | HRMS (ESI⁺): 441.20411 [M+H]⁺ |
| 6 - 8 3 | 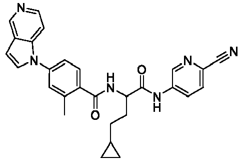 | ¹H-NMR (DMSO-D₆, 400MHz) δ: 0.01-0.10 (2H, m), 0.35-0.46 (2H, m), 0.66-0.78 (1H, m), 1.29-1.45 (2H, m), 1.78-1.99 (2H, m), 2.44 (3H, s), 4.53-4.63 (1H, m), 6.92 (1H, d, J = 3.6 Hz), 7.50-7.63 (4H, m), 7.84 (1H, d, J = 3.0 Hz), 8.00 (1H, d, J = 8.5 Hz), 8.29-8.35 (2H, m), 8.75 (1H, d, J = 7.3 Hz), 8.91 (1H, d, J = 3.0 Hz), 8.99 (1H, d, J = 1.2 Hz), 10.86 (1H, s). |
| | | HRMS (ESI⁺): 479.21974 [M+H]⁺ |

**[Table 108]**

| Example | Structure | Equipment Data |
|---|---|---|
| 6 - 8 4 | 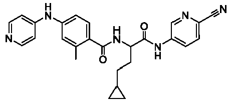 | ¹H-NMR (DMSO-D₆, 400MHz) δ: -0.03-0.10 (2H, m), 0.35-0.44 (2H, m), 0.63-0.76 (1H, m), 1.21-1.42 (2H, m), 1.75-1.96 (2H, m), 2.33 (3H, s), 4.48-4.58 (1H, m), 6.96 (2H, dd, J = 4.8, 1.8 Hz), 7.02-7.10 (2H, m), 7.39 (1H, d, J = 7.9 Hz), 7.99 (1H, d, J = 8.5 Hz), 8.22 (2H, dd, J = 4.8, 1.8 Hz), 8.31 (1H, dd, J = 8.5, 2.4 Hz), 8.49 (1H, d, J = 7.3 Hz), 8.90 (1H, d, J = 2.4 Hz), 9.08 (1H, s), 10.81 (1H, s). |
| | | HRMS (ESI⁺): 455.21971 [M+H]⁺ |
| 6 - 8 5 | 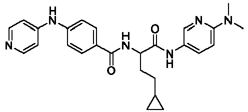 | ¹H-NMR (DMSO-D₆, 400MHz) δ: -0.04-0.08 (2H, m), 0.33-0.44 (2H, m), 0.65⁻0.77 (1H, m), 1.18-1.38 (2H, m), 1.82-1.95 (2H, m), 2.97 (6H, s), 4.51-4.60 (1H, m), 6.61 (1H, d, J = 9.2 Hz), 7.00 (2H, dd, J = 4.9, 1.5 Hz), 7.23 (2H, d, J = 9.2 Hz), 7.73 (1H, dd, J = 9.2, 3.1 Hz), 7.89 (2H, d, J = 9.2 Hz), 8.22-8.27 (3H, m), 8.37 (1H, d, J = 7.9 Hz), 9.09 (1H, s), 9.83 (1H, s). |
| | | HRMS (ESI⁺): 459.25028 [M+H]⁺ |
| 6 - 8 6 | 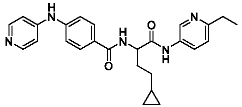 | ¹H-NMR (DMSO-D₆, 400MHz) δ: -0.02-0.08 (2H, m), 0.34-0.43 (2H, m), 0.65-0.77 (1H, m), 1.18 (3H, t, J = 7.9 Hz), 1.21-1.40 (2H, m), 1.86-1.95 (2H, m), 2.68 (2H, q, J = 7.9 Hz), 4.57 (1H, q, J = 7.9 Hz), 7.00 (2H, dd, J = 4.9, 1.2 Hz), 7.20 (1H, d, J = 8.6 Hz), 7.23 (2H, d, J = 8.6 Hz), 7.89 (2H, d, J = 8.6 Hz), 7.95 (1H, dd, J = 8.6, 2.4 Hz), 8.25 (2H, dd, J = 4.9, 1.2 Hz), 8.44 (1H, d, J = 7.9 Hz), 8.65 (1H, d, J = 2.4 Hz), 9.09 (1H, s), 10.20 (1H, s). |
| | | HRMS (ESI⁺): 444.23982 [M+H]⁺ |
| 6 - 8 7 | 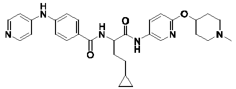 | ¹H-NMR (DMSO-D₆, 400MHz) δ: -0.05-0.09 (2H, m), 0.34-0.43 (2H, m), 0.65-0.77 (1H, m), 1.20-1.38 (2H, m), 1.55-1.68 (2H, m), 1.84-1.96 (4H, m), 2.07-2.18 (5H, m), 2.56-2.65 (2H, m), 4.56 (1H, q, J = 7.5 Hz), 4.84-4.94 (1H, m), 6.74 (1H, d, J = 9.1 Hz), 7.00 (2H, dd, J = 4.8, 1.8 Hz), 7.23 (2H, d, J = 9.1Hz), 7.87-7.92 (3H, m), 8.25 (2H, dd, J = 4.8, 1.8 Hz), 8.33 (1H, d, J = 2.4 Hz), 8.43 (1H, d, J = 7.3 Hz), 9.09 (1H, s), 10.09 (1H, s). |
| | | HRMS (ESI⁺): 529.29328 [M+H]⁺ |

**[Table 109]**

| Example | Structure | Equipment Data |
|---|---|---|
| 6 - 8 8 | 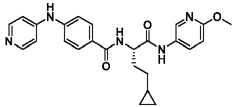 | ¹H-NMR (DMSO-D₆, 400MHz) δ: -0.03-0.04 (2H, m), 0.33-0.38 (2H, m), 0.63-0.73 (1H, m), 1.20-1.34 (2H, m), 1.83-1.91 (2H, m), 3.78 (3H, s), 4.50-4.57 (1H, m), 6.76 (1H, d, J = 8.6 Hz), 6.98 (2H, d, J = 6.1 Hz), 7.21 (2H, d, J = 8.6 Hz), 7.85-7.90 (3H, m), 8.22 (2H, d, J = 6.1 Hz), 8.34 (1H, d, J = 2.4 Hz), 8.40 (1H, d, J = 7.9 Hz), 9.07 (1H, s), 10.08 (1H, s). |
| | | HRMS (ESI⁺):446.21990 [M+H]⁺ |
| 6 - 8 9 | 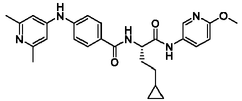 | ¹H-NMR (DMSO-D₆, 400MHz) δ: -0.03-0.08 (2H, m), 0.34-0.43 (2H, m), 0.65-0.77 (1H, m), 1.20-1.39 (2H, m), 1.84-1.95 (2H, m), 2.30 (6H, s), 3.80 (3H, s), 4.56 (1H, q, J = 7.5 Hz), 6.68 (2H, s), 6.79 (1H, d, J = 9.2 Hz), 7.20 (2H, d, J = 8.6 Hz), 7.87 (2H, d, J = 9.2 Hz), 7.91 (1H, dd, J = 8.6, 2.8 Hz), 8.35-8.42 (2H, m), 8.88 (1H, s), 10.09 (1H, s). |
| | | HRMS (ESI⁺): 474.25063 [M+H]⁺ |
| 6 - 9 0 | 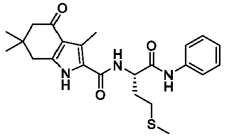 | ¹H-NMR (270 MHz, CDCl₃) δ: 1.10 (6H, s), 2.15 (3H, s), 2.15-2.34 (2H, m), 2.60-2.80 (2H, m), 2.35 (2H, s), 2.65 (2H, s), 2.68 (3H, s), 4.91 (1H, q, J = 7.6 Hz), 6.72 (1H, d, J = 7.6 Hz), 7.13 (1H, dd, J = 7.7, 7.7 Hz), 7.33 (2H, dd, J = 7.7, 8.1 Hz), 7.53 (2H, d, J = 8.1 Hz), 8.47 (1H, brs), 9.30 (1H, brs). |
| | | MS (ESI⁺): 428.47[M+H]⁺ |
| 6 - 9 1 | 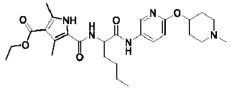 | MS (ESI⁺): 514.31[M+H]⁺ |
| 6 - 9 2 | 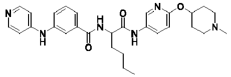 | MS (ESI⁺): 517.42[M+H]⁺ |
| 6 - 9 3 | 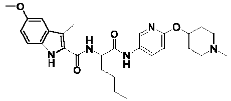 | MS (ESI⁺): 508.34[M+H]⁺ |

**[Table 110]**

| Example | Structure | Equipment Data |
|---|---|---|
| 6 - 9 4 | 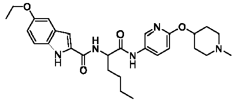 | MS (ESI⁺): 508.4[M+H]⁺ |
| 6 - 9 5 | 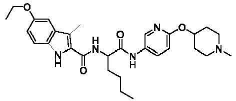 | MS (ESI⁺): 522.44[M+H]⁺ |
| 6 - 9 6 | 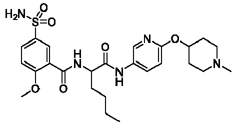 | MS (ESI⁺): 534.32[M+H]⁺ |
| 6 - 9 7 | 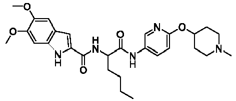 | MS (ESI⁺): 524.34[M+H]⁺ |
| 6 - 9 8 | 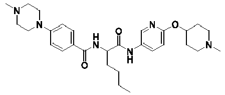 | MS (ESI⁺): 523.0[M+H]⁺ |
| 6 - 9 9 | 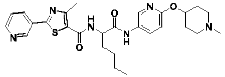 | MS (ESI⁺): 523.0[M+H]⁺ |
| 6 - 1 0 0 | 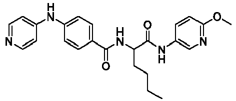 | ¹H-NMR (DMSO-D₆, 400MHz) δ: 0.87 (3H, t, J = 7.0 Hz), 1.25-1.48 (4H, m), 1.74-1.86 (2H, m), 3.80 (3H, s), 4.53 (1H, q, J = 7.9 Hz), 6.79 (1H, d, J = 8.5 Hz), 7.00 (2H, dd, J = 4.8, 1.2 Hz), 7.23 (2H, d, J = 8.5 Hz), 7.87-7.94 (3H, m), 8.25 (2H, dd, J = 4.8, 1.2 Hz), 8.37 (1H, d, J = 2.4 Hz), 8.43 (1H, d, J = 7.9 Hz), 9.09 (1H, s), 10.11 (1H, s). |
| | | HRMS (ESI⁺): 434.22008 [M+H]⁺ |

**[Table 111]**

| Example | Structure | Equipment Data |
|---|---|---|
| 6 - 1 0 1 | 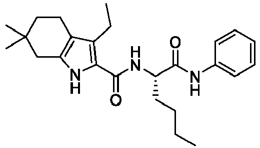 | ¹H-NMR (270 MHz, CDCl₃) δ: 0.91 (3H, t, J = 7.2 Hz), 0.98 (6H, s), 1.22 (3H, t, J = 7.6 Hz), 1.25-1.50 (4H, m), 1.52 (2H, t, J= 6.5Hz), 1.70-1.95 (1H, m), 2.00 - 2.20 (1H, m), 2.31 (2H, s), 2.41 (2H, t, J = 6.2 Hz), 2.66 (2H, q, J = 7.7 Hz), 4.73 (1H, dt, J = 5.7, 7.8 Hz), 6.29 (1H, d, J = 7.6Hz), 7.08 (1H, t, J = 7.4 Hz), 7.29 (2H, dd, J = 7.4, 7.6 Hz), 7.51 (2H, d, J = 7.6 Hz), 870 (1H, brs), 8.75 (1H, brs). |
| | | MS (ESI+): 410.52 [M+H]⁺ |

**[Table 112]**

| **Example** | | **¹HNMR Data** | **MS Data** [M+1]⁺ |
|---|---|---|---|
| 6-102 | 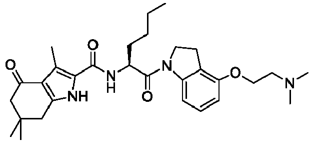 | ¹H-NMR (DMSO-D₆, 400 MHz) *δ*: 0.88 (3H, t, J = 6.9 Hz), 1.02 (6H, s), 1.24-1.50 (4H, m), 1.62-1.76 (1H, m), 1.76-1.88 (1H, m), 2.22 (8H, m), 2.46 (3H, s), 2.60-2.66 (4H, m), 3.06 (2H, t, J = 8.7 Hz), 4.09 (2H, t, J = 5.7 Hz), 4.20 (1H, dt, J = 8.7, 8.7 Hz), 4.34 (1H, dt, J = 8.7, 8.7 Hz), 4.66-4.78 (1H, m), 6.71 (1H, d, J = 8.2 Hz), 7.12 (1H, dd, J = 8.2, 8.2 Hz), 7.70 (1H, d, J = 8.2 Hz), 7.77 (1H, d, J = 7.8 Hz), 11.65 (1H, s). | 523.7 |
| 6-103 | 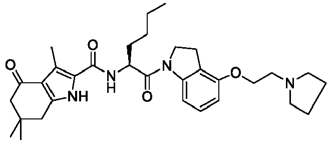 | ¹H-NMR (DMSO-D₆, 400 MHz) *δ* : 0.88 (3H, t, J = 6.9 Hz), 1.02 (6H, s), 1.21-1.47 (4H, m), 1.58-1.75 (5H, m), 1.75-1.88 (1H, m), 2.22 (2H, s), 2.46 (3H, s), 2.48-2.58 (4H, m), 2.64 (2H, s), 2.79 (2H, t, J = 5.7 Hz), 3.06 (2H, t, J = 8.7 Hz), 4.10 (2H, t, J = 5.7 Hz), 4.20 (1H, dt, J = 8.7, 8.7 Hz), 4.34 (1H, dt, J = 8.7, 8.7 Hz), 4.66-4.78 (1H, m), 6.70 (1H, d, J = 8.2 Hz), 7.12 (1H, dd, J = 8.2, 8.2 Hz), 7.70 (1H, d, J = 8.2 Hz), 7.77 (1H, d, J = 7.3 Hz), 11.66 (1H, s). | 550.1 |
| 6-104 | 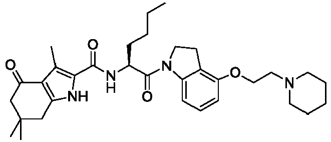 | ¹H-NMR (DMSO-D₆, 400 MHz) *δ* : 0.88 (3H, t, J = 6.9 Hz), 1.02 (6H, m), 1.26-1.45 (6H, m), 1.45-1.53 (4H, m), 1.62-176 (1H, m), 1.76-1.88 (1H, m), 2.22 (2H, s), 2.40-2.48 (7H, m), 2.62-2.69 (4H, m), 3.05 (2H, t, J = 8.7 Hz), 4.10 (2H, t, J = 5.7 Hz), 4.20 (1H, dt, J = 8.7, 8.7 Hz), 4.33 (1H, dt, J = 8.7, 8.7 Hz), 4.67-4.76 (1H, m), 6.71 (1H, d, J = 8.2 Hz), 7.12 (1H, dd, J = 8.2, 8.2 Hz), 7.70 (1H, d, J = 8.2 Hz), 7.77 (1H, d, J = 7.8 Hz), 11.65 (1H, s ). | 563.3 |
| 6-105 | 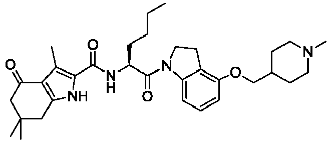 | ¹H-NMR (DMSO-D₆, 400 MHz) *δ* : 0.88 (3H, t, J = 6.9 Hz), 1.02 (6H, s), 1.21-1.48 (6H, m), 1.62-1.77 (4H, m), 1.77-1.92 (3H, m), 2.16 (3H, s), 2.22 (2H, s), 2.46 (3H, s), 2.64 (2H, s), 2.75-2.82 (2H, m), 3.06 (2H, t, J = 8.6 Hz), 3.86 (2H, d, J = 5.5 Hz), 4.20 (1H, dt, J = 8.6, 8.6 Hz), 4.34 (1H, dt, J = 8.6, 8.6 Hz), 4.66-4.77 (1H, m), 6.68 (1H, d, J = 8.0 Hz), 7.11 (1H, dd, J = 8.0, 8.0 Hz), 7.69 (1H, d, J = 8.0 Hz), 7.77 (1H, d, J = 7.3 Hz), 11.67 (1H, s). | 563.3 |
| 6-106 | 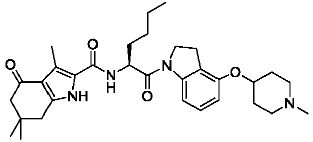 | ¹H-NMR (DMSO-D₆, 400 MHz) δ: 0.88 (3H, t, J = 6.9 Hz), 1.02 (6H, s), 1.22-1.51 (4H, m), 1.58-1.75 (3H, m), 1.75-1.96 (3H, m), 2.14-2.25 (7H, m), 2.46 (3H, s), 2.50-2.59 (2H, m), 2.64 (2H, s), 3.07 (2H, t, J = 8.6 Hz), 4.20 (1H, dt, J = 8.6, 8.6 Hz), 4.27-4.46 (2H, m,), 4.67-4.77 (1H, m), 6.73 (1H, d, J = 8.2 Hz), 7.10 (1H, dd, J = 8.2, 8.2 Hz), 7.69 (1H, d, J = 8.2 Hz), 7.76 (1H, d, J = 7.8 Hz), 11.64 (1H, s). | 549.6 |
| 6-107 | 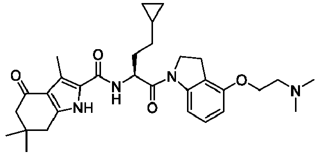 | ¹H-NMR (DMSO-D₆, 400 MHz) δ: -0.02-0.09 (2H, m), 0.34-0.46 (2H, m), 0.67-0.79 (1H, m), 1.02 (6H, s), 1.32 (2H, dt, J = 7.3, 7.3 Hz), 1.72-1.85 (1H, m), 1.85-1.97 (1H, m), 2.20-2.25 (8H, m), 2.46 (3H, s), 2.59-2.69 (4H, m), 3.06 (2H, t, J = 8.7 Hz), 4.09 (2H, t, J = 5.8 Hz), 4.22 (1H, dt, J = 8.7, 8.7 Hz), 4.33 (1H, dt, J = 8.7, 8.7 Hz), 4.74-4.83 (1H, m), 6.71 (1H, d, J = 8.1 Hz), 7.12 (1H, dd, J = 8.1, 8.1 Hz), 7.70 (1H, d, J = 8.1 Hz), 7.78 (1H, d, J = 7.8 Hz), 11.64 (1H, s). | 535 |

### <Example 7>

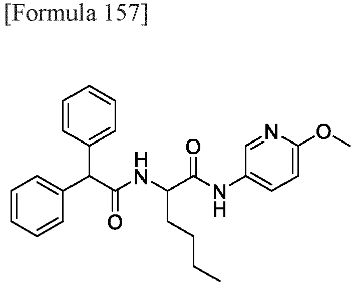

Under argon atmosphere, trifluoroacetic acid (0.458 mL) was added to a solution of tert-butyl N-[1-[(6-methoxypyridin-3-yl)amino]-1-oxohexan-2-yl]carbamate (61.8 mg) in dichloromethane (0.458 mL), the mixture was stirred at room temperature for 30 minutes, and then the reaction mixture was concentrated under reduced pressure. 2,2-diphenylacetyl chloride (46.4 mg) and N,N-diisopropylethylamine (0.125 mL) were added to the obtained crude product in dichloromethane (0.916 mL), and the mixture was stirred at room temperature for 24 hours. After water was added, the mixture was extracted with ethyl acetate. The organic layer was washed with saturated brine, dried over anhydrous sodium sulfate, and concentrated under reduced pressure after filtration of the insoluble material. The residue was purified by silica gel column chromatography (hexane : ethyl acetate = 2 : 1), and the resulting solid was washed with diisopropyl ether to obtain 2-[(2,2-diphenylacetyl)amino]-N-(6-methoxypyridin-3-yl)hexaneamide (64.7 mg).

¹H-NMR (DMSO-D₆, 400MHz) δ: 0.79 (3H, t, J = 6.7 Hz), 1.17-1.28 (4H, m), 1.54-1.73 (2H, m), 3.79 (3H, s), 4.41 (1H, td, J = 7.9, 5.4 Hz), 5.13 (1H, s), 6.78 (1H, d, J = 9.1 Hz), 7.17-7.30 (10H, m), 7.86 (1H, dd, J = 9.1, 2.4 Hz), 8.33 (1H, d, J = 2.4 Hz), 8.58 (1H, d, J = 7.9 Hz), 10.12 (1H, s).

HRMS (ESI⁺): 43.222900 [M+H]⁺

### <Example 8>

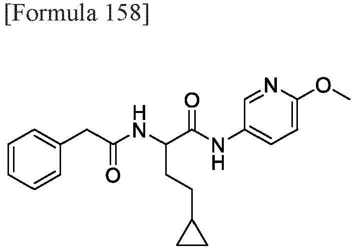

Under argon atmosphere, trifluoroacetic acid (0.363 mL) was added to a solution of tert-butyl N-[4-cyclopropyl-1-[(6-methoxypyridin-3-yl)amino]-1-oxobutan-2-yl]carbamate (50.8 mg) in dichloromethane (0.363 mL), and the mixture was stirred at room temperature for 30 minutes, and then the reaction mixture was concentrated under reduced pressure. 2-phenylacetyl chloride (0.021 mL) and N,N-diisopropylethylamine (0.099 mL) were added to the obtained crude product in dichloromethane (0.727 mL), and the mixture was stirred at room temperature for 3 hours. After water was added, the mixture was extracted with ethyl acetate. The organic layer was washed with saturated brine, dried over anhydrous sodium sulfate, and concentrated under reduced pressure after filtration of the insoluble material. the residue was purified by silica gel column chromatography (hexane : ethyl acetate = 3 : 2), the resulting solid was washed with diisopropyl ether to obtain 4-cyclopropyl-N-(6-methoxypyridin-3-yl)-2-[(2-phenylacetyl)amino]butaneamide (32.3 mg).

¹H-NMR (DMSO-D₆, 400MHz) δ: -0.11-0.02 (2H, m), 0.34 (2H, d, J = 7.9 Hz), 0.59-0.68 (1H, m), 1.12-1.22 (2H, m), 1.63-1.82 (2H, m), 3.44 (1H, d, J = 13.9 Hz), 3.51 (1H, d, J = 13.9 Hz), 3.79 (3H, s), 4.37 (1H, td, J = 7.9, 5.4 Hz), 6.78 (1H, d, J = 8.5 Hz), 7.15-7.29 (5H, m), 7.87 (1H, dd, J = 8.5, 2.4 Hz), 8.31-8.38 (2H, m) , 10.07 (1H, s).

HRMS (ESI⁺): 368.19644 [M+H]⁺

### <Example 9-1>

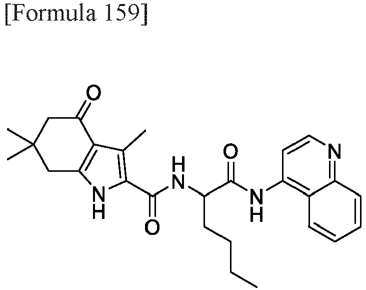

In argon atmosphere, 10% palladium carbon (15.3 mg) was added to a solution of benzyl N-[1-oxo-1-(quinolin-4-ylamino)hexan-2-yl]carbamate (76.7 mg) in ethanol (0.50 mL) and tetrahydrofuran (0.50 mL) at room temperature, and the mixture was stirred under a hydrogen atmosphere for 5 hours. After replacing with a argon atmosphere, the reaction mixture was filtered through Celite, and the filtrate was concentrated under reduced pressure. After dissolving the residue in N,N-dimethylformamide (1.00 mL), 3,6,6-trimethyl-4-oxo-5,7-dihydro-1H-indole-2-carboxylic acid (47.5 mg), 1-(3-dimethylaminopropyl)-3-ethylcarbodiimide hydrochloride (47.0 mg), 1-hydroxybenzotriazole (46.4 mg) and N,N-diisopropylethylamine (71.0 µL) were added at room temperature, and the mixture was stirred for 4 hours. The reaction mixture was added to water, and extracted twice with ethyl acetate. The combined organic layer was washed twice with saturated brine, dried over anhydrous sodium sulfate, and concentrated under reduced pressure. The residue was purified by silica gel column chromatography (ethyl acetate : methanol = 99 : 1 to 11 : 1) to obtain 3,6,6-trimethyl-4-oxo-N-[1-oxo-1-(quinolin-4-ylamino)hexan-2-yl]-5,7-dihydro-1H-indole-2-carboxamide (78.0 mg).

¹H-NMR (DMSO-D₆, 400MHz) δ: 0.89 (3H, t, J = 7.3 Hz), 1.02 (6H, s), 1.27-1.49 (4H, m), 1.73-1.95 (2H, m), 2.22 (2H, s), 2.50 (3H, s), 2.64 (2H, s), 4.80-4.94 (1H, m), 7.65 (1H, t, J = 7.9 Hz), 7.73-7.80 (2H, m), 8.00 (1H, d, J = 7.6 Hz), 8.04 (1H, d, J = 5.2 Hz), 8.34 (1H, d, J = 7.6 Hz), 8.80 (1H, d, J = 5.2 Hz), 10.44 (1H, s), 11.70 (1H, s).

HRMS (ESI⁺): 461.25486 [M+H]⁺

Using the corresponding starting material and reactant, the following Example 9-2 can be prepared by the same method in Example 9-1, the method described in Step 3-2 or Step 3-3, or a method similar thereto.

**[Table 113]**

| Example | Structure | Equipment Data |
|---|---|---|
| 9 - 2 | 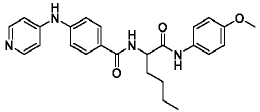 | ¹H-NMR (DMSO-D₆, 400MHz) δ: 0.87 (3H, t, J = 7.0 Hz), 1.26-1.48 (4H, m), 1.74-1.84 (2H, m), 3.70 (3H, s), 4.52 (1H, q, J = 7.5 Hz), 6.87 (2H, d, J = 9.1 Hz), 7.00 (2H, dd, J = 4.8, 1.2 Hz), 7.23 (2H, d, J = 9.1 Hz), 7.52 (2H, d, J = 9.1 Hz), 7.90 (2H, d, J = 9.1 Hz), 8.25 (2H, dd, J = 4.8, 1.2 Hz), 8.37 (1H, d, J = 7.9 Hz), 9.09 (1H, s), 9.93 (1H, s). |
| | | HRMS (ESI⁺): 433.22414 [M+H]⁺ |

### <Example 10>

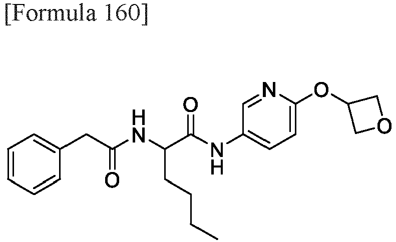

Under argon atmosphere, 10% palladium carbon (20.0 mg) was added to a solution of benzyl N-[1-[[6-(oxetan-3-yloxy)pyridin-3-yl]amino]-1-oxohexan-2-yl]carbamate (100 mg) in ethanol (1.50 mL) at room temperature, and the mixture was stirred under a hydrogen atmosphere for 2 hours, After replacing with a argon atmosphere, the reaction mixture was filtered through Celite, and the filtrate was concentrated under reduced pressure. Diisopropylamine (61.7 µL) and 2-phenylacetyl chloride (32.0 µL) were added to a solution of the residue in dichloromethane (2.50 mL) at 0 °C, the mixture was stirred for 1.5 hours. The reaction mixture was added to water, and extracted twice with ethyl acetate. The combined organic layer was washed with water and saturated brine, dried over anhydrous sodium sulfate, and concentrated under reduced pressure after filtration of the insoluble material. The residue was purified by silica gel column chromatography (hexane : ethyl acetate = 5 : 1 to 0 : 1) to obtain N-[6-(oxetan-3-yloxy)pyridin-3-yl]-2-[(2-phenylacetyl)amino]hexaneamide (80.0 mg).

¹H-NMR (DMSO-D₆, 400MHz) δ: 0.82 (3H, t, J = 6.7 Hz), 1.14-1.35 (4H, m), 1.50-1.76 (2H, m), 3.45 (1H, d, J = 13.9 Hz), 3.51 (1H, d, J = 13.9 Hz), 4.30-4.41 (1H, m), 4.52 (2H, dd, J = 7.6, 5.1 Hz), 4.81-4.90 (2H, m), 5.46-5.52 (1H, m), 6.87 (1H, d, J = 8.5 Hz), 7.16-7.30 (5H, m), 7.92 (1H, dd, J = 8.5, 2.4 Hz), 8.29 (1H, d, J = 2.4 Hz), 8.37 (1H, d, J = 7.9 Hz), 10.11 (1H, s).

HRMS (ESI⁺): 398.20803 [M+H]⁺

### <Reference example 72-1>

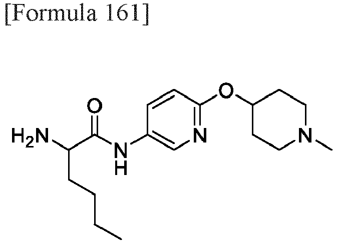

Using Benzyl N-[1-[[6-(1-methylpiperidin-4-yl)oxypyridin-3-yl]amino]-1-oxohexan-2-yl]carbamate, the title compound was synthesized by the same method in Reference Example 5.

¹H-NMR (DMSO-D₆, 400MHz) δ: 0.85 (3H, t, J = 7.0 Hz), 1.20-1.48 (5H, m), 1.54-1.68 (3H, m), 1.87-1.96 (2H, m) , 2.06-2.18 (5H, m), 2.55-2.66 (2H, m), 3.25 (1H, dd, J = 7.9, 5.4 Hz), 4.83-4.94 (1H, m), 6.72 (1H, d, J = 9.1 Hz), 7.92 (1H, dd, J = 9.1, 2.4 Hz), 8.34 (1H, d, J = 2.4 Hz).

MS (ESI⁺): 321.2 [M+H]⁺

Using the corresponding starting materials and reactants, the following Reference Example 72-2 was obtained by the same method in Reference Example 72-1, the method described in Step 3-2, or a method similar thereto.

**[Table 114]**

| Reference Example | Structure | Equipment Data |
|---|---|---|
| 7 2 - 2 | 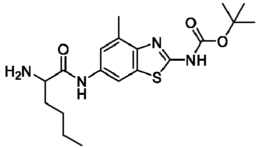 | ¹H-NMR (DMSO-D₆, 400MHz) δ: 0.86 (3H, t, J = 6.7 Hz), 1.22-1.35 (5H, m), 1.49 (9H, s), 1.57-1.70 (1H, m), 2.49 (3H, s), 3.27 (1H, dd, J = 7.3, 5.4 Hz), 7.33-7.37 (1H, m), 8.12 (1H, d, J = 1.8 Hz). |
| | | MS (ESI⁺): 393.2 [M+H]⁺ |

### <Example 11-1>

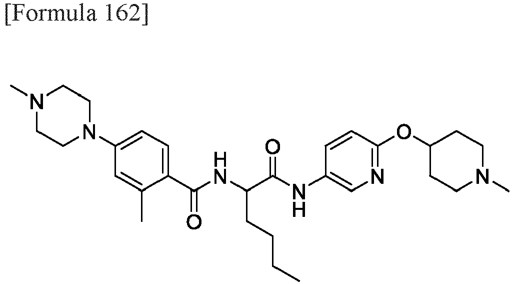

Under argon atmosphere, 2-Amino-N-[6-(1-methylpiperidin-4-yl)oxypyridin-3-yl]hexanamide (50.0 mg) and 1-hydroxybenzotriazole monohydrate (25.3 mg) were added to a solution of 2-methyl-4-(4-methylpiperazin-1-yl)benzoic acid (36.5 mg) in N,N-dimethylformamide (0.780 mL). N,N-diisopropylethylamine (0.040 mL) and 1-ethyl-3-(3-dimethylaminopropyl)carbodiimide hydrochloride (33.0 mg) were added to the mixture under ice cooling condition. The mixture was stirred at room temperature for 6 hours. After water was added, the mixture was extracted with ethyl acetate. The organic layer was washed with saturated brine, dried over anhydrous sodium sulfate, and concentrated under reduced pressure after filtration of the insoluble material. The residue was purified by amino-silica gel column chromatography (ethyl acetate : methanol = 9 : 1), and the resulting solid was washed with (hexane : ethyl acetate = 5 : 1) to obtain 2-methyl-4-(4-methylpiperazin-1-yl)-N-[1-[[6-(1-methylpiperidin-4yl)oxypyridin-3-yl]amino]-1-oxohexan-2-yl]benzamide (26.5 mg).

¹H-NMR (DMSO-D₆, 400MHz) δ: 0.87 (3H, t, J = 6.7 Hz), 1.25-1.38 (4H, m), 1.57-1.75 (4H, m), 1.88-1.96 (2H, m) , 2.07-2.13 (2H, m), 2.16 (3H, s), 2.20 (3H, s), 2.31 (3H, s), 2.42 (4H, t, J = 4.8 Hz), 2.57-2.64 (2H, m), 3.17 (4H, t, J = 4.8 Hz), 4.45 (1H, td, J = 7.9, 6.1 Hz), 4.85-4.93 (1H, m), 6.72-6.76 (3H, m), 7.31 (1H, d, J = 9.1 Hz), 7.89 (1H, dd, J = 9.1, 2.4 Hz), 8.11 (1H, d, J = 7.9 Hz), 8.32 (1H, d, J = 2.4 Hz), 10.05 (1H, s).

HRMS (ESI⁺): 537.35620 [M+H]⁺

Using the corresponding starting materials and reactants, the following Examples 11-2 to 11-11 were obtained by the same method in Example 11-1, the method described in Step 3-3, or a method similar thereto.

**[Table 115]**

| Example | Structure | Equipment Data |
|---|---|---|
| 1 1 - 2 | 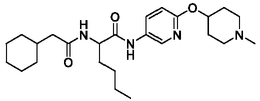 | ¹H-NMR (DMSO-D₆, 400MHz) δ: 0.80-0.94 (5H, m), 1.00-1.36 (7H, m), 1.52-1.69 (10H, m), 1.87-1.95 (2H, m), 2.01 (2H, dd, J = 7.3, 5.4 Hz), 2.07-2.13 (2H, m), 2.15 (3H, s), 2.55-2.67 (2H, m), 4.34 (1H, td, J = 7.9, 5.4 Hz), 4.84-4.92 (1H, m), 6.73 (1H, d, J = 8.5 Hz), 7.86 (1H, dd, J = 8.5, 2.4 Hz), 8.02 (1H, d, J = 7.9 Hz), 8.30 (1H, d, J = 2.4 Hz), 10.01 (1H, s). |
| | | HRMS (ESI⁺):445.31852 [M+H]⁺ |

**[Table 116]**

| Example | Structure | Equipment Data |
|---|---|---|
| 1 1 - 3 | 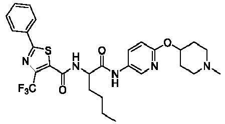 | ¹H-NMR (DMSO-D₆, 400MHz) δ: 0.88 (3H, t, J = 6.7 Hz), 1.28-1.38 (4H, m), 1.56-1.82 (4H, m), 1.89-1.97 (2H, m), 2.09-2.14 (2H, m), 2.16 (3H, s), 2.56-2.67 (2H, m), 4.54 (1H, td, J = 7.9, 6.1 Hz), 4.86-4.94 (1H, m), 6.76 (1H, d, J = 9.1 Hz), 7.53-7.60 (3H, m), 7.90 (1H, dd, J = 9.1, 2.4 Hz), 8.00 (2H, dd, J = 7.9, 1.8 Hz), 8.33 (1H, d, J = 2.4 Hz), 9.32 (1H, d, J = 7.9 Hz), 10.22 (1H, s). |
| | | HRMS (ESI⁺):576.22544 [M+H]⁺ |
| 1 1 - 4 | 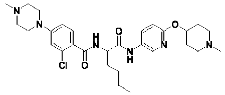 | ¹H-NMR (DMSO-D₆, 400MHz) δ: 0.86 (3H, t, J = 6.7 Hz), 1.27-1.42 (4H, m), 1.57-1.75 (4H, m), 1.88-1.96 (2H, m), 2.08-2.13 (2H, m), 2.16 (3H, s), 2.20 (3H, s), 2.41 (4H, t, J = 4.8 Hz), 2.57-2.64 (2H, m), 3.21 (4H, t, J = 4.8 Hz), 4.48 (1H, td, J = 8.5, 5.4 Hz), 4.85-4.93 (1H, m), 6.74 (1H, d, J = 9.1 Hz), 6.89-6.94 (2H, m), 7.35 (1H, d, J = 8.5 Hz), 7.89 (1H, dd, J = 9.1, 2.4 Hz), 8.31-8.35 (2H, m), 10.07 (1H, s). |
| | | HRMS (ESI⁺):557.30001 [M+H]⁺ |
| 1 1 - 5 | 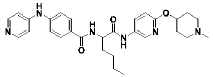 | ¹H-NMR (DMSO-D₆, 400MHz) δ: 0.86 (3H, t, J = 6.7 Hz), 1.28-1.44 (4H, m), 1.56-1.67 (2H, m), 1.75-1.83 (2H, m), 1.88-1.96 (2H, m), 2.09-2.14 (2H, m), 2.16 (3H, s), 2.57-2.65 (2H, m), 4.53 (1H, q, J = 7.3 Hz), 4.86-4.92 (1H, m), 6.74 (1H, d, J = 9.1 Hz), 7.00 (2H, dd, J = 5.4, 1.2 Hz), 7.23 (2H, d, J = 9.1 Hz), 7.87-7.92 (3H, m), 8.25 (2H, dd, J = 5.4, 1.2 Hz), 8.34 (1H, d, J = 3.0 Hz), 8.41 (1H, d, J = 7.9 Hz), 9.09 (1H, s), 10.09 (1H, s). |
| | | HRMS (ESI⁺):517.29291 [M+H]⁺ |
| 1 1 - 6 | 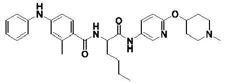 | ¹H-NMR (DMSO-D₆, 400MHz) δ: 0.87 (3H, t, J = 6.7 Hz), 1.27-1.44 (4H, m), 1.58-1.66 (2H, m), 1.70-1.79 (2H, m), 1.88-1.96 (2H, m), 2.08-2.14 (2H, m), 2.16 (3H, s), 2.30 (3H, s), 2.57-2.64 (2H, m), 4.42-4.50 (1H, m), 4.86-4.93 (1H, m), 6.74 (1H, d, J = 8.5 Hz), 6.84-6.90 (3H, m), 7.09 (2H, dd, J = 8.5, 1.2 Hz), 7.25 (2H, dd, J = 8.5, 7.3 Hz), 7.32 (1H, d, J = 7.9 Hz), 7.90 (1H, dd, J = 8.5, 2.4 Hz), 8.16 (1H, d, J = 7.9 Hz), 8.31-8.34 (2H, m), 10.06 (1H, s). |
| | | HRMS (ESI⁺):530.31249 [M+H]⁺ |

**[Table 117]**

| Example | Structure | Equipment Data |
|---|---|---|
| 1 1 - 7 | 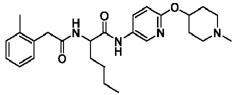 | ¹H-NMR (DMSO-D₆, 400MHz) δ: 0.84 (3H, t, J = 7.0 Hz), 1.17-1.39 (4H, m), 1.52-1.75 (4H, m), 1.87-1.97 (2H, m), 2.07-2.19 (5H, m), 2.23 (3H, s), 2.57-2.68 (2H, m), 3.49 (1H, d, J = 15.1 Hz), 3.54 (1H, d, J = 15.1 Hz), 4.31-4.42 (1H, m), 4.83-4.94 (1H, m), 6.74 (1H, d, J = 9.1 Hz), 7.04-7.20 (4H, m), 7.86 (1H, dd, J = 8.8, 2.7 Hz), 8.24-8.32 (2H, m), 10.06 (1H, s). |
| | | HRMS (ESI⁺): 453.28676 [M+H]⁺ |
| 1 1 - 8 | 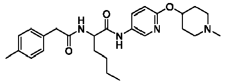 | ¹H-NMR (DMSO-D₆, 400MHz) δ: 0.82 (3H, t, J = 7.0 Hz), 1.13-1.35 (4H, m), 1.49-1.75 (4H, m), 1.83-1.99 (2H, m), 2.04-2.18 (5H, m), 2.24 (3H, s), 2.52-2.68 (2H, m), 3.39 (1H, d, J = 13.9 Hz), 3.45 (1H, d, J = 13.9 Hz), 4.30-4.39 (1H, m), 4.83-4.94 (1H, m), 6.73 (1H, d, J = 9.1 Hz), 7.07 (2H, d, J = 8.5 Hz), 7.14 (2H, d, J = 8.5 Hz), 7.85 (1H, dd, J = 9.1, 2.7 Hz), 8.26-8.34 (2H, m), 10.05 (1H, s). |
| | | HRMS (ESI⁺): 453.28637 [M+H]⁺ |
| 1 1 - 9 | 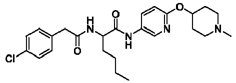 | ¹H-NMR (DMSO-D₆, 400MHz) δ: 0.82 (3H, t, J = 6.7 Hz), 1.16-1.32 (4H, m), 1.51-1.74 (4H, m), 1.87-1.96 (2H, m), 2.05-2.18 (5H, m), 2.56-2.65 (2H, m), 3.46 (1H, d, J = 13.9 Hz), 3.52 (1H, d, J= 13.9 Hz), 4.30-4.40 (1H, m), 4.83-4.93 (1H, m), 6.73 (1H, d, J = 9.1 Hz), 7.28 (2H, d, J = 8.5 Hz), 7.34 (2H, d, J = 8.5 Hz), 7.86 (1H, dd, J = 9.1, 3.0 Hz), 8.30 (1H, d, J = 3.0 Hz), 8.40 (1H, d, J = 7.9 Hz), 10.07 (1H, s). |
| | | HRMS (ESI⁺): 473.23180 [M+H]⁺ |
| 1 1 - 1 0 | 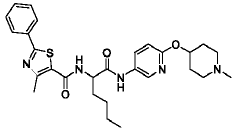 | ¹H-NMR (DMSO-D₆, 400MHz) δ: 0.88 (3H, t, J = 7.3 Hz), 1.24-1.47 (4H, m), 1.56-1.68 (2H, m), 1.72-1.85 (2H, m), 1.88-1.97 (2H, m), 2.07-2.18 (5H, m), 2.56-2.65 (5H, m), 4.44-4.55 (1H, m), 4.85-4.95 (1H, m), 6.75 (1H, d, J = 9.1 Hz), 7.49-7.54 (3H, m), 7.90 (1H, dd, J = 9.1, 2.4 Hz), 7.92-7.98 (2H, m), 8.33 (1H, d, J = 2.4 Hz), 8.49 (1H, d, J = 7.3 Hz), 10.16 (1H, s). |
| | | HRMS (ESI⁺): 522.25372 [M+H]⁺ |

**[Table 118]**

| Example | Structure | Equipment Data |
|---|---|---|
| 1 1 - 1 1 | 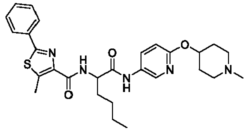 | ¹H-NMR (DMSO-D₆, 400MHz) δ: 0.88 (3H, t, J = 7.3 Hz), 1.26-1.46 (4H, m), 1.56-1.68 (2H, m), 1.73-1.84 (2H, m), 1.87-1.97 (2H, m), 2.06-2.19 (5H, m), 2.55-2.67 (5H, m), 4.45-4.55 (1H, m), 4.85-4.94 (1H, m), 6.75 (1H, d, J = 9.1 Hz), 7.49-7.55 (3H, m), 7.87-7.97 (3H, m), 8.33 (1H, d, J = 2.4 Hz), 8.48 (1H, d, J = 7.9 Hz), 10.15 (1H, s). |
| | | HRMS (ESI⁺): 522.25428 [M+H]⁺ |

### <Example 12-1>

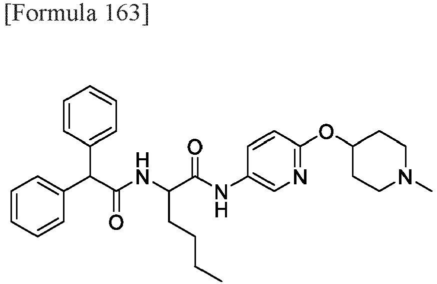

Under argon atmosphere, pyridine (18.9 µL) and 2,2-diphenylacetyl chloride (39.7 mg) were added to a solution of 2-amino-N-[6-(1-methylpiperidin-4-yl)oxypyridin-3-yl]hexanamide (50.0 mg) in tetrahydrofuran (1.00 mL) at 0 °C, and the mixture was stirred at room temperature for 3 hours. The reaction mixture was purified by amino-silica gel column chromatography (methanol ethyl acetate = 0 1 to 1 9) to obtain 2-[(2,2-diphenylacetyl)amino]-N-[6-(1-methylpiperidin-4-yl)oxypyridin-3-yl]hexane amide (67.0 mg).

¹H-NMR (DMSO-D₆, 400MHz) δ: 0.79 (3H, t, J = 7.0 Hz), 1.10-1.31 (4H, m), 1.50-1.75 (4H, m), 1.86-1.96 (2H, m) , 2.07-2.18 (5H, m), 2.55-2.64 (2H, m), 4.35-4.46 (1H, m), 4.83-4.93 (1H, m), 5.13 (1H, s), 6.73 (1H, d, J = 9.1 Hz), 7.16-7.35 (10H, m), 7.85 (1H, dd, J = 9.1, 3.0 Hz), 8.29 (1H, d, J = 3.0 Hz), 8.57 (1H, d, J = 7.3 Hz), 10.10 (1H, s).

HRMS (ESI⁺): 515.30139 [M+H]⁺

Using the corresponding starting materials and reactants, the following Example 12-2 was obtained by the same method in Example 12-1, the method described in Step 3-3, or a method similar thereto.

**[Table 119]**

| Example | Structure | Equipment Data |
|---|---|---|
| 1 2 - 2 | 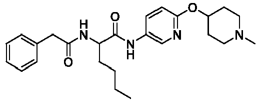 | ¹H-NMR (DMSO-D₆, 400MHz) δ: 0.70-0.90 (3H, m), 1.22-1.28 (4H, m), 1.56-1.66 (4H, m), 1.87-1.95 (2H, m), 2.07-2.13 (2H, m), 2.15 (3H, s), 2.56-2.64 (2H, m), 3.31 (2H, s), 4.32-4.39 (1H, m), 4.84-4.92 (1H, m), 6.73 (1H, d, J = 8.5 Hz), 7.24-7.32 (5H, m), 7.86 (1H, d, J = 8.5 Hz), 8.31 (1H, s), 8.36 (1H, d, J = 7.9 Hz), 10.06 (1H, s). |
| | | HRMS (ESI⁺):439.27014 [M+H]⁺ |

### <Reference example 73-1>

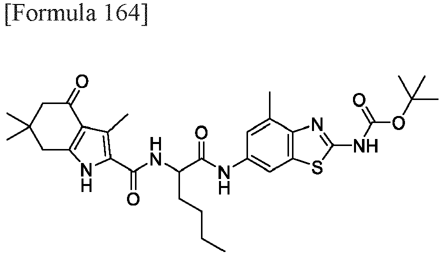

Using 3,6,6-trimethyl-4-oxo-5,7-dihydro-1H-indole-2-carboxylic acid and tert-butyl N-[6-(2-aminohexanoylamino)-4-methyl-1,3-benzothiazol-2-yl]carbamate, the title compound was synthesized by the same method in Reference in Example 6-1.

¹H-NMR (DMSO-D₆, 400MHz) δ: 0.87 (3H, t, J = 7.0 Hz), 1.01 (6H, s), 1.27-1.42 (4H, m), 1.48 (9H, s), 1.66-1.86 (2H, m), 2.22 (2H, s), 2.47 (3H, s), 2.64 (2H, s), 3.30 (3H, s), 4.52-4.60 (1H, m), 7.32 (1H, d, J = 1.8 Hz), 7.57 (1H, d, J = 7.3 Hz), 8.09 (1H, d, J = 1.8 Hz), 10.15 (1H, s), 11.68 (1H, s), 11.72 (1H, s).

MS (ESI⁺): 596.3 [M+H]⁺

Using the corresponding starting materials and reactants, the following Reference Examples 73-2 to 73-4 were obtained by the same method in Reference Example 73-1, the method described in Step 3-2 or Step 3-3, or a method similar thereto.

**[Table 120]**

| Reference Example | Structure | Equipment Data |
|---|---|---|
| 7 3 - 2 | 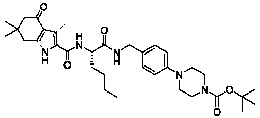 | ¹H-NMR (398.98MHz, CDCl₃) δ: 0.88 (3H, t, J = 7.0 Hz), 1.09 (6H, s), 1.27-1.40 (4H, m), 1.48 (9H, s), 1.65-1.80 (1H, m), 1.90-2.00 (1H, m), 2.33 (2H, s), 2.62 (2H, s), 2.66 (3H, s), 3.08-3.13 (4H, m), 3.54-3.59 (4H, m), 4.34 (1H, dd, J = 6.0, 14.8 Hz), 4.43 (1H, dd, J = 6.0, 14.8 Hz), 4.54 (1H, m), 6.28 (1H, m), 6.56 (1H, d, J = 7.6 Hz), 6.86 (2H, d, J = 8.8 Hz), 7.16 (2H, d, J = 8.8 Hz), 9.36 (1H, m). |
| | | MS (ESI⁺): 608.34 [M+H]⁺ |
| 7 3 - 3 | 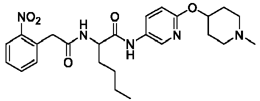 | ¹H-NMR (DMSO-D₆, 400MHz) δ: 0.85 (3H, t, J = 6.7 Hz), 1.20-1.39 (4H, m), 1.52-1.77 (4H, m), 1.87-1.96 (2H, m), 2.07-2.18 (5H, m), 2.56-2.65 (2H, m), 3.88 (1H, d, J = 16.3 Hz), 4.01 (1H, d, J = 16.3 Hz), 4.30-4.38 (1H, m), 4.83-4.94 (1H, m), 6.73 (1H, d, J = 9.1 Hz), 7.46-7.55 (2H, m), 7.61-7.69 (1H, m), 7.86 (1H, dd, J = 9.1, 2.4 Hz), 7.95-8.00 (1H, m), 8.30 (1H, d, J = 2.4 Hz), 8.40 (1H, d, J = 7.9 Hz), 10.01 (1H, s). |
| | | HRMS (ESI⁺): 484.25616 [M+H]⁺ |
| 7 3 - 4 | 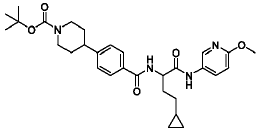 | ¹H-NMR (DMSO-D₆, 400MHz) δ: -0.04-0.07 (2H, m), 0.33-0.43 (2H, m), 0.64-0.76 (1H, m), 1.19-1.35 (2H, m), 1.41 (9H, s), 1.44-1.57 (2H, m), 1.69-1.79 (2H, m), 1.84-1.95 (2H, m), 2.67-2.97 (3H, m), 3.80 (3H, s), 4.02-4.15 (2H, m), 4.52-4.61 (1H, m), 6.78 (1H, d, J = 9.2 Hz), 7.33 (2H, d, J = 8.6 Hz), 7.84 (2H, d, J = 8.6 Hz), 7.90 (1H, dd, J = 9.2, 2.4 Hz), 8.36 (1H, d, J = 2.4 Hz), 8.49 (1H, d, J = 7.9 Hz), 10.08 (1H, s). |
| | | HRMS (ESI⁺): 537.30840 [M+H]⁺ |

### <Example 13-1>

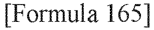

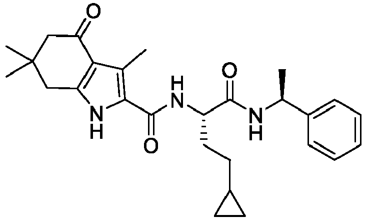

Using tert-butyl(4-cyclopropyl-1-oxo-1-(((S)-1-phenylethyl)amino)butan-2-yl)carbamate, N-((S)-4-cyclopropyl-1-oxo-1-(((S)-1-phenyl ethyl)amino)butan-2-yl)-3,6,6-trimethyl-4-oxo-4,5,6,7-tetrahydro-1H-indole-2-carboxamide and its isomers were obtained by the same method in Reference Example 6-1.

¹H-NMR (270MHz, CDCl₃) δ: -0.23-0.07 (2H, m), 0.23-0.43 (2H, m), 0.45-0.65 (1H, m), 0.93-1.29 (8H, m), 1.38 (3H, d, J = 6.9 Hz), 1.65-2.01 (2H, m), 2.29 (2H, s), 2.51 (2H, s), 2.65 (3H, s), 4.69 (1H, dd, J = 13.5, 7.6 Hz), 4.99-5.10 (1H, m), 7.18-7.40 (7H, m), 10.34 (1H, s).

MS (ESI⁺): 450.37 [M+H]⁺

### <Example 13-2>

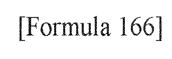

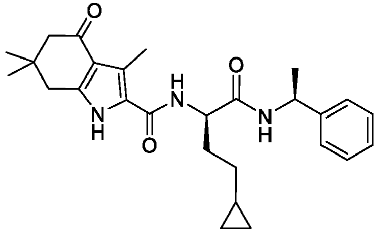

N-((R)-4-Cyclopropyl-1-oxo-1-(((S)-1-phenylethyl)amino)butan-2-yl)-3,6,6-trimethyl-4-oxo-4,5,6,7-tetrahydro-1H-indole-2-carboxamide.

¹H-NMR (270MHz, CDCl₃) δ: -0.05-0.10 (2H, m), 0.38-0.50 (2H, m), 0.57-0.80 (1H, m), 1.08 (6H, s), 1.17-1.42 (2H, m), 1.50 (3H, d, J = 6.9 Hz), 1.72-1.92 (1H, m), 1.97-2.18 (1H, m) ), 2.32 (2H, s), 2.57-2.64 (5H, m), 4.48-4.68 (1H, m), 5.02-5.19 (1H, m), 6.34 (1H, d, J = 7.6 Hz), 6.50 ( 1H, d, J = 7.9 Hz), 7.17-7.40 (5H, m), 9.28 (1H, s).

MS (ESI⁺): 450.37 [M+H]⁺

Using the corresponding starting material and reactant, the following Examples 13-3 to 13-6 were obtained by the same method in Example 13-1, the method described in Step 3-2 or Step 3-3, or a method similar thereto.

**[Table 121]**

| Example | Structure | Equipment Data |
|---|---|---|
| 1 3 - 3 | 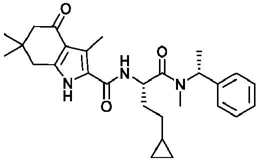 | ¹H-NMR (270 MHz, CDCl₃) δ: -0.13-0.15 (2H, m), 0.29-0.52 (2H, m), 0.53-0.82 (1H, m), 1.00-1.13 (7H, m), 1.13-1.60 (4H, m), 1.64-2.15 (2H, m), 2.31 (2H, s), 2.56-2.83 (9H, m), 5.02-5.41 (1H, m), 5.95-6.17 (1H, m), 7.01-7.50 (5H, m), 9.96 (1H, s). |
| | | MS (ESI⁺): 464.39 [M+H]⁺ |

**[Table 122]**

| Example | Structure | Equipment Data |
|---|---|---|
| 1 3 - 4 | 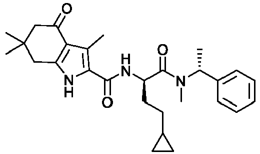 | ¹H-NMR (270 MHz, CDCl₃) δ: -0.13-0.17 (2H, m), 0.32-0.53 (2H, m), 0.60-0.82 (1H, m), 1.03-1.62 (11H), 1.62-2.17 (3H, m), 2.35 (2H, s), 2.59-2.84 (7H, m), 5.06-5.22 (1H, m), 5.96-6.13 (1H, m), 7.09-7.47 (6H, m), 9.84 (1H, m). |
| | | MS (ESI⁺): 464.39 [M+H]⁺ |
| 1 3 - 5 | 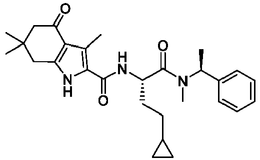 | ¹H-NMR (270 MHz, CDCl₃) δ: -0.12-0.17 (2H, m), 0.28-0.56 (2H, m), 0.56-0.87 (1H, m), 1.02-1.29 (7H, m), 1.37-2.06 (6H, m), 2.34 (2H, s), 2.59-2.79 (8H, m), 5.06-5.19 (1H, m), 5.97-6.03 (1H, q J = 6.9 Hz), 6.98-7.11 (1H, m), 7.22-7.44 (5H, m), 9.33 (1H, s). |
| | | MS (ESI⁺): 464.41 [M+H]⁺ |
| 1 3 - 6 | 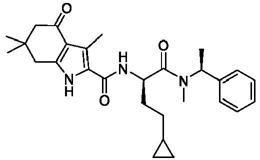 | ¹H-NMR (270 MHz, CDCl₃) δ: 0.00-0.08 (2H, m), 0.42-0.76 (3H, m), 1.08-2.03 (13H, m), 2.33 (2H, s), 2.63-2.79 (8H, m), 5.09-5.34 (1H, m), 6.04-6.09 (1H, m), 7.04-7.41 (6H, m), 9.52 (1H, s). |
| | | MS (ESI⁺): 464.42 [M+H]⁺ |

### <Reference example 74-1>

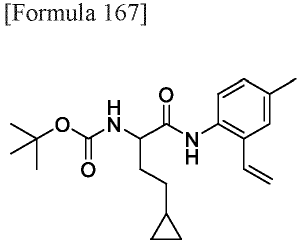

Under argon atmosphere, 2-ethaneyl-4,4,5,5-tetramethyl-1,3,2-dioxaborolane (0.100 mL), 2 mol/L sodium carbonate aqueous solution (0.730 mL) and bis(triphenylphosphine) palladium (II) dichloride (34.1 mg) were added to a solution of tert-butyl N-[1-(2-bromo-4-methylanilino)-4-cyclopropyl-1-oxobutan-2-yl]carbamate (200 mg) in 1,2-dimethoxyethane (2.43 mL), and the mixture was stirred at 80 °C for 3 hours. After water was added, the mixture was extracted with ethyl acetate. The organic layer was washed with saturated brine, dried over anhydrous sodium sulfate, and concentrated under reduced pressure after filtration of the insoluble material. The residue was purified by silica gel column chromatography (ethyl acetate / hexane = 0 to 33%) to obtain tert-butyl N-[4-cyclopropyl-1-(2-ethenyl-4-methylanilino)-1-oxobutan-2-yl]carbamate (86.7 mg).

¹H-NMR (CDCl₃, 400MHz) δ: 0.03-0.07 (2H, m), 0.43-0.48 (2H, m), 0.66-0.72 (1H, m), 1.30-1.38 (2H, m), 1.47 (9H, s), 1.74-1.81 (1H, m), 2.04-2.14 (1H, m), 2.32 (3H, s), 4.15-4.30 (1H, m), 4.85-5.05 (1H, m), 5.36 (1H, d, J = 10.9 Hz), 5.66 (1H, d, J = 17.0 Hz), 6.78 (1H, dd, J = 17.0, 10.9 Hz), 7.09 (1H, dd, J = 8.5, 1.8 Hz), 7.25 (1H, d, J = 1.8 Hz), 7.69 (1H, d, J = 8.5 Hz), 7.93 (1H, brs).

HRMS (ESI⁺): 359.23318 [M+H]⁺

Using the corresponding starting materials and reactants, the following Reference Example 74-2 was obtained by the same method in Reference Example 74-1, the method described in Step 36-1, or a method similar thereto.

**[Table 123]**

| Reference Example | Structure | Equipment Data |
|---|---|---|
| 7 4 - 2 | 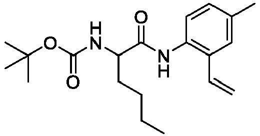 | ¹H-NMR (CDCl₃, 400MHz) δ: 0.92 (3H, t, J = 6.7 Hz), 1.36-1.41 (4H, m), 1.47 (9H, s), 1.63-1.71 (1H, m), 1.94-2.00 (1H, m), 2.32 (3H, s), 4.05-4.25 (1H, m), 4.85-5.05 (1H, m), 5.37 (1H, d, J = 11.0 Hz), 5.66 (1H, d, J = 17.1 Hz), 6.78 (1H, dd, J = 17.1, 11.0 Hz), 7.09 (1H, dd, J = 8.6, 1.8 Hz), 7.25 (1H, d, J = 1.8 Hz), 7.70 (1H, d, J = 8.6 Hz), 7.89 (1H, brs). |
| | | HRMS (ESI⁺):347.23266 [M+H]⁺ |

### <Reference example 75-1>

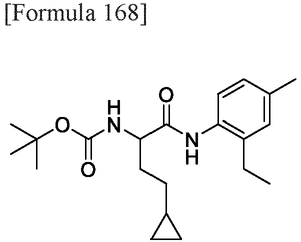

Under argon atmosphere, 5% palladium carbon (17.3 mg) was added to a solution of tert-butyl N-[4-cyclopropyl-1-(2-ethenyl-4-methylanilino)-1-oxobutan-2-yl]carbamate (86.7 mg) in tetrahydrofuran-ethanol (1 : 1) mixed solution(1.21 mL). The mixture was stirred at room temperature for 9 hours under a hydrogen atmosphere. After replacing with an argon atmosphere, the reaction mixture was filtered through Celite, and the filtrate was concentrated. The residue was purified by silica gel column chromatography (ethyl acetate / hexane = 0 to 30%) to obtain tert-butyl N-[4-cyclopropyl-1-(2-ethyl-4-methylanilino)-1-oxobutan-2-yl]carbamate (76.1 mg).

¹H-NMR (CDCl₃, 400MHz) δ: 0.03-0.07 (2H, m), 0.43-0.48 (2H, m), 0.65-0.74 (1H, m), 1.21 (3H, t, J = 7.3 Hz), 1.31-1.38 (2H, m), 1.46 (9H, s), 1.73-1.83 (1H, m), 2.07-2.15 (1H, m), 2.30 (3H, s), 2.56 (2H, q, J = 7.3 Hz), 4.15-4.30 (1H, m), 4.95 (1H, brs), 6.99-7.03 (2H, m), 7.66 (1H, d, J = 8.6 Hz), 7.87 (1H, brs).

HRMS (ESI⁺): 361.24877 [M+H]⁺

Using the corresponding starting materials and reactants, the following Reference Example 75-2 was obtained by the same method in Reference Example 75-1, the method described in Step 36-2, or a method similar thereto.

**[Table 124]**

| Reference Example | Structure | Equipment Data |
|---|---|---|
| 7 5 - 2 | 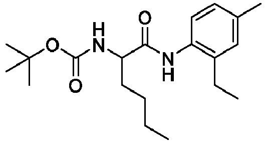 | ¹H-NMR (CDCl₃, 400 MHz) δ: 0.92 (3H, t, J = 6.7 Hz), 1.21 (3H, t, J = 7.3 Hz), 1.38-1.42 (4H, m), 1.47 (9H, s), 1.62-1.72 (1H, m), 1.96-2.01 (1H, m), 2.31 (3H, s), 2.56 (2H, q, J = 7.3 Hz), 4.16-4.19 (1H, m), 4.96 (1H, brs), 7.00-7.03 (2H, m), 7.67 (1H, d, J = 8.6 Hz), 7.83 (1H, brs). |
| | | HRMS (ESI⁺):349.24873 [M+H]⁺ |

### <Example 14-1>

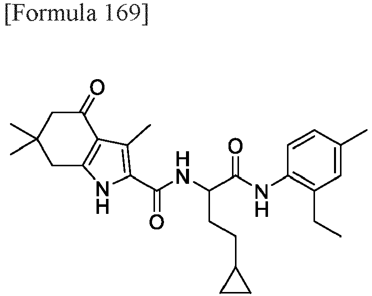

Under argon atmosphere, trifluoroacetic acid (0.528 mL) was added to a solution of tert-butyl N-[4-cyclopropyl-1-(2-ethyl-4-methylanilino)-1-oxobutan-2-yl]carbamate (76.1 mg) in dichloromethane (0.528 mL), and the mixture was stirred at room temperature for 30 minutes. The reaction mixture was concentrated under reduced pressure to obtain a crude product. 3,6,6-Trimethyl-4-oxo-5,7-dihydro-1H-indole-2-carboxylic acid (46.7 mg), 1-hydroxybenzotriazole monohydrate (38.7 mg), 1-ethyl-3-(3-dimethylaminopropyl) carbodiimide hydrochloride (44.5 mg), and N,N-diisopropylethylamine (0.144 mL) were added to the crude product in N,N-dimethylformamide (1.06 mL), and the mixture was stirred at room temperature for 24 hours. After water was added, the mixture was extracted with ethyl acetate. The organic layer was washed with saturated brine, dried over anhydrous sodium sulfate, and concentrated under reduced pressure after filtration of the insoluble material. The residue was purified by silica gel column chromatography (ethyl acetate / hexane = 10 to 50%), the resulting solid was washed with diisopropyl ether to obtain N-[4-cyclopropyl-1-(2-ethyl-4-methylanilino)-1-oxobutan-2-yl]-3,6,6-trimethyl-4-oxo-5,7-dihydro-1H-indole-2-carboxamide (61.6 mg).

¹H-NMR (DMSO-D₆, 400MHz) δ: 0.00-0.05 (2H, m), 0.39-0.44 (2H, m), 0.69-0.77 (1H, m), 1.02 (6H, s), 1.08 (3H, t, J = 7.3 Hz), 1.28-1.35 (2H, m), 1.81-1.99 (2H, m), 2.22 (2H, s), 2.26 (3H, s), 2.48 (3H, s), 2.49-2.55 (2H, m), 2.64 (2H, s), 4.65 (1H, td, J = 7.9, 6.1 Hz), 6.97 (1H, dd, J = 7.9, 1.8 Hz), 7.04 (1H, d, J = 1.8 Hz), 7.16 (1H, d, J = 7.9 Hz), 7.56 (1H, d, J = 7.9 Hz), 9.41 (1H, s), 11.69 (1H, brs).

HRMS (ESI⁺): 464.29052 [M+H]⁺

Using the corresponding starting material and reactant, the following Example 14-2 was obtained by the same method in Example 14-1, the method described in Step 3-2 or Step 3-3, or a method similar thereto.

**[Table 125]**

| Example | Structure | Equipment Data |
|---|---|---|
| 1 4 - 2 | 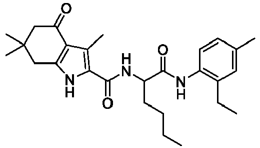 | ¹H-NMR (DMSO-D₆, 400MHz) δ: 0.89 (3H, t, J = 6.7 Hz), 1.02 (6H, s), 1.08 (3H, t, J = 7.3 Hz), 1.31-1.42 (4H, m), 1.69-1.87 (2H, m), 2.22 (2H, s), 2.26 (3H, s), 2.48 (3H, s), 2.49-2.56 (2H, m), 2.64 (2H, s), 4.63 (1H, td, J = 7.9, 6.1 Hz), 6.97 (1H, dd, J = 7.9, 1.8 Hz), 7.04 (1H, d, J = 1.8 Hz), 7.17 (1H, d, J = 7.9 Hz), 7.55 (1H, d, J = 7.9 Hz), 9.42 (1H, s), 11.71 (1H, br s). |
| | | HRMS (ESI⁺):452.29181 [M+H]⁺ |

### <Reference example 76-1>

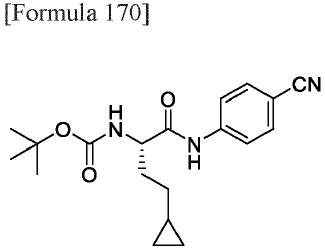

Under argon atmosphere, zinc cyanide (363 mg) was added to a solution of tert-butyl N-[(2S)-4-cyclopropyl-1-(4-iodoanilino)-1-oxobutan-2-yl]carbamate (275 mg) in N,N-dimethylformamide (1.55 mL), the mixture was stirred at 90 °C for 15 minutes, and then dibenzylideneacetone palladium (0) (72.0 mg), 2-dicyclohexylphosphino-2',6'-dimethoxybiphenyl (51.0 mg) were added, and the mixture was stirred at 90 °C for 3 hours, and then dibenzylideneacetone palladium (0) (72.0 mg), 2-dicyclohexylphosphino-2',6'-dimethoxybiphenyl (51.0 mg) were added, and the mixture was stirred at 90 °C for 3 hours. After water was added, the reaction mixture was extracted with ethyl acetate. The organic layer was washed with saturated brine, dried over anhydrous sodium sulfate, and concentrated under reduced pressure after filtration of the insoluble material. The residue was purified by silica gel column chromatography (ethyl acetate / hexane = 8 to 33%) to obtain tert-butyl N-[(2S)-1-(4-cyanoanilino)-4-cyclopropyl-1-oxobutan-2-yl]carbamate (145 mg).

¹H-NMR (CDCl₃, 400MHz) δ: -0.01-0.03 (2H, m), 0.41-0.44 (2H, m), 0.61-0.69 (1H, m), 1.27-1.32 (2H, m), 1.43 (9H, s), 1.68-1.78 (1H, m), 2.03-2.08 (1H, m), 4.16-4.22 (1H, m), 4.88 (1H, brs), 7.55 (2H, d, J = 8.5 Hz) , 7.60 (2H, d, J = 8.5 Hz), 8.79 (1H, brs).

HRMS (ESI⁺): 344.19695 [M+H]⁺

Using the corresponding starting materials and reactants, the following Reference Example 76-2 was obtained by the same method in Reference Example 76-1, the method described in Step 37-1, or a method similar thereto.

**[Table 126]**

| Reference Example | Structure | Equipment Data |
|---|---|---|
| 7 6 - 2 | 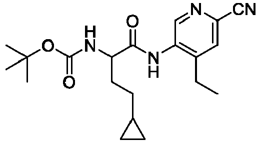 | ¹H-NMR (CDCl₃, 400 MHz) δ: -0.01-0.02 (2H, m), 0.40-0.44 (2H, m), 0.60-0.66 (1H, m), 1.25 (3H, t, J = 7.3 Hz), 1.28-1.34 (2H, m), 1.42 (9H, s), 1.71-1.80 (1H, m), 2.05-2.12 (1H, m), 2.57-2.65 (2H, m), 4.18-4.23 (1H, m), 4.85 (1H, brs), 7.51 (1H, s), 8.84 (1H, brs), 9.39 (1H, s). |
| | | HRMS (ESI⁺):373.22437 [M+H]⁺ |

### <Example 15-1>

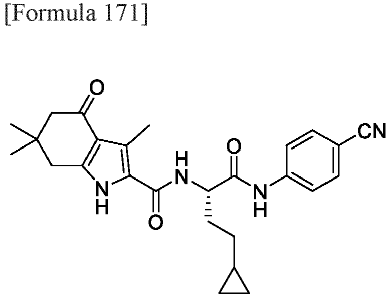

Under argon atmosphere, trifluoroacetic acid (1.06 mL) was added to a solution of tert-butyl N-[(2S)-1-(4-cyanoanilino)-4-cyclopropyl-1-oxobutan-2-yl]carbamate (145 mg) in dichloromethane (1.06 mL), the mixture was stirred at room temperature for 30 minutes, and then the reaction mixture was concentrated under reduced pressure. To a solution of the obtained crude product in N,N-dimethylformamide (2.11 mL), 3,6,6-Trimethyl-4-oxo-5,7-dihydro-1H-indole-2-carboxylic acid (93.4 mg), 1-hydroxybenzotriazole monohydrate (77.6 mg), N,N-diisopropylethylamine (0.287 mL), and 1-ethyl-3-(3-dimethylaminopropyl)carbodiimide hydrochloride (88.9 mg) were added, and the mixture was stirred at room temperature for 17 hours. After water was added, the reaction mixture was extracted with ethyl acetate. The organic layer was washed with saturated brine, dried over anhydrous sodium sulfate, and concentrated under reduced pressure after filtration of the insoluble material. The residue was purified by silica gel column chromatography (ethyl acetate / hexane = 10 to 66%), and the resulting solid was washed with diisopropyl ether to obtain N-[(2S)-1-(4-cyanoanilino)-4-cyclopropyl-1-oxobutan-2-yl]-3,6,6-trimethyl-4-oxo-5,7-dihydro-1H-indole-2-carboxamide (150 mg).

¹H-NMR (DMSO-D₆, 400MHz) δ: -0.01-0.02 (2H, m), 0.36-0.40 (2H, m), 0.66-0.74 (1H, m), 1.00 (6H, s), 1.21-1.33 (2H, m), 1.77-1.90 (2H, m), 2.20 (2H, s), 2.44 (3H, s), 2.62 (2H, s), 4.55 (1H, td, J = 7.9, 5.4 Hz), 7.71 (1H, d, J = 7.9 Hz), 7.76 (2H, d, J = 9.1 Hz), 7.79 (2H, d, J = 9.1 Hz), 10.58 (1H, s), 11.66 (1H, s).

HRMS (ESI⁺): 447.23893 [M+H]⁺

Using the corresponding starting materials and reactants, the following Examples 15-2 to 15-4 was obtained by the same method in Example 15-1, the method described in Step 3-2 or Step 3-3, or a method similar thereto.

**[Table 127]**

| Example | Structure | Equipment Data |
|---|---|---|
| 1 5 - 2 | 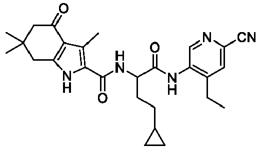 | ¹H-NMR (DMSO-D₆, 400MHz) δ: -0.02-0.03 (2H, m), 0.35-0.40 (2H, m), 0.66-0.74 (1H, m), 0.99 (6H, s), 1.12 (3H, t, J = 7.3 Hz), 1.26-1.34 (2H, m), 1.79-1.96 (2H, m), 2.19 (2H, s), 2.44 (3H, s), 2.61 (2H, s), 2.67 (2H, q, J = 7.3 Hz), 4.67 (1H, td, J = 7.9, 5.4 Hz), 7.68 (1H, d, J = 7.9 Hz), 7.95 (1H, s), 8.76 (1H, s), 9.95 (1H, s), 11.62 (1H, s). |
| | | HRMS (ESI⁺):476.26686 [M+H]⁺ |

**[Table 128]**

| **Example** | | **¹HNMR Data** | **MS Data [M+1]⁺** |
|---|---|---|---|
| 15-3 | 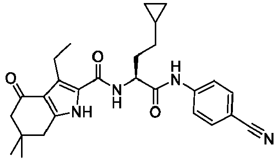 | ¹H-NMR (DMSO-D₆, 400 MHz) *δ*: -0.00-0.04 (2H, m), 0.37-0.43 (2H, m), 0.67-0.76 (1H, m), 1.02 (6H, s), 1.03 (3H, t, J=7.3 Hz), 1.21-1.38 (2H, m), 1.75-1.96 (2H, m), 2.23 (2H, s), 2.66 (2H, s), 2.99 (2H, q, J=7.3 Hz), 4.55-4.61 (1H, m), 7.72 (1H, d, J=7.4 Hz), 7.78 (2H, d, J=9.4 Hz), 7.80 (2H, s), 10.60 (1H, s), 11.62 (1H, s). | 461.21 |
| 15-4 | 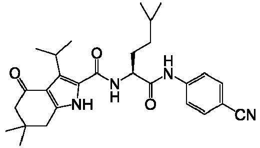 | ¹H-NMR (DMSO-D₆, 400 MHz) *δ*: 0.00-0.05 (2H, m), 0.37-0.43 (2H, m), 0.66-0.76 (1H, m), 1.02 (3H, s), 1.03 (3H, s), 1.18 (3H, d, J=7.0 Hz), 1.20 (3H, d, J=7.0 Hz), 1.23-1.38 (2H, m), 1.76-1.92 (2H, m), 2.26 (2H, s), 2.67 (2H, s), 3.96 (1H, p, J=7.0 Hz), 4.53 (1H, td, J=6.7, 7.4 Hz), 7.78 (2H, d, J=9.3 Hz), 7.82 (2H, d, J=9.3 Hz), 7.85 (1H, d, J=7.4 Hz), 10.57 (1H, s), 11.57 (1H, s). | 475.23 |

### <Reference example 77>

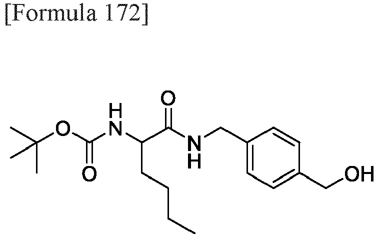

Under argon atmosphere, methyl 4-[[2-[(2-methylpropan-2-yl)oxycarbonylamino]hexanoylamino]methyl]benzoate (133 mg) in tetrahydrofuran (1.00 mL ) were added to a solution of lithium aluminum hydride (26.7 mg) in tetrahydrofuran (0.760 mL) under ice cooling condition, and the mixture was stirred at room temperature for 3 hours. After water (0.027 mL), 15% aqueous sodium hydroxide solution (0.027 mL), and water (0.080 mL) were added stepwise, the reaction mixture was filtered through Celite, and concentrated under reduced pressure. The residue was purified by silica gel column chromatography (ethyl acetate / hexane = 50%) to obtain tert-butyl N-[1-[[4-(hydroxymethyl)phenyl]methylamino]-1-oxohexan-2-yl]carbamate (81.4 mg).

¹H-NMR (CDCl₃, 400MHz) δ: 0.87 (3H, t, J = 6.7 Hz), 1.28-1.33 (4H, m), 1.40 (9H, s), 1.55-1.61 (1H, m), 1.80-1.89 (1H, m), 4.03 (1H, brs), 4.42 (2H, s), 4.66 (2H, s), 4.93 (1H, brs), 6.40 (1H, brs), 7.24 (2H, d, J = 7.9 Hz), 7.30 (2H, d, J = 7.9 Hz).

HRMS (ESI⁺): 351.22875 [M+H]⁺

### <Example 16>

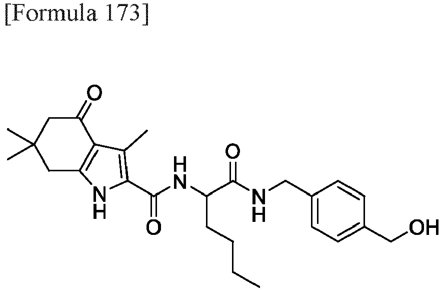

Under argon atmosphere, trifluoroacetic acid (0.581 mL) was added to a solution of tert-butyl N-[1-[[4-(hydroxymethyl)phenyl]methylamino]-1-oxohexan-2-yl]carbamate (81.4 mg) in dichloromethane (0.581 mL), and the mixture was stirred at room temperature for 30 minutes, and then the reaction mixture was concentrated under reduced pressure. To a solution of the obtained crude product in N,N-dimethylformamide (1.66 mL), 3,6,6-trimethyl-4-oxo-5,7-dihydro-1H-indole-2-carboxylic acid (51.3 mg), 1-hydroxybenzotriazole monohydrate (42.7 mg), N,N-diisopropylethylamine (0.158 mL) and 1-ethyl-3-(3-dimethylaminopropyl) carbodiimide hydrochloride (48.9 mg) were added, and the mixture was stirred at room temperature for 5 hours. After water was added, the reaction mixture was extracted with ethyl acetate. The organic layer was washed with saturated brine, dried over anhydrous sodium sulfate, and concentrated under reduced pressure after filtration of the insoluble material. The residue was purified by silica gel column chromatography (ethyl acetate) to obtain N-[1-[[4-(hydroxymethyl)phenyl]methylamino]-1-oxohexan-2-yl]-3,6,6-trimethyl-4-oxo-5,7-dihydro-1H-indole-2-carboxamide (73.8 mg).

¹H-NMR (DMSO-D₆, 400MHz) δ: 0.84 (3H, t, J = 7.0 Hz), 1.01 (6H, s), 1.24-1.31 (4H, m), 1.59-1.77 (2H, m), 2.21 (2H, s), 2.46 (3H, s), 2.63 (2H, s), 4.23 (1H, dd, J = 15.1, 6.1 Hz), 4.29 (1H, dd, J = 15.1, 6.1 Hz), 4.40-4.43 (1H, m), 4.44 (2H, d, J = 5.4 Hz), 5.12 (1H, t, J = 5.4 Hz), 7.19 (2H, d, J = 7.9 Hz), 7.23 (2H, d, J = 7.9 Hz), 7.48 (1H, d, J = 7.9 Hz), 8.54 (1H, t, J = 6.1 Hz), 11.72 (1H, s).

HRMS (ESI⁺): 454.27086 [M+H]⁺

### <Reference example 78-1>

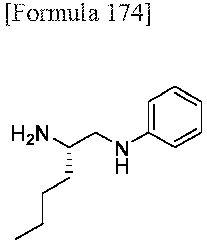

Trifluoroacetic acid (1 mL) was added to a solution of tert-butyl (S)-(1-oxo-1-(phenylamino)hexan-2-yl)carbamate (194 mg) in dichloromethane (10 mL), and stirred for overnight. 2 N aqueous sodium hydroxide solution was added to the reaction mixture, and the pH was adjusted between 13 and 14. The mixture was extracted three times with dichloromethane, dried over anhydrous sodium sulfate, and concentrated under reduced pressure after filtration. Borane-tetrahydrofuran complex (1 M in tetrahydrofuran) (2.3 mL) was added to a solution of the residue in tetrahydrofuran (10 mL), and the mixture was stirred at 75 °C for 1 day. After 2 N aqueous hydrochloric acid solution (1 mL) was added, the reaction mixture was stirred at 75 °C for 1 hour. After adjusting the pH between 13 and 14 with 2 N aqueous sodium hydroxide solution, the mixture was extracted three times with dichloromethane, dried over anhydrous sodium sulfate, and concentrated under reduced pressure after filtration to obtain (S)-N1-phenylhexane- 1,2-diamine (116 mg).

¹H-NMR (CDCl₃, 270MHz) δ: 0.92 (3H, t, J = 6.9 Hz), 1.25-1.59 (6H, m), 2.83 (1H, dd, J = 11.9, 8.2 Hz), 2.89-3.03 (1H, m), 3.18 (1H, dd, J = 11.9, 3.6 Hz), 4.10 (1H, br, s), 6.58-6.73 (3H, m), 7.10-7.22 (2H, m).

MS (ESI⁺): 193.23 [M+H]⁺

Using the corresponding starting materials and reactants, the following Reference Example 78-2 was obtained by the same method in Reference Example 78-1, the method described in Step 3-2 and Step 46-1 or a method similar thereto.

**[Table 129]**

| Reference Example | Structure | Equipment Data |
|---|---|---|
| 7 8 - 2 | 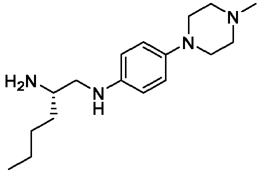 | ¹H-NMR (CDCl₃, 270 MHz) δ: 0.91 (3H, t, J = 6.8 Hz), 1.21-1.56 (6H, m), 2.34 (3H, s), 2.51-2.65 (4H, m), 2.80 (1H, dd, J = 11.9, 8.2 Hz), 2.87-2.99 (1H, m), 3.01-3.10 (4H, m), 3.15 (1H, dd, J = 11.9, 3.6 Hz), 6.60 (2H, dt, J = 9.6, 2.8 Hz), 6.85 (2H, dt, J = 9.6, 2.8 Hz). MS (ESI⁺): 305.44 [M+H]⁺ |

### <Example 17-1>

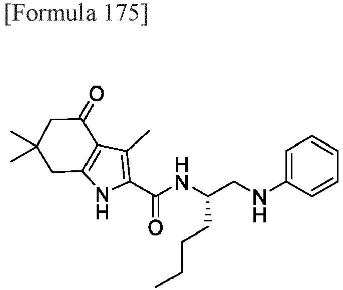

Using 3,6,6-trimethyl-4-oxo-5,7-dihydro-1H-indole-2-carboxylic acid and (S)-N1-phenylhexane-1,2-diamine, (S)-3,6,6-trimethyl-4-oxo-N-(1-(phenylamino)hexan-2-yl)-4,5,6,7-tetrahydro-1H-indole-2-carboxamide (153 mg) was obtained by the same method in Example 6-1.

¹H-NMR (CDCl₃, 270MHz) δ: 0.92 (3H, t, J = 7.1 Hz), 1.08 (6H, s), 1.30-1.51 (4H, m), 1.53-1.82 (2H, m), 2.32 ( 2H, s), 2.54 (3H, s), 2.65 (2H, s), 3.38-3.20 (2H, m), 3.99-4.20 (1H, m), 4.27-4.49 (1H, m), 5.77 (1H, d, J = 8.6 Hz), 6.53-6.76 (3H, m), 7.07-7.22 (2H, m), 9.84 (1H, brs).

MS (ESI⁺): 396.59 [M+H]⁺

Using the corresponding starting materials and reactants, the following Example 17-2 was obtained by the same method in Example 17-1, the method described in step 46-2, or a method similar thereto.

**[Table 130]**

| Example | Structure | Equipment Data |
|---|---|---|
| 1 7 - 2 | 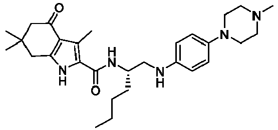 | ¹H-NMR (CDCl₃, 270 MHz) δ: 0.91 (3H, t, J = 6.9 Hz), 1.07 (6H, s), 1.26-1.51 (4H, m), 1.50-1.81 (2H, m), 2.32 (2H, s), 2.35 (3H, s), 2.51-2.69 (9H, m), 2.95-3.12 (4H, m), 3.12-3.39 (2H, m), 4.25-4.42 (1H, m), 5.86 (1H, d, J = 8.6 Hz), 6.60 (2H, d, J = 8.7 Hz), 6.81 (2H, d, J = 8.7 Hz), 10.11 (1H, br s). MS (ESI⁺): 494.71 [M+H]⁺ |

### <Reference example 79-1>

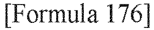

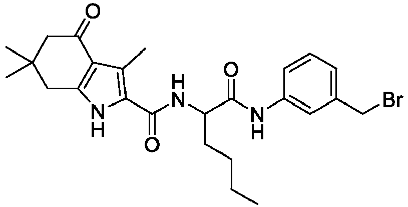

Under argon atmosphere, triphenylphosphine (86.0 mg) and carbon tetrabromide (127 mg) were added to a solution of N-[1-[3-(hydroxymethyl)anilino]-1-oxohexan-2-yl]-3,6,6-trimethyl-4-oxo-5,7-dihydro-1H-indole-2-carboxamide (120 mg) in a tetrahydrofurandichloromethane solution (1.37 mL, 1 : 1) at room temperature, and the mixture was stirred for 7 hours. After the reaction mixture was concentrated under reduced pressure, the residue was purified by silica gel column chromatography (ethyl acetate / hexane = 8 to 66%) to obtain N-[1-[3-(bromomethyl)anilino]-1-oxohexan-2-yl]-3,6,6-trimethyl-4-oxo-5,7-dihydro-1H-indole-2-carboxamide (85.1 mg).

¹H-NMR (DMSO-D₆, 400MHz) δ: 0.88 (3H, t, J = 6.7 Hz), 1.03 (6H, s), 1.29-1.37 (4H, m), 1.70-1.83 (2H, m), 2.23 (2H, s), 2.48 (3H, s), 2.65 (2H, s), 4.55 (1H, td, J = 7.9, 4.9 Hz), 4.68 (2H, s), 7.14 (1H, d, J = 7.9 Hz), 7.30 (1H, t, J = 7.9 Hz), 7.53 (1H, d, J = 7.9 Hz), 7.60 (1H, d, J = 7.9 Hz), 7.75 (1H, s), 10.22 (1H, s), 11.68 (1H, s).

HRMS (ESI⁺): 502.17119 [M+H]⁺

Using the corresponding starting materials and reactants, the following Reference Examples 79-2 to 79-3 were obtained by the same method in Reference Example 79-1, the method described in Step 4-1 or a method similar thereto.

**[Table 131]**

| Reference Example | Structure | Equipment Data |
|---|---|---|
| 7 9 - 2 | 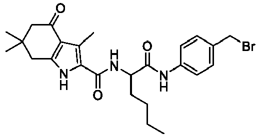 | ¹H-NMR (DMSO-D₆, 400MHz) δ: 0.88 (3H, t, J = 6.7 Hz), 1.03 (6H, s), 1.30-1.37 (4H, m), 1.70-1.82 (2H, m), 2.23 (2H, s), 2.47 (3H, s), 2.65 (2H, s), 4.56 (1H, td, J = 8.6, 4.9 Hz), 4.69 (2H, s), 7.39 (2H, d, J = 8.6 Hz), 7.60 (2H, d, J = 8.6 Hz), 7.78 (1H, d, J = 8.6 Hz), 10.24 (1H, s), 11.68 (1H, s). |

**[Table 132]**

| Reference Example | Structure | Equipment Data |
|---|---|---|
| 7 9 - 3 | 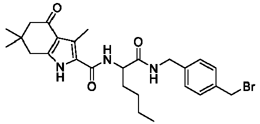 | ¹H-NMR (DMSO-D₆, 400MHz) δ: 0.82-0.88 (3H, m), 1.01 (6H, s), 1.26-1.32 (4H, m), 1.60-1.78 (2H, m), 2.21 (2H, s), 2.46 (3H, s), 2.63 (2H, s), 4.26-4.37 (2H, m), 4.39-4.47 (1H, m), 4.68 (2H, s), 7.22 (1H, d, J = 7.9 Hz), 7.31-7.49 (4H, m), 8.56 (1H, t, J = 5.4 Hz), 11.65 (1H, s). HRMS (ESI⁺):516.18600 [M+H]⁺ |

### <Example 18-1>

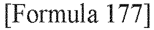

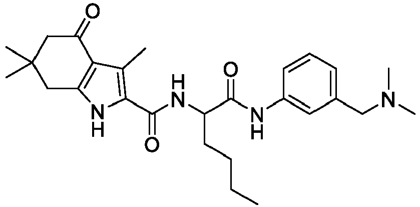

Under an argon atmosphere, dimethylamine (0.047 mL, 2 mol/L tetrahydrofuran solution) and potassium carbonate (12.9 mg) were added to a solution of N-[1-[3-(bromomethyl) anilino]-1-oxohexan-2-yl]-3,6,6-trimethyl-4-oxo-5,7-dihydro-1H-indole-2-carboxamide (42.5 mg) in N, N-dimethylformamide (0.211 mL) and the mixture was stirred at room temperature for 3 hours. After water was added, the reaction mixture was extracted with ethyl acetate. The organic layer was washed with saturated brine, dried over anhydrous sodium sulfate, and concentrated under reduced pressure after filtration of the insoluble material. The residue was purified by amino-silica gel column chromatography (ethyl acetate : methanol = 1 : 0 to 4 : 1), and the resulting solid was washed with diisopropyl ether to obtain N-[1-[3-[(dimethylamino)methyl] anilino]-1-oxohexan-2-yl]-3,6,6-trimethyl-4-oxo-5,7-dihydro-1H-indole-2-carboxamide (14.3 mg).

¹H-NMR (DMSO-D₆, 400MHz) δ: 0.88 (3H, t, J = 6.7 Hz), 1.02 (6H, s), 1.32-1.39 (4H, m), 1.70-1.78 (2H, m), 2.13 (6H, s), 2.23 (2H, s), 2.48 (3H, s), 2.65 (2H, s), 3.34 (2H, s), 4.54 (1H, td, J = 8.6, 4.9 Hz), 6.96 ( 1H, d, J = 7.3 Hz), 7.24 (1H, t, J = 7.3 Hz), 7.52 (1H, d, J = 8.6 Hz), 7.56-7.62 (2H, m), 10.10 (1H, s), 11.71 (1H, s).

HRMS (ESI⁺): 467.30234 [M+H]⁺

Using the corresponding starting materials and reactants, the following Examples 18-2 to 18-14 were obtained by the same method as in Example 18-1, the method described in Step 4-2, or a method similar thereto.

**[Table 133]**

| Example | Structure | Equipment Data |
|---|---|---|
| 1 8 - 2 | 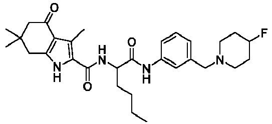 | ¹H-NMR (DMSO-D₆, 400MHz) δ: 0.88 (3H, t, J = 6.7 Hz), 1.02 (6H, s), 1.29-1.37 (4H, m), 1.66-1.82 (6H, m), 2.22 (2H, s), 2.25-2.42 (4H, m), 2.48 (3H, s), 2.65 (2H, s), 3.42 (2H, s), 4.55 (1H, td, J = 8.6, 4.9 Hz), 4.60-4.75 (1H, m), 6.97 (1H, d, J = 7.3 Hz), 7.24 (1H, t, J = 7.3 Hz), 7.52-7.57 (2H, m), 7.62 (1H, d, J = 7.3 Hz), 10.12 (1H, s), 11.72 (1H, s). HRMS (ESI⁺):525.32385 [M+H]⁺ |
| 1 8 - 3 | 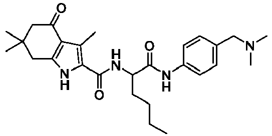 | ¹H-NMR (DMSO-D₆, 400MHz) δ: 0.88 (3H, t, J = 6.7 Hz), 1.02 (6H, s), 1.30-1.37 (4H, m), 1.68-1.82 (2H, m), 2.11 (6H, s), 2.22 (2H, s), 2.48 (3H, s), 2.65 (2H, s), 3.31 (2H, s), 4.56 (1H, td, J = 8.6, 4.9 Hz), 7.21 (2H, d, J = 8.6 Hz), 7.55 (2H, d, J = 8.6 Hz), 7.62 (1H, d, J = 8.6 Hz), 10.12 (1H, s), 11.71 (1H, s). HRMS (ESI⁺):467.30296 [M+H]⁺ |
| 1 8 - 4 | 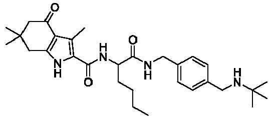 | ¹H-NMR (DMSO-D₆, 400MHz) δ: 0.80-0.95 (3H, m), 1.04 (6H, s), 1.09 (9H, s), 1.27-1.34 (4H, m), 1.62-1.79 (2H, m), 2.24 (2H, s), 2.49 (3H, s), 2.66 (2H, s), 3.63 (2H, s), 4.25 (1H, dd, J = 15.1, 6.1 Hz), 4.31 (1H, dd, J = 15.1, 6.1 Hz), 4.46 (1H, td, J = 7.9, 5.4 Hz), 7.18 (2H, d, J = 8.5 Hz), 7.28 (2H, d, J = 8.5 Hz), 7.46 (1H, d, J = 7.9 Hz), 8.55 (1H, t, J = 6.1 Hz), 11.69 (1H, s). HRMS (ESI⁺):509.34846 [M+H]⁺ |
| 1 8 - 5 | 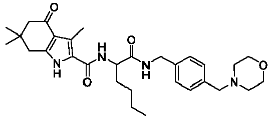 | ¹H-NMR (DMSO-D₆, 400MHz) δ: 0.80-0.90 (3H, m), 1.01 (6H, s), 1.24-1.30 (4H, m), 1.60-1.76 (2H, m), 2.21 (2H, s), 2.28-2.32 (4H, m), 2.46 (3H, s), 2.63 (2H, s), 3.40 (2H, s), 3.53 (4H, t, J = 4.8 Hz), 4.23 (1H, dd, J = 15.1, 6.1 Hz), 4.29 (1H, dd, J = 15.1, 6.1 Hz), 4.43 (1H, td, J = 7.9, 6.1 Hz), 7.19 (2H, d, J = 8.5 Hz), 7.22 (2H, d, J = 8.5 Hz), 7.43 (1H, d, J = 7.9 Hz), 8.53 (1H, t, J = 6.1 Hz), 11.66 (1H, s). HRMS (ESI⁺):523.32816 [M+H]⁺ |

**[Table 134]**

| Example | Structure | Equipment Data |
|---|---|---|
| 1 8 - 6 | 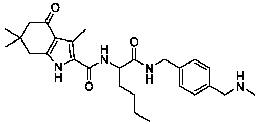 | ¹H-NMR (DMSO-D₆, 400MHz) δ: 0.81-0.87 (3H, m), 1.01 (6H, s), 1.21-1.33 (4H, m), 1.59-1.77 (2H, m), 2.21 (5H, s), 2.46 (3H, s), 2.63 (2H, s), 3.57 (2H, s), 4.23 (1H, dd, J = 15.1, 6.1 Hz), 4.29 (1H, dd, J = 15.1, 6.1 Hz), 4.43 (1H, td, J = 8.5, 6.1 Hz), 7.17 (2H, d, J = 8.5 Hz), 7.20-7.27 (3H, m), 7.44 (1H, d, J = 8.5 Hz), 8.50-8.56 (1H, m), 11.67 (1H, brs). HRMS (ESI⁺):467.30283 [M+H]⁺ |
| 1 8 - 7 | 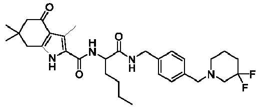 | ¹H-NMR (DMSO-D₆, 400MHz) δ: 0.80-0.90 (3H, m), 1.01 (6H, s), 1.25-1.29 (4H, m), 1.59-1.65 (3H, m), 1.70-1.76 (1H, m), 1.78-1.88 (2H, m), 2.21 (2H, s), 2.30-2.40 (2H, m), 2.46 (3H, s), 2.48-2.60 (2H, m), 2.63 (2H, s), 3.52 (2H, s), 4.24 (1H, dd, J = 15.1, 6.1 Hz), 4.30 (1H, dd, J = 15.1, 6.1 Hz), 4.43 (1H, td, J = 7.9, 6.1 Hz), 7.15-7.25 (4H, m), 7.44 (1H, d, J = 7.9 Hz), 8.54 (1H, t, J = 6.1 Hz), 11.67 (1H, s). HRMS (ESI⁺):557.33032 [M+H]⁺ |
| 1 8 - 8 | 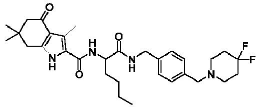 | ¹H-NMR (DMSO-D₆, 400MHz) δ: 0.84 (3H, t, J = 6.7 Hz), 1.01 (6H, s), 1.24-1.31 (4H, m), 1.61-1.74 (2H, m), 1.86-1.98 (4H, m), 2.21 (2H, s), 2.40-2.45 (4H, m), 2.46 (3H, s), 2.63 (2H, s), 3.49 (2H, s), 4.24 (1H, dd, J = 15.4, 6.4 Hz), 4.29 (1H, dd, J = 15.4, 6.4 Hz), 4.43 (1H, td, J = 8.5, 6.1 Hz), 7.19 (2H, d, J = 7.9 Hz), 7.23 (2H, d, J = 7.9 Hz), 7.44 (1H, d, J = 7.9 Hz), 8.54 (1H, t, J = 6.1 Hz), 11.66 (1H, s). HRMS (ESI⁺):557.32988 [M+H]⁺ |
| 1 8 - 9 | 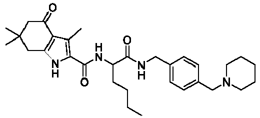 | ¹H-NMR (DMSO-D₆, 400MHz) δ: 0.83 (3H, t, J = 6.7 Hz), 1.01 (6H, s), 1.24-1.29 (4H, m), 1.33-1.38 (2H, m), 1.42-1.48 (4H, m), 1.62-1.73 (2H, m), 2.21 (2H, s), 2.23-2.29 (4H, m), 2.46 (3H, s), 2.63 (2H, s), 3.36 (2H, s), 4.23 (1H, dd, J = 15.1, 6.1 Hz), 4.29 (1H, dd, J = 15.1, 6.1 Hz), 4.43 (1H, td, J = 7.9, 6.1 Hz), 7.17 (2H, d, J = 8.5 Hz), 7.20 (2H, d, J = 8.5 Hz), 7.43 (1H, d, J = 7.9 Hz), 8.53 (1H, t, J = 6.1 Hz), 11.66 (1H, s). HRMS (ESI⁺):521.34833 [M+H]⁺ |

**[Table 135]**

| Example | Structure | Equipment Data |
|---|---|---|
| 1 8 - 1 0 | 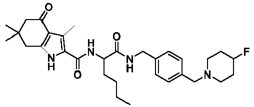 | ¹H-NMR (DMSO-D₆, 400MHz) δ: 0.80-0.88 (3H, m), 1.01 (6H, s), 1.22-1.32 (4H, m), 1.57-1.89 (6H, m), 2.18-2.29 (4H, m), 2.42-2.47 (5H, m), 2.63 (2H, s), 3.41 (2H, s), 4.23-4.33 (2H, m), 4.39-4.47 (1H, m), 4.56-4.74 (1H, m), 7.16-7.25 (4H, m), 7.43 (1H, d, J = 7.3 Hz), 8.53 (1H, t, J = 6.1 Hz), 11.66 (1H, brs). HRMS (ESI⁺):539.33914 [M+H]⁺ |
| 1 8 - 1 1 | 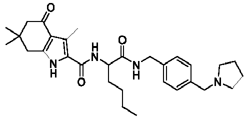 | ¹H-NMR (DMSO-D₆, 400MHz) δ: 0.84 (3H, t, J = 6.7 Hz), 1.01 (6H, s), 1.22-1.31 (4H, m), 1.60-1.79 (6H, m), 2.21 (2H, s), 2.35-2.39 (4H, m), 2.46 (3H, s), 2.63 (2H, s), 3.51 (2H, s), 4.23 (1H, dd, J = 15.1, 6.1 Hz), 4.29 (1H, dd, J = 15.1, 6.1 Hz), 4.43 (1H, td, J = 7.9, 5.4 Hz), 7.17 (2H, d, J = 7.9 Hz), 7.21 (2H, d, J = 7.9 Hz), 7.43 (1H, d, J = 7.9 Hz), 8.52 (1H, t, J = 5.4 Hz), 11.66 (1H, s). HRMS (ESI⁺):507.33361 [M+H]⁺ |
| 1 8 - 1 2 | 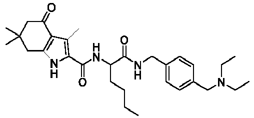 | ¹H-NMR (DMSO-D₆, 400MHz) δ: 0.84 (3H, t, J = 6.7 Hz), 0.94 (6H, t, J = 7.3 Hz), 1.01 (6H, s), 1.24-1.30 (4H, m), 1.61-1.75 (2H, m), 2.21 (2H, s), 2.41 (4H, q, J = 7.3 Hz), 2.46 (3H, s), 2.63 (2H, s), 3.46 (2H, s), 4.23 (1H, dd, J = 15.1, 6.1 Hz), 4.29 (1H, dd, J = 15.1, 6.1 Hz), 4.43 (1H, td, J = 7.9, 6.1 Hz), 7.17 (2H, d, J = 7.9 Hz), 7.22 (2H, d, J = 7.9 Hz), 7.43 (1H, d, J = 7.9 Hz), 8.52 (1H, t, J = 6.1 Hz), 11.66 (1H, s). HRMS (ESI⁺):509.34957 [M+H]⁺ |
| 1 8 - 1 3 | 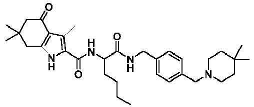 | ¹H-NMR (DMSO-D₆, 400MHz) δ: 0.80-0.95 (3H, m), 0.87 (6H, s), 1.02 (6H, s), 1.26-1.31 (8H, m), 1.61-1.78 (2H, m), 2.22 (2H, s), 2.27-2.30 (4H, m), 2.47 (3H, s), 2.64 (2H, s), 3.41 (2H, s), 4.24 (1H, dd, J = 15.1, 6.1 Hz), 4.30 (1H, dd, J = 15.1, 6.1 Hz), 4.44 (1H, td, J = 7.9, 5.4 Hz), 7.18 (2H, d, J = 8.5 Hz), 7.21 (2H, d, J = 8.5 Hz), 7.43 (1H, d, J = 7.9 Hz), 8.53 (1H, t, J = 6.1 Hz), 11.67 (1H, s). HRMS (ESI⁺):549.38145 [M+H]⁺ |

**[Table 136]**

| Example | Structure | Equipment Data |
|---|---|---|
| 1 8 - 1 4 | 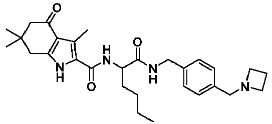 | ¹H-NMR (DMSO-D₆, 400MHz) δ: 0.85 (3H, t, J = 6.7 Hz), 1.02 (6H, s), 1.26-1.31 (4H, m), 1.61-1.77 (2H, m), 1.91-1.98 (2H, m), 2.22 (2H, s), 2.47 (3H, s), 2.64 (2H, s), 3.07 (4H, t, J = 7.3 Hz), 3.46 (2H, s), 4.23 (1H, dd, J = 15.1, 6.1 Hz), 4.29 (1H, dd, J = 15.1, 6.1 Hz), 4.44 (1H, td, J = 7.9, 5.4 Hz), 7.17 (4H, s), 7.43 (1H, d, J = 7.9 Hz), 8.51 (1H, d, J = 6.1 Hz), 11.66 (1H, s). HRMS (ESI⁺):493.31799 [M+H]⁺ |

### <Reference example 80>

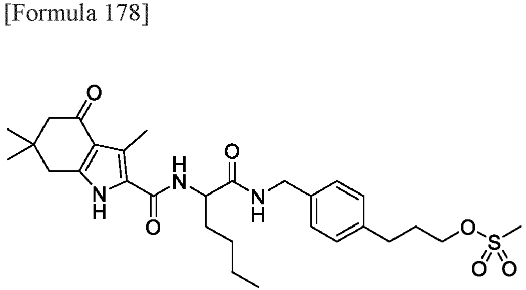

Under an argon atmosphere, triethylamine (21.7 µL) and methanesulfonyl chloride (9.67 µL) were added to a solution of N-[1-[[4-(3-hydroxypropyl)phenyl]methylamino]-1-oxohexan-2-yl]-3,6,6-trimethyl-4-oxo-5,7-dihydro-1H-indole-2-carboxamide (50.0 mg) in dichloromethane (1.00 mL) at 0 °C and the mixture was stirred for 1 hour. The reaction mixture was purified by silica gel column chromatography (hexane : ethyl acetate = 3 : 1 to 0 : 1) to obtain 3-[4-[[2-[(3,6,6-trimethyl-4-oxo-5,7-dihydro-1H-indole-2-carbonyl)amino]hexanoylamino]methyl]phenyl]propyl methanesulfonate (50.0 mg).

¹H-NMR (DMSO-D₆, 400MHz) δ: 0.84 (3H, t, J = 7.0 Hz), 1.01 (6H, s), 1.21-1.35 (4H, m), 1.57-1.78 (2H, m), 1.88-1.97 (2H, m), 2.20 (2H, s), 2.46 (3H, s), 2.59-2.66 (4H, m), 3.16 (3H, s), 4.17 (2H, t, J = 6.4 Hz), 4.19-4.31 (2H, m), 4.38-4.47 (1H, m), 7.13-7.19 (4H, m), 7.47-7.55 (1H, m), 8.48-8.56 (1H, m), 11.75 (1H, s).

MS (ESI⁺): 560.3 [M+H]⁺

### <Reference example 81>

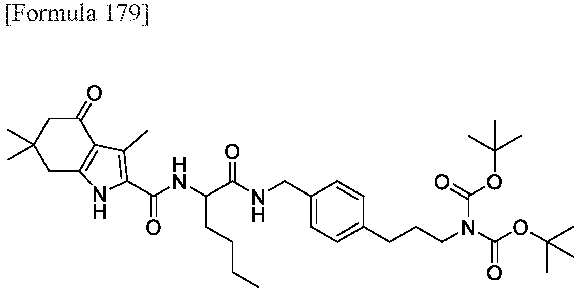

Under an argon atmosphere, potassium carbonate (37.0 mg) was added to a solution of 3-[4-[[2-[(3,6,6-trimethyl-4-oxo-5,7-dihydro-lH-indole-2-carbonyl)amino] hexanoylamino]methyl]phenyl]propyl methanesulfonate (50.0 mg) and di-tert-butyl iminodicarboxylic acid (58.2 mg) in N, N-dimethylformamide (1.00 mL) at room temperature and the mixture was stirred at 50 °C for 8 hours. The reaction mixture was added to water, extracted twice with ethyl acetate, and the combined organic layer was washed with water and saturated brine, dried over anhydrous sodium sulfate, and concentrated under reduced pressure after filtration of the insoluble material. The residue was purified by silica gel column chromatography (hexane : ethyl acetate = 9 : 1 to 0 : 1) to obtain tert-butyl N-[(2-methylpropan-2-yl)oxycarbonyl]-N-[3-[4-[[2-[(3,6,6-trimethyl-4-oxo-5,7-dihydro-1H-indole-2-carbonyl)amino]hexanoylamino]methyl]phenyl]propyl]carbamate (46.0 mg).

¹H-NMR (DMSO-D₆, 400MHz) δ: 0.79-0.89 (3H, m), 1.01 (6H, s), 1.20-1.31 (6H, m), 1.40 (18H, s), 1.70-1.79 (2H, m), 2.20 (2H, s), 2.46 (3H, s), 2.50-2.54 (2H, m), 2.62 (2H, s), 3.40-3.48 (2H, m), 4.16-4.31 (2H, m) , 4.38-4.48 (1H, m), 7.08-7.18 (4H, m), 7.46 (1H, d, J = 7.9 Hz), 8.52 (1H, t, J = 6.1 Hz), 11.70 (1H, s).

MS (ESI⁺): 681.4 [M+H]⁺

### <Example 19>

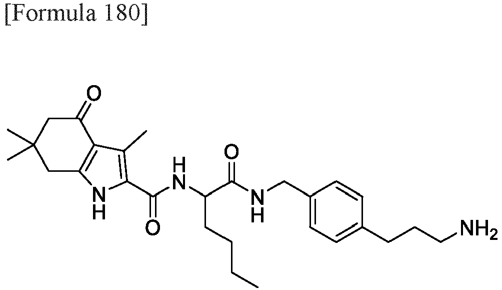

Trifluoroacetic acid (0.250 mL) was added to a solution of tert-butyl N-[(2-methylpropan-2-yl)oxycarbonyl]-N-[3-[4-[[2-[(3,6,6-trimethyl-4-oxo-5,7-dihydro-1H-indole-2-carbonyl)amino]hexanoylamino]methyl]phenyl]propyl]carbamate (40.0 mg) in dichloromethane (0.250 mL) at room temperature and the mixture was stirred for 2 hours. After the reaction mixture was concentrated under reduced pressure, the residue was purified by amino-silica gel column chromatography (ethyl acetate : methanol = 1 : 0 to 4 : 1) to obtain N-[1-[[4-(3-aminopropyl)phenyl]methylamino]-1-oxohexan-2-yl]-3,6,6-trimethyl-4-oxo-5,7-dihydro-1H-indole-2-carboxamide (29.0 mg).

¹H-NMR (DMSO-D₆, 400MHz) δ: 0.83-0.91 (3H, m), 1.04 (6H, s), 1.23-1.37 (4H, m), 1.56-1.83 (4H, m), 2.24 (2H, s), 2.49 (3H, s), 2.53-2.60 (4H, m), 2.66 (2H, s), 4.20-4.34 (2H, m), 4.42-4.50 (1H, m), 7.12-7.20 (4H, m), 7.52 (1H, d, J = 7.9 Hz), 8.54 (1H, t, J = 5.8 Hz).

HRMS (ESI⁺): 481.31785 [M+H]⁺

### <Reference example 82-1>

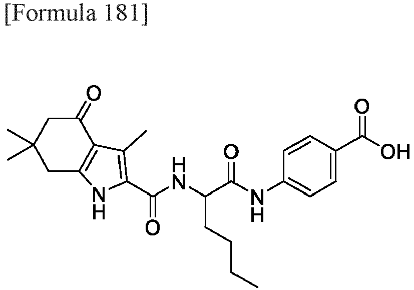

Under an argon atmosphere, lithium hydroxide monohydrate (60.0 mg) was added to a solution of methyl 4-[2-[(3,6,6-trimethyl-4-oxo-5,7-dihydro-1H-indole-2-carbonyl) amino]hexanoylamino]benzoate (134 mg) in a tetrahydrofuran-methanol-water (3 : 3: 1) solution (1.43 mL), and the mixture was stirred at room temperature for 24 hours. The reaction mixture was concentrated under reduced pressure and neutralized with 1 mol/L hydrochloric acid. The resulting solid was collected by filtration to obtain 4-[2-[(3,6,6-trimethyl-4-oxo-5,7-dihydro-1H-indole-2-carbonyl)amino]hexanoylamino]benzoic acid (122 mg).

¹H-NMR (DMSO-D₆, 400MHz) δ: 0.88 (3H, t, J = 6.7 Hz), 1.02 (6H, s), 1.30-1.44 (4H, m), 1.70-1.84 (2H, m), 2.23 (2H, s), 2.47 (3H, s), 2.65 (2H, s), 4.56 (1H, td, J = 8.6, 4.9 Hz), 7.68 (1H, d, J = 8.6 Hz), 7.74 (2H, d, J = 8.6 Hz), 7.90 (2H, d, J = 8.6 Hz), 10.46 (1H, s), 11.69 (1H, s), 12.71 (1H, s).

HRMS (ESI⁺): 454.23456 [M+H]⁺

Using the corresponding starting materials and reactants, the following Reference Example 82-2 was obtained by the same method in Reference Example 82-1, the method described in Step 5-1 or a method similar thereto.

**[Table 137]**

| Reference Example | Structure | Equipment Data |
|---|---|---|
| 8 2 - 2 | 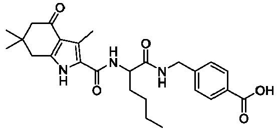 | ¹H-NMR (DMSO-D₆, 400MHz) δ: 0.84 (3H, t, J = 6.7 Hz), 1.01 (6H, s), 1.25-1.34 (4H, m), 1.64-1.78 (2H, m), 2.21 (2H, s), 2.47 (3H, s), 2.63 (2H, s), 4.35 (2H, dd, J = 6.1, 1.8 Hz), 4.43 (1H, td, J = 7.9, 5.4 Hz), 7.35 (2H, d, J = 7.9 Hz), 7.51 (1H, d, J = 5.4 Hz), 7.86 (2H, d, J = 7.9 Hz), 8.64 (1H, t, J = 6.1 Hz), 11.71 (1H, s), 12.88 (1H, brs). HRMS (ESI⁺):468.25026 [M+H]⁺ |

### <Example 20-1>

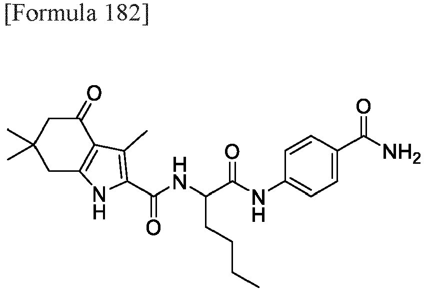

Under an argon atmosphere, ammonium chloride (29.5 mg), O-(7-azabenzotriazol-1-yl)-N,N,N',N'-tetramethyluronium hexafluorophosphate (50.2 mg) and N,N-diisopropylethylamine (0.094 mL) were added to a solution of 4-[2-[(3,6,6-trimethyl-4-oxo-5,7-dihydro-1H-indole-2-carbonyl)amino]hexanoylamino]benzoic acid (50.0 mg) in N,N-dimethylformamide (0.551 mL) and the mixture was stirred at room temperature for 8 hours. After water was added, the reaction mixture was extracted with ethyl acetate. The organic layer was washed with saturated brine, dried over anhydrous sodium sulfate, and concentrated under reduced pressure after filtration of the insoluble material. The residue was purified by silica gel column chromatography (ethyl acetate / hexane = 25% to 100% and methanol / ethyl acetate = 20%), and the resulting solid was washed with diisopropyl ether to obtain N-[1-(4-carbamoylanilino)-1-oxohexan-2-yl]-3,6,6-trimethyl-4-oxo-5,7-dihydro-1H-indole-2-carboxamide (34.7 mg).

¹H-NMR (DMSO-D₆, 400MHz) δ: 0.88 (3H, t, J = 6.7 Hz), 1.03 (6H, s), 1.32-1.42 (4H, m), 1.71-1.82 (2H, m), 2.23 (2H, s), 2.48 (3H, s), 2.65 (2H, s), 4.57 (1H, td, J = 8.6, 4.9 Hz), 7.25 (1H, br s), 7.67 (2H, d, J = 8.6 Hz), 7.84 (2H, d, J = 8.6 Hz), 7.95 (2H, br s), 10.36 (1H, s), 11.69 (1H, s).

HRMS (ESI⁺): 453.24986 [M+H]⁺

Using the corresponding starting materials and reactants, the following Example 20-2 was obtained by the same method as in Example 20-1, the method described in Step 5-2, or a method similar thereto.

**[Table 138]**

| Example | Structure | Equipment Data |
|---|---|---|
| 2 0 - 2 | 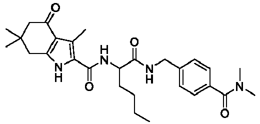 | ¹H-NMR (DMSO-D₆, 400MHz) δ: 0.84 (3H, t, J = 6.7 Hz), 1.01 (6H, s), 1.25-1.32 (4H, m), 1.61-1.78 (2H, m), 2.21 (2H, s), 2.46 (3H, s), 2.63 (2H, s), 2.88 (3H, s), 2.95 (3H, s), 4.29 (1H, dd, J = 15.1, 5.4 Hz), 4.35 (1H, dd, J = 15.1, 5.4 Hz), 4.44 (1H, td, J = 7.9, 5.4 Hz), 7.29 (2H, d, J = 8.5 Hz), 7.33 (2H, d, J = 8.5 Hz), 7.46 (1H, d, J = 7.9 Hz), 8.61 (1H, t, J = 5.4 Hz), 11.65 (1H, s). |
| | | HRMS (ESI⁺):495.29726 [M+H]⁺ |

### <Example 21>

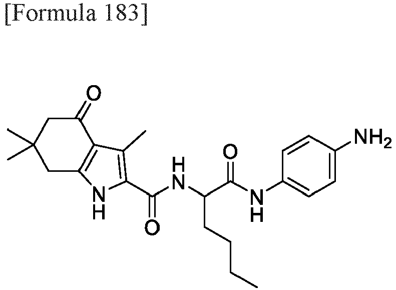

Under an argon atmosphere, 10% palladium carbon (3.00 mg) was added to a solution of 3,6,6-trimethyl-N-[l-(4-nitroanilino)-1-oxohexan-2-yl]-4-oxo-5,7-dihydro-1H-indole-2-carboxamide in methanol (0.500 mL) and tetrahydrofuran (0.500 mL) at room temperature and the mixture was stirred for 2 hours under a hydrogen atmosphere. After replacing with an argon atmosphere, the reaction mixture was filtered through Celite, and the filtrate was concentrated under reduced pressure to obtain N-[1-(4-aminoanilino)-1-oxohexan-2-yl]-3,6,6-trimethyl-4-oxo-5,7-dihydro-1H-indole-2-carboxamide (27.0 mg).

¹H-NMR (DMSO-D₆, 400MHz) δ: 0.88 (3H, t, J = 7.0 Hz), 1.04 (6H, s), 1.24-1.43 (4H, m), 1.62-1.85 (2H, m), 2.23 (2H, s), 2.49 (3H, s), 2.65 (2H, s), 4.50-4.58 (1H, m), 4.87 (2H, s), 6.51 (2H, d, J = 8.5 Hz), 7.24 (2H, d, J = 8.5 Hz), 7.54 (1H, d, J = 7.9 Hz), 9.71 (1H, s), 11.75 (1H, s).

HRMS (ESI⁺): 425.25574 [M+H]⁺

### <Example 22-1>

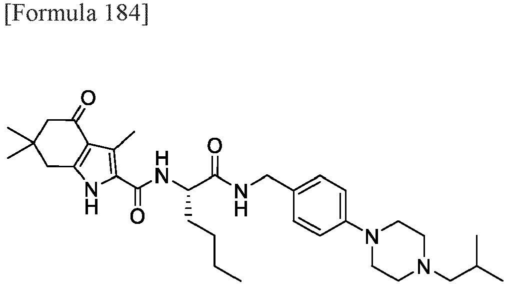

Sodium triacetoxyborohydride (51 mg) was added to a solution of (S)-3,6,6-trimethyl-4-oxo-N-(1-oxo-1- ((4-(piperazin-1-yl)benzyl)amino)hexan-2-yl)-4,5,6,7-tetrahydro-1H-indole-2-carboxamide (62 mg), isobutyraldehyde (110 µL), N,N-diisopropylethylamine (72 µL) in dichloromethane (630 µL) under ice cooling condition and the mixture was warmed to room temperature, and stirred overnight. After saturated aqueous sodium hydrogen carbonate solution (2 mL) and 2 M aqueous potassium carbonate solution (1 mL) were added, the reaction mixture was extracted twice with dichloromethane. The combined organic layer was dried over sodium sulfate, filtered, and concentrated under reduced pressure. The residue was purified by amino-silica gel column chromatography (dichloromethane-methanol) to obtain (S)-N-(1-((4-(4-isopropylpiperazin-1-yl)benzyl)amino)-1-oxohexan-2-yl)-3,6,6-trimethyl-4-oxo-4,5,6,7-tetrahydro-1H-indole-2-carboxamide (28.2 mg).

¹H-NMR (399MHz, CDCl₃) δ: 0.88 (3H, t, J = 7.3 Hz), 0.93 (6H, d, J = 6.4 Hz), 1.09 (6H, s), 1.28-1.40 (4H, m) , 1.63-1.99 (3H, m), 2.17 (2H, d, J = 7.8 Hz), 2.33 (2H, s), 2.53-2.60 (4H, m), 2.62 (2H, s), 2.66 (3H, s), 3.18 (4H, t, J = 4.8 Hz), 4.32 (1H, dd, J = 14.6, 5.5 Hz), 4.42 (1H, dd, J = 14.6, 5.7 Hz), 4.55 (1H, q, J = 6.9 Hz), 6.27 (1H, s), 6.57 (1H, d, J = 7.8 Hz), 6.85 (2H, d, J = 8.7 Hz), 7.15 (2H, d, J = 8.7 Hz), 9.35 (1H, s).

MS (ESI⁺): 565.23 [M+H]⁺

Using the corresponding starting materials and reactants, the following Examples 22-2 to 22-5 were obtained by the same method in Example 22-1, the method described in Step 7-1 or a method similar thereto.

**[Table 139]**

| Example | Structure | Equipment Data |
|---|---|---|
| 2 2 - 2 | 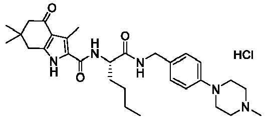 | ¹H-NMR (399 MHz, DMSO-D₆) δ: 0.85 (3H, t, J= 6.9 Hz), 1.02 (6H, s), 1.23-1.37 (4H, m), 1.57-1.80 (2H, m), 2.22 (2H, s), 2.47 (3H, s), 2.64 (2H, s), 2.79 (3H, d, J = 4.6 Hz), 2.98-3.22 (4H, m), 3.46 (2H, d, J = 11.4 Hz), 3.77 (2H, d, J = 12.3 Hz), 4.17 (1H, dd, J = 15.1, 5.9 Hz), 4.23 (1H dd, J = 15.1, 5.9 Hz,), 4.42 (1H, td, J = 8.2, 5.5 Hz), 6.94 (2H, d, J = 8.7 Hz), 7.15 (2H, d, J = 8.7 Hz), 7.59 (1H, d, J = 8.2 Hz), 8.49 (1H, t, J = 5.9 Hz), 10.80 (1H, brs), 11.87 (1H, s). |
| | | MS (ESI⁺): 523.04 [M+H]⁺ |
| 2 2 - 3 | 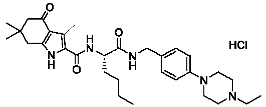 | ¹H-NMR (399 MHz, DMSO-D₆) δ: 0.85 (3H, t, J = 6.6 Hz), 1.02 (6H, s), 1.23-1.36 (7H, m), 1.58-1.77 (2H, m), 2.22 (2H, s), 2.47 (3H, s), 2.64 (2H, s), 2.99-3.21 (6H, m), 3.48-3.58 (2H, m), 3.74-3.81 (2H, m), 4.12-4.28 (2H, m), 4.36-4.51 (1H, m), 6.94 (2H, d, J = 8.7 Hz), 7.15 (2H, d, J = 8.7 Hz), 7.62 (1H, d, J = 7.3 Hz), 8.49 (1H, t, J = 5.9 Hz), 10.83 (1H, s), 11.90 (1H, s). |
| | | MS (ESI⁺): 537.09 [M+H]⁺ |
| 2 2 - 4 | 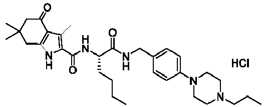 | ¹H-NMR (399 MHz, DMSO-D₆) δ: 0.85 (3H, t, J = 6.9 Hz), 0.92 (3H, t, J = 7.3 Hz), 1.02 (6H, s), 1.28-1.30 (4H, m), 1.60-1.80 (4H, m), 2.22 (2H, s), 2.47 (3H, s), 2.64 (2H, s), 3.00-3.16 (6H, m), 3.52 (2H, d, J = 5.9 Hz), 3.76 (2H, d, J = 10.1 Hz), 4.15-4.26 (2H, m), 4.42 (1H, dd, J = 13.7, 8.2 Hz), 6.94 (2H, d, J = 8.7 Hz), 7.15 (2H, d, J = 8.7 Hz), 7.56 (1H, dd, J = 13.0, 8.0 Hz), 8.49 (1H, t, J = 5.9 Hz), 10.38-10.74 (1H, brs), 11.78-11.89 (1H, brs). |
| | | MS (ESI⁺): 551.12 [M+H]⁺ |

**[Table 140]**

| Example | Structure | Equipment Data |
|---|---|---|
| 2 2 - 5 | 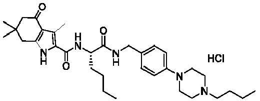 | ¹H-NMR (399 MHz, DMSO-D₆) δ: 0.85 (3H, t, J = 6.9 Hz), 0.92 (3H, t, J = 7.3 Hz), 1.02 (6H, s), 1.21-1.39 (6H, m), 1.58-1.79 (4H, m), 2.22 (2H, s), 2.47 (3H, s), 2.64 (2H, s), 3.14-3.04 (6H, m), 3.46-3.59 (2H, m), 3.76 (2H, d, J = 9.6 Hz), 4.26-4.15 (2H, m), 4.42 (1H, dd, J = 13.2, 8.2 Hz), 6.94 (2H, d, J = 8.7 Hz), 7.15 (2H, d, J = 8.7 Hz), 7.50-7.65 (1H, m), 8.49 (1H, t, J = 5.9 Hz), 10.40-10.85 (1H, brs), 11.78-11.90 (1H, brs). |
| | | MS (ESI⁺): 565.14 [M+H]⁺ |

### <Reference example 83>

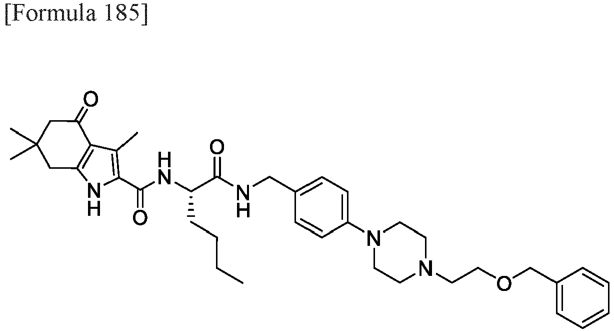

Sodium triacetoxyborohydride (153 mg) was added to a solution of (S)-3,6,6-trimethyl-4-oxo-N-(1-oxo-1-((4-(piperazin-1-yl)benzyl)amino)hexan-2-yl)-4,5,6,7-tetrahydro-1H-indole-2-carboxamide (187 mg), 2-(benzyloxy)acetaldehyde (68 mg), N,N-diisopropylethylamine (10.5 µL) in dichloromethane (1.5 mL) under ice cooling condition, and the mixture was warmed to room temperature and stirred overnight. After saturated aqueous sodium hydrogen carbonate solution (2 mL) and 2 M aqueous potassium carbonate solution (2 mL) were added, the reaction mixture was extracted twice with dichloromethane. The combined organic layer was dried over sodium sulfate, filtered, and concentrated under reduced pressure. The residue was purified by amino-silica gel column chromatography (dichloromethane-methanol) to obtain (S)-N-(1-((4-(4-(2-(benzyloxy)ethyl)piperazin-1-yl)benzyl)amino)-1-oxohexan-2-yl)-3,6,6-trimethyl-4-oxo-4,5,6,7-tetrahydro-1H-indole-2-carboxamide (177.9 mg).

¹H-NMR (399MHz, CDCl₃) δ: 0.88 (3H, t, J = 7.2 Hz), 1.09 (6H, s), 1.26-1.38 (4H, m), 1.63-1.80 (1H, m), 1.88-2.00 (1H, m), 2.33 (2H, s), 2.61 (2H, t, J = 5.3 Hz), 2.62 (2H, s), 2.65-2.70 (9H, m), 3.19 (4H, t, J = 5.2 Hz), 3.67 (2H, t, J = 6.4 Hz), 4.31 (1H, dd, J = 14.6, 4.8 Hz), 4.42 (1H, dd, J = 14.6, 4.8 Hz), 4.5-4.56 (1H, 1H, m), 6.20 (1H, m), 6.55 (1H, d, J = 8.0 Hz), 6.86 (2H, d, J = 8.8 Hz), 7.15 (2H, d, J = 8.8 Hz), 7.35 (5H, m), 9.30 (1H brs).

MS (ESI⁺): 642.18 [M+H]⁺

### <Example 23>

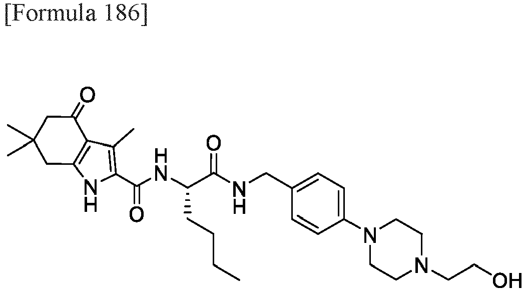

A solution of (S)-N-(1-((4-(4-(2-(benzyloxy)ethyl)piperazin-1-yl)benzyl)amino)-1-oxohexan-2-yl)-3,6,6-trimethyl-4-oxo-4,5,6,7-tetrahydro-1H-indole-2-carboxamide (154 mg), hydrous 10% palladium carbon (containing 50% water) (150 mg), trifluoroacetic acid (55 µL) in methanol (40 mL) was stirred at room temperature for 2 days under a hydrogen atmosphere. The reaction mixture was filtered through Celite, and the filtrate was concentrated under reduced pressure, and dichloromethane-saturated aqueous sodium hydrogen carbonate solution was added to the residue. The aqueous layer was extracted twice, and the combined organic layer was dried over sodium sulfate, filtered, and concentrated under reduced pressure. The residue was purified by silica gel column chromatography (dichloromethane-methanol) to obtain (S)-N-(1-((4-(4-(2-hydroxyethyl)piperazin-1-yl)benzyl)amino)-1-oxohexan-2-yl)-3,6,6-trimethyl-4-oxo-4,5,6,7-tetrahydro-1H-indole-2-carboxamide (58.3 mg).

¹H-NMR (399MHz, CDCl₃) δ: 0.88 (3H, t, J = 7.1 Hz), 1.08 (6H, s), 1.29-1.38 (4H, m), 1.73 (1H, td, J = 14.6, 7.2 Hz), 1.88-2.00 (1H, m), 2.32 (2H, s), 2.61 (2H, t, J = 5.3 Hz), 2.61 (2H, s), 2.65 (3H, s), 2.67 (4H, t, J = 4.8 Hz), 3.17 (4H, t, J = 5.0 Hz), 3.67 (2H, t, J = 5.3 Hz), 4.31 (1H, dd, J = 14.6, 5.5 Hz), 4.42 (1H, dd, J = 14.6, 5.5 Hz), 4.56 (1H, dt, J = 6.9, 6.9 Hz), 6.39 (1H, t, J = 5.5 Hz), 6.63 (1H, d, J = 7.8 Hz), 6.85 (2H, d, J = 8.7 Hz), 7.15 (2H, d, J = 8.7 Hz), 9.55 (1H, brs).

MS (ESI⁺): 552.13 [M+H]⁺

### <Example 24-1>

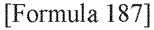

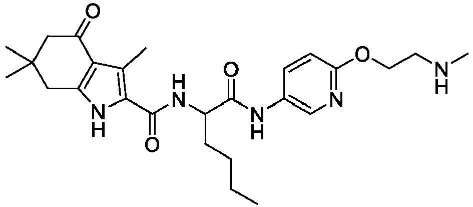

Under an argon atmosphere, trifluoroacetic acid (1.41 mL) was added to a solution of tert-butyl N-methyl-N-[2-[5-[2-[(3,6,6-trimethyl-4-oxo-5,7-dihydro-1H-indole-2-carbonyl)amino]hexanoylamino]pyridin-2-yl]oxyethyl]carbamate (330 mg) in dichloromethane (1.41 mL) and the mixture was stirred at room temperature for 2 hours and then concentrated. After saturated aqueous sodium hydrogen carbonate solution was added, the reaction mixture was extracted with ethyl acetate. The organic layer was washed with saturated brine, dried over anhydrous sodium sulfate, and concentrated after filtration to obtain 3,6,6-trimethyl-N-[1-[[6-[2-(methylamino)ethoxy]pyridin-3-yl]amino]-1-oxohexan-2-yl]- 4-oxo-5,7-dihydro-1H-indole-2-carboxamide (210 mg).

¹H-NMR (DMSO-D₆, 400MHz) δ: 0.87 (3H, t, J = 6.7 Hz), 1.01 (6H, s), 1.25-1.42 (4H, m), 1.65-1.81 (2H, m), 2.21 (2H, s), 2.30 (3H, s), 2.46 (3H, s), 2.63 (2H, s), 2.77 (2H, t, J = 5.8 Hz), 4.22 (2H, t, J = 5.8 Hz), 4.53 (1H, td, J = 8.6, 5.5 Hz), 6.77 (1H, d, J = 8.6 Hz), 7.74 (1H, brs), 7.90 (1H, dd, J = 8.6, 2.4 Hz), 8.35 (1H, d, J = 2.4 Hz), 10.20 (1H, s), 11.80 (1H, brs).

HRMS (ESI⁺): 484.29239 [M+H]⁺

Using the corresponding starting materials and reactants, the following Examples 24-2 to 24-5 were obtained by the same method in Example 24-1, the method described in Step 8-1, or a method similar thereto.

**[Table 141]**

| Example | Structure | Equipment Data |
|---|---|---|
| 2 4 - 2 | 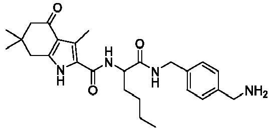 | ¹H-NMR (DMSO-D₆, 400 MHz) δ: 0.80-0.90 (3H, m), 1.01 (6H, s), 1.21-1.33 (4H, m), 1.58-1.77 (2H, m), 2.21 (2H, s), 2.46 (3H, s), 2.63 (2H, s), 3.31 (2H, brs), 3.66 (2H, s), 4.22 (1H, dd, J = 16.3, 5.4 Hz), 4.28 (1H, dd, J = 16.3, 5.4 Hz), 4.39-4.47 (1H, m), 7.16 (2H, d, J = 7.9 Hz), 7.24 (2H, d, J = 7.9 Hz), 7.44 (1H, d, J = 7.9 Hz), 8.52 (1H, t, J = 5.4 Hz), 11.70 (1H, brs). |
| | | HRMS (ESI⁺):453.28691 [M+H]⁺ |
| 2 4 - 3 | 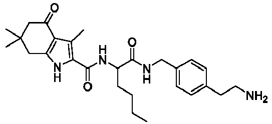 | ¹H-NMR (DMSO-D₆, 400 MHz) δ: 0.84 (3H, t, J = 6.7 Hz), 1.01 (6H, s), 1.22-1.31 (4H, m), 1.59-1.76 (2H, m), 2.21 (2H, s), 2.46 (3H, s), 2.56-2.61 (2H, m), 2.62 (2H, s), 2.68-2.73 (2H, m), 4.21 (1H, dd, J = 15.1, 5.4 Hz), 4.27 (1H, dd, J = 15.1, 5.4 Hz), 4.43 (1H, td, J = 7.9, 5.4 Hz), 7.11 (2H, d, J = 8.5 Hz), 7.15 (2H, d, J = 8.5 Hz), 7.45 (1H, d, J = 7.9 Hz), 8.51 (1H, t, J = 5.4 Hz). |
| | | HRMS (ESI⁺):467.30219 [M+H]⁺ |
| 2 4 - 4 | 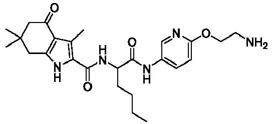 | ¹H-NMR (DMSO-D₆, 400 MHz) δ: 0.87 (3H, t, J = 6.7 Hz), 1.01 (6H, s), 1.26-1.41 (4H, m), 1.66-1.80 (2H, m), 2.21 (2H, s), 2.46 (3H, s), 2.63 (2H, s), 2.83 (2H, t, J = 6.1 Hz), 4.13 (2H, t, J = 6.1 Hz), 4.53 (1H, td, J = 8.6, 5.5 Hz), 6.78 (1H, d, J = 8.6 Hz), 7.71 (1H, brs), 7.90 (1H, dd, J = 8.6, 2.4 Hz), 8.35 (1H, d, J = 2.4 Hz), 10.19 (1H, s), 11.73 (1H, brs). |
| | | HRMS (ESI⁺):470.27654 [M+H]⁺ |

**[Table 142]**

| Example | Structure | Equipment Data |
|---|---|---|
| 2 4 - 5 | 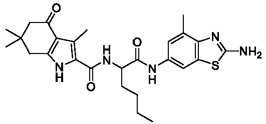 | ¹H-NMR (DMSO-D₆, 400 MHz) δ: 0.89 (3H, t, J = 7.0 Hz), 1.03 (6H, s), 1.28-1.45 (4H, m), 1.65-1.88 (2H, m), 2.24 (2H, s), 2.39 (3H, s), 2.49 (3H, s), 2.66 (2H, s), 4.53-4.61 (1H, m), 7.18 (1H, d, J = 1.8 Hz), 7.38 (2H, s), 7.56 (1H, d, J = 7.9 Hz), 7.85 (1H, d, J = 1.8 Hz), 10.00 (1H, s), 11.71 (1H, s). |
| | | HRMS (ESI⁺): 496.23788 [M+H]⁺ |

### <Example 25-1>

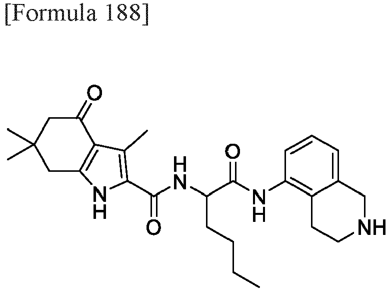

Trifluoroacetic acid (0.50 mL) was added to a solution of tert-butyl 5-[2-[(3,6,6-trimethyl-4-oxo-5,7-dihydro-1H-indole-2-carbonyl)amino]hexanoylamino]-3,4-dihydro-1H-isoquinoline-2-carboxylate (55.0 mg) in dichloromethane (0.50 mL) at room temperature and the mixture was stirred for 2 hours. After the reaction mixture was concentrated under reduced pressure to remove solvents, the residue was purified by amino-silica gel column chromatography (ethyl acetate : methanol = 97 : 3 to 4 : 1) to obtain 3,6,6-trimethyl-4-oxo-N-[1-oxo-1-(1,2,3,4-tetrahydroisoquinolin-5-ylamino)hexan-2-yl]-5,7-dihydro-1H-indole-2-carboxamide (31.8 mg).

¹H-NMR (DMSO-D₆, 400MHz) δ: 0.96 (3H, t, J = 6.7 Hz), 1.09 (6H, s), 1.30-1.52 (4H, m), 1.74-1.96 (2H, m), 2.29 (2H, s), 2.55 (3H, s), 2.58-2.63 (2H, m), 2.71 (2H, s), 2.93-3.03 (2H, m), 3.89 (2H, s), 4.66-4.74 ( 1H, m), 6.92 (1H, d, J = 7.3 Hz), 7.14 (1H, t, J = 7.6 Hz), 7.27 (1H, d, J = 7.9 Hz), 7.64 (1H, d, J = 7.9 Hz), 9.43 (1H, s), 11.78 (1H, s).

HRMS (ESI⁺): 465.2857 [M+H]⁺

Using the corresponding starting materials and reactants, the following Examples 25-2 to 25-3 were obtained by the same method as in Example 25-1, the method described in Step 9-1, or a method similar thereto.

**[Table 143]**

| Example | Structure | Equipment Data |
|---|---|---|
| 2 5 - 2 | 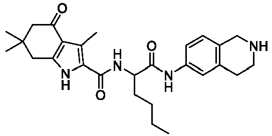 | ¹H-NMR (DMSO-D₆, 400 MHz) δ: 0.88 (3H, t, J = 7.0 Hz), 1.04 (6H, s), 1.25-1.45 (4H, m), 1.63-1.85 (2H, m), 2.24 (2H, s), 2.49 (3H, s), 2.62-2.70 (4H, m), 2.92 (2H, t, J = 6.1 Hz), 3.78 (2H, s), 4.51-4.60 (1H, m), 6.94 (1H, d, J = 8.2 Hz), 7.32 (1H, d, J = 8.2 Hz), 7.36 (1H, s), 7.52-7.65 (1H, m), 9.98 (1H, s), 11.72 (1H, brs). |
| | | HRMS (ESI⁺): 465.28679 [M+H]⁺ |
| 2 5 - 3 | 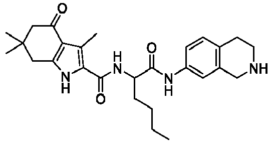 | ¹H-NMR (DMSO-D₆, 400 MHz) δ: 0.86 (3H, t, J = 7.0 Hz), 1.01 (6H, s), 1.23-1.43 (4H, m), 1.61-1.82 (2H, m), 2.21 (2H, s), 2.46 (3H, s), 2.63 (2H, s), 2.71 (2H, t, J = 5.8 Hz), 3.05 (2H, t, J = 5.8 Hz), 3.94 (2H, s), 4.48-4.58 (1H, m), 7.04 (1H, d, J = 8.6 Hz), 7.32 (1H, dd, J = 8.6, 1.8 Hz), 7.37 (1H, s), 7.54 (1H, d, J = 7.9 Hz), 10.03 (1H, s), 11.67 (1H, s). |
| | | HRMS (ESI⁺): 465.28653 [M+H]⁺ |

### <Example 26-1>

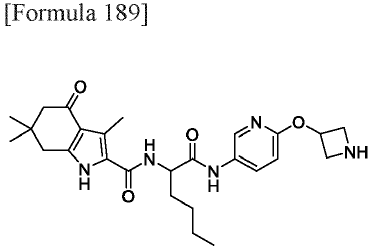

Using tert-butyl 3-[5-[2-[(3,6,6-trimethyl-4-oxo-5,7-dihydro-1H-indole-2-carbonyl)amino]hexanoylamino]pyridin-2-yl]oxyazetidine-1-carboxylate, the title compound was synthesized in the same manner as Example 19.

¹H-NMR (DMSO-D₆, 400MHz) δ: 0.87 (3H, t, J = 7.3 Hz), 1.01 (6H, s), 1.25-1.41 (4H, m), 1.64-1.83 (2H, m), 2.21 (2H, s), 2.46 (3H, s), 2.63 (2H, s), 3.44-3.52 (2H, m), 3.68-3.75 (2H, m), 4.50-4.58 (1H, m), 5.22- 5.30 (1H, m), 6.80 (1H, d, J = 8.5 Hz), 7.56-7.64 (1H, m), 7.90 (1H, dd, J = 8.5, 3.0 Hz), 8.31 (1H, d, J = 3.0 Hz), 10.16 (1H, s), 11.66 (1H, br s).

HRMS (ESI⁺): 482.27709 [M+H]⁺

Using the corresponding starting materials and reactants, the following Examples 26-2 to 26-8 were obtained by the same method as in Example 26-1, the method described in Step 10-1, or a method similar thereto.

**[Table 144]**

| Example | Structure | Equipment Data |
|---|---|---|
| 2 6 - 2 | 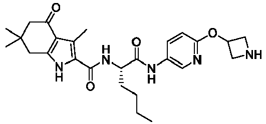 | ¹H-NMR (DMSO-D₆, 400 MHz) δ: 0.86 (3H, t, J = 7.0 Hz), 1.01 (6H, s), 1.22-1.42 (4H, m), 1.64-1.83 (2H, m), 2.21 (2H, s), 2.46 (3H, s), 2.63 (2H, s), 3.39-3.51 (2H, m), 3.65-3.75 (2H, m), 4.45-4.58 (1H, m), 5.21-5.30 (1H, m), 6.79 (1H, d, J = 9.1 Hz), 7.66-7.75 (1H, m), 7.91 (1H, dd, J = 9.1, 3.0 Hz), 8.32 (1H, d, J = 2.4 Hz), 10.19 (1H, s), 11.76 (1H, br s). |
| | | HRMS (ESI⁺): 482.27716 [M+H]⁺ |
| 2 6 - 3 | 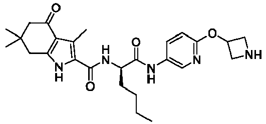 | ¹H-NMR (DMSO-D₆, 400 MHz) δ: 0.89 (3H, t, J = 7.0 Hz), 1.04 (6H, s), 1.24-1.48 (4H, m), 1.56-1.87 (2H, m), 2.24 (2H, s), 2.49 (3H, s), 2.66 (2H, s), 3.46-3.54 (2H, m), 3.68-3.81 (2H, m), 4.48-4.62 (1H, m), 5.25-5.36 (1H, m), 6.82 (1H, d, J = 9.1 Hz), 7.68-7.82 (1H, m), 7.94 (1H, dd, J = 8.8, 2.7 Hz), 8.35 (1H, d, J = 2.4 Hz), 10.23 (1H, s), 11.79 (1H, br s). |
| | | HRMS (ESI⁺): 482.27732 [M+H]⁺ |
| 2 6 - 4 | 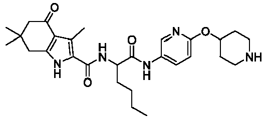 | ¹H-NMR (DMSO-D₆, 400 MHz) δ: 0.89 (3H, t, J = 6.7 Hz), 1.04 (6H, s), 1.26-1.52 (6H, m), 1.66-1.85 (2H, m), 1.88-1.97 (2H, m), 2.24 (2H, s), 2.49 (3H, s), 2.53-2.60 (2H, m), 2.66 (2H, s), 2.95 (2H, td, J = 8.3, 3.9 Hz), 4.51-4.61 (1H, m), 4.92-5.02 (1H, m), 6.76 (1H, d, J = 8.6 Hz), 7.68 (1H, d, J = 6.7 Hz), 7.91 (1H, dd, J = 8.6, 2.4 Hz), 8.35 (1H, d, J = 2.4 Hz), 10.19 (1H, s), 11.74 (1H, s). |
| | | HRMS (FD⁺): 510.30753 [M+H]⁺ |
| 2 6 - 5 | 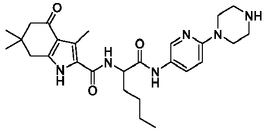 | ¹H-NMR (DMSO-D₆, 400 MHz) δ: 0.89 (3H, t, J = 7.0 Hz), 1.04 (6H, s), 1.26-1.48 (4H, m), 1.65-1.86 (2H, m), 2.24 (2H, s), 2.49 (3H, s), 2.66 (2H, s), 2.74-2.81 (4H, m), 3.28-3.38 (4H, m), 4.50-4.61 (1H, m), 6.79 (1H, d, J = 9.1 Hz), 7.62 (1H, d, J = 7.9 Hz), 7.77 (1H, dd, J = 9.1, 2.4 Hz), 8.31 (1H, d, J = 2.4 Hz), 10.00 (1H, s), 11.74 (1H, s). |
| | | HRMS (ESI⁺): 495.30765 [M+H]⁺ |
| 2 6 - 6 | 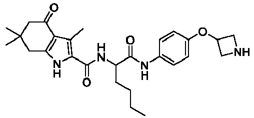 | ¹H-NMR (DMSO-D₆, 400 MHz) δ: 0.89 (3H, t, J = 7.0 Hz), 1.04 (6H, s), 1.28-1.46 (4H, m), 1.64-1.86 (2H, m), 2.24 (2H, s), 2.49 (3H, s), 2.66 (2H, s), 3.45-3.53 (2H, m), 3.70-3.79 (2H, m), 4.51-4.61 (1H, m), 4.87-4.96 (1H, m), 6.76 (2H, d, J = 9.1 Hz), 7.52 (2H, d, J = 9.1 Hz), 7.62 (1H, d, J = 7.9 Hz), 10.03 (1H, s), 11.74 (1H, s). |
| | | HRMS (ESI⁺): 481.28121 [M+H]⁺ |

**[Table 145]**

| Example | Structure | Equipment Data |
|---|---|---|
| 2 6 - 7 | 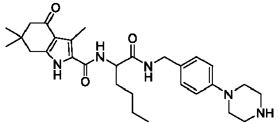 | ¹H-NMR (270 MHz, CDCl₃) δ: 0.87 (3H, t, J = 6.8 Hz), 1.08 (6H, s), 1.26-1.38 (4H, m), 1.62-2.00 (2H, m), 2.33 (2H, s), 2.61 (2H, s), 2.66 (3H, s), 3.00-3.06 (4H, m), 3.10-3.13 (4H, m), 4.31 (1H, dd, J = 5.3, 14.3 Hz), 4.43 (1H, dd, J = 5.3, 14.3 Hz), 4.56 (1H, dt, J = 8.2, 7.0 Hz), 6.67 (1H, d, J = 8.2Hz), 6.86 (2H, dd, J = 8.6, 1.7 Hz), 7.15 (2H, d, J = 8.6 Hz), 9.56 (1H, brs). |
| | | MS (ESI⁺): 508.69 [M+H]⁺ |
| 2 6 - 8 | 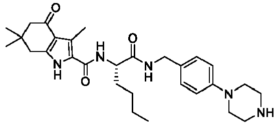 | ¹H-NMR (399 MHz, CDCl₃) δ: 0.88 (3H, t, J = 7.1 Hz), 1.08 (6H, s), 1.28-1.41 (4H, m), 1.67-2.00 (2H, m), 2.33 (2H, s), 2.61 (2H, s), 2.65 (3H, s), 3.02 (4H, t, J = 4.8 Hz), 3.11 (4H, t, J = 4.8 Hz), 4.32 (1H, dd, J = 14.6, 5.5 Hz), 4.41 (1H, dd, J = 14.6, 5.5 Hz), 4.55 (1H, dt, J = 6.9, 6.9 Hz), 6.38 (1H, brs), 6.62 (1H, d, J = 7.8 Hz), 6.85 (2H, d, J = 8.7Hz), 7.15 (2H, d, J = 8.7 Hz), 9.51 (1H, brs). |
| | | MS (ESI⁺): 508.71 [M+H]⁺ |

### <Example 27>

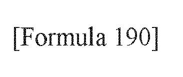

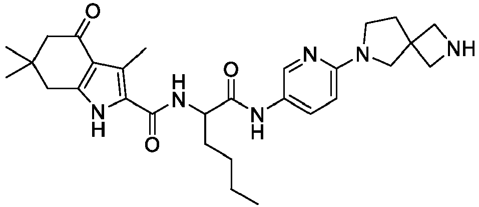

Using tert-butyl 7-[5-[2-[(3,6,6-trimethyl-4-oxo-5,7-dihydro-1H-indole-2-carbonyl)amino]hexanoylamino]pyridin-2-yl]-2,7-diazaspiro[3.4]octane-2-carboxylate, the title compound was synthesized in the same manner in Example 19.

¹H-NMR (DMSO-D₆, 400MHz) δ: 0.86 (3H, t, J = 6.7 Hz), 1.01 (6H, s), 1.21-1.42 (4H, m), 1.63-1.80 (2H, m), 2.03-2.12 (2H, m), 2.21 (2H, s), 2.46 (3H, s), 2.63 (2H, s), 3.32-3.37 (4H, m), 3.39-3.50 (4H, m), 4.48-4.57 (1H, m), 6.39 (1H, d, J = 9.1 Hz), 7.63-7.74 (2H, m), 8.22 (1H, d, J = 2.4 Hz), 9.91 (1H, s).

HRMS (ESI⁺): 521.32408 [M+H]⁺

### <Example 28>

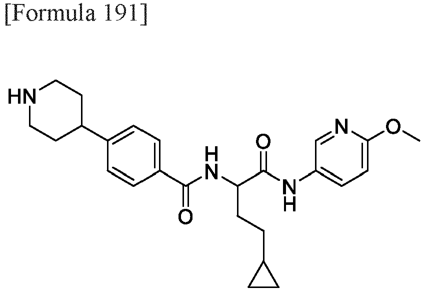

Trifluoroacetic acid (0.500 mL) was added to a solution of tert-butyl 4-[4-[[4-cyclopropyl-1-[(6-methoxypyridin-3-yl) amino]-1-oxobutan-2-yl]carbamoyl]phenyl]piperidine-1-carboxylate (52.0 mg) in dichloromethane (0.500 mL) at room temperature and the mixture was stirred for 15 minutes. After the reaction mixture was concentrated under reduced pressure to remove solvents, diisopropylamine (0.100 mL) was added to a solution of the residue in tetrahydrofuran (0.500 mL) at room temperature and the mixture was stirred for 15 minutes. After the reaction mixture was concentrated under reduced pressure, the residue was purified by amino-silica gel column chromatography (ethyl acetate : methanol = 1 : 0 to 9 : 1) to obtain N-[4-cyclopropyl-1-[(6-methoxypyridine-3-yl)amino]-1-oxobutan-2-yl]-4-piperidin-4-ylbenzamide (39.0 mg).

¹H-NMR (DMSO-D₆, 400MHz) δ: -0.05-0.08 (2H, m), 0.32-0.43 (2H, m), 0.64-0.76 (1H, m), 1.19-1.38 (2H, m), 1.50 (2H, ddd, J = 24.5, 12.2, 3.7 Hz), 1.62-1.72 (2H, m), 1.84-1.95 (2H, m), 2.52-2.68 (3H, m), 2.96-3.06 (2H, m), 3.80 (3H, s), 4.56 (1H, q, J = 7.9 Hz), 6.78 (1H, d, J = 8.6 Hz), 7.30 (2H, d, J = 8.6 Hz), 7.84 (2H, d, J = 8.6 Hz), 7.90 (1H, dd, J = 8.6, 2.4 Hz), 8.36 (1H, d, J = 2.4 Hz), 8.48 (1H, d, J = 7.9 Hz), 10.09 (1H, s).

HRMS (ESI⁺): 437.25501 [M+H]⁺

### <Example 29>

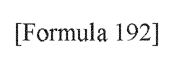

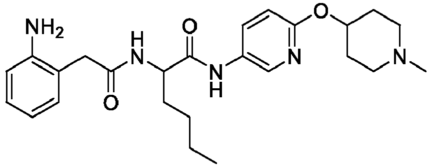

Using N-[6-(1-methylpiperidin-4-yl)oxypyridin-3-yl]-2-[[2-(2-nitrophenyl)acetyl]amino]hexanamide, the title compound was synthesized in the same manner in Reference Example 5.

¹H-NMR (DMSO-D₆, 400MHz) δ: 0.82 (3H, t, J = 7.0 Hz), 1.17-1.33 (4H, m), 1.52-1.75 (4H, m), 1.88-1.97 (2H, m), 2.12-2.24 (5H, m), 2.58-2.70 (2H, m), 3.32-3.35 (1H, m), 3.38 (1H, d, J = 13.9 Hz), 4.30-4.40 (1H, m), 4.85-4.94 (1H, m), 5.04 (2H, s), 6.49 (1H, td, J = 7.3, 1.2 Hz), 6.62 (1H, d, J = 7.3 Hz), 6.73 (1H, d, J = 9.1 Hz), 6.91 (1H, td, J = 7.6, 1.2 Hz), 7.00 (1H, dd, J = 7.6, 1.2 Hz), 7.86 (1H, dd, J = 9.1, 2.4 Hz), 8.30 (1H, d, J = 2.4 Hz), 8.35 (1H, d, J = 7.9 Hz), 10.05 (1H, s).

HRMS (ESI⁺): 454.28121 [M+H]⁺

### <Reference example 84-1>

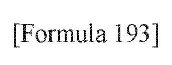

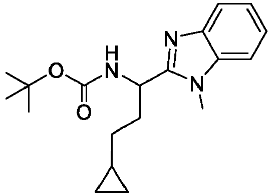

Under an argon atmosphere, 2-N-methylbenzene-1,2-diamine (0.047 mL), 1-hydroxybenzotriazole monohydrate (75.5 mg), N,N-diisopropylethylamine (0.105 mL), and 1-ethyl-3-(3-dimethylaminopropyl)carbodiimide hydrochloride (86.6 mg) were added to a solution of 4-cyclopropyl-2-[(2-methylpropan-2-yl)oxycarbonylamino]butanoic acid (100 mg) in N,N-dimethylformamide (2.06 mL), and the mixture was stirred at room temperature for 22 hours. After water was added, the reaction mixture was extracted with ethyl acetate. The organic layer was washed with saturated brine, dried over anhydrous sodium sulfate, and concentrated after filtration of the insoluble material. Acetic acid (1.37 mL) was added to the residue and the mixture was stirred at 60 °C for 4 hours. The reaction mixture was neutralized with saturated aqueous sodium hydrogen carbonate solution and extracted with ethyl acetate. The organic layer was washed with saturated brine, dried over anhydrous sodium sulfate, and concentrated after filtration of the insoluble material. The residue was purified by silica gel column chromatography (hexane : ethyl acetate = 4 : 1) to obtain tert-butyl N-[3-cyclopropyl-1-(1-methylbenzoimidazol-2-yl)propyl]carbamate (96.1 mg).

¹H-NMR (CDCl₃, 400MHz) δ: -0.03-0.06 (2H, m), 0.40-0.43 (2H, m), 0.67-0.75 (1H, m), 1.20-1.33 (2H, m), 1.43 ( 9H, s), 2.01-2.19 (2H, m), 3.83 (3H, s), 5.11 (1H, td, J = 7.9, 6.1Hz), 5.29 (1H, d, J = 8.6 Hz), 7.24-7.31 (2H, m), 7.32-7.36 (1H, m), 7.73 (1H, dd, J = 7.3, 1.8 Hz).

HRMS (ESI⁺): 330.21755 [M+H]⁺

Using the corresponding starting materials and reactants, the following Reference Examples 84-2 to 84-6 were obtained by the same method as in Reference Example 84-1, the method described in Step 39-1, or a method similar thereto.

**[Table 146]**

| Reference Example | Structure | Equipment Data |
|---|---|---|
| 8 4 - 2 | 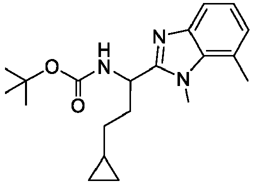 | ¹H-NMR (CDCl₃, 400 MHz) δ: -0.03-0.07 (2H, m), 0.39-0.44 (2H, m), 0.67-0.75 (1H, m), 1.20-1.38 (2H, m), 1.43 (9H, s), 2.01-2.17 (2H, m), 2.75 (3H, s), 4.05 (3H, s), 5.09 (1H, td, J = 8.6, 6.1 Hz), 5.31 (1H, d, J = 8.6 Hz), 6.97 (1H, d, J = 7.9 Hz), 7.11 (1H, t, J = 7.9 Hz), 7.56 (1H, d, J = 7.9 Hz). |
| | | HRMS (ESI⁺):344.23327 [M+H]⁺ |

**[Table 147]**

| Reference Example | Structure | Equipment Data |
|---|---|---|
| 8 4 - 3 | 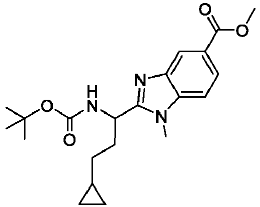 | ¹H-NMR (CDCl₃, 400 MHz) δ: -0.01-0.10 (2H, m), 0.44-0.49 (2H, m), 0.70-0.78 (1H, m), 1.29-1.38 (2H, m), 1.46 (9H, s), 2.17-2.24 (2H, m), 3.94 (3H, s), 3.98 (3H, s), 5.14-5.21 (1H, m), 7.44 (1H, d, J = 8.5 Hz), 8.10 (1H, d, J = 8.5 Hz), 8.53 (1H, s). |
| | | HRMS (ESI⁺):388.22371 [M+H]⁺ |
| 8 4 - 4 | 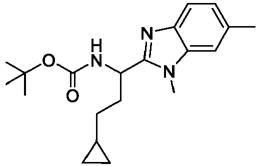 | ¹H-NMR (CDCl₃, 400 MHz) δ: -0.02-0.12 (2H, m), 0.43-0.49 (2H, m), 0.72-0.79 (1H, m), 1.28-1.38 (2H, m), 1.47 (9H, s), 2.09-2.25 (2H, m), 2.56 (3H, s), 3.86 (3H, s), 5.11-5.18 (1H, m), 5.46 (1H, brs), 7.16 (1H, d, J = 8.5 Hz), 7.20 (1H, s), 7.67 (1H, d, J = 8.5 Hz). |
| | | HRMS (EI⁺):343.22505 [M]⁺ |
| 8 4 - 5 | 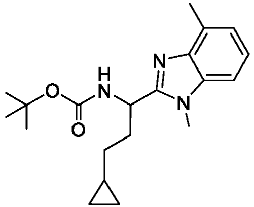 | ¹H-NMR (CDCl₃, 400 MHz) δ: -0.01-0.10 (2H, m), 0.40-0.50 (2H, m), 0.68-0.77 (1H, m), 1.28-1.35 (2H, m), 1.44 (9H, s), 2.13-2.34 (2H, m), 2.74 (3H, s), 3.92 (3H, s), 5.19 (1H, q, J = 7.9 Hz), 7.18 (1H, d, J = 5.4 Hz), 7.23-7.31 (2H, m). |
| | | HRMS (ESI⁺):344.23347 [M+H]⁺ |
| 8 4 - 6 | 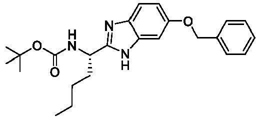 | ¹H-NMR (399 MHz, CDCl₃) δ: 0.88 (3H, t, J= 7.0 Hz), 1.26-1.45 (4H, m), 1.44 (9H, s), 1.85- 2.00 (1H, m), 2.10-2.25 (1H, m), 4.79 (1H, dt, J = 8.0, 6.8 Hz), 5.07 (2H, s), 5.29 (1H, d, J = 8.0 Hz), 6.92-6.95 (1H, m), 7.29-7.45 (7H, m). |
| | | MS (ESI⁺): 410.8 [M+H]⁺ |

### <Example 30-1>

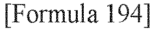

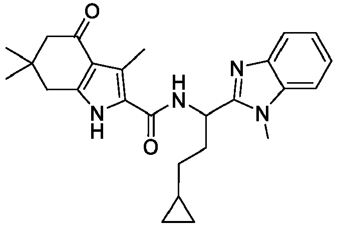

Under an argon atmosphere, trifluoroacetic acid (0.729 mL) was added to a solution of tert-butyl N-[3-cyclopropyl-1-(1-methylbenzoimidazol-2-yl)propyl]carbamate (96.1 mg) in dichloromethane (0.729 mL). and the mixture was stirred at room temperature for 30 minutes. After the reaction mixture was concentrated under reduced pressure, 3,6,6-trimethyl-4-oxo-5,7-dihydro-1H-indole-2-carboxylic acid (64.6 mg), 1-hydroxybenzotriazole monohydrate (53.6 mg), N,N-diisopropylethylamine (0.199 mL), 1-ethyl-3-(3-dimethylaminopropyl)carbodiimide hydrochloride (61.5 mg) were added to a solution of the obtained crude product in N,N-dimethylformamide (1.46 mL), and the mixture was stirred at room temperature for 24 hours. After water was added, the reaction mixture was extracted with ethyl acetate. The organic layer was washed with saturated brine, dried over anhydrous sodium sulfate, and concentrated after filtration of the insoluble material. The residue was purified by silica gel column chromatography (hexane : ethyl acetate = 1 : 1), and the resulting solid was washed with diisopropyl ether to obtain N-[3-cyclopropyl-1-(1-methylbenzoimidazol-2-yl)propyl]-3,6,6-trimethyl-4-oxo-5,7-dihydro-1H-indole-2-carboxamide (92.8 mg).

¹H-NMR (DMSO-D₆, 400MHz) δ: 0.00-0.10 (2H, m), 0.43 (2H, dd, J = 7.9, 1.8 Hz), 0.74-0.82 (1H, m), 1.04 (6H, s), 1.26-1.37 (2H, m), 2.11-2.21 (2H, m), 2.24 (2H, s), 2.48 (3H, s), 2.65 (2H, s), 3.85 (3H, s), 5.50 (1H, td, J = 8.6, 6.1 Hz), 7.22 (1H, ddd, J = 8.6, 7.3, 1.2 Hz), 7.27 (1H, ddd, J = 8.6, 7.3, 1.2 Hz), 7.56 (1H, dd, J = 7.3, 1.2 Hz), 7.63 (1H, dd, J = 7.3, 1.2 Hz), 8.05 (1H, d, J = 8.6 Hz), 11.61 (1H, s).

HRMS (ESI⁺): 433.25981 [M+H]⁺

Using the corresponding starting materials and reactants, the following Examples 30-2 to 30-4 were obtained by the same method as in Example 30-1, the method described in Steps 39-2 to 39-3, or a method similar thereto.

**[Table 148]**

| Example | Structure | Equipment Data |
|---|---|---|
| 3 0 - 2 | 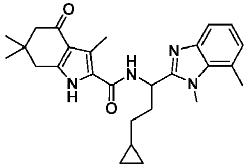 | ¹H-NMR (DMSO-D₆, 400MHz) δ: -0.02-0.09 (2H, m), 0.40-0.45 (2H, m), 0.73-0.82 (1H, m), 1.03 (6H, s), 1.26-1.37 (2H, m), 2.05-2.21 (2H, m), 2.23 (2H, s), 2.48 (3H, s), 2.64 (2H, s), 2.73 (3H, s), 4.04 (3H, s), 5.50 (1H, td, J = 8.6, 5.5 Hz), 6.95 (1H, d, J = 7.3 Hz), 7.05 (1H, t, J = 7.3 Hz), 7.43 (1H, d, J = 7.3 Hz), 8.01 (1H, d, J = 8.6 Hz), 11.59 (1H, s). |
| | | HRMS (ESI⁺):447.27555 [M+H]⁺ |
| 3 0 - 3 | 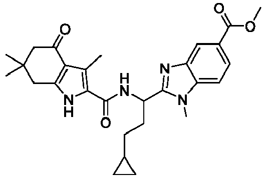 | ¹H-NMR (DMSO-D₆, 400MHz) δ: -0.02-0.08 (2H, m), 0.42 (2H, dd, J = 7.9, 1.8 Hz), 0.73-0.81 (1H, m), 1.02 (6H, s), 1.28-1.36 (2H, m), 2.09-2.20 (2H, m), 2.22 (2H, s), 2.45 (3H, s), 2.63 (2H, s), 3.879 (3H, s), 3.882 (3H, s), 5.48 (1H, td, J = 8.5, 6.1 Hz), 7.67 (1H, d, J = 8.5 Hz), 7.90 (1H, dd, J = 8.5, 1.8 Hz), 8.13 (1H, d, J = 8.5 Hz), 8.21 (1H, d, J = 1.8 Hz), 11.62 (1H, s). |
| | | HRMS (ESI⁺):491.26546 [M+H]⁺ |

**[Table 149]**

| Example | Structure | Equipment Data |
|---|---|---|
| 3 0 - 4 | 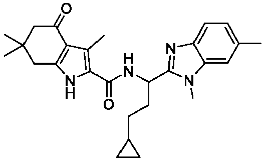 | ¹H-NMR (DMSO-D₆, 400MHz) δ: -0.03-0.07 (2H, m), 0.38-0.43 (2H, m), 0.71-0.80 (1H, m), 1.02 (6H, s), 1.25-1.34 (2H, m), 2.03-2.19 (2H, m), 2.22 (2H, s), 2.51 (6H, s), 2.62 (2H, s), 3.80 (3H, s), 5.41-5.50 (1H, m), 7.05 (1H, br s), 7.36 (1H, s), 7.49 (1H, d, J = 7.3 Hz), 8.01 (1H, d, J = 7.3 Hz), 11.59 (1H, s). |
| | | HRMS (ESI⁺):447.27548 [M+H]⁺ |

### <Reference example 85>

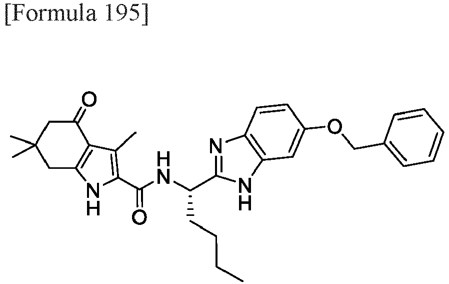

4-(4,6-Dimethoxy-1,3,5-triazin-2-yl)-4-methylmorpholinium chloride (906.5 mg) was added to a solution of 3,6,6-trimethyl-4-oxo-4,5,6,7-tetrahydro-1H-indole-2-carboxylic acid (604 mg) and (S)-1-(6-(benzyloxy)-1H-benz[d]imidazol-2-yl)pentan-1-amine (845 mg) in tetrahydrofuran (10 mL) under ice cooling condition. The mixture was warmed to room temperature and stirred overnight. Ethyl acetate, water, and a saturated aqueous sodium hydrogen carbonate solution were added to the reaction mixture, and the organic layer was separated. The aqueous layer was extracted once with ethyl acetate. The combined organic

layer was washed with saturated aqueous brine-sodium hydrogen carbonate solution, dried over sodium sulfate, filtered, and concentrated under reduced pressure. The residue was purified by silica gel column chromatography (ethyl acetate-hexane), crystallized from chloroform under concentration, washed with heptane to obtain (S)-N-(1-(6-(benzyloxy)-1H-benzo[d]imidazol-2-yl)pentyl)-3,6,6-trimethyl-4-oxo-4,5,6,7-tetrahydro-1H-indole-2-carboxamide (990 mg).

¹H-NMR (399MHz, CDCl₃) δ: 0.83 (3H, t, J = 7.0 Hz), 0.99 (6H, s), 1.23-1.39 (4H, m), 1.83-1.95 (1H, m), 1.98-2.11 (1H, m), 2.19 (2H, s), 2.48 (3H, s), 261 (2H, s), 5.09 (2H, s), 5.19 (1H, q, J = 6.0 Hz), 6.85 (1H, m), 7.01 (0.6H, s), 7.17 (0.4H, s), 7.29 (1H, d, J = 7.2 Hz), 7.36 (3H, m), 7.43 (2H, d, J = 7.2 Hz), 7.80 (1H, d, J = 8.4 Hz), 11.59 (1H, s), 12.12 (1H, m).

MS (ESI⁺): 513.14 [M+H]⁺

### <Reference example 86-1>

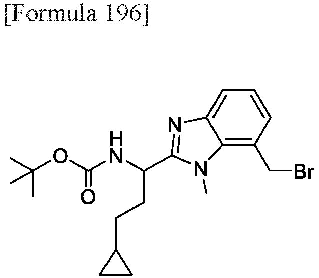

Under an argon atmosphere, N-bromosuccinimide (104 mg) and 2,2'-azobis(isobutyronitrile) (4.8 mg) were added to a solution of tert-butyl N-[3-cyclopropyl-1-(1,7-dimethylbenzoimidazol-2-yl)propyl]carbamate (200 mg) in carbon tetrachloride (5.82 mL) at room temperature, and the mixture was stirred at 80 °C for 5 hours. After a saturated aqueous sodium thiosulfate solution was added, the reaction mixture was extracted with ethyl acetate. The organic layer was washed with saturated brine, dried over anhydrous sodium sulfate, and concentrated after filtration of the insoluble material. The residue was purified by silica gel column chromatography (hexane : ethyl acetate = 3 : 1) to obtain tert-butyl N-[1-[7-(bromomethyl)-1-methylbenzoimidazol-2-yl]-3-cyclopropylpropyl]carbamate (149 mg).

¹H-NMR (CDCl₃, 400MHz) δ: -0.01-0.09 (2H, m), 0.42-0.47 (2H, m), 0.69-0.77 (1H, m), 1.25-1.36 (2H, m), 1.45 ( 9H, s), 2.13-2.20 (2H, m), 4.26 (3H, s), 4.87 (1H, d, J = 10.9 Hz), 4.93 (1H, d, J = 10.9 Hz), 5.15-5.23 (1H, m), 7.24-7.27 (2H, m), 7.77 (1H, t, J = 4.8 Hz).

HRMS (ESI⁺): 422.14443 [M+H]⁺

Using the corresponding starting materials and reactants, the following Reference Examples 86-2 to 86-3 were obtained by the same method in Reference Example 86-1, the method described in Step 40-1, or a method similar thereto.

**[Table 150]**

| Reference Example | Structure | Equipment Data |
|---|---|---|
| 8 6 - 2 | 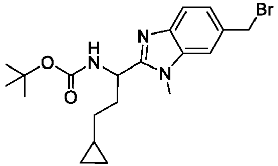 | ¹H-NMR (CDCl₃, 400 MHz) δ: 0.01-0.10 (2H, m), 0.48-0.51 (2H, m), 0.74-0.79 (1H, m), 1.32-1.38 (2H, m), 1.49 (9H, s), 2.18-2.25 (2H, m), 3.93 (3H, s), 4.76 (2H, s), 5.14-5.21 (1H, m), 5.49 (1H, brs), 7.40 (1H, d, J = 8.5 Hz), 7.47 (1H, d, J = 1.2 Hz), 7.77 (1H, d, J = 8.5 Hz). |
| | | HRMS (ESI⁺):422.14397 [M+H]⁺ |
| 8 6 - 3 | 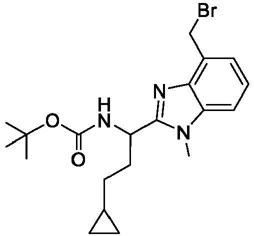 | ¹H-NMR (CDCl₃, 400 MHz) δ: -0.02-0.02 (2H, m), 0.38-0.42 (2H, m), 0.61-0.68 (1H, m), 1.16-1.31 (2H, m), 1.34 (9H, s), 2.12-2.21 (1H, m), 2.52-2.61 (1H, m), 4.04 (3H, s), 5.03 (1H, d, J = 10.9 Hz), 5.15 (1H, d, J = 10.9 Hz), 5.21-5.27 (1H, m), 7.44 (1H, d, J = 7.9 Hz), 7.50 (1H, t, J = 7.9 Hz), 7.58 (1H, d, J = 7.9 Hz). |
| | | HRMS (ESI⁺):422.14423 [M+H]⁺ |

### <Reference example 87-1>

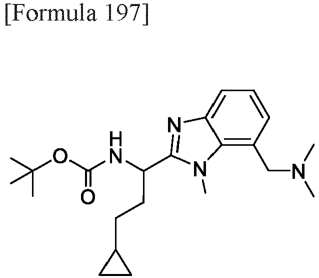

Under an argon atmosphere, dimethylamine (0.353 mL, 2 mol/L tetrahydrofuran solution) was added to a solution of tert-butyl N-[1-[7-(bromomethyl)-1-methylbenzoimidazol-2-yl]-3-cyclopropylpropyl]carbamate (149 mg) in tetrahydrofuran (1.76 mL) at room temperature, and the mixture was stirred at room temperature for 4 hours. After a saturated aqueous sodium hydrogen carbonate solution was added, the reaction mixture was extracted with ethyl acetate. The organic layer was washed with saturated brine, dried over anhydrous sodium sulfate, and concentrated after filtration of the insoluble material. The residue was purified by silica gel column chromatography (hexane : ethyl acetate = 1 : 1) to obtain tert-butyl N-[3-cyclopropyl-1-[7-[(dimethylamino)methyl]-1-methylbenzoimidazol-2-yl]propyl]carbamate (100 mg).

HRMS (ESI⁺): 387.27547 [M+H]⁺

Using the corresponding starting materials and reactants, the following Reference Examples 87-2 to 87-7 were obtained by the same method in Reference Example 87-1, the method described in Step 40-2, or a method similar thereto.

**[Table 151]**

| Reference Example | Structure | Equipment Data |
|---|---|---|
| 8 7 - 2 | 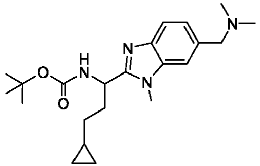 | ¹H-NMR (CDCl₃, 400 MHz) δ: -0.03-0.11 (2H, m), 0.41-0.48 (2H, m), 0.68-0.79 (1H, m), 1.23-1.39 (2H, m), 1.46 (9H, s), 2.04-2.22 (2H, m), 2.56 (6H, brs), 3.10 (2H, s), 3.89 (3H, s), 5.09-5.18 (1H, m), 5.31 (1H, d, J = 9.1 Hz), 7.22 (1H, dd, J = 8.5, 1.8 Hz), 7.32 (1H, dd, J = 8.5, 1.8 Hz), 7.42 (1H, d, J = 1.8 Hz). |
| | | HRMS (ESI⁺):387.27522 [M+H]⁺ |
| 8 7 - 3 | 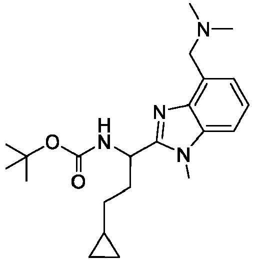 | ¹H-NMR (DMSO-D₆, 400MHz) δ: 0.02-0.08 (2H, m), 0.39-0.45 (2H, m), 0.71-0.77 (1H, m), 1.26-1.30 (2H, m), 1.39 (9H, s), 1.98-2.02 (2H, m), 3.36 (6H, s), 3.80 (3H, s), 3.82 (2H, s), 4.93 (1H, td, J = 8.5, 6.1 Hz), 7.18-7.25 (2H, m), 7.40 (1H, dd, J = 7.3, 1.8 Hz), 7.46 (1H, d, J = 8.5 Hz). |
| | | HRMS (ESI⁺):387.27647 [M+H]⁺ |

**[Table 152]**

| Reference Example | Structure | Equipment Data |
|---|---|---|
| 8 7 - 4 | 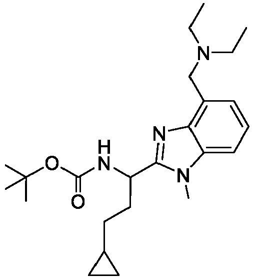 | ¹H-NMR (DMSO-D₆, 400MHz) δ: 0.01-0.06 (2H, m), 0.40-0.44 (2H, m), 0.70-0.77 (1H, m), 1.03 (6H, t, J = 7.3 Hz), 1.25-1.28 (2H, m), 1.40 (9H, s), 1.98-2.09 (2H, m), 2.52 (4H, q, J = 7.3 Hz), 3.80 (3H, s), 3.94 (1H, d, J = 14.5 Hz), 3.99 (1H, d, J = 14.5 Hz), 4.93 (1H, td, J = 8.5, 6.7 Hz), 7.21 (1H, t, J = 7.3 Hz), 7.25 (1H, d, J = 7.3 Hz), 7.38 (1H, dd, J = 7.3, 1.8 Hz), 7.45 (1H, d, J = 8.5 Hz). |
| | | HRMS (ESI⁺):415.30768 [M+H]⁺ |
| 8 7 - 5 | 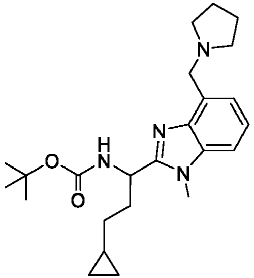 | ¹H-NMR (DMSO-D₆, 400MHz) δ: 0.01-0.06 (2H, m), 0.38-0.42 (2H, m), 0.69-0.74 (1H, m), 1.21-1.29 (2H, m), 1.38 (9H, s), 1.67-1.71 (4H, m), 1.97-2.03 (2H, m), 2.46-2.48 (4H, m), 3.79 (3H, s), 3.98 (2H, s), 4.92 (1H, td, J = 8.5, 6.1 Hz), 7.18-7.21 (2H, m), 7.35-7.39 (1H, m), 7.45 (1H, d, J = 8.5 Hz). |
| | | HRMS (ESI⁺):413.29110 [M+H]⁺ |
| 8 7 - 6 | 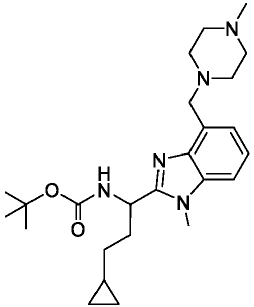 | ¹H-NMR (DMSO-D₆, 400MHz) δ: -0.03-0.02 (2H, m), 0.35-0.39 (2H, m), 0.67-0.72 (1H, m), 1.18-1.26 (2H, m), 1.35 (9H, s), 1.91-2.04 (2H, m), 2.12 (3H, s), 2.29-2.40 (8H, m), 3.76 (3H, s), 3.84 (2H, s), 4.85-4.92 (1H, m), 7.16-7.18 (2H, m), 7.35 (1H, dd, J = 6.7, 2.4 Hz), 7.42 (1H, d, J = 8.5 Hz). |
| | | HRMS (ESI⁺):442.31878 [M+H]⁺ |
| 8 7 - 7 | 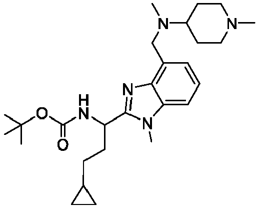 | ¹H-NMR (DMSO-D₆, 400MHz) δ: -0.02-0.02 (2H, m), 0.35-0.39 (2H, m), 0.66-0.72 (1H, m), 1.20-1.25 (4H, m), 1.35 (9H, s), 1.51-1.56 (2H, m), 1.74-1.80 (4H, m), 1.94-2.02 (2H, m), 2.10 (3H, s), 2.15 (3H, s), 2.72-2.80 (1H, m), 3.76 (3H, s), 3.89 (1H, d, J = 13.9 Hz), 3.96 (1H, d, J = 13.9 Hz), 4.87-4.91 (1H, m), 7.16-7.19 (2H, m), 7.33 (1H, dd, J = 6.7, 2.4 Hz), 7.39 (1H, d, J = 8.5 Hz). |
| | | HRMS (ESI⁺):470.34937 [M+H]⁺ |

### <Example 31-1>

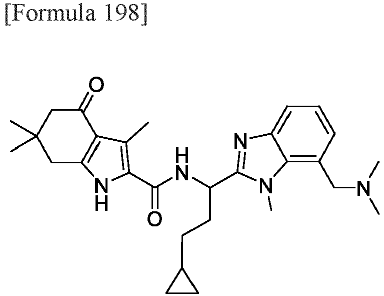

Under an argon atmosphere, trifluoroacetic acid (0.647 mL) was added to a solution of tert-butyl N-[3-cyclopropyl-1-[7-[(dimethylamino)methyl]-1-methylbenzimidazol-2-yl]propyl]carbamate (100 mg) in dichloromethane (0.647 mL), and the mixture was stirred at room temperature for 30 minutes, and then the reaction mixture was concentrated under reduced pressure. 3,6,6-Trimethyl-4-oxo-5,7-dihydro-1H-indole-2-carboxylic acid (57. 3 mg), 1-hydroxybenzotriazole monohydrate (47.5 mg), N,N-diisopropylethylamine (0.175 mL), and 1-ethyl-3-(3-dimethylaminopropyl)carbodiimide hydrochloride (54.6 mg) were added to a solution of the obtained crude product in N,N-dimethylformamide (1.29 mL), and stirred at room temperature for 8 hours. After water was added, the reaction mixture was extracted with ethyl acetate. The organic layer was washed with saturated brine, dried over anhydrous sodium sulfate, and concentrated after filtration of the insoluble material. The residue was purified by amino-silica gel column chromatography (hexane : ethyl acetate = 1 : 1), and the resulting solid was washed with diisopropyl ether to obtain N-[3-cyclopropyl-1-[7-[(dimethylamino)methyl]-1-methylbenzoimidazol-2-yl]propyl]-3,6,6-trimethyl-4-oxo-5,7-dihydro-1H-indole-2-carboxamide (73. 6 mg).

¹H-NMR (DMSO-D₆, 400MHz) δ: 0.00-0.11 (2H, m), 0.42-0.46 (2H, m), 0.75-0.83 (1H, m), 1.05 (6H, s), 1.28-1.39 (2H, m), 2.09-2.15 (2H, m), 2.19 (6H, s), 2.25 (2H, s), 2.50 (3H, s), 2.66 (2H, s), 3.64 (1H, d, J = 12.7 Hz), 3.75 (1H, d, J = 12.7 Hz), 4.15 (3H, s), 5.53 (1H, td, J = 8.5, 5.4 Hz), 7.03 (1H, d, J = 7.9 Hz), 7.13 (1H, t, J = 7.9 Hz), 7.58 (1H, dd, J = 7.9, 1.2 Hz), 8.05 (1H, d, J = 8.5 Hz), 11.62 (1H, s).

HRMS (ESI⁺): 490.31795 [M+H]⁺

Using the corresponding starting materials and reactants, the following Examples 31-2 to 31-7 were obtained by the same method as in Example 31-1, the method described in Steps 39-2 to 39-3 or a method similar thereto.

**[Table 153]**

| Example | Structure | Equipment Data |
|---|---|---|
| 3 1- 2 | 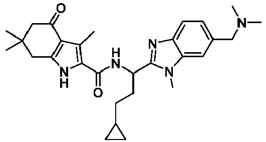 | ¹H-NMR (DMSO-D₆, 400 MHz) δ: 0.00-0.09 (2H, m), 0.38-0.48 (2H, m), 0.73-0.83 (1H, m), 1.03 (3H, s), 1.04 (3H, s), 1.26-1.41 (2H, m), 2.06-2.14 (2H, m), 2.18 (6H, s), 2.24 (2H, s), 2.48 (3H, s), 2.64 (2H, s), 3.52 (2H, s), 3.83 (3H, s), 5.48 (1H, td, J = 8.5, 6.1 Hz), 7.16 (1H, dd, J = 8.5, 1.8 Hz), 7.45 (1H, d, J = 1.8 Hz), 7.55 (1H, d, J = 8.5 Hz), 8.05 (1H, d, J = 8.5 Hz), 11.65 (1H, brs). |
| | | HRMS (ESI⁺):490.31779 [M+H]⁺ |

**[Table 154]**

| Example | Structure | Equipment Data |
|---|---|---|
| 3 1 - 3 | 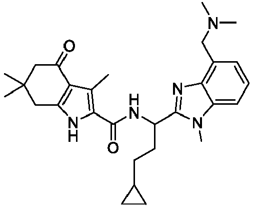 | ¹H-NMR (DMSO-D₆, 400 MHz) δ: -0.01-0.10 (2H, m), 0.43 (2H, d, J = 7.9 Hz), 0.74-0.82 (1H, m), 1.04 (6H, s), 1.28-1.35 (2H, m), 2.11-2.18 (2H, m), 2.22 (6H, s), 2.24 (2H, s), 2.50 (3H, s), 2.64 (2H, s), 3.80-3.90 (5H, m), 5.48 (1H, td, J = 7.9, 6.1 Hz), 7.20-7.26 (2H, m), 7.42 (1H, dd, J = 7.3, 1.8 Hz), 8.08 (1H, d, J = 7.9 Hz), 11.63 (1H, s). |
| | | HRMS (ESI+):490.31726 [M+H]⁺ |
| 3 1 - 4 | 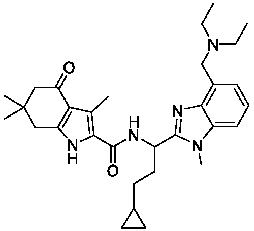 | ¹H-NMR (DMSO-D₆, 400 MHz) δ: -0.02-0.04 (2H, m), 0.37-0.41 (2H, m), 0.71-0.78 (1H, m), 0.90-1.10 (12H, m), 1.25-1.32 (2H, m), 2.05-2.16 (2H, m), 2.21 (2H, s), 2.47 (4H, q, J = 7.3 Hz), 2.48 (3H, s), 2.61 (2H, s), 3.80 (3H, s), 3.90 (1H, d, J = 14.5 Hz), 3.99 (1H, d, J = 14.5 Hz), 5.41-5.48 (1H, m), 7.17-7.24 (2H, m), 7.36 (1H, d, J = 7.9 Hz), 8.00 (1H, d, J = 7.9 Hz), 11.56 (1H, s). |
| | | HRMS (ESI⁺):518.34906 [M+H]⁺ |
| 3 1 - 5 | 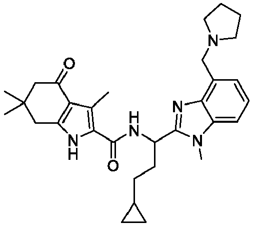 | ¹H-NMR (DMSO-D₆, 400 MHz) δ: -0.01-0.04 (2H, m), 0.38-0.42 (2H, m), 0.72-0.78 (1H, m), 1.00 (6H, s), 1.26-1.32 (2H, m), 1.66-1.70 (4H, m), 2.07-2.19 (2H, m), 2.21 (2H, s), 2.40-2.58 (7H, m), 2.61 (2H, s), 3.80 (3H, s), 3.98 (2H, s), 5.45 (1H, td, J = 8.5, 6.1 Hz), 7.18-7.21 (2H, m), 7.36-7.39 (1H, m), 8.03 (1H, d, J = 8.5 Hz), 11.56 (1H, s). |
| | | HRMS (ESI⁺):516.33311 [M+H]⁺ |
| 3 1 - 6 | 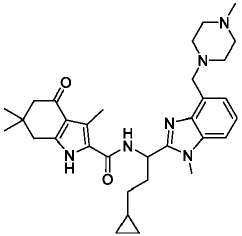 | ¹H-NMR (DMSO-D₆, 400 MHz) δ: -0.02-0.04 (2H, m), 0.38-0.41 (2H, m), 0.71-0.78 (1H, m), 1.00 (6H, s), 1.25-1.32 (2H, m), 2.04-2.11 (2H, m), 2.13 (3H, s), 2.21 (2H, s), 2.26-2.44 (8H, m), 2.47 (3H, s), 2.61 (2H, s), 3.80 (3H, s), 3.85 (1H, d, J = 13.9 Hz), 3.90 (1H, d, J = 13.9 Hz), 5.44 (1H, td, J = 8.5, 6.1 Hz), 7.18-7.22 (2H, m), 7.39 (1H, dd, J = 6.7, 2.4 Hz), 8.02 (1H, d, J = 8.5 Hz), 11.56 (1H, s). |
| | | HRMS (ESI⁺):545.36102 [M+H]⁺ |

**[Table 155]**

| Example | Structure | Equipment Data |
|---|---|---|
| 3 1 - 7 | 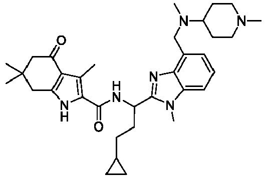 | ¹H-NMR (DMSO-D₆, 400 MHz) δ: -0.02-0.05 (2H, m), 0.37-0.42 (2H, m), 0.71-0.78 (1H, m), 1.00 (6H, s), 1.27-1.30 (2H, m), 1.49-1.56 (2H, m), 1.72-1.79 (4H, m), 2.09 (3H, s), 2.17 (3H, s), 2.20 (2H, s), 2.47 (3H, s), 2.61 (2H, s), 2.69-2.85 (4H, m), 2.94-3.00 (1H, m), 3.80 (3H, s), 3.92 (1H, d, J = 13.9 Hz), 4.00 (1H, d, J = 13.9 Hz), 5.41-5.49 (1H, m), 7.17-7.23 (2H, m), 7.24-7.48 (1H, m), 8.00 (1H, d, J = 7.3 Hz), 11.57 (1H, s). |
| | | HRMS (ESI⁺):573.39200 [M+H]⁺ |

### <Reference example 88>

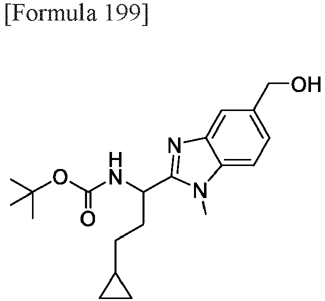

Under an argon atmosphere, a solution of methyl 2-[3-cyclopropyl-1-[(2-methylpropan-2-yl)oxycarbonylamino]propyl]-1-methylbenzoimidazole-5-carboxylate (418 mg) in tetrahydrofuran (2.39 mL) was added to a solution of lithium aluminum hydride (49.0 mg) in tetrahydrofuran (3.00 mL) under ice cooling condition. The mixture was stirred at room temperature for 2 hours. After water (0.050 mL), 15% aqueous sodium hydroxide solution (0.050 mL), and water (0.150 mL) were added stepwise, the mixture was filtered through Celite and concentrated. The residue was purified by silica gel column chromatography (ethyl acetate / hexane = 50%) to obtain tert-butyl N-[3-cyclopropyl-1- [5-(hydroxymethyl)-1-methylbenzoimidazol-2-yl]propyl]carbamate (224 mg).

¹H-NMR (CDCl₃, 400MHz) δ: 0.00-0.08 (2H, m), 0.42-0.47 (2H, m), 0.65-0.73 (1H, m), 1.26-1.35 (2H, m), 1.38 (9H, s), 2.17-2.28 (1H, m), 2.47-2.57 (1H, m), 4.07 (3H, s), 4.84 (2H, s), 5.23-5.28 (1H, m), 7.51 (1H, d, J = 8.5 Hz), 7.58 (1H, dd, J = 8.5, 1.2 Hz), 7.93 (1H, d, J = 1.2 Hz).

HRMS (ESI⁺): 360.22903 [M+H]⁺

### <Reference example 89>

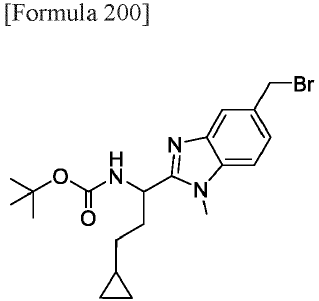

Under an argon atmosphere, triphenylphosphine (130 mg) and carbon tetrabromide (191 mg) were added to a solution of tert-butyl N-[3-cyclopropyl-1-[5- (hydroxymethyl)-1-methylbenzoimidazol-2-yl]propyl]carbamate (148 mg) in dichloromethane (2.06mL) at room temperature, and the mixture was stirred at room temperature for 4 hours. After the reaction mixture was concentrated under reduced pressure, the residue was purified by silica gel column chromatography (ethyl acetate : hexane = 1 : 2) to obtain tert-butyl N-[1-[5-(bromomethyl)-1-methylbenzoimidazol-2-yl]-3-cyclopropylpropyl]carbamate (118 mg).

¹H-NMR (CDCl₃, 400MHz) δ: 0.01-0.07 (2H, m), 0.43-0.48 (2H, m), 0.66-0.72 (1H, m), 1.25-1.34 (2H, m), 1.38 (9H, s), 2.18-2.28 (1H, m), 2.49-2.59 (1H, m), 4.08 (3H, s), 4.59 (2H, s), 5.25 (1H, brs), 7.52 (1H, d, J = 8.5 Hz), 7.60 (1H, d, J = 8.5 Hz), 8.02 (1H, s).

MS (ESI⁺): 422.2 [M+H]⁺

### <Reference example 90>

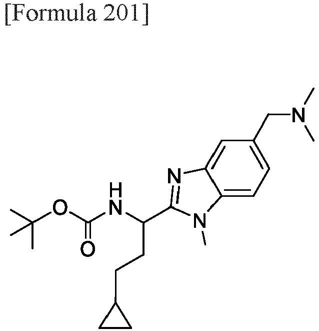

Under an argon atmosphere, dimethylamine (0.279 mL, 2 mol/L tetrahydrofuran solution) was added to a solution of tert-butyl N-[1-[5-(bromomethyl)-1-methylbenzoimidazol-2-yl]-3-cyclopropylpropyl]carbamate (118 mg) in tetrahydrofuran (1.40 mL) at room temperature, and the mixture was stirred at room temperature for 7 hours. After a saturated aqueous sodium hydrogen carbonate solution was added, the reaction mixture was extracted with ethyl acetate. The organic layer was washed with saturated brine, dried over anhydrous sodium sulfate, and concentrated after filtration of the insoluble material. The residue was purified by amino-silica gel column chromatography (hexane : ethyl acetate = 1 : 1) to obtain tert-butyl N-[3-cyclopropyl-1-[5-[(dimethylamino)methyl]-1-methylbenzoimidazol-2-yl] propyl]carbamate (98.4 mg).

¹H-NMR (CDCl₃, 400MHz) δ: 0.00-0.06 (2H, m), 0.40-0.44 (2H, m), 0.70-0.77 (1H, m), 1.25-1.29 (2H, m), 1.40 (9H, s), 1.96-2.06 (2H, m), 2.16 (6H, s), 3.49 (2H, s), 3.80 (3H, s), 4.94 (1H, td, J = 8.5, 6.1 Hz), 7.20 ( 1H, dd, J = 8.5, 1.2 Hz), 7.42-7.47 (2H, m).

HRMS (ESI⁺): 387.27542 [M+H]⁺

### <Example 32>

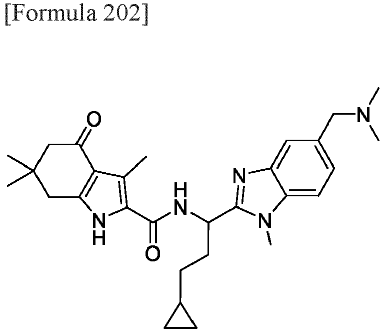

Under an argon atmosphere, trifluoroacetic acid (0.636 mL) was added to a solution of tert-butyl N-[3-cyclopropyl-1-[5-[(dimethylamino)methyl]-1-methylbenzoimidazol-2-yl]propyl]carbamate (98.4 mg) in dichloromethane (0.636 mL), and the mixture was stirred at room temperature for 30 minutes. After the reaction mixture was concentrated under reduced pressure, 3,6,6-Trimethyl-4-oxo-5,7-dihydro-1H-indole-2-carboxylic acid (56. 4 mg), 1-hydroxybenzotriazole monohydrate (46.7 mg), N,N-diisopropylethylamine (0.173 mL), and 1-ethyl-3-(3-dimethylaminopropyl)carbodiimide hydrochloride (53.7 mg) were added to a solution of the obtained crude product in N,N-dimethylformamide (1.27 mL), and the mixture was stirred at room temperature for 7 hours. After water was added, the reaction mixture was extracted with ethyl acetate. The organic layer was washed with saturated brine, dried over anhydrous sodium sulfate, and concentrated after filtration of the insoluble material. The residue was purified by amino-silica gel column chromatography (methanol : ethyl acetate = 1 : 9), and the resulting solid was washed with diisopropyl ether to obtain N-[3-cyclopropyl-1-[5-[(dimethylamino)methyl]-1-methylbenzoimidazol-2-yl]propyl]-3,6,6-trimethyl-4-oxo-5,7-dihydro-1H-indole-2-carboxamide (81.5 mg).

¹H-NMR (DMSO-D₆, 400MHz) δ: -0.01-0.10 (2H, m), 0.43 (2H, dd, J = 8.5, 1.8Hz), 0.72-0.83 (1H, m), 1.03 (3H, s), 1.04 (3H, s), 1.24-1.39 (2H, m), 2.03-2.15 (2H, m), 2.16 (6H, s), 2.24 (2H, s), 2.48 (3H, s), 2.64 ( 2H, s), 3.49 (2H, s), 3.83 (3H, s), 5.43-5.51 (1H, m), 7.21 (1H, dd, J = 8.5, 1.8Hz), 7.46-7.51 (2H, m), 8.07 (1H, d, J = 8.5Hz), 11.67 (1H, brs).

HRMS (ESI⁺): 490.31747 [M+H]⁺

### <Example 33>

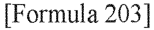

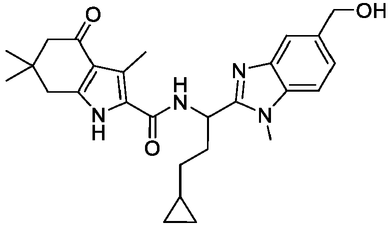

Under an argon atmosphere, trifluoroacetic acid (0.487 mL) was added to a solution of tert-butyl N-[3-cyclopropyl-1-[5-(hydroxymethyl)-1-methylbenzimidazole2-yl]propyl]carbamate (70.0 mg) and the mixture was stirred at room temperature for 30 minutes. After the reaction mixture was concentrated under reduced pressure, 3,6,6-trimethyl-4-oxo-5,7-dihydro-1H-indole-2-carboxylic acid (43.1 mg), 1-hydroxybenzotriazole monohydrate (35.8 mg), N,N-diisopropylethylamine (0.132 mL), and 1-ethyl-3-(3-dimethylaminopropyl)carbodiimide hydrochloride (41.0 mg) were added to a solution of the obtained crude product in N,N-dimethylformamide (0.974 mL), and the mixture was stirred at room temperature for 5 hours. After water was added, the reaction mixture was extracted with ethyl acetate. The organic layer was washed with saturated brine, dried over anhydrous sodium sulfate, and concentrated after filtration of the insoluble material. The residue was purified by silica gel column chromatography (ethyl acetate), and the resulting solid was washed with diisopropyl ether to obtain N-[3-cyclopropyl-1-[5-(hydroxymethyl)-1-methylbenzoimidazol-2-yl]propyl]-3,6,6-trimethyl-4-oxo-5,7-dihydro-1H-indole-2-carboxamide (40.8 mg).

¹H-NMR (DMSO-D₆, 400MHz) δ: -0.01-0.09 (2H, m), 0.43 (2H, dd, J = 7.9, 2.4 Hz), 0.73-0.81 (1H, m), 1.04 (6H, s) ), 1.27-1.35 (2H, m), 2.08-2.22 (2H, m), 2.24 (2H, s), 2.47 (3H, s), 2.65 (2H, s), 3.84 (3H, s), 4.61 ( 2H, d, J = 5.4 Hz), 5.16 (1H, t, J = 5.4 Hz), 5.48 (1H, td, J = 8.5, 6.1 Hz), 7.24 (1H, d, J = 8.5 Hz), 7.49 ( 1H, d, J = 8.5 Hz), 7.56 (1H, s), 8.06 (1H, d, J = 8.5 Hz), 11.61 (1H, s).

HRMS (ESI⁺): 463.27046 [M+H]⁺

### <Reference example 91>

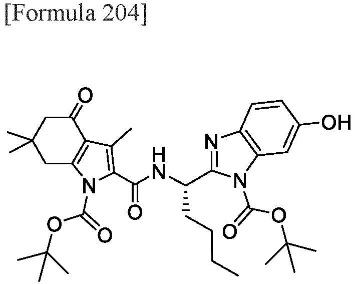

1.01 M di-tert-Butyl dicarbonate in ethyl acetate (441 µL) and 4-dimethylaminopyridine (25 mg) were added to a solution of (S)-N-(1-(6-(benzyloxy)-1H-benzo[d]imidazol-2-yl)pentyl)-3,6,6-trimethyl-4-oxo-4,5,6,7-tetrahydro-1H-indole-2-carboxamide (100 mg) in dichloromethane (1.3 mL) under ice cooling condition, and the mixture was stirred for 1 hour. The reaction mixture was concentrated, and the residue was purified by silica gel column chromatography (ethyl acetate-heptane) to obtain tert-butyl (S)-6-(benzyloxy)-2-(1-(1- (tert-butoxycarbonyl)-3,6,6-trimethyl-4-oxo-4,5,6,7-tetrahydro-1H-indole-2-carboxamide)pentyl)-1H-benzo[d]imidazole-1-carbonate (141 mg).

A suspension of tert-butyl (S)-6-(benzyloxy)-2-(1-(1-(tert-butoxycarbonyl)-3,6,6- trimethyl-4-oxo-4,5,6,7-tetrahydro-1H-indole-2-carboxamide)pentyl)-1H-benzo[d]imidazole-1-carbonate (147 mg) and hydrous 10% palladium carbon (containing 50% water) (44.7 mg) in methanol (5 mL) was stirred under a hydrogen atmosphere for 2 hours. The reaction mixture was filtered through Celite, concentrated under reduced pressure, and azeotroped with acetone to obtain tert-butyl (S)-2-(1-(1-(tert-butoxycarbonyl)-3,6,6-trimethyl-4-oxo-4,5,6,7-tetrahydro-1H-indole-2-carboxamido)pentyl)-6-hydroxy-1H-benzo[d]imidazole-1-carboxylate (127 mg). MS (ESI⁺): 623.19 [M+H]⁺

### <Example 34-1>

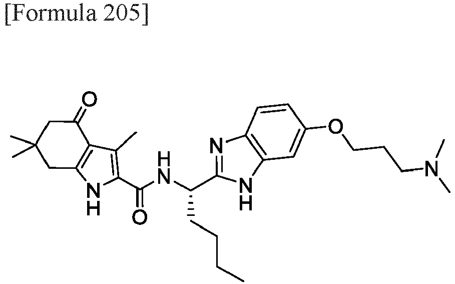

Triphenylphosphine (131 mg), 3-(dimethylamino)propan-1-ol (51.6 mg) and diisopropyl azodicarboxylate (97 µL) were added to a solution of tert-butyl (S)-2-(1-(1-(tert-butoxycarbonyl)-3,6,6-trimethyl-4-oxo-4,5,6,7-tetrahydro-1H-indole-2-carboxamido)pentyl)-6-hydroxy-1H-benzo[d]imidazole-1-carboxylate (125 mg) in toluene (1 mL) under ice-salt cooling condition, then the mixture was warmed to room temperature and stirred overnight. The reaction mixture was concentrated under reduced pressure, and the residue was purified by silica gel column chromatography (dichloromethane-methanol) to obtain tert-butyl (S)-2-(1-(1-(tert-butoxycarbonyl)-3,6,6-trimethyl-4-oxo-4,5,6,7-tetrahydro-1H-indole-2-carboxamido)pentyl)-6-(3-(dimethylamino)propyloxy)-1H-benzo[d]imidazole-1-carboxylate (81.6 mg).

Trifluoroacetic acid (1.8 mL) was added to a solution of tert-butyl (S)-2-(1-(1-(tert-butoxycarbonyl)-3,6,6-trimethyl-4-oxo-4,5,6,7-tetrahydro-1H-indole-2-carboxamido)pentyl)-6-(3-(dimethylamino)propyloxy)-1H-benzo[d]imidazole-1-carboxylate (81.6 mg) in dichloromethane (3.7 mL) and the mixture was stirred overnight at room temperature. The reaction mixture was concentrated under reduced pressure and azeptroped with dichloromethane. The residue was purified by silica gel column chromatography (dichloromethane-methanol) to obtain (S)-N-(1-(6-(3-(dimethylamino)propyloxy)-1H-benzo[d]imidazol-2-yl)pentyl)-3,6,6-trimethyl-4-oxo-4,5,6,7-tetrahydro-1H-indole-2-carboxamide (57.2 mg).

¹H-NMR (400 MHz, DMSO-D₆) δ: 0.87 (3H, t, J = 6.6 Hz), 1.02 (6H, s), 1.26-1.38 (4H, m), 1.78-1.96 (3H, m), 1.98-2.13 (1H, m), 2.16 (6H, s), 2.23 (2H, s), 2.38 (2H, t, J = 7.1 Hz), 2.49 (3H, s), 2.65 (2H, s), 3.99 (2H, t, J = 6.4 Hz), 5.22 (1H, dd, J = 13.7, 7.8 Hz), 6.75 (1H, dd, J = 8.7, 2.3 Hz), 6.93 (0.6H, d, J = 2.3 Hz), 7.07 (0.4H, d, J = 2.3 Hz), 7.30 (0.4H, d, J = 8.7 Hz), 7.42 ( 0.6H, d, J = 8.7 Hz), 7.86 (1H, d, J = 8.2 Hz), 11.64 (1H, s), 12.10 (0.6H, brs), 12.15 (0.4H, brs).

MS (ESI+) : 508.15 [M+H]⁺

Using the corresponding starting material and reactant, the following Examples 34-2 to 34-4 were obtained by the same method in Example 34-1 and the method described in Steps 42-3 to 42-4 or a method similar thereto.

**[Table 156]**

| Example | Structure | Equipment Data |
|---|---|---|
| 34-2 | 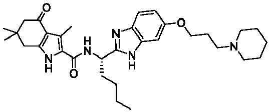 | ¹H-NMR (399 MHz, DMSO-D₆) δ: 0.87 (3H, t, J= 6.6 Hz), 1.02 (6H, s), 1.27-1.41 (6H, m), 1.46-1.51 (4H, m), 1.82-2.09 (3H, m), 2.09-2.15 (1H, m), 2.22 (2H, s), 2.28-2.35 (4H, m), 2.39 (2H, t, J = 6.4 Hz), 2.49 (3H, s), 2.65 (2H, s), 3.98 (2H, t, J = 6.4 Hz), 5.22 (1H, dd, J = 14.4, 8.0 Hz), 6.75 (0.6H, dd, J = 9.1, 2.3 Hz), 6.78 (0.4H, dd, J = 8.7, 1.8 Hz), 6.92 (0.6H, d, J = 2.3 Hz), 7.08 (0.4H, d, J = 1.8 Hz), 7.30 (0.4H, d, J = 8.7 Hz), 7.42 (0.6H, d, J = 9.1 Hz), 7.86 (1H, d, J = 8.2 Hz), 11.64 (1H, s), 12.09 (0.6H, brs), 12.14 (0.4H, brs). MS (ESI⁺): 548.09 [M+H]⁺ |
| 34-3 | 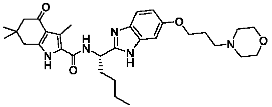 | ¹H-NMR (399 MHz, DMSO-D₆) δ: 0.87 (3H, t, J = 6.9 Hz), 1.02 (6H, s), 1.27-1.38 (4H, m), 1.83-1.95 (3H, m), 1.99-2.09 (1H, m), 2.22 (2H, s), 2.36 (4H, brs), 2.43 (2H, t, J = 7.1 Hz), 2.49 (3H, s), 2.65 (2H, s), 3.57 (4H, t, J = 4.6 Hz), 4.00 (2H, t, J = 6.4 Hz), 5.22 (1H, dt, J = 8.2, 7.5 Hz), 6.75 (0.6H, dd, J = 8.7, 2.3 Hz), 6.78 (0.4H, dd, J = 8.7, 2.3 Hz), 6.93 (0.6H, d, J = 2.3 Hz), 7.08 (0.4H, d, J = 1.8 Hz), 7.31 (0.4H, d, J = 8.7 Hz), 7.42 (0.6H, d, J= 8.7 Hz), 7.85 (1H, d, J = 8.2 Hz), 11.63 (1H, s), 12.09 (0.6H, s), 12.14 (0.4H, s). MS (ESI⁺): 550.07 [M+H]⁺ |

**[Table 157]**

| Example | Structure | Equipment Data |
|---|---|---|
| 34-4 | 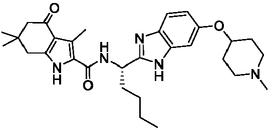 | ¹H-NMR (399 MHz, DMSO-D₆) δ: 0.87 (3H, t, J= 6.2 Hz), 1.02 (6H, s), 1.08-1.71 (8H, m), 1.78-2.06 (4H, m), 2.09-2.15 (2H, m), 2.17 (3H, s), 2.22 (2H, s), 2.49 (3H, s), 2.64 (2H, s), 4.28 (1H, brs), 5.21 (1H, dt, J = 7.8, 7.3 Hz), 6.75-6.82 (1H, m), 6.96 (0.5H, d, J = 1.8 Hz), 7.12 (0.5H, d, J = 1.8 Hz), 7.30 (0.5H, d, J = 8.7 Hz), 7.42 (0.5H, d, J = 8.7 Hz), 7.85 (1H, d, J = 7.8 Hz), 11.63 (1H, s), 12.07 (0.5H, s), 12.15 (0.5H, s). MS (ESI⁺): 520.07 [M+H]⁺ |

### <Example 35-1>

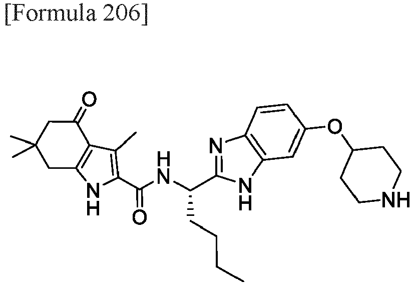

Triphenylphosphine (262 mg), benzyl 4-hydroxypiperidine-1-carboxylate (235 mg) and diisopropyl azodicarboxylate (194 µL) were added to a solution of tert-butyl (S)-2-(1-(1-(tert-butoxycarbonyl)-3,6,6-trimethyl-4-oxo-4,5,6,7-tetrahydro-1H-indole-2-carboxamido)pentyl)-6-hydroxy-1H-benzo[d]imidazole-1-carboxylate (249 mg) in toluene (2.1 mL) under ice-salt cooling condition, and then the mixture was warmed to room temperature and stirred overnight. The reaction mixture was concentrated under reduced pressure, and the residue was purified by silica gel column chromatography (dichloromethane-methanol) to obtain tert-butyl (S)-6-((1-((benzyloxy)carbonyl)piperidin-4-yl)oxy)-2-(1-(1-(tert-butoxycarbonyl)-3,6,6-trimethyl-4-oxo-4,5,6,7-tetrahydro-1H-indole-2-carboxamido)pentyl)-1H-benzo[d]imidazole-1-carboxylate (489 mg).

Trifluoroacetic acid (45 µL) was added to a solution of tert-butyl (S)-6-((1-((benzyloxy)carbonyl)piperidin-4-yl)oxy)-2-(1-(1-(tert-butoxycarbonyl)-3,6,6-trimethyl-4-oxo-4,5,6,7-tetrahydro-lH-indole-2-carboxamido)pentyl)-lH-benzo[d]imidazole-l-carboxylate (489 mg) in dichloromethane (38 µL), and the mixture was stirred overnight at room temperature. The reaction mixture was concentrated under reduced pressure and azeotroped with dichloromethane. The residue was purified by silica gel column chromatography (dichloromethane-methanol) to obtain (S)-3,6,6-trimethyl-4-oxo-N-(1-(6-(piperidin-4-yloxy)-1H-benzo[d]imidazol-2-yl)pentyl)-4,5,6,7-tetrahydro-1H-indole-2-carboxamide (127 mg).

¹H-NMR (399MHz, DMSO-D₆) δ: 0.87 (3H, t, J = 6.9 Hz), 1.02 (6H, s), 1.22-1.40 (4H, m), 1.45-1.65 (2H, m), 1.80-2.10 (4H, m), 2.22 (2H, s), 2.49 (3H, s), 2.55-2.82 (2H, m), 2.65 (2H, s), 2.95-3.09 (2H, m), 3.35 (1H, brs), 4.21-4.47 (1H, m), 5.21 (1H, dd, J = 15.3, 7.1 Hz), 6.79 (1H, d, J = 6.9 Hz), 6.97 (0.6H, brs), 7.14 ( 0.4H, brs), 7.32 (0.4H, brs), 7.42 (0.6H, brs), 7.86 (1H, d, J = 7.8 Hz), 11.63 (1H, s), 12.10 (0.6H, brs), 12.16 (0.4H, brs).

MS (ESI⁺): 506.22 [M+H]⁺

Using the corresponding starting material and reactant, the following Example 35-2 was obtained by the same method in Example 35-1, the method described in Steps 43-1 to 43-3, or a method similar thereto.

**[Table 158]**

| Example | Structure | Equipment Data |
|---|---|---|
| 35-2 | 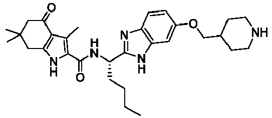 | ¹H-NMR (399 MHz, DMSO-D₆) δ: 0.87 (t, J= 6.6 Hz, 3H), 1.02 (s, 6H), 1.10-1.38 (8H, m), 1.56-2.10 (3H, m), 2.22 (2H, s), 2.40-2.70 (2H, m), 2.49 (3H, s), 2.65 (2H, s), 2.94-3.07 (2H, m), 3.30 (1H, brs), 3.81 (2H, d, J = 6.4 Hz), 5.22 (1H, dd, J = 14.2, 8.2 Hz), 6.76 (1H, d, J = 8.7 Hz), 6.89-7.07 (1H, brs), 7.30-7.46 (1H, brs), 7.88 (1H, d, J = 7.8 Hz), 11.70 (1H, brs), 12.15 (1H, brs). MS (ESI+): 520.24 [M+H]⁺ |

### <Example 36>

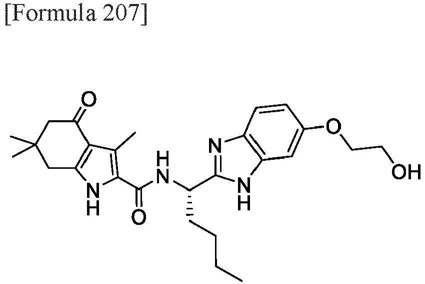

Triphenylphosphine (262 mg), 2-((t-butyldimethylsilyl)oxy)ethanol (176 mg) and diisopropyl azodicarboxylate (194 µL) were added to a solution of tert-butyl (S)-2-(1-(1-(tert-butoxycarbonyl)-3,6,6-trimethyl-4-oxo-4,5,6,7-tetrahydro-1H-indole-2-carboxamido)pentyl)-6-hydroxy-1H-benzo[d]imidazole-1-carboxylate (249 mg) in toluene (2.13 mL) under ice-salt cooling condition, and then the mixture was warmed to room temperature and stirred overnight. The reaction mixture was concentrated under reduced pressure, and the residue was purified by silica gel column chromatography (dichloromethane-methanol) to obtain tert-butyl (S)-2-(1-(1-(tert-butoxycarbonyl)-3,6,6-trimethyl-4-oxo-4,5,6,7-tetrahydro-1H-indole-2-carboxamido)pentyl)-6-(2-((tert-butyldimethylsilyl)oxy)ethoxy)-1H-benzo[d]imidazole-1-carboxylate (260 mg).

Trifluoroacetic acid (12.8 mL) was added to a solution of tert-butyl (S)-2-(1-(1-(tert-butoxycarbonyl)-3,6,6-trimethyl-4-oxo-4,5,6,7-tetrahydro-1H-indole-2-carboxamido) pentyl)-6-(2-((tert-butyldimethylsilyl)oxy)ethoxy)-1H-benzo[d]imidazole-1-carboxylate (260 mg) in dichloromethane (2.14 mL) and the mixture was stirred overnight at room temperature. The reaction mixture was concentrated under reduced pressure and azeotroped twice with dichloromethane. The residue was purified by silica gel column chromatography (dichloromethane-methanol-ammonia) to obtain (S)-N-(1-(6-(2-hydroxyethoxy)-1H-benzo[d]imidazol-2-yl)pentyl)-3,6,6-trimethyl-4-oxo-4,5,6,7-tetrahydro-1H-indole-2-carboxamide (49.5 mg).

¹H-NMR (399MHz, DMSO-D₆) δ: 0.87 (3H, t, J = 6.9 Hz), 1.03 (6H, s), 1.25-1.41 (4H, m), 1.85-2.14 (2H, m), 2.23 (2H, s), 2.49 (3H, s), 2.65 (2H, s), 3.34 (1H, brs), 3.73 (2H, t, J = 5.0 Hz), 3.99 (2H, t, J = 5.0 Hz) ), 4.87 (1H, brs), 5.23 (1H, dd, J = 14.2, 8.2 Hz), 6.84 (1H, dd, J = 8.7, 1.8 Hz), 7.04 (1H, d, J = 1.8 Hz), 7.43 (1H, d, J = 8.7 Hz), 7.90 (1H, d, J = 7.8 Hz), 11.65 (1H, s).

MS (ESI⁺): 467.18 [M+H]⁺

### <Reference example 92>

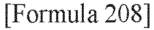

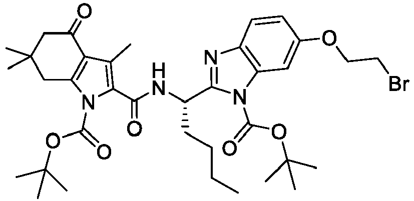

Triphenylphosphine (26.2 mg), 2-bromoethanol (9.4 mg) and diisopropyl azodicarboxylate (20.2 µL) were added to a solution of tert-butyl (S)-2-(1-(1-(tert-butoxycarbonyl)-3,6,6-trimethyl-4-oxo-4,5,6,7-tetrahydro-1H-indole-2-carboxamido)pentyl)-6-hydroxy-1H-benzo[d]imidazole-1-carboxylate (31.1 mg) in toluene (230 µL) under ice-salt cooling condition, and then the mixture was warmed to room temperature and stirred overnight. The reaction mixture was concentrated under reduced pressure, and the residue was purified by silica gel column chromatography (dichloromethane-methanol) to obtain tert-butyl (S)-6-(2-bromoethoxy)-2-(1-(1-(tert-butoxycarbonyl)-3,6,6-trimethyl-4-oxo-4,5,6,7-tetrahydro-1H-indole-2-carboxamido)pentyl)-1H-benzo[d]imidazole-1-carboxylate (48.4 mg).

MS (ESI⁺): 729.06 [M+H]⁺

### <Reference example 93>

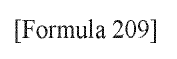

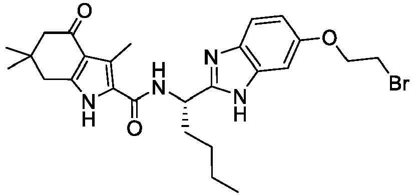

Trifluoroacetic acid (51.1 µL) was added to a solution of tert-butyl (S)-6-(2-bromoethoxy)-2-(1-(1-(tert-butoxycarbonyl)-3,6,6-trimethyl-4-oxo-4,5,6,7-tetrahydro-1H-indole-2-carboxamido)pentyl)-1H-benzo[d]imidazole-1-carboxylate (48.4 mg) in dichloromethane (563 µL) and the mixture was stirred overnight at room temperature. The reaction mixture was concentrated under reduced pressure and azeotroped with dichloromethane. Ethyl acetate and saturated sodium hydrogen carbonate were added, and the organic layer was separated. The aqueous layer was extracted twice with ethyl acetate, and the combined organic layer was dried over sodium sulfate, filtered, and concentrated under reduced pressure. The residue was purified by silica gel column chromatography (dichloromethane-methanol) to obtain (S)-N-(1-(6-(2-bromoethoxy)-1H-benzo[d]imidazol-2-yl)pentyl)-3,6,6-trimethyl-4-oxo-4,5,6,7-tetrahydro-1H-indole-2-carboxamide (37.5 mg).

MS (ESI⁺): 529.00 [M+H]⁺

### <Example 37-1>

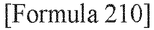

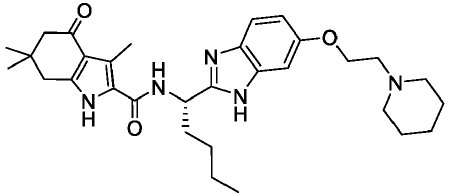

Piperidine (25.5 mg) was added to a solution of (S)-N-(1-(6-(2-bromoethoxy)-1H-benzo[d]imidazol-2-yl)pentyl)-3,6,6-trimethyl-4-oxo-4,5,6,7-tetrahydro-1H-indole-2-carboxamide (53 mg) in acetonitrile (2 mL), and the mixture was stirred overnight at 50 °C. The reaction mixture was concentrated under reduced pressure, and the residue was purified by silica gel column chromatography (dichloromethane-methanol) to obtain (S)-3,6,6-trimethyl-4-oxo-N-(1-(6-(2-(piperidin-1-yl)ethoxy)-1H-benzo[d]imidazol-2-yl)pentyl)-4,5,6,7-tetrahydro-1H-indole-2-carboxamide (17.5 mg).

¹H-NMR (500MHz, DMSO-D₆) δ: 0.87 (3H, s), 1.03 (6H, s), 1.12-1.43 (6H, m), 1.45-1.58 (4H, m), 1.84-1.97 (1H, m), 1.97-2.15 (1H, m), 2.23 (2H, s), 2.45 (3H, s), 2.51 (4H, m), 2.66 (4H, m), 4.06 (2H, m), 5.23 ( 1H, m), 6.77 (1H, m), 6.95 (0.6H, m), 7.10 (0.4H, m), 7.32 (0.4H, m), 7.43 (0.6H, m), 7.87 (1H, m) , 11.65 (1H, s), 12.12 (1H, s).

MS (ESI⁺): 534.13 [M+H]⁺

Using the corresponding starting materials and reactants, the following Examples 37-2 to 37-4 were obtained by the same method in Example 37-1, the method described in Step 45-3, or a method similar thereto.

**[Table 159]**

| Example | Structure | Equipment Data |
|---|---|---|
| 37-2 | 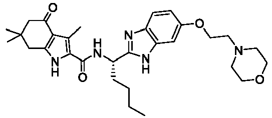 | ¹H-NMR (500 MHz, DMSO-D₆) δ: 0.87 (3H, m), 1.02 (6H, s), 1.11-1.43 (4H, m), 1.85-1.97 (1H, m), 1.97-2.10 (1H, m), 2.23 (2H, s), 2.51 (6H, m), 2.65 (3H, s), 2.70 (2H, s), 3.58 (4H, m), 4.08 (2H, s), 5.21-5.21 (1H, m), 6.80-6.75 (1H, m), 6.95 (0.6H, s), 7.11 (0.4H, s), 7.31 (0.4H, d, J = 7.5 Hz), 7.43 (0.6H, d, J = 10.6 Hz), 7.86-7.86 (1H, m), 11.64 (1H, brs), 12.14 (1H, m). MS (ESI⁺): 536.08 [M+H]⁺ |
| 37-3 | 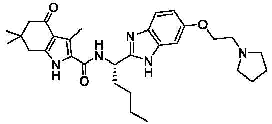 | H-NMR (399 MHz, DMSO-D₆) δ: 0.87 (3H, t, J = 6.6 Hz), 1.02 (6H, s), 1.26-1.39 (4H, m), 1.65-1.73 (4H, m), 1.81-1.97 (1H, m), 1.97-2.10 (1H, m), 2.22 (2H, s), 2.47-2.57 (7H, m), 2.65 (2H, s), 2.79 (2H, t, J = 5.9 Hz), 4.06 (2H, t, J = 5.7 Hz), 5.22 (1H, dd, J = 14.4, 8.0 Hz), 6.73-6.82 (1H, m), 6.94 (0.5H, d, J = 2.3 Hz), 7.10 (0.5H, d, J = 2.3 Hz), 7.31 (0.5H, d, J = 8.7 Hz ), 7.43 (0.5H, d, J = 8.7 Hz), 7.85 (1H, d, J = 7.8 Hz), 11.64 (1H, s), 12.11 (0.5H, s), 12.15 (0.5H, s) MS (ESI⁺): 520.14 [M+H]⁺ |
| 37-4 | 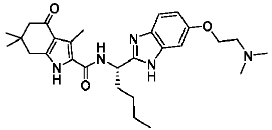 | ¹H-NMR (399 MHz, DMSO-D₆) δ: 0.87 (3H, t, J 6.6 Hz), 1.02 (6H, s), 1.25-1.40 (4H, m), 1.78-2.10 (2H, m), 2.22 (8H, s), 2.49 (3H, s), 2.63 (2H, t, J = 5.7 Hz), 2.65 (2H, s), 4.04 (2H, t, J = 5.7 Hz), 5.22 (1H, dd, J = 14.2, 8.2 Hz), 6.74-6.80 (1H, m), 6.95 (0.5H, d, J = 2.3 Hz), 7.10 (0.5H, d, J = 1.8 Hz), 7.31 (0.5H, d, J = 8.7 Hz), 7.43 (0.5H, d, J = 9.1 Hz), 7.86 (1H, d, J = 8.2 Hz), 11.64 (1H, s), 12.11 (0.5H, brs), 12.15 (0.5H, brs) MS (ESI⁺): 494.14 [M+H]⁺ |

### <Reference example 94>

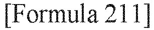

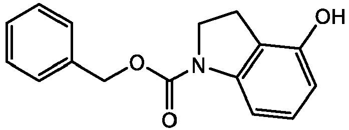

### Synthesis of benzyl 4-hydroxyindoline-1-carboxylate.

A solution of 4-hydroxyindole (5.32 g) in acetic acid (100 mL) was cooled under ice cooling condition, and sodium cyanoborohydride (7.54 g) was added portionwise over about 30 minutes at 15 to 20 °C. The ice bath was removed, and the mixture was stirred at room temperature for 1 hour and concentrated under reduced pressure. The mixture was azeotroped with methanol, ethanol and toluene, and the residue was diluted with saturated aqueous sodium hydrogen carbonate (150 mL) and tetrahydrofuran (30 mL). A solution of benzyl chloroformate (5.64 g) in ethyl acetate (5 mL) was added to the mixture under ice cooling condition, and the mixture was stirred at room temperature for 30 minutes. The organic layer was separated, washed with saturated brine, dried over anhydrous sodium sulfate, and concentrated. The precipitate was washed with ethyl acetate/hexane (1/9) to obtain benzyl 4-hydroxyindoline-1-carboxylate 7.16 g (66.5%).

¹H-NMR (CDCl₃, 400MHz) δ: 3.05 (2H, t, J = 8.6 Hz), 4.09 (2H, t, J = 8.6 Hz), 5.09 (1H, s), 5.26 (2H, s), 6.44 (1H, d, J = 8.4 Hz), 7.05 (1H, br s), 7.33-7.55 (6H, m).

MS (ESI⁺): 270 [M+H]⁺; (ESI-): 268 [M-H]⁻

### <Reference example 95>

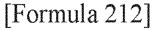

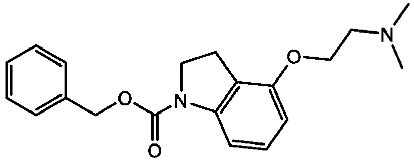

### Synthesis of benzyl 4-(2-(dimethylamino)ethoxy)indoline-1-carboxylate.

2-(Dimethylamino)ethanol (267 mg), triphenylphosphine (918 mg) and bis(2-methoxyethyl)azodicarboxylate (703 mg) were sequentially added to a solution of benzyl 4-hydroxyindoline-1-carboxylate (269 mg) in tetrahydrofuran (5 mL) under ice cooling condition. After stirring overnight at room temperature, the reaction mixture was concentrated, and the residue was purified by amino-silica gel column chromatography (10-50% ethyl acetate-hexane) to obtain 252 mg (74.0%) of benzyl 4-(2-(dimethylamino)ethoxy)indoline-1-carboxylate.

¹H-NMR (CDCl₃, 400MHz) δ: 2.33 (6H, s), 2.72 (2H, t, J = 6.0 Hz), 3.04 (2H, t, J = 8.0 Hz), 4.05 (2H, t, J = 8.0 Hz), 4.10 (2H, t, J = 6.0 Hz), 5.25 (2H, s), 6.51 (1H, d, J = 8.4 Hz), 7.14 (1H, br. s), 7.32-7.55 (6H, m).

MS (ESI⁺): 341.3 [M+H]⁺

### <Reference example 96-1>

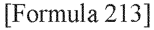

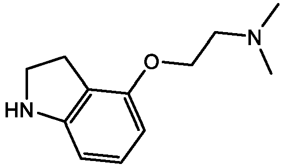

### Synthesis of 2-(indolin-4-yloxy)-N,N-dimethylethan-1-amine.

20% Palladium carbon (40% wet, 100 mg) was added to a solution of benzyl 4-(2-(dimethylamino)ethoxy)indoline-1-carboxylate (240 mg) in methanol (5 mL) under ice cooling condition, and the mixture was stirred under a hydrogen atmosphere for 30 minutes. The reaction mixture was filtered through Celite and concentrated to obtain 2-(indolin-4-yloxy)-N,N-dimethylethan-1-amine as a residue of the filtrate. This residue was used in the next condensation step without further purification for the synthesis of the compound of Example 1-122.

MS (ESI⁺): 207 [M+H]⁺

Using the corresponding starting materials and reactants, the following Reference Examples 96-2 to 96-9 were obtained by the same method in Reference Example 94 to 96-1 or a method similar thereto.

**[Table 160]**

| **Reference Example** | | **¹HNMR Data** | **MS Data [M+1]⁺** |
|---|---|---|---|
| 96-2 | 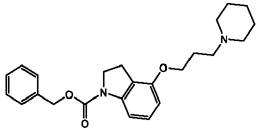 | ¹H-NMR (CDCl₃, 400 MHz) δ: 1.38-1.49 (2H, m), 1.54-1.64 (4H, m), 1.91-2.01 (2H, m), 2.31-2.49 (6H, m), 3.03 (2H, t, J = 8.7 Hz), 3.99-4.10 (4H, m), 5.25 (2H, s), 6.51 (1H, d, J = 8.2 Hz), 7.12 (1H, br. s), 7.29-7.58 (6H, m) | 395 |
| 96-3 | 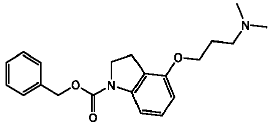 | ¹H-NMR (CDCl₃, 400 MHz) δ: 1.89-1.99 (2H, m), 2.25 (6H, s), 2.41-2.47 (2H, m), 3.03 (2H, t, J = 8.7 Hz), 3.97-4.10 (4H, m), 5.25 (2H, br. s), 6.52 (1H, d, J = 8.2 Hz), 7.12 (1H, br. s), 7.28-7.58 (6H, m). | 355 |
| 96-4 | 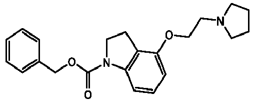 | ¹H-NMR (CDCl₃, 400 MHz) δ: 1.74-1.84 (4H, m), 2.60-2.67 (4H, m), 2.89 (2H, t, J = 6.0 Hz), 3.04 (2H, t, J = 8.7 Hz), 4.05 (2H, t, J = 8.7 Hz), 4.15 (2H, t, J = 6.0 Hz), 5.25 (2H, br. s), 6.52 (1H, d, J = 8.2 Hz), 7.12 (1H, br. s), 7.28-7.58 (5H, m), 7.62-7.72 (1H, m). | 367 |
| 96-5 | 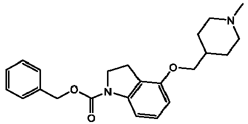 | ¹H-NMR (CDCl₃, 400 MHz) δ: 1.36-1.50 (2H, m), 1.63-2.01 (5H, m), 2.28 (3H, s), 2.85-2.93 (2H, m), 3.04 (2H, t, J = 8.7 Hz), 3.83 (2H, d, J = 5.9 Hz), 4.06 (2H, t, J = 8.7 Hz), 5.25 (2H, br. s), 6.49 (1H, d, J = 8.2 Hz), 7.13 (1H, br. s), 7.28-7.71 (6H, m). | 381 |
| 96-6 | 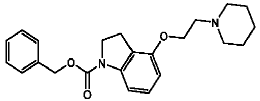 | ¹H-NMR (CDCl₃, 400 MHz) δ: 1.38-1.49 (2H, m), 1.54-1.65 (4H, m), 2.39-2.59 (4H, m), 2.77 (2H, t, J = 6.0 Hz), 3.03 (2H, t, J = 8.6 Hz), 4.06 (2H, t, J = 8.6 Hz), 4.13 (2H, t, J = 6.0 Hz), 5.25 (2H, br.s), 6.51 (1H, d, J = 8.2 Hz), 7.13 (2H, s), 7.30-7.60 (5H, m). | 381 |
| 96-7 | 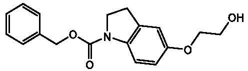 | ¹H-NMR (CDCl₃, 400 MHz) δ: 2.10 (1H, s), 3.08 (2H, t, J = 8.6 Hz), 3.89-3.97 (2H, m), 3.99-4.10 (4H, m), 5.24 (2H, s), 6.59-6.78 (2H, m), 7.29-7.48 (5H, m), 7.79 (1H, d, J = 8.6 Hz). | 314 |
| 96-8 | 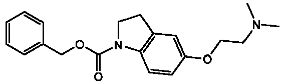 | | 341 |
| 96-9 | 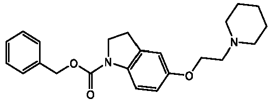 | | 381 |

### <Reference example 97>

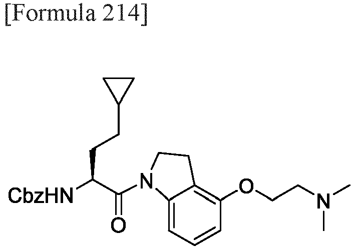

### Synthesis of benzyl (S)-(4-cyclopropyl-1-(4-(2-(dimethylamino)ethoxy)indolin-1-yl)-1-oxobutan-2-yl)carbamate.

A suspension of 20% palladium carbon (40% wet, 106 mg) in methanol (3.0 mL) was added to a solution of benzyl 4-hydroxyindoline-1-carboxylate (408 mg) in methanol (7.0 mL). The mixture was stirred at room temperature under a hydrogen atmosphere for 1 hour. The reaction mixture was filtered through Celite, and the filtrate was concentrated under reduced pressure.

The residue was diluted in dimethylformamide (8.0 mL) under a nitrogen stream, and (S)-2-(((benzyloxy)carbonyl)amino)-4-cyclopropylbutyric acid (277 mg), 1-hydroxy benzotriazole monohydrate (230 mg) and diisopropylethylamine (261 µL) were added and the mixture was cooled under ice bath. 1-Ethyl-3-(3-dimethylaminopropyl)carbodiimide hydrochloride (288 mg) was added, and the mixture was stirred at room temperature for 2.5 hours. After water (30 mL) was added to the reaction mixture, the aqueous layer was extracted with ethyl acetate (7 mL × 5). The organic layer was washed with saturated brine (20 mL) and dried over sodium sulfate. The concentrated residue was purified twice by silica gel column chromatography (0-8% methanol-dichloromethane) and amino-silica gel chromatography (10-50% ethyl acetate-hexane) to obtain 268 mg (58%) of benzyl (S)-(4-cyclopropyl-1-(4-(2-(dimethylamino)ethoxy)indolin-1-yl)-1-oxobutan-2-yl)carbamate.

¹H-NMR (DMSO-d₆, 400MHz) δ: -0.07-0.09 (2H, m), 0.34-0.46 (2H, m), 0.60-0.73 (1H, m), 1.16-1.43 (2H, m), 1.66-1.80 (1H, m), 1.82-1.94 (1H, m), 2.33 (6H, s), 2.72 (2H, t, J = 5.8 Hz), 3.09-3.18 (2H, m), 4.10 (2H, t, J = 5.8 Hz), 4.05-4.14 (1H, m), 4.26-4.33 (1H, m), 4.59-4.66 (1H, m), 5.06 (1H, d, J = 12.3 Hz), 5.10 (1H, d, J = 12.3 Hz), 5.61 (1H, d, J = 9.0Hz), 6.59 (1H, d, J = 8.2 Hz), 7.14 (1H, t, J = 8.2 Hz), 7.10-7.18 (5H, s), 7.81 (1H, d, J = 8.2 Hz).

MS (ESI⁺): 466 [M+H]⁺

### <Reference example 98>

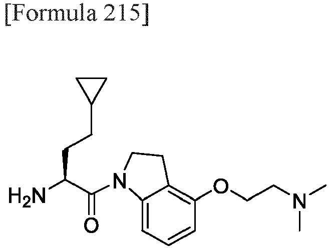

Synthesis of (S)-2-amino-4-cyclopropyl-1-(4-(2-(dimethylamino)ethoxy)indolin-1-yl)butan-1-one. A suspension of 20% palladium carbon (40% wet, 33.9 mg) in methanol (3.0 mL) was added to a solution of benzyl (S)-(4-cyclopropyl-1-(4-(2-(dimethylamino)ethoxy)indolin-1-yl)-1-oxobutan-2-yl)carbamate (178 mg) in methanol (5.0 mL) and the mixture was stirred at room temperature under a hydrogen atmosphere for 1 hour. The reaction mixture was filtered through Celite to obtain (S)-2-amino-4-cyclopropyl-1-(4-(2-(dimethylamino)ethoxy)indolin-1-yl)butan-1-one. The filtrate was concentrated under reduced pressure and the residue was used in the next step for the synthesis of the compound of Example 6-107 without further purification.

MS (ESI⁺): 332 [M+H]⁺

### <Test Example 1>

### G9a inhibitory activity test

The G9a inhibitory activity of each compound was evaluated by measuring the enzymatic activity of G9a by amplified luminescence proximity homogeneous assay (ALPHA). First, 7.5 µL of a dilution of recombinant human G9a protein (BPS Bioscience Inc., #51001) diluted to 0.05 to 0.1 nM with a Tris buffer solution (50 mM Tris-HCl [pH 9.0], 50 mM NaCl, 0.01% Tween-20, 1 mM DTT), and 0.5 µL of the test compound were added to each well of a 384-well microplate (AlphaPlate-384 Shallow Well, PerkinElmer, Inc., #6008359), mixed by vortex, and incubated at room temperature for 10 minutes. 500 nM biotinylated histone H3 peptide (1-21) (AnaSpec Inc., #61702) and 150 µM SAM (Sigma-Aldrich Co. LLC, #A7007) were mixed at 1:1 in advance, and 2 µL of the mixture was added to each well, mixed by vortex, and then incubated at room temperature for 1 hour. AlphaLISA anti-H3K9me2 acceptor beads (PerkinElmer, Inc., #AL117C) and AlphaScreen streptavidin donor beads (PerkinElmer, Inc., #6760002B) were each diluted with an epigenetic buffer (PerkinElmer, Inc., AlphaLISA Epigenetics Buffer Kit #AL008C) to a final concentration of 10 µg/ml after addition. These dilutions were added to the reaction solution in the dark and incubated at room temperature for 1 hour in the dark. Then, measurement was performed using the EnSpire Alpha plate reader (PerkinElmer, Inc., Waltham, MA, USA). The G9a inhibitory activity of the test compound was calculated as a compound concentration necessary for suppressing G9a enzymatic activity by 50% (IC₅₀ value), by determining the percent inhibition of the compound when the value of a control without the addition of the compound was defined as 0% and the value of a control without the addition of the enzyme was defined as 100%. The results are shown below.

The tables indicate IC₅₀ < 50 nM: +++, 50 nM ≤ IC₅₀ < 200 nM: ++, 200 nM ≤ IC₅₀: +.

**[Table 161]**

| Example No. | Inhibitory activity |
|---|---|
| 1-1 | + |
| 1-2 | ++ |
| 1-3 | ++ |
| 1-4 | +++ |
| 1-5 | +++ |
| 1-6 | ++ |
| 1-7 | + |
| 1-8 | +++ |
| 1-9 | + |
| 1-10 | + |
| 1-11 | + |
| 1-12 | + |
| 1-13 | + |
| 1-14 | + |
| 1-15 | + |
| 1-16 | ++ |
| 1-17 | + |
| 1-18 | ++ |
| 1-19 | + |
| 1-20 | +++ |
| 1-21 | + |
| 1-22 | + |
| 1-23 | ++ |

**[Table 162]**

| Example No. | Inhibitory activity |
|---|---|
| 1-24 | + |
| 1-25 | + |
| 1-26 | + |
| 1-27 | + |
| 1-28 | + |
| 1-29 | + |
| 1-30 | + |
| 1-31 | + |
| 1-32 | +++ |
| 1-33 | + |
| 1-34 | + |
| 1-35 | + |
| 1-36 | + |
| 1-37 | + |
| 1-38 | + |
| 1-39 | + |
| 1-40 | + |
| 1-41 | + |
| 1-42 | + |
| 1-43 | + |
| 1-44 | + |
| 1-45 | + |
| 1-46 | + |
| 1-47 | ++ |
| 1-48 | + |
| 1-49 | + |
| 1-50 | + |
| 1-51 | +++ |
| 1-52 | + |
| 1-53 | ++ |
| 1-54 | + |
| 1-55 | + |
| 1-56 | + |
| 1-57 | + |
| 1-58 | + |
| 1-59 | ++ |
| 1-60 | + |
| 1-61 | ++ |
| 1-62 | ++ |
| 1-63 | ++ |
| 1-64 | + |
| 1-65 | + |
| 1-66 | +++ |

**[Table 163]**

| Example No. | Inhibitory activity |
|---|---|
| 1-67 | +++ |
| 1-68 | + |
| 1-69 | + |
| 1-70 | ++ |
| 1-71 | + |
| 1-72 | + |
| 1-73 | + |
| 1-74 | ++ |
| 1-75 | + |
| 1-76 | + |
| 1-77 | + |
| 1-78 | + |
| 1-79 | ++ |
| 1-80 | + |
| 1-81 | ++ |
| 1-82 | ++ |
| 1-83 | +++ |
| 1-84 | +++ |
| 1-85 | +++ |
| 1-86 | + |
| 1-87 | ++ |
| 1-88 | +++ |
| 1-89 | +++ |
| 1-90 | + |
| 1-91 | + |
| 1-92 | + |
| 1-93 | ++ |
| 1-94 | + |
| 1-95 | + |
| 1-96 | + |
| 1-97 | + |
| 1-98 | +++ |
| 1-99 | + |
| 1-100 | +++ |
| 1-101 | + |
| 1-102 | + |
| 1-103 | + |
| 1-104 | ++ |
| 1-105 | + |
| 1-106 | + |
| 1-107 | + |
| 1-108 | +++ |
| 1-109 | + |

**[Table 164]**

| Example No. | Inhibitory activity |
|---|---|
| 1-110 | ++ |
| 1-111 | ++ |
| 1-112 | + |
| 1-113 | +++ |
| 1-114 | + |
| 1-115 | + |
| 2-1 | ++ |
| 2-2 | + |
| 2-3 | ++ |
| 2-4 | + |
| 3-1 | ++ |
| 3-2 | + |
| 4 | + |
| 5-1 | + |
| 5-2 | + |
| 5-3 | + |
| 6-1 | +++ |
| 6-2 | +++ |
| 6-3 | ++ |
| 6-4 | ++ |
| 6-5 | ++ |
| 6-6 | ++ |
| 6-7 | + |
| 6-8 | ++ |
| 6-9 | + |
| 6-10 | +++ |
| 6-11 | ++ |
| 6-12 | ++ |
| 6-13 | + |
| 6-14 | ++ |
| 6-15 | +++ |
| 6-16 | + |
| 6-17 | ++ |
| 6-18 | ++ |
| 6-19 | + |
| 6-20 | +++ |
| 6-21 | + |
| 6-22 | ++ |
| 6-23 | + |
| 6-24 | + |
| 6-25 | + |
| 6-26 | +++ |
| 6-27 | ++ |

**[Table 165]**

| Example No. | Inhibitory activity |
|---|---|
| 6-28 | + |
| 6-29 | + |
| 6-30 | +++ |
| 6-31 | +++ |
| 6-32 | +++ |
| 6-33 | + |
| 6-34 | +++ |
| 6-35 | ++ |
| 6-36 | ++ |
| 6-37 | + |
| 6-38 | +++ |
| 6-39 | ++ |
| 6-40 | ++ |
| 6-41 | ++ |
| 6-42 | ++ |
| 6-43 | + |
| 6-44 | + |
| 6-45 | ++ |
| 6-46 | ++ |
| 6-47 | +++ |
| 6-48 | + |
| 6-49 | +++ |
| 6-50 | + |
| 6-51 | +++ |
| 6-52 | + |
| 6-53 | + |
| 6-54 | + |
| 6-55 | +++ |
| 6-56 | + |
| 6-57 | +++ |
| 6-58 | ++ |
| 6-59 | + |
| 6-60 | + |
| 6-61 | + |
| 6-62 | +++ |
| 6-63 | +++ |
| 6-64 | + |
| 6-65 | +++ |
| 6-66 | + |
| 6-67 | + |
| 6-68 | + |
| 6-69 | + |
| 6-70 | +++ |

**[Table 166]**

| Example No. | Inhibitory activity |
|---|---|
| 6-71 | + |
| 6-72 | + |
| 6-73 | + |
| 6-74 | ++ |
| 6-75 | + |
| 6-76 | + |
| 6-77 | +++ |
| 6-78 | +++ |
| 6-79 | +++ |
| 6-80 | +++ |
| 6-81 | +++ |
| 6-82 | +++ |
| 6-83 | ++ |
| 6-84 | +++ |
| 6-85 | +++ |
| 6-86 | +++ |
| 6-87 | +++ |
| 6-88 | +++ |
| 6-89 | +++ |
| 6-90 | + |
| 6-91 | + |
| 6-92 | ++ |
| 6-93 | + |
| 6-94 | + |
| 6-95 | + |
| 6-96 | + |
| 6-97 | + |
| 6-98 | + |
| 6-99 | + |
| 6-100 | +++ |
| 6-101 | + |
| 7 | ++ |
| 8 | + |
| 9-1 | + |
| 9-2 | ++ |
| 10 | ++ |
| 11-1 | + |
| 11-2 | + |
| 11-3 | ++ |
| 11-4 | + |
| 11-5 | +++ |
| 11-6 | + |
| 11-7 | + |

**[Table 167]**

| Example No. | Inhibitory activity |
|---|---|
| 11-8 | + |
| 11-9 | + |
| 11-10 | + |
| 11-11 | + |
| 12-1 | ++ |
| 12-2 | + |
| 13-1 | +++ |
| 13-2 | + |
| 13-3 | + |
| 13-4 | + |
| 13-5 | + |
| 13-6 | + |
| 14-1 | +++ |
| 14-2 | +++ |
| 15-1 | +++ |
| 15-2 | ++ |
| 16 | + |
| 17-1 | + |
| 17-2 | + |
| 18-1 | +++ |
| 18-2 | +++ |
| 18-3 | +++ |
| 18-4 | +++ |
| 18-5 | + |
| 18-6 | +++ |
| 18-7 | + |
| 18-8 | + |
| 18-9 | +++ |
| 18-10 | +++ |
| 18-11 | +++ |
| 18-12 | +++ |
| 18-13 | +++ |
| 18-14 | +++ |
| 19 | +++ |
| 20-1 | + |
| 20-2 | + |
| 21 | + |
| 22-1 | + |
| 22-2 | ++ |
| 22-3 | + |
| 22-4 | + |
| 22-5 | ++ |
| 23 | + |

**[Table 168]**

| Example No. | Inhibitory activity |
|---|---|
| 24-1 | ++ |
| 24-2 | +++ |
| 24-3 | +++ |
| 24-4 | ++ |
| 24-5 | + |
| 25-1 | + |
| 25-2 | ++ |
| 25-3 | ++ |
| 26-1 | ++ |
| 26-2 | +++ |
| 26-3 | ++ |
| 26-4 | +++ |
| 26-5 | ++ |
| 26-6 | ++ |
| 26-7 | +++ |
| 26-8 | +++ |
| 27 | ++ |
| 28 | +++ |
| 29 | + |
| 30-1 | ++ |
| 30-2 | ++ |
| 30-3 | + |
| 30-4 | + |
| 31-1 | + |
| 31-2 | + |
| 31-3 | + |
| 31-4 | + |
| 31-5 | ++ |
| 31-6 | + |
| 31-7 | + |
| 32 | + |
| 33 | + |
| 34-1 | + |
| 34-2 | ++ |
| 34-3 | + |
| 34-4 | + |
| 35-1 | ++ |
| 35-2 | +++ |
| 36 | + |
| 37-1 | + |
| 37-2 | + |
| 37-3 | + |
| 37-4 | + |

**[Table 169]**

| **Example No.** | **Inhibitory activity** |
|---|---|
| 1-116 | + |
| 1-117 | + |
| 1-118 | + |
| 1-119 | + |
| 1-120 | ++ |
| 1-121 | + |
| 1-122 | ++ |
| 1-123 | ++ |
| 1-124 | ++ |
| 1-125 | + |
| 1-126 | +++ |
| 1-127 | ++ |
| 1-128 | +++ |
| 1-129 | + |
| 1-130 | + |
| 1-131 | + |
| 1-132 | + |
| 1-133 | + |
| 1-134 | ++ |
| 1-135 | ++ |
| 1-136 | + |
| 1-137 | +++ |
| 1-138 | +++ |
| 1-139 | +++ |
| 1-140 | + |
| 1-141 | ++ |
| 1-142 | ++ |

**[Table 170]**

| **Example No.** | **Inhibitory activity** |
|---|---|
| 1-143 | + |
| 1-144 | + |
| 1-145 | + |
| 1-146 | + |
| 1-147 | + |
| 1-148 | +++ |
| 1-149 | +++ |
| 6-102 | + |
| 6-103 | + |
| 6-104 | + |
| 6-105 | + |
| 6-106 | + |
| 6-107 | ++ |
| 15-3 | ++ |
| 15-4 | ++ |

### <Test Example 2>

### Histone H3K9 dimethylation inhibitory activity test

The inhibitory activity of each compound against G9a was evaluated by adding the compound to a human lung cancer cell line NCI-H460, and detecting change in intracellular histone H3K9 dimethylation level by Western blotting. The NCI-H460 cells were cultured in a DMEM medium (FUJIFILM Wako Pure Chemical Corp., 044-29765) containing 10% fetal bovine serum and 4 mM glutamine. The cultured cells were washed with PBS and then detached with trypsin/EDTA, and the cell suspension was adjusted to 2.5 × 10⁴ cells/mL. The cell suspension was inoculated at 500 µL/well to a 24-well microplate (Thermo Fisher Scientific, Inc., 142475) and cultured overnight under conditions of 37°C and 5% CO₂. On the next day, the test compound (solution in DMSO) was added at 0.5 µL/well and reacted for 72 hours under conditions of 37°C and 5% CO₂. DMSO was added at 0.5 µL/well to control wells (final DMSO concentration: 0.1%). The medium was removed, and the cells were washed with PBS. Then, a 2 × SDS-PAGE sample buffer (100 mM Tris-HCl (pH 6.8), 4% sodium lauryl sulfate, 2% 2-mercaptoethanol, 20% glycerol, 0.005% bromophenol blue) was added at 50 µL/well to prepare a sample. The recovered sample was heated at 95 to 100°C for 10 minutes, and protein was separated by polyacrylamide gel electrophoresis (SDS-PAGE). Subsequently, the separated protein was transferred to a polyvinylidene fluoride (PVDF) membrane by semi-dry blotting. The PVDF membrane was dipped in 3% skimmed milk (dissolved in PBS containing 0.05% Tween 20 (PBST)), blocked at room temperature for 30 to 60 minutes, then dipped in an anti-dimethylated histone H3K9 antibody (Abcam plc, ab1220) diluted 1000-fold with 3% skimmed milk, and reacted overnight at 4°C. On the next day, the PVDF membrane was washed three times with PBST, then dipped in a HRP (horseradish peroxidase)-labeled anti-mouse IgG antibody diluted 10000-fold with skimmed milk, and reacted at room temperature for 1 hour. The PVDF membrane was washed three times with PBST, then dipped in chemiluminescent substrate solution Immobilon Western Chemiluminescent HRP Substrate (Merck Millipore, WBKLS0500), incubated at room temperature for 5 minutes, and photographed with Lumino Image Analyzer (GE Healthcare Japan Corp., ImageQuant LAS4000).

Then, the antibody was stripped from the PVDF membrane using re-probe reagent EzReprobe (ATTO Corp., WSE-7240), and trimethylated histone H3K9 and histone H3 were detected by the same procedures as above. An anti-trimethyl histone H3K9 antibody (39161, diluted 1000-fold) from Active Motif, Inc. and an anti-histone H3 antibody (ab1791, diluted 2000-fold) from Abcam plc were used in the detection. The band of each protein was quantified from the taken image using image processing software ImageJ (NIH). The ratios of dimethylated histone H3K9 and trimethylated histone H3K9 to the histone H3 protein were calculated with the numeric value of a compound-unsupplemented sample defined as 100%. A compound concentration at which the ratio of dimethylated histone H3K9 was 50% was calculated as a 50% inhibition concentration (IC₅₀ value) using data analysis software Origin (LightStone Corp.). The results are shown below.

The table indicates IC₅₀ < 50 nM: +++, 50 nM ≤ IC₅₀ < 500 nM: ++, 500 nM ≤ IC₅₀: +.

**[Table 171]**

| Example No. | Inhibitory activity |
|---|---|
| 1-68 | ++ |
| 1-79 | +++ |
| 1-80 | ++ |
| 1-81 | ++ |
| 1-82 | +++ |
| 1-87 | ++ |
| 2-1 | +++ |
| 6-10 | ++ |
| 6-17 | +++ |
| 6-26 | +++ |
| 6-30 | +++ |
| 6-32 | +++ |
| 6-33 | ++ |
| 6-34 | +++ |
| 6-49 | ++ |
| 6-51 | ++ |
| 6-52 | ++ |
| 6-55 | ++ |
| 6-62 | ++ |
| 6-88 | +++ |
| 6-89 | ++ |
| 11-4 | + |
| 12-2 | ++ |
| 22-2 | +++ |
| 35-1 | + |
| 35-2 | + |

### <Test Example 3>

### Colony formation inhibition test

Each compound was evaluated for its colony formation inhibitory activity against a human lung cancer cell line NCI-H460. The NCI-H460 cells were cultured in a DMEM medium (FUJIFILM Wako Pure Chemical Corp., 044-29765) containing 10% fetal bovine serum and 4 mM glutamine. The cultured cells were washed with PBS, then detached with trypsin/EDTA, and adjusted to 2 × 10³ cells/mL to prepare a cell suspension. The cell suspension was inoculated at 1 mL/well to a 12-well microplate (Corning Inc., 3513), and the test compound (DMSO solution) was added at 1 µL/well, followed by culture for 12 days under conditions of 37°C and 5% CO₂. DMSO was added at 1 µL/well to control wells with a final DMSO concentration set to 0.1%. The medium was discarded every day or every two days, and a fresh medium was added at 1 mL/well. The test compound or DMSO was added at 1 µL/well. The proportion of a colony of each well was measured using a high-speed cell imaging system (Tomy Digital Biology Co., Ltd., Celigo). The ratio of the colony in each compound-supplemented group was determined with the ratio of the colony formed in a test compound solution-unsupplemented well defined as 1. A compound concentration necessary for suppressing the rate of colony formation by 50% of the control (GI₅₀) was calculated using data analysis software Origin (LightStone Crop.). The results are shown below.

The table indicates GI₅₀ < 500 nM: +++, 500 nM ≤ GI₅₀ < 1000 nM: ++, 1000 nM ≤ GI₅₀: +.

**[Table 172]**

| Example No. | Inhibitory activity |
|---|---|
| 1-68 | + |
| 1-79 | +++ |
| 1-80 | ++ |
| 1-81 | +++ |
| 1-82 | + |
| 1-87 | + |
| 2-1 | ++ |
| 6-10 | ++ |
| 6-17 | ++ |
| 6-26 | +++ |
| 6-30 | +++ |
| 6-32 | +++ |
| 6-33 | + |
| 6-34 | ++ |
| 6-49 | +++ |
| 6-51 | +++ |
| 6-52 | ++ |
| 6-55 | +++ |
| 6-62 | +++ |
| 6-88 | +++ |
| 6-89 | +++ |
| 11-4 | + |
| 12-2 | + |
| 22-2 | ++ |
| 35-1 | + |
| 35-2 | + |

### <Test Example 4>

### Globin gene expression test

Effect of each obtained compound on a fetal globin gene expression in a human erythroblast cell line HUDEP-2 was evaluated by quantitative PCR. The HUDEP-2 cells were cultured in Stemline II Hematopoietic Stem Cell Expansion Medium (Sigma-Aldrich Co. LLC) containing 1 µM dexamethasone (Sigma-Aldrich Co. LLC), 1 µg/ml doxycycline (Sigma-Aldrich Co. LLC), 50 ng/ml recombinant human SCF (R&D Systems, Inc.), and 3 IU/mL Epoetin Alfa (Toho Pharmaceutical Co., Ltd.). The cultured cells were adjusted to 1 × 10⁵ cells/mL with a cell culture medium to prepare a cell suspension. Subsequently, the cell suspension was inoculated at 2 mL/well to a 6-well plate (VIOLAMO), and the test compound (solution in DMSO) was added at 2 µL/well (final DMSO solution: 0.1%), followed by culture for 4 days under conditions of 37°C and 5% CO₂. A DMSO solution was added at 2 µL/well as a control. The cultured cells were recovered by centrifugation at 7500 rpm at 4°C for 5 minutes, and RNA was extracted using Tissue Total RNA Mini Kit (Chiyoda Science Co., Ltd.). Then, cDNA was synthesized through reverse transcription reaction at 37°C for 15 minutes and then at 50°C for 5 minutes using ReverTra Ace qPCR RT Master Mix (Toyobo Co., Ltd.). Diluted cDNA was mixed as a template with THUNDERBIRD SYBR qPCR (Toyobo Co., Ltd.), and fetal β-globin gene, adult β-globin gene, and GAPDH gene as a reference were measured using CFX Connect Real-Time PCR Detection System (Bio-Rad Laboratories, Inc.). The primers used for the genes are as follows: fetal β-globin, forward: 5'-TGGATGATCTCAAGGGCAC-3'; reverse: 5'-TCAGTGGTATCTGGAGGACA-3', adult β-globin, forward: 5'-CAGTGCAGGCTGCCTATC-3'; reverse: 5'-ATACTTGTGGGCCAGGGCAT-3', and GAPDH, forward: 5'-GCACCGTCAAGGCTGAGAAC-3'; reverse: 5'-TGGTGAAGACGCCAGTGGA-3'. In gene expression analysis, the gene expression was compared by the ΔΔCt method with the control supplemented with the 0.1% DMSO solution, defined as 1.

### Industrial Applicability

Owing to their G9a enzyme inhibitory activity, the compounds according to the present invention are useful as therapeutic or prophylactic drugs for proliferative diseases such as cancer, β-globin abnormality, fibrosis, pain, neurodegenerative diseases, Prader-Willi syndrome, malaria, viral infections, myopathy, autism, and the like.

## Claims

1. A compound represented by the general formula (I): 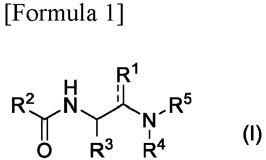 wherein
R¹ is an oxygen atom, a nitrogen atom or a hydrogen atom;
when R¹ is an oxygen atom or a nitrogen atom, the bond between R¹ and the carbon atom is a double bond;
when R¹ is a hydrogen atom, the bond between R¹ and the carbon atom is a single bond;
R² is the following A1), A2) or A3), and ^{∗} represents a binding position to -CO- in the formula (I): 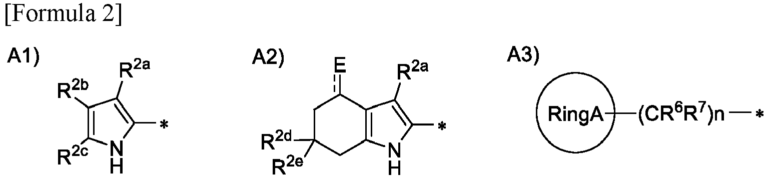 E is an oxygen atom or a hydrogen atom;
when E is an oxygen atom, the bond between E and the carbon atom is a double bond;
when E is a hydrogen atom, the bond between E and the carbon atom is a single bond;
R^{2a}, R^{2b} and R^{2c} are each independently a hydrogen atom, a C₁ to C₆ alkyl group, a C₂ to C₆ alkenyl group, a halo-Ci to C₆ alkyl group, a C₁ to C₆ alkoxy group, a C₁ to C₆ alkylamino group, a C₁ to C₆ acyl group, a C₁ to C₆ alkoxycarbonyl group, a C₃ to C₁₀ cycloalkyl group or a hydroxy-Ci to C₆ alkyl group (the C₁ to C₆ alkyl group, the C₂ to C₆ alkenyl group, the halo-C₁ to C₆ alkyl group, the C₁ to C₆ alkoxy group, the C₁ to C₆ alkylamino group, the C₁ to C₆ acyl group, the C₁ to C₆ alkoxycarbonyl group, the C₃ to C₁₀ cycloalkyl group and the hydroxy-Ci to C₆ alkyl group are each optionally substituted with one or more substituents selected from the group consisting of a C₁ to C₆ alkyl group, a C₁ to C₆ alkoxy group, a C₁ to C₆ alkylamino group and a hydroxy-Ci to C₆ alkyl group, and these substituents are optionally bonded to each other to form a ring);
R^{2b} and R^{2c} are optionally bonded to each other to form a ring;
R^{2d} and R^{2e} are each independently a C₁ to C₆ alkyl group or a hydroxy-Ci to C₆ alkyl group;
R^{2d} and R^{2e} are optionally bonded to each other to form a ring;
R⁶ and R⁷ are each independently a hydrogen atom, a C₁ to C₆ alkyl group, an aromatic hydrocarbon ring group, a C₃ to C₁₀ cycloalkyl group, a 5- to 10-membered heteroaryl group or a 3- to 10-membered heterocycloalkyl group;
R⁶ and R⁷ are optionally bonded to each other to form a ring;
n is 0 or 1;
RingA is an aromatic hydrocarbon ring group, a C₃ to C₁₀ cycloalkyl group, a 5- to 10-membered heteroaryl group or a 3- to 10-membered heterocycloalkyl group and is optionally substituted with R⁸ and R⁹;
R⁸ is a hydrogen atom, a halogen atom, a cyano group, an amino group, an aminosulfonyl group (-SO₂NH₂), a C₁ to C₆ alkyl group, a halo-C₁ to C₆ alkyl group or a C₁ to C₆ alkoxy group;
R⁹ is -Y-Z;
Y is a bond, -O-, -NR¹⁰- or -(CR¹¹R¹²)ₛ-;
R¹⁰, R¹¹ and R¹² are each independently a hydrogen atom or a C₁ to C₆ alkyl group;
s is an integer of 0 to 6;
Z is a hydrogen atom, a C₁ to C₆ alkyl group, a halo-C₁ to C₆ alkyl group, a C₁ to C₆ alkoxy group, a C₁ to C₆ alkylamino group, an aromatic hydrocarbon ring group, a C₃ to C₁₀ cycloalkyl group, a 5- to 10-membered heteroaryl group or a 3- to 10-membered heterocycloalkyl group (the C₁ to C₆ alkyl group, the aromatic hydrocarbon ring group, the C₃ to C₁₀ cycloalkyl group, the 5- to 10-membered heteroaryl group and the 3- to 10-membered heterocycloalkyl group are each optionally substituted with one or more substituents selected from the group consisting of a C₁ to C₆ alkyl group, a halo-Ci to C₆ alkyl group, a C₁ to C₆ alkoxy group, a C₁ to C₆ alkylamino group, a C₁ to C₆ acyl group and a C₁ to C₆ alkoxycarbonyl group);
R³ is ^{∗}-(CH₂)ₓ-T-(CR¹³R¹⁴)_{y}-U, and ^{∗} represents a binding position to -CH- in the formula (I); T is a bond, -NH-, -O- or -S(O)ₚ-;
U is a hydrogen atom, a C₃ to C₁₀ cycloalkyl group or an aromatic hydrocarbon ring group (the aromatic hydrocarbon ring group is optionally substituted with one or more halogen atoms); R¹³ and R¹⁴ are each independently a hydrogen atom or a C₁ to C₆ alkyl group;
x and y are each independently an integer of 0 to 4;
p is an integer of 0 to 2;
R⁴ is a hydrogen atom or a C₁ to C₆ alkyl group;
R⁵ is the following B1) or a C₁ to C₆ alkyl group, and ^{∗} represents a binding position to -N- in the formula (I): 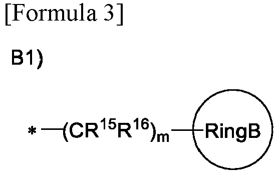
R¹⁵ and R¹⁶ are each independently a hydrogen atom, a C₁ to C₆ alkyl group or a hydroxy-Ci to C₆ alkyl group;
R¹⁵ and R¹⁶ are optionally bonded to each other to form a ring;
R¹⁵ and R¹⁶ are optionally bonded to RingB to form a ring;
m is 0 or 1;
RingB is an aromatic hydrocarbon ring group, a C₃ to C₁₀ cycloalkyl group, a 5- to 10-membered heteroaryl group or a 3- to 10-membered heterocycloalkyl group and is optionally substituted with R¹⁷ , R¹⁸ and R¹⁹;
R¹⁷ and R¹⁸ are each independently a hydrogen atom, a halogen atom, a hydroxy group, an amino group, a cyano group, a carbamoyl group (-CONH₂), a C₁ to C₆ alkyl group, a halo-Ci to C₆ alkyl group, a hydroxy-Ci to C₆ alkyl group, a C₁ to C₆ alkoxy group, a C₁ to C₆ alkylamino group, a C₁ to C₆ alkylsulfanyl group, a halo-Ci to C₆ alkylsulfanyl group, a C₁ to C₆ acyl group, a C₁ to C₆ alkoxycarbonyl group, a C₁ to C₆ alkylaminocarbonyl group or a C₁ to C₆ acylamino group;
R¹⁹ is -(CR²⁰R²¹)ᵣ-V-(CR2²R²³)_{q}-Q;
V is a bond, -O-, -NR²⁴- or -S(O)ₜ-;
t is an integer of 0 to 2;
R²⁰, R²¹, R²², R²³ and R²⁴ are each independently a hydrogen atom or a C₁ to C₆ alkyl group;
q and r are each independently an integer of 0 to 6;
Q is a hydrogen atom, an amino group, a hydroxy group, a C₁ to C₆ alkyl group, a C₁ to C₆ alkylamino group, a C₃ to C₁₀ cycloalkyl group, a 5- to 10-membered heteroaryl group or a 3-to 10-membered heterocycloalkyl group (the C₁ to C₆ alkylamino group, the C₃ to C₁₀ cycloalkyl group, the 5- to 10-membered heteroaryl group and the 3- to 10-membered heterocycloalkyl group are each optionally substituted with one or more substituents selected from the group consisting of a halogen atom, a C₁ to C₆ alkyl group, a C₁ to C₆ alkoxy group, a C₁ to C₆ alkylamino group, a C₁ to C₆ alkoxycarbonyl group, a C₁ to C₆ alkylaminocarbonyl group and a hydroxy-Ci to C₆ alkyl group);
R¹⁷, R¹⁸ and R¹⁹ are optionally bonded to each other to form a ring;
R⁴ and R⁵ are optionally bonded to each other to form a ring; and
when R¹ is a nitrogen atom, m is 0, and RingB is a phenyl group optionally substituted with R¹⁷, R¹⁸ and R¹⁹, R¹ is optionally bonded to the phenyl group to form a benzimidazole ring,
or a pharmacologically acceptable salt thereof.

2. The compound according to claim 1 or a pharmacologically acceptable salt thereof, wherein in the formula (I), R¹ is an oxygen atom.

3. The compound according to claim 2 or a pharmacologically acceptable salt thereof, wherein in the formula (I), R² is the following A1) or A2):

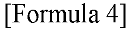

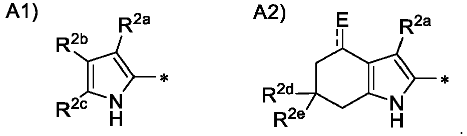

4. The compound according to claim 3 or a pharmacologically acceptable salt thereof, wherein in the formula (I), R² is the following A2): 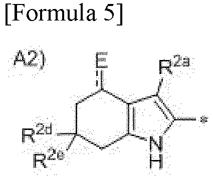 wherein R^{2a} is a C₁ to C₆ alkyl group, a C₃ to C₁₀ cycloalkyl group or a hydroxy-Ci to C₆ alkyl group.

5. The compound according to claim 4 or a pharmacologically acceptable salt thereof, wherein in the formula (I), R² is the following A2b):

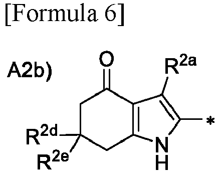

6. The compound according to claim 5 or a pharmacologically acceptable salt thereof, wherein in the formula (I), R² is the following A2b): 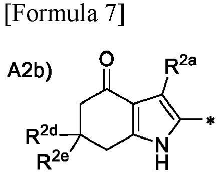
R^{2a} is a C₁ to C₆ alkyl group or a C₃ to C₁₀ cycloalkyl group;
R^{2d} and R^{2e} are each independently a C₁ to C₆ alkyl group; and
R^{2d} and R^{2e} are optionally bonded to each other to form a ring.

7. The compound according to claim 6 or a pharmacologically acceptable salt thereof, wherein in the formula (I),
R³ is ^{∗}-(CH₂)ₓ-T-(CR¹³R¹⁴)_{y}-U, and ^{∗} represents a binding position to -CH- in the formula (I);
T is a bond or -S(O)ₚ-;
U is a hydrogen atom, a C₃ to C₁₀ cycloalkyl group or an aromatic hydrocarbon ring group (the aromatic hydrocarbon ring group is optionally substituted with one or more halogen atoms);
R¹³ and R¹⁴ are each independently a hydrogen atom or a C₁ to C₆ alkyl group;
x and y are each independently an integer of 0 to 2;
p is 0;
(except for the case where R³ is a hydrogen atom or a methyl group)
R⁵ is the following B1) or a C₁ to C₆ alkyl group, and ^{∗} represents a binding position to -N- in the formula (I): 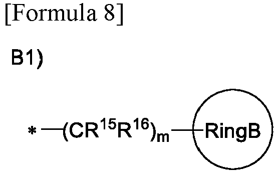
RingB is an aromatic hydrocarbon ring group, a C₃ to C₁₀ cycloalkyl group, a 5- to 10-membered heteroaryl group or a 3- to 10-membered heterocycloalkyl group and is optionally substituted with R¹⁷, R¹⁸ and R¹⁹; and
R¹⁷ and R¹⁸ are each independently a hydrogen atom, a halogen atom, a hydroxy group, an amino group, a cyano group, a C₁ to C₆ alkyl group, a halo-C₁ to C₆ alkyl group, a hydroxy-Ci to C₆ alkyl group, a C₁ to C₆ alkoxy group, a C₁ to C₆ alkylamino group, a C₁ to C₆ alkylsulfanyl group, a C₁ to C₆ alkoxycarbonyl group, a C₁ to C₆ alkylaminocarbonyl group or a C₁ to C₆ acylamino group.

8. The compound according to claim 7 or a pharmacologically acceptable salt thereof, wherein in the formula (I),
R³ is a group represented by ^{∗}-(CH₂)ₓ-T-(CR¹³R¹⁴)_{y}-U, and ^{∗} represents a binding position to - CH- in the formula (I);
T is a bond;
U is a hydrogen atom or a C₃ to C₁₀ cycloalkyl group;
each of R¹³ and R¹⁴ is a hydrogen atom;
x and y are each independently an integer of 1 or 2;
R⁵ is the following B1) or a C₁ to C₆ alkyl group, and ^{∗} represents a binding position to -N- in the formula (I): 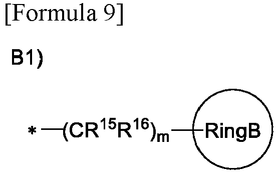
RingB is an aromatic hydrocarbon ring group, a C₃ to C₁₀ cycloalkyl group, a 5- to 10-membered heteroaryl group or a 3- to 10-membered heterocycloalkyl group and is optionally substituted with R¹⁷, R¹⁸ and R¹⁹; and
R¹⁷ and R¹⁸ are each independently a hydrogen atom, a halogen atom, a hydroxy group, an amino group, a cyano group, a C₁ to C₆ alkyl group, a halo-C₁ to C₆ alkyl group, a hydroxy-Ci to C₆ alkyl group or a C₁ to C₆ alkoxy group.

9. The compound according to claim 8 or a pharmacologically acceptable salt thereof, wherein in the formula (I),
R^{2a}, R^{2d} and R^{2e} are each independently a C₁ to C₃ alkyl group; and
R³ is a n-butyl group or a 2-cyclopropylethan-1-yl group.

10. The compound according to claim 3 or a pharmacologically acceptable salt thereof, wherein in the formula (I),
R² is the following A1): 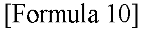 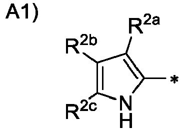 R^{2a}, R^{2b} and R^{2c} are each independently a C₁ to C₆ alkyl group, a C₁ to C₆ alkoxycarbonyl group or a C₃ to C₁₀ cycloalkyl group;
R³ is ^{∗}-(CH₂)ₓ-T-(CR¹³R¹⁴)_{y}-U, and ^{∗} represents a binding position to -CH- in the formula (I):
T is a bond;
U is a hydrogen atom or a C₃ to C₁₀ cycloalkyl group;
each of R¹³ and R¹⁴ is a hydrogen atom; and
x and y are each independently an integer of 1 or 2.

11. The compound according to claim 3 or a pharmacologically acceptable salt thereof, wherein in the formula (I),
R² is the following A1a): 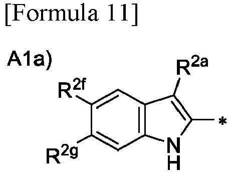 R^{2a} is a hydrogen atom or a C₁ to C₆ alkyl group;
R^{2f} is a C₁ to C₆ alkoxy group;
R^{2g} is a hydrogen atom or a C₁ to C₆ alkoxy group;
R³ is ^{∗}-(CH₂)ₓ-T-(CR¹³R¹⁴)_{y}-U, and ^{∗} represents a binding position to -CH- in the formula (I):
T is a bond;
U is a hydrogen atom or a C₃ to C₁₀ cycloalkyl group;
each of R¹³ and R¹⁴ is a hydrogen atom; and
x and y are each independently an integer of 1 or 2.

12. The compound according to claim 2 or a pharmacologically acceptable salt thereof, wherein in the formula (I),
R⁵ is the following B1a), and ^{∗} represents a binding position to -N- in the formula (I): 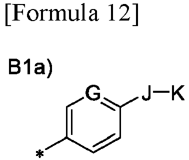 G is CH or N;
J is a bond, -O- or -NR²⁵-;
R²⁵ is a hydrogen atom or a C₁ to C₆ alkyl group; and
K is a hydrogen atom, a cyano group, a C₁ to C₆ alkyl group, a halo-C₁ to C₆ alkyl group or a 3- to 10-membered heterocycloalkyl group (the 3- to 10-membered heterocycloalkyl group is optionally substituted with one or more C₁ to C₆ alkyl groups).

13. The compound according to claim 12 or a pharmacologically acceptable salt thereof, wherein in the formula (I),
R³ is ^{∗}-(CH₂)ₓ-T-(CR¹³R¹⁴)_{y}-U, and ^{∗} represents a binding position to -CH- in the formula (I);
T is a bond;
U is a hydrogen atom or a C₃ to C₁₀ cycloalkyl group;
each of R¹³ and R¹⁴ is a hydrogen atom;
x and y are each independently an integer of 1 or 2;
R⁵ is the following B1b), and ^{∗} represents a binding position to -N- in the formula (I): 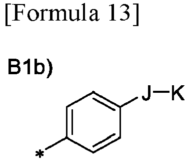
J is a bond, -O- or -NR²⁵-;
R²⁵ is a hydrogen atom or a C₁ to C₆ alkyl group; and
K is a hydrogen atom, a cyano group, a C₁ to C₆ alkyl group, a halo-C₁ to C₆ alkyl group or a 3- to 10-membered heterocycloalkyl group (the 3- to 10-membered heterocycloalkyl group is optionally substituted with one or more C₁ to C₆ alkyl groups).

14. The compound according to claim 12 or a pharmacologically acceptable salt thereof, wherein in the formula (I),
R⁵ is the following B1c), and ^{∗} represents a binding position to -N- in the formula (I): 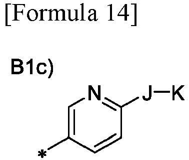 J is a bond, -O- or -NR²⁵-;
R²⁵ is a hydrogen atom or a C₁ to C₆ alkyl group; and
K is a hydrogen atom, a cyano group, a C₁ to C₆ alkyl group, a halo-C₁ to C₆ alkyl group or a 3- to 10-membered heterocycloalkyl group (the 3- to 10-membered heterocycloalkyl group is optionally substituted with one or more C₁ to C₆ alkyl groups).

15. The compound according to claim 14 or a pharmacologically acceptable salt thereof, wherein in the formula (I), R² is the following A3):

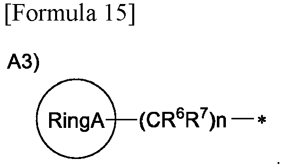

16. The compound according to claim 15 or a pharmacologically acceptable salt thereof, wherein in the formula (I),
R² is the following A3): 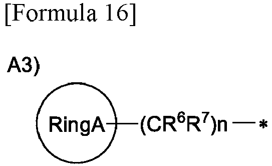 n is 0;
R³ is ^{∗}-(CH₂)ₓ-T-(CR¹³R¹⁴)_{y}-U, and ^{∗} represents a binding position to -CH- in the formula (I);
T is a bond;
U is a hydrogen atom or a C₃ to C₁₀ cycloalkyl group;
each of R¹³ and R¹⁴ is a hydrogen atom; and
x and y are each independently an integer of 1 or 2.

17. The compound according to claim 16 or a pharmacologically acceptable salt thereof, wherein in the formula (I),
R² is the following A3): 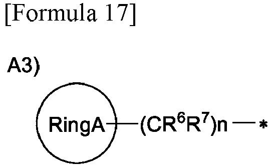 n is 0; and
RingA is an aromatic hydrocarbon ring group optionally substituted with R⁸ and R⁹.

18. The compound according to claim 17 or a pharmacologically acceptable salt thereof, wherein in the formula (I),
R² is the following A3a): 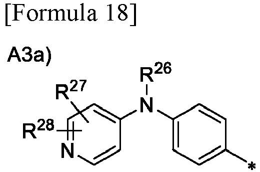 R²⁶ is a hydrogen atom or a C₁ to C₆ alkyl group; and
R²⁷ and R²⁸ are each independently a hydrogen atom, a C₁ to C₆ alkyl group or a halo-C₁ to C₆ alkyl group.

19. The compound according to claim 18 or a pharmacologically acceptable salt thereof, wherein in the formula (I),
R⁵ is the following B1c), and ^{∗} represents a binding position to -N- in the formula (I): 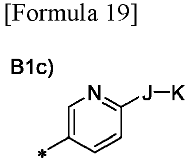 J is a bond or -O-; and
K is a hydrogen atom or a C₁ to C₆ alkyl group.

20. The compound according to claim 15 or a pharmacologically acceptable salt thereof, wherein in the formula (I),
R² is the following A3): 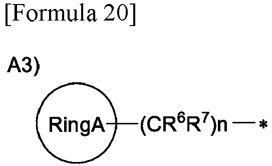 n is 1;
R³ is ^{∗}-(CH₂)ₓ-T-(CR¹³R¹⁴)_{y}-U, and ^{∗} represents a binding position to CH- in the formula (I);
T is a bond;
U is a hydrogen atom or a C₃ to C₁₀ cycloalkyl group;
each of R¹³ and R¹⁴ is a hydrogen atom; and
x and y are each independently an integer of 1 or 2.

21. The compound according to claim 20 or a pharmacologically acceptable salt thereof, wherein in the formula (I),
R² is the following A3): 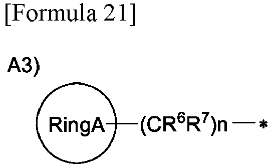 n is 1;
RingA is an aromatic hydrocarbon ring group optionally substituted with R⁸ and R⁹;
R⁹ is -Y-Z;
Y is a bond, -O-, -NR¹⁰- or -(CR¹¹R¹²)ₛ-;
R¹⁰, R¹¹ and R¹² are each independently a hydrogen atom or a C₁ to C₆ alkyl group;
s is an integer of 0 to 6; and
Z is a hydrogen atom, a C₁ to C₆ alkyl group, a halo-C₁ to C₆ alkyl group, a C₁ to C₆ alkoxy group or a C₁ to C₆ alkyl amino group.

22. The compound according to claim 1 or a pharmacologically acceptable salt thereof, wherein the compound represented by the general formula (I) is represented by the following formula (II):

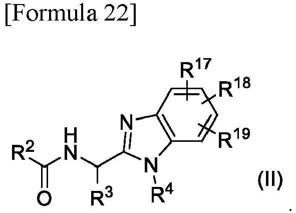

23. The compound according to claim 22 or a pharmacologically acceptable salt thereof, wherein the compound represented by the general formula (I) is represented by the following formula (II): 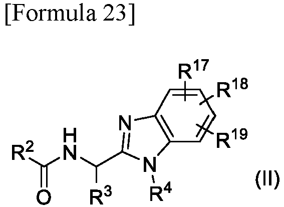 wherein
R² is the following A2b): 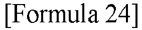 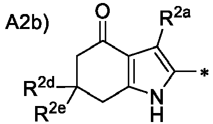 R^{2a} is a C₁ to C₆ alkyl group, a C₃ to C₁₀ cycloalkyl group or a hydroxy-Ci to C₆ alkyl group;
R^{2d} and R^{2e} are each independently a C₁ to C₆ alkyl group or a hydroxy-Ci to C₆ alkyl group;
R^{2d} and R^{2e} are optionally bonded to each other to form a ring;
R³ is ^{∗}-(CH₂)ₓ-T-(CR¹³R¹⁴)_{y}-U, and ^{∗} represents a binding position to -CH- in the formula (II);
T is a bond;
U is a hydrogen atom or a C₃ to C₁₀ cycloalkyl group;
each of R¹³ and R¹⁴ is a hydrogen atom; and
x and y are each independently an integer of 1 or 2.

24. The compound according to claim 1 or a pharmacologically acceptable salt thereof, wherein the compound represented by the general formula (I) is represented by the following formula (III): 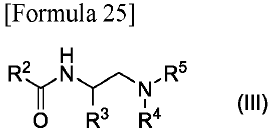 wherein
R² is the following A2b): 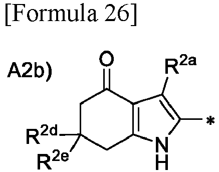 R^{2a}, R^{2d}, and R^{2e} are each independently a C₁ to C₃ alkyl group;
R³ is ^{∗}-(CH₂)ₓ-T-(CR¹³R¹⁴)_{y}-U, and ^{∗} represents a binding position to -CH- in the formula (III);
T is a bond;
U is a hydrogen atom or a C₃ to C₁₀ cycloalkyl group;
each of R¹³ and R¹⁴ is a hydrogen atom;
x and y are each independently an integer of 1 or 2;
R⁵ is the following B1), and ^{∗} represents a binding position to -N- in the formula (I): 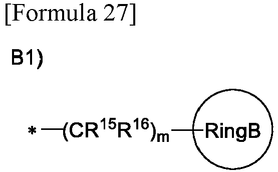
R¹⁵ and R¹⁶ are each independently a hydrogen atom or a C₁ to C₆ alkyl group;
m is 0 or 1;
RingB is an aromatic hydrocarbon ring group or a 5- to 10-membered heteroaryl group and is optionally substituted with R¹⁷, R¹⁸ and R¹⁹;
R¹⁷ and R¹⁸ are each independently a hydrogen atom, a cyano group, a C₁ to C₆ alkyl group or
a C₁ to C₆ alkoxy group;
R¹⁹ is -(CR²⁰R²¹)ᵣ-V-(CR2²²R²³)_{q}-Q;
V is a bond, -O- or -NR²⁴-;
each of R²⁰, R²¹, R²², R²³ and R²⁴ is a hydrogen atom;
q and r are each independently an integer of 0 to 2;
Q is a hydrogen atom, a C₁ to C₆ alkylamino group or a 3- to 10-membered heterocycloalkyl group (the 3- to 10-membered heterocycloalkyl group is optionally substituted with one or
more C₁ to C₆ alkyl groups); and
R⁴ and R⁵ are optionally bonded to each other to form a ring.

25. A compound selected from the following: 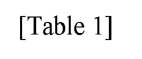 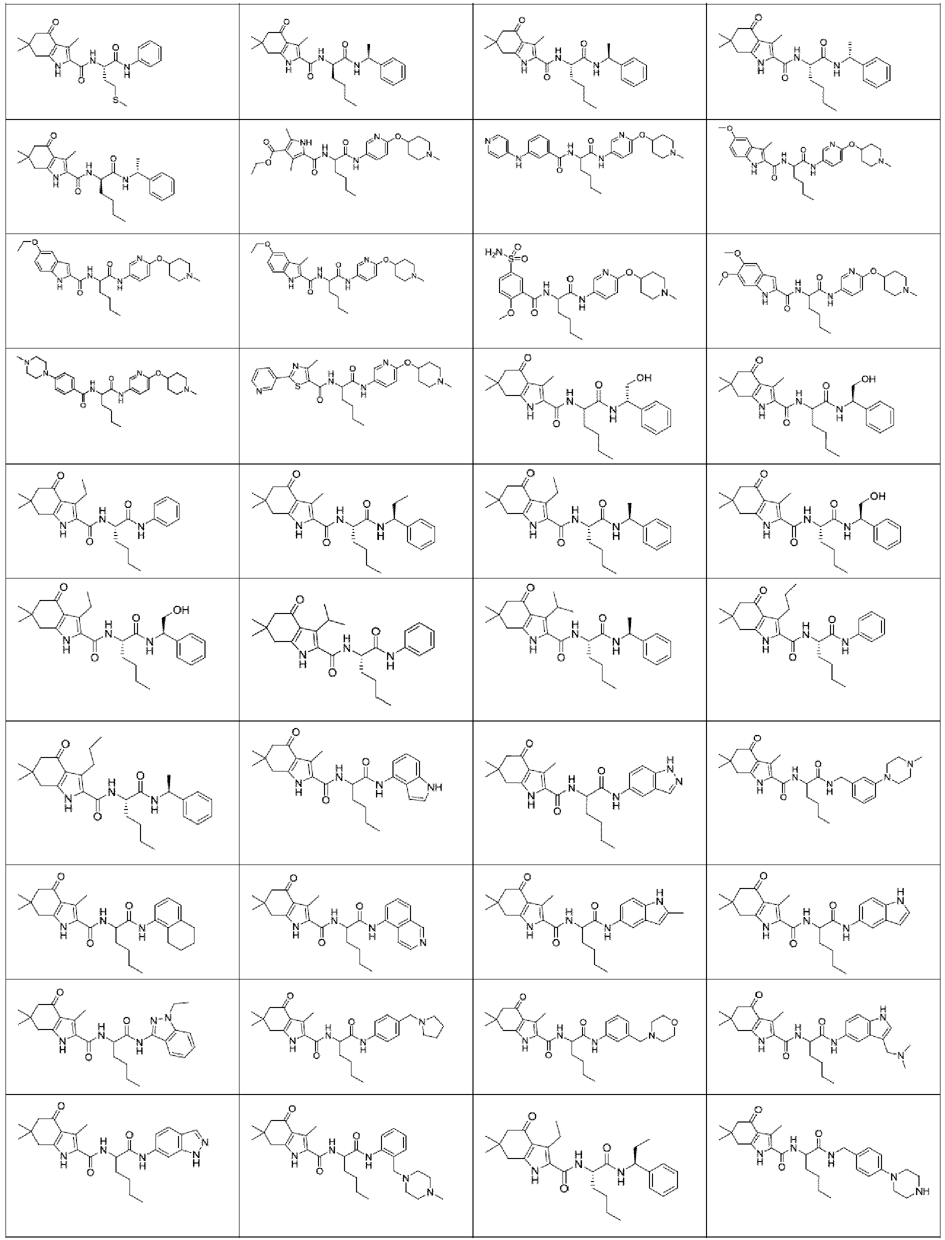 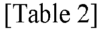 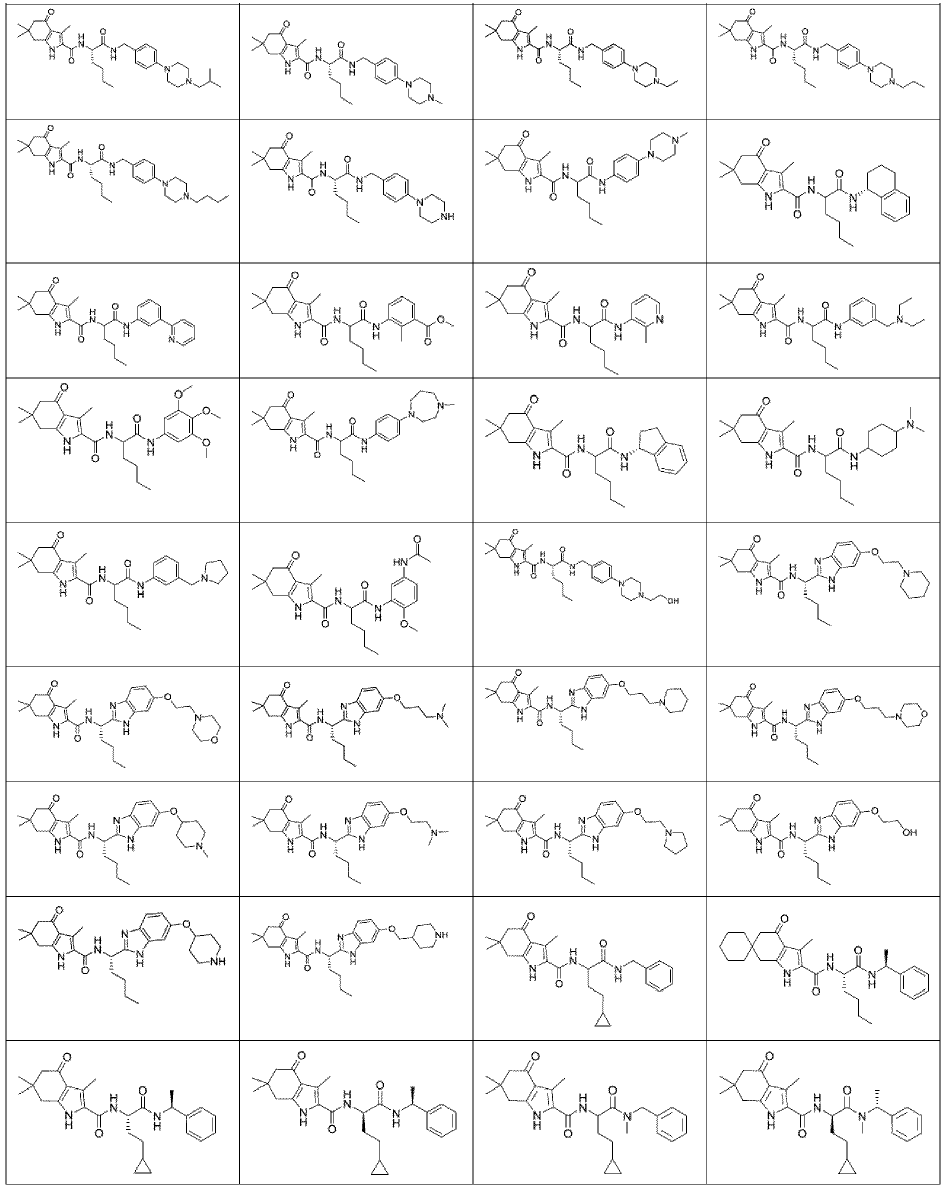 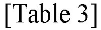 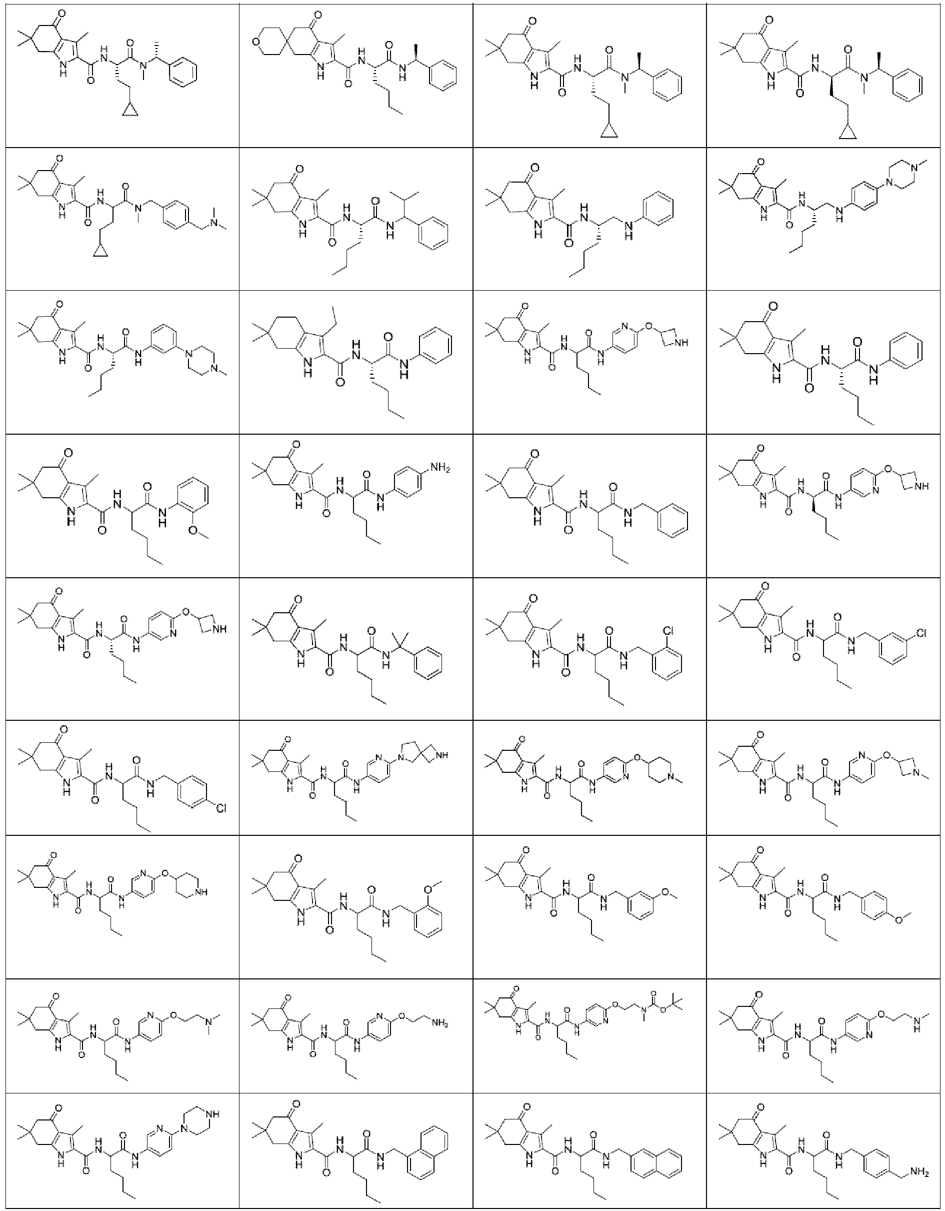 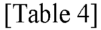 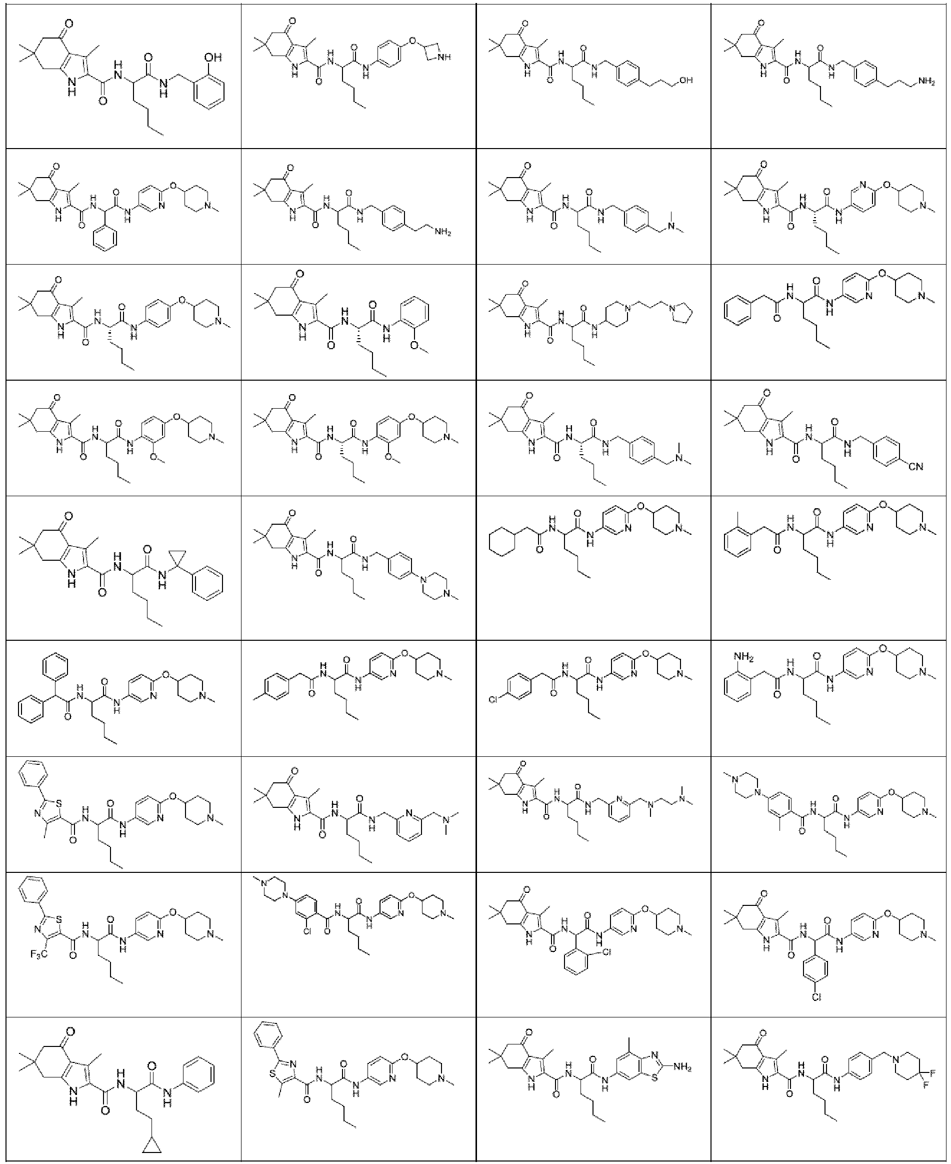 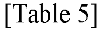 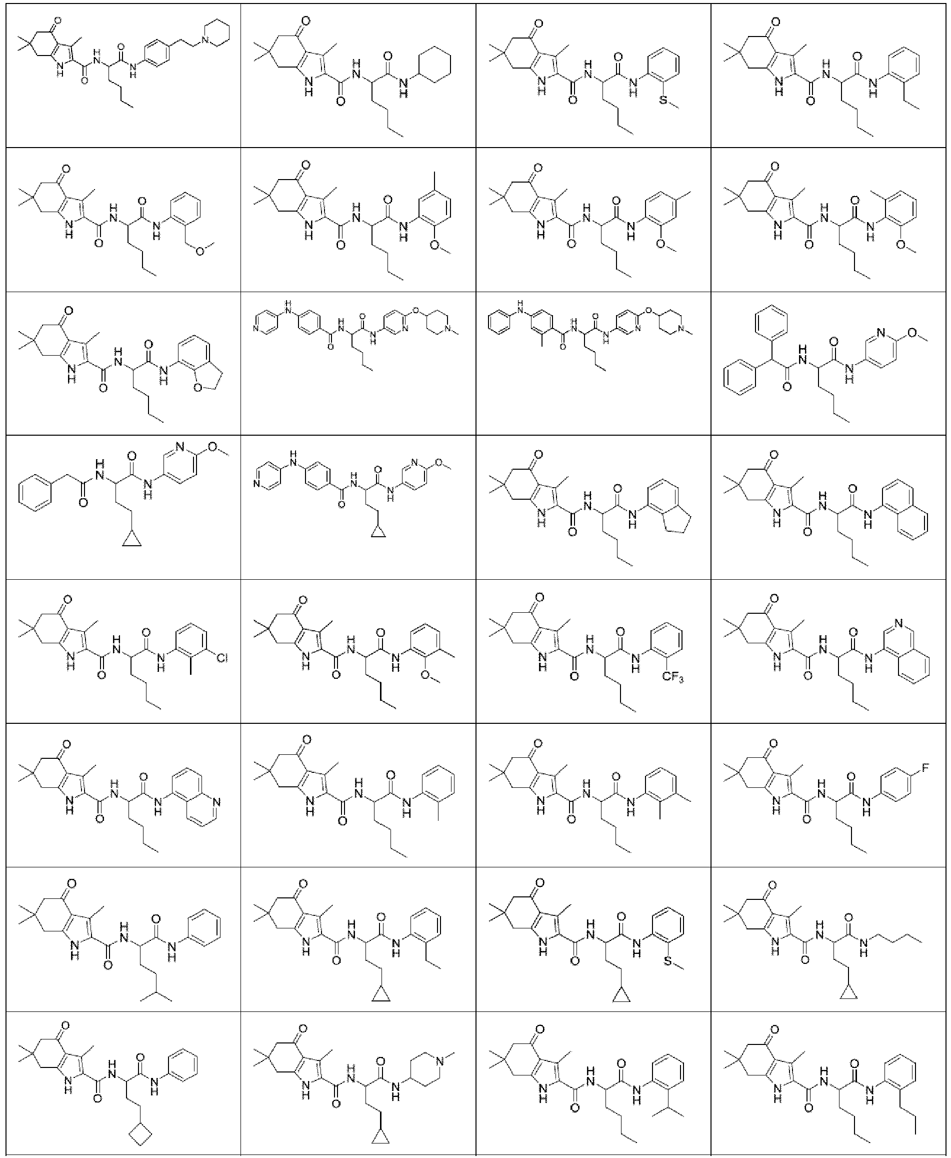 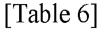 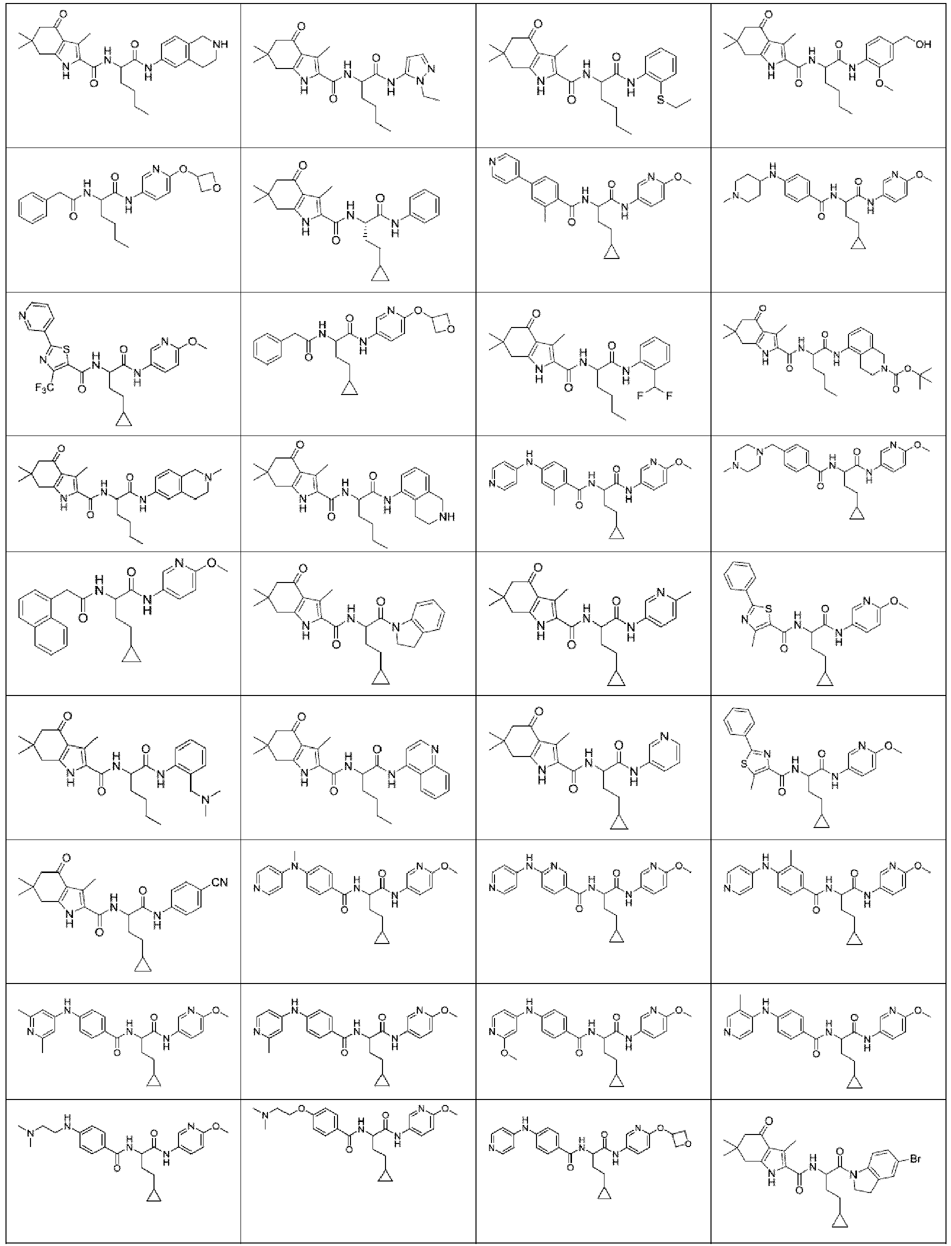 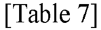 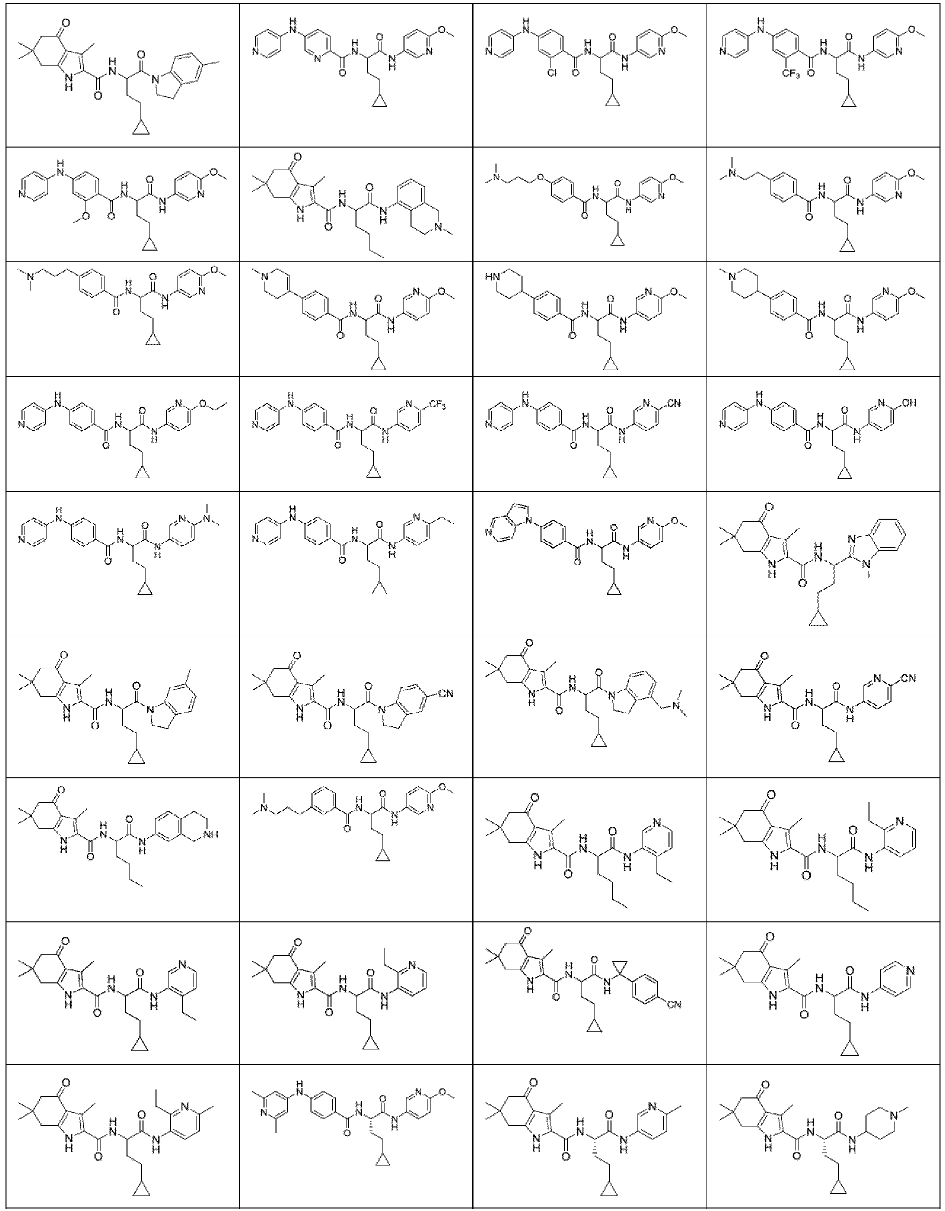 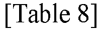 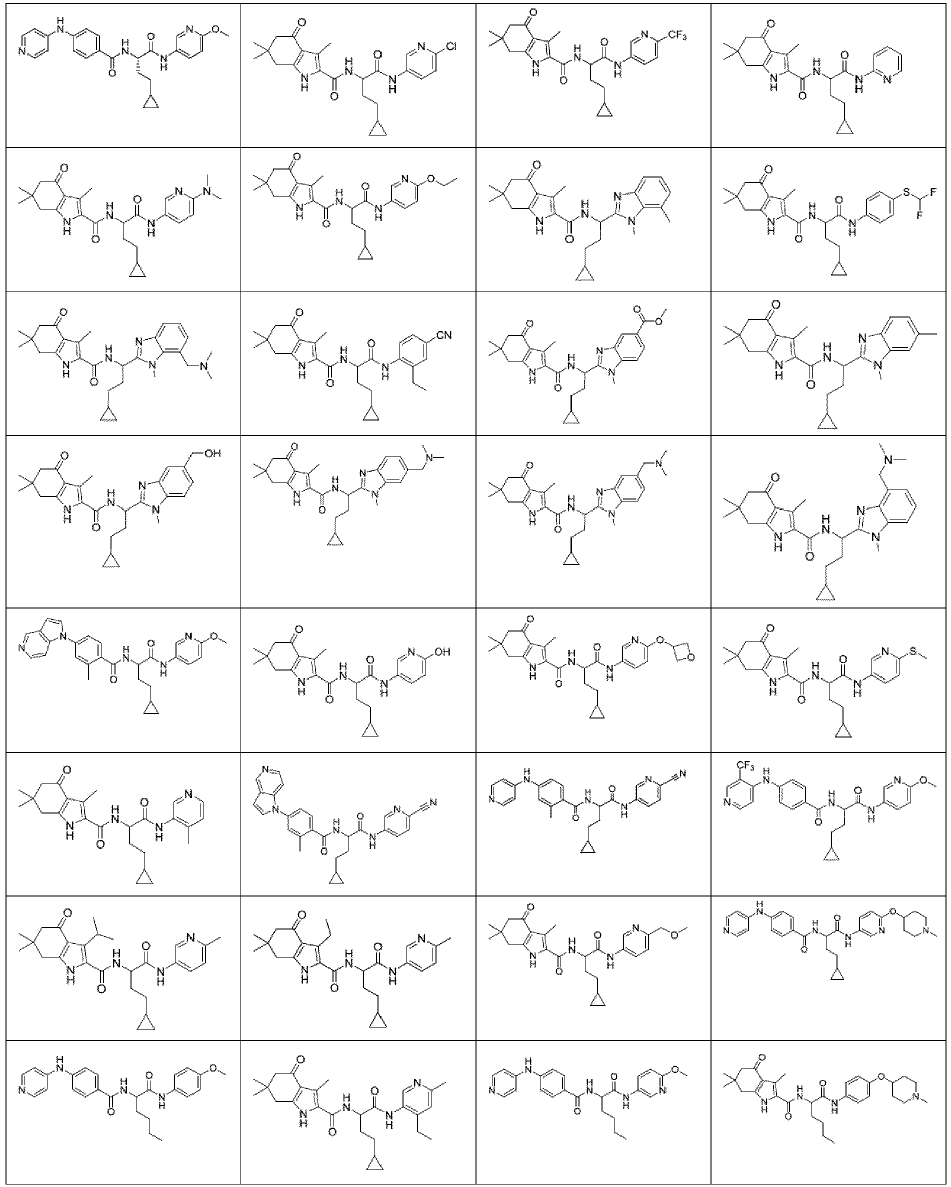 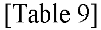 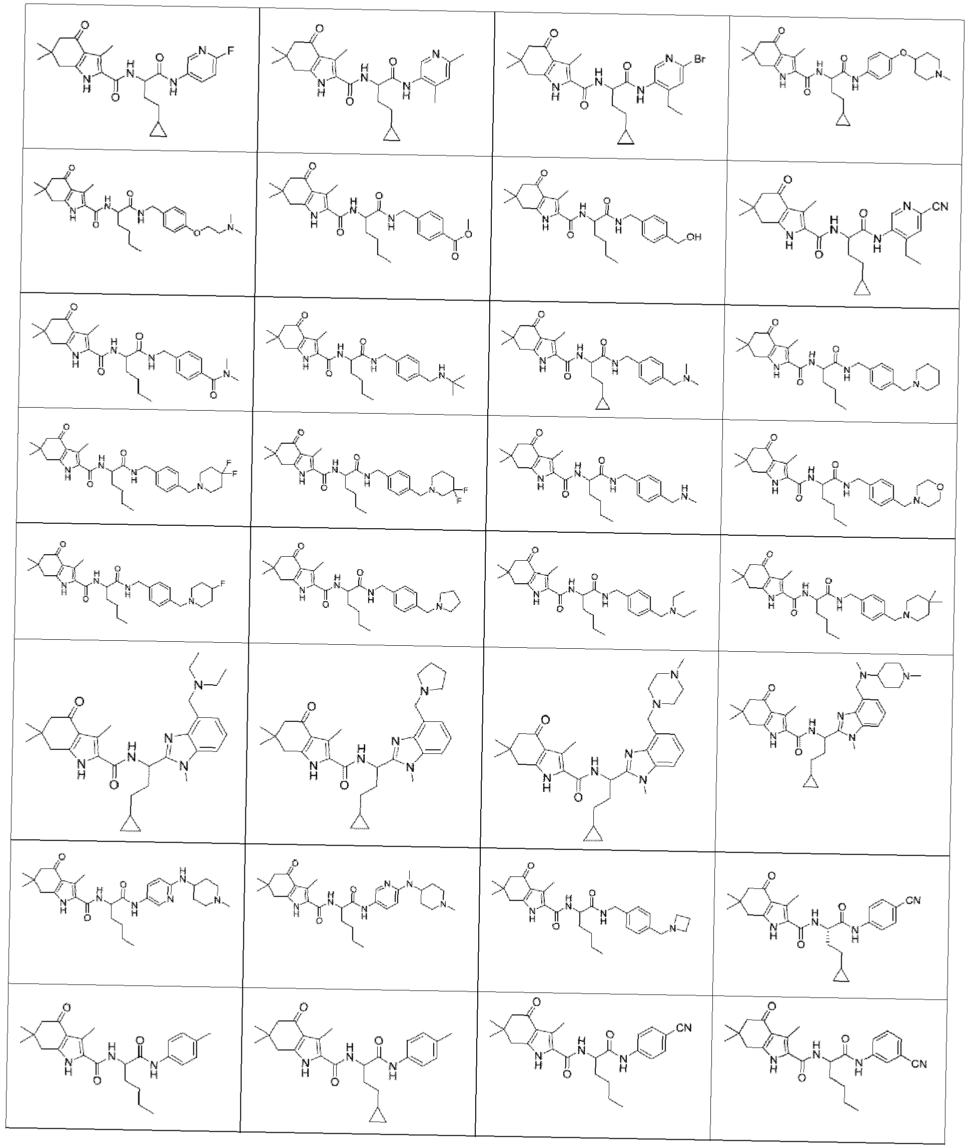 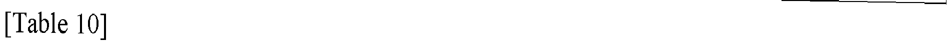 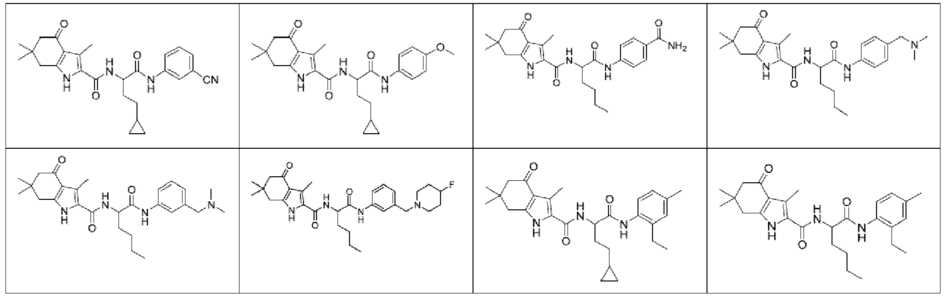 or a pharmacologically acceptable salt thereof.

26. A G9a enzyme-inhibiting composition comprising a compound according to any one of claims 1 to 25 or a pharmacologically acceptable salt thereof as an active ingredient.

27. A pharmaceutical composition comprising a compound according to any one of claims 1 to 25 or a pharmacologically acceptable salt thereof as an active ingredient.

28. A method for preventing or treating at least one disease selected from the disease group consisting of proliferative disease such as cancer, β-globin abnormality, fibrosis, pain, neurodegenerative disease, Prader-Willi syndrome, malaria, viral infection, myopathy, and autism, comprising administering a compound according to any one of claims 1 to 25 or a pharmacologically acceptable salt thereof.

29. Use of a compound according to any one of claims 1 to 25 or a pharmacologically acceptable salt thereof for producing a medicament for the prevention or treatment of at least one disease selected from the disease group consisting of proliferative disease such as cancer, β-globin abnormality, fibrosis, pain, neurodegenerative disease, Prader-Willi syndrome, malaria, viral infection, myopathy, and autism.

30. A pharmaceutical composition comprising a compound according to any one of claims 1 to 25 or a pharmacologically acceptable salt thereof and a pharmaceutically acceptable carrier for use in the prevention or treatment of at least one disease selected from the disease group consisting of proliferative disease such as cancer, β-globin abnormality, fibrosis, pain, neurodegenerative disease, Prader-Willi syndrome, malaria, viral infection, myopathy, and autism.
